(19) **Europäisches Patentamt / European Patent Office / Office européen des brevets**

(11) **EP 2 518 166 B1**

(12) **EUROPEAN PATENT SPECIFICATION**

(45) Date of publication and mention
of the grant of the patent:
**03.12.2014 Bulletin 2014/49**

(21) Application number: **12166440.3**

(22) Date of filing: **18.05.2006**

(51) Int Cl.:
*C12Q 1/68* *(2006.01)*       *G01N 33/53* *(2006.01)*

(54) **Thyroid fine needle aspiration molecular assay**

Molekulartest zur Schilddrüsen-Feinnadelaspiration

Dosage moléculaire par aspiration de la thyroïde au moyen d'une aiguille fine

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.05.2005 US 683173 P**

(43) Date of publication of application:
**31.10.2012 Bulletin 2012/44**

(62) Document number(s) of the earlier application(s) in
accordance with Art. 76 EPC:
**06770766.1 / 1 888 785**

(73) Proprietor: **Janssen Diagnostics, LLC
Raritan, NJ 08869 (US)**

(72) Inventors:
- **Jiang, Yuqiu
  San Diego, CA 92130 (US)**
- **Backus, John W.
  Ontario, NY 14519 (US)**
- **Mazumder, Abhijit
  Basking Ridge, NJ 07970 (US)**
- **Chowdary, Dondapati
  Princeton Junction, NJ 08550 (US)**
- **Yang, Fei
  San Diego, CA 92130 (US)**
- **Wang, Yixin
  Basking Ridge, NJ 07920 (US)**
- **Jatkoe, Timothy
  Bedminster, NJ 07921 (US)**

(74) Representative: **Goodfellow, Hugh Robin
Carpmaels & Ransford LLP
One Southampton Row
London WC1B 5HA (GB)**

(56) References cited:
**WO-A2-01/94629**

- **"[HG-U133A] Affymetrix Human Genome U133A Array", GEO,, 11 March 2002 (2002-03-11), XP002527544,**
- **HUANG Y ET AL: "Gene expression in papillary thyroid carcinoma reveals highly consistent profiles", PROCEEDINGS OF THE NATIONAL ACADEMY OF SCIENCES, NATIONAL ACADEMY OF SCIENCES, US, vol. 98, no. 26, 18 December 2001 (2001-12-18), pages 15044-15049, XP002343971, ISSN: 0027-8424, DOI: 10.1073/PNAS.251547398**
- **WEBER F ET AL: "Genetic classification of benign and malignant thyroid follicular neoplasia based on a three-gene combination", JOURNAL OF CLINICAL ENDOCRINOLOGY AND METABOLISM, THE ENDOCRINE SOCIETY, US, vol. 90, no. 5, 1 May 2005 (2005-05-01), pages 2512-2521, XP002579798, ISSN: 0021-972X, DOI: 10.1210/JC.2004-2028 [retrieved on 2005-02-15]**
- **HAMADA A ET AL: "Diagnostic usefulness of PCR profiling of the differentially expressed marker genes in thyroid papillary carcinomas", CANCER LETTERS, NEW YORK, NY, US, vol. 224, no. 2, 18 November 2004 (2004-11-18), pages 289-301, XP004899151, ISSN: 0304-3835, DOI: 10.1016/J.CANLET.2004.10.012**
- **KHOLOVÁ I ET AL: "Diagnostic role of markers dipeptidyl peptidase IV and thyroid peroxidase in thyroid tumors", ANTICANCER RESEARCH, INTERNATIONAL INSTITUTE OF ANTICANCER RESEARCH, GR, vol. 23, no. 2A, 1 March 2003 (2003-03-01) , pages 871-875, XP009125721, ISSN: 0250-7005**

**(Cont. next page)**

- **MAZZANTI C ET AL: "Using gene expression profiling to differentiate benign versus malignant thyroid tumors", CANCER RESEARCH, AMERICAN ASSOCIATION FOR CANCER RESEARCH, BALTIMORE, MD., US, vol. 64, 15 April 2004 (2004-04-15), pages 2898-2903, XP002343973, ISSN: 0008-5472**
- **JARZAB B ET AL: "Gene expression profile of papillary thyroid cancer: sources of variability and diagnostic implications", CANCER RESEARCH, AMERICAN ASSOCIATION FOR CANCER RESEARCH, BALTIMORE, MD., US, vol. 65, no. 4, 15 February 2005 (2005-02-15), pages 1587-1597, XP002465072, ISSN: 0008-5472**
- **POLEEV ANDREJ ET AL: "PAX8, a human paired box gene: Isolation and expression in developing thyroid, kidney and Wilms' tumors", DEVELOPMENT (CAMBRIDGE), vol. 116, no. 3, 1992, pages 611-623, XP002555969, ISSN: 0950-1991**
- **PAWITAN Y ET AL: "Gene expression profiling for prognosis using Cox regression", STATISTICS IN MEDICINE, vol. 23, no. 11, 15 June 2004 (2004-06-15), pages 1767-1780, XP002555970, ISSN: 0277-6715**

Remarks:

The complete document including Reference Tables and the Sequence Listing can be downloaded from the EPO website

**Description**

BACKGROUND OF THE INVENTION

There are approximately 25,600 new cases of thyroid carcinoma diagnosed in the United States each year, and 1,400 patients will die of the disease. About 75% of all thyroid cancers belong to the papillary thyroid carcinoma type. The rest consist of 10% follicular carcinoma, 5% to 9% medullary thyroid cancer, 1% to 2% anaplastic cancer, 1% to 3% lymphoma, and less than 1% sarcoma and other rare tumors. Usually a lump (nodule) in the thyroid is the first sign of thyroid cancer. There are 10 to 18 million people in US with a single thyroid nodule, and approximately 490,000 become clinically apparent each year. Fortunately only about 5% of these nodules are cancerous.

The commonly used method for thyroid cancer diagnosis is fine needle aspiration (FNA) biopsy. FNA samples are examined cytologically to determine whether the nodules are benign or cancerous. The sensitivity and specificity of FNA range from 68% to 98%, and 72% to 100% respectively, depending on institutions and doctors. Unfortunately, in 25% of the cases the specimens are either inadequate for diagnosis or indeterminable by cytology. In current medical practice, patients with indeterminate results are sent to surgery, with consequence that only 25% have cancer and 75% end up with unnecessary surgery. A molecular assay with high sensitivity and a better specificity (higher than 25%) would greatly improve current diagnostic accuracy of thyroid cancer, and omit unnecessary surgery for non-cancerous patients.

Comparative genomic hybridization (CGH), serial analysis of gene expression (SAGE), and DNA microarray have been used to identify genetic events occurring in thyroid cancers such as loss of heterozygosity, up and down gene regulation, and genetic rearrangements. PAX8 and PPARγ genetic rearrangement event has been demonstrated to be associated with follicular thyroid cancer (FTC). Rearrangement of the ret proto-oncogene is related to papillary thyroid cancer (PTC). Down-regulation of thyroid peroxidase (TPO) gene is observed in both FTC and PTC. Galectin-3 was reported to be a candidate marker to differentiate malignant thyroid neoplasms from benign lesions. However, there are other studies demonstrating that Galectin-3 is not a cancer-specific marker. Many genes purported to be useful in thyroid cancer diagnosis lack the sensitivity and specificity required for an accurate molecular assay.

Huang et al. (PNAS 2001 Dec 18;98(26):15044-9.) describes a study using oligobased DNA arrays to study the expression profiles of eight matched pairs of normal thyroid and PTC tissues. In this study it was found that a number of genes were overexpressed in Papillary thyroid carcinoma.

Weber et al. (J Clin Endocrinol Metab. 2005 May;90(5):2512-21) reported that a three gene signature including cyclin D2 (CCND2), protein convertase 2 (PCSK2), and prostate differentiation factor (PLAB), allowed the molecular classification of follicular thyroid carcinoma and follicular adenomas.

SUMMARY OF THE INVENTION

The present invention encompasses methods of diagnosing thyroid cancer by obtaining a biological sample from a patient; and measuring the expression levels in the sample of genes encoding mRNA: corresponding to SEQ ID NOs: 199, 207, 255 and 354 where the gene expression levels above or below pre-determined cut-off levels are indicative of thyroid cancer; and gene expression is (a) measured on a microarray or gene chip, or (b) determined by nucleic acid amplification conducted by polymerase chain reaction (PCR) of RNA extracted from the sample.

The present invention encompasses methods of differentiating between thyroid carcinoma and benign thyroid diseases by obtaining a sample from a patient; and measuring the expression levels in the sample of genes encoding mRNA corresponding to SEQ ID NOs: 199, 207, 255 and 354; where the gene expression levels above or below pre-determined cut-off levels are indicative of thyroid carcinoma; and gene expression is (a) measured on a microarray or gene chip, or (b) determined by nucleic acid amplification conducted by polymerase chain reaction (PCR) of RNA extracted from the sample.

The present invention encompasses methods of testing indeterminate thyroid fine needle aspirate (FNA) of thyroid nodule samples by: obtaining a sample from a patient; and measuring the expression levels in the sample of genes encoding mRNA corresponding to SEQ ID NOs: 199, 207, 255 and 354; where the gene expression levels above or below pre-determined cut-off levels are indicative of thyroid cancer; and gene expression is (a) measured on a microarray or gene chip, or (b) determined by nucleic acid amplification conducted by polymerase chain reaction (PCR) of RNA extracted from the sample.

The present disclosure encompasses methods of determining thyroid cancer patient treatment protocol by: obtaining a biological sample from a thyroid cancer patient; and measuring the expression levels in the sample of genes selected from the group consisting of those encoding mRNA: corresponding to SEQ ID NOs: 36, 53, 73, 211 and 242; and/or corresponding to SEQ ID NOs: 199, 207, 255 and 354; or recognized specifically by the probe sets selected from the group consisting of psids corresponding to SEQ ID NOs: 36, 53, 73, 211 and 242 as depicted in Table 25; and/or recognized specifically by the probe sets selected from the group consisting of psids corresponding to SEQ ID NOs:

199, 207, 255 and 354 as depicted in Table 25; where the gene expression levels above or below pre-determined cut-off levels are sufficiently indicative of cancer to enable a physician to determine the type of surgery and/or therapy recommend to treat the disease.

The present disclosure encompasses methods of treating a thyroid cancer patient by obtaining a biological sample from a thyroid cancer patient; and measuring the expression levels in the sample of genes selected from the group consisting of those encoding mRNA: corresponding to SEQ ID NOs: 36, 53, 73, 211 and 242; and/or corresponding to SEQ ID NOs: 199, 207, 255 and 354; or recognized specifically by the probe sets selected from the group consisting of psids corresponding to SEQ ID NOs: 36, 53, 73, 211 and 242 as depicted in Table 25; and/or recognized specifically by the probe sets selected from the group consisting of psids corresponding to SEQ ID NOs: 199, 207, 255 and 354 as depicted in Table 25; where the gene expression levels above or below pre-determined cut-off levels are indicative of cancer; and treating the patient with a thyroidectomy if they are cancer positive.

The present disclosure encompasses methods of cross validating a gene expression profile for thyroid carcinoma patients by: a. obtaining gene expression data from a statistically significant number of patient biological samples; b. randomizing sample order; c. setting aside data from about 10% - 50% of samples; d. computing, for the remaining samples, for factor of interest on all variables and selecting variables that meet a p-value cutoff (p); e. selecting variables that fit a prediction model using a forward search and evaluating the training error until it hits a predetermined error rate; f. testing the prediction model on the left-out 10-50% of samples; g. repeating steps c., -g. with a new set of samples removed; and h. continuing steps c) -g) until 100% of samples have been tested and record classification performance.

The present disclosure encompasses methods of independently validating a gene expression profile and gene profiles obtained thereby for thyroid carcinoma patients by obtaining gene expression data from a statistically significant number of patient biological samples; normalizing the source variabilities in the gene expression data; computing for factor of interest on all variables that were selected previously; and testing the prediction model on the sample and record classification performance.

The present disclosure encompasses a method of generating a posterior probability score to enable diagnosis of thyroid carcinoma patients by: obtaining gene expression data from a statistically significant number of patient biological samples; applying linear discrimination analysis to the data to obtain selected genes; and applying weighted expression levels to the selected genes with discriminate function factor to obtain a prediction model that can be applied as a posterior probability score.

The present disclosure encompasses methods of generating a thyroid carcinoma prognostic patient report and reports obtained thereby, by obtaining a biological sample from the patient; measuring gene expression of the sample; applying a posterior probability thereto; and using the results obtained thereby to generate the report.

The present invention encompasses the use of compositions containing at least one probe set consisting of: SEQ ID NOs: 199, 207, 255 and 354 as defined in the claims.

The present invention encompasses the use of kits for conducting an assay as defined in the claims to determine thyroid carcinoma diagnosis in a biological sample containing: materials for detecting isolated nucleic acid sequences, their complements, or portions thereof of a combination of genes encoding mRNA corresponding to SEQ ID NOs: 199, 207, 255 and 354.

The present disclosure encompasses articles for assessing thyroid carcinoma status containing: materials for detecting isolated nucleic acid sequences, their complements, or portions thereof of a combination of genes selected from the group consisting of those encoding mRNA: corresponding to SEQ ID NOs: 36, 53, 73, 211 and 242; and/or corresponding to SEQ ID NOs: 199, 207, 255 and 354; or recognized specifically by the probe sets selected from the group consisting of psids corresponding to SEQ ID NOs: 36, 53, 73, 211 and 242 as depicted in Table 25; and/or recognized specifically by the probe sets selected from the group consisting of psids corresponding to SEQ ID NOs: 199, 207, 255 and 354 as depicted in Table 25.

The present invention encompasses microarrays or gene chips for performing the methods provided herein, as defined in the claims.

The present disclosure encompasses diagnostic/prognostic portfolios containing isolated nucleic acid sequences, their complements, or portions thereof of a combination of genes selected from the group consisting of those encoding mRNA: corresponding to SEQ ID NOs: 36, 53, 73, 211 and 242; and/or corresponding to SEQ ID NOs: 199, 207, 255 and 354; or recognized specifically by the probe sets selected from the group consisting of psids corresponding to SEQ ID NOs: 36, 53, 73, 211 and 242 as depicted in Table 25; and/or recognized specifically by the probe sets selected from the group consisting of psids corresponding to SEQ ID NOs: 199, 207, 255 and 354 as depicted in Table 25 where the combination is sufficient to characterize thyroid carcinoma status or risk of relapse in a biological sample.

BRIEF DESCRIPTION OF THE DRAWINGS

Figure 1 is an ROC curve of the LOOCV of the 4-gene signature in 98 training samples.

Figure 2 is an ROC curve of the 5-gene signature in 98 training samples.

Figure 3a is an ROC curve of the 4-gene signature in 74 independent validation samples; 3b is an ROC curve of the 5-gene signature in 74 independent validation samples.

Figure 4a is an ROC curve of the 4-gene signature that is normalized to the three-thyroid control genes; 4b is an ROC curve of the 5-gene signature that is normalized to the three-thyroid control genes.

Figure 5a is an ROC curve of the 4-gene signature with one-round amplification in 47 thyroid samples; 5b is an ROC curve of the 4-gene signature with two-round amplification in 47 thyroid samples; 5c is an ROC curve of the 5-gene signature with one-round amplification in 47 thyroid samples; 5d is an ROC curve of the 5-gene signature with two-round amplification in 47 thyroid samples.

Figures 6a and 6b depict the ROC curves for cross validation with the 83 independent fresh frozen thyroid samples.

Figures 7a and 7b depict the ROC curves for signature validation with the 47 fine needle aspirate (FNA) thyroid samples.

Figures 8a and 8b depict the ROC curves for signature performance in 28 paired fresh frozen and FNA thyroid samples.

## DETAILED DESCRIPTION

In this study the goal was to identify signatures that can be used in assays such as DNA chip-based assay to differentiate thyroid carcinomas from benign thyroid diseases. 31 primary papillary thyroid tumors, 21 follicular thyroid cancers, 33 follicular adenoma samples, and 13 benign thyroid diseases were analyzed by using the Affymetrix human U133A Gene Chip. Comparison of gene expression profiles between thyroid cancers and benign tissues has enabled us to identify two signatures: a 5-gene signature identified by percentile analysis and manual selection, and a 4-gene signature selected by Linear Discrimination Analysis (LDA) approach. These two signatures have the performance of sensitivity/specificity 92%/70% and 92%/61%, respectively, and have been validated in 74 independent thyroid samples. The results presented herein demonstrate that these candidate signatures facilitate the diagnosis of thyroid cancers with better sensitivity and specificity than currently available diagnostic procedures. These two signatures are suitable for use in testing indeterminate FNA samples.

By performing gene profiling on 98 representative thyroid benign and tumor samples on Affymetrix U133a chips, we have selected two gene signatures, a 5-gene signature and a 4-gene signature, for thyroid FNA molecular assay. Signatures were selected to achieve the best sensitivity of the assay at a close to 95%. Except for fibronectin and thyroid peroxidase, the other seven genes from the two signatures have not been implicated previously in thyroid tumorogenesis. Both signatures have been validated with an independent 74 thyroid samples, and achieved performance that is equivalent to the one in the 98 training samples. The performances of the two gene signatures are 92% sensitivity and 70%/61% specificity, respectively. When these two signatures are normalized to the specific thyroid control genes the performances are improved relative to the ones of the non-normalized signatures. Furthermore, the signatures performed equivalently with two different target preparations, namely one-round amplification and two-round amplifications. This validation is extremely important for thyroid assays that are FNA samples, which usually contain limited numbers of thyroid cells.

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA (such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumorogenesis, metastasis, apoptosis, and other clinically relevant phenomena. Relative indications of the degree to which genes are active or inactive can be found in gene expression profiles. The gene expression profiles of this invention are used to provide a diagnosis and treat patients for thyroid cancer.

Sample preparation requires the collection of patient samples. Patient samples used in the inventive method are those that are suspected of containing diseased cells such as cells taken from a nodule in a fine needle aspirate (FNA) of thyroid tissue. Bulk tissue preparation obtained from a biopsy or a surgical specimen and laser capture microdissection are also suitable for use. Laser Capture Microdissection (LCM) technology is one way to select the cells to be studied, minimizing variability caused by cell type heterogeneity. Consequently, moderate or small changes in gene expression between normal or benign and cancerous cells can be readily detected. Samples can also comprise circulating epithelial cells extracted from peripheral blood. These can be obtained according to a number of methods but the most preferred method is the magnetic separation technique described in U.S. Patent 6,136,182. Once the sample containing the cells of interest has been obtained, RNA is extracted and amplified and a gene expression profile is obtained, preferably via microarray, for genes in the appropriate portfolios.

The present invention encompasses methods of diagnosing thyroid cancer by obtaining a biological sample from a patient; and measuring the expression levels in the sample of genes from those encoding mRNA corresponding to SEQ ID NOs: 199, 207, 255 and 354; where the gene expression levels above or below pre-determined cut-off levels are indicative of thyroid cancer and gene expression is (a) measured on a microarray or gene chip, or (b) determined by nucleic acid amplification conducted by polymerase chain reaction (PCR) of RNA extracted from the sample.

The present invention encompasses methods of differentiating between thyroid carcinoma and benign thyroid diseases by obtaining a sample from a patient; and measuring the expression levels in the sample of genes from those encoding mRNA corresponding to SEQ ID NOs: 199, 207, 255 and 354; where the gene expression levels above or below pre-determined cut-off levels are indicative of thyroid carcinoma; and gene expression is (a) measured on a microarray or gene chip, or (b) determined by nucleic acid amplification conducted by polymerase chain reaction (PCR) of RNA extracted from the sample

The present invention encompasses methods of testing indeterminate thyroid fine needle aspirate (FNA) of thyroid nodule samples by: obtaining a sample from a patient; and measuring the expression levels in the sample of genes from those encoding mRNA corresponding to SEQ ID NOs: 199, 207, 255 and 354; where the gene expression levels above or below pre-determined cut-off levels are indicative of thyroid cancer; and gene expression is (a) measured on a microarray or gene chip, or (b) determined by nucleic acid amplification conducted by polymerase chain reaction (PCR) of RNA extracted from the sample.

The present disclosure encompasses methods of determining thyroid cancer patient treatment protocol by: obtaining a biological sample from a thyroid cancer patient; and measuring the expression levels in the sample of genes from those encoding mRNA: corresponding to SEQ ID NOs: 36, 53, 73, 211 and 242; and/or corresponding to SEQ ID NOs: 199, 207, 255 and 354; or recognized specifically by the probe sets from psids corresponding to SEQ ID NOs: 36, 53, 73, 211 and 242 as depicted in Table 25; and/or recognized specifically by the probe sets from psids corresponding to SEQ ID NOs: 199, 207, 255 and 354 as depicted in Table 25; where the gene expression levels above or below pre-determined cut-off levels are sufficiently indicative of cancer to enable a physician to determine the type of surgery and/or therapy recommend to treat the disease.

The present disclosure encompasses methods of treating a thyroid cancer patient by obtaining a biological sample from a thyroid cancer patient; and measuring the expression levels in the sample of genes from those encoding mRNA: corresponding to SEQ ID NOs: 36, 53, 73, 211 and 242; and/or corresponding to SEQ ID NOs: 199, 207, 255 and 354; or recognized specifically by the probe sets from psids corresponding to SEQ ID NOs: 36, 53, 73, 211 and 242 as depicted in Table 25; and/or recognized specifically by the probe sets from psids corresponding to SEQ ID NOs: 199, 207, 255 and 354 as depicted in Table 25; where the gene expression levels above or below pre-determined cut-off levels are indicative of cancer; and treating the patient with thyroidectomy if they are cancer positive.

The SEQ ID NOs in the above methods can be 36, 53, 73, 211 and 242 or 199, 207, 255 and 354, or 45, 215, 65, 29, 190, 199, 207, 255 and 354.

The invention also encompasses the above methods containing the steps of further measuring the expression level of at least one gene encoding mRNA: corresponding to SEQ ID NOs: 142, 219 and 309; and/or corresponding to SEQ ID NOs: 9, 12 and 18 as defined in the claims. The invention also encompasses the above methods containing the steps of further measuring the expression level of at least one gene constitutively expressed in the sample as defined in the claims.

Cadherin 3, type 1 (SEQ ID NO: 53) is mentioned in US20030194406; US 20050037439; and US 20040137539. Fibronectin (SEQ ID NO: 242) is mentioned in US6436642 and US20030104419. Secretory granule, neuroendocrine protein 1 (SEQ ID NO: 76) is mentioned in US20030232350; and US20040002067. Testican-1 (SEQ ID NO: 36) is mentioned in US20030108963; and US20050037463. Thyroid peroxidase (SEQ ID NO: 211) is mentioned in US6066449, US20030118553; US20030054571; WO9102061; and WO9856953. Chemokine C (C-C) motif ligand 18 (SEQ ID NO: 354) is mentioned in WO2005005601 and US20020114806. Pulmonary surfactant-associated protein B (SEQ ID NO: 355) is mentioned in US20030219760; and US20030232350. K+ channel beta subunit (SEQ ID NO: 207) is mentioned in US20030096782; and US 20020168638. Putative prostate cancer suppressor (SEQ ID NO: 178) is mentioned in WO2005020784. Bone marrow stromal cell antigen 1 (SEQ ID NO: 142) is mentioned in WO2004040014; and WO2005020784. Leucocyte immunoglobulin-like receptor-6b (SEQ ID NO: 219) is mentioned in US20030060614. Bridging integrator 2 (SEQ ID NO: 309) is mentioned in EP1393776; WO02057414; WO0116158 and US6831063. Cysteine-rich, angiogenic inducer, 61 (SEQ ID NO: 9) is mentioned in WO2004030615; and WO9733995. Selenoprotein P, Plasma 1 (SEQ ID NO: 12) is mentioned in US20040241653 and WO2005015236. Insulin-like growth factor-binding protein 4 (SEQ ID NO: 18) is mentioned in WO2005015236; WO9203469; WO9203152; and EP0546053.

In this invention, the most preferred method for analyzing the gene expression pattern of a patient in the methods provided herein is through the use of a linear discrimination analysis program. The present invention encompasses a method of generating a posterior probability score to enable diagnosis of thyroid carcinoma patients by: obtaining gene expression data from a statistically significant number of patient biological samples; applying linear discrimination analysis to the data to obtain selected genes; and applying weighted expression levels to the selected genes with discriminate function factor to obtain a prediction model that can be applied as a posterior probability score. Other analytical tools can also be used to answer the same question such as, logistic regression and neural network approaches.

For instance, the following can be used for linear discriminant analysis:

$$p_{(CP)} = \frac{e^{d_{(CP)} - d_{(CN)}}}{1 + e^{d_{(CP)} - d_{(CN)}}}$$

$$p_{(CN)} = \frac{1}{1 + e^{d_{(CP)} - d_{(CN)}}}$$

where,

$I_{(psid)}$ = The log base 2 intensity of the probe set enclosed in parenthesis.
$d_{(CP)}$ = The discriminant function for the cancer positive class
$d_{(CN)}$ = The discriminant function for the cancer negative class
$P_{(CP)}$ = The posterior p-value for the cancer positive class
$P_{(CN)}$ = The posterior p-value for the cancer negative class

Numerous other well-known methods of pattern recognition are available. The following references provide some examples: Weighted Voting: Golub et al. (1999); Support Vector Machines: Su et al. (2001); and Ramaswamy et al. (2001); K-nearest Neighbors: Ramaswamy (2001); and Correlation Coefficients: van't Veer et al. (2002).

Preferably, portfolios are established such that the combination of genes in the portfolio exhibit improved sensitivity and specificity relative to individual genes or randomly selected combinations of genes. In the context of the instant disclosure, the sensitivity of the portfolio can be reflected in the fold differences exhibited by a gene's expression in the diseased state relative to the normal state. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used in this capacity. The invention also encompasses the above methods where the specificity is at least about 40%, at least about 50% and at least about 60%. The invention also encompasses the above methods where the sensitivity is at least at least about 90% and at least about 92%.

The invention also encompasses the above methods where the comparison of expression patterns is conducted with pattern recognition methods. One method of the invention involves comparing gene expression profiles for various genes (or portfolios) to ascribe diagnoses. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal, benign or diseased. In a more sophisticated embodiment, patterns of the expression signals (e.g., fluorescent intensity) are recorded digitally or graphically. The gene expression patterns from the gene portfolios used in conjunction with patient samples are then compared to the expression patterns.

Pattern comparison software can then be used to determine whether the patient samples have a pattern indicative of the disease. Of course, these comparisons can also be used to determine whether the patient is not likely to experience the disease. The expression profiles of the samples are then compared to the portfolio of a control cell. If the sample expression patterns are consistent with the expression pattern for cancer then (in the absence of countervailing medical considerations) the patient is treated as one would treat a thyroid cancer patient. If the sample expression patterns are consistent with the expression pattern from the normal/control cell then the patient is diagnosed negative for cancer.

Preferably, levels of up and down regulation are distinguished based on fold changes of the intensity measurements of hybridized microarray probes. A 1.5 fold difference is preferred for making such distinctions (or a p-value less than 0.05). That is, before a gene is said to be differentially expressed in diseased versus normal cells, the diseased cell is found to yield at least about 1.5 times more, or 1.5 times less intensity than the normal cells. The greater the fold difference, the more preferred is use of the gene as a diagnostic or prognostic tool. Genes selected for the gene expression profiles of this invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's T-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, one is unlikely to see small p-values just by chance and adjustments for this using a Sidak correction as well as a randomization/permutation experiment can be made. A p-value less than 0.05 by the T-test is evidence that the gene is significantly different. More compelling evidence is a p-value less then 0.05 after the Sidak correction is factored in. For a large number of samples in each group, a p-value less than 0.05 after the randomization/permutation test is the most compelling evidence of a significant difference.

The present invention encompasses the use of microarrays or gene chips for performing the methods provided herein as defined in the claims. The microarrays can contain isolated nucleic acid sequences, their complements, or portions thereof of a combination of genes from those encoding mRNA: corresponding to SEQ ID NOs: 36, 53, 73, 211 and 242; and/or corresponding to SEQ ID NOs: 199, 207, 255 and 354; or recognized specifically by the probe sets from psids corresponding to SEQ ID NOs: 36, 53, 73, 211 and 242 as depicted in Table 25; and/or recognized specifically by the probe sets from psids corresponding to SEQ ID NOs: 199, 207, 255 and 354 as depicted in Table 25 where the combination is sufficient to characterize thyroid carcinoma or risk of relapse in a biological sample. The microarray preferably measures or characterizes at least about 1.5-fold over- or under-expression, provides a statistically significant p-value over- or under-expression, or a p-value is less than 0.05. Preferably, the microarray contains a cDNA array or an oligonucleotide array and may contain one or more internal control reagents. One preferred internal control reagent is a method of detecting PAX8 gene expression which can be measured using SEQ ID NOs: 409-411.

Preferably, an oligonucleotide in the array corresponds to the 3' non-coding region of the gene the expression of which is being measured.

Another parameter that can be used to select genes that generate a signal that is greater than that of the non-modulated gene or noise is the use of a measurement of absolute signal difference. Preferably, the signal generated by the modulated gene expression is at least 20% different than those of the normal or non-modulated gene (on an absolute basis). It is even more preferred that such genes produce expression patterns that are at least 30% different than those of normal or non-modulated genes.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complementary DNA (cDNA) or complementary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002; 6,218,122; 6,218,114; and 6,004,755.

Analysis of the expression levels is conducted by comparing such signal intensities. This is best done by generating a ratio matrix of the expression intensities of genes in a test sample versus those in a control sample. For instance, the gene expression intensities from a diseased tissue can be compared with the expression intensities generated from benign or normal tissue of the same type. A ratio of these expression intensities indicates the fold-change in gene expression between the test and control samples.

Gene expression profiles can also be displayed in a number of ways. The most common method is to arrange raw fluorescence intensities or ratio matrix into a graphical dendogram where columns indicate test samples and rows indicate genes. The data are arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRING" from Silicon Genetics, Inc. and "DISCOVERY" and "INFER" software from Partek, Inc.

Modulated genes used in the methods of the invention are described in the Examples. The genes that are differentially expressed are either up regulated or down regulated in patients with thyroid cancer relative to those with benign thyroid diseases. Up regulation and down regulation are relative terms meaning that a detectable difference (beyond the contribution of noise in the system used to measure it) is found in the amount of expression of the genes relative to some baseline. In this case, the baseline is the measured gene expression of a benign disease patient. The genes of interest in the diseased cells are then either up regulated or down regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect of that person's genotype or phenotype is consistent with the presence of the disease. However, the act of conducting a diagnosis or prognosis includes the determination of disease/status issues such as determining the likelihood of relapse, type of therapy and therapy monitoring. In therapy monitoring, clinical judgments are made regarding the effect of a given course of therapy by comparing the expression of genes over time to determine whether the gene expression profiles have changed or are changing to patterns more consistent with normal tissue.

Genes can be grouped so that information obtained about the set of genes in the group provides a sound basis for making a clinically relevant judgment such as a diagnosis, prognosis, or treatment choice. These sets of genes make up the portfolios of the invention. As with most diagnostic markers, it is often desirable to use the fewest number of markers sufficient to make a correct medical judgment. This prevents a delay in treatment pending further analysis as well unproductive use of time and resources.

One method of establishing gene expression portfolios is through the use of optimization algorithms such as the mean variance algorithm widely used in establishing stock portfolios. This method is described in detail in US patent publication number 20030194734. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application," referred to as "Wagner Software" throughout this specification, is preferred. This software uses functions from the "Wagner Associates Mean-Variance Optimization Library" to determine an efficient frontier and optimal portfolios in the Markowitz sense is preferred. Use of this type of software requires that microarray data be transformed so that it can be treated as an input in the way stock return and risk measurements are used when the software is used for its intended financial analysis purposes.

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with cancer. Output from the method would be an optimized set of genes that could include some genes that are expressed in peripheral blood as well as in diseased tissue. If samples used in the testing method are obtained from peripheral blood and certain genes differentially expressed in instances of cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply a rule that only a prescribed percentage of the portfolio can be represented by a particular gene or group of genes. Commercially available software such as the Wagner Software readily accommodates these types of heuristics. This can be useful, for example, when factors other than accuracy and precision (e.g., anticipated licensing fees) have an impact on the desirability of including one or more genes.

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., Cancer Antigen 27.29 ("CA 27.29")). A range of such markers exists including such analytes as CA 27.29. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate (FNA) is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous results.

The present disclosure encompasses methods of cross validating a gene expression profile and the profiles thus obtained, for thyroid carcinoma patients by: a. obtaining gene expression data from a statistically significant number of patient biological samples; b. randomizing sample order; c. setting aside data from about 10% - 50% of samples; d. computing, for the remaining samples, for factor of interest on all variables and selecting variables that meet a p-value cutoff (p); e. selecting variables that fit a prediction model using a forward search and evaluating the training error until it hits a predetermined error rate; f. testing the prediction model on the left-out 10-50% of samples; g. repeating steps c., -g. with a new set of samples removed; and h. continuing steps c) -g) until 100% of samples have been tested and record classification performance. In this method, preferably, the gene expression data obtained in step h. is represented by genes from those encoding mRNA: corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363; or recognized specifically by the probe sets from psids in Table 25 corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363.

The present disclosure encompasses methods of independently validating a gene expression profile and the profiles thus obtained, for thyroid cancer patients by obtaining gene expression data from a statistically significant number of patient biological samples; normalizing the source variabilities in the gene expression data; computing for factor of interest on all variables that were selected previously; and testing the prediction model on the sample and record classification performance. In this method, preferably, the gene expression data obtained in step d. is represented by genes from those encoding mRNA: corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363; or recognized specifically by the probe sets from psids in Table 25 corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363.

The present disclosure encompasses methods of generating a posterior probability to enable diagnosis of thyroid carcinoma patients by obtaining gene expression data from a statistically significant number of patient biological samples; applying linear discrimination analysis to the data to obtain selected genes; applying weighted expression levels to the selected genes with discriminate function factor to obtain a prediction model that can be applied as a posterior probability score. For instance, the following can be used for Linear Discriminant Analysis:

$$p_{(CP)} = \frac{e^{d_{(CP)} - d_{(CN)}}}{1 + e^{d_{(CP)} - d_{(CN)}}}$$

$$p_{(CN)} = \frac{1}{1 + e^{d_{(CP)} - d_{(CN)}}}$$

where,

$I_{(psid)}$ = The log base 2 intensity of the probe set enclosed in parenthesis.
$d_{(CP)}$ = The discriminant function for the cancer positive class
$d_{(CN)}$ = The discriminant function for the cancer negative class
$P_{(CP)}$ = The posterior p-value for the cancer positive class
$P_{(CN)}$ = The posterior p-value for the cancer negative class

The present disclosure encompasses methods of generating a thyroid carcinoma diagnostic patient report and reports obtained thereby, by obtaining a biological sample from the patient; measuring gene expression of the sample; applying a posterior probability score thereto; and using the results obtained thereby to generate the report. The report can also contain an assessment of patient outcome and/or probability of risk relative to the patient population.

The present invention encompasses the use of compositions containing at least one probe set consisting of: SEQ ID NOs: 199, 207, 255 and 354; as defined in the claims.

The present invention encompasses the use of kits for conducting an assay to determine thyroid carcinoma diagnosis in a biological sample as defined in the claims containing: materials for detecting isolated nucleic acid sequences, their complements, or portions thereof of a combination of genes from those encoding mRNA corresponding to SEQ ID NOs: 199, 207, 255 and 354The SEQ ID NOs. can be 36, 53, 73, 211 and 242, 199, 207, 255 and 354 and 45, 215, 65, 29, 190, 199, 207, 255 and 354.

Kits made according to the invention include formatted assays for determining the gene expression profiles. These can include all or some of the materials needed to conduct the assays such as reagents and instructions and a medium through which nucleic acid sequences, their complements, or portions thereof are assayed.

Articles of this disclosure include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "DISCOVERY" and "INFER" software from Partek, Inc. mentioned above can best assist in the visualization of such data.

Different types of articles of manufacture according to the disclosure are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the disclosure can be fashioned into reagent kits for conducting hybridization, amplification, and signal generation indicative of the level of expression of the genes of interest for detecting cancer.

The present disclosure encompasses articles for assessing thyroid carcinoma status containing: materials for detecting isolated nucleic acid sequences, their complements, or portions thereof of a combination of genes from those encoding mRNA: corresponding to SEQ ID NOs: 36, 53, 73, 211 and 242; and/or corresponding to SEQ ID NOs: 199, 207, 255 and 354; or recognized specifically by the probe sets from psids corresponding to SEQ ID NOs: 36, 53, 73, 211 and 242 as depicted in Table 25; and/or recognized specifically by the probe sets from psids corresponding to SEQ ID NOs: 199, 207, 255 and 354 as depicted in Table 25. The SEQ ID NOs. can be 36, 53, 73, 211 and 242; 199, 207, 255 and 354; or 45, 215, 65, 29, 190, 199, 207, 255 and 354.

The present disclosure encompasses diagnostic/prognostic portfolios containing isolated nucleic acid sequences, their complements, or portions thereof of a combination of genes from those encoding mRNA: corresponding to SEQ ID NOs: 36, 53, 73, 211 and 242; and/or corresponding to SEQ ID NOs: 199, 207, 255 and 354; or recognized specifically by the probe sets from psids corresponding to SEQ ID NOs: 36, 53, 73, 211 and 242 as depicted in Table 25; and/or recognized specifically by the probe sets from psids corresponding to SEQ ID NOs: 199, 207, 255 and 354 as depicted in Table 25 where the combination is sufficient to characterize thyroid carcinoma status or risk of relapse in a biological sample. Preferably, the portfolio measures or characterizes at least about 1.5-fold over- or under-expression or provides a statistically significant p-value over- or under-expression. Preferably, the p-value is less than 0.05.

The following examples are provided to illustrate but not limit the claimed invention.

**Example 1**

**Materials and Methods**

Tissue samples

Fresh frozen thyroid benign diseases, follicular adenoma, follicular carcinoma, and papillary carcinoma samples were obtained from different commercial vendors including Genomics Collaborative, Inc. (Cambridge, MA), Asterand (Detroit, MI), and Proteogenex (Los Angeles, CA). All samples were collected according to an Institutional Review Board approval protocol. Patients demographic and pathology information were also collected. The histopathological features of each sample were reviewed to confirm diagnosis, estimate sample preservation and tumor content.

RNA isolation

Standard TriZol protocol was used for all the RNA isolations. Tissue was homogenized in TriZol reagent (Invitrogen, Carlsbad, CA). Total RNA was isolated from TriZol and precipitated at -20°C with isopropyl alcohol. RNA pellets were washed with 75% ethanol, dissolved in water and stored at -80°C until use. RNA integrity was examined with Agilent 2100 Bioanalyzer RNA 6000 NanoAssay (Agilent Technologies, Palo Alto, CA).

Linear Discrimination Analysis

Linear Discriminant Analysis was performed using these steps: calculation of a common (pooled) covariance matrix and within-group means; calculation of the set of linear discriminant functions from the common covariance and the within-group means; and classification using the linear discriminant functions.

Plugging the chip intensity readings for each probe into the following equation can be used to derive the posterior probability of an unknown thyroid sample as either cancer positive or negative. For example, if a thyroid sample is tested with the assay and gives a $p_{(CP)} > 0.5$ this sample will be classified as thyroid cancer.
For the 4 gene signature:

$$d_{(CP)} = -50.9964 + 0.220424(I_{(32128\_at)}) + 1.520185(I_{(209810\_at)}) + 3.09431(I_{(210078\_s\_at)}) + 6.11283(I_{(213423\_x\_at)})$$

$$d_{(CN)} = -46.7445 + 0.374751(I_{(32128\_at)}) + 1.010852(I_{(209810\_at)}) + 3.645515(I_{(210078\_s\_at)}) + 5.337296(I_{(213423\_x\_at)})$$

For the 5 gene signature:

$$d_{(CP)} = -135.931 + 4.737838(I_{(202363\_at)}) - 1.23763(I_{(203256\_at)}) + 1.984148(I_{(203889\_at)}) + 6.638082(I_{(210342\_s\_at)}) + 10.7704(I_{(212464\_s\_at)})$$

$$d_{(CN)} = -128.978 + 4.610498 (I_{(202363\_at)}) - 1.28685 (I_{(203256\_at)}) + 1.656772 (I_{(203889\_at)}) + 6.859133 (I_{(210342\_s\_at)}) + 10.24482 (I_{(212464\_s\_at)})$$

$$p_{(CP)} = \frac{e^{d_{(CP)}-d_{(CN)}}}{1 + e^{d_{(CP)}-d_{(CN)}}}$$

$$p_{(CN)} = \frac{1}{1 + e^{d_{(CP)}-d_{(CN)}}}$$

where,

$I_{(psid)}$ = The log base 2 intensity of the probe set enclosed in parenthesis.
$d_{(CP)}$ = The discriminant function for the cancer positive class
$d_{(CN)}$ = The discriminant function for the cancer negative class
$P_{(CP)}$ = The posterior p-value for the cancer positive class
$P_{(CN)}$ = The posterior p-value for the cancer negative class

Two-round aRNA amplification

aRNA was amplified from 10 ng total RNA using the RiboBeast 2-Round Aminoallyl-aRNA Amplification kit (Epicentre, WI), a T7 based RNA linear amplification protocol, with some modifications. Total RNA was reverse transcribed using an oligo(dT) primer containing a T7 RNA polymerase promoter sequence and Superscript III RT. The second-strand synthesis was carried out using Bst DNA polymerase. An extra step of incubation with an exonuclease mix of Exo I and Exo VII was performed to reduce background. The double-stranded cDNA served as the template for T7-mediated linear amplification by in vitro transcription. For the second round of amplification, instead of using the RiboBeast reagents, the ENZO BioArray HighYield RNA Transcript Labeling kit (Affymetrix, CA) was used in place of the in vitro transcription step of Aminoallyl-aRNA. The aRNA was quantified by Agilent Nano Chip technology.

## Example 2

### Microarray analysis

Labeled cRNA was prepared and hybridized with the high-density oligonucleotide array Hu133A Gene Chip (Affymetrix, Santa Clara, CA) containing a total of 22,000 probe sets. Hybridization was performed according to a standard protocol provided by the manufacturer. Arrays were scanned using Affymetrix protocols and scanners. For subsequent analysis, each probe set was considered as an independent gene. Expression values for each gene were calculated by using Affymetrix Gene Chip analysis software MAS 5.0. All chips met the following quality control standards: the percentage of "presence" call, the scaling factor, the background level, and the noise level have to be within the range of mean plus or minus 3 standard deviation. All chips used for subsequent analysis have passed these quality control criteria. Sample collection for signature selection and independent validation is summarized in Table 1.

Table 1. Sample collection for signature training and validation

Training Sample Set

| Category | Number of Samples |
| --- | --- |
| Follicular Adenoma (FA) | 33 |
| Follicular Carcinoma (FC) | 21 |
| Benign Diseases (BN) | 13 |
| Papillary Carcinoma (PC) | 31 |

Validation Sample Set

| Category | Number of Samples |
| --- | --- |
| Follicular Adenoma (FA) | 38 |
| Follicular Carcinoma (FC) | 5 |
| Follicular Variant of Papillary Carcinoma (FVPTC) | 11 |
| Papillary Carcinoma (PC) | 20 |

## Example 3

### Results Signature Identification

A. Gene Selection

A total of 98 samples including 31 primary papillary thyroid tumors, 21 follicular thyroid cancers, 33 follicular adenoma, and 13 benign thyroid tissues were analyzed by using Affymetrix human U133A gene chips. Five gene selection criteria were applied to the entire data set to obtain a limited number of genes for subsequent gene marker or signature identification:

1. Genes with at least one "Present Call" in this sample set were considered.
2. Genes with more than one "Present Call" in 12 PBL samples were excluded.
3. Only genes with chip intensity larger than 200 in all samples were selected.
4. Using genes that passed the above three criteria, we performed a variety of analyses, as listed in Table 2, to identify genes that are either up-regulated or down-regulated in thyroid tumors.
5. Finally, genes with expression change greater than 1.4-fold were selected.

Table 2. Summary of different types of percentile analyses

| | Type of Percentile Analysis |
| --- | --- |
| 1 | 20% FC vs 100% Benign |
| 2 | 30% FC vs 90% Benign |

(continued)

| | Type of Percentile Analysis |
|---|---|
| 3 | 30% PC vs 90% Benign |
| 4 | 70% FC vs 50% Benign |
| 5 | 70% PC vs 50% Benign |
| 6 | 90% Benign vs 30% FC |
| 7 | 90% Benign vs 30% PC |

The final number of selected genes for signature identification is 322, described in Table 25, SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363. The data obtained from the 322 selected genes are provided in Table 3 and summarized in Table 4.

Table 3

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 354 | 10235.3 | 163.8 | 12.2 | 99.1 | 111.6 | 2506.3 | 1780.7 | 29.3 | 4.1 |
| 355 | 508.5 | 150.8 | 31.5 | 83.8 | 63.1 | 457.5 | 189.9 | 48.2 | 45.3 |
| 356 | 82.7 | 754 | 707.3 | 477.3 | 1120.7 | 827.5 | 246.3 | 1068.3 | 960.1 |
| 357 | 834 | 758.9 | 718 | 249.4 | 883.6 | 554.5 | 377.5 | 616.1 | 706.9 |
| 358 | 2490.4 | 593.3 | 565.5 | 540.3 | 1132.7 | 750.4 | 803.7 | 377.4 | 534.6 |
| 359 | 533.5 | 342 | 412 | 409.4 | 413.3 | 396.5 | 309.5 | 352.8 | 317.7 |
| 362 | 136.4 | 550 | 399.6 | 653.8 | 573.4 | 322.4 | 539.5 | 317.5 | 324.9 |
| 360 | 476.2 | 324.5 | 352.5 | 348.2 | 473.3 | 342.9 | 170.3 | 190.1 | 279.4 |
| 361 | 196.4 | 995.8 | 839.3 | 382.6 | 926.9 | 480.8 | 305.3 | 913.9 | 1123.4 |
| 363 | 642.7 | 392.9 | 410.2 | 742.3 | 447.2 | 504.9 | 457.6 | 397 | 490.3 |
| 1 | 57.5 | 694.1 | 410.9 | 413.8 | 950.3 | 946.6 | 351.2 | 317.7 | 346.4 |
| 2 | 854 | 3127.2 | 2912.3 | 675.3 | 4484 | 3866 | 950.5 | 2514.3 | 2438.1 |
| 7 | 6.6 | 584 | 1380.1 | 315.4 | 543 | 393.6 | 303.9 | 713.9 | 821.6 |
| 8 | 6606.3 | 941.2 | 1104.5 | 736.7 | 749.7 | 1740.5 | 1083.6 | 423.7 | 583.3 |
| 9 | 1852.9 | 10149.7 | 6856.2 | 2912.1 | 6808.7 | 1233.4 | 3010.6 | 3890.5 | 2225.4 |
| 10 | 213.9 | 56.5 | 66.8 | 70.8 | 121.8 | 102.5 | 426.1 | 58.3 | 149.9 |
| 11 | 463.3 | 163.4 | 131.5 | 187.4 | 350.7 | 210.3 | 129.1 | 340.2 | 336.3 |
| 13 | 286.2 | 180.9 | 111.8 | 233.1 | 197.5 | 335.1 | 173.8 | 70.7 | 108.3 |
| 14 | 1590.2 | 437.8 | 323.5 | 558.1 | 897.5 | 1161.3 | 642.8 | 105.9 | 170.6 |
| 15 | 9293.6 | 467.4 | 332.9 | 823.3 | 3718.3 | 204 | 959.7 | 506.8 | 670.5 |
| 16 | 180 | 1093.1 | 911.1 | 674.5 | 960.2 | 1600.8 | 467 | 1244.5 | 995.6 |
| 17 | 850.8 | 1221.7 | 2320.1 | 687.9 | 1614.9 | 1605.7 | 404.6 | 1911.4 | 2114 |
| 19 | 411.1 | 1415.6 | 184.1 | 723.5 | 1035.5 | 370.5 | 548.9 | 147 | 192.6 |
| 20 | 733.9 | 1.1 | 1.1 | 74.5 | 256.4 | 1.1 | 326.7 | 1.1 | 1.2 |
| 21 | 72.3 | 200.7 | 12.9 | 81.5 | 88.3 | 242.5 | 67 | 11.5 | 37.4 |
| 22 | 3094.8 | 9706.3 | 7329.5 | 3225.3 | 6075.3 | 6859.5 | 2406.5 | 6706.8 | 8884.7 |

(continued)

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 23 | 4749.1 | 837.4 | 1.1 | 835.6 | 2674.6 | 364.3 | 1127.5 | 1.1 | 31.7 |
| 24 | 965.6 | 221 | 39.2 | 361.9 | 738.2 | 167.3 | 169.5 | 71.9 | 121.6 |
| 26 | 7416.9 | 7158.3 | 127.4 | 6838.8 | 8238 | 2252.3 | 5033.6 | 65.1 | 119.9 |
| 27 | 58.1 | 722.5 | 759 | 815.4 | 651.8 | 689.9 | 359.8 | 573 | 381.9 |
| 29 | 215.3 | 111.1 | 55.4 | 103.7 | 90.2 | 70.5 | 65.9 | 199.3 | 105.7 |
| 30 | 5508.4 | 259.4 | 11.7 | 410.1 | 853.5 | 143.9 | 596.3 | 20.7 | 45.8 |
| 31 | 141.5 | 311.1 | 66.8 | 198.7 | 183.8 | 290.6 | 464.1 | 50.5 | 108.6 |
| 33 | 20935.4 | 341.8 | 144.8 | 828.3 | 7608.5 | 425 | 359.1 | 89.7 | 315.9 |
| 34 | 82.5 | 4387.6 | 4002.6 | 2634.3 | 4784.6 | 4798 | 2420.6 | 3104.2 | 3558 |
| 35 | 289.7 | 142.4 | 32.9 | 436.5 | 220.8 | 143.9 | 838.9 | 24.4 | 27.4 |
| 36 | 760.5 | 205.8 | 124 | 156.6 | 338.1 | 167.8 | 148.6 | 42.9 | 140.8 |
| 37 | 339.4 | 237.6 | 117.2 | 340.6 | 200.9 | 294.7 | 332.7 | 183.3 | 276.3 |
| 38 | 15634.5 | 361.2 | 546.7 | 505.1 | 4617.1 | 382.4 | 256.9 | 914.7 | 676.5 |
| 40 | 622.8 | 479.8 | 437.7 | 682.8 | 423.9 | 523.7 | 477.9 | 481.4 | 742.7 |
| 41 | 217.2 | 359.7 | 234.4 | 221.2 | 757.4 | 528.7 | 139.2 | 226.1 | 164.4 |
| 42 | 219.8 | 238.2 | 203.5 | 163.5 | 270.5 | 212.1 | 176.6 | 169.7 | 210.4 |
| 43 | 234.7 | 301 | 366.1 | 430.4 | 277 | 390.7 | 298.8 | 366.6 | 431.1 |
| 44 | 1194.4 | 428.6 | 352.7 | 678.6 | 1038.4 | 590.6 | 502.4 | 312.5 | 412.6 |
| 45 | 93.2 | 348.4 | 537.2 | 498.2 | 362.4 | 526.8 | 286.3 | 593.7 | 721.8 |
| 46 | 353.4 | 362.7 | 228.1 | 486.3 | 390 | 348.8 | 264.3 | 223.9 | 308.2 |
| 47 | 1440 | 122.7 | 195.5 | 267.7 | 384.4 | 279.8 | 279.8 | 186 | 120.1 |
| 48 | 199.4 | 203.4 | 16.7 | 1371.4 | 795.6 | 261.9 | 2274.7 | 36.6 | 21 |
| 49 | 195.9 | 337.7 | 268 | 285.8 | 341.3 | 420.2 | 392.8 | 175.7 | 207.1 |
| 50 | 8413 | 277.9 | 72.9 | 481 | 1790.1 | 240.3 | 276.3 | 19.6 | 144.4 |
| 51 | 548.4 | 42 | 29 | 436.1 | 66.9 | 35.2 | 265.4 | 38.8 | 35.4 |
| 52 | 189.3 | 199.3 | 189.3 | 144.9 | 217.9 | 179.8 | 189.7 | 265.6 | 263.1 |
| 53 | 90.2 | 79.3 | 46.2 | 51 | 36.3 | 81.2 | 109.4 | 83.5 | 33.9 |
| 54 | 879.2 | 383 | 1133.4 | 796.7 | 378.8 | 853 | 508.4 | 1350.7 | 462.2 |
| 55 | 69.1 | 130.4 | 81.7 | 126.9 | 65.7 | 123.7 | 96.6 | 86.1 | 118.9 |
| 56 | 29.9 | 53.2 | 83.6 | 82.1 | 125.5 | 80.4 | 57 | 64.5 | 59.6 |
| 57 | 5589.3 | 468.9 | 208.8 | 519 | 338.3 | 2738.1 | 2814.6 | 95.3 | 120 |
| 58 | 25483 | 492.1 | 294.6 | 702.4 | 495.2 | 2804.2 | 3544.8 | 95.7 | 164.7 |
| 59 | 57.6 | 1695.3 | 1278.9 | 884.1 | 1841.6 | 2339.6 | 560.2 | 1800.2 | 2204 |
| 60 | 308 | 154.2 | 148.1 | 188 | 151.4 | 261 | 115.3 | 120.2 | 177.2 |
| 61 | 193.7 | 168.2 | 134.2 | 228 | 153.6 | 138.7 | 132.1 | 83.1 | 176.1 |
| 62 | 119.4 | 478.9 | 203.5 | 1627.2 | 1442.2 | 1810.1 | 903.6 | 75 | 2303.9 |
| 63 | 98.7 | 380.9 | 470 | 197.8 | 346.6 | 224.8 | 242.2 | 297.5 | 192 |

(continued)

| 3a | | | | | | | | | |
| --- | --- | --- | --- | --- | --- | --- | --- | --- | --- |
| SEQ ID NO: | 800TT | 801TT | 802TT | 804TT | 805TT | 806TT | 807TT | 818TT | 819TT |
| 64 | 143.1 | 133.4 | 210.8 | 393.3 | 197.5 | 194.5 | 165.5 | 154.4 | 209.9 |
| 65 | 328.5 | 124 | 64.1 | 172.8 | 348.1 | 151.2 | 192.8 | 101.1 | 127.9 |
| 66 | 249.1 | 732 | 1332.3 | 450.2 | 923.7 | 721.7 | 524.1 | 1163.3 | 1056 |
| 67 | 293.1 | 31554.3 | 31050.5 | 34557.4 | 35093.9 | 33778.1 | 21708.9 | 33200.2 | 37492 |
| 68 | 63 | 2405 | 2694.9 | 2196.1 | 5092.2 | 382 | 1972.5 | 2314.2 | 2245.9 |
| 69 | 756.5 | 5955.8 | 7077.6 | 5234.2 | 6301.1 | 1133.5 | 5870 | 6218.4 | 8519.4 |
| 70 | 424.6 | 2064.6 | 2173.1 | 4141.4 | 1212.7 | 1829.6 | 2418 | 1388.8 | 1591.4 |
| 71 | 499.3 | 545.4 | 159.2 | 507.5 | 2187.3 | 258.8 | 298.2 | 106.5 | 73.5 |
| 72 | 431.6 | 355.2 | 472.7 | 512.9 | 768.2 | 712.7 | 665.7 | 1451.1 | 410 |
| 73 | 272.6 | 264.5 | 266.7 | 235 | 473.2 | 341 | 218 | 433.5 | 361.9 |
| 75 | 120.4 | 364.8 | 176 | 279 | 491.9 | 225.9 | 244.3 | 377.5 | 440.7 |
| 76 | 153.3 | 176.5 | 46 | 143.8 | 95.5 | 498 | 182.1 | 2 | 34.8 |
| 77 | 102.1 | 3322.2 | 3704.4 | 3549.3 | 7422.7 | 3279.7 | 2337.5 | 2786.8 | 6811 |
| 78 | 297.4 | 973.1 | 1410.8 | 487 | 1074.9 | 411.4 | 315 | 1025.7 | 834.1 |
| 79 | 126.7 | 120.4 | 132.9 | 181.3 | 166.4 | 87.6 | 155.4 | 114.9 | 113.9 |
| 80 | 153.8 | 328.9 | 160.6 | 571.7 | 267 | 307.1 | 430.9 | 345.1 | 451.1 |
| 81 | 179.7 | 181.8 | 101.5 | 996.9 | 161.1 | 619.2 | 1156.4 | 73 | 105.5 |
| 82 | 54.9 | 362.8 | 385.4 | 367 | 264.5 | 251.3 | 241.3 | 414.2 | 220 |
| 84 | 101.2 | 291.6 | 129.7 | 162.2 | 330.9 | 286.7 | 196.3 | 75.3 | 77.5 |
| 85 | 558.7 | 171.1 | 121.8 | 116.1 | 116.9 | 188.5 | 368.6 | 191 | 161.4 |
| 86 | 100.5 | 90.5 | 67.6 | 100.4 | 71.3 | 78.5 | 104.6 | 80.5 | 101.4 |
| 87 | 144.9 | 375.2 | 299.1 | 190 | 267 | 227.3 | 94.1 | 70.7 | 488.1 |
| 88 | 136.1 | 4022.9 | 4905.4 | 2121.4 | 4345.4 | 4155.7 | 1621.6 | 5239.4 | 7618.2 |
| 89 | 361.1 | 158.9 | 159.8 | 335 | 218.5 | 244.6 | 224.8 | 136.2 | 163 |
| 90 | 1105.6 | 113.4 | 92.7 | 212.6 | 301.1 | 131.7 | 229.6 | 81.7 | 75.7 |
| 91 | 41283.8 | 405.2 | 83.4 | 373.4 | 101.5 | 3129.9 | 2037.3 | 10.9 | 73.8 |
| 92 | 125.5 | 4171.4 | 3562.5 | 2666.8 | 2820.2 | 7133.6 | 1929 | 11013.3 | 6783.5 |
| 93 | 102.9 | 336.1 | 636.6 | 108.4 | 214.1 | 218.9 | 125.8 | 330.1 | 222.8 |
| 94 | 173.3 | 927 | 600.9 | 384.5 | 1060.6 | 985.2 | 285.3 | 819.3 | 759 |
| 95 | 43.6 | 796 | 225.2 | 699.2 | 229.7 | 1129 | 264.2 | 589.6 | 347.5 |
| 96 | 166.4 | 173.4 | 234 | 108.8 | 198.5 | 171.6 | 90.4 | 154.2 | 139.9 |
| 97 | 28.3 | 196.3 | 256.1 | 110.4 | 302.3 | 307.3 | 112.4 | 520.9 | 415.1 |
| 98 | 33 | 47.3 | 44.5 | 6.1 | 33.2 | 34.4 | 62.9 | 9.7 | 34 |
| 99 | 315.9 | 219.6 | 221.2 | 281.5 | 254.6 | 306.8 | 218.9 | 200 | 240.6 |
| 100 | 40.3 | 12275.1 | 7465.8 | 4158.8 | 5537.2 | 19961.1 | 1057.9 | 1835.4 | 28160.9 |
| 101 | 169.6 | 185 | 264.2 | 312.3 | 225.5 | 277.8 | 165.2 | 258.7 | 276.2 |
| 102 | 208.7 | 160.9 | 193 | 308.2 | 155.3 | 201.8 | 154.2 | 171.3 | 189.5 |

(continued)

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 103 | 402 | 302.9 | 300.1 | 382.9 | 342.4 | 374.1 | 315.3 | 292.4 | 280.2 |
| 104 | 25.2 | 569 | 354.3 | 402.2 | 554.5 | 1086.3 | 407.5 | 469.3 | 840.9 |
| 105 | 46.4 | 550.9 | 418 | 437.1 | 724.9 | 1141 | 481.6 | 596 | 1001.3 |
| 106 | 15.2 | 622.8 | 398.8 | 419.8 | 577.5 | 1074.2 | 377.8 | 444.5 | 794.4 |
| 107 | 6337.1 | 255.7 | 210 | 448.7 | 353.8 | 639.6 | 315 | 91.3 | 155.9 |
| 108 | 167.9 | 316.2 | 436.9 | 315 | 429.5 | 269.2 | 221.7 | 417.3 | 467 |
| 109 | 64.8 | 1110.3 | 875.6 | 160.6 | 318.4 | 942.9 | 215.8 | 1481.1 | 53.8 |
| 110 | 1.1 | 1261.5 | 1589.1 | 263.4 | 923.8 | 4084.9 | 415 | 4417 | 6.3 |
| 111 | 33.1 | 1767.8 | 1964.9 | 1872.4 | 427.3 | 1690.7 | 1292.2 | 749.5 | 1008.9 |
| 112 | 1.3 | 407.2 | 104.1 | 401.9 | 271.4 | 454.9 | 209.1 | 219.9 | 30.2 |
| 113 | 139 | 51.3 | 189.9 | 275.6 | 197.3 | 169.2 | 225.7 | 104.1 | 185.9 |
| 114 | 268.1 | 679 | 853.3 | 378.5 | 772.9 | 883.3 | 311 | 353.1 | 443.1 |
| 115 | 357.8 | 569.5 | 376 | 383.7 | 574.8 | 380.6 | 337.8 | 221.5 | 323.5 |
| 116 | 291.2 | 223 | 163.2 | 334.1 | 242.5 | 288.6 | 199.1 | 177.2 | 195.2 |
| 117 | 289.8 | 5414.4 | 4654.4 | 1394.2 | 3310.6 | 2815.7 | 721.7 | 3557.6 | 5696.6 |
| 118 | 92 | 533.6 | 90.4 | 527.7 | 275.1 | 665.7 | 306.8 | 60.3 | 99.6 |
| 119 | 114.7 | 226.3 | 336.7 | 194.9 | 102.7 | 168.3 | 397.7 | 81.7 | 65 |
| 120 | 78.6 | 116.4 | 118.2 | 137 | 338.5 | 139 | 164.3 | 51.8 | 73.3 |
| 121 | 161.2 | 788.8 | 997.1 | 260.6 | 745.8 | 1450.3 | 199.5 | 403.7 | 483.3 |
| 122 | 90.3 | 263.5 | 89.9 | 186.6 | 106.4 | 226.4 | 124.7 | 51.1 | 205 |
| 123 | 172.4 | 136.9 | 175 | 193.8 | 162.9 | 133.6 | 111.5 | 92.3 | 143.1 |
| 124 | 182.9 | 130.4 | 129.2 | 229.5 | 137.2 | 159 | 182.4 | 106.9 | 127 |
| 125 | 771.9 | 885.8 | 846.9 | 1521.7 | 915.3 | 963.4 | 856.9 | 836.5 | 775.4 |
| 126 | 25.4 | 1583.4 | 1549.9 | 728 | 2100.4 | 1526.4 | 558.8 | 1490.2 | 1266 |
| 127 | 101.3 | 121.8 | 97.6 | 163.5 | 99.6 | 101.1 | 101.1 | 74.1 | 160.2 |
| 128 | 346 | 234 | 238.5 | 469 | 239.1 | 325 | 198.4 | 245.9 | 311.8 |
| 129 | 4494.1 | 248.8 | 234 | 274.6 | 301.2 | 383.9 | 270 | 72.7 | 148.1 |
| 130 | 117.9 | 100.2 | 138.4 | 121.9 | 98.9 | 104 | 97.7 | 33 | 120.6 |
| 131 | 95.6 | 67.8 | 71.9 | 133.7 | 83.3 | 81.3 | 92 | 127.2 | 160.1 |
| 132 | 232 | 188.4 | 200.5 | 251 | 174 | 266.8 | 171.5 | 186.5 | 257.7 |
| 133 | 31.9 | 91.9 | 61.8 | 130.6 | 56.7 | 175.5 | 98.6 | 96.9 | 108.1 |
| 134 | 68.7 | 109.8 | 181.9 | 126.1 | 162.2 | 149.1 | 117.5 | 169.9 | 162.7 |
| 135 | 342.4 | 599 | 423.7 | 336.6 | 593.1 | 454.2 | 196.2 | 246.7 | 390.2 |
| 138 | 376.2 | 149.8 | 97.5 | 172.2 | 167.1 | 141.1 | 170.9 | 102.7 | 126.1 |
| 139 | 109.4 | 107.8 | 120.5 | 114.4 | 131 | 110 | 134.2 | 47.6 | 78.6 |
| 140 | 50.4 | 4099.6 | 2561.4 | 1129.9 | 2225.8 | 4646.8 | 179.3 | 3587 | 3225.4 |
| 141 | 28.7 | 1684.3 | 2067.9 | 1954.8 | 1743.8 | 2519.7 | 838.9 | 2235.9 | 1940.3 |

(continued)

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 144 | 530.5 | 489.8 | 450.9 | 680.6 | 482.9 | 521.4 | 406.9 | 3706.9 | 433.4 |
| 145 | 237.2 | 196.3 | 199.6 | 376.6 | 227.5 | 244 | 164 | 175.5 | 217.2 |
| 146 | 240 | 207.7 | 196.5 | 287 | 216.2 | 221.3 | 171.1 | 170.3 | 240.1 |
| 147 | 34.8 | 59.1 | 32.6 | 71.1 | 106.8 | 38 | 76.9 | 66.7 | 44.2 |
| 148 | 677.5 | 348.2 | 249.8 | 461.1 | 251.5 | 501.4 | 616.9 | 292.6 | 327.2 |
| 149 | 35.2 | 397.1 | 361.6 | 203.1 | 742.4 | 464.8 | 110.1 | 706.6 | 571 |
| 150 | 205.4 | 160.9 | 229.9 | 251 | 200.4 | 189.2 | 332.7 | 156.6 | 501.8 |
| 151 | 173.8 | 466.2 | 220 | 285.9 | 235.8 | 499.7 | 139.8 | 284.8 | 258.5 |
| 153 | 96.7 | 149.4 | 109.5 | 120 | 139.6 | 106.3 | 110.4 | 101.5 | 94.3 |
| 154 | 353.7 | 288.8 | 62.8 | 174.6 | 358.1 | 579.2 | 122.3 | 45.3 | 359.9 |
| 155 | 497.4 | 136.2 | 109.5 | 189.4 | 118.5 | 147.5 | 131.9 | 115.5 | 137.5 |
| 156 | 73 | 97.7 | 84.6 | 132.7 | 100.1 | 118.9 | 87 | 64.6 | 73 |
| 157 | 153 | 149.2 | 105 | 143.1 | 123.7 | 160.2 | 159 | 151.6 | 147.2 |
| 158 | 1.3 | 8.9 | 102.4 | 99.5 | 1.1 | 23.1 | 74.8 | 7.9 | 93.2 |
| 159 | 33 | 2887.3 | 3372.9 | 2710.7 | 3432.1 | 3979.2 | 1070.5 | 3809.9 | 4336.8 |
| 161 | 1.3 | 134.2 | 113.3 | 263.6 | 128.2 | 276.1 | 557.1 | 118.4 | 2.9 |
| 162 | 1735.6 | 170 | 24.1 | 264.5 | 702.3 | 122.3 | 365 | 31.1 | 85.1 |
| 164 | 94.6 | 209.1 | 2869.2 | 348.2 | 520.8 | 2102.6 | 33.2 | 1670.7 | 436.5 |
| 166 | 700.4 | 6433.5 | 9921.6 | 4090.9 | 7108.3 | 3609.5 | 3473.4 | 7815.7 | 5478.4 |
| 167 | 1.2 | 2320.4 | 1318 | 1994.5 | 3064.9 | 2291.7 | 729.2 | 3184.3 | 2565.5 |
| 168 | 190 | 593.2 | 775.5 | 598.1 | 769.2 | 610.1 | 414.8 | 1931.6 | 1123.8 |
| 169 | 512.2 | 4400.2 | 2669.8 | 2975.6 | 2812.9 | 6215.7 | 3878.6 | 1890.1 | 829.9 |
| 170 | 73.5 | 1014.4 | 2146.7 | 775.8 | 1454.9 | 1033.1 | 409.3 | 2535.1 | 867.2 |
| 171 | 5104.5 | 1332.2 | 272.6 | 2234.5 | 4145.4 | 565.7 | 3636.7 | 1.3 | 1.2 |
| 172 | 697.5 | 650.4 | 797.2 | 721.1 | 621.5 | 792.4 | 573.5 | 666.6 | 657 |
| 173 | 117 | 169.1 | 136.9 | 226.4 | 229 | 245.8 | 226.4 | 407.6 | 310.5 |
| 176 | 1.2 | 1.2 | 3906.6 | 275.7 | 74.8 | 809.2 | 3.4 | 2700.3 | 62.8 |
| 177 | 101 | 617.1 | 461.8 | 602.7 | 515.7 | 623.3 | 414 | 876.6 | 749.6 |
| 178 | 167.1 | 612.8 | 1577.1 | 197.3 | 112.9 | 554.5 | 318.8 | 310.2 | 64.7 |
| 179 | 153.4 | 58.6 | 90.9 | 90 | 66.8 | 93.5 | 114.4 | 42.3 | 93.2 |
| 180 | 501.7 | 70.9 | 72.4 | 141.5 | 208.3 | 85.6 | 126.3 | 91.4 | 102.8 |
| 181 | 624.8 | 12366.2 | 9356.2 | 8736.7 | 10384.9 | 9225.7 | 6284 | 9589 | 10736.6 |
| 182 | 416.7 | 513.2 | 472.4 | 300.9 | 658.9 | 537.7 | 246.8 | 533.1 | 527.2 |
| 183 | 2674.8 | 1193.3 | 1.1 | 989.7 | 2589.1 | 336.1 | 836.3 | 1.1 | 10.3 |
| 184 | 424.7 | 204.6 | 210.9 | 228.6 | 394.8 | 275.7 | 205.5 | 185.6 | 179.6 |
| 185 | 1535 | 1703.8 | 1133.3 | 2133.8 | 2209.6 | 911.3 | 1201.8 | 1162.9 | 1148.4 |
| 186 | 489.1 | 547.1 | 422.7 | 473 | 508.7 | 434.6 | 349.5 | 507.5 | 315 |

(continued)

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 187 | 1394 | 260.6 | 57.1 | 168.9 | 226.7 | 84.5 | 424 | 34.5 | 60.7 |
| 188 | 257.6 | 162.3 | 197.5 | 380.9 | 488.7 | 103.9 | 313.1 | 102.5 | 160.7 |
| 189 | 66.4 | 86.3 | 121.9 | 244.8 | 239.7 | 78.6 | 190.3 | 75.4 | 112.2 |
| 190 | 78.1 | 83.5 | 429.5 | 280.5 | 102.5 | 357.6 | 91.9 | 97.6 | 84.8 |
| 191 | 5186 | 883.9 | 1804.8 | 892.9 | 1302.6 | 986.6 | 662.9 | 1537.4 | 1728.1 |
| 192 | 183.6 | 117.1 | 105.4 | 207.3 | 160.4 | 130.7 | 133.3 | 86.8 | 109.4 |
| 193 | 75.2 | 1034.1 | 1775.6 | 473 | 1246.2 | 728.5 | 400.4 | 2163.8 | 1704.8 |
| 194 | 35.4 | 141 | 109.3 | 203.6 | 162.4 | 216.6 | 81.4 | 282.5 | 166.3 |
| 195 | 166.4 | 286.5 | 375.7 | 319.3 | 152.4 | 274.8 | 209.4 | 184.4 | 172.6 |
| 196 | 3.1 | 6834.9 | 4672.9 | 5030.2 | 2309.2 | 6216.8 | 4103.7 | 6137.9 | 6356.5 |
| 197 | 99.9 | 104 | 74.8 | 123.9 | 144.8 | 74.6 | 78.7 | 42.6 | 76.1 |
| 198 | 64.1 | 63.1 | 53.4 | 170.6 | 81.1 | 107.7 | 53.7 | 58.8 | 105 |
| 199 | 831.8 | 240 | 58.3 | 107.9 | 91.4 | 602.1 | 232 | 56.8 | 73.6 |
| 200 | 526.8 | 384.3 | 120.9 | 388.4 | 798.8 | 293.1 | 355.9 | 148.2 | 153 |
| 201 | 8960.4 | 165 | 22.2 | 144.7 | 80.9 | 2818 | 2028.7 | 40.3 | 67.2 |
| 203 | 81.3 | 472.4 | 610.9 | 91.8 | 451.2 | 472.3 | 101.5 | 229.9 | 173.5 |
| 204 | 111.5 | 557.7 | 815 | 343.5 | 524.4 | 484.1 | 188.1 | 623.8 | 532.3 |
| 205 | 110.1 | 6177.9 | 4916.2 | 3074.2 | 4380.7 | 5662.9 | 2513.5 | 6031.4 | 4413.3 |
| 206 | 327.4 | 232 | 221.4 | 2279.4 | 1294.7 | 242.3 | 23853.3 | 182.8 | 201.2 |
| 207 | 140.6 | 1170.8 | 430.3 | 478 | 1596.2 | 1348.5 | 347.2 | 2523.2 | 2245.3 |
| 208 | 102.4 | 611.4 | 208 | 94.9 | 323 | 317.4 | 205.4 | 75 | 371.1 |
| 209 | 19.6 | 3466.7 | 4058.3 | 3621 | 1656.7 | 3787.3 | 1837.3 | 3395.9 | 2777.2 |
| 211 | 68.5 | 13748.3 | 30156 | 19158.6 | 20412.6 | 11472.5 | 5548.4 | 13311.6 | 18245.4 |
| 212 | 97.3 | 492.1 | 523.3 | 1191.7 | 274.3 | 490.1 | 563.4 | 430.6 | 523.7 |
| 213 | 118.2 | 98.7 | 138 | 131.3 | 79.4 | 72.3 | 107.5 | 56.1 | 87.5 |
| 215 | 74.5 | 1676.2 | 558.9 | 803.5 | 1642.3 | 2856.5 | 476.4 | 4663.2 | 3720.1 |
| 216 | 75.1 | 912.5 | 1082.4 | 602 | 874 | 1309.1 | 366.3 | 1248.6 | 1513.4 |
| 217 | 253.9 | 338.8 | 345.6 | 232.8 | 478.1 | 255.5 | 260.8 | 183.8 | 153.8 |
| 218 | 217.6 | 281.1 | 360.1 | 283.7 | 411.2 | 483.8 | 185.6 | 541.5 | 461.7 |
| 220 | 116.2 | 103.9 | 66.9 | 97.5 | 83.3 | 99.7 | 69.5 | 88.4 | 87.1 |
| 221 | 587 | 961.9 | 558.8 | 297.6 | 954.6 | 365.8 | 346.2 | 594 | 456.9 |
| 223 | 239.4 | 255 | 135.7 | 374.9 | 272.4 | 283.3 | 188.8 | 205.6 | 338.9 |
| 227 | 16526.9 | 687.5 | 402.1 | 801.2 | 8633.7 | 549.7 | 683 | 662.9 | 533.2 |
| 228 | 109.5 | 590.7 | 434.3 | 616.8 | 410.6 | 745.3 | 291.4 | 596.4 | 397.3 |
| 229 | 238.4 | 255.8 | 193.3 | 117.2 | 266.5 | 145.6 | 228.4 | 102.2 | 491.9 |
| 230 | 226.6 | 172.7 | 87.5 | 128.9 | 178.7 | 109 | 152.9 | 81.6 | 97.2 |
| 231 | 164.7 | 239.9 | 241 | 177.8 | 240.8 | 204.3 | 82.9 | 126.8 | 110.2 |

(continued)

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 232 | 726.2 | 6011.4 | 69.4 | 4687.8 | 4109.5 | 1565.9 | 3685.2 | 55.1 | 121.4 |
| 233 | 1921.3 | 10660.6 | 3028 | 2711.7 | 7662.7 | 7862.6 | 1829 | 3283.3 | 5519.5 |
| 235 | 1985.5 | 721.4 | 238.4 | 931.8 | 2117.4 | 492.2 | 1437 | 77 | 96.1 |
| 236 | 111.5 | 389.5 | 124.3 | 281.9 | 194.3 | 199.7 | 268.5 | 87 | 132.1 |
| 237 | 678.2 | 2297.4 | 1864.1 | 1203 | 1986.1 | 1743 | 1173.9 | 1963.8 | 2872.9 |
| 238 | 100.7 | 1551.2 | 1160.8 | 572.2 | 1309.9 | 1228.5 | 451 | 1210.1 | 642.7 |
| 239 | 332.1 | 763.7 | 202 | 225.7 | 393.5 | 575.5 | 339 | 267.4 | 73.6 |
| 240 | 2452.6 | 853.2 | 251.5 | 167.9 | 951.3 | 599.5 | 378.3 | 208.9 | 113.2 |
| 241 | 3976.2 | 1815.3 | 487.3 | 320 | 1245.6 | 1299.5 | 844.5 | 423.8 | 113.2 |
| 242 | 22247.9 | 1204.2 | 676.7 | 2441.5 | 3900.1 | 2073.4 | 2060.1 | 1433.3 | 2211.8 |
| 243 | 500.8 | 602.3 | 147.7 | 417.4 | 460.7 | 206.9 | 420.7 | 210.1 | 264.1 |
| 244 | 408.8 | 194.7 | 250 | 139.3 | 215 | 163.3 | 362.3 | 191.9 | 185.1 |
| 246 | 346.4 | 311.5 | 153.6 | 129.5 | 426.7 | 193.8 | 297.6 | 237.1 | 115.3 |
| 247 | 228 | 128.5 | 128.2 | 180.1 | 149.8 | 122.1 | 119 | 95 | 82.6 |
| 248 | 84.9 | 198.9 | 142.5 | 115.1 | 171.7 | 266.5 | 76.9 | 113.4 | 199.3 |
| 249 | 1035.7 | 288.9 | 2257.9 | 857.3 | 1164 | 1466.9 | 537.1 | 513.5 | 696.2 |
| 251 | 264 | 88.8 | 78.2 | 123 | 232.6 | 138 | 74.6 | 121.3 | 124.6 |
| 253 | 108.7 | 314.2 | 326.6 | 251.4 | 350.9 | 226.2 | 192.2 | 227.9 | 226.6 |
| 254 | 141.8 | 397.1 | 285.7 | 91.7 | 668.4 | 932.2 | 144.2 | 644.2 | 128.5 |
| 255 | 158.7 | 631.9 | 1918.6 | 177.7 | 94.3 | 560.5 | 328.5 | 275.4 | 74 |
| 256 | 28050 | 525.2 | 120.3 | 424.5 | 151.7 | 1961.1 | 1539.7 | 156.2 | 137.1 |
| 257 | 114.5 | 99.8 | 83 | 100.6 | 201.5 | 126.4 | 86.7 | 157.4 | 157.6 |
| 258 | 158.6 | 49.9 | 1.2 | 230.7 | 87 | 162.9 | 312.2 | 9.5 | 188.6 |
| 259 | 1056.7 | 678.8 | 74.6 | 435 | 975.6 | 278.5 | 381.2 | 78.8 | 19.9 |
| 260 | 181.2 | 203.6 | 112.1 | 206.3 | 156.5 | 223.4 | 178.5 | 122.7 | 146.6 |
| 261 | 102.9 | 308.1 | 236.7 | 105 | 398.6 | 247.2 | 179 | 878.1 | 456.3 |
| 263 | 715.4 | 341.5 | 448.7 | 152.3 | 586.5 | 224.7 | 469.7 | 458.1 | 389 |
| 263 | 497.6 | 226.9 | 137.3 | 149.6 | 251 | 166.4 | 214.5 | 85.4 | 135.2 |
| 265 | 94.8 | 312.7 | 829.9 | 450.4 | 436.1 | 369.5 | 258.6 | 592.9 | 638.8 |
| 266 | 265.8 | 327 | 344.5 | 288.3 | 382.4 | 344.9 | 252.4 | 143.5 | 246 |
| 256 | 250.1 | 118.4 | 95.6 | 163.6 | 115.3 | 138.5 | 119.7 | 91.9 | 107.1 |
| 268 | 9.8 | 979.7 | 661.1 | 894.3 | 411.9 | 1696.9 | 366.1 | 409.7 | 1018.8 |
| 269 | 54 | 49.9 | 40.5 | 67.3 | 34.6 | 39.1 | 37.9 | 27 | 41.9 |
| 270 | 250 | 173.8 | 181.8 | 187.3 | 170 | 224.5 | 142.3 | 228.7 | 279.3 |
| 271 | 480.9 | 213.9 | 276.2 | 361 | 316 | 273.2 | 153.9 | 288.4 | 298 |
| 272 | 848.6 | 504.7 | 506.3 | 704.7 | 547.1 | 495.8 | 552.5 | 478.3 | 596.9 |
| 273 | 1.2 | 2102.3 | 4434.2 | 854.4 | 4432 | 2983.7 | 1404 | 2522.8 | 2416.9 |

(continued)

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 274 | 1684.7 | 1062 | 218.2 | 668.5 | 812.7 | 391.8 | 925.2 | 159.9 | 290.7 |
| 275 | 287.7 | 447.3 | 282.7 | 622.1 | 481 | 245.2 | 328.9 | 276.9 | 234.7 |
| 276 | 24704.9 | 1039.3 | 565 | 1641.6 | 11368.8 | 805.3 | 1068.9 | 882.8 | 583 |
| 277 | 242 | 197.4 | 155.1 | 247.2 | 180.4 | 234.4 | 184.7 | 192.8 | 192.5 |
| 278 | 1231.4 | 973 | 145.3 | 901.4 | 1474.2 | 594.6 | 925.8 | 126 | 131.6 |
| 279 | 17.3 | 2232.4 | 2050.1 | 1268 | 1303.2 | 2923.4 | 900.4 | 2377.3 | 1977.2 |
| 280 | 59.4 | 2645.5 | 2730.2 | 1625.9 | 2545.4 | 3355.1 | 1233.4 | 2801.5 | 3378.5 |
| 281 | 11.1 | 384.8 | 65 | 38.7 | 71.4 | 100.9 | 103.7 | 14.1 | 17.9 |
| 282 | 608.4 | 296.3 | 256.6 | 463.7 | 205.6 | 325.1 | 415.2 | 194.5 | 210.3 |
| 283 | 332.9 | 264.5 | 234.2 | 544.1 | 264.3 | 374.3 | 400.4 | 291.5 | 271.4 |
| 284 | 78.3 | 67.8 | 69.4 | 78.9 | 68 | 92 | 71.1 | 59.9 | 88.3 |
| 285 | 128.1 | 76.9 | 110.6 | 115.5 | 106.6 | 107.8 | 83.1 | 89.2 | 95.8 |
| 286 | 136.9 | 882.8 | 1547.8 | 797.2 | 698 | 1112.3 | 458.4 | 673.7 | 251.9 |
| 287 | 133.4 | 121.2 | 112.9 | 117.4 | 91.9 | 158.2 | 87 | 70.4 | 94.5 |
| 288 | 171.7 | 149.6 | 158.2 | 178.1 | 157.8 | 201 | 117 | 123.4 | 124.4 |
| 289 | 2065.2 | 477.9 | 207.9 | 783.6 | 1036.1 | 420.3 | 2425.5 | 18.4 | 1.3 |
| 291 | 133.5 | 64.4 | 97.5 | 72.7 | 132.7 | 73.5 | 87.3 | 58.9 | 49.4 |
| 292 | 584.5 | 63.2 | 25 | 154.1 | 1191.9 | 660.3 | 757.5 | 27.5 | 40.9 |
| 293 | 85.6 | 186.6 | 107 | 288.9 | 87.8 | 189 | 235.3 | 128.1 | 55.8 |
| 295 | 99.7 | 791.3 | 242.3 | 325.4 | 327.2 | 175.4 | 230.6 | 133.1 | 144.9 |
| 296 | 676.2 | 918.1 | 709.4 | 934.2 | 1877.1 | 1141.7 | 589.6 | 753.4 | 857.2 |
| 297 | 208.1 | 61.9 | 45 | 125.1 | 78.1 | 67 | 128.8 | 33.5 | 56.2 |
| 298 | 486.9 | 293.3 | 320.6 | 257.4 | 410.5 | 356.9 | 211.9 | 196.2 | 400.4 |
| 299 | 238.1 | 923.6 | 448.9 | 1006.6 | 1054.5 | 910 | 660.9 | 696.1 | 686.7 |
| 300 | 215.4 | 203.8 | 395.4 | 255.6 | 312.3 | 188.4 | 202.2 | 244.5 | 347.9 |
| 301 | 212.9 | 290 | 820.4 | 372.3 | 708.3 | 367.5 | 860.1 | 1368.3 | 1185.7 |
| 302 | 29.5 | 25.8 | 10.5 | 19 | 34.5 | 45.7 | 38.4 | 9 | 11.2 |
| 303 | 244.8 | 782.2 | 902.3 | 349.1 | 907.8 | 713.9 | 323.7 | 823.8 | 976.6 |
| 304 | 1861.6 | 166.7 | 207.3 | 308.3 | 367.6 | 378.4 | 394.1 | 160.5 | 243.8 |
| 305 | 175.3 | 159.5 | 1205.8 | 156.7 | 144.6 | 223.3 | 176.9 | 191.7 | 180 |
| 306 | 257.6 | 154.3 | 146.1 | 240.3 | 149 | 206.8 | 136.3 | 126 | 182.9 |
| 307 | 140.9 | 142.6 | 126.5 | 207 | 130.3 | 179.3 | 122.6 | 127.1 | 144.6 |
| 308 | 51.2 | 1843.1 | 1030.9 | 1212.9 | 1456.7 | 2208.3 | 994.9 | 1319.8 | 3640.6 |
| 310 | 56.1 | 389.3 | 310.4 | 316.3 | 466.8 | 385.1 | 278.8 | 726.7 | 401.6 |
| 311 | 95.8 | 109.3 | 154.9 | 90 | 107.1 | 120.1 | 96.3 | 40.5 | 202.1 |
| 312 | 1.1 | 193.8 | 599 | 390.5 | 611.6 | 728.7 | 365.5 | 1657.6 | 289.8 |
| 313 | 114.1 | 119.7 | 208.9 | 142.2 | 124.9 | 120.7 | 102.2 | 171.4 | 137.5 |

(continued)

| 3a | | | | | | | | | |
|---|---|---|---|---|---|---|---|---|---|
| SEQ ID NO: | 800TT | 801TT | 802TT | 804TT | 805TT | 806TT | 807TT | 818TT | 819TT |
| 314 | 228.4 | 414.8 | 146.7 | 189.2 | 419.3 | 985 | 268.1 | 164.1 | 805.7 |
| 315 | 106.4 | 758.2 | 540.2 | 634.9 | 351.4 | 466.9 | 317.2 | 369.3 | 322.4 |
| 316 | 87.3 | 363.4 | 151 | 353 | 425.1 | 312.8 | 284.4 | 214.7 | 159.2 |
| 317 | 349.5 | 178.8 | 142.4 | 246 | 187.7 | 213.8 | 173.4 | 172.5 | 170.8 |
| 318 | 41 | 165 | 47.1 | 88.3 | 62.8 | 174.6 | 58.9 | 29.9 | 36.8 |
| 319 | 434.9 | 249.1 | 85.8 | 420.7 | 189.3 | 189.1 | 249.8 | 96.7 | 149.6 |
| 320 | 107.4 | 586.7 | 418.3 | 403.8 | 488.4 | 523.9 | 404.2 | 689.4 | 494.1 |
| 321 | 543.8 | 319.1 | 469.7 | 514.1 | 488.3 | 438.7 | 255 | 599.8 | 413.8 |
| 322 | 143.6 | 271.4 | 269.5 | 298.2 | 237 | 237.4 | 211.8 | 133.2 | 239 |
| 323 | 70.7 | 3285.6 | 3624.3 | 1105.3 | 4318.5 | 2487.4 | 569 | 2285.5 | 3132.6 |
| 324 | 83.1 | 109.6 | 285.4 | 143.7 | 116.7 | 132.5 | 85.8 | 120.8 | 77.4 |
| 325 | 110.9 | 251.9 | 316.5 | 290 | 236.5 | 383.2 | 230.7 | 313.5 | 303.7 |
| 326 | 135.5 | 3242.5 | 8125.6 | 4127.5 | 5469.6 | 4502.9 | 1608.5 | 3693.9 | 2895.8 |
| 327 | 58.5 | 45 | 1.2 | 25 | 59.9 | 35 | 17.5 | 37.7 | 1.1 |
| 328 | 65.8 | 655.8 | 464.1 | 320.5 | 288.4 | 542.9 | 197.8 | 325.2 | 508.2 |
| 329 | 80 | 64.2 | 69 | 112.3 | 56 | 94.8 | 79.6 | 45.7 | 59.5 |
| 330 | 40.5 | 80.1 | 60.7 | 58 | 25.8 | 201.8 | 52.3 | 15.2 | 35 |
| 331 | 432.9 | 313.6 | 325.8 | 596 | 282.5 | 296.4 | 296.4 | 323.7 | 289.3 |
| 333 | 130.6 | 705.5 | 966.7 | 555.7 | 561.6 | 839.7 | 421.8 | 765.6 | 867.7 |
| 334 | 249.3 | 142.3 | 160.4 | 202.9 | 176.2 | 182.3 | 124.3 | 206.3 | 169.1 |
| 335 | 82.5 | 80.4 | 83 | 109.4 | 123.6 | 101.9 | 119.4 | 73.9 | 145.7 |
| 336 | 190.4 | 460 | 647.9 | 449.1 | 599.6 | 811.8 | 357.9 | 794.4 | 514 |
| 337 | 325.2 | 155.4 | 169.1 | 87.4 | 565.9 | 331.4 | 139.4 | 121 | 218.6 |
| 338 | 456.9 | 208.6 | 167.7 | 232 | 253.9 | 347.6 | 196.9 | 230.3 | 166.2 |
| 339 | 286.9 | 555.1 | 405.8 | 559 | 379.8 | 628.6 | 310.9 | 351.5 | 538.6 |
| 340 | 512.3 | 229.9 | 206.7 | 351.4 | 259.7 | 284.5 | 241.8 | 186.5 | 258.4 |
| 341 | 149.5 | 767.9 | 579.4 | 500.3 | 838.9 | 598.2 | 472.1 | 804.7 | 865.1 |
| 343 | 152.9 | 40.5 | 579 | 1479.8 | 52.4 | 120.2 | 411.9 | 326.7 | 85 |
| 344 | 233.7 | 1144.2 | 879.2 | 1306.4 | 1034.5 | 1267 | 1054.1 | 1516.9 | 1049.3 |
| 345 | 371.6 | 243.5 | 219 | 304.9 | 271.1 | 232.5 | 238.5 | 219.9 | 295.3 |
| 347 | 98.2 | 3633.1 | 2744.6 | 2838.4 | 3198.4 | 4569.8 | 1600.1 | 1448.8 | 3315.8 |
| 348 | 78.5 | 6124.8 | 6613.5 | 4006.5 | 4485.3 | 7289.4 | 3180.7 | 2561.5 | 5272.3 |
| 349 | 582.7 | 260 | 240.8 | 164.6 | 205.7 | 203.8 | 225.1 | 195.3 | 203.1 |
| 350 | 355.9 | 281.6 | 330.4 | 285.5 | 454.2 | 255 | 105.2 | 161.7 | 183.4 |
| 351 | 732.9 | 348.3 | 47.4 | 261.9 | 443.7 | 347.8 | 457.3 | 73.6 | 169.9 |
| 352 | 162.3 | 297 | 339.9 | 197.7 | 488.4 | 390.6 | 178.5 | 201.7 | 300.5 |
| 353 | 186.4 | 405.1 | 211.5 | 354.7 | 282.6 | 265.3 | 148.4 | 203 | 378.9 |

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 354 | 101.8 | 32.1 | 469 | 263.8 | 1085.8 | 22 | 126.1 | 35 | 1609.8 |
| 355 | 50.1 | 45.1 | 504.2 | 78.3 | 2521.5 | 71 | 173 | 79.8 | 1017.3 |
| 356 | 697.4 | 939.2 | 1065.9 | 788.9 | 148.7 | 218.1 | 645.8 | 867.3 | 539.6 |
| 357 | 470.4 | 512.9 | 724.1 | 516.5 | 395.9 | 181 | 909.4 | 596 | 552.3 |
| 358 | 572.8 | 424.1 | 483.2 | 901.7 | 829.4 | 659.8 | 1454.2 | 582.7 | 1006.9 |
| 359 | 347.5 | 382.6 | 400.4 | 351.2 | 192.3 | 373.5 | 325.1 | 300.2 | 434.7 |
| 362 | 518.5 | 320.1 | 492.7 | 704.3 | 136.5 | 151.1 | 355.4 | 543.3 | 388.6 |
| 360 | 319.8 | 228.6 | 329.3 | 403.7 | 774.2 | 179.6 | 366.3 | 240.3 | 358.5 |
| 361 | 480.9 | 1229.9 | 857.7 | 620.4 | 288.1 | 256 | 1180.4 | 566.6 | 668.1 |
| 363 | 418.7 | 427.1 | 382.7 | 545 | 359 | 1019.2 | 338.9 | 487.8 | 695 |
| 1 | 693.8 | 684 | 807.9 | 656.3 | 335.6 | 360.3 | 549.1 | 242.6 | 662.7 |
| 2 | 1188.2 | 4262.8 | 2973.5 | 2345.3 | 485.6 | 836.8 | 2548.2 | 5155 | 2907.2 |
| 7 | 285 | 570.3 | 706.5 | 933.7 | 231.3 | 398 | 465.3 | 243.7 | 598.5 |
| 8 | 772 | 941.1 | 585.4 | 1410 | 767.6 | 899.7 | 976.4 | 715.3 | 1364.2 |
| 9 | 6790.1 | 6640.5 | 7386.3 | 3225.8 | 5435.7 | 126.3 | 4807.5 | 1611.9 | 3114.7 |
| 10 | 127.8 | 73.6 | 88.9 | 165.8 | 1940.4 | 149.4 | 211.5 | 105.7 | 161.7 |
| 11 | 219.2 | 282.4 | 325.1 | 111.4 | 558 | 78.5 | 557.8 | 260.1 | 213.2 |
| 13 | 97.3 | 102.3 | 156.9 | 158.8 | 964.8 | 160.6 | 157.6 | 106.2 | 231.8 |
| 14 | 280.4 | 281.5 | 511.4 | 335.9 | 1351.5 | 111.1 | 708.2 | 213 | 543.4 |
| 15 | 407.6 | 174.9 | 613.5 | 2405.8 | 5398.8 | 42.5 | 1013.9 | 261.5 | 818.7 |
| 16 | 996 | 867.4 | 1065.2 | 1313.3 | 639.8 | 600.3 | 893.2 | 655.3 | 1176.4 |
| 17 | 905 | 1286.8 | 1004.5 | 1341.4 | 3130.7 | 458.2 | 571.6 | 1357.1 | 567.7 |
| 19 | 176.2 | 160.5 | 2137 | 215.5 | 362.5 | 258.3 | 1756.9 | 171.6 | 407.1 |
| 20 | 2.8 | 1.1 | 5.6 | 55.2 | 3077.9 | 1.1 | 341.3 | 1.2 | 120.3 |
| 21 | 198.4 | 8.3 | 87.4 | 53.3 | 32.8 | 37.4 | 1005.1 | 37.9 | 87.5 |
| 22 | 6631.1 | 1931.6 | 3566.8 | 5632 | 5189.5 | 1534.9 | 4150.7 | 7366.1 | 5102.6 |
| 23 | 182.8 | 21.2 | 532.3 | 340.7 | 1711.8 | 1.1 | 1721.4 | 40.1 | 764.8 |
| 24 | 77.8 | 33.2 | 358.6 | 267.6 | 422.3 | 74.6 | 370.7 | 37.1 | 206.7 |
| 26 | 295.9 | 149.3 | 5582.1 | 822 | 2610.5 | 73.9 | 6672.8 | 337.8 | 2827.2 |
| 27 | 919.8 | 264.6 | 443.1 | 611.7 | 683.8 | 209.2 | 1842.7 | 221 | 356.4 |
| 29 | 70.7 | 70.7 | 74.8 | 144.2 | 604.4 | 984.4 | 261 | 138.9 | 191.1 |
| 30 | 121.9 | 25.4 | 650.1 | 375.7 | 4468.9 | 46.5 | 561.6 | 45.4 | 242.8 |
| 31 | 5697.5 | 123.5 | 224.1 | 147.9 | 1115.1 | 83.1 | 610.3 | 182 | 319.6 |
| 33 | 468.4 | 153 | 716.1 | 13385.7 | 6334.6 | 140.7 | 1847 | 224.8 | 1175.1 |
| 34 | 1720.2 | 2051.3 | 3037.5 | 1584.2 | 336.1 | 3724.7 | 2119 | 5586.2 | 2611 |
| 35 | 244.1 | 41.7 | 84.2 | 62.7 | 824.1 | 52.2 | 178.2 | 17.4 | 1328.5 |
| 36 | 119.6 | 118 | 169.3 | 230.6 | 1819.9 | 929.8 | 683 | 492.9 | 214.8 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 37 | 155.7 | 184.2 | 228.5 | 240.8 | 319.5 | 467.7 | 184.9 | 199.9 | 387.7 |
| 38 | 585.2 | 232.7 | 573.6 | 6068.7 | 4340.6 | 74.3 | 1214.6 | 360.9 | 671.8 |
| 40 | 591.8 | 535.9 | 446.1 | 487.3 | 508.4 | 640.3 | 459 | 382.6 | 707.6 |
| 41 | 125.3 | 451.1 | 286.6 | 226.1 | 178.8 | 297.4 | 551.6 | 110.9 | 268.1 |
| 42 | 208.6 | 409.7 | 329.7 | 258.1 | 211.2 | 288.8 | 404.4 | 466.9 | 264.3 |
| 43 | 1026.6 | 218.9 | 326.6 | 574.3 | 230.1 | 671.1 | 309 | 239.3 | 385 |
| 44 | 615.3 | 369.3 | 535.2 | 505 | 392.7 | 412.7 | 711.3 | 532.7 | 637 |
| 45 | 520.7 | 482.1 | 383.7 | 299.9 | 78.6 | 389.4 | 649.9 | 316.5 | 478.7 |
| 46 | 320.2 | 300.6 | 305.2 | 401.7 | 263.7 | 512.6 | 527.8 | 382.7 | 432.7 |
| 47 | 139.1 | 150.5 | 229.5 | 149.7 | 531.9 | 197.1 | 197.3 | 127 | 210.4 |
| 48 | 1.3 | 21.1 | 476.7 | 92.9 | 168.2 | 12.9 | 551.2 | 39.1 | 389.5 |
| 49 | 166.1 | 218.5 | 298.7 | 280.5 | 457.8 | 255.1 | 900.8 | 192.7 | 389.9 |
| 50 | 347.9 | 99.6 | 314.6 | 484.2 | 1930.4 | 50 | 732 | 80.5 | 471.8 |
| 51 | 242.8 | 1.5 | 41.1 | 67.7 | 580.8 | 328.7 | 355.4 | 25.4 | 109.3 |
| 52 | 192.8 | 248.6 | 243.7 | 205.8 | 370.9 | 381.6 | 276.2 | 205.6 | 177.2 |
| 53 | 96.2 | 87.9 | 70.9 | 47.3 | 43.3 | 73.7 | 337.6 | 95.3 | 151.9 |
| 54 | 407.4 | 1349.6 | 375.9 | 248.1 | 781 | 299.7 | 1508.8 | 490.7 | 389.2 |
| 55 | 121.8 | 106.2 | 97.6 | 87.5 | 86 | 144.3 | 103.2 | 108.1 | 117.5 |
| 56 | 45.3 | 148.5 | 66.5 | 67 | 89.3 | 49.7 | 49.3 | 42.5 | 86.4 |
| 57 | 676 | 281.8 | 885.5 | 948.4 | 804.4 | 353.9 | 504.6 | 82.9 | 3332.6 |
| 58 | 778.6 | 312.3 | 1214.5 | 1060.8 | 1872 | 293.1 | 691.4 | 84.7 | 3886.7 |
| 59 | 1030.8 | 2434.1 | 2195.8 | 1055.8 | 320 | 324.2 | 1155.1 | 1409.3 | 875.3 |
| 60 | 135.3 | 164.3 | 164.2 | 188.6 | 621.3 | 165.8 | 256 | 106.3 | 154.4 |
| 61 | 162.8 | 141.6 | 158.3 | 159.3 | 275.5 | 228.7 | 281.2 | 168.6 | 177.2 |
| 62 | 4949.9 | 24.3 | 607 | 1227.9 | 429.7 | 1067.9 | 763.2 | 300.6 | 2120.4 |
| 63 | 484.5 | 316.4 | 328.9 | 307.7 | 341.1 | 48.2 | 473.5 | 163.4 | 154.3 |
| 64 | 204.6 | 186.8 | 246.6 | 401.8 | 70.3 | 580.2 | 1442.7 | 100.3 | 242.1 |
| 65 | 65.5 | 179.7 | 174.9 | 187.7 | 722.1 | 50 | 248.4 | 72.4 | 115.2 |
| 66 | 773.7 | 1116.3 | 780.2 | 512.2 | 301 | 358.5 | 869.5 | 898.9 | 607.4 |
| 67 | 28999.8 | 29358.2 | 32402.2 | 37818.6 | 3337.4 | 25748.4 | 22954.2 | 36641 | 37486.1 |
| 68 | 416.7 | 3006.9 | 3943.7 | 3062.4 | 153 | 2880.9 | 1180.9 | 1284.8 | 2957.1 |
| 69 | 1079.4 | 7132.7 | 8048.4 | 4826.8 | 649.2 | 8495.4 | 5023.1 | 8078.4 | 7915.3 |
| 70 | 6077.8 | 1872.9 | 984 | 682.7 | 249.3 | 3261.4 | 4520.3 | 1401.4 | 911.7 |
| 71 | 132.3 | 112.4 | 422.9 | 210.1 | 2216.8 | 82.9 | 814.1 | 97.5 | 287.6 |
| 72 | 490.8 | 1451.8 | 1261.7 | 235.1 | 471.1 | 74.1 | 306.4 | 974.8 | 1198.6 |
| 73 | 348.5 | 369.6 | 410.2 | 281.9 | 216.9 | 255.6 | 357.1 | 221.4 | 359.5 |
| 75 | 419.5 | 82.9 | 224.3 | 363.6 | 248.7 | 233.6 | 379.3 | 555 | 287.7 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 76 | 11.9 | 119.6 | 100.8 | 434.7 | 2481.2 | 1185 | 1252.2 | 685.6 | 485.4 |
| 77 | 4580.1 | 3450.4 | 5301.7 | 6090.7 | 261.8 | 3157.9 | 2146 | 4545.6 | 3500.3 |
| 78 | 1117.1 | 545.1 | 716.9 | 909.9 | 254.8 | 255.9 | 868 | 1420.7 | 711.9 |
| 79 | 530 | 164.2 | 183.5 | 180.9 | 240.5 | 155.8 | 185.8 | 142.8 | 115.9 |
| 80 | 603.5 | 1468.6 | 828.2 | 186.8 | 153.5 | 1495.3 | 273.8 | 761.5 | 324.2 |
| 81 | 1295.1 | 100.2 | 158.7 | 133.6 | 628.6 | 130 | 314.3 | 106.5 | 383.7 |
| 82 | 337 | 330.3 | 245.9 | 228.3 | 61.7 | 460.2 | 141 | 303.9 | 207.5 |
| 84 | 106.4 | 568.8 | 287.1 | 105.9 | 114.1 | 486.2 | 497.7 | 114.6 | 176.9 |
| 85 | 172.3 | 125.3 | 120 | 175.2 | 169 | 193.1 | 218.4 | 131.9 | 194 |
| 86 | 87 | 87.2 | 53.6 | 86.3 | 89.1 | 131.6 | 85 | 639.6 | 137 |
| 87 | 214.5 | 168.9 | 252.9 | 285.8 | 1403.4 | 202.4 | 230.4 | 184.8 | 289.6 |
| 88 | 1660.9 | 2332 | 6323.6 | 5821 | 275.2 | 1272.1 | 4080.3 | 7866.9 | 5791.5 |
| 89 | 206 | 177.6 | 198 | 327.1 | 1314.5 | 479.1 | 253.4 | 182.8 | 368.1 |
| 90 | 115.4 | 102.5 | 134.4 | 235.2 | 2070.3 | 140.2 | 141 | 111.9 | 226.2 |
| 91 | 502.7 | 105.1 | 1042 | 284.8 | 1947.8 | 244.5 | 511.1 | 18.4 | 4872 |
| 92 | 6425 | 5848 | 4350.4 | 2893.7 | 766.3 | 1143 | 8256.4 | 4985.1 | 2390.7 |
| 93 | 205.6 | 230.3 | 307.5 | 325 | 218.5 | 186.3 | 253.4 | 252 | 154.8 |
| 94 | 357.1 | 1091.1 | 1411.5 | 311.1 | 158.3 | 46.1 | 509.8 | 632.4 | 557.7 |
| 95 | 241.3 | 283.6 | 505 | 576.3 | 62.8 | 172.8 | 115.5 | 216.1 | 532 |
| 96 | 280.5 | 181.9 | 147 | 142.4 | 142.7 | 170.7 | 216.5 | 116.6 | 156.5 |
| 97 | 466.6 | 344 | 322 | 221.4 | 70.5 | 218.7 | 225.5 | 294.9 | 178.2 |
| 98 | 23.3 | 32.5 | 29.3 | 22.1 | 31.8 | 317.2 | 35.8 | 22 | 26.8 |
| 99 | 200.6 | 212.8 | 216.1 | 258.2 | 243.6 | 366.3 | 323 | 184.2 | 327.9 |
| 100 | 1421.1 | 302.2 | 3029.1 | 5780.9 | 673.2 | 1623.5 | 2932.3 | 3304.8 | 11319.8 |
| 101 | 272 | 218 | 267.6 | 467.2 | 55.2 | 386.1 | 265.8 | 234.8 | 421.9 |
| 102 | 152.6 | 160.8 | 134.1 | 185.4 | 141.5 | 525.5 | 8297.9 | 131.6 | 193.5 |
| 103 | 330.3 | 366.8 | 262.3 | 438.6 | 370.5 | 687.6 | 328.6 | 308.6 | 379.9 |
| 104 | 678.2 | 302.2 | 864.8 | 623.8 | 62.2 | 907.1 | 333.9 | 1068.4 | 672.3 |
| 105 | 495.9 | 329.5 | 905.2 | 487.5 | 77.9 | 996.4 | 425 | 1136.1 | 616.5 |
| 106 | 612.3 | 277.5 | 775 | 557.3 | 34.6 | 814.9 | 280 | 957.1 | 583.4 |
| 107 | 308.9 | 188.7 | 250.9 | 591.6 | 1517.4 | 241.8 | 305 | 161.8 | 1102.6 |
| 108 | 388.5 | 475.8 | 557.1 | 574.6 | 533.2 | 365.7 | 509.2 | 634 | 321.6 |
| 109 | 47.9 | 211.4 | 334.1 | 275.6 | 373.3 | 113.2 | 1573 | 359.4 | 1203.1 |
| 110 | 1.1 | 367.7 | 820.1 | 614 | 750.8 | 2.3 | 2128.4 | 450.7 | 2481.1 |
| 111 | 105.4 | 111.5 | 1115.1 | 1085.4 | 31.9 | 1134.1 | 919.7 | 2507.5 | 1720.3 |
| 112 | 62.4 | 132.4 | 118.7 | 104.6 | 124.9 | 88.9 | 1090.7 | 12.5 | 512.4 |
| 113 | 149.1 | 66.5 | 145.6 | 225.3 | 1395.8 | 112.7 | 316.5 | 230.1 | 145.4 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 114 | 929.6 | 594 | 672 | 804.4 | 155.5 | 238.7 | 339.5 | 618.6 | 378.6 |
| 115 | 473.6 | 276.3 | 355.8 | 431 | 779.1 | 286.2 | 826.4 | 464.8 | 562.1 |
| 116 | 228.8 | 183.6 | 205.8 | 214.2 | 113.4 | 286 | 233.8 | 157.3 | 281.6 |
| 117 | 1259.3 | 645.9 | 2714.7 | 1854.7 | 284.3 | 889.6 | 776.5 | 5906.8 | 1981.4 |
| 118 | 165 | 99.4 | 148.2 | 154 | 104.5 | 952.7 | 926.2 | 63.2 | 218.8 |
| 119 | 74.1 | 55.5 | 95.2 | 192.8 | 129.2 | 122.6 | 138.9 | 69 | 252.5 |
| 120 | 154.7 | 88.6 | 159.7 | 229.3 | 533 | 78.8 | 126.8 | 101.7 | 185.5 |
| 121 | 470.8 | 523.4 | 1077.7 | 284.1 | 270.2 | 79.9 | 1935.6 | 440.9 | 773.2 |
| 122 | 139.1 | 32 | 51.8 | 203.5 | 559 | 116.7 | 160.9 | 98.4 | 167.8 |
| 123 | 127.6 | 127.8 | 146.6 | 223 | 86.8 | 290.5 | 147 | 161 | 152.7 |
| 124 | 323.6 | 121.6 | 123 | 147.7 | 138.1 | 317.3 | 2203.4 | 125.6 | 200.2 |
| 125 | 909.9 | 886.8 | 770.6 | 1100 | 653.6 | 1873.2 | 823.8 | 980.3 | 1308.7 |
| 126 | 865.4 | 1870.3 | 1455.2 | 950.6 | 132.1 | 353.6 | 443.8 | 1299.3 | 756.7 |
| 127 | 146.8 | 89.1 | 116.4 | 108.3 | 102.8 | 176.3 | 89.7 | 112.1 | 164.4 |
| 128 | 199.2 | 243.1 | 243.5 | 292.8 | 184.9 | 435.5 | 169371.4 | 255.3 | 380.5 |
| 129 | 201 | 129.2 | 160.1 | 346.8 | 964 | 143.6 | 229.7 | 180.9 | 349.9 |
| 130 | 436.5 | 199.9 | 143.8 | 114.3 | 61.5 | 134.4 | 300.8 | 73.2 | 120.4 |
| 131 | 133.9 | 92.3 | 74.4 | 88.5 | 83.4 | 229.7 | 410.5 | 60.2 | 101.7 |
| 132 | 170.5 | 183.6 | 217.5 | 279 | 177.9 | 291.2 | 172.6 | 176.3 | 285.9 |
| 133 | 76.5 | 158.5 | 71.8 | 115.2 | 44.6 | 280.4 | 133.9 | 76.6 | 58.9 |
| 134 | 177.7 | 117.6 | 92.6 | 87.1 | 47.4 | 135.3 | 194.3 | 131.1 | 125.4 |
| 135 | 668.9 | 332.2 | 491.2 | 604 | 177.1 | 258 | 426.2 | 374.2 | 376.3 |
| 138 | 89.5 | 107.6 | 139.2 | 104.1 | 252.7 | 204.2 | 408.1 | 146.3 | 173.8 |
| 139 | 94.5 | 61.7 | 100.1 | 154 | 66.7 | 109.2 | 217.6 | 69.7 | 69.7 |
| 140 | 488.1 | 5040.7 | 5478 | 884.2 | 19.9 | 864.2 | 414.1 | 2686.3 | 2320.6 |
| 141 | 1820.2 | 2069.9 | 1896.4 | 1208 | 195.6 | 666 | 1637.7 | 1312.8 | 1389.1 |
| 144 | 389.9 | 2941 | 519.1 | 509.5 | 290.3 | 566.4 | 3501.1 | 2333.5 | 594.6 |
| 145 | 176.7 | 233.5 | 205.8 | 273.5 | 163.7 | 364.5 | 169 | 239.5 | 307.6 |
| 146 | 180.4 | 184.7 | 182 | 241.3 | 165.7 | 371.9 | 208.9 | 218.9 | 287.1 |
| 147 | 60.6 | 67.5 | 36.5 | 35.9 | 45 | 33 | 48.8 | 39.7 | 84.2 |
| 148 | 218 | 311.7 | 680.6 | 305.9 | 613.6 | 301.8 | 333.6 | 170.7 | 855.4 |
| 149 | 389.2 | 615.3 | 612.9 | 411.2 | 46.9 | 38.6 | 377.1 | 483.8 | 290.4 |
| 150 | 292.4 | 123.3 | 170.9 | 249.3 | 169.9 | 382.6 | 1326 | 246.2 | 272.1 |
| 151 | 172.2 | 109.5 | 207.1 | 176.5 | 173 | 197.7 | 201.3 | 53.4 | 217.8 |
| 153 | 100.2 | 143.8 | 139 | 113.1 | 97.6 | 119 | 115.6 | 97.7 | 102.5 |
| 154 | 965.5 | 130.7 | 240.3 | 422.3 | 239.6 | 103.1 | 344.9 | 332 | 536.6 |
| 155 | 150.4 | 145.1 | 158.1 | 122 | 114.6 | 231.1 | 116.7 | 136.3 | 190.3 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 156 | 72.3 | 57.3 | 76.9 | 97.8 | 60.5 | 106.5 | 85.4 | 49.1 | 91.7 |
| 157 | 92.1 | 101.4 | 129.5 | 97.6 | 197.6 | 183.4 | 266.9 | 102.3 | 169 |
| 158 | 105.7 | 18.1 | 95.3 | 31 | 1.1 | 1.5 | 124 | 10.3 | 17.7 |
| 159 | 3118.9 | 2438.5 | 2254 | 4357.3 | 169.7 | 2658.7 | 1376.2 | 3763.9 | 2939.4 |
| 161 | 5.6 | 102 | 74.9 | 43.7 | 13.3 | 206.3 | 5.9 | 19.5 | 43 |
| 162 | 86.4 | 52.8 | 243.7 | 212.1 | 2021.6 | 52.6 | 430 | 59.9 | 191.7 |
| 164 | 75.9 | 635.3 | 125.4 | 46.6 | 62.7 | 90.5 | 4939.7 | 520.4 | 106.5 |
| 166 | 8478.4 | 8844.4 | 7517.8 | 9251.3 | 668.6 | 5048 | 2532 | 11114.9 | 5155.6 |
| 167 | 944.4 | 2088.2 | 2168.9 | 3078.3 | 35.4 | 2685.3 | 569.9 | 438.5 | 2149.5 |
| 168 | 981.2 | 741.9 | 536.9 | 381.5 | 101.8 | 263.9 | 1589.1 | 1090 | 1157.8 |
| 169 | 998.2 | 828.4 | 1558.6 | 3346.8 | 4596.6 | 373.2 | 2769.4 | 732.9 | 18910.1 |
| 170 | 1690.4 | 2036.1 | 1458.2 | 1172.6 | 1929.3 | 472.1 | 1536.6 | 1074.1 | 748.2 |
| 171 | 35.6 | 1.2 | 1357.1 | 179.9 | 2986.6 | 1.2 | 1399.7 | 39.7 | 3790.7 |
| 172 | 660.5 | 696.5 | 574.5 | 822 | 549.6 | 679.2 | 517.6 | 539 | 873.1 |
| 173 | 172.3 | 120.5 | 182.9 | 172.3 | 441.6 | 91 | 875.5 | 186.4 | 445.7 |
| 176 | 1.1 | 32.1 | 22.8 | 1.1 | 1.1 | 269.9 | 3233.2 | 1.1 | 160.9 |
| 177 | 675.5 | 641.9 | 541.6 | 1175.8 | 196.5 | 1163.3 | 437.1 | 345.5 | 599.4 |
| 178 | 144.2 | 121.8 | 72.4 | 360.4 | 1481.8 | 1025 | 2178.6 | 88.7 | 313.7 |
| 179 | 52.1 | 87.1 | 93.5 | 143.3 | 81.8 | 124.1 | 75.4 | 43.4 | 153.8 |
| 180 | 95.7 | 66.1 | 137.1 | 160.5 | 253.7 | 190.5 | 496.2 | 69.1 | 199.3 |
| 181 | 8377.6 | 9185.5 | 10162.2 | 11258.5 | 1081.7 | 11429.6 | 5621.9 | 12118.4 | 10006.8 |
| 182 | 458.5 | 488.6 | 593.1 | 336.2 | 742.2 | 148.2 | 383.4 | 287.6 | 393.9 |
| 183 | 41.7 | 8.1 | 711.7 | 110.5 | 624.4 | 1.2 | 1507 | 41.7 | 551.1 |
| 184 | 241.8 | 165.4 | 281.2 | 458.9 | 346.3 | 189.5 | 244.1 | 175.4 | 339.3 |
| 185 | 1648.2 | 527.4 | 1463 | 1633.2 | 3854.7 | 591.4 | 1805.1 | 1354.3 | 962 |
| 186 | 852.3 | 370.9 | 554.5 | 405.8 | 293.7 | 714.5 | 452.3 | 164.8 | 319.5 |
| 187 | 81.4 | 76.3 | 149.6 | 122.4 | 192.9 | 83.5 | 4783 | 75.8 | 136.5 |
| 188 | 325.5 | 64.9 | 281 | 153.1 | 548.5 | 72.7 | 239.9 | 148.5 | 260.2 |
| 189 | 179.1 | 36.6 | 199.4 | 99.5 | 226.7 | 74.1 | 111.4 | 100 | 151.7 |
| 190 | 65.2 | 56.7 | 57.5 | 206 | 147 | 170.8 | 473.9 | 107.2 | 158.5 |
| 191 | 1282.4 | 180.6 | 706.5 | 991.9 | 1859.6 | 193.4 | 671.8 | 1181.2 | 884.9 |
| 192 | 133.1 | 59.7 | 97.6 | 101 | 117.7 | 186.9 | 1346.8 | 80.1 | 111.3 |
| 193 | 2817 | 1551 | 1158.4 | 1627.3 | 211 | 677.6 | 728.5 | 2190.6 | 745.3 |
| 194 | 224.1 | 133.7 | 191.1 | 211.3 | 94.6 | 57.1 | 198.5 | 79.7 | 112.4 |
| 195 | 210.1 | 378.3 | 415.7 | 457.7 | 91.8 | 391.3 | 167.4 | 250.9 | 161.2 |
| 196 | 2059.1 | 6068.9 | 8250.3 | 6100.2 | 260.4 | 9272.9 | 4421.5 | 2709.1 | 7446.4 |
| 197 | 116.3 | 74.9 | 72.5 | 69.9 | 1094.7 | 92.5 | 126.6 | 97.4 | 137.7 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 198 | 69.1 | 54.5 | 79.3 | 90.4 | 64.8 | 142.2 | 658.4 | 74.2 | 125.9 |
| 199 | 73.1 | 63 | 674.4 | 113.7 | 3555.6 | 87.8 | 295.6 | 125.9 | 1666.8 |
| 200 | 152.4 | 193.6 | 440 | 136.7 | 1166.9 | 233.9 | 450.3 | 193.7 | 343.6 |
| 201 | 101.2 | 31 | 560.4 | 302.8 | 1214.5 | 17.7 | 90 | 30.4 | 1612.8 |
| 203 | 703.7 | 907.4 | 1270.8 | 514 | 92.1 | 126.9 | 1145.2 | 1552.7 | 207.6 |
| 204 | 1285.1 | 377.7 | 403 | 705.2 | 88.6 | 338.5 | 595.2 | 922.9 | 476.3 |
| 205 | 2463.4 | 3054.5 | 3730.8 | 4980.4 | 361.5 | 572.3 | 3463.4 | 6141.2 | 3833.2 |
| 206 | 187.4 | 167.3 | 168.6 | 267.3 | 1097.9 | 453.7 | 228.3 | 296.2 | 3177.1 |
| 207 | 288.4 | 3037.3 | 2123.7 | 206.1 | 61.4 | 218.8 | 485.6 | 2438.3 | 1258.7 |
| 208 | 190.3 | 97.4 | 336.5 | 323 | 109.9 | 9.2 | 306.2 | 227.8 | 548.3 |
| 209 | 3259.2 | 3551.6 | 5575.6 | 2237.4 | 150.6 | 4727.5 | 1832.2 | 732.2 | 3005.4 |
| 211 | 8683.6 | 22986.2 | 20317.7 | 33405.4 | 565 | 7384.7 | 9662.3 | 29932.5 | 20413.1 |
| 212 | 1572 | 515.7 | 339.7 | 208.5 | 80.1 | 1016.3 | 1086.1 | 339.2 | 299.9 |
| 213 | 94.5 | 90 | 92.1 | 102.5 | 61.4 | 224.9 | 80.9 | 66.5 | 121.6 |
| 215 | 366.7 | 5578.5 | 4327 | 285.6 | 78.2 | 266 | 478.1 | 3071.2 | 1903.3 |
| 216 | 633.5 | 670 | 697.1 | 660.4 | 547 | 267.5 | 566.7 | 785.5 | 412.5 |
| 217 | 235.7 | 147.3 | 281.6 | 384 | 479.9 | 98.7 | 277.7 | 221.7 | 271.5 |
| 218 | 191.6 | 481.1 | 295.1 | 384 | 112.2 | 335.8 | 274 | 687.3 | 319.7 |
| 220 | 74 | 79.4 | 131.9 | 86.9 | 109.2 | 139.9 | 74.3 | 78.7 | 98.2 |
| 221 | 739.7 | 775 | 1236 | 738.2 | 1516.5 | 159.5 | 796.8 | 459.3 | 439.4 |
| 223 | 226.1 | 262.3 | 230.6 | 220.4 | 235.4 | 419.6 | 210.6 | 191.4 | 360.2 |
| 227 | 784.7 | 236.7 | 712.8 | 3864 | 4971.9 | 50.2 | 2112.1 | 483.2 | 1731 |
| 228 | 427.4 | 369.6 | 410.7 | 756.3 | 119 | 453 | 308.8 | 321.2 | 777.9 |
| 229 | 121.4 | 178.2 | 180.7 | 386.6 | 270.9 | 497.6 | 489 | 353.6 | 172.9 |
| 230 | 85 | 63.3 | 105.6 | 210.3 | 1243.7 | 40.9 | 331.4 | 106.7 | 325.5 |
| 231 | 337.9 | 417.5 | 650 | 305 | 129.2 | 163.1 | 486.4 | 499 | 193.8 |
| 232 | 213 | 116.6 | 3960.5 | 604.6 | 1673.4 | 54.4 | 4508.4 | 185.8 | 2113.5 |
| 233 | 2564 | 1822.6 | 6196.9 | 2781.4 | 3667.3 | 1118.9 | 3084 | 6379.7 | 4116.8 |
| 235 | 137.6 | 63.4 | 580.8 | 361.2 | 1679.5 | 92.8 | 833 | 86.2 | 2441.7 |
| 236 | 160.2 | 120 | 182.2 | 264.3 | 417.6 | 254.8 | 288.4 | 125.5 | 310.6 |
| 237 | 2392.5 | 1737.2 | 1778.8 | 1851.2 | 3102.3 | 638.8 | 2558.1 | 2048.7 | 1204.1 |
| 238 | 934.8 | 1575.1 | 1087.9 | 1078.8 | 215.5 | 179.1 | 1369.6 | 2083.3 | 807.8 |
| 239 | 256.3 | 59.9 | 259.2 | 442 | 208.4 | 101.6 | 275.9 | 58.6 | 485 |
| 240 | 316.9 | 27.3 | 330.2 | 381.2 | 683 | 52.8 | 381.6 | 41.4 | 575.1 |
| 241 | 602.7 | 36.7 | 562.8 | 985.9 | 886.1 | 83.4 | 734.2 | 126.7 | 1214 |
| 242 | 2714.7 | 325 | 2278 | 12129 | 22000.8 | 874.8 | 2544.9 | 845.1 | 6760 |
| 243 | 477.1 | 168.4 | 373 | 453.4 | 7356.1 | 182.8 | 441.2 | 223 | 259.3 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 244 | 137.2 | 198.3 | 172.5 | 137.8 | 308.4 | 111.1 | 215.7 | 213.9 | 147.9 |
| 246 | 262 | 306.4 | 301.9 | 426.2 | 407.7 | 346.6 | 609.8 | 874.2 | 145.9 |
| 247 | 117.5 | 142.6 | 77 | 156.9 | 119.1 | 289.1 | 120.2 | 120.9 | 213.4 |
| 248 | 216.4 | 124.1 | 74.2 | 115.8 | 92.4 | 107.5 | 317.2 | 126.7 | 106.4 |
| 249 | 180 | 341.2 | 320.1 | 176.7 | 185.5 | 885.1 | 399.6 | 131.7 | 791.8 |
| 251 | 63.8 | 107.9 | 106.3 | 84.5 | 742.7 | 44.5 | 117.5 | 94.5 | 111.7 |
| 253 | 190.7 | 244.8 | 204.4 | 375.3 | 374.5 | 218.4 | 376.5 | 486.5 | 269.6 |
| 254 | 145.4 | 1957.1 | 1159.5 | 243.2 | 123.7 | 228.2 | 1365 | 142.3 | 312.9 |
| 255 | 170.9 | 82.9 | 51.9 | 379.1 | 1787.5 | 1107.5 | 2481.4 | 91.2 | 318.4 |
| 256 | 517.8 | 132.4 | 995.4 | 353.1 | 1340.6 | 331.8 | 499.8 | 141.8 | 3605.8 |
| 257 | 124.3 | 174.8 | 165.9 | 97.2 | 172.3 | 54 | 209.3 | 115.4 | 173.8 |
| 258 | 24 | 1.2 | 253.4 | 53.4 | 345.6 | 104.4 | 131.7 | 3.9 | 456.5 |
| 259 | 692.2 | 65.2 | 512.5 | 236.2 | 422.2 | 1.2 | 669.1 | 79.7 | 638.4 |
| 260 | 153.5 | 168.4 | 123.3 | 295.4 | 151.6 | 561.5 | 224.9 | 163.9 | 266.2 |
| 261 | 197.8 | 221.1 | 313.1 | 301.5 | 77.8 | 119.5 | 496.8 | 1094.7 | 301.6 |
| 263 | 698.9 | 553.8 | 407.2 | 370.6 | 1231.9 | 141.3 | 387.3 | 692.4 | 333.4 |
| 263 | 135.5 | 78.4 | 109.7 | 225.9 | 1211 | 201.2 | 434.5 | 96.2 | 404.4 |
| 265 | 362.9 | 618.8 | 519.4 | 342.3 | 102 | 258.8 | 178.2 | 477.2 | 588.4 |
| 266 | 246.8 | 201.9 | 307.4 | 607.3 | 257.6 | 820.3 | 300.7 | 537.1 | 367 |
| 256 | 115.8 | 100.6 | 138.3 | 134.8 | 325.1 | 134.2 | 161.2 | 75.9 | 165.2 |
| 268 | 209.2 | 366.9 | 653.7 | 922.5 | 10 | 480.6 | 333.1 | 299.5 | 775.9 |
| 269 | 40.7 | 48.5 | 42.3 | 72.2 | 126 | 90.5 | 30 | 60.5 | 91.4 |
| 270 | 466.8 | 148.2 | 169.3 | 215.5 | 180.7 | 290.2 | 237 | 153.1 | 262.6 |
| 271 | 340.4 | 250.6 | 292 | 363.1 | 191.9 | 147.4 | 231.1 | 338.8 | 278.4 |
| 272 | 419.7 | 600.7 | 550.3 | 549.6 | 590.7 | 925.3 | 506 | 506.9 | 675.1 |
| 273 | 1329.6 | 4064.8 | 5723.5 | 4200.8 | 53.9 | 141.9 | 2361.5 | 2682.8 | 4782.9 |
| 274 | 572.9 | 159.6 | 459.6 | 777.2 | 2112.5 | 590 | 1292.6 | 264.9 | 670.4 |
| 275 | 400.8 | 151.3 | 339.1 | 398 | 1769 | 221.5 | 415.8 | 329.2 | 264.8 |
| 276 | 1260.7 | 304 | 1311.2 | 5788.9 | 9405.7 | 142.2 | 3231.8 | 599.6 | 2504.9 |
| 277 | 175.4 | 164.3 | 192.2 | 231.9 | 150.5 | 254.7 | 152.2 | 197.2 | 281.6 |
| 278 | 1103 | 87.3 | 544.7 | 209.6 | 729.1 | 38.2 | 801.4 | 62.2 | 875.4 |
| 279 | 1853 | 3010.3 | 1676.6 | 3236.4 | 131.7 | 425.7 | 2072.3 | 2745.7 | 1987 |
| 280 | 2108.7 | 4444.5 | 2655.3 | 3526 | 338.9 | 391.3 | 2886.5 | 4540.8 | 2685.8 |
| 281 | 18.4 | 25 | 53.6 | 118.3 | 67.5 | 103.2 | 129.1 | 36 | 49.1 |
| 282 | 208.4 | 189.9 | 229.3 | 225.9 | 218 | 274.6 | 281.5 | 269.8 | 574.2 |
| 283 | 226.2 | 239 | 236.5 | 269.1 | 179.9 | 647.8 | 210.9 | 202.5 | 338.1 |
| 284 | 76.5 | 83 | 85.5 | 84.8 | 61.1 | 154.1 | 83.4 | 51.9 | 87.9 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 285 | 83.4 | 81.8 | 98.2 | 109.9 | 70.2 | 153.3 | 87 | 104.4 | 126.6 |
| 286 | 2043.9 | 638.5 | 1891.2 | 121.4 | 602.8 | 343.8 | 417.5 | 688.5 | 824.4 |
| 287 | 102.2 | 122.8 | 119.2 | 106.4 | 113.9 | 133.5 | 95.5 | 144.2 | 149.6 |
| 288 | 142 | 147.4 | 138.8 | 152.9 | 154.3 | 269 | 139.9 | 140.5 | 203.7 |
| 289 | 673.8 | 1.2 | 569.8 | 199.4 | 1260.8 | 269.4 | 831.4 | 65.9 | 2672.1 |
| 291 | 209.6 | 44 | 112.8 | 181.3 | 341.7 | 44 | 78.9 | 99.5 | 126.6 |
| 292 | 62.9 | 40.3 | 181.8 | 49.2 | 103.4 | 47.8 | 786.4 | 36.9 | 412.2 |
| 293 | 489.3 | 121.1 | 95.1 | 72.6 | 52.1 | 99.7 | 409.7 | 50.8 | 113 |
| 295 | 527.5 | 128.5 | 168 | 466.6 | 768.9 | 354.3 | 354.7 | 222.9 | 256.1 |
| 296 | 773.6 | 924.1 | 1329.9 | 843.8 | 1063 | 956.8 | 1070.2 | 873.2 | 688.9 |
| 297 | 52 | 51.9 | 85.2 | 60.1 | 2284.1 | 95.4 | 70.8 | 52.2 | 96.9 |
| 298 | 295.2 | 198.2 | 300.3 | 295.1 | 502.3 | 161.6 | 292.8 | 372.6 | 290 |
| 299 | 1170.6 | 1365.1 | 561.9 | 926.9 | 160.3 | 431.4 | 1139.9 | 604 | 851.1 |
| 300 | 79.1 | 638.7 | 458 | 158.4 | 272.1 | 104.6 | 617.9 | 383.6 | 205.3 |
| 301 | 1366.1 | 435.2 | 1418.5 | 798.3 | 317.5 | 423.3 | 664.2 | 750.6 | 2119.8 |
| 302 | 14.8 | 21.6 | 21.1 | 13.6 | 121.5 | 39.5 | 24.7 | 9.5 | 34.9 |
| 303 | 1272.6 | 1149.6 | 761.8 | 834.5 | 159.9 | 477.7 | 449.7 | 1072.6 | 432.1 |
| 304 | 1151.3 | 128.6 | 230.5 | 335.2 | 454.3 | 186.6 | 1221.7 | 235 | 379.7 |
| 305 | 151.1 | 238.9 | 140.3 | 950.2 | 110.9 | 1077 | 1043.9 | 166.7 | 211.5 |
| 306 | 136.4 | 144.5 | 117.6 | 155.2 | 192.6 | 291.2 | 118.6 | 150.5 | 228.4 |
| 307 | 132.8 | 177.7 | 207.1 | 196.3 | 120 | 249.1 | 112.4 | 122.8 | 281.5 |
| 308 | 2569.4 | 1758.7 | 2241.2 | 2859.3 | 230.6 | 2770.2 | 677.2 | 2894.9 | 1090.1 |
| 310 | 980 | 334 | 456.4 | 390 | 114.5 | 278.1 | 997.8 | 654.8 | 472.6 |
| 311 | 142.6 | 73.1 | 84.3 | 121.4 | 78.4 | 146.9 | 148.3 | 200.6 | 115.7 |
| 312 | 2560.4 | 57.2 | 426.8 | 89.2 | 18.4 | 574.8 | 506.6 | 402.8 | 956.5 |
| 313 | 209.5 | 119.1 | 121.2 | 222.7 | 91.7 | 201.4 | 478.6 | 92.9 | 117.2 |
| 314 | 158.8 | 162.7 | 249.8 | 188.5 | 309.4 | 89.2 | 547.1 | 122.9 | 576.3 |
| 315 | 702.8 | 557.9 | 900 | 546.6 | 104 | 247.6 | 529.5 | 657.3 | 404.1 |
| 316 | 250.6 | 332.6 | 259.8 | 311.9 | 69.6 | 518.6 | 460.2 | 314.7 | 248.1 |
| 317 | 150.5 | 169.2 | 177 | 177.5 | 133.8 | 233 | 171.7 | 173.6 | 261.6 |
| 318 | 40.7 | 117.2 | 213.8 | 44.5 | 47.3 | 18.6 | 57.8 | 59.8 | 9.1 |
| 319 | 309.7 | 173 | 162.4 | 217 | 97.8 | 1038.4 | 828.1 | 117.5 | 167.2 |
| 320 | 822.5 | 511 | 664.1 | 815.9 | 185 | 371.3 | 845 | 592.1 | 437.2 |
| 321 | 629.1 | 258.6 | 321.1 | 511.8 | 309.4 | 452.1 | 355 | 573.3 | 321.3 |
| 322 | 195.9 | 274 | 200.6 | 228.2 | 502.2 | 565.2 | 318 | 241 | 250.5 |
| 323 | 5483.1 | 3828.6 | 3464.5 | 1733.4 | 233.8 | 941.9 | 1329.5 | 2376 | 2289.1 |
| 324 | 224.9 | 61 | 44.8 | 85.4 | 32 | 56.6 | 246 | 103.9 | 70.2 |

(continued)

| 3b | | | | | | | | | |
|---|---|---|---|---|---|---|---|---|---|
| SEQ ID NO: | 820TT | 821TT | 822TT | 823TT | 824TT | 825TT | 827TT | 828TT | 829TT |
| 325 | 342.8 | 379 | 339.3 | 282.9 | 230.2 | 339.8 | 313.3 | 331.3 | 331 |
| 326 | 2895.3 | 6732.3 | 6959.5 | 716.8 | 385.8 | 689.6 | 1354.3 | 3462.4 | 810.2 |
| 327 | 14 | 26.8 | 9 | 107.3 | 745.3 | 31.8 | 92.3 | 30.5 | 40.5 |
| 328 | 281.4 | 393 | 463 | 538.5 | 44.8 | 160.9 | 160.6 | 240 | 510.8 |
| 329 | 83.3 | 80 | 72.1 | 51.7 | 315.4 | 197.3 | 209.5 | 65.5 | 97.5 |
| 330 | 51.4 | 40.9 | 62.5 | 38.8 | 64.6 | 86 | 49.8 | 45.3 | 75.4 |
| 331 | 310.9 | 265.9 | 292.9 | 290.5 | 301.2 | 1151 | 377.7 | 262.6 | 363.2 |
| 333 | 770.8 | 635.6 | 1012.3 | 910.6 | 190.7 | 762.8 | 346 | 908.1 | 711.7 |
| 334 | 207.8 | 219.4 | 166 | 215.4 | 137.5 | 112.8 | 565.2 | 135.4 | 170 |
| 335 | 155.6 | 80.9 | 114.5 | 131.9 | 90.7 | 718.1 | 215.7 | 922.8 | 155.9 |
| 336 | 340.7 | 427.2 | 563.8 | 689.1 | 96.3 | 436.7 | 487.1 | 782.5 | 499 |
| 337 | 2849 | 76.8 | 200.7 | 463 | 932.4 | 112.2 | 638.2 | 474.2 | 194.5 |
| 338 | 177.2 | 183.9 | 190.4 | 175.1 | 273.4 | 59.6 | 256.4 | 142.1 | 229.6 |
| 339 | 395.4 | 525.8 | 437.1 | 581 | 225 | 445.4 | 326.7 | 362.3 | 485.5 |
| 340 | 223.5 | 251.1 | 290.8 | 318.2 | 197.1 | 253.7 | 187.5 | 285.3 | 397.3 |
| 341 | 473 | 874 | 1273.4 | 721.4 | 508.7 | 1207.3 | 566.8 | 660.8 | 584.1 |
| 343 | 469.4 | 161.5 | 78.2 | 124.9 | 366 | 1807.9 | 1281 | 53.8 | 117.8 |
| 344 | 1783.5 | 1064.1 | 1156.4 | 1751.4 | 281.3 | 986.5 | 1814.7 | 1291.3 | 1259.5 |
| 345 | 244.8 | 232.7 | 258.2 | 265.4 | 279 | 383.5 | 225.4 | 192.1 | 278.1 |
| 347 | 939.3 | 1308.9 | 2012 | 2968.2 | 324.4 | 1367.9 | 2442.7 | 1031.3 | 2634.6 |
| 348 | 1619 | 2746.3 | 3836 | 4352.3 | 628.4 | 2022.4 | 5951.2 | 4142.1 | 3535.5 |
| 349 | 208.6 | 294.7 | 249.4 | 334.4 | 413.9 | 154.9 | 222.4 | 262.8 | 343 |
| 350 | 265 | 151.9 | 221.4 | 354.3 | 1016.4 | 1.2 | 367.5 | 179 | 283.6 |
| 351 | 121.2 | 184 | 512.2 | 66.7 | 230.2 | 220.7 | 229.7 | 69.7 | 320.1 |
| 352 | 468.7 | 405.1 | 432.1 | 461.6 | 124.7 | 253.5 | 262.2 | 439 | 377.6 |
| 353 | 422.8 | 154.1 | 375.8 | 272.6 | 206.3 | 272.5 | 306.9 | 342 | 275.3 |

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 354 | 1607.7 | 1269.4 | 69.2 | 410.9 | 2189.2 | 23.1 | 352.6 | 432.9 | 560.6 |
| 355 | 1226.2 | 9155.4 | 818.2 | 3259.7 | 4192.9 | 60.2 | 6955.8 | 2712.6 | 387 |
| 356 | 589.5 | 420.2 | 584 | 481.7 | 485.2 | 628.1 | 781.8 | 356.3 | 432.6 |
| 357 | 639.1 | 496.4 | 606.3 | 873.1 | 453.3 | 762.3 | 556.9 | 747.8 | 483.8 |
| 358 | 1141.1 | 1537.2 | 2093.3 | 1332.4 | 1450.6 | 785 | 1727.4 | 1490.9 | 867.6 |
| 359 | 410.8 | 324.8 | 302.5 | 275.6 | 347.4 | 372 | 401.6 | 274.5 | 352.8 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 362 | 433.7 | 1188.9 | 809.4 | 714.8 | 722 | 536.8 | 1286.6 | 790.9 | 408.6 |
| 360 | 355.3 | 785.8 | 548.6 | 473.7 | 527.2 | 237.8 | 648 | 589.2 | 258.9 |
| 361 | 669.1 | 117.2 | 502.4 | 332.7 | 153.1 | 491.8 | 673.8 | 155.9 | 634.6 |
| 363 | 624.8 | 516.7 | 452.9 | 386.1 | 575.4 | 348.2 | 555.6 | 423.3 | 578.6 |
| 1 | 681.6 | 1339.4 | 926.7 | 1627.5 | 1378.3 | 825.9 | 1180.5 | 1556.8 | 483 |
| 2 | 2308.2 | 2517.8 | 2273.1 | 5347 | 1263.4 | 2813.7 | 1449.6 | 2931.3 | 2339.6 |
| 7 | 760.1 | 396.2 | 691.3 | 1209.2 | 602.6 | 682.5 | 797.8 | 924.6 | 504.6 |
| 8 | 1540.3 | 559.2 | 932.1 | 520.1 | 496.7 | 1011.1 | 563.4 | 319.8 | 942.6 |
| 9 | 2980.9 | 1665.3 | 5198.9 | 2703.9 | 1212.7 | 4689.7 | 7367.2 | 4021.7 | 2722.5 |
| 10 | 122.4 | 197.5 | 65.2 | 110.5 | 139.5 | 59.4 | 99.3 | 137.7 | 236.6 |
| 11 | 376.8 | 703.1 | 345.6 | 651.8 | 578.7 | 208.4 | 539.6 | 862.8 | 174 |
| 13 | 181.2 | 792.6 | 515 | 259.6 | 570.1 | 291.6 | 711.6 | 596.7 | 310.5 |
| 14 | 618.3 | 2196.3 | 1802 | 1423.9 | 1281.3 | 1502.7 | 2410.1 | 2087.7 | 533.5 |
| 15 | 807.1 | 1589.6 | 1001.5 | 828.3 | 1241.5 | 307.4 | 2913.4 | 4498.6 | 625.1 |
| 16 | 1484.5 | 3932 | 1622.5 | 2238.6 | 3413.6 | 1419.6 | 2274.4 | 2625.1 | 831.6 |
| 17 | 699.7 | 876.3 | 1400.3 | 1300.9 | 988.9 | 1281 | 1049.5 | 2078.4 | 694.1 |
| 19 | 482.6 | 465.6 | 2749.2 | 461.1 | 810 | 290.3 | 1716.7 | 1582.7 | 1076.2 |
| 20 | 84 | 450.8 | 263.1 | 290.5 | 360 | 1.1 | 182.4 | 324.9 | 165.7 |
| 21 | 91.3 | 3467.2 | 514.9 | 1814.1 | 3225.9 | 499.2 | 2094.7 | 1840.3 | 181.3 |
| 22 | 3156.1 | 4671.9 | 5203.4 | 6611.6 | 3435 | 2468.1 | 9279.3 | 6745.6 | 4447.3 |
| 23 | 495.3 | 1234.6 | 1083.4 | 798.1 | 1519.6 | 356.2 | 1191.8 | 2950.2 | 871.6 |
| 24 | 274.4 | 317.3 | 405.7 | 185.5 | 432.2 | 235.1 | 795.2 | 871.3 | 137.7 |
| 26 | 2752.2 | 4302.1 | 8189.5 | 1864.3 | 3917.4 | 2490.7 | 6974 | 5535.2 | 4706 |
| 27 | 424.6 | 749.9 | 1258.8 | 1611.9 | 871.3 | 505 | 1476.7 | 1146.7 | 506.1 |
| 29 | 165.7 | 162.7 | 75.4 | 90.5 | 104.2 | 94.3 | 114.4 | 146.2 | 101 |
| 30 | 312.4 | 592.5 | 409.5 | 348 | 418.1 | 136.7 | 1284.6 | 1307.6 | 408.1 |
| 31 | 331.7 | 12536.5 | 1834 | 9042.7 | 16967 | 303.8 | 5146.5 | 6843.3 | 352.9 |
| 33 | 1256.2 | 4507.9 | 1474.3 | 2328.6 | 4981.8 | 451.7 | 4542.5 | 11875.5 | 544.7 |
| 34 | 2831.9 | 492 | 3213.2 | 870 | 569.7 | 1873.2 | 2278.6 | 454.6 | 2785.1 |
| 35 | 1217 | 1751.1 | 260.4 | 933.6 | 1418.4 | 120.2 | 423.2 | 768.6 | 680.3 |
| 36 | 145.6 | 258.1 | 974.8 | 3626.3 | 913.3 | 524.5 | 893.8 | 1391.4 | 185.2 |
| 37 | 286.6 | 268.9 | 312.8 | 203.1 | 264.8 | 203 | 424.3 | 264.8 | 270.4 |
| 38 | 709.7 | 3086.6 | 1199.9 | 882.2 | 1931.9 | 384.4 | 1810.3 | 7524 | 348.4 |
| 40 | 603.3 | 849.4 | 466.7 | 631.9 | 695.6 | 357.4 | 654.4 | 633.1 | 633.2 |
| 41 | 285.5 | 513.6 | 628.4 | 721.9 | 2482 | 1004.7 | 621.2 | 2126 | 360.3 |
| 42 | 229.9 | 548.9 | 402.2 | 516.1 | 450.5 | 519.8 | 332.6 | 612.9 | 222.9 |
| 43 | 390.1 | 489.1 | 363.3 | 192.2 | 399.6 | 388.1 | 369 | 222.3 | 314.1 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 44 | 652.8 | 952.5 | 925.3 | 1044.2 | 930.7 | 784.2 | 962.5 | 1223.4 | 468 |
| 45 | 691.2 | 1619.8 | 791.5 | 1433.2 | 1616.2 | 618.5 | 1030.5 | 1531.9 | 335.2 |
| 46 | 351.6 | 412.4 | 339 | 330.5 | 453.3 | 276.5 | 345.7 | 346.9 | 376.6 |
| 47 | 256.3 | 423.2 | 349.8 | 448.9 | 546.2 | 138.6 | 305.9 | 635.7 | 188.2 |
| 48 | 297.1 | 112.1 | 708.1 | 397.5 | 367.9 | 134.4 | 1885.5 | 680.1 | 1204.7 |
| 49 | 497.2 | 2981.6 | 1179.9 | 712 | 5248.7 | 553.9 | 1047.2 | 640.1 | 381.8 |
| 50 | 350.3 | 659.2 | 627.4 | 427.2 | 665.1 | 230.3 | 1754.6 | 2654 | 228.2 |
| 51 | 80.2 | 1018.5 | 175.1 | 392.4 | 669.4 | 374.5 | 326.8 | 448.4 | 77.7 |
| 52 | 219.7 | 261.6 | 189.5 | 266.9 | 275.7 | 214.6 | 245.4 | 224.4 | 208.5 |
| 53 | 97.5 | 1385.3 | 1668.8 | 2690.6 | 1207.6 | 509 | 1096 | 2281.1 | 132.5 |
| 54 | 401.9 | 1101.6 | 1148.7 | 1527.2 | 880.3 | 1550.3 | 682.6 | 1648.3 | 318.8 |
| 55 | 87.2 | 79.5 | 120.6 | 81 | 64 | 126 | 101.5 | 117.8 | 130.8 |
| 56 | 41.5 | 356.3 | 290.8 | 220 | 321.6 | 255.3 | 116.9 | 178.9 | 114.6 |
| 57 | 4709.8 | 826 | 3670.5 | 13995.1 | 2735.5 | 1208.7 | 2495.3 | 8987.2 | 1600.1 |
| 58 | 5493.8 | 942.8 | 3824 | 13347.2 | 3431.8 | 1473.6 | 2604.6 | 9793.3 | 1896.9 |
| 59 | 990.8 | 4570.9 | 2467.2 | 2820.8 | 3584.1 | 1578.8 | 2600.2 | 2511.9 | 1043.9 |
| 60 | 86.6 | 409 | 328.3 | 697.7 | 313.2 | 195.4 | 394 | 769.5 | 205.8 |
| 61 | 148.6 | 102.4 | 113.5 | 119.1 | 96.3 | 180.5 | 151.5 | 144.5 | 90.7 |
| 62 | 2552.4 | 3419 | 2087.8 | 1947.4 | 3982.4 | 632.7 | 4686.3 | 1552 | 893.8 |
| 63 | 138.1 | 500.7 | 504.9 | 621.5 | 477.8 | 416.2 | 765.6 | 623.8 | 200.9 |
| 64 | 194.3 | 430.2 | 1152 | 334.2 | 1238.6 | 157.1 | 225.5 | 301.4 | 184.8 |
| 65 | 119.3 | 275.4 | 393.9 | 220.9 | 330.6 | 146.6 | 362.6 | 472.9 | 192.6 |
| 66 | 644.5 | 1716.5 | 799.8 | 2089.9 | 1882.3 | 860.9 | 884.9 | 1790.8 | 494.1 |
| 67 | 33664.7 | 18698 | 25385.3 | 29495.1 | 22746.1 | 32326.6 | 35830.4 | 25673.3 | 30440.8 |
| 68 | 4863.4 | 757.4 | 639.2 | 500.3 | 628.5 | 1067 | 877.5 | 537.2 | 2729.1 |
| 69 | 7538.6 | 1746.3 | 2633.5 | 2161.4 | 1446.6 | 3285.4 | 3619.9 | 2224.2 | 3873.7 |
| 70 | 1167.7 | 4005.5 | 2775 | 4480.9 | 5935.2 | 2888.4 | 2408.7 | 3524.2 | 1112.2 |
| 71 | 331.3 | 7296.2 | 3308.3 | 4722.7 | 6620.9 | 641.7 | 3323 | 7752.1 | 521.9 |
| 72 | 867.2 | 2722 | 587.7 | 1727.1 | 2480.6 | 715.1 | 1071.1 | 2441.2 | 729.3 |
| 73 | 248.7 | 702.4 | 513.9 | 998.3 | 674.5 | 351.8 | 560.2 | 646.8 | 291.3 |
| 75 | 191.9 | 299.8 | 1021.2 | 2627.9 | 481 | 184.1 | 684.5 | 3040.4 | 241.4 |
| 76 | 602.9 | 419.6 | 424.1 | 687.6 | 576.5 | 1929.4 | 374.1 | 1713.5 | 249.7 |
| 77 | 3969.7 | 1046.9 | 2803 | 1502.3 | 1123.7 | 3525.2 | 3967.9 | 610.4 | 3028 |
| 78 | 461 | 260.7 | 681.4 | 434.3 | 255.4 | 719 | 658.2 | 440.9 | 425.5 |
| 79 | 112.1 | 359.7 | 118.8 | 269.2 | 354.3 | 241.7 | 238 | 391.9 | 129.7 |
| 80 | 211.4 | 800.8 | 565.5 | 1265 | 877.2 | 479.3 | 445.8 | 1163.2 | 181.6 |
| 81 | 381.4 | 8963.9 | 1038.9 | 7150.5 | 8082.3 | 289.1 | 2966.6 | 5268.9 | 339.8 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 82 | 204.4 | 189.8 | 246.1 | 203.2 | 216.8 | 260.9 | 253.2 | 152.4 | 345.8 |
| 84 | 167 | 297.3 | 276.3 | 381.1 | 417.7 | 274.7 | 303.1 | 497.5 | 238.9 |
| 85 | 186.4 | 1174.1 | 201.5 | 1066.1 | 1674.8 | 143.6 | 434.1 | 306 | 203.8 |
| 86 | 75.7 | 748.2 | 119 | 174.2 | 654.4 | 84.9 | 219.2 | 164.9 | 102.9 |
| 87 | 252.7 | 2338.9 | 668.2 | 445 | 1007.4 | 249.1 | 1199.6 | 589.5 | 215 |
| 88 | 6167.9 | 470.3 | 4262.6 | 1023.8 | 548.1 | 5577.9 | 4604.6 | 683.8 | 3916.7 |
| 89 | 267 | 506.7 | 193 | 233.1 | 541.1 | 192.3 | 266.6 | 262.9 | 321.8 |
| 90 | 190.7 | 110.5 | 214.3 | 244.9 | 280 | 124.7 | 260 | 290 | 113.5 |
| 91 | 7102.8 | 1126.2 | 1543.8 | 6389.7 | 2050.8 | 529.2 | 760.6 | 7087.2 | 1108.2 |
| 92 | 1861.9 | 9980.6 | 9744 | 7280.9 | 5486.5 | 7913.2 | 7006.7 | 5412 | 2498.8 |
| 93 | 163.2 | 108.9 | 245.5 | 157.5 | 112.4 | 254.2 | 250.4 | 119 | 193 |
| 94 | 839.4 | 577 | 1488.5 | 1006.1 | 405.5 | 1082.5 | 1126.2 | 964.8 | 634.9 |
| 95 | 515.4 | 1234.6 | 807.9 | 454 | 959.8 | 864.4 | 770.5 | 494.4 | 662.2 |
| 96 | 144.9 | 212.1 | 199 | 351.5 | 306.3 | 223.8 | 227.7 | 302.5 | 175 |
| 97 | 130.8 | 471.3 | 333.4 | 484 | 448.6 | 329.5 | 480.1 | 568.4 | 147.1 |
| 98 | 15.8 | 32.4 | 37.6 | 34.4 | 51.3 | 39 | 38.4 | 65.9 | 38.2 |
| 99 | 318.3 | 435.4 | 289.7 | 489.3 | 399.5 | 198.2 | 351.7 | 473.1 | 298 |
| 100 | 12916 | 51.7 | 13251.4 | 1990.7 | 423.3 | 4429.8 | 13164.2 | 230 | 5552.1 |
| 101 | 532.8 | 290.8 | 329.4 | 342.8 | 316.8 | 363.7 | 300.1 | 234.7 | 333.5 |
| 102 | 229.2 | 186.8 | 199.6 | 138.3 | 206.2 | 149.6 | 226.5 | 175.3 | 202.1 |
| 103 | 342.5 | 406.4 | 434 | 355.9 | 371.1 | 306.7 | 526.4 | 435.6 | 362.1 |
| 104 | 596.1 | 90.6 | 698.7 | 267.8 | 65.4 | 947.3 | 366 | 148.9 | 436.1 |
| 105 | 533.9 | 86.9 | 770.1 | 333.9 | 108.7 | 831.3 | 274.7 | 239.4 | 443.9 |
| 106 | 573.6 | 70.7 | 537.2 | 270.6 | 112.2 | 911.2 | 341.9 | 194.3 | 486.5 |
| 107 | 1027.4 | 294.7 | 183.4 | 222.5 | 716.1 | 250.8 | 263.1 | 280.6 | 335.9 |
| 108 | 241.6 | 145 | 283.6 | 211.1 | 169.4 | 333 | 276.2 | 235.5 | 308.9 |
| 109 | 1066.5 | 2807.4 | 2388.4 | 1135.5 | 1428.2 | 2931.4 | 1315.4 | 2683.7 | 95.3 |
| 110 | 2960.7 | 7199 | 6101.1 | 2633.5 | 3373.8 | 5614 | 5706.1 | 6310.8 | 130.7 |
| 111 | 2125.4 | 189.5 | 742.5 | 359.1 | 253 | 1180.3 | 925.2 | 140.9 | 1262.1 |
| 112 | 523.3 | 2050.6 | 1308.3 | 1693.4 | 1084.8 | 1386.3 | 718.3 | 1809.1 | 394.9 |
| 113 | 74.8 | 326 | 591.7 | 308.2 | 365.7 | 404.6 | 253 | 437.5 | 190.6 |
| 114 | 494.6 | 580.3 | 510.6 | 830.3 | 436.6 | 489.3 | 361.3 | 460.5 | 588.4 |
| 115 | 501.5 | 966.3 | 740.2 | 1215.5 | 1149 | 363.5 | 1225 | 1118.5 | 466.4 |
| 116 | 279 | 621.8 | 666.9 | 515.9 | 520.1 | 199.1 | 1091 | 891.9 | 310.3 |
| 117 | 1708.9 | 122.3 | 1731.3 | 467 | 162.6 | 817.7 | 1395.5 | 183.9 | 1888.5 |
| 118 | 136.1 | 129.6 | 287.9 | 133.8 | 97 | 154.8 | 231.6 | 230.9 | 391.1 |
| 119 | 250.9 | 375.2 | 298.4 | 289.2 | 272.3 | 298.3 | 279.5 | 171.8 | 367.9 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 120 | 135 | 98.5 | 266 | 117.9 | 142.2 | 119.8 | 265.8 | 210.2 | 170.8 |
| 121 | 925.3 | 1636.7 | 2341 | 5981.4 | 2305 | 1239.6 | 3217.7 | 4469.4 | 424.6 |
| 122 | 146.6 | 403.5 | 321.9 | 294.5 | 387.4 | 169.6 | 475.8 | 415.8 | 99.5 |
| 123 | 185.4 | 288.7 | 188.9 | 285.9 | 269.3 | 240.8 | 291.1 | 457.9 | 164 |
| 124 | 178.1 | 141 | 152.7 | 97.1 | 156.7 | 104.4 | 167.4 | 144.7 | 176.4 |
| 125 | 1161.1 | 1141.8 | 2587.8 | 5502.6 | 1399.3 | 689 | 1600.7 | 2696.8 | 1040.4 |
| 126 | 656.8 | 38.1 | 771.3 | 130.6 | 17.2 | 1187.1 | 477 | 64 | 905 |
| 127 | 102.9 | 244.7 | 132 | 189 | 183 | 113.8 | 209.9 | 130.6 | 133 |
| 128 | 387 | 69514.5 | 255.7 | 68429 | 281 | 269 | 317.9 | 214941 | 338.3 |
| 129 | 357 | 1983.4 | 2117 | 559.5 | 1969.4 | 382.4 | 899 | 1412 | 221.4 |
| 130 | 80.2 | 488.5 | 349.7 | 368.2 | 469.6 | 255.7 | 169.9 | 378.5 | 109 |
| 131 | 97.6 | 577.1 | 413.3 | 535.1 | 592.4 | 154 | 308.1 | 504.5 | 115.2 |
| 132 | 259 | 267.5 | 225.2 | 362.6 | 285.3 | 231.6 | 294.2 | 316.4 | 233.1 |
| 133 | 62 | 284.1 | 126.4 | 152.8 | 224.7 | 170.9 | 160.7 | 281.1 | 70.9 |
| 134 | 129.3 | 363.2 | 256.8 | 186.3 | 308.2 | 228.3 | 147.2 | 117.2 | 137.1 |
| 135 | 398.3 | 284.6 | 523.7 | 620.2 | 375.8 | 623.2 | 554.6 | 638.2 | 372.3 |
| 138 | 150.3 | 205.6 | 166.6 | 165.6 | 244.7 | 180.3 | 238.1 | 192.4 | 141.7 |
| 139 | 87.2 | 82.8 | 324.7 | 238 | 168 | 198.4 | 204.8 | 178.3 | 147.6 |
| 140 | 2117.8 | 107.3 | 1157 | 1151.6 | 68.3 | 2112.4 | 1062.3 | 199.7 | 2021.8 |
| 141 | 1371.7 | 677 | 2205.3 | 517.1 | 203.1 | 1460.2 | 1313.5 | 110 | 1344.6 |
| 144 | 487.8 | 6712.6 | 9651.1 | 13878.1 | 3393.4 | 9082.5 | 5985.5 | 22188.6 | 513.2 |
| 145 | 262 | 429.3 | 287.6 | 252.2 | 268.9 | 217 | 336.2 | 203.2 | 226.4 |
| 146 | 194.8 | 463.3 | 178.9 | 314.7 | 540.9 | 163.7 | 240.6 | 520.1 | 224.1 |
| 147 | 59.7 | 56.9 | 119.9 | 366.3 | 152 | 50.5 | 148.4 | 263.9 | 103.4 |
| 148 | 821.7 | 868.3 | 548.5 | 469.3 | 1125.9 | 334.8 | 1013.8 | 676.8 | 741 |
| 149 | 242.9 | 174.5 | 271.1 | 252.6 | 219.5 | 363.8 | 434.4 | 169 | 218.3 |
| 150 | 183.1 | 227.8 | 233.6 | 259.9 | 424.1 | 141.2 | 359.8 | 423 | 249.3 |
| 151 | 211.4 | 1131.6 | 570 | 576.6 | 580.9 | 369.8 | 475.6 | 262.8 | 327.6 |
| 153 | 81.4 | 122.6 | 274.3 | 196.9 | 115.5 | 453.6 | 156.4 | 725.7 | 123.9 |
| 154 | 687.5 | 4823.4 | 1405.3 | 5742.3 | 4211.6 | 942.8 | 2873.5 | 9112.2 | 480.4 |
| 155 | 139.2 | 1013.1 | 289.7 | 320.7 | 766.9 | 289.3 | 405.9 | 264.7 | 116.9 |
| 156 | 77.8 | 638.3 | 346.5 | 465.6 | 740.7 | 128.6 | 262.3 | 511.3 | 127.6 |
| 157 | 210.4 | 137.4 | 176.3 | 144 | 198 | 97.2 | 246 | 151.5 | 150.2 |
| 158 | 14.7 | 1191.6 | 243.3 | 631.9 | 852.7 | 303.1 | 733.1 | 924.1 | 12.1 |
| 159 | 3335.3 | 1313.5 | 2648.7 | 1400.3 | 1293.6 | 2577.5 | 2106.8 | 1174.1 | 3077.4 |
| 161 | 58.4 | 6972 | 2447.5 | 5870.8 | 5806.5 | 3314.6 | 521.9 | 4390.4 | 98.3 |
| 162 | 145 | 582 | 315.4 | 818.2 | 813.3 | 91.8 | 982.1 | 2165.6 | 316.3 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 164 | 75.4 | 1096.7 | 5139.7 | 1786.4 | 674.5 | 836.8 | 3086.4 | 577.7 | 211.6 |
| 166 | 5882.4 | 1758.7 | 3139.9 | 1274 | 662.7 | 3213.1 | 4469.7 | 1150.4 | 3810.8 |
| 167 | 2593.9 | 1006.1 | 2947.1 | 987 | 1134.4 | 2040.2 | 2587.3 | 1035.8 | 2569 |
| 168 | 1092.3 | 472.4 | 620.1 | 812.6 | 879.6 | 719.1 | 508.4 | 613 | 801.1 |
| 169 | 16935.6 | 11084.6 | 7798.3 | 11788.6 | 6525.4 | 4941.2 | 10219 | 13134.4 | 4408.7 |
| 170 | 640.4 | 2234.2 | 2081.1 | 2242.8 | 2170.8 | 2086 | 1389.5 | 2450 | 684.4 |
| 171 | 4002.8 | 860 | 1755.1 | 540.2 | 1265.9 | 373.5 | 3814.4 | 1969.2 | 2887.5 |
| 172 | 871.6 | 562.5 | 496.9 | 524.4 | 536.8 | 741.7 | 618.8 | 487.5 | 795.1 |
| 173 | 575.8 | 843.7 | 419.7 | 1448.7 | 916.9 | 512.9 | 1159.7 | 2172.2 | 361 |
| 176 | 217.5 | 1069.1 | 1369.4 | 1083.4 | 461.5 | 2385.1 | 823 | 271.7 | 71.1 |
| 177 | 712.3 | 592 | 497.4 | 495.9 | 559.8 | 651.8 | 489.3 | 372.4 | 574 |
| 178 | 333 | 1722 | 1370.5 | 2400 | 1863.3 | 1852.3 | 1112.4 | 2050.7 | 135.6 |
| 179 | 148.9 | 1165.2 | 914 | 1121.1 | 1438.7 | 84.3 | 739.1 | 1727.8 | 116.9 |
| 180 | 178 | 394.9 | 592.7 | 482.6 | 468.6 | 261.9 | 679.3 | 584.8 | 124.9 |
| 181 | 8559.6 | 3563.7 | 9515.2 | 5496.6 | 3150.6 | 6350.8 | 8535 | 5034.9 | 9024.4 |
| 182 | 303.1 | 363.2 | 607 | 437.8 | 284.5 | 355 | 547.5 | 405.8 | 347.6 |
| 183 | 463.4 | 743.1 | 1416.6 | 409.4 | 692.8 | 313.6 | 1066.3 | 1567.7 | 752.9 |
| 184 | 321.8 | 771.5 | 17054.7 | 1022.1 | 346 | 4736.3 | 714.2 | 527.3 | 221.3 |
| 185 | 858.5 | 7670.7 | 3976.3 | 3238.8 | 6074.4 | 967.8 | 6474.3 | 5307.2 | 1275.6 |
| 186 | 422.8 | 223.9 | 502 | 187.1 | 245.8 | 411.1 | 334.5 | 155.1 | 496.2 |
| 187 | 130.6 | 301.4 | 247.9 | 382.4 | 430.9 | 98 | 251.4 | 429.4 | 499.9 |
| 188 | 231.3 | 443.1 | 490.2 | 209.5 | 672.4 | 207.6 | 460.1 | 490.2 | 230.3 |
| 189 | 186.2 | 290.4 | 253.1 | 112 | 418.8 | 162.1 | 356.5 | 338.2 | 129.7 |
| 190 | 115.7 | 719.2 | 278 | 442.3 | 529.6 | 419.8 | 501.5 | 634 | 110.1 |
| 191 | 762.2 | 2944.3 | 1482.9 | 1491 | 1153.8 | 593.3 | 2253.3 | 2972.3 | 855.8 |
| 192 | 90.9 | 135.5 | 169.1 | 82.5 | 159.1 | 122.1 | 164.6 | 95.6 | 111.4 |
| 193 | 830.6 | 156.9 | 650.7 | 332 | 73.5 | 1566.3 | 778.2 | 246.1 | 749.5 |
| 194 | 116.9 | 307.8 | 210.6 | 367 | 243.7 | 248.1 | 220.6 | 242.1 | 121.1 |
| 195 | 167.1 | 213.3 | 230.4 | 93.7 | 205.8 | 349.5 | 194.4 | 147.8 | 226.8 |
| 196 | 8492.8 | 4271.5 | 4837.9 | 4245 | 4837.6 | 5613.6 | 5166.8 | 2741.2 | 7143.4 |
| 197 | 126 | 137.6 | 88 | 126 | 163.1 | 62.4 | 170 | 153.7 | 99.7 |
| 198 | 77.9 | 1237.6 | 107.3 | 1714.1 | 2069.7 | 160.7 | 379.3 | 3850.1 | 42.8 |
| 199 | 1975.9 | 15850.1 | 1067.1 | 3670.6 | 6331.3 | 92.9 | 10936.9 | 4209.3 | 574.9 |
| 200 | 442.4 | 367.6 | 806.6 | 641.4 | 731.8 | 272.8 | 852.3 | 608.7 | 378.9 |
| 201 | 1717.3 | 1466.3 | 62.7 | 409 | 2205.6 | 81.5 | 409.1 | 388.1 | 531.5 |
| 203 | 185.5 | 1325.5 | 1994.8 | 2659.6 | 1146.7 | 553 | 426.7 | 1650.6 | 294.4 |
| 204 | 640.5 | 373.8 | 990 | 1221.5 | 639.1 | 971.7 | 716.9 | 673.8 | 413.2 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 205 | 3638.5 | 3132.4 | 5021.1 | 4836.2 | 2548.8 | 4867.6 | 5773.1 | 5664.6 | 4500.1 |
| 206 | 4305.4 | 266.2 | 226.9 | 194.4 | 2367.5 | 186.1 | 1565.6 | 1933.1 | 13603.8 |
| 207 | 991.4 | 24.6 | 637.6 | 252.9 | 75.4 | 1349.4 | 407.4 | 119.4 | 589.5 |
| 208 | 589.9 | 1696 | 384.8 | 2423.4 | 990.6 | 458.5 | 465 | 2841.2 | 361.9 |
| 209 | 3044.4 | 1250.1 | 3110.3 | 684.4 | 220.2 | 3097.8 | 4613.7 | 225.9 | 2810 |
| 211 | 21702.9 | 163 | 6106.7 | 1726.7 | 272.3 | 20338.2 | 14214.4 | 218.1 | 13108.3 |
| 212 | 363.1 | 1146.1 | 984.2 | 1161.8 | 1648.5 | 1165.9 | 720.9 | 1062.1 | 324.3 |
| 213 | 117.5 | 85.6 | 93.1 | 80.5 | 140.7 | 104 | 160.1 | 103.1 | 133.2 |
| 215 | 1547.8 | 71 | 895.2 | 308.8 | 53.3 | 2847.9 | 790 | 201.2 | 863.8 |
| 216 | 466.2 | 184 | 743.2 | 255.8 | 157.4 | 648.4 | 522.1 | 231.6 | 528.4 |
| 217 | 253.9 | 1156.6 | 956.5 | 1388.7 | 916.7 | 655.7 | 993.9 | 1488 | 283.6 |
| 218 | 273.9 | 156.2 | 297.3 | 168.4 | 134.5 | 253.5 | 206.7 | 182.5 | 309 |
| 220 | 111.8 | 108 | 178.4 | 87.6 | 87.4 | 78.9 | 114.5 | 73.1 | 95 |
| 221 | 401.7 | 980.8 | 956.6 | 1765.3 | 1118.9 | 944.1 | 2190.1 | 2120.8 | 463.1 |
| 223 | 313.8 | 693.9 | 257 | 526.7 | 856.5 | 230.4 | 725.6 | 679.9 | 230.5 |
| 227 | 1309.4 | 2741.4 | 829.9 | 1455.7 | 2656.4 | 513.5 | 2644.7 | 7955 | 811.4 |
| 228 | 788.4 | 341.7 | 662.1 | 341 | 400 | 605.3 | 554.8 | 321.3 | 480.1 |
| 229 | 165.7 | 231.4 | 242.7 | 245.2 | 169.4 | 132.9 | 206.6 | 199.8 | 256.2 |
| 230 | 378.3 | 3159.3 | 602.9 | 2495.6 | 2807.6 | 427.9 | 1760.9 | 2607.2 | 253.5 |
| 231 | 179.7 | 503.8 | 1002.1 | 932.9 | 465.2 | 263.4 | 286.4 | 683.6 | 168.8 |
| 232 | 2120.5 | 2917.6 | 6467.5 | 1163.8 | 3285.8 | 1825.2 | 5213.4 | 4175.9 | 3969.4 |
| 233 | 3812.2 | 8994.9 | 10159.2 | 16687 | 8022.5 | 3071.4 | 10101.6 | 14509.8 | 5294.2 |
| 235 | 2787.4 | 609.3 | 1195.2 | 414.8 | 721.3 | 253.4 | 2159.8 | 921 | 1447.2 |
| 236 | 205.5 | 420.9 | 541.4 | 310.6 | 425.8 | 168.2 | 568.3 | 431.3 | 257.5 |
| 237 | 1075.9 | 1872.5 | 2343.1 | 2170.9 | 1947 | 1599.7 | 1714 | 1850.9 | 1457.9 |
| 238 | 595.7 | 686.4 | 1928.8 | 1067.3 | 636.7 | 1278.6 | 1084.7 | 907.4 | 839.5 |
| 239 | 408.9 | 910 | 1240.6 | 343.7 | 979.2 | 698.3 | 1207.4 | 1043.8 | 297.9 |
| 240 | 439.8 | 896.6 | 923.9 | 386.1 | 1012.3 | 671.2 | 1029.8 | 1706.7 | 346.5 |
| 241 | 1039.9 | 2207 | 2261 | 786.1 | 1746.5 | 1431 | 2286 | 2914.4 | 614.4 |
| 242 | 6785 | 25733 | 9423.8 | 18037.6 | 23053.8 | 1044.8 | 22442.7 | 27313.9 | 3641 |
| 243 | 172.2 | 719.2 | 374.5 | 427.7 | 727.7 | 198.8 | 793.6 | 730.7 | 379 |
| 244 | 117.1 | 158.2 | 359.4 | 1692.5 | 225.5 | 224.6 | 205.9 | 781.3 | 255.6 |
| 246 | 205.2 | 1124.9 | 754.6 | 1864.1 | 1124.1 | 998.2 | 993.1 | 2067 | 192.7 |
| 247 | 158.1 | 122.8 | 98.9 | 90.5 | 167.3 | 80.1 | 192 | 119.8 | 170.2 |
| 248 | 93.3 | 964 | 2395.2 | 4780.4 | 1534.5 | 1401.2 | 1160.4 | 3767.9 | 132.8 |
| 249 | 628.3 | 275.9 | 966.5 | 609.4 | 278.5 | 1711.8 | 1017 | 717.3 | 746.2 |
| 251 | 86.7 | 1115.8 | 467.4 | 572.2 | 1064.5 | 196 | 697.9 | 947.7 | 78.7 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 253 | 180.8 | 267.8 | 313.8 | 390.7 | 438.7 | 298.7 | 304.7 | 693.5 | 229.6 |
| 254 | 323.1 | 411.8 | 1816.5 | 1559.8 | 527.5 | 2075 | 1017.1 | 1776.1 | 350.4 |
| 255 | 307.7 | 1612.2 | 1295.2 | 2395.7 | 1944.5 | 1881.3 | 1003.5 | 2216.6 | 117.3 |
| 256 | 4984 | 885.5 | 1242.8 | 5330.6 | 1414.4 | 583.9 | 717.6 | 2463.5 | 988.4 |
| 257 | 229.6 | 347.5 | 146.2 | 392.4 | 283.4 | 105.2 | 231.5 | 331.8 | 93.6 |
| 258 | 467.5 | 1932.3 | 306.9 | 580.9 | 1669.9 | 50.7 | 1440.1 | 419.2 | 185.1 |
| 259 | 656.1 | 739.8 | 1239.2 | 816.5 | 649.5 | 213.6 | 1710.4 | 612.8 | 550.2 |
| 260 | 211.1 | 149.6 | 119.9 | 165.1 | 155.4 | 190.5 | 169.4 | 142.4 | 201.5 |
| 261 | 304.8 | 92.6 | 456.1 | 380.7 | 123.1 | 205.9 | 183.4 | 133.3 | 296.1 |
| 263 | 416.5 | 696.6 | 508.2 | 595.2 | 545.5 | 479.7 | 789.5 | 725.8 | 408.1 |
| 263 | 565.6 | 2856 | 535.6 | 2244.5 | 2669.9 | 321.7 | 1544.8 | 2324 | 280 |
| 265 | 470.5 | 69.8 | 209.8 | 116.1 | 97.9 | 338.5 | 324.8 | 100.9 | 325.4 |
| 266 | 256 | 195.2 | 345.7 | 246.5 | 313.5 | 349.5 | 292.8 | 235.5 | 347.8 |
| 256 | 173.1 | 291.4 | 341.1 | 568.6 | 490.5 | 185.1 | 300.4 | 785.2 | 162.7 |
| 268 | 527.3 | 595.7 | 2023.3 | 935.8 | 545 | 1146.6 | 808.3 | 526.4 | 721.4 |
| 269 | 32.6 | 266.5 | 71.6 | 170.1 | 494.5 | 52.6 | 298.3 | 240.8 | 65.9 |
| 270 | 279.9 | 197.9 | 274.9 | 183 | 205.6 | 244.4 | 305.9 | 202.5 | 162.7 |
| 271 | 242.7 | 1141.1 | 567 | 357.7 | 1086 | 274.5 | 625.4 | 1269.3 | 250.7 |
| 272 | 700.4 | 967.8 | 477.4 | 599.8 | 826.1 | 428.3 | 698 | 698.6 | 480 |
| 273 | 4697.9 | 211 | 2811.9 | 916.2 | 234.7 | 3500.5 | 4802.8 | 1550 | 1104.3 |
| 274 | 603.8 | 1411.8 | 1737.5 | 867.4 | 1091 | 321.9 | 1389.7 | 1216 | 664.2 |
| 275 | 225.9 | 3619.5 | 1297.8 | 806.9 | 2484.1 | 321.7 | 2165.1 | 1639.8 | 396.9 |
| 276 | 2732 | 4767.8 | 2036.4 | 2069.3 | 5010.2 | 844 | 6695.5 | 12351.9 | 1238.7 |
| 277 | 255.2 | 264.7 | 225.8 | 355.7 | 325.1 | 200.6 | 250.2 | 259 | 206.6 |
| 278 | 765.8 | 1291.5 | 1132.1 | 1008.7 | 1492.3 | 255.4 | 1550.7 | 1198.8 | 598.7 |
| 279 | 2233.9 | 1837 | 1527.8 | 2169.7 | 1934.2 | 2261.9 | 1828.4 | 2407.2 | 2843.7 |
| 280 | 3349.3 | 2489.9 | 1693.7 | 3130.8 | 2269.8 | 3147.5 | 2337 | 3287.7 | 2513.8 |
| 281 | 37.7 | 385.1 | 326.7 | 815.7 | 570.2 | 488.3 | 255.1 | 762.4 | 74 |
| 282 | 523 | 447 | 277.8 | 1273.6 | 749.8 | 235.4 | 421.2 | 3058.5 | 317.7 |
| 283 | 447.5 | 340.8 | 310 | 351.6 | 440.4 | 246.8 | 393 | 386.9 | 348.1 |
| 284 | 74.4 | 83 | 130.8 | 103.8 | 100.8 | 71.2 | 141.1 | 145.8 | 89.2 |
| 285 | 116 | 109.8 | 107.8 | 96.2 | 136.8 | 95 | 105.2 | 105.6 | 93.8 |
| 286 | 1056.7 | 63.5 | 148.9 | 68.8 | 54.6 | 307.6 | 176.7 | 60.5 | 344.1 |
| 287 | 126.9 | 81.8 | 136.5 | 115.2 | 128.5 | 110 | 153.7 | 95.5 | 99.4 |
| 288 | 172.6 | 145.5 | 166.4 | 146.9 | 195.1 | 144.4 | 165.3 | 173.2 | 184 |
| 289 | 2816.8 | 4208.5 | 1023.7 | 2660.2 | 4312.6 | 404.1 | 3191.6 | 3539 | 1715.5 |
| 291 | 120 | 338.3 | 132.7 | 186.1 | 234.4 | 129.8 | 203 | 425.5 | 113 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 292 | 473.6 | 820.1 | 4181.1 | 6680.4 | 826.4 | 110.4 | 8212.2 | 16919.5 | 977.5 |
| 293 | 120 | 1421.4 | 1229.9 | 592.8 | 1834.6 | 509 | 674.6 | 986.7 | 171.8 |
| 295 | 341.6 | 1948.4 | 1254.9 | 2043.1 | 2299.8 | 1064.4 | 1671.9 | 2245.9 | 229.1 |
| 296 | 552.7 | 1443.5 | 1644.1 | 1603.5 | 1908.8 | 822.2 | 1505.8 | 1432.6 | 753.8 |
| 297 | 45.3 | 90.8 | 94.6 | 50.3 | 139 | 53.5 | 88.5 | 154.3 | 116.4 |
| 298 | 440.1 | 517.5 | 501.4 | 487.3 | 470.9 | 471.4 | 489.6 | 506.6 | 219.8 |
| 299 | 725.6 | 819.4 | 1275.9 | 4386.6 | 3103.3 | 1663.7 | 910.5 | 4069.9 | 1147.8 |
| 300 | 177.4 | 1033.2 | 826.4 | 1230.7 | 1114.8 | 748.3 | 543.4 | 1306.1 | 250.1 |
| 301 | 2124.1 | 211.2 | 358.6 | 194.9 | 158 | 913.5 | 300.7 | 228.4 | 485.7 |
| 302 | 37.2 | 267 | 6.5 | 240.6 | 211.9 | 26.1 | 121.2 | 193.6 | 46.4 |
| 303 | 324.2 | 270.8 | 472.7 | 273.6 | 263 | 997.6 | 487 | 248.9 | 581.6 |
| 304 | 344.9 | 585.6 | 322.9 | 461.8 | 884.2 | 214.1 | 543 | 356.5 | 364.9 |
| 305 | 175.5 | 190.4 | 287.7 | 598.2 | 791.2 | 551.1 | 859.4 | 2550.8 | 158.7 |
| 306 | 189.2 | 803.1 | 191.1 | 557.8 | 620.8 | 163.1 | 322.9 | 218.6 | 195.4 |
| 307 | 302 | 238.1 | 145.1 | 553.9 | 295.1 | 159.8 | 280.9 | 449.1 | 226.3 |
| 308 | 1190.6 | 794.8 | 1165.2 | 705.2 | 515.1 | 1800.1 | 914.4 | 520.9 | 1641.8 |
| 310 | 362.3 | 134.8 | 505.7 | 103.4 | 125.4 | 229.5 | 318.3 | 62.5 | 326.1 |
| 311 | 62.7 | 499.8 | 263.8 | 932.2 | 246.8 | 162.7 | 224.5 | 1036.4 | 115.8 |
| 312 | 902.2 | 50.7 | 593.6 | 151.6 | 54.4 | 843.6 | 810.1 | 19.4 | 523.1 |
| 313 | 132.9 | 517.9 | 687.5 | 313.3 | 244.1 | 457.1 | 385.1 | 576.9 | 131.9 |
| 314 | 522.6 | 650.8 | 980.1 | 1264.1 | 1058 | 586.8 | 1856.2 | 1354.1 | 372 |
| 315 | 391.8 | 154.3 | 554.7 | 155.4 | 153.4 | 591.4 | 395.7 | 235.9 | 458 |
| 316 | 210.3 | 558.3 | 491 | 565.6 | 525.6 | 484.4 | 470.4 | 509 | 234 |
| 317 | 314.7 | 271 | 195.4 | 240.9 | 303.1 | 168.8 | 387.8 | 1026.2 | 214.7 |
| 318 | 45.9 | 383.2 | 425.9 | 527.9 | 428.5 | 97 | 264.8 | 364.3 | 127.7 |
| 319 | 174.9 | 528.4 | 259 | 454.7 | 535.4 | 302.5 | 225.5 | 319.9 | 223.3 |
| 320 | 572.9 | 159.3 | 518.2 | 278.5 | 211.4 | 569.8 | 518.8 | 239.3 | 464.9 |
| 321 | 307.2 | 232.7 | 332.4 | 169.5 | 222.3 | 311.8 | 367.9 | 203.1 | 354.6 |
| 322 | 164.7 | 350.9 | 214.6 | 345.1 | 354.5 | 264.7 | 257.5 | 318.3 | 243.3 |
| 323 | 2580.9 | 1349.5 | 3402.6 | 2592.2 | 1137.5 | 3939.8 | 2125.9 | 1848.5 | 1426.6 |
| 324 | 69 | 597.2 | 475.4 | 645.4 | 435.8 | 233.3 | 224 | 688.1 | 72.4 |
| 325 | 317.1 | 681.6 | 505.4 | 818.9 | 491.5 | 395.8 | 558.8 | 605.8 | 254.6 |
| 326 | 790 | 1838.6 | 2873.2 | 3607 | 2251.9 | 4068.7 | 6144 | 3552.4 | 3136 |
| 327 | 6.7 | 409.3 | 78.4 | 615.9 | 402.1 | 10 | 18.7 | 342.6 | 30.3 |
| 328 | 751.2 | 151 | 376.6 | 307 | 255.2 | 542.9 | 560.4 | 300.2 | 662.8 |
| 329 | 85.3 | 109.9 | 91 | 74 | 63 | 94.7 | 77.9 | 45.9 | 80.6 |
| 330 | 54.9 | 951 | 436.1 | 1119 | 408.8 | 55.7 | 678 | 877.4 | 141.4 |

(continued)

| 3c | | | | | | | | | |
|---|---|---|---|---|---|---|---|---|---|
| SEQ ID NO: | 830TT | 831TT | 832TT | 834TT | 835TT | 836TT | 837TT | 838TT | 839TT |
| 331 | 403.9 | 382.1 | 215 | 302.1 | 371.7 | 380.3 | 325.4 | 324.5 | 332 |
| 333 | 571.4 | 156 | 462.8 | 160.5 | 140.1 | 1058.8 | 443.4 | 150.5 | 603.1 |
| 334 | 195.4 | 500.9 | 1027.6 | 250.9 | 655 | 196.6 | 332.8 | 432.2 | 161.6 |
| 335 | 155.1 | 109.1 | 732.5 | 572.2 | 165.5 | 62 | 194.8 | 923.7 | 127.6 |
| 336 | 668 | 210.4 | 480.1 | 280.3 | 175.5 | 389.2 | 344.2 | 252.7 | 445.1 |
| 337 | 197.1 | 220.6 | 207.5 | 266.7 | 387.8 | 309.2 | 374 | 240.3 | 256.6 |
| 338 | 217.2 | 890.9 | 464.6 | 1427.6 | 896.6 | 189.4 | 1039 | 1728.1 | 231.7 |
| 339 | 377 | 346.8 | 345.1 | 332.7 | 324.7 | 435.1 | 457.4 | 277.5 | 454.9 |
| 340 | 330.6 | 95874.2 | 237.5 | 123339.8 | 92245.2 | 222.4 | 696.6 | 74884.9 | 304.2 |
| 341 | 657.2 | 434.5 | 521.8 | 461 | 442.6 | 785.8 | 552.9 | 485.6 | 713.7 |
| 343 | 109.4 | 4793.6 | 207.9 | 601.9 | 2590 | 773.6 | 1138 | 1171.9 | 57.1 |
| 344 | 1167 | 249.7 | 893.9 | 545.2 | 581.2 | 963.4 | 1019.3 | 554.7 | 1114.2 |
| 345 | 252.6 | 620.5 | 437.9 | 408.2 | 509.3 | 204.8 | 544.8 | 404.8 | 294.1 |
| 347 | 2279.3 | 1845.7 | 6453.3 | 3490 | 1999.5 | 3041.5 | 2644 | 1859.9 | 2280.8 |
| 348 | 2856.1 | 2533.8 | 7636.1 | 6329.8 | 3556.3 | 5813.9 | 5133.1 | 4694.9 | 5037.6 |
| 349 | 353.3 | 256.7 | 290.3 | 316.7 | 301.8 | 407.4 | 319.1 | 485.8 | 258.4 |
| 350 | 386 | 804.7 | 473.6 | 475.2 | 611.1 | 257.1 | 657.4 | 638.3 | 199.2 |
| 351 | 308.4 | 2206.4 | 1213.1 | 2566.3 | 1996.9 | 246.9 | 1150.3 | 2079.9 | 292.1 |
| 352 | 355.6 | 83.4 | 314.2 | 140.3 | 111.5 | 364 | 265.6 | 231 | 294.5 |
| 353 | 232.6 | 402.1 | 295 | 294.2 | 423.7 | 229.4 | 384.5 | 414.6 | 243.7 |

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 354 | 413.4 | 103.1 | 28.5 | 89.1 | 50.8 | 119.4 | 632.2 | 9.2 | 48.7 |
| 355 | 518.1 | 63.6 | 16 | 83.3 | 29 | 114.5 | 55.8 | 49.5 | 44 |
| 356 | 617.8 | 1163.1 | 1507.8 | 789.4 | 1313.2 | 1067.3 | 1224.9 | 824.3 | 740.8 |
| 357 | 750.6 | 663.3 | 1662.6 | 876.7 | 839.2 | 1660 | 1243 | 1043.5 | 843.5 |
| 358 | 647.8 | 509.5 | 567.7 | 1088.7 | 1049.5 | 795 | 1349.4 | 535 | 1699.5 |
| 359 | 330.5 | 214.7 | 472.1 | 313.5 | 373.5 | 481.3 | 371.9 | 361.8 | 235.3 |
| 362 | 288.4 | 245.9 | 172.7 | 589.6 | 377.5 | 866.5 | 666.6 | 454.5 | 954 |
| 360 | 434.1 | 305.6 | 182.5 | 297.3 | 370.2 | 347.1 | 290.1 | 218.1 | 444.6 |
| 361 | 682.3 | 740.4 | 589.9 | 765.7 | 495.7 | 894.5 | 527.5 | 1472.7 | 327.2 |
| 363 | 435.5 | 328.8 | 483.4 | 387 | 360.5 | 673 | 502.1 | 431.2 | 367 |
| 1 | 532.1 | 658.9 | 827.5 | 640.1 | 768.3 | 484.6 | 619.1 | 510.9 | 964.5 |
| 2 | 4059.8 | 3830.7 | 4516.5 | 3770.5 | 2512.9 | 3756.6 | 2448.4 | 3524.2 | 3003.6 |
| 7 | 483.8 | 209.7 | 470.6 | 825.4 | 484.2 | 153.7 | 393.7 | 965 | 873.7 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 8 | 1199.1 | 1312 | 934.6 | 1643.2 | 1176.8 | 871.7 | 1342.3 | 768.5 | 732 |
| 9 | 8223.9 | 2596.3 | 7524.8 | 6220.9 | 3456.3 | 6027 | 7023.5 | 7427.5 | 3975.6 |
| 10 | 51.2 | 96.2 | 67.9 | 101.6 | 111.8 | 139.3 | 75.7 | 46.4 | 157.4 |
| 11 | 171.2 | 198.5 | 495.5 | 218.3 | 206 | 597.6 | 634.9 | 288.5 | 318.8 |
| 13 | 537.4 | 265 | 36.3 | 777.1 | 442.1 | 95.7 | 614.9 | 65.2 | 238.9 |
| 14 | 1233.6 | 1157.9 | 134.4 | 2731 | 1781.1 | 837.6 | 2365 | 195.6 | 747.9 |
| 15 | 699.2 | 133.5 | 191.7 | 417.5 | 408.8 | 260 | 185.5 | 349.2 | 539.4 |
| 16 | 662.4 | 1257.3 | 1518.2 | 1301 | 966.8 | 1066.2 | 1385 | 714.3 | 1347.5 |
| 17 | 1194.5 | 1224.2 | 2909.6 | 1355.9 | 1395.5 | 1413.2 | 1584.1 | 1543.7 | 1301.1 |
| 19 | 1305.5 | 169.4 | 164.2 | 167.4 | 153.8 | 335.4 | 192.4 | 217.8 | 159 |
| 20 | 11.1 | 1.1 | 55 | 1.1 | 1.2 | 69.6 | 36.1 | 1.7 | 2.7 |
| 21 | 240.8 | 87.2 | 45.6 | 290.6 | 14.9 | 100.6 | 193.6 | 1.2 | 665.2 |
| 22 | 9947.1 | 7799.8 | 11063.4 | 5454.8 | 5808.5 | 5943.2 | 5158.2 | 8719.7 | 4509.2 |
| 23 | 956.7 | 1.1 | 48.1 | 157.6 | 1.1 | 4.3 | 28.8 | 77.3 | 62.7 |
| 24 | 218 | 30.6 | 818.7 | 223.9 | 55.2 | 225 | 83.6 | 138.2 | 313.6 |
| 26 | 9557.1 | 76.6 | 228.7 | 772.1 | 58.3 | 169.6 | 349.8 | 241.6 | 342.1 |
| 27 | 776.7 | 416.1 | 446.1 | 321.7 | 837 | 194.8 | 624.3 | 214.8 | 248.9 |
| 29 | 59.3 | 307.6 | 113.8 | 111.9 | 151.8 | 93.7 | 155.2 | 94.7 | 165.9 |
| 30 | 1162 | 61.1 | 36.1 | 74 | 37.7 | 338 | 95.9 | 132.1 | 81.4 |
| 31 | 656.3 | 70.7 | 13.1 | 264 | 24.8 | 107.5 | 243.8 | 63.6 | 1804.1 |
| 33 | 863 | 622.5 | 646.3 | 338.4 | 225.9 | 1364.6 | 528.1 | 298.3 | 824.1 |
| 34 | 4801 | 4062.8 | 7041.9 | 2172.2 | 2709.9 | 7154.6 | 4874 | 3483.4 | 1898.9 |
| 35 | 349.5 | 48.6 | 21 | 69.1 | 220.8 | 140.3 | 127.8 | 42.4 | 29.6 |
| 36 | 247.6 | 112.1 | 70.6 | 952.1 | 127.4 | 437.9 | 132.7 | 244.9 | 2851.6 |
| 37 | 359.5 | 154.3 | 129.5 | 201 | 163.4 | 356.6 | 166.6 | 166.1 | 147.5 |
| 38 | 818.9 | 343.8 | 681 | 579.2 | 1066.9 | 635.4 | 454.1 | 452.9 | 944.3 |
| 40 | 584.7 | 371.3 | 337.3 | 437.1 | 313.3 | 551.1 | 403.2 | 378 | 412.8 |
| 41 | 247.9 | 211.8 | 28 | 324.9 | 206.9 | 141.2 | 142.1 | 246.9 | 1045.2 |
| 42 | 201.2 | 77.8 | 271.3 | 370.1 | 220.5 | 370.8 | 243.3 | 311.3 | 477.7 |
| 43 | 319.5 | 237 | 319.5 | 348.9 | 367.9 | 367.4 | 376.6 | 359.5 | 514.5 |
| 44 | 340.2 | 338.9 | 375.8 | 466.7 | 295.2 | 1064.8 | 611.2 | 385.6 | 862.4 |
| 45 | 387.6 | 484.6 | 587.1 | 607.2 | 566.7 | 339.3 | 634 | 461.7 | 686.8 |
| 46 | 312.8 | 273 | 211.3 | 310.6 | 243.5 | 430.8 | 275.4 | 232.3 | 243.1 |
| 47 | 267.1 | 166.9 | 95.5 | 209.9 | 200.1 | 356 | 306.7 | 200 | 468.1 |
| 48 | 586.4 | 32.7 | 36.8 | 85.4 | 15.6 | 170.4 | 44.6 | 128.7 | 52.4 |
| 49 | 559.1 | 182.8 | 87.8 | 323.1 | 320.3 | 292.7 | 479.7 | 171.1 | 244 |
| 50 | 183 | 51.5 | 313.7 | 151.2 | 54.7 | 307.2 | 159.4 | 147.7 | 107.5 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 51 | 50.2 | 57.3 | 25.1 | 53.5 | 27.6 | 365.3 | 326.7 | 129.5 | 249.3 |
| 52 | 195.7 | 196.6 | 173.8 | 234.1 | 186.2 | 225.4 | 159.6 | 208.9 | 221.8 |
| 53 | 82.5 | 34.1 | 142.8 | 294.3 | 73 | 168.9 | 158.4 | 50.3 | 1001.9 |
| 54 | 497.5 | 1164.8 | 197.8 | 1471.8 | 1002.7 | 709.6 | 1166.2 | 163.1 | 1728.2 |
| 55 | 119.7 | 111.7 | 100 | 104.8 | 90.9 | 118.7 | 116 | 94.5 | 92.2 |
| 56 | 50.4 | 78.9 | 15.7 | 207.8 | 125.4 | 69.1 | 86.5 | 91.8 | 155 |
| 57 | 913.7 | 120.1 | 39.3 | 348.7 | 96 | 112.4 | 648.3 | 63.7 | 1449.6 |
| 58 | 937.3 | 144.4 | 33.7 | 395.5 | 111.4 | 501.8 | 1139.1 | 62.2 | 1741.7 |
| 59 | 1524.7 | 2456.9 | 2314.6 | 1572.7 | 1908.7 | 1437.1 | 1816.2 | 851.3 | 1581 |
| 60 | 239.9 | 177.8 | 101.9 | 208.9 | 122.9 | 164.2 | 115.8 | 114.4 | 341.6 |
| 61 | 138.8 | 102.4 | 167.6 | 195.1 | 161.4 | 602.8 | 171.7 | 220.1 | 121.5 |
| 62 | 3218.4 | 1230.8 | 93.3 | 144.4 | 1893.7 | 776.3 | 1111.1 | 1116.3 | 275.7 |
| 63 | 476.2 | 251.9 | 390.8 | 305.5 | 390.5 | 446.7 | 360.3 | 186.2 | 416 |
| 64 | 260 | 108.5 | 72.5 | 133 | 186.3 | 125.9 | 184.1 | 121.4 | 174.1 |
| 65 | 130.1 | 114.1 | 115.9 | 113.8 | 100.7 | 57.6 | 84 | 103.6 | 167.3 |
| 66 | 736.8 | 1620 | 528.8 | 1200.9 | 1060.8 | 922.4 | 1062.5 | 613.3 | 1936.4 |
| 67 | 36967.4 | 32327.9 | 35416.1 | 33262.7 | 33086.4 | 50663.1 | 33055.8 | 33098.2 | 33842.1 |
| 68 | 3604.6 | 765.6 | 2711 | 2612.8 | 1433.6 | 3309.4 | 1090.5 | 3575.3 | 2217.7 |
| 69 | 4370.2 | 2244.8 | 8123.8 | 4669.2 | 2913.3 | 9134.7 | 2674.3 | 9458.6 | 4732.4 |
| 70 | 2223.5 | 1376.4 | 751.7 | 2378 | 1890.8 | 2166.2 | 2162.9 | 1456.9 | 3220.2 |
| 71 | 1018.7 | 90.7 | 156 | 1072.2 | 120.5 | 96.6 | 145.6 | 125.6 | 898.5 |
| 72 | 552.9 | 1523.3 | 537.5 | 355.6 | 575.3 | 229.2 | 397.7 | 248.8 | 869.3 |
| 73 | 447.7 | 234.9 | 263.5 | 297.8 | 293.7 | 265.4 | 305 | 231.6 | 465.4 |
| 75 | 260.1 | 159 | 459.2 | 194.5 | 212.8 | 460 | 245.4 | 508.2 | 472.1 |
| 76 | 273.6 | 135.4 | 1.1 | 2171.1 | 611.1 | 559.9 | 220.4 | 108.3 | 1748.8 |
| 77 | 4806.2 | 4952.7 | 7485.3 | 2585.4 | 4993.9 | 9359.9 | 6144.5 | 5753.5 | 2422 |
| 78 | 623.9 | 418.4 | 269 | 898.4 | 765.7 | 441 | 601.4 | 703 | 1341.8 |
| 79 | 121.6 | 161.5 | 129.6 | 160.7 | 133.3 | 143.3 | 133.6 | 130.7 | 177.4 |
| 80 | 132.5 | 767.8 | 1091.7 | 413.8 | 130.3 | 423 | 489.5 | 238.5 | 763.7 |
| 81 | 512.5 | 282.4 | 123.8 | 218.8 | 242.4 | 297 | 1287.3 | 81.3 | 806.9 |
| 82 | 309.9 | 198.2 | 168.6 | 248.4 | 267 | 111.1 | 195.8 | 299.2 | 260.3 |
| 84 | 387.2 | 136.7 | 73.1 | 108.8 | 68.4 | 133.5 | 96.7 | 102.1 | 324.1 |
| 85 | 248.9 | 142.3 | 90.9 | 119.5 | 146.4 | 305.5 | 231.4 | 148.7 | 109.4 |
| 86 | 128.2 | 81.2 | 328 | 73.6 | 54.4 | 101.3 | 98.7 | 95.6 | 101 |
| 87 | 389.7 | 249.9 | 208.9 | 161 | 233.2 | 395.2 | 213.6 | 276.9 | 167.9 |
| 88 | 3944.8 | 2735.3 | 8374.9 | 5614.3 | 5926.1 | 7145.8 | 6096.2 | 9499.1 | 4340.8 |
| 89 | 354 | 171.2 | 148.1 | 205.8 | 141.7 | 276.5 | 145.7 | 180.3 | 128.5 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 90 | 115.9 | 91.9 | 105.1 | 103.9 | 93 | 163.4 | 108.3 | 110.6 | 69.3 |
| 91 | 1164.3 | 208.6 | 1.2 | 284.2 | 96.6 | 444.2 | 1453.8 | 67 | 464.2 |
| 92 | 4085 | 6778.2 | 2708.1 | 9676.9 | 5168.6 | 3758.8 | 6575.2 | 2375.7 | 10249.7 |
| 93 | 301.2 | 226.8 | 421.5 | 194.9 | 232.2 | 375.5 | 171.1 | 428.1 | 163.7 |
| 94 | 995.1 | 541.1 | 897.8 | 657.7 | 595.7 | 381.9 | 809 | 335 | 1275.8 |
| 95 | 1045.2 | 582.5 | 118.6 | 543.6 | 452.1 | 179.3 | 209.1 | 188.8 | 345.6 |
| 96 | 151.5 | 196 | 167.6 | 244 | 181.7 | 245.6 | 205.7 | 171.9 | 230.8 |
| 97 | 166.6 | 276.5 | 308 | 289.5 | 386.8 | 228.9 | 347 | 249.1 | 374.6 |
| 98 | 60.4 | 31.5 | 27 | 23.1 | 9.2 | 31.1 | 20 | 38.2 | 21.7 |
| 99 | 253.6 | 187.7 | 194.3 | 246 | 183.1 | 311.2 | 227.1 | 164.5 | 250.1 |
| 100 | 19287.6 | 6400.4 | 8181.3 | 2605.3 | 10229.3 | 7403 | 9672.3 | 11255.4 | 738.2 |
| 101 | 266.7 | 227.3 | 183.5 | 187 | 210.8 | 246.9 | 215.4 | 175.2 | 303.8 |
| 102 | 171.5 | 108.4 | 168.3 | 145.6 | 139.2 | 216.4 | 161.6 | 192.6 | 129.1 |
| 103 | 278.8 | 262.5 | 300.2 | 251.6 | 305.7 | 614.4 | 341.7 | 296.7 | 354.3 |
| 104 | 443.1 | 801.2 | 207.5 | 609.2 | 639.4 | 662.8 | 235.7 | 480.5 | 507.8 |
| 105 | 463.1 | 1053.8 | 415.1 | 584.5 | 740.7 | 772.3 | 384.2 | 617 | 387.3 |
| 106 | 518.9 | 705.8 | 207.9 | 540.7 | 585.7 | 524.4 | 227 | 513.5 | 481.3 |
| 107 | 364.9 | 105 | 41.3 | 163.2 | 64.4 | 139.2 | 185.1 | 113.3 | 132.5 |
| 108 | 330.2 | 330.9 | 645.2 | 275.1 | 408 | 425.5 | 324.6 | 567.1 | 309.2 |
| 109 | 67.3 | 6034.5 | 117 | 7025.2 | 3555.9 | 130.1 | 4629.2 | 56.5 | 4997.5 |
| 110 | 28.9 | 11743.3 | 96.5 | 11643.7 | 6913.6 | 1.2 | 6469 | 1.1 | 12381.4 |
| 111 | 1421.1 | 1571 | 367.8 | 1848.8 | 3058.4 | 428.3 | 1123.4 | 1923.1 | 1485.6 |
| 112 | 527 | 115.6 | 213 | 921.9 | 582.1 | 50.6 | 951.8 | 441.7 | 1495.5 |
| 113 | 455.9 | 246.9 | 53.3 | 182.6 | 309 | 119 | 257 | 158.9 | 473.4 |
| 114 | 507.9 | 943.6 | 1193 | 488.7 | 600.7 | 470.7 | 525.8 | 646.5 | 1445.6 |
| 115 | 579.2 | 287 | 238.3 | 468.6 | 331.5 | 339.6 | 406.9 | 347.8 | 648.7 |
| 116 | 323 | 237.9 | 245 | 203.6 | 210.8 | 323.5 | 238.4 | 227.4 | 251.7 |
| 117 | 2477.5 | 2049 | 5384.6 | 1932.8 | 2356.6 | 2151 | 1724 | 4147.6 | 916.2 |
| 118 | 442.9 | 1260.5 | 217.3 | 146.6 | 481 | 247.5 | 132.8 | 138.7 | 375.2 |
| 119 | 196.2 | 294.2 | 59.3 | 102.7 | 207.4 | 22.6 | 112.9 | 103.5 | 412.3 |
| 120 | 173.7 | 63.5 | 76.2 | 116 | 85.4 | 51.5 | 83.3 | 88.4 | 65.8 |
| 121 | 572.4 | 1198.8 | 854.2 | 827.2 | 1024.8 | 671 | 1242.7 | 359.7 | 1485.9 |
| 122 | 290.1 | 202.3 | 237.7 | 134.1 | 153.2 | 200.7 | 314.3 | 99.7 | 125.7 |
| 123 | 49.5 | 102.7 | 113.5 | 109.8 | 125.4 | 167.4 | 124.8 | 161 | 127.5 |
| 124 | 122 | 156.7 | 104.5 | 141 | 126.9 | 183.5 | 129.5 | 127.1 | 87.6 |
| 125 | 901.7 | 798.3 | 548.8 | 603.5 | 613.7 | 1170.8 | 823 | 708 | 576.3 |
| 126 | 1558.5 | 2022.3 | 1906.6 | 1073.1 | 1537.3 | 1698.3 | 1203.7 | 1574 | 734.6 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 127 | 67.5 | 94.2 | 121.1 | 129.3 | 177.1 | 229.6 | 142.1 | 113.2 | 149.3 |
| 128 | 270.3 | 227.2 | 174 | 236.1 | 175.5 | 523.6 | 265.8 | 219.6 | 215.4 |
| 129 | 252.6 | 119.8 | 55.2 | 205.4 | 120.9 | 238.2 | 283.6 | 118.6 | 158.2 |
| 130 | 71 | 74.9 | 81.3 | 173.4 | 64.2 | 151.7 | 101.2 | 72.2 | 206.8 |
| 131 | 99.7 | 56.1 | 56.9 | 101.9 | 71.1 | 71.4 | 85.6 | 39.6 | 410 |
| 132 | 179.1 | 167.2 | 163.5 | 226.3 | 168.3 | 291.1 | 198.8 | 168.5 | 289.6 |
| 133 | 65.3 | 135.6 | 129.8 | 134.1 | 108.6 | 90.5 | 155.9 | 90.6 | 247.7 |
| 134 | 141.8 | 67.6 | 374.3 | 220.5 | 242.9 | 331.8 | 297.9 | 192.8 | 232 |
| 135 | 323.6 | 502.2 | 986.3 | 429.9 | 398.9 | 720.6 | 576.6 | 551 | 856.1 |
| 138 | 179.5 | 85.7 | 141.7 | 111.3 | 90.4 | 263.4 | 150 | 100.7 | 95.9 |
| 139 | 106.9 | 129.9 | 131.6 | 116.9 | 120.4 | 167.8 | 111.3 | 119.2 | 214.6 |
| 140 | 1721.8 | 3036.3 | 2185.5 | 2508.8 | 4344.2 | 551 | 489.1 | 3284.4 | 2492 |
| 141 | 2410.6 | 3225.5 | 1665.6 | 1916.7 | 2935.3 | 1825.5 | 2844.6 | 1474.7 | 1924.7 |
| 144 | 467.5 | 2221 | 378.9 | 13755 | 448 | 595.3 | 8268.8 | 333.6 | 24504.7 |
| 145 | 184.9 | 199.7 | 201.3 | 201.7 | 182.6 | 355.7 | 205.2 | 181.5 | 193.7 |
| 146 | 199.9 | 232.9 | 156.3 | 218.2 | 193.1 | 318.3 | 215.5 | 183.7 | 234.1 |
| 147 | 39 | 23.2 | 35 | 23.8 | 39.4 | 81 | 29.4 | 8.2 | 43.7 |
| 148 | 623.9 | 168.1 | 255.4 | 231.2 | 256.6 | 443 | 311.3 | 211.5 | 208.8 |
| 149 | 298.4 | 748.7 | 946.9 | 488 | 781.4 | 682.8 | 843.3 | 449.2 | 418 |
| 150 | 271.8 | 256.3 | 118.7 | 145.6 | 239.5 | 283.8 | 224.4 | 131.5 | 178.9 |
| 151 | 386 | 739.9 | 194.8 | 394.3 | 448.1 | 199.1 | 376.1 | 165.6 | 142 |
| 153 | 101.1 | 82.9 | 117.5 | 170.7 | 94.7 | 158.3 | 93.7 | 98.7 | 124.8 |
| 154 | 696.5 | 806.1 | 188.7 | 316.4 | 89.7 | 503.2 | 343.3 | 153.6 | 615.8 |
| 155 | 126.3 | 91.9 | 134.1 | 121.4 | 87.9 | 243.6 | 235.9 | 104.5 | 96.7 |
| 156 | 119.2 | 40.6 | 68.7 | 94.7 | 77.3 | 67 | 131.6 | 62.7 | 173.3 |
| 157 | 77.5 | 165.7 | 132.1 | 100.7 | 88 | 103.8 | 133.9 | 113.1 | 166.4 |
| 158 | 30.3 | 9.6 | 1.4 | 158.3 | 97.2 | 41.9 | 159.9 | 1.2 | 1019.8 |
| 159 | 3785.9 | 3917 | 4901.3 | 3467.1 | 3669.5 | 3653.4 | 3209.2 | 3643.5 | 3005.3 |
| 161 | 90.5 | 73.4 | 50 | 2779.2 | 176 | 170.6 | 3362.6 | 1.2 | 4114.9 |
| 162 | 165.6 | 48.8 | 50.3 | 49 | 23.6 | 75.1 | 52.8 | 83.8 | 63.7 |
| 164 | 274.7 | 1048.7 | 159.5 | 2491.4 | 3031 | 446.5 | 2413.5 | 213.4 | 2997.7 |
| 166 | 5679.8 | 5894 | 6666.6 | 4629.2 | 8306.1 | 5972 | 3859.7 | 5542.7 | 4096.6 |
| 167 | 2803.8 | 865.7 | 583.6 | 1447.2 | 1517.1 | 468.1 | 1020.9 | 895.3 | 1756 |
| 168 | 664.5 | 1070.8 | 946.4 | 1307.6 | 1154.7 | 804.8 | 787.3 | 1131.5 | 1135.4 |
| 169 | 4025.8 | 9912.9 | 783 | 3510.5 | 4082.6 | 1690.6 | 3122.8 | 3606.3 | 3273.9 |
| 170 | 630.2 | 1665.5 | 1244.3 | 2308 | 1487.7 | 895.5 | 1906.2 | 884.1 | 2192.6 |
| 171 | 1597.9 | 18.2 | 1.3 | 52.3 | 123.4 | 3 | 174 | 103.7 | 50.6 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 172 | 648.7 | 336.4 | 566.3 | 568.5 | 546.7 | 747 | 570.3 | 569.6 | 634.3 |
| 173 | 301.5 | 351.8 | 502.4 | 419.8 | 242.7 | 82.9 | 106.8 | 377.3 | 1153.3 |
| 176 | 205 | 103.6 | 37.4 | 2372.3 | 1244.7 | 431 | 2046.7 | 1.1 | 6658.9 |
| 177 | 657.2 | 340.2 | 225.5 | 561.7 | 411 | 195 | 319.8 | 255.7 | 474.3 |
| 178 | 133.2 | 340.8 | 44 | 588.7 | 77.1 | 236.2 | 156 | 90.1 | 2026.1 |
| 179 | 114.7 | 48.7 | 65.5 | 86.7 | 56.7 | 89.4 | 59.6 | 95.5 | 53.8 |
| 180 | 85.1 | 88.5 | 71.9 | 296.6 | 369.5 | 193.3 | 269.4 | 71.5 | 700 |
| 181 | 13466.7 | 11481.6 | 14492 | 10957.2 | 9935.2 | 21897 | 10184 | 12851.6 | 7312.1 |
| 182 | 654.9 | 653.9 | 427.9 | 406.4 | 491.3 | 347.1 | 403.3 | 562.8 | 449.5 |
| 183 | 1741.8 | 1.2 | 72 | 216.6 | 1.5 | 13.4 | 127.3 | 74.7 | 60.8 |
| 184 | 210.8 | 167 | 156.2 | 4707.4 | 163.4 | 275.2 | 340.4 | 183.2 | 408.7 |
| 185 | 2742.8 | 559.8 | 331.2 | 1091.6 | 875.4 | 2220.6 | 1051 | 563.7 | 1819.8 |
| 186 | 540.1 | 408.4 | 119.6 | 379 | 511.7 | 293.6 | 378.6 | 390.8 | 334.8 |
| 187 | 461 | 78.3 | 76.6 | 73.6 | 56.4 | 324 | 75.8 | 168 | 671.7 |
| 188 | 583.1 | 57.1 | 288.4 | 239 | 128.3 | 224 | 113.7 | 356.9 | 137.6 |
| 189 | 194.8 | 29.2 | 85.5 | 119.5 | 62.4 | 143.8 | 67.8 | 131.3 | 68 |
| 190 | 123.9 | 206.8 | 248.5 | 85.4 | 260.6 | 70.6 | 194.8 | 276 | 275.5 |
| 191 | 1270.2 | 2209.5 | 1240.3 | 741.8 | 1132.9 | 3308.2 | 2393.3 | 694.6 | 828.7 |
| 192 | 119.5 | 81 | 147 | 79.8 | 63.3 | 171.5 | 100.5 | 89 | 245.3 |
| 193 | 572 | 747.2 | 2493.9 | 1776.2 | 1406.7 | 2749.4 | 771.4 | 2411.6 | 1560.3 |
| 194 | 156.3 | 196.3 | 74.6 | 184.9 | 231.7 | 72.8 | 155.2 | 181 | 247.3 |
| 195 | 308.3 | 259.1 | 115.5 | 265.8 | 182.4 | 266.7 | 269.9 | 321.4 | 172.6 |
| 196 | 8888.9 | 1967.3 | 1862.4 | 4252.6 | 4150.7 | 1524.3 | 2417.5 | 2838 | 4062.6 |
| 197 | 123.1 | 56.4 | 64.3 | 55.9 | 63.4 | 61 | 92.7 | 88.5 | 67.4 |
| 198 | 72.7 | 46.7 | 59.1 | 128.1 | 86.2 | 134.7 | 76 | 89.2 | 1231.8 |
| 199 | 715.5 | 149.9 | 77.4 | 200.9 | 99.1 | 117.1 | 93.7 | 83.2 | 58.1 |
| 200 | 436.8 | 119 | 110 | 279 | 144.7 | 161.1 | 188.8 | 132.1 | 183.8 |
| 201 | 420.4 | 136.7 | 34.5 | 76 | 82.5 | 105.4 | 714.7 | 46.9 | 48.3 |
| 203 | 216.9 | 328.6 | 478.5 | 1783 | 339.6 | 318.5 | 429.8 | 215.3 | 696.8 |
| 204 | 424.2 | 534.9 | 2224.1 | 826.2 | 1409.9 | 1071.2 | 1167.6 | 730.8 | 1179.7 |
| 205 | 5342.3 | 6991.4 | 6610.9 | 7662.4 | 6557.2 | 4601.1 | 6434.7 | 5994.9 | 5922.6 |
| 206 | 196.9 | 136.2 | 256 | 226 | 167.3 | 461.3 | 381.3 | 255.3 | 194 |
| 207 | 856.9 | 2195.1 | 498.1 | 3374 | 2428.9 | 655.5 | 1311 | 1015.2 | 1559.2 |
| 208 | 394.5 | 627.5 | 228.8 | 462.5 | 481.5 | 375.3 | 249.2 | 214.1 | 366.3 |
| 209 | 4454.3 | 1325.4 | 400 | 2326.1 | 2927.3 | 297.9 | 2136.1 | 2044.2 | 4109.1 |
| 211 | 9691.9 | 10693.8 | 23350.1 | 14624.2 | 22270.2 | 30888 | 17057.7 | 24224.7 | 20568.5 |
| 212 | 572.3 | 271.2 | 156.5 | 584.8 | 425.7 | 440.5 | 458.5 | 393.6 | 829.4 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 213 | 67 | 114 | 61.5 | 73.5 | 71.3 | 151.9 | 87.1 | 81 | 82.8 |
| 215 | 822.1 | 2240.3 | 284 | 3771.2 | 3251.7 | 364.9 | 1154.1 | 891.6 | 1823.7 |
| 216 | 1084.8 | 1208.4 | 774.3 | 825.1 | 931.3 | 1019 | 1459.9 | 886.9 | 394.2 |
| 217 | 263.5 | 128.1 | 178 | 388.9 | 253.4 | 229.3 | 192.2 | 287.8 | 619.7 |
| 218 | 247 | 369 | 430.6 | 309.9 | 285.4 | 378.5 | 371.4 | 431.1 | 300.2 |
| 220 | 192.1 | 65.1 | 72.8 | 82.9 | 64.6 | 195.1 | 82 | 97.9 | 152.1 |
| 221 | 874.9 | 273.4 | 348.7 | 610.2 | 398.6 | 589.1 | 442 | 576.1 | 781.3 |
| 223 | 203.5 | 216.6 | 257.1 | 227.9 | 193 | 239.1 | 307.3 | 269.5 | 181.6 |
| 227 | 1491.4 | 508.8 | 973.3 | 663 | 611.5 | 1289.2 | 657.2 | 715.4 | 1113.4 |
| 228 | 479.7 | 346.7 | 305.5 | 399.5 | 432.4 | 168.3 | 334.5 | 406.8 | 398 |
| 229 | 344.9 | 179.3 | 263.5 | 202.7 | 135.8 | 258.4 | 146.7 | 307 | 178.3 |
| 230 | 408 | 173.8 | 53.4 | 230 | 72.6 | 146.6 | 89.4 | 72.6 | 1044.4 |
| 231 | 60.9 | 169.5 | 179.7 | 565.7 | 166.8 | 279.1 | 189.2 | 170.4 | 295.4 |
| 232 | 7655.5 | 69.6 | 144.4 | 522.1 | 6.3 | 156.1 | 223.7 | 143.5 | 237.3 |
| 233 | 10823.9 | 8532.5 | 7380 | 6335.2 | 4653 | 14890.4 | 6151.6 | 6357.9 | 8900.2 |
| 235 | 964.3 | 116.8 | 99.5 | 118.8 | 232 | 172.3 | 135.3 | 109.6 | 68.6 |
| 236 | 284.7 | 110.1 | 85.2 | 190 | 113.2 | 251.9 | 125 | 160.6 | 109 |
| 237 | 2149.2 | 2342.6 | 1981.7 | 1910 | 1532.6 | 1925.6 | 1630.1 | 2187 | 2001.9 |
| 238 | 1376.8 | 1774.9 | 1294.3 | 1639.9 | 1873.9 | 1331 | 1069.9 | 1075.9 | 920.9 |
| 239 | 677.1 | 102.3 | 55.7 | 598 | 88.8 | 415.3 | 790.4 | 144.5 | 446.3 |
| 240 | 910 | 202.5 | 215.3 | 1413.1 | 146.1 | 590.9 | 1856.1 | 149.8 | 658.9 |
| 241 | 1746.2 | 288.3 | 229.3 | 1845.3 | 213.4 | 1394.4 | 2581 | 292.1 | 1300.3 |
| 242 | 3459.7 | 3959.5 | 1573.1 | 930.4 | 1419.2 | 4708.4 | 2169.8 | 1081.9 | 3241.5 |
| 243 | 789.8 | 205.4 | 201.9 | 189.4 | 191.5 | 359.6 | 385.6 | 187.1 | 231.5 |
| 244 | 166.8 | 289.4 | 346.4 | 297.2 | 266.9 | 336.1 | 253.7 | 298.8 | 291.7 |
| 246 | 735.5 | 339.5 | 415.4 | 754.5 | 305.6 | 434.1 | 389.1 | 182.4 | 1612.5 |
| 247 | 152.5 | 91.8 | 114.1 | 105.7 | 117.4 | 162.4 | 111.1 | 109 | 77.5 |
| 248 | 113.4 | 311.4 | 156.1 | 827.2 | 163.8 | 295.1 | 550.6 | 57.7 | 969 |
| 249 | 546.3 | 2609.6 | 77.3 | 1171.4 | 2406.9 | 1453.1 | 2505.9 | 1690.1 | 2558.1 |
| 251 | 114.6 | 75.4 | 190.3 | 144.5 | 67.4 | 139.8 | 164.5 | 86.3 | 272.4 |
| 253 | 343.5 | 454 | 621.6 | 336.8 | 375.4 | 294.5 | 353 | 308 | 370.8 |
| 254 | 634.2 | 878.2 | 135.8 | 1836.5 | 1289.6 | 195.3 | 904.4 | 151.2 | 1561.8 |
| 255 | 104.9 | 294.1 | 41.8 | 691.5 | 87 | 372.2 | 205.6 | 116.5 | 1995.4 |
| 256 | 859.7 | 310.3 | 98.5 | 401.3 | 162.4 | 559.4 | 1488.4 | 155 | 514.5 |
| 257 | 100.3 | 86.9 | 144.3 | 100.1 | 101.5 | 226.9 | 206.1 | 125.7 | 172.8 |
| 258 | 150.3 | 110.9 | 556.9 | 31 | 96.4 | 364.7 | 435.1 | 1.3 | 56.5 |
| 259 | 1252.6 | 22.5 | 14.5 | 71.4 | 242.1 | 53.8 | 318.2 | 52.7 | 72.5 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 260 | 153.5 | 162.6 | 101.6 | 142.8 | 117.7 | 170.7 | 162.1 | 100.8 | 140.1 |
| 261 | 284.9 | 426 | 473.6 | 367.3 | 285.2 | 584.1 | 539.9 | 954.5 | 991.9 |
| 263 | 387.1 | 519.8 | 1024.7 | 612.1 | 575.4 | 914.8 | 292.9 | 533.1 | 500.2 |
| 263 | 443.5 | 164.6 | 135.3 | 170.4 | 102.9 | 275.3 | 155 | 114.2 | 855 |
| 265 | 418.7 | 323.5 | 784.9 | 1333.6 | 878.4 | 655 | 510.2 | 758 | 222 |
| 266 | 365.9 | 238.9 | 328.9 | 335.6 | 303.6 | 404.8 | 506.9 | 500.5 | 362.2 |
| 256 | 232 | 48.3 | 68.8 | 144.5 | 86.5 | 161 | 74.1 | 81 | 263.7 |
| 268 | 789.4 | 359.6 | 223.2 | 619.4 | 600 | 188.7 | 380.9 | 385.6 | 616.2 |
| 269 | 29.5 | 23.7 | 45.2 | 37.7 | 42.9 | 141.6 | 73.5 | 56.7 | 27.4 |
| 270 | 318.9 | 330.1 | 251.6 | 249.2 | 249.1 | 310.4 | 140.9 | 168.8 | 208.5 |
| 271 | 239.6 | 226.6 | 272.6 | 240.3 | 278.2 | 416.5 | 239.5 | 262.2 | 282.4 |
| 272 | 529.2 | 443 | 502.9 | 351.9 | 421.7 | 790.6 | 423.8 | 540.7 | 390.3 |
| 273 | 2412.2 | 538 | 5359.4 | 2677 | 2738.3 | 2513.7 | 2568.7 | 3343.1 | 6538 |
| 274 | 860.6 | 187.6 | 415.1 | 523.9 | 197.7 | 408.9 | 297.8 | 848.1 | 225.6 |
| 275 | 810.8 | 120.9 | 107.2 | 223.4 | 149.2 | 360.4 | 213.5 | 132.2 | 481.2 |
| 276 | 3000.6 | 430.1 | 824.6 | 833 | 608.9 | 992.7 | 603.1 | 628.9 | 1368.2 |
| 277 | 143.4 | 150.6 | 195.4 | 175.5 | 161.1 | 288.2 | 217.2 | 177.8 | 321.7 |
| 278 | 1181.4 | 13.4 | 74.4 | 130.2 | 391.9 | 174.1 | 641.2 | 103.4 | 104.3 |
| 279 | 3167.2 | 3036.4 | 3286.5 | 3373.9 | 2195.2 | 1542 | 1783.5 | 1888.5 | 2287.8 |
| 280 | 2799.6 | 4372.9 | 8233.1 | 4783.5 | 3720.7 | 5792.3 | 3596.6 | 2627.6 | 3750.3 |
| 281 | 198.3 | 45.4 | 21.1 | 878.5 | 124.3 | 131.5 | 432.7 | 26.2 | 503.4 |
| 282 | 262.4 | 214.2 | 212.7 | 210.6 | 257.2 | 367.2 | 315.7 | 214.2 | 206 |
| 283 | 200.9 | 307.7 | 203.2 | 180 | 205.1 | 205.9 | 289.5 | 199.1 | 361.3 |
| 284 | 86.1 | 79 | 44.8 | 62.9 | 58.3 | 68.8 | 54.7 | 64.2 | 67.5 |
| 285 | 87.3 | 96.6 | 120.5 | 99.3 | 90.3 | 149.5 | 127.6 | 90 | 82.7 |
| 286 | 786.2 | 1509.2 | 1245.6 | 480.5 | 1419.1 | 1355.4 | 1398.3 | 986.2 | 463.7 |
| 287 | 130.9 | 88.7 | 115.7 | 133.4 | 101 | 206.6 | 124.4 | 129.4 | 95.2 |
| 288 | 121.5 | 110.5 | 133.2 | 130.9 | 129.7 | 239.3 | 147.4 | 130.8 | 98.9 |
| 289 | 817 | 97.8 | 1.2 | 264.2 | 130.1 | 209 | 209.4 | 93.1 | 162 |
| 291 | 128.3 | 84.3 | 184.7 | 105.8 | 127.5 | 140.4 | 106.4 | 110.2 | 175.4 |
| 292 | 325.5 | 712.8 | 127.1 | 172.1 | 269.8 | 126.4 | 1240.7 | 58 | 79 |
| 293 | 169.4 | 70.3 | 44.4 | 339.3 | 157.8 | 14.6 | 329.2 | 57 | 289.7 |
| 295 | 1114.8 | 74.6 | 91 | 388.6 | 176.3 | 215.7 | 303 | 129.7 | 708.9 |
| 296 | 1100.3 | 1475.6 | 747.1 | 869.3 | 1179.1 | 893.3 | 1093.5 | 664 | 1046.4 |
| 297 | 80.1 | 103.7 | 60.5 | 51.8 | 26.9 | 86 | 54.9 | 51.9 | 60.9 |
| 298 | 262.5 | 378 | 262.4 | 322.1 | 347.3 | 553.8 | 442.5 | 361.5 | 482.8 |
| 299 | 1244.6 | 880.6 | 660.4 | 1666.7 | 1114.4 | 999.2 | 1190.8 | 835.1 | 1409.5 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 300 | 221.5 | 287.8 | 78.8 | 1021.2 | 396.7 | 142.9 | 552.3 | 600.2 | 1201.1 |
| 301 | 345.6 | 176.4 | 916.7 | 791.8 | 996.1 | 2261.6 | 747.6 | 1879.6 | 867.3 |
| 302 | 30.1 | 11.5 | 9.6 | 1.2 | 9.5 | 52.1 | 8.2 | 31.1 | 20.4 |
| 303 | 659.7 | 792.4 | 1951.7 | 791.9 | 1024.8 | 1077.6 | 1052.7 | 1025.2 | 591.6 |
| 304 | 338.3 | 113.8 | 145.9 | 218.3 | 159.1 | 368.5 | 315.3 | 213.9 | 279.5 |
| 305 | 126.3 | 179.5 | 829.3 | 410.1 | 205.7 | 213 | 328.4 | 168.9 | 2353 |
| 306 | 137.6 | 129.3 | 119.7 | 111.1 | 136.2 | 360.3 | 171.7 | 145.8 | 121.9 |
| 307 | 116.1 | 98.9 | 137.8 | 143.6 | 128.3 | 221.3 | 155.3 | 111.8 | 121.6 |
| 308 | 1839.8 | 1691.4 | 3877.1 | 1787.2 | 1771.6 | 990 | 1269.4 | 1210.9 | 1188.7 |
| 310 | 746.5 | 649 | 365.2 | 639.4 | 842.4 | 331.6 | 428.2 | 770.7 | 283.9 |
| 311 | 148.4 | 156.4 | 215.3 | 228.8 | 178.6 | 94.4 | 194.5 | 255.6 | 341 |
| 312 | 960.4 | 1273.3 | 387.3 | 768.1 | 1668 | 504.5 | 546.2 | 772.8 | 843.7 |
| 313 | 116.8 | 110.3 | 118.2 | 691.9 | 301.6 | 156.3 | 349.8 | 120.8 | 1297 |
| 314 | 813.7 | 765 | 104 | 454.5 | 738.3 | 343 | 480.5 | 177.9 | 358 |
| 315 | 511.2 | 104.2 | 79.8 | 249.6 | 127.6 | 186.6 | 134.3 | 235.8 | 364 |
| 316 | 333.7 | 362.7 | 292.4 | 420 | 274.7 | 331.6 | 438.9 | 346.4 | 445.6 |
| 317 | 135.8 | 140 | 147.1 | 152.2 | 131.7 | 314.3 | 145.4 | 127.2 | 161.3 |
| 318 | 177.4 | 80.7 | 163.7 | 110.3 | 146.9 | 54.5 | 212.4 | 38.7 | 48.6 |
| 319 | 137.7 | 159.9 | 208.9 | 347.1 | 302.7 | 212.6 | 492.3 | 225 | 299.2 |
| 320 | 786.7 | 569.4 | 419.9 | 576.6 | 664 | 261.9 | 358.7 | 564.8 | 362.1 |
| 321 | 430.1 | 418.3 | 554.5 | 345.7 | 495.1 | 766.8 | 520.2 | 522.1 | 417.8 |
| 322 | 313.5 | 258.9 | 206 | 327.9 | 350.1 | 237.1 | 317.3 | 284.4 | 302.2 |
| 323 | 1640.1 | 3893.2 | 6623 | 4933.3 | 4083.6 | 4146.3 | 4123.6 | 2877.7 | 8765.3 |
| 324 | 191.6 | 52.6 | 89.7 | 451.6 | 184.5 | 161.8 | 224.9 | 94.8 | 485.6 |
| 325 | 252.3 | 394.8 | 205 | 336.6 | 342.2 | 306 | 382.9 | 200 | 472.4 |
| 326 | 6503.5 | 4515.1 | 5659.1 | 3116.9 | 4025.8 | 4887.6 | 5753.4 | 5207.4 | 3124.3 |
| 327 | 32.7 | 114.3 | 47.6 | 44.2 | 34.9 | 28.1 | 45.1 | 32.7 | 122 |
| 328 | 572.1 | 311.6 | 141.9 | 444 | 207.9 | 70.7 | 242.6 | 305.2 | 432.5 |
| 329 | 55.7 | 56.6 | 98.4 | 87.9 | 54.4 | 253.9 | 95 | 87.8 | 62.1 |
| 330 | 248.9 | 21.4 | 18.4 | 33.3 | 71.6 | 70.8 | 50.4 | 26.1 | 41.6 |
| 331 | 262.5 | 211 | 258.2 | 272.6 | 238.9 | 488.3 | 289.6 | 242.5 | 276.5 |
| 333 | 527 | 469.3 | 712.6 | 701.7 | 769.3 | 702.2 | 868.3 | 980.3 | 1069.6 |
| 334 | 178.8 | 178 | 143.4 | 202.5 | 157.4 | 217.1 | 144 | 129.4 | 147.4 |
| 335 | 109.7 | 80.4 | 65 | 59.9 | 71 | 237.8 | 69.3 | 69.9 | 140.5 |
| 336 | 478.1 | 1866 | 439.4 | 413 | 717.7 | 515.6 | 725.6 | 440.9 | 491.5 |
| 337 | 250.5 | 86.1 | 605.5 | 216 | 112.1 | 769 | 301 | 201.7 | 436.9 |
| 338 | 207.4 | 381.7 | 182.5 | 222.8 | 212.2 | 291.2 | 398.5 | 122.7 | 422.9 |

(continued)

| 3d | | | | | | | | | |
|---|---|---|---|---|---|---|---|---|---|
| SEQ ID NO: | 840TT | 842TT | 862TT | 863TT | 864TT | 865TT | 866TT | 867TT | 869TT |
| 339 | 491.6 | 370.5 | 242.7 | 371.6 | 357.1 | 291.6 | 301 | 329.6 | 333.1 |
| 340 | 262.2 | 228.9 | 221.7 | 277.5 | 209.8 | 433.8 | 234.6 | 244 | 238.8 |
| 341 | 553.7 | 859.2 | 680.7 | 749.1 | 833.5 | 945.2 | 877.8 | 1040.5 | 836.3 |
| 343 | 82.4 | 129.6 | 166.7 | 112.2 | 227 | 121.6 | 417.5 | 99.3 | 261.1 |
| 344 | 1494.7 | 1234.1 | 923 | 1174 | 1290.7 | 695.2 | 1049.8 | 1243.6 | 776.1 |
| 345 | 229.2 | 243.3 | 208 | 263.4 | 197.6 | 424 | 253.1 | 228.2 | 190 |
| 347 | 2522.7 | 2002 | 1475.1 | 3154.4 | 2419.4 | 3643.7 | 3353.4 | 2213 | 2257.1 |
| 348 | 5357.3 | 8676.5 | 4846.9 | 7151 | 6595.2 | 9416.2 | 8225.1 | 8754.7 | 7625.2 |
| 349 | 199 | 234.6 | 342.6 | 366.5 | 251 | 342.4 | 222.3 | 330.6 | 366.8 |
| 350 | 425.9 | 228.7 | 186 | 194.8 | 307.5 | 295.9 | 344.4 | 186.6 | 478.3 |
| 351 | 639.7 | 438.8 | 120.8 | 247.4 | 113.3 | 139.6 | 323 | 154.5 | 311.5 |
| 352 | 383.1 | 438.1 | 904.1 | 394.6 | 424.3 | 618.5 | 412.3 | 428.9 | 250.7 |
| 353 | 440.7 | 413.1 | 270.3 | 281.2 | 203 | 315.4 | 237.6 | 265.9 | 206.9 |

| 3e | | | | | | | | |
|---|---|---|---|---|---|---|---|---|
| SEQ ID NO: | 871TT | 879TT | 881TT | 882TT | 883TT | 884TT | 885TT | 886TT |
| 354 | 116.3 | 981.8 | 502.2 | 324.4 | 156.7 | 786.8 | 376.6 | 642.7 |
| 355 | 66.1 | 8420.7 | 2645.1 | 6152 | 13553.8 | 2564.7 | 3412.8 | 12344.6 |
| 356 | 338.5 | 1495.5 | 623.7 | 501.4 | 743.6 | 903.3 | 1811.4 | 741.6 |
| 357 | 443.4 | 1347.3 | 566 | 608.2 | 679.7 | 484.6 | 1559 | 983.6 |
| 358 | 700.6 | 1921.2 | 2381.5 | 2373.2 | 1674.3 | 2027.6 | 3756.9 | 1590.4 |
| 359 | 376 | 185.1 | 264.6 | 206.9 | 308.2 | 331.9 | 281.8 | 247.9 |
| 362 | 655.5 | 1294.9 | 729.9 | 1088.1 | 864.4 | 1725.1 | 1214.9 | 1246.6 |
| 360 | 266.2 | 611.8 | 464.8 | 543.6 | 452.1 | 537.3 | 693.4 | 474.9 |
| 361 | 152.3 | 149.9 | 338.7 | 186.5 | 167.3 | 256.5 | 235.5 | 312.1 |
| 363 | 570.7 | 493.8 | 575.1 | 513.3 | 517.2 | 615.3 | 759.7 | 454.4 |
| 1 | 130.2 | 622.4 | 1944.7 | 1584.5 | 1981.6 | 1283.3 | 420.9 | 1894.3 |
| 2 | 411.7 | 1993.4 | 3797.1 | 2512.7 | 4511.4 | 2897.7 | 1254 | 3862.9 |
| 7 | 372.9 | 193.8 | 680.5 | 596.1 | 614.8 | 129.5 | 2.7 | 504.2 |
| 8 | 2363.3 | 511.5 | 342.5 | 363.9 | 358 | 497.4 | 221.6 | 355.6 |
| 9 | 381.6 | 1198.7 | 1487.5 | 781.6 | 985.4 | 2024.6 | 3933.7 | 1448.2 |
| 10 | 171.5 | 160.4 | 133.2 | 137.6 | 129.1 | 154.3 | 182.4 | 107 |
| 11 | 324 | 858.2 | 937 | 906.4 | 953.3 | 1879.7 | 1481 | 2251.2 |
| 13 | 126.1 | 303.3 | 531.1 | 319.9 | 664.9 | 496 | 223.2 | 472.7 |
| 14 | 295.2 | 932.1 | 1713.8 | 1521.7 | 2129.6 | 2134.5 | 1840.1 | 2225.4 |
| 15 | 95.6 | 2064.6 | 1333.1 | 540.9 | 265.7 | 569.7 | 709.8 | 1795.9 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 16 | 511.2 | 3919.7 | 2909.7 | 3131.8 | 3773.6 | 3125.2 | 3619.3 | 3331.5 |
| 17 | 214.6 | 800.5 | 1475.1 | 1075.3 | 1483.6 | 1162 | 1482.8 | 1281.8 |
| 19 | 198 | 274.5 | 768.1 | 268.8 | 208 | 509.4 | 276.1 | 570.1 |
| 20 | 1988.3 | 739.3 | 319.5 | 316.5 | 192 | 142.3 | 242.8 | 575.4 |
| 21 | 34.6 | 2492 | 1542.8 | 2495.1 | 1851.5 | 2668.6 | 1631.9 | 2770.3 |
| 22 | 1078.1 | 4211.3 | 4116 | 3543.7 | 3242.1 | 4723.4 | 3467.8 | 4881.8 |
| 23 | 1.1 | 825.2 | 1432.3 | 370.9 | 163.9 | 1192.4 | 221.9 | 1517 |
| 24 | 74.6 | 391.1 | 433 | 388.2 | 211.7 | 258.7 | 300.5 | 572.7 |
| 26 | 48.4 | 921.9 | 4466.3 | 587.5 | 349.2 | 1513.7 | 644.9 | 1870.3 |
| 27 | 495.1 | 654.3 | 1266.2 | 1076 | 1461.5 | 696.9 | 334.7 | 1531.4 |
| 29 | 381.5 | 152.8 | 91.8 | 35.9 | 162.7 | 135.1 | 152 | 238.5 |
| 30 | 31.3 | 686.6 | 444 | 223.3 | 222 | 321.8 | 356.5 | 825.6 |
| 31 | 635.6 | 18297.7 | 14048.6 | 8744.1 | 15394.7 | 17798.2 | 16472.5 | 12208.2 |
| 33 | 439.9 | 7923.9 | 4124.5 | 3804.6 | 3932.5 | 2361.7 | 3149.1 | 7173.6 |
| 34 | 3213.6 | 825.9 | 1104.9 | 697 | 285.8 | 603.4 | 436.7 | 1025.5 |
| 35 | 22.6 | 2143.6 | 1770.3 | 1187.9 | 1220.4 | 1620.3 | 1651.3 | 529 |
| 36 | 492.1 | 322.5 | 1635.1 | 387.3 | 554.6 | 818.8 | 268.4 | 1021.4 |
| 37 | 429.6 | 140.1 | 245.5 | 191.1 | 224.1 | 241.8 | 207.1 | 178.5 |
| 38 | 237.9 | 1844.2 | 2220.8 | 794.4 | 644.7 | 984 | 292.1 | 3477.4 |
| 40 | 451.5 | 617.3 | 495.8 | 1186.4 | 725.4 | 800.6 | 880.8 | 1036.1 |
| 41 | 252.1 | 1258.8 | 943 | 4956.1 | 2394.2 | 566.6 | 987.6 | 3025.4 |
| 42 | 579.4 | 532.8 | 258.1 | 1104 | 396.5 | 335.9 | 491.3 | 504 |
| 43 | 1029 | 384.9 | 273.6 | 344 | 416.5 | 403.9 | 517.2 | 325.7 |
| 44 | 565.3 | 1802.4 | 1501.6 | 1596.1 | 1100.9 | 1275.9 | 1583.2 | 1199 |
| 45 | 1500.8 | 1346.9 | 1175.4 | 1573.6 | 2053.9 | 971.8 | 332 | 1783.9 |
| 46 | 307.7 | 539 | 343 | 380.7 | 553.4 | 414.6 | 398.2 | 340.8 |
| 47 | 67.8 | 1213 | 521.9 | 742.8 | 498.1 | 507 | 1389.1 | 810.8 |
| 48 | 6.2 | 645.9 | 561.5 | 325.6 | 92.6 | 189.4 | 537.5 | 190.6 |
| 49 | 192.1 | 1573.1 | 2319.6 | 1719.2 | 1210.4 | 6469.1 | 1691.6 | 1799.3 |
| 50 | 2 | 1030.9 | 954.7 | 379.1 | 309.7 | 799.1 | 597.4 | 2116.3 |
| 51 | 772.5 | 259.7 | 304 | 170.3 | 108.1 | 1357.1 | 408.8 | 394.9 |
| 52 | 193.2 | 298.8 | 343.6 | 347.9 | 276 | 289.4 | 348.2 | 335.9 |
| 53 | 153.5 | 1632.6 | 2144.7 | 2293.9 | 2026.5 | 1732.8 | 446.5 | 1982.9 |
| 54 | 809.7 | 1147.3 | 1387.6 | 2014.6 | 1631.7 | 1524.6 | 1231.9 | 2093.8 |
| 55 | 147.7 | 86.2 | 101.7 | 108.3 | 85.2 | 103.3 | 116.6 | 62.5 |
| 56 | 130 | 94.8 | 277.7 | 121.8 | 341.8 | 98.5 | 180.9 | 241.4 |
| 57 | 777.3 | 1976.6 | 14161.4 | 3909.2 | 3643.6 | 667.8 | 1053.7 | 5922.8 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 58 | 1225.7 | 5418.7 | 14855.8 | 6952.4 | 4309.6 | 3516.6 | 6571.6 | 9242 |
| 59 | 271.6 | 3644.3 | 3754.4 | 4801.4 | 3817.3 | 6657.4 | 4444.2 | 3360.8 |
| 60 | 159.2 | 218.9 | 601.1 | 529.4 | 311.4 | 171.9 | 231.9 | 506.7 |
| 61 | 138.9 | 159.4 | 118.4 | 73.6 | 104.8 | 116.1 | 164.5 | 81.1 |
| 62 | 3244.1 | 5818 | 1646.1 | 2645.7 | 3666.2 | 3358 | 2535.7 | 4029.7 |
| 63 | 157.2 | 628.9 | 667.9 | 804.6 | 516.3 | 778 | 703.2 | 578.3 |
| 64 | 994.5 | 119.1 | 251.9 | 202.5 | 258.2 | 236.6 | 151.1 | 722.1 |
| 65 | 35.2 | 209.3 | 234.7 | 217.1 | 113.7 | 118.8 | 146.7 | 305.4 |
| 66 | 437.5 | 2105.5 | 1849.4 | 2646.8 | 2322.7 | 1754.7 | 1801.8 | 1830.2 |
| 67 | 13299.5 | 26691.3 | 29363.4 | 21948.4 | 30861.2 | 27520.3 | 32791 | 27456.3 |
| 68 | 1527.1 | 379.2 | 840.8 | 286.8 | 238.8 | 383.7 | 53.6 | 2104.6 |
| 69 | 5084.1 | 1418.5 | 1727.3 | 964.1 | 782.1 | 2036.1 | 478 | 3532.1 |
| 70 | 9067.8 | 5289.8 | 5151.5 | 5378.2 | 4923.7 | 7248.8 | 5765.7 | 6685.7 |
| 71 | 356.7 | 3218.2 | 6059.4 | 4739.2 | 7588 | 3114 | 2518 | 4665.5 |
| 72 | 126.9 | 2024.9 | 3174.2 | 2185.2 | 2596.3 | 2512.4 | 2145.9 | 1991.3 |
| 73 | 163.4 | 917.4 | 942.3 | 785.8 | 642.5 | 1021.7 | 1109.2 | 1059.2 |
| 75 | 234.3 | 480.2 | 1234.8 | 2124.7 | 1532.8 | 329.3 | 830.5 | 2611.1 |
| 76 | 1819.9 | 1037.3 | 1473 | 1162.8 | 2498.4 | 959.8 | 921.6 | 1764.5 |
| 77 | 5259 | 3101.8 | 1035.9 | 1814.1 | 885.5 | 1999.1 | 3528.7 | 1319.6 |
| 78 | 568.7 | 520.5 | 454.4 | 245.9 | 225.2 | 250.6 | 256.7 | 390.8 |
| 79 | 402.6 | 303.3 | 288.4 | 416.4 | 496.9 | 377.6 | 309.8 | 241.7 |
| 80 | 141.1 | 1486.2 | 1527.9 | 1160.9 | 747.9 | 1195.7 | 1756.4 | 792.8 |
| 81 | 189.9 | 13371 | 7841.3 | 9074.6 | 16493.4 | 8121 | 5097.9 | 11272.1 |
| 82 | 435.3 | 141.8 | 224.9 | 197.1 | 249.7 | 31 | 6.8 | 170.9 |
| 84 | 71.2 | 138.6 | 259.2 | 151.7 | 138.9 | 415.1 | 303 | 326.8 |
| 85 | 187.8 | 1315 | 612.4 | 336.4 | 240.8 | 318.3 | 399.8 | 1550.2 |
| 86 | 93.5 | 90.2 | 120.7 | 294.3 | 724.1 | 302.7 | 89.1 | 261.7 |
| 87 | 539.5 | 1122.1 | 581.5 | 1395.1 | 1476 | 1619.4 | 1043.7 | 2328.9 |
| 88 | 3161.6 | 561.1 | 1162.2 | 632.3 | 466 | 750.6 | 597.3 | 1050.6 |
| 89 | 317.9 | 237.2 | 318.3 | 196.3 | 337.5 | 343.7 | 255.7 | 239.7 |
| 90 | 111.8 | 266.6 | 953.6 | 334.9 | 123.2 | 226 | 325.2 | 129.6 |
| 91 | 1023.4 | 3728.1 | 4506.6 | 4033.4 | 2043.2 | 2095 | 2191.9 | 3082.3 |
| 92 | 2374.9 | 4865.1 | 6296.6 | 8454.9 | 11642.6 | 11587.7 | 9943.6 | 10568 |
| 93 | 103.8 | 133.1 | 109.8 | 121.6 | 41.7 | 69.9 | 17.1 | 166 |
| 94 | 59.2 | 296.8 | 784.9 | 944.1 | 600.5 | 776.8 | 481.1 | 1257.8 |
| 95 | 69 | 126.3 | 450.5 | 453.8 | 310.8 | 189.3 | 165.4 | 142.4 |
| 96 | 147.8 | 682.4 | 387.8 | 387.3 | 404.3 | 357.9 | 565.3 | 420.4 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 97 | 204.3 | 883.1 | 454.1 | 708.8 | 421.4 | 468 | 686.4 | 732.8 |
| 98 | 728.4 | 10.5 | 42.6 | 72.2 | 43.5 | 29.7 | 53 | 36.4 |
| 99 | 210.6 | 821.1 | 369.4 | 814.2 | 595.6 | 485.9 | 696.6 | 541.1 |
| 100 | 995.9 | 102.8 | 3585.7 | 89.5 | 34.3 | 2798.5 | 112 | 3456 |
| 101 | 133.4 | 146.6 | 270.3 | 174.9 | 310.3 | 198.2 | 121.7 | 192.7 |
| 102 | 190.6 | 110.7 | 244 | 152.9 | 172.3 | 180.7 | 158 | 123.8 |
| 103 | 379.6 | 430.7 | 535.3 | 399.6 | 560.3 | 572.8 | 586.9 | 406.8 |
| 104 | 252 | 69.6 | 93.2 | 189.4 | 28.6 | 67.4 | 16.2 | 92.3 |
| 105 | 363.6 | 168.9 | 116.4 | 217.8 | 70.2 | 146.4 | 118.2 | 171.8 |
| 106 | 442.3 | 95.1 | 136.6 | 154 | 56.3 | 79.8 | 12.9 | 109.4 |
| 107 | 301 | 538.7 | 356.6 | 408.6 | 301.5 | 322.4 | 420.8 | 596.8 |
| 108 | 255.8 | 236.3 | 168 | 137.3 | 106.8 | 179.7 | 237 | 170.7 |
| 109 | 169.9 | 1482.1 | 544.7 | 1597 | 294.1 | 1205.6 | 1691 | 683.9 |
| 110 | 322.7 | 1903.7 | 1404.2 | 4278.2 | 691.9 | 2791.8 | 2817.9 | 2504.6 |
| 111 | 252.7 | 25.4 | 233.3 | 24.4 | 63.4 | 101.2 | 47.1 | 118.3 |
| 112 | 633.2 | 1314.2 | 1405.6 | 2124.5 | 1859.3 | 1085.3 | 587.6 | 1601.9 |
| 113 | 164.4 | 330.6 | 333.5 | 268.5 | 218.6 | 285.2 | 237.3 | 350.6 |
| 114 | 88.6 | 309.3 | 1164.6 | 333.2 | 513.7 | 630.7 | 664.7 | 778.3 |
| 115 | 326.5 | 1299.5 | 1101.1 | 1198.8 | 1051.2 | 817.4 | 1102.2 | 1084.6 |
| 116 | 252.3 | 360.5 | 666.1 | 1347.3 | 817.6 | 327.9 | 554.6 | 825.9 |
| 117 | 42.1 | 101.1 | 493.9 | 114.6 | 74 | 399.7 | 133.8 | 234.5 |
| 118 | 1176.7 | 116.7 | 300.6 | 130.5 | 200.8 | 470.8 | 207.3 | 112.5 |
| 119 | 98.3 | 184.2 | 281.1 | 245.5 | 317.6 | 213 | 191.2 | 336 |
| 120 | 99.5 | 98.8 | 155.8 | 124.7 | 95.6 | 98.9 | 123.9 | 78.9 |
| 121 | 234.8 | 2992.4 | 4320.2 | 4055.8 | 3020 | 5336.5 | 3158.4 | 5803.1 |
| 122 | 645.9 | 446.7 | 248.2 | 356.4 | 312.1 | 428.7 | 329.5 | 346.1 |
| 123 | 300.8 | 313.4 | 352.6 | 504.1 | 413.5 | 386.8 | 444 | 417.5 |
| 124 | 4390.2 | 96.1 | 118.6 | 125.8 | 161.7 | 134.4 | 182.6 | 94.9 |
| 125 | 1484.8 | 805.4 | 27732 | 982.2 | 7375.5 | 984.2 | 1310.9 | 807.1 |
| 126 | 278.6 | 56.9 | 192.9 | 24.9 | 31.8 | 77.8 | 104.9 | 66.8 |
| 127 | 114.5 | 332.1 | 225.5 | 171.6 | 237.5 | 193.8 | 339.3 | 225.1 |
| 128 | 318.6 | 181.2 | 386.4 | 213 | 69850.3 | 108521.8 | 356.1 | 93954.5 |
| 129 | 176.1 | 1916.3 | 1120.7 | 2375.9 | 2049.1 | 1464.3 | 1602.6 | 1660 |
| 130 | 114.6 | 735.8 | 381 | 595.7 | 599.3 | 446.9 | 434.4 | 456.6 |
| 131 | 236.1 | 156.9 | 297.1 | 426.8 | 385.2 | 268.2 | 149.3 | 279.8 |
| 132 | 169.8 | 303.3 | 464.3 | 423.7 | 368.6 | 256 | 428.6 | 304.5 |
| 133 | 102.5 | 245.3 | 224.6 | 394 | 186.7 | 265.2 | 300 | 269.3 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 134 | 175.1 | 516.6 | 316.3 | 534.3 | 524 | 626.6 | 679.7 | 687.4 |
| 135 | 170 | 964.7 | 971.6 | 756.7 | 562.2 | 683.1 | 1544.5 | 931 |
| 138 | 145.9 | 173.1 | 201.3 | 127.5 | 189.1 | 187.4 | 173.9 | 166 |
| 139 | 66.8 | 286.4 | 170 | 167 | 270.8 | 159 | 281 | 340.1 |
| 140 | 26 | 16.5 | 135.6 | 163.6 | 1.2 | 183.8 | 98.2 | 1064.1 |
| 141 | 407.3 | 178 | 380.3 | 594.8 | 275 | 1127.7 | 586.1 | 346.3 |
| 144 | 370.2 | 23200.8 | 7070.4 | 20031.2 | 20055.7 | 5695.1 | 21008 | 16416.4 |
| 145 | 203 | 197.5 | 215.6 | 241.1 | 234 | 295.8 | 284.7 | 197.3 |
| 146 | 299.6 | 606.7 | 853.8 | 1054.9 | 249.3 | 683.3 | 966.5 | 411.5 |
| 147 | 34.5 | 138.1 | 361.4 | 219.1 | 120.1 | 69 | 110.2 | 51.6 |
| 148 | 200.3 | 1072.9 | 641.5 | 371.8 | 309.3 | 1298 | 734.4 | 563.6 |
| 149 | 147.1 | 811.8 | 330.1 | 243.5 | 389.9 | 584.1 | 960.6 | 374.5 |
| 150 | 243.6 | 194.2 | 614.8 | 339.8 | 227.4 | 390.5 | 510.2 | 508.8 |
| 151 | 103.4 | 200.9 | 536 | 659.6 | 1097.9 | 831.2 | 572.5 | 1463 |
| 153 | 102.7 | 134.4 | 117.8 | 319.9 | 659.8 | 147 | 158.2 | 276.8 |
| 154 | 64.7 | 5340.9 | 7684.3 | 4496.6 | 6819 | 3595.6 | 1313.4 | 7444 |
| 155 | 155.1 | 604.1 | 224.8 | 527.6 | 443.5 | 980.8 | 352 | 708.4 |
| 156 | 117.9 | 162.1 | 382.4 | 213.5 | 503.7 | 174 | 219.3 | 257.1 |
| 157 | 134.1 | 215.4 | 212.5 | 227.7 | 257.4 | 258.4 | 309.6 | 331.4 |
| 158 | 857.5 | 149.9 | 598.8 | 544.5 | 850.2 | 506.6 | 301.4 | 728.3 |
| 159 | 1385.8 | 1366 | 1517.3 | 1101.7 | 1387.1 | 1799.8 | 2020.5 | 1476 |
| 161 | 950.1 | 6758.6 | 7657.2 | 10885 | 16984 | 4705.2 | 7562.3 | 6065.7 |
| 162 | 12.5 | 256.2 | 584.1 | 235.7 | 456.1 | 369.3 | 231.2 | 451.5 |
| 164 | 47.5 | 347.7 | 1040.5 | 4140.2 | 1526.9 | 2205.5 | 1430.7 | 7468.9 |
| 166 | 4175.2 | 1086.4 | 1301.1 | 700.5 | 641.4 | 1360.7 | 585.8 | 1191.3 |
| 167 | 545.5 | 254.9 | 1335.1 | 355.9 | 1787.4 | 118.5 | 231.5 | 771.1 |
| 168 | 140.6 | 1535.5 | 1213 | 549.9 | 594.1 | 1616.8 | 2190.3 | 639.2 |
| 169 | 582.9 | 12292.6 | 13216.5 | 15240.1 | 14867.1 | 7648.8 | 9941.3 | 13159.9 |
| 170 | 857.8 | 2119.5 | 2344.7 | 3361.9 | 2260.9 | 2642.5 | 1920 | 3323.2 |
| 171 | 1.1 | 1063.9 | 967.8 | 79.8 | 105 | 514 | 339.5 | 801 |
| 172 | 489.3 | 575.3 | 552.3 | 483.9 | 608.6 | 582.7 | 664.1 | 514.2 |
| 173 | 168.3 | 410.3 | 1713.9 | 878.7 | 690.8 | 1123.5 | 254.3 | 2373.9 |
| 176 | 50.7 | 173.8 | 993.1 | 155.4 | 1107.2 | 1814.1 | 400.9 | 1423.1 |
| 177 | 610.5 | 234 | 350.8 | 201.7 | 455.2 | 147.8 | 113.6 | 315.4 |
| 178 | 670.2 | 457.3 | 1924 | 1409.8 | 1033.8 | 1365.3 | 281.5 | 700.6 |
| 179 | 188.9 | 540.8 | 1136 | 1826.7 | 836.8 | 964.6 | 165.7 | 1383.2 |
| 180 | 208.1 | 750.5 | 880.8 | 997.3 | 460.6 | 628.8 | 717.2 | 618.1 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 181 | 9102.8 | 4805.7 | 6228.9 | 4111.8 | 3157.6 | 5112.8 | 5426.8 | 4235.7 |
| 182 | 244.4 | 524.8 | 434.5 | 395.3 | 395.9 | 340.8 | 570.4 | 455 |
| 183 | 1.1 | 286.3 | 920.2 | 156 | 69.6 | 540.1 | 211.4 | 691 |
| 184 | 202.5 | 365.3 | 361.1 | 2170.5 | 3042.7 | 341.2 | 479.3 | 909.8 |
| 185 | 707.4 | 2338.2 | 5269.7 | 5295.8 | 3116 | 6542.9 | 3238.5 | 6677.1 |
| 186 | 1338.6 | 98.2 | 262.6 | 165.5 | 183.3 | 170.6 | 131.9 | 218.6 |
| 187 | 301.2 | 421.4 | 792.2 | 247.6 | 123.3 | 291.1 | 366.9 | 720.2 |
| 188 | 54.4 | 507.9 | 399.8 | 687.8 | 469.2 | 348.4 | 605.1 | 684.6 |
| 189 | 68.7 | 209 | 287.2 | 379.3 | 305.1 | 161.4 | 160.7 | 404.9 |
| 190 | 199.8 | 242.2 | 483.5 | 455.5 | 631.9 | 241 | 71.1 | 375.7 |
| 191 | 1726.7 | 4039 | 3532.7 | 2210.1 | 1524.2 | 4609 | 6889.4 | 3737.3 |
| 192 | 89.6 | 89.6 | 157.2 | 211.4 | 92.6 | 233.7 | 145.8 | 105.4 |
| 193 | 85 | 216.9 | 219.4 | 119.4 | 69.2 | 392.2 | 176.5 | 239.5 |
| 194 | 49.9 | 282 | 251.7 | 297.8 | 199.9 | 135 | 172.5 | 243.3 |
| 195 | 712.1 | 117.7 | 174.3 | 152.9 | 85.3 | 174.3 | 73.7 | 152.1 |
| 196 | 2873.3 | 845.8 | 1650.6 | 1499.5 | 1608.9 | 962 | 584.5 | 1404 |
| 197 | 483.3 | 253.5 | 190.9 | 146 | 112.4 | 139 | 144.8 | 141.2 |
| 198 | 126.6 | 4231 | 1176.9 | 1159.9 | 1263.8 | 999 | 925 | 2035.1 |
| 199 | 87.2 | 9967.3 | 4014.3 | 8315.9 | 19492.1 | 4175.4 | 4540.2 | 17229.4 |
| 200 | 344.7 | 460.3 | 1008.1 | 823.2 | 437.1 | 394.5 | 655.2 | 945.4 |
| 201 | 122.7 | 1085.1 | 434.8 | 378.2 | 229.8 | 766.3 | 506.3 | 515.7 |
| 203 | 678.5 | 1088.3 | 1322.9 | 1133.7 | 3167.7 | 436.2 | 563 | 3185.7 |
| 204 | 634.2 | 3003.4 | 1397.7 | 1339.9 | 1300.3 | 1299.2 | 2900.2 | 1073 |
| 205 | 946.3 | 5589.7 | 3415.4 | 3931.9 | 4602.2 | 5034.5 | 4943.8 | 6059.1 |
| 206 | 278.8 | 299.7 | 268.4 | 293.8 | 350.2 | 398.3 | 635.4 | 241.1 |
| 207 | 125.7 | 79.6 | 212.3 | 46.6 | 106.6 | 58.1 | 57.3 | 679.7 |
| 208 | 5.4 | 3861.8 | 679.2 | 3099.7 | 462.3 | 711 | 799 | 604.6 |
| 209 | 648.2 | 26.6 | 610.8 | 71.6 | 104.3 | 77.3 | 72 | 161.4 |
| 211 | 5817.8 | 134.3 | 575.9 | 84.6 | 135.1 | 1200.4 | 233.2 | 1507.1 |
| 212 | 1458.6 | 689.4 | 1135 | 1092.6 | 988.6 | 986.3 | 460.6 | 1341.5 |
| 213 | 7576.6 | 117.6 | 121.9 | 100.6 | 96.3 | 151.6 | 183.1 | 136.2 |
| 215 | 71.8 | 73.2 | 323.2 | 71.1 | 161.2 | 100.5 | 30.8 | 676.7 |
| 216 | 415.9 | 276.5 | 268.1 | 173.7 | 199.3 | 277.1 | 434.2 | 282.3 |
| 217 | 78.7 | 959.5 | 2746.7 | 1024.9 | 2616.6 | 671.5 | 359.6 | 850.8 |
| 218 | 268.3 | 172.1 | 161.1 | 189.2 | 123.2 | 160.7 | 128.5 | 186.8 |
| 220 | 134.5 | 72.8 | 184.9 | 96.1 | 84.3 | 650.4 | 100.4 | 81.9 |
| 221 | 207.5 | 1438 | 1268.5 | 2111.3 | 963.7 | 1155.3 | 1048.4 | 1581.4 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 223 | 308.1 | 673.6 | 497.3 | 535.5 | 748.5 | 534.7 | 710.2 | 869.9 |
| 227 | 474 | 3740.6 | 3123.8 | 1451.8 | 740.1 | 1207.8 | 1221.4 | 3703.7 |
| 228 | 553.9 | 202.8 | 342.4 | 247.9 | 322.9 | 245.3 | 315.9 | 260.5 |
| 229 | 978.6 | 365 | 204.1 | 229.3 | 104.9 | 176.7 | 197.5 | 149.4 |
| 230 | 157 | 4381.3 | 4364.6 | 3469 | 4117.2 | 2984.9 | 4092.3 | 2935.6 |
| 231 | 299.2 | 338.1 | 519.6 | 436.9 | 1304.6 | 261.4 | 183.1 | 1209.9 |
| 232 | 1.2 | 479.7 | 2967.9 | 397.7 | 283.8 | 1161.6 | 447.9 | 1442.7 |
| 233 | 1408.3 | 17920.3 | 13126.5 | 7241.8 | 19318.8 | 13487.2 | 18348.9 | 15152.5 |
| 235 | 22.6 | 659.3 | 624.9 | 172.2 | 155.8 | 342.8 | 152.7 | 485.3 |
| 236 | 244.8 | 153.5 | 397.3 | 304.6 | 449.7 | 331.3 | 200.6 | 282.5 |
| 237 | 841.8 | 1675 | 2348.2 | 1931.5 | 1633.1 | 2253.2 | 749.1 | 1687.1 |
| 238 | 606.8 | 847.7 | 670.6 | 582.6 | 625.6 | 723.4 | 726.5 | 667.2 |
| 239 | 455.9 | 304.4 | 1179.8 | 492.8 | 201.3 | 464.9 | 311.9 | 376.9 |
| 240 | 756.3 | 795.3 | 1590.4 | 747.3 | 330.3 | 1193.3 | 846 | 709.6 |
| 241 | 1604.8 | 957.3 | 2984.8 | 1412.5 | 619.3 | 2269.1 | 981.8 | 1321 |
| 242 | 13722.5 | 46283.3 | 32477.3 | 33142.3 | 38026.7 | 42087.5 | 49249 | 39172.5 |
| 243 | 322.6 | 586.6 | 483.3 | 483.6 | 499.4 | 723.7 | 534 | 603.1 |
| 244 | 80.8 | 1208.5 | 771.3 | 1636.9 | 1032.2 | 928.2 | 507.9 | 553.8 |
| 246 | 167.7 | 2506.3 | 1683.7 | 2500.3 | 1675.8 | 557.3 | 1133.6 | 1677.5 |
| 247 | 116.8 | 115.5 | 128.4 | 104.8 | 125.8 | 135.7 | 147.3 | 96.5 |
| 248 | 309.3 | 1728.9 | 1916.3 | 3434.3 | 3874.7 | 1004.6 | 2401.7 | 5089.3 |
| 249 | 432 | 681.6 | 804.8 | 479.8 | 417.3 | 1208.2 | 1265.1 | 742.5 |
| 251 | 134 | 671 | 368.8 | 2493.8 | 784.3 | 1646.7 | 587.9 | 1794.9 |
| 253 | 375.8 | 544.2 | 623.3 | 589.2 | 333.1 | 383.4 | 859.9 | 310.8 |
| 254 | 716.8 | 989.8 | 1029.3 | 1067.7 | 790.5 | 817 | 991.7 | 1396.7 |
| 255 | 777.9 | 706 | 2286.6 | 1766.1 | 1100.5 | 1765.9 | 286 | 948.3 |
| 256 | 645.4 | 3136 | 3129.7 | 3456.4 | 1905.4 | 1995.5 | 3000.3 | 2611 |
| 257 | 107.8 | 348.2 | 360.1 | 289.9 | 300.9 | 240.8 | 403 | 750 |
| 258 | 83.1 | 3074.5 | 844.5 | 2046.8 | 1476.7 | 3628.7 | 3511.2 | 2536.1 |
| 259 | 1.2 | 668.5 | 1027.2 | 351 | 524.2 | 1117.3 | 356.7 | 500 |
| 260 | 185.4 | 108.7 | 158.6 | 251.8 | 168.6 | 182 | 256.7 | 116.2 |
| 261 | 73.8 | 130.7 | 187.3 | 61.6 | 210.2 | 242.6 | 130.1 | 258.1 |
| 263 | 919.6 | 1233.9 | 819.4 | 1299.4 | 572.7 | 912.1 | 1233 | 872.7 |
| 263 | 199.8 | 4626.1 | 3402.3 | 3087.9 | 3813.2 | 3582 | 6403.5 | 3036.9 |
| 265 | 310 | 125 | 156 | 135.2 | 128.1 | 234.7 | 96.8 | 155.8 |
| 266 | 381.6 | 899.6 | 433.3 | 530.4 | 242.9 | 358.6 | 641.2 | 241.1 |
| 256 | 130.8 | 141.8 | 211.4 | 344.3 | 408.2 | 195.6 | 211 | 388.4 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 268 | 155.9 | 135.7 | 783.8 | 251.7 | 990.9 | 145.6 | 77.9 | 262.5 |
| 269 | 70.4 | 137.9 | 127.4 | 87.5 | 920.7 | 144.7 | 106.4 | 123.1 |
| 270 | 906 | 197.1 | 177.3 | 148.3 | 194.1 | 214.8 | 161.2 | 198.9 |
| 271 | 197.7 | 411.2 | 613.2 | 278.3 | 1153.3 | 2568.1 | 824.5 | 1254.6 |
| 272 | 530.2 | 865.4 | 861.9 | 814.5 | 776.6 | 929.7 | 1402.4 | 1012 |
| 273 | 2.1 | 207.7 | 884.3 | 165.7 | 2070.8 | 969.7 | 200.6 | 1967.3 |
| 274 | 1050.2 | 932.1 | 1127.6 | 1151.3 | 1481.1 | 2100.5 | 1603.4 | 1010.4 |
| 275 | 277.8 | 674 | 1507.8 | 1511.2 | 956.4 | 1603.8 | 960.9 | 2338.9 |
| 276 | 496.5 | 4907.8 | 5071 | 1824.8 | 921.9 | 1584.4 | 1043 | 6199.7 |
| 277 | 159.6 | 251.2 | 318.9 | 445.6 | 355.2 | 318.1 | 503.6 | 277.7 |
| 278 | 15.3 | 1224.6 | 1434.2 | 602.7 | 1133.3 | 1898 | 1401.1 | 984.1 |
| 279 | 678.5 | 1074.4 | 1101.7 | 1030.5 | 1878 | 1425 | 1541.1 | 2160.3 |
| 280 | 1096.5 | 3203.9 | 2152.5 | 2126.2 | 3768.5 | 4309.6 | 7516.9 | 4768.7 |
| 281 | 38.3 | 590.9 | 605.4 | 938.5 | 832.1 | 257.9 | 315.3 | 385.4 |
| 282 | 407.5 | 416.5 | 704.2 | 1079.1 | 1604.5 | 485.4 | 482.3 | 1146.7 |
| 283 | 603.1 | 605 | 593.6 | 521.9 | 638.2 | 488.6 | 789.7 | 516.5 |
| 284 | 131.4 | 59.7 | 136.2 | 49.9 | 188.8 | 106.8 | 101.5 | 85 |
| 285 | 154.1 | 117 | 110.9 | 109.1 | 135.7 | 131.9 | 163 | 116 |
| 286 | 530.4 | 43.8 | 59.5 | 27.7 | 22.9 | 109.8 | 66.7 | 34.6 |
| 287 | 102.4 | 116.9 | 133.6 | 99.1 | 113.6 | 295 | 171.3 | 79.7 |
| 288 | 160.1 | 126.6 | 179.5 | 153.3 | 164.7 | 202.8 | 216.9 | 139.7 |
| 289 | 1536.2 | 4488.8 | 4944.9 | 1532.8 | 3646.8 | 5911.3 | 4344.5 | 8246.5 |
| 291 | 102.7 | 248.4 | 286.1 | 208.5 | 228.9 | 130.1 | 253.2 | 374 |
| 292 | 42.7 | 732.8 | 8793.1 | 18089.3 | 3318.5 | 3787.1 | 10588.9 | 8088.8 |
| 293 | 271.4 | 344.8 | 740.3 | 493 | 462.9 | 499.8 | 453.7 | 383.2 |
| 295 | 727.5 | 890.4 | 2593.2 | 1314 | 3115.5 | 310.9 | 729.5 | 1143.9 |
| 296 | 887.9 | 2346.4 | 1719.1 | 1863.5 | 1576.8 | 2166.6 | 2085.1 | 1930.4 |
| 297 | 96.5 | 59.7 | 110.1 | 83.1 | 71.1 | 60.1 | 112 | 82.2 |
| 298 | 546.1 | 730.6 | 785.4 | 968.7 | 730.8 | 563 | 1034.8 | 859.8 |
| 299 | 691.2 | 3305.3 | 2824.9 | 5439.5 | 1735.1 | 1756.8 | 2682.4 | 4873.4 |
| 300 | 323.5 | 1392.4 | 991.4 | 1256.6 | 1607.8 | 1164.4 | 1068.3 | 1558.2 |
| 301 | 728.3 | 209.7 | 186.7 | 221.8 | 162.3 | 294.8 | 281.5 | 306.3 |
| 302 | 7.7 | 347.9 | 205.9 | 264.9 | 249.4 | 213.4 | 340.1 | 128.4 |
| 303 | 488.4 | 309.5 | 299.3 | 350 | 256.8 | 366.4 | 288.4 | 319.4 |
| 304 | 190.4 | 376.7 | 480.7 | 406.4 | 314.9 | 1106.8 | 764.9 | 973.2 |
| 305 | 478.8 | 246.1 | 628.7 | 2896.4 | 633.1 | 802.4 | 305.6 | 892.8 |
| 306 | 159.1 | 618.4 | 450.3 | 475.8 | 439.9 | 1419.5 | 763.8 | 450.3 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 307 | 132.8 | 254.7 | 425.8 | 462.3 | 354.6 | 254.6 | 307.1 | 237 |
| 308 | 5822.9 | 744.5 | 524.5 | 787 | 589.2 | 606.8 | 354.7 | 424.9 |
| 310 | 1068.6 | 151.4 | 133.4 | 116.4 | 77.7 | 177.3 | 68 | 124.3 |
| 311 | 36.2 | 641.7 | 499 | 622.8 | 349.4 | 351.8 | 356 | 454.7 |
| 312 | 4778.5 | 245 | 143.2 | 236.8 | 4.3 | 250.7 | 224.5 | 335 |
| 313 | 136.4 | 173 | 383.6 | 419 | 537.8 | 354.8 | 230.1 | 549.2 |
| 314 | 485.9 | 926.3 | 1589 | 956.6 | 1048.3 | 1458.6 | 629.4 | 877.4 |
| 315 | 125.9 | 82.4 | 187.2 | 88 | 58.7 | 77 | 93.2 | 43.3 |
| 316 | 367.6 | 574.4 | 633.7 | 462.8 | 337.8 | 583 | 763.4 | 450.7 |
| 317 | 145.2 | 202 | 1956.5 | 215.8 | 199.9 | 250.1 | 242.6 | 647.4 |
| 318 | 22.4 | 277.6 | 212 | 450.5 | 289.2 | 439.1 | 285.9 | 458.3 |
| 319 | 1838.4 | 512.3 | 276.4 | 733 | 268.8 | 487.6 | 670 | 354.9 |
| 320 | 477 | 181.1 | 267.8 | 128.4 | 160.8 | 244 | 142.2 | 214.4 |
| 321 | 718.7 | 163.3 | 292.4 | 134.5 | 159.2 | 263 | 227.2 | 257.6 |
| 322 | 440.4 | 379.6 | 285.7 | 360.6 | 238.3 | 342.7 | 305 | 208.4 |
| 323 | 1582.3 | 3954.1 | 3885.8 | 3447.2 | 3490.5 | 3121.3 | 3460.9 | 3724.3 |
| 324 | 112.2 | 445.5 | 257.9 | 704.3 | 519.5 | 235.2 | 528.1 | 737.5 |
| 325 | 190.1 | 374.9 | 430.9 | 503.9 | 541.5 | 326.1 | 367.3 | 610.4 |
| 326 | 422.9 | 4053.9 | 6121.7 | 4456.6 | 1364.3 | 5707.5 | 3498.2 | 4848.9 |
| 327 | 5.6 | 292.7 | 203.1 | 609.7 | 707.7 | 133.8 | 263.6 | 273.5 |
| 328 | 72.2 | 52 | 299.8 | 94.6 | 299.9 | 87.1 | 27.6 | 114.9 |
| 329 | 216.1 | 65.5 | 49.1 | 96.4 | 60.5 | 118.7 | 102.7 | 76.4 |
| 330 | 74.1 | 438.1 | 1060 | 824 | 632 | 896 | 708.8 | 558.4 |
| 331 | 783.2 | 297.5 | 466.2 | 372.3 | 422.4 | 374.2 | 562 | 297.2 |
| 333 | 216.1 | 167.4 | 206.9 | 194.6 | 174.5 | 38.1 | 190.6 | 149.2 |
| 334 | 107.8 | 126.8 | 470.1 | 153.1 | 513.7 | 220 | 141.5 | 193.3 |
| 335 | 570.7 | 191.9 | 697.3 | 783.9 | 505.5 | 208 | 243.3 | 414.7 |
| 336 | 491.6 | 342.6 | 210.4 | 272.6 | 218 | 256.8 | 428.3 | 243 |
| 337 | 127.4 | 619.5 | 632.8 | 711.3 | 511.9 | 972.3 | 606.6 | 804.8 |
| 338 | 180.6 | 1559 | 1838 | 1292.5 | 2158.9 | 837.9 | 1460.8 | 1632.3 |
| 339 | 303.9 | 217.3 | 347.8 | 220 | 329.4 | 258.1 | 219.3 | 184.2 |
| 340 | 185.5 | 137573.8 | 177311.9 | 54500.9 | 184533.6 | 59673.9 | 314613.6 | 86723.1 |
| 341 | 412.2 | 585 | 708 | 530.7 | 576.4 | 437.9 | 557.3 | 684.6 |
| 343 | 7951.1 | 2880.9 | 1575.6 | 1520.5 | 3477.9 | 2425 | 2379.2 | 1087.2 |
| 344 | 1755.5 | 649.1 | 436.9 | 434.5 | 285.2 | 463.7 | 452.7 | 599.1 |
| 345 | 236.2 | 296.5 | 511.3 | 662 | 580.8 | 429.8 | 424.2 | 488.6 |
| 347 | 821.3 | 1063 | 3642.7 | 1447.8 | 4119.2 | 1163.9 | 1210.2 | 1782.6 |

(continued)

| 3e | | | | | | | | |
|---|---|---|---|---|---|---|---|---|
| SEQ ID NO: | 871TT | 879TT | 881TT | 882TT | 883TT | 884TT | 885TT | 886TT |
| 348 | 1679 | 4474.7 | 7575.3 | 4999.7 | 6225.7 | 3803.1 | 3979.1 | 4537.6 |
| 349 | 256.8 | 526.7 | 376.5 | 477.4 | 483.9 | 423.5 | 503.2 | 641.4 |
| 350 | 210.5 | 768.8 | 682.2 | 677.9 | 628.8 | 579 | 671.7 | 500.6 |
| 351 | 119.8 | 929.5 | 1553 | 2794.5 | 1558.8 | 1728.2 | 788.7 | 1446.6 |
| 352 | 435.7 | 264.1 | 190.3 | 177.4 | 180.3 | 233.4 | 304.2 | 190.7 |
| 353 | 385 | 500.7 | 566.6 | 574.8 | 346.1 | 382.1 | 487.3 | 473 |

| 3f | | | | | | | | |
|---|---|---|---|---|---|---|---|---|
| SEQ ID NO: | 892TT | 893TT | 894TT | 896TT | 898TT | 899TT | 900TT | 901TT |
| 354 | 838.5 | 571.4 | 438.3 | 31.6 | 78.4 | 438.1 | 332.3 | 995.7 |
| 355 | 5801.6 | 1326.3 | 4267.6 | 187.6 | 121 | 2990.3 | 3907.9 | 3595.2 |
| 356 | 672.9 | 416.1 | 1076.3 | 2648.9 | 200.3 | 682.2 | 309.3 | 200.9 |
| 357 | 815.8 | 844.6 | 869 | 1303.9 | 212 | 719.3 | 729.3 | 131.3 |
| 358 | 1742.6 | 1693.2 | 2440.3 | 1481.7 | 366.4 | 2077.8 | 2415.2 | 704.2 |
| 359 | 186 | 222.6 | 304.4 | 405.5 | 466.7 | 343.4 | 245.5 | 236.6 |
| 362 | 2087.5 | 1335.3 | 1239.7 | 326.8 | 171.4 | 870.6 | 879.2 | 101.1 |
| 360 | 472.7 | 502.8 | 559.4 | 1042.8 | 290.1 | 549.2 | 506 | 174.5 |
| 361 | 390.4 | 80 | 162.4 | 329.8 | 212.1 | 162.1 | 138.5 | 149.9 |
| 363 | 335.6 | 549.7 | 748.6 | 666.9 | 742.2 | 461 | 356.5 | 506.8 |
| 1 | 1364 | 864.1 | 2300.1 | 1111.5 | 725.5 | 1891.2 | 735.8 | 1211.8 |
| 2 | 8223.2 | 2863.6 | 1612.1 | 3492.1 | 669.6 | 1116 | 3477.7 | 10076.1 |
| 7 | 621.3 | 68.9 | 83.5 | 28.3 | 339.5 | 548.2 | 237.7 | 108.6 |
| 8 | 434.3 | 297.2 | 236.7 | 314.4 | 1057.9 | 469 | 735.8 | 1382.2 |
| 9 | 2209.5 | 1428.7 | 2874.5 | 9019.8 | 219.4 | 1483.2 | 931.8 | 299 |
| 10 | 87.4 | 318.6 | 123 | 113.9 | 359.9 | 132.2 | 797.4 | 116.1 |
| 11 | 2437.4 | 1234.9 | 867.7 | 1328.7 | 332.7 | 722 | 898.4 | 645.5 |
| 13 | 397 | 162.2 | 347.3 | 290.4 | 118.5 | 313.3 | 718 | 1070.4 |
| 14 | 2294.4 | 977.8 | 1525.3 | 1055.3 | 256.2 | 1105.9 | 2608.5 | 4093.2 |
| 15 | 828.3 | 416.9 | 482.6 | 728.2 | 16.4 | 502.2 | 3166.5 | 179.1 |
| 16 | 3234 | 2475.7 | 4710 | 1027.1 | 239.9 | 3055.6 | 4422.5 | 721.2 |
| 17 | 2718.1 | 742.6 | 607.2 | 522 | 240.9 | 762.6 | 1568.9 | 129.7 |
| 19 | 1086.6 | 248.6 | 333.5 | 226.7 | 290.8 | 444.8 | 7182.8 | 1716.2 |
| 20 | 192.8 | 641.7 | 12.5 | 39 | 1.2 | 792.4 | 372.3 | 36.3 |
| 21 | 1826.1 | 4120.6 | 1923.8 | 941.2 | 238.4 | 2518.7 | 2236.8 | 94.6 |
| 22 | 8192.1 | 2215.6 | 1368.7 | 1592.1 | 753.1 | 5336.9 | 2477.2 | 299.2 |
| 23 | 687.5 | 415.7 | 227.3 | 1.1 | 1.1 | 709.6 | 2476.1 | 179.7 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 24 | 326.7 | 113.9 | 163.5 | 127.1 | 110.4 | 165 | 803.4 | 105 |
| 26 | 1128.9 | 408.7 | 398.2 | 63.7 | 106.1 | 1433.6 | 5983.2 | 1234.8 |
| 27 | 1213 | 865.2 | 488.6 | 772.5 | 725.7 | 1107.3 | 619.8 | 58.3 |
| 29 | 134.2 | 85.5 | 245.2 | 242.8 | 215.1 | 196.6 | 356.2 | 198.7 |
| 30 | 362.2 | 248.6 | 151.7 | 102.9 | 16.7 | 261.5 | 1169.1 | 236 |
| 31 | 9692.5 | 20154 | 18540.6 | 1550.9 | 250.2 | 15669.5 | 3557.5 | 5.2 |
| 33 | 1841.1 | 2277.7 | 1079.8 | 442.8 | 137.2 | 3329.2 | 14318.3 | 588.8 |
| 34 | 826.4 | 839.3 | 441.4 | 624 | 4011.4 | 566.1 | 579.8 | 2078.9 |
| 35 | 1512.1 | 1217.1 | 1163.2 | 194.2 | 132.8 | 1055.6 | 1451.8 | 26.9 |
| 36 | 2188 | 735.7 | 379.8 | 646.9 | 507.5 | 1041.8 | 675.2 | 8116.1 |
| 37 | 133.7 | 152.8 | 191.6 | 188 | 737.6 | 240.4 | 318.6 | 263.7 |
| 38 | 776.1 | 895.4 | 248.4 | 519.4 | 99.4 | 1003.5 | 6688.2 | 339.4 |
| 40 | 910.9 | 789.8 | 648.8 | 518.5 | 810.2 | 652.7 | 553.1 | 417.3 |
| 41 | 922.5 | 3053 | 1250.6 | 119.4 | 254.6 | 779.1 | 3979.3 | 333.2 |
| 42 | 343.8 | 889 | 400.7 | 444.1 | 260.1 | 318.1 | 535.4 | 200.6 |
| 43 | 219.4 | 422 | 417 | 1244.1 | 1305.2 | 310.9 | 593.5 | 1335.2 |
| 44 | 1388 | 1454.8 | 1175.7 | 1009.9 | 401.4 | 945.2 | 1312.6 | 316.6 |
| 45 | 1335.6 | 1007.6 | 839.8 | 405.9 | 730.7 | 1723.4 | 1554 | 740.1 |
| 46 | 281.1 | 267.6 | 425 | 376.4 | 402.6 | 326.4 | 1021.7 | 863 |
| 47 | 739.5 | 340.9 | 354.1 | 543.9 | 308.4 | 485.9 | 555.9 | 1064.5 |
| 48 | 570.5 | 23 | 230 | 40.3 | 27.6 | 142.1 | 56.8 | 273.5 |
| 49 | 2690.8 | 7059.8 | 2365.9 | 162.5 | 247.7 | 3550.1 | 610.3 | 195.8 |
| 50 | 582.4 | 319.5 | 412.6 | 124.5 | 80 | 377.8 | 1255 | 230 |
| 51 | 530 | 1015.7 | 325.9 | 138.5 | 2289.9 | 333.2 | 623.9 | 65.3 |
| 52 | 333.7 | 327.9 | 286.2 | 281.6 | 329.4 | 221.1 | 347.5 | 493.3 |
| 53 | 3598.9 | 1864.6 | 1743.5 | 365.8 | 89.1 | 1567.9 | 1670 | 73.1 |
| 54 | 1988 | 1754.5 | 1175.5 | 286.1 | 91.4 | 1181.1 | 1478.9 | 2364.4 |
| 55 | 85.9 | 96.8 | 95.9 | 124.3 | 145 | 85.6 | 80 | 140.6 |
| 56 | 213.9 | 163.6 | 156.6 | 39 | 62.8 | 229.7 | 214.4 | 57.5 |
| 57 | 6568.1 | 82.1 | 618.1 | 951.4 | 256.7 | 1930 | 764.7 | 814 |
| 58 | 10305.1 | 843.3 | 1639.6 | 3341.1 | 274.3 | 2514.5 | 1398 | 1879.6 |
| 59 | 4632.7 | 8361.8 | 4125.6 | 1656.3 | 319.1 | 4755.6 | 2037.8 | 1675.3 |
| 60 | 510.1 | 221.7 | 136.9 | 117.4 | 148.1 | 354.9 | 336.7 | 187.1 |
| 61 | 98.3 | 111.4 | 123.3 | 179.6 | 275.5 | 108.5 | 72.9 | 74.6 |
| 62 | 5423.2 | 3854.1 | 2373.9 | 8567.7 | 2395.3 | 3812.3 | 2208.6 | 1481 |
| 63 | 830.4 | 1299.6 | 461.4 | 605.2 | 69.5 | 803.8 | 256.1 | 37.7 |
| 64 | 479.7 | 114.8 | 188.8 | 126.9 | 488.9 | 197.6 | 329.8 | 245.9 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 65 | 389.5 | 110.3 | 87.8 | 50.1 | 50.1 | 176 | 383.4 | 101.2 |
| 66 | 1831.2 | 3206.9 | 2835.9 | 601 | 634.9 | 2474.7 | 1318.9 | 103.3 |
| 67 | 26156.4 | 24869.8 | 31314 | 34527.1 | 10176.9 | 25736.9 | 1905.1 | 173.4 |
| 68 | 821.8 | 40.7 | 2360.3 | 789.4 | 2432.7 | 943.9 | 68.9 | 1.1 |
| 69 | 2579.5 | 781.6 | 1976.3 | 1894.3 | 5728.2 | 2305.4 | 397.5 | 111.3 |
| 70 | 5688.9 | 8491.7 | 8887.2 | 2267.9 | 5973.9 | 8571 | 4098.7 | 128.4 |
| 71 | 3496.6 | 5511.6 | 2751.2 | 100.6 | 65.6 | 3748.2 | 10193.5 | 626.2 |
| 72 | 3296 | 2026.7 | 1766.1 | 530.8 | 106.1 | 922 | 2699.1 | 99.9 |
| 73 | 763.6 | 839.9 | 660.7 | 520.4 | 264.6 | 888.6 | 359.2 | 419.4 |
| 75 | 4452.7 | 1125.7 | 275.2 | 310.3 | 145 | 804.4 | 156.6 | 359.9 |
| 76 | 2427.6 | 2043.5 | 940.2 | 329.2 | 2388.1 | 841.4 | 3184.4 | 22133.7 |
| 77 | 563.4 | 2247.6 | 3062.3 | 6825.9 | 2972.1 | 1722.2 | 280.8 | 2325.7 |
| 78 | 623.5 | 124.1 | 227.5 | 554.4 | 340.2 | 302.3 | 207.6 | 170.9 |
| 79 | 297.9 | 552.3 | 257.1 | 484.8 | 185.7 | 287.4 | 938.8 | 230.9 |
| 80 | 2294.3 | 1015.3 | 932.1 | 949.4 | 267.8 | 937.2 | 1004.6 | 2105.3 |
| 81 | 8681.4 | 6802.8 | 13448.3 | 318.1 | 159.4 | 16138.8 | 9121 | 168.3 |
| 82 | 128.2 | 99.7 | 135 | 270.9 | 561.7 | 187.3 | 146.7 | 52.5 |
| 84 | 308.9 | 120.8 | 130.3 | 138.1 | 893.7 | 256.5 | 336 | 131.7 |
| 85 | 410.7 | 2865.5 | 317.4 | 179.7 | 186.4 | 225.1 | 1173 | 198.2 |
| 86 | 71.5 | 641.8 | 296.9 | 391.1 | 257.7 | 123.2 | 2621.8 | 23448.8 |
| 87 | 689 | 3701.3 | 2676.7 | 616.5 | 261 | 857 | 2313.9 | 324 |
| 88 | 712.7 | 622.2 | 840 | 1105.6 | 1000.6 | 639.5 | 272.4 | 1569.4 |
| 89 | 295 | 186 | 158.6 | 179.6 | 961.8 | 277.3 | 453.9 | 155.9 |
| 90 | 215.5 | 68.7 | 167.8 | 107.4 | 122.9 | 164.7 | 1000.2 | 289 |
| 91 | 7519.7 | 924.8 | 1647.3 | 799.8 | 217.9 | 817.9 | 2001.9 | 2046.3 |
| 92 | 11830.9 | 13031.1 | 13383.8 | 7238.6 | 302.1 | 3811.1 | 5046.1 | 174.3 |
| 93 | 115.5 | 87.8 | 34 | 89.4 | 211.9 | 78.4 | 109.2 | 75.8 |
| 94 | 767.1 | 1362.1 | 410.1 | 191.2 | 97.7 | 553.9 | 380.2 | 39.9 |
| 95 | 299.5 | 345.9 | 216.6 | 55.5 | 193.5 | 512 | 708 | 62.4 |
| 96 | 353.2 | 438.1 | 419.9 | 460.6 | 210.2 | 326 | 144.2 | 304.2 |
| 97 | 808.2 | 693.9 | 524.2 | 2786.4 | 252.2 | 405.5 | 344.9 | 189.9 |
| 98 | 20.1 | 38.9 | 44.1 | 21.6 | 22.7 | 45.4 | 14.9 | 853 |
| 99 | 1151.9 | 1198.4 | 389.3 | 273.9 | 337.4 | 383.1 | 950.5 | 207.7 |
| 100 | 152.9 | 90.9 | 122.7 | 552.8 | 61.1 | 313.3 | 41.7 | 36022.7 |
| 101 | 260.8 | 135.8 | 150.6 | 140.2 | 234.4 | 326.1 | 275.4 | 121.1 |
| 102 | 117.3 | 164.9 | 193.6 | 214.6 | 452.9 | 190.4 | 161.9 | 15084.6 |
| 103 | 359.7 | 366.5 | 553.1 | 618.6 | 458.1 | 556.4 | 352.4 | 349.3 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 104 | 72.3 | 85.7 | 95.1 | 84.2 | 660.1 | 174.1 | 49.8 | 301.7 |
| 105 | 76.8 | 146 | 192.9 | 201.7 | 396.4 | 190.8 | 55 | 576.1 |
| 106 | 80.9 | 148.1 | 99.6 | 97.2 | 589.1 | 212.8 | 79.7 | 301 |
| 107 | 207.3 | 184.2 | 599.8 | 177.7 | 156.9 | 201.1 | 710.6 | 224.5 |
| 108 | 163.3 | 204.4 | 177.6 | 565.4 | 277.2 | 123.7 | 285.7 | 396 |
| 109 | 1770.9 | 611.2 | 2356.1 | 78.1 | 65.3 | 737.6 | 812.4 | 1225.9 |
| 110 | 3284.9 | 1445.7 | 5145.6 | 1.2 | 1.3 | 2012.1 | 2144.5 | 3019.9 |
| 111 | 125.1 | 46 | 62.7 | 29.8 | 254.8 | 274.1 | 161.3 | 39.9 |
| 112 | 616 | 1456.3 | 1415 | 289.8 | 56.8 | 1850.3 | 2408.5 | 94.2 |
| 113 | 484.5 | 533.3 | 277.4 | 98.1 | 241.5 | 317.7 | 339.8 | 66.4 |
| 114 | 600.1 | 371.3 | 562.4 | 290 | 103.7 | 987.5 | 502.2 | 156.9 |
| 115 | 1041.7 | 1742.3 | 1015.4 | 1023 | 384.6 | 1053.1 | 733.8 | 268.3 |
| 116 | 932 | 296 | 381 | 243.7 | 236.9 | 432.4 | 1273.7 | 147.7 |
| 117 | 339.7 | 124.5 | 167.1 | 263.6 | 190.4 | 177.4 | 107.7 | 55.5 |
| 118 | 69.7 | 220.3 | 177.8 | 130.5 | 3113.8 | 104.8 | 101.3 | 865.7 |
| 119 | 298.6 | 219.6 | 301.2 | 94.8 | 80.9 | 379.1 | 460.6 | 53 |
| 120 | 98.3 | 53.4 | 99.6 | 177.4 | 150.1 | 98.1 | 1050.9 | 126.1 |
| 121 | 5866 | 1900 | 3972.7 | 505.4 | 92.2 | 6630 | 207 | 2844.8 |
| 122 | 322.1 | 911.3 | 404.3 | 385.8 | 240.5 | 366.3 | 568.3 | 63.3 |
| 123 | 404.9 | 634.2 | 345.1 | 149 | 392 | 340.4 | 234.3 | 311.6 |
| 124 | 131.4 | 129.1 | 160.1 | 787.8 | 2835.4 | 130.4 | 121.4 | 150.7 |
| 125 | 723.6 | 926.1 | 1036 | 939.9 | 2182.9 | 947.8 | 12974.8 | 522.7 |
| 126 | 141.1 | 30.5 | 89 | 159 | 1503.2 | 35.6 | 3.1 | 355.9 |
| 127 | 191.9 | 184.3 | 258.1 | 319.5 | 139.6 | 201.4 | 155.4 | 104.9 |
| 128 | 179 | 235943.8 | 208.7 | 264 | 359.9 | 236127.3 | 90211.9 | 230929.7 |
| 129 | 1267.5 | 3432.6 | 1214.7 | 246.9 | 149.3 | 1110.6 | 5445.8 | 153.6 |
| 130 | 540.5 | 632.3 | 587.9 | 375.8 | 113.6 | 347.2 | 1074.4 | 82.4 |
| 131 | 856.8 | 607.9 | 246.2 | 90.3 | 123.9 | 357 | 480 | 54.8 |
| 132 | 387.6 | 420.8 | 334.4 | 228.4 | 227.3 | 360.2 | 314.6 | 225.9 |
| 133 | 237.3 | 363.1 | 302.2 | 130.6 | 230.6 | 311.5 | 218 | 603.9 |
| 134 | 594.4 | 1140 | 498.1 | 384 | 104 | 345.6 | 401.6 | 224.8 |
| 135 | 1075.3 | 533.8 | 887.3 | 1588.4 | 220 | 677.5 | 175.4 | 301.4 |
| 138 | 231.7 | 110.1 | 175.8 | 232.6 | 169.6 | 171 | 141.3 | 203.3 |
| 139 | 298.9 | 162.7 | 156.5 | 459.8 | 119.7 | 182.1 | 162 | 227.8 |
| 140 | 762.9 | 725.5 | 154.5 | 31.8 | 1.2 | 92.1 | 35.4 | 1.1 |
| 141 | 621.6 | 930.7 | 688.4 | 687.8 | 23 | 420.1 | 36.5 | 666.8 |
| 144 | 37803 | 9899.9 | 22316.3 | 645.5 | 490.7 | 13084 | 9054.7 | 316.7 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 145 | 177 | 368.3 | 269.9 | 232.7 | 310.9 | 289.2 | 250 | 219.2 |
| 146 | 243 | 250.9 | 892.8 | 185.8 | 329 | 1096 | 513.5 | 231 |
| 147 | 120.4 | 64.3 | 79.7 | 78.1 | 34.2 | 140.2 | 94 | 23.6 |
| 148 | 677.9 | 631.3 | 630.4 | 298.9 | 225.4 | 335.1 | 307.8 | 192.7 |
| 149 | 398.6 | 217.3 | 602.8 | 1656.9 | 31.3 | 377.5 | 134 | 54.5 |
| 150 | 374.1 | 454.4 | 196.3 | 972.4 | 157.6 | 149.2 | 283.3 | 5740.8 |
| 151 | 1052 | 1195 | 656.8 | 163.7 | 191.8 | 924.2 | 636.7 | 216.6 |
| 153 | 139.4 | 146.7 | 146.2 | 319.5 | 194.7 | 88.4 | 724.1 | 99.2 |
| 154 | 8645.2 | 16591.3 | 5176.3 | 424.8 | 109.1 | 3577 | 4066.1 | 75.6 |
| 155 | 246.6 | 937.6 | 813.8 | 187.8 | 175.2 | 624.4 | 381.4 | 146.7 |
| 156 | 295.3 | 187.6 | 212 | 76.1 | 53.7 | 543.4 | 250.4 | 52.4 |
| 157 | 223.9 | 203.1 | 318.4 | 199.3 | 184.1 | 199.3 | 122.6 | 224.2 |
| 158 | 537.6 | 821.6 | 265.2 | 1.4 | 27 | 713.1 | 498.5 | 9.4 |
| 159 | 1344.7 | 1973.3 | 1599.7 | 3234 | 904.2 | 1579.4 | 405.5 | 1.3 |
| 161 | 3301.2 | 7782.7 | 14054.5 | 63.2 | 66.7 | 8990.6 | 4295.2 | 228.4 |
| 162 | 701.6 | 131.2 | 171.7 | 65.8 | 32 | 330.5 | 1161.7 | 70.7 |
| 164 | 8016.9 | 3022.2 | 1600.5 | 253.8 | 89.7 | 771.6 | 851.6 | 88.2 |
| 166 | 1326.9 | 1067.9 | 409.9 | 3604 | 3611.9 | 1114.7 | 1254.5 | 258.6 |
| 167 | 457.9 | 95.7 | 482.2 | 1592.1 | 1309.9 | 959.2 | 81.5 | 1.2 |
| 168 | 1090.3 | 935.1 | 1205.2 | 793.6 | 90.8 | 552 | 89.1 | 34.2 |
| 169 | 13698.9 | 8961.4 | 4979.8 | 1588.3 | 221.9 | 15295.7 | 6424.9 | 919.2 |
| 170 | 2889.3 | 2125.1 | 1875.6 | 2018.5 | 38 | 2738.1 | 2166.4 | 1941.3 |
| 171 | 575.6 | 86.9 | 234.3 | 1.3 | 1.2 | 326.8 | 1162.6 | 272.5 |
| 172 | 480.7 | 479.3 | 537.5 | 625.7 | 696.9 | 476.9 | 451.9 | 506.7 |
| 173 | 1793.3 | 647.9 | 338.5 | 142.8 | 256.6 | 1057.8 | 862.7 | 492.4 |
| 176 | 1468.4 | 1365.5 | 272.9 | 1.2 | 75.3 | 744.9 | 117.3 | 10085.2 |
| 177 | 275.1 | 98.7 | 141 | 183.5 | 592.4 | 278.4 | 235.9 | 131.1 |
| 178 | 1912.6 | 1129.2 | 203.6 | 86.2 | 728 | 1472.4 | 2389 | 12289 |
| 179 | 485.6 | 1376.2 | 243.4 | 60 | 153.3 | 1065.3 | 3098.7 | 53.9 |
| 180 | 1011.7 | 464.5 | 526.5 | 263.1 | 154.4 | 957.8 | 1085 | 118.1 |
| 181 | 5130.4 | 3292.8 | 3724 | 13102.1 | 11246.2 | 5277.8 | 2419.3 | 389.1 |
| 182 | 530.4 | 324.9 | 404 | 767.8 | 204.4 | 388.4 | 384.7 | 169.2 |
| 183 | 447.6 | 199 | 135.7 | 1.1 | 1.1 | 259.9 | 1258.2 | 281.4 |
| 184 | 620 | 357.8 | 340.4 | 291.6 | 192 | 428.8 | 14293.9 | 682.8 |
| 185 | 3627.9 | 10011.6 | 2963.4 | 729 | 355.6 | 10614.7 | 3033.4 | 880 |
| 186 | 173.9 | 184.8 | 258.3 | 1106 | 716.2 | 173.4 | 287.8 | 2619 |
| 187 | 333.3 | 525.2 | 136.5 | 200.2 | 239.2 | 314.3 | 689.4 | 176.4 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 188 | 435.3 | 350.4 | 378.3 | 132.3 | 145.2 | 161.9 | 1419.6 | 133.9 |
| 189 | 181.3 | 130.5 | 201.3 | 91.3 | 144.2 | 92.7 | 674.1 | 79.5 |
| 190 | 359.9 | 238.5 | 312 | 108.8 | 110.1 | 485.5 | 163.7 | 58.4 |
| 191 | 5707 | 3740.1 | 3029.6 | 3256.5 | 886.2 | 2447.8 | 3778.5 | 97.4 |
| 192 | 150.4 | 95.8 | 139.2 | 130.9 | 129.3 | 109.1 | 506.3 | 2753.6 |
| 193 | 361.7 | 114.3 | 88.9 | 6063.3 | 105.9 | 289.6 | 160.1 | 46.2 |
| 194 | 261.5 | 249.3 | 203.6 | 200.9 | 134.1 | 251.6 | 372.2 | 471.3 |
| 195 | 132 | 110.9 | 158.3 | 96.7 | 440.4 | 150 | 189.2 | 297.3 |
| 196 | 1327.6 | 1039.5 | 1104 | 311.8 | 7746.9 | 3084.9 | 1670.6 | 448.2 |
| 197 | 132.2 | 98.3 | 139.2 | 194 | 218.3 | 141.3 | 868.5 | 520.5 |
| 198 | 532.6 | 1476.5 | 1624.7 | 83.8 | 94.6 | 2377.6 | 755.4 | 70.4 |
| 199 | 7184.6 | 2171.3 | 7934.6 | 482.3 | 52.5 | 4537.3 | 5623.9 | 4564.9 |
| 200 | 975.6 | 370.7 | 300.2 | 182.2 | 210.4 | 482.6 | 427.5 | 1652.6 |
| 201 | 957 | 604.8 | 467.3 | 35.1 | 11.1 | 420.7 | 334 | 1094.3 |
| 203 | 3711.3 | 1570.8 | 1229.6 | 5308.7 | 248.9 | 965.6 | 1358.9 | 47.1 |
| 204 | 1750.8 | 1898.9 | 2112.9 | 4011.7 | 204.1 | 1174.1 | 290.9 | 27.1 |
| 205 | 6039 | 4435.4 | 3880.5 | 6493.9 | 2337.3 | 4612.6 | 1166.9 | 3.2 |
| 206 | 163.8 | 374.4 | 608.3 | 472.7 | 308.5 | 390.5 | 250 | 327.8 |
| 207 | 480.6 | 47.7 | 74.7 | 206.6 | 34.9 | 103.4 | 58.1 | 59.8 |
| 208 | 585.4 | 1688.4 | 1780.1 | 191.9 | 58.9 | 866.4 | 278.1 | 1.6 |
| 209 | 197.5 | 4.4 | 60.6 | 353.2 | 5030.7 | 400.5 | 66.4 | 207.9 |
| 211 | 1550.5 | 219.1 | 1271.8 | 1356.7 | 942.5 | 164.7 | 120.1 | 87.4 |
| 212 | 1426 | 1312.8 | 870.1 | 225.4 | 1412.1 | 2035.3 | 1106.7 | 52.1 |
| 213 | 104 | 191.1 | 118.8 | 124.5 | 3087.3 | 123.3 | 121.9 | 77.7 |
| 215 | 475.1 | 55.9 | 84.6 | 15 | 9 | 131.4 | 77.7 | 93 |
| 216 | 397.6 | 259.8 | 310.5 | 423.2 | 377.9 | 179.3 | 226.1 | 222.2 |
| 217 | 1130.5 | 517.6 | 725 | 187.5 | 173.5 | 2109.1 | 1336.7 | 189.2 |
| 218 | 159.4 | 224.9 | 205.2 | 353.5 | 294.1 | 236.6 | 199.5 | 89 |
| 220 | 79.6 | 70 | 204.6 | 1293.4 | 119 | 128.5 | 270.9 | 68484.3 |
| 221 | 2459.4 | 1795.5 | 739.3 | 429.3 | 254.4 | 1453.4 | 860.5 | 219.7 |
| 223 | 981.9 | 680.4 | 687.3 | 439.1 | 267.8 | 500.2 | 459.3 | 290.4 |
| 227 | 1489.9 | 1104.5 | 879.4 | 1033.7 | 822.7 | 1224.7 | 5386.3 | 435.5 |
| 228 | 186.1 | 178.2 | 271.2 | 198.1 | 433.9 | 318.4 | 220.6 | 177.1 |
| 229 | 180.5 | 334 | 201.9 | 302.5 | 172.6 | 189.2 | 253.5 | 582.6 |
| 230 | 3245.4 | 2835.9 | 3394.8 | 163.3 | 99.6 | 3274.1 | 4388 | 147.1 |
| 231 | 1258.8 | 702.6 | 436 | 985.6 | 204.9 | 418.8 | 591.9 | 75.2 |
| 232 | 704.3 | 266.5 | 271.6 | 59.5 | 31.8 | 1000 | 4104.9 | 782 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 233 | 15997.8 | 9251.8 | 21829 | 5239.6 | 15022 | 18292.9 | 3490 | 3738.3 |
| 235 | 405.2 | 135 | 205.3 | 218.8 | 77.9 | 242.7 | 688.4 | 216.2 |
| 236 | 345.4 | 176.8 | 244.3 | 51.8 | 289.1 | 251.1 | 569.9 | 355.3 |
| 237 | 2087.1 | 2074.2 | 977.3 | 667.1 | 647.6 | 2376.1 | 1252 | 2105 |
| 238 | 1029.7 | 709.6 | 957.5 | 1263.7 | 456.5 | 1002.9 | 733.2 | 417.9 |
| 239 | 398.5 | 111.4 | 252.1 | 162.3 | 97.3 | 806.1 | 929.5 | 1253.4 |
| 240 | 590.8 | 219.9 | 517.4 | 355.8 | 74.6 | 973.1 | 1561.1 | 1235 |
| 241 | 1224.2 | 353.4 | 781.2 | 558.8 | 62.3 | 2210.8 | 2833.8 | 2772.1 |
| 242 | 31599.6 | 34700.3 | 41193.8 | 1998.1 | 39786.8 | 38117 | 29127.6 | 4032.5 |
| 243 | 496 | 1167.3 | 574.5 | 293.2 | 363.2 | 674.9 | 937.7 | 478.8 |
| 244 | 619.3 | 993.1 | 600.4 | 303.2 | 63.1 | 271.1 | 782.3 | 352.7 |
| 246 | 1856.6 | 1243.2 | 1238.1 | 641.8 | 111.2 | 2226.5 | 1235.4 | 797.6 |
| 247 | 90.8 | 89.3 | 158.9 | 200.2 | 539.1 | 130.5 | 88.4 | 2091.6 |
| 248 | 3748.4 | 2617 | 1198.3 | 214.5 | 80.7 | 1744 | 935.6 | 108.1 |
| 249 | 2042.6 | 468.9 | 513.5 | 50.4 | 326.4 | 336.8 | 254.7 | 971.7 |
| 251 | 595 | 1004.5 | 592 | 56.1 | 35 | 1047.9 | 2445.5 | 71.5 |
| 253 | 465.8 | 497.1 | 469.6 | 410 | 220.1 | 449.3 | 295.9 | 531.1 |
| 254 | 2043.5 | 910.3 | 939.7 | 363.4 | 86 | 1038.8 | 861.8 | 107 |
| 255 | 2304.8 | 1354.8 | 344.7 | 122.3 | 762.2 | 1679.9 | 2631.1 | 14516.5 |
| 256 | 7209.5 | 1050.3 | 1841.6 | 927.6 | 282.3 | 701.3 | 1193.8 | 1494.3 |
| 257 | 856.2 | 248.4 | 216.9 | 662.9 | 150 | 341.6 | 378.8 | 234.1 |
| 258 | 1862.6 | 2711.5 | 2864.4 | 273.4 | 23.2 | 1388.3 | 3786.6 | 38.9 |
| 259 | 690.8 | 1255 | 315.5 | 8.9 | 8.9 | 666.1 | 567.4 | 35.6 |
| 260 | 171.5 | 136.4 | 233.1 | 141.7 | 251.1 | 174 | 130.8 | 576.4 |
| 261 | 647.4 | 192.8 | 70.8 | 118.2 | 87.7 | 108 | 72.5 | 91.1 |
| 263 | 797.9 | 1445.4 | 889.1 | 1265 | 3327 | 572.6 | 1048.4 | 4844.5 |
| 263 | 2900.5 | 3388 | 4354 | 382 | 258.7 | 2740.8 | 3471.4 | 176.5 |
| 265 | 89.2 | 115.6 | 179.2 | 394.1 | 219 | 100.9 | 131.6 | 85.6 |
| 266 | 213.6 | 237.5 | 772.5 | 877 | 652.8 | 477.8 | 159.6 | 124 |
| 256 | 293.8 | 159.8 | 170.3 | 96.7 | 137.6 | 266.6 | 612.1 | 111.9 |
| 268 | 427.2 | 47.3 | 133.1 | 64.4 | 531.8 | 497.3 | 80.3 | 18.9 |
| 269 | 144.1 | 171.6 | 184 | 64.4 | 137.5 | 256.6 | 159.2 | 47.1 |
| 270 | 149.1 | 221.6 | 249.7 | 540.1 | 727.4 | 204.9 | 126.7 | 413 |
| 271 | 928.9 | 1477.3 | 659.3 | 228.8 | 157 | 361 | 939.9 | 257.9 |
| 272 | 2065.5 | 769.5 | 961.5 | 709.2 | 889.7 | 644.3 | 583.3 | 1336.4 |
| 273 | 460 | 254.4 | 1932.6 | 1972 | 55.7 | 1557.3 | 1.2 | 1.1 |
| 274 | 1272 | 1303.2 | 959.1 | 381.8 | 692.6 | 817.7 | 3288.6 | 1936.2 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 275 | 1067.2 | 3809.3 | 819 | 189.7 | 89.6 | 3279.9 | 984.3 | 183.6 |
| 276 | 1560.5 | 1665.4 | 856.1 | 437.3 | 864.3 | 1770.9 | 8639.5 | 830.5 |
| 277 | 420.2 | 373.4 | 347.1 | 255.6 | 230.4 | 352.6 | 225.8 | 197.8 |
| 278 | 1479.6 | 2479.2 | 413.9 | 38.1 | 16.1 | 1327.7 | 1485.3 | 1104.2 |
| 279 | 4418.5 | 1252.4 | 1647.9 | 1229.2 | 826.2 | 1907.3 | 690.5 | 1.2 |
| 280 | 6980.6 | 3103.2 | 5603.5 | 9202.3 | 973.7 | 3117.3 | 1054 | 1.4 |
| 281 | 893.5 | 469.5 | 390.8 | 50.9 | 76.1 | 645.6 | 28.3 | 2949.8 |
| 282 | 436.5 | 866.2 | 712.4 | 604.9 | 432.1 | 429.3 | 4533.3 | 239.2 |
| 283 | 378.5 | 494.1 | 618.3 | 438.7 | 610.6 | 397.3 | 345.7 | 2247.9 |
| 284 | 86.5 | 78.4 | 84.7 | 59.9 | 151.4 | 121.6 | 104 | 90 |
| 285 | 100.1 | 125 | 140.6 | 171.6 | 130.3 | 107.2 | 95.4 | 109.5 |
| 286 | 21.9 | 47 | 34.4 | 361.5 | 622.2 | 17.4 | 51 | 14.1 |
| 287 | 85 | 103.5 | 147.3 | 266.3 | 140.8 | 110.4 | 90.8 | 52593.6 |
| 288 | 106.7 | 108.9 | 226 | 255.8 | 302 | 141.1 | 141.3 | 161.5 |
| 289 | 16536.4 | 1225.1 | 3454 | 1845.6 | 187.6 | 1317.1 | 1594.5 | 336.7 |
| 291 | 197.9 | 131.8 | 167.2 | 170.5 | 27.6 | 264.2 | 372 | 222.7 |
| 292 | 10808.4 | 363.8 | 202.4 | 174.8 | 66 | 1720 | 15444.1 | 227.7 |
| 293 | 409.1 | 361.7 | 288.2 | 206.3 | 82.6 | 711.2 | 306.9 | 55.7 |
| 295 | 812 | 283.8 | 1193.9 | 554.6 | 477.9 | 2370 | 701.1 | 344.9 |
| 296 | 2134.1 | 3003.7 | 1806.5 | 1160.2 | 1121 | 1902.2 | 1362.4 | 253.2 |
| 297 | 38.5 | 59.7 | 102.3 | 75.6 | 2740.8 | 70.7 | 113.3 | 39.2 |
| 298 | 610.2 | 990 | 998 | 952 | 469.4 | 525.7 | 390.2 | 600.9 |
| 299 | 2108.9 | 4536.4 | 3222.2 | 2152.4 | 419.3 | 4258 | 1379.7 | 77.2 |
| 300 | 1679.8 | 1409.5 | 1107.2 | 90.1 | 244.2 | 1361.8 | 952.6 | 239.4 |
| 301 | 221.2 | 271 | 284.8 | 845.7 | 261 | 211.4 | 223.5 | 162.3 |
| 302 | 216.8 | 246.1 | 174.8 | 34.5 | 40.3 | 171.5 | 310.6 | 21.7 |
| 303 | 266.3 | 414.9 | 259.1 | 1838.2 | 984.5 | 263.8 | 215.7 | 52.1 |
| 304 | 1007.4 | 636.1 | 791.1 | 1190.1 | 791 | 396.5 | 803.9 | 2631.2 |
| 305 | 2780.8 | 1913 | 203.5 | 627.5 | 451.4 | 213.2 | 4287.8 | 28703.6 |
| 306 | 600.9 | 1369.7 | 510.6 | 266.5 | 278.1 | 451.2 | 469.4 | 196.1 |
| 307 | 427.2 | 183.4 | 239.2 | 150 | 194.3 | 189.6 | 485.2 | 145.3 |
| 308 | 296.6 | 711.6 | 941.5 | 559.6 | 3583.4 | 734 | 515.2 | 1057.1 |
| 310 | 71.1 | 164.3 | 307 | 1005.9 | 1091.8 | 92.5 | 194.1 | 204.9 |
| 311 | 819.5 | 309.6 | 177.4 | 119.9 | 48.5 | 805.6 | 148.5 | 343.4 |
| 312 | 35.7 | 488.7 | 553.3 | 5485.3 | 1957.5 | 229.2 | 282.3 | 1084.7 |
| 313 | 624.7 | 219.7 | 192.2 | 247.3 | 117.1 | 393.7 | 632.5 | 548.7 |
| 314 | 1060.4 | 958.7 | 775.5 | 190.2 | 164.3 | 1171 | 521.3 | 302.7 |

(continued)

| 3f | | | | | | | | |
|---|---|---|---|---|---|---|---|---|
| SEQ ID NO: | 892TT | 893TT | 894TT | 896TT | 898TT | 899TT | 900TT | 901TT |
| 315 | 208.4 | 52.1 | 72 | 131.8 | 117.4 | 149.4 | 73.1 | 78.3 |
| 316 | 967.1 | 497.3 | 525.1 | 285.3 | 123.4 | 509.3 | 313.1 | 1093.3 |
| 317 | 1983.8 | 165 | 184.2 | 186.9 | 201.3 | 230.5 | 235.8 | 160.7 |
| 318 | 535.8 | 1159.4 | 335.7 | 57.8 | 34.2 | 275.6 | 327.6 | 202.1 |
| 319 | 303 | 615.9 | 648.6 | 565.7 | 660.6 | 420.7 | 486.2 | 82.1 |
| 320 | 250.3 | 171.3 | 229.3 | 898.4 | 581.8 | 283.3 | 182.8 | 91.2 |
| 321 | 153.9 | 170.4 | 222.1 | 487.3 | 395.4 | 195.2 | 275.7 | 152.1 |
| 322 | 272.1 | 366 | 269.8 | 234.9 | 505.1 | 327 | 291.7 | 323.8 |
| 323 | 4149 | 2529.2 | 4790.2 | 11767.2 | 291.7 | 2101.2 | 1031.5 | 36.9 |
| 324 | 597.9 | 1026.6 | 425.6 | 216.6 | 66.4 | 520.2 | 374.4 | 38.3 |
| 325 | 600.8 | 475.6 | 489.7 | 427.7 | 333.3 | 614.4 | 461.4 | 102.3 |
| 326 | 6135.2 | 4645.8 | 2435.9 | 4623.7 | 489.7 | 3385.8 | 361.8 | 54.9 |
| 327 | 137 | 39.2 | 140.2 | 11.6 | 18.1 | 772 | 1166.8 | 47 |
| 328 | 139.5 | 41.9 | 42 | 35.3 | 115.4 | 264.6 | 82.5 | 28.1 |
| 329 | 45.1 | 69.1 | 52.6 | 106.4 | 190.4 | 59.3 | 53.4 | 158.6 |
| 330 | 1850.5 | 187.5 | 197.4 | 57.5 | 63.1 | 611.9 | 92.7 | 48.8 |
| 331 | 306.7 | 290.7 | 381.2 | 458.9 | 680.9 | 301.1 | 355.5 | 305.4 |
| 333 | 168.8 | 202.7 | 113.7 | 654.4 | 440.1 | 99.6 | 101.7 | 733.2 |
| 334 | 157.4 | 151.2 | 147.9 | 185.5 | 154.5 | 491.6 | 227.7 | 175.6 |
| 335 | 355 | 141.1 | 215.2 | 128.2 | 621.4 | 273.8 | 122.1 | 95.7 |
| 336 | 199.6 | 235 | 357 | 392.5 | 544.7 | 194.9 | 201.4 | 419.4 |
| 337 | 529.5 | 776.2 | 670.2 | 5029.2 | 12.6 | 247.4 | 1259.7 | 37.2 |
| 338 | 2393.6 | 467.5 | 1714.9 | 222.6 | 93.8 | 1228.2 | 573.6 | 80.8 |
| 339 | 268.9 | 201 | 224.4 | 249 | 562.8 | 317.5 | 248.1 | 316.4 |
| 340 | 108216.2 | 107282.5 | 151414.7 | 284.8 | 351.6 | 104374 | 125245.9 | 415.7 |
| 341 | 557.6 | 400 | 640.4 | 937.5 | 796.5 | 538.1 | 314.8 | 530.6 |
| 343 | 4739.2 | 4407 | 2670 | 1153.2 | 3880.9 | 3677.6 | 847.9 | 199.5 |
| 344 | 535.4 | 284.2 | 615.6 | 2191.3 | 1660.9 | 465.9 | 413.9 | 155.3 |
| 345 | 437.9 | 418.5 | 469.5 | 233.5 | 318 | 503.8 | 379.4 | 297.9 |
| 347 | 2204.6 | 607.2 | 2427.6 | 366.1 | 1391.9 | 1925.4 | 461.5 | 76.1 |
| 348 | 7719.8 | 2494 | 5392.3 | 378.6 | 2314.6 | 5267.1 | 1183.1 | 125.8 |
| 349 | 570.2 | 609 | 636 | 458.1 | 172.2 | 320.7 | 665.1 | 493.5 |
| 350 | 497.3 | 566.4 | 585.7 | 901.1 | 105.7 | 491.3 | 539.6 | 37.9 |
| 351 | 1925.2 | 1358.6 | 1065.7 | 133.7 | 96.1 | 1962.8 | 717.3 | 32.9 |
| 352 | 177.5 | 197.1 | 290.5 | 1585.5 | 569.8 | 165.4 | 101.5 | 302.3 |
| 353 | 734.5 | 602 | 399.5 | 357.6 | 924 | 415.5 | 202.7 | 175.3 |

| 3g | | | | | | | | |
|---|---|---|---|---|---|---|---|---|
| SEQ ID NO: | 902TT | 903TT | 904TT | 907TT | 908TT | 909TT | 910TT | 913TT |
| 354 | 1260.9 | 3539.8 | 3818.1 | 9.2 | 180.3 | 221.3 | 64.7 | 837.9 |
| 355 | 83.5 | 5857.1 | 8246.9 | 48.4 | 247 | 60.3 | 53.8 | 151.4 |
| 356 | 105.3 | 500.4 | 419.6 | 781.6 | 797.8 | 102.4 | 278.5 | 575.7 |
| 357 | 249.4 | 689.9 | 517.8 | 798.6 | 569.9 | 361.7 | 600.7 | 655.3 |
| 358 | 975.3 | 2387.6 | 1281.2 | 823.5 | 857.3 | 1419.9 | 874.2 | 849.4 |
| 359 | 419.6 | 370.8 | 337.1 | 471.8 | 328.4 | 383.9 | 347.2 | 356.6 |
| 362 | 373.2 | 1448.2 | 359.4 | 401 | 473.1 | 781.6 | 303.4 | 262.5 |
| 360 | 268 | 446.7 | 558.4 | 242 | 366.4 | 1651.7 | 276.5 | 241.5 |
| 361 | 172.3 | 318.3 | 567.6 | 1196.1 | 670.7 | 316.5 | 236.7 | 426.2 |
| 363 | 1097.1 | 422.7 | 450.5 | 468.3 | 468.2 | 570.7 | 425.1 | 561.6 |
| 1 | 242.7 | 1509.1 | 565.3 | 1975.2 | 843.7 | 742.5 | 644.2 | 516.1 |
| 2 | 425.6 | 4753.1 | 2426.5 | 2640.2 | 3242 | 1866.5 | 1738.3 | 2465.6 |
| 7 | 96.7 | 1020.9 | 322.7 | 1442.2 | 913.4 | 2.1 | 688.8 | 390.1 |
| 8 | 1309.4 | 1177 | 1828.8 | 572.9 | 1092.8 | 1041.7 | 904 | 1781.9 |
| 9 | 496.7 | 2818.2 | 4872.6 | 1566 | 6508.7 | 2940.7 | 542 | 8678 |
| 10 | 150.7 | 81.4 | 218 | 97.4 | 90.5 | 118.5 | 139 | 57.6 |
| 11 | 623.6 | 578.7 | 535.7 | 283.7 | 341.2 | 219.9 | 168.8 | 310 |
| 13 | 173.4 | 526.7 | 1528.1 | 95.2 | 286 | 227.3 | 1018.4 | 322 |
| 14 | 424.5 | 1784.7 | 2754 | 255.4 | 584.8 | 1148.7 | 2517.6 | 721.5 |
| 15 | 192.6 | 231.1 | 6970.4 | 559.7 | 866.8 | 2354.9 | 135.8 | 2247.9 |
| 16 | 814.4 | 2337.1 | 1020.3 | 571 | 1207.1 | 216.6 | 1179.9 | 947 |
| 17 | 417 | 1095.8 | 1202.6 | 1279.3 | 1125.9 | 1162.1 | 297.9 | 1237.4 |
| 19 | 320.6 | 619.6 | 2250.9 | 274.2 | 4651.6 | 16678.5 | 159.1 | 586.3 |
| 20 | 41.5 | 263.6 | 1643.5 | 94.1 | 70.4 | 35.5 | 1.3 | 42.3 |
| 21 | 128 | 2017.2 | 451.4 | 191.3 | 326.9 | 52.2 | 97.2 | 172.4 |
| 22 | 2089.5 | 4271.9 | 6617.2 | 4571.3 | 6361.4 | 2003.9 | 1809.4 | 7708.1 |
| 23 | 1.1 | 427.6 | 5067.6 | 455.9 | 966.7 | 2190.6 | 113.7 | 2551.2 |
| 24 | 140.7 | 218.4 | 963.7 | 245.9 | 328.3 | 456.7 | 114.4 | 572.7 |
| 26 | 110.4 | 1938 | 14240.4 | 1024 | 7609.6 | 17839.9 | 409.3 | 5874.4 |
| 27 | 736 | 1194.6 | 387.1 | 374.1 | 866.3 | 660.5 | 336.8 | 622.3 |
| 29 | 1746.3 | 207.1 | 150.7 | 96.2 | 129.5 | 375.2 | 87.3 | 132.6 |
| 30 | 60.4 | 204.1 | 7348.8 | 247 | 268.9 | 1830.2 | 51.2 | 900.4 |
| 31 | 250.5 | 4640.9 | 1272.4 | 54.2 | 361.6 | 246.2 | 125.6 | 11511.9 |
| 33 | 726.1 | 1138.2 | 22827.9 | 1763 | 1784.5 | 2090.6 | 172.6 | 8499.2 |
| 34 | 1224.5 | 1241.4 | 1284.7 | 2615.8 | 4040.9 | 1589 | 2170.1 | 3921.1 |
| 35 | 265.2 | 1740 | 1410.3 | 169.9 | 408.1 | 178.3 | 49.7 | 274.5 |
| 36 | 306.6 | 2846 | 753.6 | 141.1 | 241.3 | 516.5 | 112.2 | 307.4 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 37 | 441.8 | 182.5 | 666.4 | 164.8 | 262.4 | 355.8 | 217.6 | 253.3 |
| 38 | 390.4 | 378.8 | 13610.5 | 1317 | 946.6 | 761.7 | 296.8 | 5444.7 |
| 40 | 678 | 819.1 | 540.9 | 3014.7 | 512.9 | 629.2 | 400 | 556.2 |
| 41 | 644.1 | 499.6 | 729.1 | 155 | 425.8 | 379.6 | 508.2 | 453.1 |
| 42 | 291 | 418.9 | 301 | 279.7 | 205.7 | 234 | 453.1 | 140.6 |
| 43 | 2139.6 | 206.7 | 327.3 | 675.6 | 397 | 893.4 | 362.6 | 369.2 |
| 44 | 737 | 1115 | 1144.4 | 735.2 | 636.8 | 1352.5 | 377.3 | 421.9 |
| 45 | 1766.4 | 1105.2 | 390.4 | 587.3 | 440.9 | 35.5 | 1234.1 | 598.6 |
| 46 | 871.9 | 344.7 | 382.8 | 237.1 | 318 | 362.4 | 260.5 | 359.7 |
| 47 | 157.7 | 796 | 425.9 | 228.6 | 277.4 | 458.4 | 103.4 | 356.2 |
| 48 | 100.3 | 309.2 | 2616.3 | 124.4 | 634.6 | 1634.5 | 65.7 | 281.8 |
| 49 | 270.1 | 6992.3 | 2626.6 | 317.7 | 377.3 | 697.2 | 177.2 | 387.6 |
| 50 | 165.6 | 199.9 | 3637.3 | 652.6 | 305.4 | 478.1 | 71.2 | 1362.6 |
| 51 | 678.9 | 706.6 | 252.1 | 581.3 | 55.1 | 329.7 | 103.8 | 97.2 |
| 52 | 336 | 238.2 | 225.3 | 231.8 | 202.9 | 192.7 | 152.9 | 196.9 |
| 53 | 318.6 | 1664.1 | 277 | 119.1 | 102.4 | 73.8 | 168.1 | 127.6 |
| 54 | 230.9 | 1387 | 467.5 | 296.1 | 274.5 | 242.3 | 1255.6 | 479.9 |
| 55 | 100.5 | 82.4 | 82.1 | 91.1 | 106 | 121.4 | 99.6 | 99.4 |
| 56 | 18.8 | 321.8 | 83.3 | 126.2 | 51 | 49.7 | 267 | 79.7 |
| 57 | 2055.1 | 6894.7 | 1056.4 | 181 | 1040.6 | 898.5 | 320.9 | 6449.5 |
| 58 | 3611 | 7181.8 | 1246 | 192.6 | 956.8 | 1419.1 | 257.5 | 8484.9 |
| 59 | 652.5 | 2674.1 | 1061 | 813.9 | 1612.9 | 824.9 | 505.6 | 837.7 |
| 60 | 153.4 | 426.9 | 592.3 | 113.9 | 288.1 | 186.2 | 123.5 | 328.9 |
| 61 | 248.3 | 108 | 110.1 | 157.8 | 116.1 | 86.1 | 104.3 | 147.4 |
| 62 | 892.6 | 4240.6 | 2145.8 | 4532 | 1616.2 | 180.2 | 167.4 | 1671.5 |
| 63 | 43.1 | 696.1 | 228.6 | 152.7 | 248 | 203.2 | 222.5 | 1249.7 |
| 64 | 754.3 | 484.8 | 96.8 | 136.8 | 172.4 | 8787.9 | 99.3 | 336.8 |
| 65 | 83.2 | 218.3 | 730.9 | 106.4 | 207.9 | 403.2 | 75.9 | 215.4 |
| 66 | 493.5 | 1210 | 670.5 | 495.6 | 581.9 | 1176.5 | 951.4 | 686.8 |
| 67 | 10133.8 | 33324.8 | 24242 | 34583.3 | 33362.6 | 3068.3 | 31350 | 37066.4 |
| 68 | 2389.5 | 1201.4 | 1568 | 9401.9 | 2614.5 | 46.9 | 4918.3 | 1993.1 |
| 69 | 6293.3 | 2834.3 | 3650.8 | 13600.4 | 4671 | 198 | 6730.8 | 2110.2 |
| 70 | 7326.8 | 5218.4 | 1465.6 | 2301.9 | 1698 | 1562.6 | 3047.7 | 1292.1 |
| 71 | 109.3 | 5006.6 | 1167.7 | 193.9 | 743.9 | 3907.9 | 456.8 | 458.4 |
| 72 | 207.7 | 2313.9 | 1722.7 | 291.3 | 396.6 | 781 | 109 | 349.6 |
| 73 | 194.2 | 657.8 | 526.2 | 409.4 | 339.5 | 863 | 203.3 | 363.5 |
| 75 | 76.6 | 1227.8 | 331.8 | 313 | 290.4 | 824.9 | 86.8 | 175.5 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 76 | 2053.9 | 1453.3 | 198.2 | 1.4 | 166 | 90.4 | 4285.2 | 181.1 |
| 77 | 1780 | 1586 | 1427 | 6365.7 | 5262.6 | 227.6 | 2010.2 | 3182.5 |
| 78 | 481.4 | 317.2 | 437.6 | 789.5 | 715.2 | 564.6 | 535.8 | 202.4 |
| 79 | 461.6 | 214.6 | 214.2 | 96.5 | 110.5 | 1271 | 235.9 | 128.6 |
| 80 | 121.1 | 746.1 | 294 | 272.1 | 130.4 | 80.8 | 247.6 | 349.5 |
| 81 | 125.1 | 6473 | 2165.1 | 112.4 | 380.6 | 161.3 | 327.9 | 257.7 |
| 82 | 704.3 | 216.5 | 171 | 431.3 | 318.7 | 149.5 | 340 | 308.5 |
| 84 | 221.9 | 611.2 | 399.9 | 105.1 | 228.8 | 2138.2 | 106 | 204.7 |
| 85 | 269.2 | 233.6 | 1982.8 | 169 | 149.9 | 211.7 | 137.7 | 320.8 |
| 86 | 135.5 | 108.9 | 106.9 | 95.7 | 105.4 | 93.8 | 73.7 | 117.7 |
| 87 | 127.6 | 465.3 | 323.2 | 95.3 | 403.9 | 62.8 | 157.6 | 1429.6 |
| 88 | 1283.6 | 2522.4 | 1743.7 | 11815.2 | 3616.8 | 446.2 | 3870.7 | 3025.4 |
| 89 | 256.7 | 275.1 | 477.6 | 154.3 | 289.3 | 541.1 | 134.8 | 307.1 |
| 90 | 116.5 | 131.5 | 622.1 | 230.7 | 165.3 | 129.6 | 92.9 | 171.2 |
| 91 | 1291.4 | 6737.9 | 681 | 75.2 | 942 | 526 | 142.5 | 8893.2 |
| 92 | 127.7 | 8921.7 | 3533.8 | 2067.5 | 4220.7 | 3041.6 | 9733.8 | 1770.2 |
| 93 | 120.4 | 181.9 | 160.9 | 277.6 | 309.8 | 97.6 | 147.2 | 189.7 |
| 94 | 1.3 | 950.7 | 333.4 | 626.4 | 1123.6 | 927.6 | 636 | 725.2 |
| 95 | 63.1 | 671.5 | 509.6 | 309 | 1194.2 | 87.6 | 1502.3 | 486 |
| 96 | 163.4 | 343.5 | 183.1 | 221 | 134.2 | 332.6 | 215.1 | 172.7 |
| 97 | 174.6 | 412.9 | 129.6 | 240.3 | 192.1 | 109.1 | 232 | 131.9 |
| 98 | 269 | 46.9 | 23.6 | 5.9 | 40.3 | 461.4 | 247.8 | 29.4 |
| 99 | 317.3 | 330.4 | 345.2 | 231.4 | 230 | 327.3 | 224.5 | 258.3 |
| 100 | 278.9 | 5487.5 | 4658.3 | 2977.6 | 17708.1 | 533.6 | 704.6 | 14117.2 |
| 101 | 148.9 | 470.7 | 172.2 | 240.4 | 366.1 | 137.6 | 433.1 | 297.3 |
| 102 | 267.2 | 130.3 | 143.5 | 163 | 138 | 256.5 | 184.4 | 174.4 |
| 103 | 345.9 | 454.6 | 332.2 | 322.3 | 296 | 331.9 | 319.9 | 317.1 |
| 104 | 620.8 | 254.8 | 155.1 | 500.3 | 426.6 | 538.2 | 415.4 | 490.4 |
| 105 | 676.6 | 264.2 | 231.1 | 551.9 | 371.6 | 831.1 | 467.2 | 475.1 |
| 106 | 832.7 | 268.5 | 316.9 | 494.6 | 516.3 | 767.4 | 474.1 | 471.9 |
| 107 | 214.1 | 396.6 | 1610.7 | 203.7 | 296.7 | 656.5 | 164.1 | 329.5 |
| 108 | 430.9 | 225.7 | 420.7 | 408.9 | 306.7 | 300.6 | 149.3 | 296.7 |
| 109 | 144.5 | 5293.3 | 3445.6 | 49.3 | 99.8 | 63.9 | 4951.6 | 88.8 |
| 110 | 156.2 | 13367.4 | 4936.4 | 1.2 | 133.1 | 66.6 | 10420.4 | 80.6 |
| 111 | 31.1 | 449 | 319.1 | 972.4 | 1746.3 | 72.4 | 634.6 | 1582.3 |
| 112 | 1208.7 | 915.8 | 358.6 | 513 | 445.2 | 79.8 | 1002.1 | 451.1 |
| 113 | 257 | 526.1 | 411.1 | 260 | 302.4 | 165.9 | 228.3 | 166.5 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 114 | 219.5 | 492.8 | 315.8 | 675.8 | 574.1 | 743.9 | 193.5 | 408.5 |
| 115 | 266.9 | 1153.1 | 535 | 357.9 | 489.5 | 1534.5 | 313.6 | 476.9 |
| 116 | 239.1 | 559.7 | 345 | 285.6 | 292.6 | 498.3 | 203 | 270.4 |
| 117 | 451.4 | 852.5 | 1167.1 | 3758.5 | 2636.4 | 220 | 694.6 | 1851.1 |
| 118 | 691.9 | 236.5 | 502.4 | 98.3 | 512.6 | 440.9 | 312.8 | 488.6 |
| 119 | 125.2 | 496.5 | 189.8 | 66 | 278.8 | 218.8 | 77.4 | 289.7 |
| 120 | 643.2 | 122 | 264.2 | 153.9 | 186.9 | 624.7 | 139.7 | 154 |
| 121 | 749.5 | 2913.7 | 860.2 | 336.8 | 764 | 1394.6 | 129.9 | 463.5 |
| 122 | 460.8 | 265.7 | 184.8 | 120.8 | 246 | 58.9 | 86.9 | 235.1 |
| 123 | 207.9 | 251.7 | 204.8 | 140.6 | 122.2 | 149.6 | 111.5 | 124.4 |
| 124 | 5670.9 | 155.5 | 148 | 368.2 | 165.8 | 183.3 | 107.3 | 167.1 |
| 125 | 1479.5 | 3998.5 | 753.1 | 813.4 | 967.4 | 1104.9 | 793.8 | 1004.9 |
| 126 | 762.9 | 199.8 | 558.9 | 519.5 | 1369.1 | 191.1 | 682.8 | 864.4 |
| 127 | 111.6 | 161.4 | 71.8 | 154.2 | 116.4 | 116.1 | 96.1 | 113.7 |
| 128 | 270.9 | 268.1 | 265.5 | 253.8 | 194.8 | 249.5 | 193.3 | 261.6 |
| 129 | 178.9 | 974.6 | 1280 | 192.4 | 245.5 | 216.2 | 155.5 | 1485 |
| 130 | 93.9 | 415 | 135.9 | 64 | 98.9 | 84.4 | 187.3 | 78.1 |
| 131 | 79.9 | 649 | 99.1 | 56.7 | 110.5 | 120.9 | 82.5 | 81.5 |
| 132 | 246.1 | 248.4 | 231.5 | 176.3 | 238.7 | 261 | 172.1 | 211 |
| 133 | 132.4 | 196 | 55.9 | 78.8 | 66.9 | 67.3 | 116.7 | 83.1 |
| 134 | 80.7 | 357.4 | 130 | 244.7 | 105.4 | 224.6 | 226.1 | 57.7 |
| 135 | 206.7 | 527.6 | 330.5 | 525.9 | 476.5 | 254.4 | 334.4 | 358.2 |
| 138 | 146.5 | 176.3 | 495.1 | 127.6 | 180.2 | 387 | 83.2 | 571.2 |
| 139 | 125.6 | 176.9 | 93.3 | 107.1 | 113.1 | 161 | 90.5 | 99 |
| 140 | 1.1 | 2124.9 | 375.1 | 3699.3 | 699.4 | 58.6 | 710.1 | 1623.8 |
| 141 | 39.2 | 1376.8 | 980.8 | 1472.7 | 1866.8 | 95.6 | 1265 | 1674.3 |
| 144 | 438.1 | 9635.2 | 440.9 | 420.6 | 469.1 | 364.9 | 21511.5 | 484.8 |
| 145 | 295.9 | 239.9 | 182.7 | 181.3 | 218.7 | 151.5 | 152.5 | 257.7 |
| 146 | 298.9 | 424.8 | 178.8 | 181 | 250.5 | 196.8 | 180.3 | 248.8 |
| 147 | 48.8 | 115.3 | 160 | 38.6 | 86.5 | 34.7 | 27.4 | 52.8 |
| 148 | 301.5 | 1046.2 | 1135.9 | 223.2 | 407.2 | 165.1 | 181.9 | 387.1 |
| 149 | 47.3 | 241.2 | 214.2 | 458.8 | 455.6 | 29.3 | 104.5 | 303.3 |
| 150 | 170 | 537.9 | 266.4 | 204.9 | 218.7 | 213.6 | 102.9 | 241.9 |
| 151 | 89.2 | 551.3 | 254.9 | 165.8 | 534.6 | 208.7 | 145 | 347.2 |
| 153 | 86.5 | 128.6 | 144.8 | 102.4 | 114 | 131.9 | 482.5 | 102.5 |
| 154 | 89.9 | 4360.8 | 958.8 | 781.1 | 686.3 | 354.2 | 99.8 | 1301.7 |
| 155 | 231.3 | 491.3 | 148.5 | 113.4 | 129.9 | 135.7 | 457.7 | 250.3 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 156 | 106.4 | 562.9 | 116.7 | 63.7 | 114.5 | 94.8 | 103.6 | 100.3 |
| 157 | 215.1 | 148.4 | 161 | 134.5 | 165.7 | 219 | 111.3 | 154.4 |
| 158 | 32.9 | 1321.3 | 40.5 | 1.2 | 17.3 | 60.4 | 325.4 | 9.7 |
| 159 | 599.7 | 2783.1 | 1317.4 | 2648.2 | 4295.6 | 54 | 3352.4 | 1844.5 |
| 161 | 46.4 | 2664.3 | 142.6 | 105.4 | 37 | 79.3 | 12174.8 | 49 |
| 162 | 70.2 | 205.9 | 2154.4 | 120.2 | 230.1 | 302.5 | 26.7 | 575.3 |
| 164 | 97.2 | 4519.6 | 284.1 | 80.3 | 241.1 | 136.1 | 4278.6 | 145.2 |
| 166 | 1463.3 | 1937 | 2689 | 6646.8 | 6511.9 | 2436.2 | 4573.3 | 3272.4 |
| 167 | 213.4 | 1628.1 | 728.3 | 2340.5 | 4783.1 | 39.7 | 2419 | 1862.8 |
| 168 | 46.2 | 1573.6 | 390.5 | 1142.4 | 452.1 | 470.9 | 298.3 | 741.5 |
| 169 | 5537.1 | 13680.4 | 14101.2 | 2418 | 4999.2 | 901.3 | 1483.3 | 4751.3 |
| 170 | 246.1 | 2210.5 | 661.2 | 1664.4 | 916.3 | 216.4 | 2618.5 | 589.3 |
| 171 | 163.7 | 592.2 | 6989.4 | 403.5 | 2812.8 | 5944.7 | 1.2 | 2024.1 |
| 172 | 503.1 | 636.3 | 612.7 | 637.8 | 678.8 | 382.4 | 649.1 | 592.8 |
| 173 | 438.7 | 1492.9 | 435.8 | 581.6 | 451.6 | 406.1 | 375.7 | 321.6 |
| 176 | 1.2 | 4078.3 | 151.3 | 721.4 | 41.7 | 21.5 | 659.5 | 126.2 |
| 177 | 504.8 | 573.3 | 573.5 | 497.6 | 548.9 | 188.7 | 524.1 | 641 |
| 178 | 763.2 | 2581.3 | 274.3 | 130.1 | 130.5 | 395.3 | 1441.3 | 106 |
| 179 | 143.7 | 1098.5 | 182.3 | 83.9 | 71.1 | 89.7 | 62.5 | 528.9 |
| 180 | 146.2 | 930.7 | 373.3 | 106 | 151.6 | 300.6 | 164 | 186.4 |
| 181 | 9206.5 | 6564.9 | 6165.7 | 13167.3 | 11548.7 | 1304.8 | 7115.7 | 9061 |
| 182 | 263.8 | 376.5 | 668.2 | 414.6 | 507 | 1400.6 | 232.9 | 393.9 |
| 183 | 1.2 | 395.2 | 3392.4 | 216.2 | 953.9 | 3944.8 | 144.7 | 1249.2 |
| 184 | 228.5 | 3067.2 | 599.8 | 207 | 334.4 | 400.4 | 10162.8 | 380.1 |
| 185 | 931 | 4108 | 3749.7 | 1981.6 | 1615.9 | 3634.4 | 834.2 | 6945.4 |
| 186 | 493.3 | 324.2 | 396.2 | 656.7 | 709.1 | 1183.3 | 453.3 | 462 |
| 187 | 96.3 | 258.3 | 1535.7 | 409.2 | 314.3 | 2757.2 | 67.4 | 650.9 |
| 188 | 66.9 | 405.5 | 832.9 | 151.3 | 247.2 | 306.9 | 89.3 | 144.4 |
| 189 | 70.6 | 239.2 | 289.3 | 112 | 199.7 | 237.6 | 59 | 78.6 |
| 190 | 1.3 | 1113.6 | 243.5 | 38 | 52 | 103.1 | 101.1 | 159.9 |
| 191 | 898.6 | 2228 | 2806.6 | 1788.2 | 1533.1 | 466 | 498.6 | 1912.7 |
| 192 | 103.7 | 377 | 267 | 65.3 | 134.7 | 128.9 | 66.1 | 119.5 |
| 193 | 140.5 | 752.9 | 580.6 | 2185.8 | 876.8 | 67.5 | 271.5 | 561.8 |
| 194 | 60.2 | 320.3 | 198.5 | 159.6 | 173.5 | 96 | 126.9 | 140.9 |
| 195 | 308.8 | 210.3 | 178.6 | 401.4 | 266.1 | 175.7 | 367.5 | 230.8 |
| 196 | 5889.2 | 6514.4 | 4504.5 | 4416.3 | 7685 | 183.7 | 6854.2 | 7068.9 |
| 197 | 374.5 | 81.9 | 129.2 | 108.8 | 122 | 174.9 | 68.1 | 178.1 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 198 | 95.3 | 300 | 73.2 | 42.5 | 55.8 | 86.5 | 39.2 | 77.1 |
| 199 | 87.1 | 7825.9 | 10594.6 | 51.3 | 344.6 | 74.9 | 63 | 274.1 |
| 200 | 130.8 | 511 | 514 | 170.4 | 544.7 | 475.6 | 154.9 | 259.7 |
| 201 | 1208.7 | 3427.4 | 3404.4 | 38.8 | 160.1 | 168.6 | 42.3 | 918.4 |
| 203 | 6000.1 | 982.6 | 359.1 | 96.8 | 204.8 | 46.1 | 331.8 | 188.3 |
| 204 | 206 | 700.4 | 262.9 | 650.9 | 591.3 | 846.3 | 674.6 | 437.2 |
| 205 | 1762.1 | 5866.2 | 3715.3 | 2132.7 | 5805.3 | 149.5 | 5381.1 | 2955.4 |
| 206 | 597.2 | 208.1 | 13097.8 | 301.2 | 1950.7 | 2675.5 | 252.6 | 329.8 |
| 207 | 77.6 | 602.1 | 745.6 | 352.7 | 484.2 | 308.4 | 1182.9 | 575 |
| 208 | 283.8 | 306.2 | 451.2 | 156.5 | 329.6 | 1044.1 | 156.9 | 149.8 |
| 209 | 254.3 | 1864.3 | 892.2 | 4970.4 | 5671.9 | 5534 | 2026.3 | 2662.4 |
| 211 | 262.3 | 3358.1 | 4615 | 29093.4 | 15999.8 | 393.6 | 7278.5 | 12689.2 |
| 212 | 1608.1 | 1431.5 | 332.2 | 551.6 | 514.2 | 482.4 | 786.4 | 353.9 |
| 213 | 3281.6 | 97.3 | 108.3 | 107.5 | 105.4 | 160 | 99.5 | 104.5 |
| 215 | 89.6 | 862.3 | 617 | 432.2 | 796.7 | 441.3 | 1738.3 | 806.2 |
| 216 | 333.1 | 284 | 385.1 | 651 | 910.8 | 264.4 | 588 | 601.9 |
| 217 | 224.3 | 1692 | 513.5 | 290.3 | 372.2 | 448.4 | 238.7 | 272.8 |
| 218 | 271.4 | 191 | 194.3 | 230.9 | 353.1 | 288.6 | 279.7 | 263.7 |
| 220 | 26824.2 | 132 | 73.9 | 124.1 | 470.8 | 129.2 | 103.4 | 350.5 |
| 221 | 215 | 1009.5 | 780.2 | 674.9 | 632.3 | 1487 | 406.7 | 578.3 |
| 223 | 352.8 | 388.2 | 438 | 286.9 | 203.4 | 160.1 | 124.3 | 1200.6 |
| 227 | 565.2 | 503.9 | 16406 | 1473.6 | 1813.5 | 2226.4 | 314.3 | 5151.9 |
| 228 | 624.4 | 455.2 | 331.2 | 808.6 | 799.2 | 205.8 | 683.1 | 604 |
| 229 | 1426.6 | 209.6 | 275.1 | 972.8 | 180.9 | 357.1 | 249.2 | 538.6 |
| 230 | 281.7 | 1542.1 | 611.5 | 209 | 207.8 | 215.8 | 97 | 574 |
| 231 | 2885.2 | 380.6 | 200 | 67.8 | 164.2 | 95.5 | 166.1 | 101.5 |
| 232 | 58 | 1351.9 | 13448.1 | 771.8 | 6104.8 | 19484.1 | 294.7 | 4379.3 |
| 233 | 2461.3 | 14608.9 | 11362.4 | 5629.1 | 11330.5 | 8712.7 | 1500.9 | 5870.1 |
| 235 | 317.4 | 465.1 | 5668.6 | 250.5 | 1690.5 | 2382.3 | 71.8 | 932.8 |
| 236 | 179.8 | 493.3 | 282.1 | 229.7 | 368.1 | 466.5 | 98.5 | 194.2 |
| 237 | 734 | 1979.9 | 2226.6 | 2347 | 1794.9 | 1757.7 | 790 | 2552.2 |
| 238 | 269.2 | 1084.1 | 803.1 | 718.3 | 1198.8 | 688.1 | 706.9 | 861.9 |
| 239 | 101.2 | 896.8 | 1025.8 | 156.8 | 1210.1 | 429.8 | 176 | 647.7 |
| 240 | 117.5 | 1116.1 | 1919.1 | 210.2 | 1169.1 | 420.8 | 189.3 | 1162.5 |
| 241 | 173.9 | 2109.5 | 3105.6 | 444 | 2419 | 956.7 | 341 | 2001 |
| 242 | 43673.6 | 18485.8 | 19181.5 | 4555.3 | 1630.4 | 3478.5 | 1273.2 | 10481.9 |
| 243 | 157.3 | 341.9 | 1232.7 | 219.9 | 483 | 699.2 | 82.1 | 1003.1 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 244 | 115 | 746.8 | 224.6 | 148.2 | 196.6 | 321 | 261 | 166.4 |
| 246 | 153.2 | 1046.6 | 224.3 | 103.9 | 143.7 | 173.8 | 1360.9 | 276.9 |
| 247 | 446 | 126.1 | 129.3 | 102.9 | 139 | 134.8 | 77.7 | 138.1 |
| 248 | 202.4 | 3011.8 | 309.9 | 182.5 | 128.8 | 133.9 | 1016.4 | 127.7 |
| 249 | 196.3 | 2527.3 | 1726.5 | 1395.5 | 337.1 | 271.3 | 233.7 | 469.7 |
| 251 | 386.5 | 1104.2 | 346.8 | 127.5 | 90.8 | 190.6 | 175.2 | 116.5 |
| 253 | 173.5 | 286.7 | 365.9 | 263 | 403.6 | 544.3 | 306.8 | 269.1 |
| 254 | 121 | 1348.6 | 394.8 | 73 | 434.2 | 259.8 | 554.3 | 264.1 |
| 255 | 953.8 | 2792.9 | 253.9 | 137.5 | 120.4 | 451.3 | 1497.2 | 98.4 |
| 256 | 1230.9 | 5411.2 | 704.5 | 85.2 | 918.6 | 465.8 | 224.1 | 7960.4 |
| 257 | 196 | 284.4 | 209 | 154.9 | 200.8 | 57.7 | 75.2 | 165.8 |
| 258 | 596.3 | 1068.8 | 1617.2 | 24.7 | 209.4 | 33.6 | 45.7 | 129.9 |
| 259 | 3.3 | 934.8 | 2120.9 | 131.2 | 687.3 | 806.3 | 46 | 531.9 |
| 260 | 248.3 | 182.9 | 167.4 | 127.1 | 173.3 | 209.8 | 128.2 | 198.6 |
| 261 | 19 | 401.9 | 345.1 | 1066.3 | 184.5 | 629.8 | 66.6 | 326.5 |
| 263 | 3859.5 | 516.2 | 662.8 | 577.5 | 720.7 | 535.7 | 488.6 | 407 |
| 263 | 418.6 | 1350.1 | 772.4 | 214.4 | 221.7 | 353.8 | 173.2 | 599.8 |
| 265 | 369.8 | 167.3 | 372.9 | 453.3 | 248.6 | 93.4 | 268.9 | 414.8 |
| 266 | 1779.8 | 263 | 318.1 | 407 | 491.3 | 511.1 | 236.7 | 401.1 |
| 256 | 117.2 | 391.4 | 130.1 | 92.5 | 181.8 | 142.5 | 146 | 152 |
| 268 | 120.1 | 1119 | 310.7 | 819.2 | 1655.3 | 121.7 | 645.8 | 1264.1 |
| 269 | 333.7 | 121.5 | 82.3 | 48 | 46.9 | 55.7 | 27.4 | 63.7 |
| 270 | 521.6 | 206.4 | 175.2 | 475.9 | 301.9 | 165.7 | 234.5 | 239.6 |
| 271 | 251.7 | 2043.7 | 362.9 | 255.5 | 197.7 | 227 | 225.6 | 192.4 |
| 272 | 620.4 | 563.5 | 600.9 | 411.3 | 451 | 639.9 | 426.2 | 524.5 |
| 273 | 136.7 | 2008 | 783.2 | 9402.2 | 3170.7 | 37.5 | 2967.5 | 1970.3 |
| 274 | 436.6 | 1279.4 | 1048.7 | 803.4 | 803.2 | 1605 | 278.3 | 616.8 |
| 275 | 267.1 | 1400.8 | 971.4 | 450.3 | 462.3 | 1341.1 | 238.2 | 2631.7 |
| 276 | 711.3 | 764 | 25930 | 2051.7 | 3808.8 | 5060.2 | 400.3 | 7898.3 |
| 277 | 188.1 | 213.8 | 210 | 151.7 | 217.1 | 196.7 | 161.8 | 199.4 |
| 278 | 65.7 | 1341 | 3512.1 | 262.6 | 1386.1 | 710.4 | 64.3 | 510.5 |
| 279 | 577.5 | 3255 | 2105.6 | 1256.3 | 3760.9 | 49.6 | 3058.3 | 2014.5 |
| 280 | 876.1 | 4218.8 | 2673.7 | 1739.2 | 3129.6 | 176.5 | 3883.1 | 2142.3 |
| 281 | 33.4 | 653.2 | 169.7 | 48.5 | 118.7 | 39 | 363.8 | 289 |
| 282 | 387.6 | 472.5 | 331.7 | 252.3 | 284.5 | 371.6 | 219.7 | 694.4 |
| 283 | 584.8 | 297.3 | 224 | 322.6 | 357.9 | 395.3 | 382.4 | 244 |
| 284 | 124.9 | 130.1 | 89.2 | 53.1 | 74.7 | 68.2 | 61.3 | 104.6 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 285 | 154 | 100.9 | 114.9 | 90.5 | 98.7 | 114.7 | 98.4 | 113.9 |
| 286 | 14.5 | 91.5 | 472.1 | 1032.5 | 277 | 1968 | 1525.5 | 442.2 |
| 287 | 744.2 | 114.2 | 131.4 | 94.7 | 147.1 | 127.5 | 65.1 | 206.5 |
| 288 | 245.8 | 103.9 | 143.6 | 161.5 | 162.8 | 177.7 | 112.5 | 161.4 |
| 289 | 231.3 | 5107.2 | 4629.4 | 93.7 | 1685.9 | 4764.5 | 141.9 | 2073.7 |
| 291 | 67.2 | 135.6 | 201.2 | 185.4 | 144.5 | 245.6 | 73.8 | 128.4 |
| 292 | 43.6 | 1910.5 | 2535.8 | 72.3 | 371.6 | 4192.7 | 50.7 | 206.7 |
| 293 | 63.9 | 1213.8 | 284.1 | 54.5 | 117 | 102.3 | 188.8 | 561 |
| 295 | 329 | 1994.3 | 668.6 | 211.8 | 267.3 | 97.1 | 684.5 | 3454 |
| 296 | 1451.1 | 1263.3 | 1341.5 | 761.3 | 1067.5 | 1967.3 | 758.9 | 687.7 |
| 297 | 593 | 73.8 | 109.1 | 49.4 | 123.5 | 88 | 42.9 | 364.8 |
| 298 | 360 | 444.6 | 275.9 | 300.9 | 273.2 | 358.5 | 306.5 | 436 |
| 299 | 1094.3 | 2223.7 | 794.8 | 1242.1 | 1051.7 | 1871.6 | 1233.7 | 605.2 |
| 300 | 186 | 1200.8 | 322.3 | 408.7 | 137.5 | 150.8 | 1157.7 | 233.1 |
| 301 | 250.2 | 525.4 | 365.9 | 4246.8 | 594.8 | 155.1 | 545.6 | 329.5 |
| 302 | 28.2 | 82.4 | 44.3 | 22.7 | 36.1 | 25.7 | 6.3 | 27.7 |
| 303 | 1397.3 | 375.7 | 390.8 | 921 | 836.9 | 531.6 | 689.2 | 589 |
| 304 | 485.2 | 1102.9 | 799.5 | 659.6 | 239.7 | 1106.9 | 120.6 | 501.1 |
| 305 | 1523.9 | 661.1 | 201.8 | 144.2 | 192.3 | 247.8 | 195.8 | 173 |
| 306 | 222.8 | 363.6 | 184.3 | 150.2 | 197.8 | 184.8 | 128.9 | 208.3 |
| 307 | 180.1 | 571.1 | 188 | 169.2 | 150.6 | 127.7 | 165.3 | 198 |
| 308 | 3026.7 | 857.8 | 1219.5 | 1191.4 | 1726.2 | 64.1 | 1855.4 | 1201.9 |
| 310 | 932.8 | 136.9 | 271.1 | 1036.8 | 498.7 | 99 | 290.2 | 343.2 |
| 311 | 48.5 | 360 | 99.1 | 180.9 | 152.4 | 76.1 | 167.7 | 104.5 |
| 312 | 4628 | 5 | 236.7 | 2257.7 | 1039.8 | 66 | 643.8 | 643 |
| 313 | 126.7 | 420.1 | 135.6 | 121 | 78.1 | 101 | 622.3 | 132.7 |
| 314 | 119.1 | 1765 | 872.1 | 133.6 | 629.1 | 211.4 | 112.9 | 473 |
| 315 | 141.7 | 212.9 | 441.6 | 286.4 | 519.9 | 1377.8 | 271.7 | 192.2 |
| 316 | 119.8 | 562.5 | 354.9 | 220.7 | 270.5 | 441.8 | 189.1 | 436.3 |
| 317 | 178.4 | 694.3 | 216.3 | 177.3 | 204.7 | 218.1 | 156.9 | 178.7 |
| 318 | 28.3 | 646.1 | 105.3 | 30.6 | 143.4 | 43.8 | 88.5 | 101.1 |
| 319 | 1682.7 | 339.1 | 154 | 339.5 | 190.3 | 129.4 | 447.1 | 293.2 |
| 320 | 816.1 | 326.7 | 425.5 | 913.6 | 680.7 | 459.9 | 482.1 | 501.5 |
| 321 | 360.2 | 245.2 | 811.4 | 486.9 | 413.3 | 931.3 | 260.1 | 454.2 |
| 322 | 604.1 | 279.1 | 205.3 | 203 | 203.2 | 198.1 | 215.6 | 180.1 |
| 323 | 620.6 | 4592.1 | 1478.5 | 13669.8 | 1883 | 297 | 3464 | 1551 |
| 324 | 106.1 | 478.7 | 118.1 | 131.8 | 77.6 | 74.8 | 294.8 | 56.2 |

(continued)

| 3g | | | | | | | | |
|---|---|---|---|---|---|---|---|---|
| SEQ ID NO: | 902TT | 903TT | 904TT | 907TT | 908TT | 909TT | 910TT | 913TT |
| 325 | 255.2 | 515.4 | 238.4 | 250.8 | 279.1 | 142.4 | 597.5 | 247.7 |
| 326 | 318.5 | 3462.4 | 2655.2 | 2527.1 | 3852.6 | 220.7 | 891.6 | 2803.7 |
| 327 | 64.1 | 44.3 | 86.6 | 1.3 | 52 | 25.3 | 11.5 | 100.1 |
| 328 | 58.8 | 534.2 | 264.2 | 543.1 | 719.4 | 95.4 | 544.4 | 515.6 |
| 329 | 122.4 | 66.8 | 60.1 | 62.8 | 59.5 | 91.3 | 45.4 | 74.1 |
| 330 | 49 | 1131.8 | 126.4 | 41.5 | 57.5 | 131.5 | 31.9 | 64 |
| 331 | 632.1 | 258 | 263 | 307.2 | 275.1 | 480 | 248.3 | 284.1 |
| 333 | 147 | 285.7 | 369.3 | 854.9 | 519 | 285.3 | 603 | 413.2 |
| 334 | 129 | 302.9 | 300.5 | 121 | 170.3 | 145.9 | 129.4 | 211.5 |
| 335 | 215.5 | 628.5 | 174.5 | 108 | 107.2 | 135.9 | 51.5 | 116.8 |
| 336 | 358.7 | 245.7 | 230.7 | 382.1 | 500.4 | 819.1 | 394.5 | 415.4 |
| 337 | 105.8 | 1112.7 | 1023.2 | 358 | 235.6 | 1517.6 | 97.2 | 364 |
| 338 | 134.6 | 567.8 | 499.9 | 131.2 | 238.2 | 332.7 | 132 | 278.1 |
| 339 | 335.4 | 352.2 | 311 | 385 | 625.4 | 323.2 | 314.7 | 428.2 |
| 340 | 303.2 | 343.2 | 263.9 | 231 | 263.3 | 223.5 | 177.2 | 315.9 |
| 341 | 408.4 | 464.7 | 438.1 | 1146.6 | 803.2 | 349.7 | 511.1 | 757.8 |
| 343 | 1287 | 814.6 | 228 | 123.5 | 59.8 | 75.2 | 658.4 | 97.9 |
| 344 | 2683.4 | 670.4 | 822.4 | 1937.2 | 1561.2 | 1319.5 | 1187.9 | 1110.2 |
| 345 | 270.8 | 356.3 | 270.7 | 213.6 | 219.2 | 281.7 | 199.4 | 223.6 |
| 347 | 321.3 | 3161.8 | 1516.8 | 2286.9 | 3928.3 | 682.1 | 1998.6 | 3960.1 |
| 348 | 592.8 | 6727.1 | 3096.6 | 4465.5 | 7652.3 | 3345.7 | 4616.8 | 6087.1 |
| 349 | 213.8 | 458.9 | 441.6 | 165.6 | 239.7 | 177.8 | 310.8 | 270.7 |
| 3350 | 139.5 | 477.9 | 625.9 | 282.7 | 381.8 | 1996.1 | 328.4 | 149.7 |
| 351 | 45.6 | 2277.1 | 515.7 | 158 | 453.8 | 105.4 | 294.7 | 668.6 |
| 352 | 470.6 | 167.1 | 213.9 | 516.8 | 305.7 | 2359 | 246.1 | 210.6 |
| 353 | 792.6 | 308.9 | 297.2 | 170.8 | 260.9 | 153.9 | 208.2 | 337.5 |

| 3h | | | | | | | | |
|---|---|---|---|---|---|---|---|---|
| SEQ ID NO: | 914TT | 915TT | 917TT | 918TT | 919TT | 920TT | 921TT | 922TT |
| 354 | 71.9 | 52.3 | 102.4 | 6332.8 | 339.5 | 28.3 | 586.4 | 1.1 |
| 355 | 77.9 | 90.7 | 59.8 | 865.8 | 218.8 | 75.4 | 827.6 | 77.1 |
| 356 | 309.5 | 389.3 | 791.7 | 762.2 | 694.7 | 578.2 | 1581.5 | 949.2 |
| 357 | 238.7 | 565.6 | 645.2 | 593.2 | 611.7 | 800.3 | 666.9 | 768.9 |
| 358 | 812.3 | 933.3 | 620.4 | 729.2 | 758.3 | 710.1 | 556.9 | 571.9 |
| 359 | 399.3 | 508 | 376.9 | 330 | 320.8 | 353.6 | 299.6 | 506 |
| 362 | 527.8 | 508.3 | 517.3 | 1171.8 | 669.6 | 758.3 | 407.4 | 431.6 |

(continued)

| 3h | | | | | | | | |
|---|---|---|---|---|---|---|---|---|
| SEQ ID NO: | 914TT | 915TT | 917TT | 918TT | 919TT | 920TT | 921TT | 922TT |
| 360 | 287.6 | 262.1 | 276.8 | 458.5 | 477.6 | 256.8 | 248.9 | 297.3 |
| 361 | 153.4 | 816.8 | 853.1 | 453.3 | 532.5 | 1170.9 | 1300 | 542.2 |
| 363 | 631.4 | 726.4 | 557.1 | 448.6 | 429.4 | 599.4 | 429.9 | 590.7 |
| 1 | 770.6 | 362.7 | 797.9 | 661.6 | 799.3 | 756.1 | 848.9 | 856.2 |
| 2 | 481 | 3910.3 | 1810.9 | 1338.3 | 3134.1 | 3947 | 4719 | 2783.8 |
| 7 | 299.1 | 2389.1 | 790.1 | 343.4 | 419.8 | 1494.7 | 829.3 | 845.9 |
| 8 | 614.3 | 810.2 | 763.6 | 1902.7 | 1081.7 | 629.1 | 831.6 | 938.2 |
| 9 | 524.5 | 1890.1 | 9055.3 | 9890.7 | 8192.7 | 2264.6 | 3685.8 | 10424.8 |
| 10 | 169.8 | 317.6 | 42 | 79.2 | 58.4 | 136.2 | 120 | 40.5 |
| 11 | 146 | 141.2 | 351.8 | 282.6 | 234.9 | 269.5 | 274.6 | 155.7 |
| 13 | 104.4 | 96.3 | 115.4 | 412.7 | 501.8 | 91 | 136.4 | 182.4 |
| 14 | 150.5 | 149.2 | 276.7 | 1839.8 | 1222 | 149.8 | 335.3 | 427.4 |
| 15 | 83.6 | 639.9 | 331.3 | 203.1 | 1168.9 | 304.5 | 277.5 | 582.7 |
| 16 | 834 | 740.5 | 1080.2 | 1040.9 | 977.4 | 761 | 1018.5 | 1197.7 |
| 17 | 95.8 | 775.8 | 1324.9 | 1290.5 | 1245.3 | 1170.3 | 1306.3 | 917.3 |
| 19 | 261.4 | 404.1 | 541.1 | 316.8 | 4263.1 | 155.9 | 150.6 | 3899.9 |
| 20 | 1.3 | 44.2 | 15.6 | 83.6 | 83 | 31 | 1.4 | 1.1 |
| 21 | 383.4 | 16.8 | 145.6 | 554.6 | 432 | 35.5 | 42.5 | 144.6 |
| 22 | 483.4 | 7044.6 | 9872.2 | 5621.2 | 6671.8 | 7593.9 | 4117.5 | 6209.5 |
| 23 | 1.2 | 758 | 271.8 | 277.7 | 2394.8 | 1.1 | 325.3 | 627.9 |
| 24 | 29 | 304.7 | 103.5 | 271.5 | 469.3 | 49.8 | 178.9 | 196.8 |
| 26 | 161.3 | 2148.7 | 2238.7 | 435.1 | 15713.7 | 60.3 | 774.4 | 7022.3 |
| 27 | 2027.3 | 253.6 | 510.6 | 701 | 703.8 | 324.3 | 324 | 728.4 |
| 29 | 167.6 | 74.4 | 68.6 | 190.8 | 125.6 | 73.1 | 84.8 | 170.1 |
| 30 | 1.1 | 246 | 188 | 525.7 | 759.9 | 74.5 | 129.7 | 286.6 |
| 31 | 256.3 | 146 | 449.7 | 1402.9 | 328.4 | 110.8 | 218.2 | 222.7 |
| 33 | 69.4 | 1248.9 | 534.4 | 1635.5 | 1154 | 322.1 | 691.6 | 434.8 |
| 34 | 2249.5 | 2411.8 | 4154.5 | 3039.8 | 4215.7 | 4564.8 | 2908.5 | 3979 |
| 35 | 118.8 | 296.6 | 55.5 | 1050.9 | 413 | 100.2 | 126.5 | 95 |
| 36 | 230.8 | 313.4 | 160.9 | 183.5 | 250.9 | 153.4 | 140.4 | 242.6 |
| 37 | 313.2 | 317.7 | 214.6 | 190.8 | 269.8 | 183.3 | 173.6 | 197.9 |
| 38 | 61.3 | 851.9 | 351.3 | 1044.4 | 981.7 | 465.1 | 612.9 | 276.6 |
| 40 | 719.3 | 813.3 | 549.5 | 535.4 | 513 | 829.2 | 442.3 | 543.7 |
| 41 | 172.6 | 171 | 190 | 284.7 | 581.1 | 145.2 | 362.8 | 549.8 |
| 42 | 343.9 | 273.7 | 144 | 130.6 | 210.4 | 229.7 | 202.5 | 160 |
| 43 | 1155.5 | 721.6 | 513.5 | 373.6 | 260 | 617.8 | 269.7 | 355.8 |
| 44 | 467.8 | 606.3 | 633.6 | 727.9 | 790.8 | 640 | 420.7 | 335.6 |

(continued)

| 3h SEQ ID NO: | 914TT | 915TT | 917TT | 918TT | 919TT | 920TT | 921TT | 922TT |
|---|---|---|---|---|---|---|---|---|
| 45 | 1102.5 | 638.1 | 519.3 | 594.7 | 368.8 | 478.2 | 608.5 | 404.8 |
| 46 | 443.3 | 467.6 | 402.9 | 285.6 | 318.8 | 295.6 | 260.9 | 360.4 |
| 47 | 246.9 | 143.9 | 102.7 | 241.9 | 285.2 | 145.3 | 226 | 182.5 |
| 48 | 48.2 | 197.7 | 222.2 | 49.7 | 1024.9 | 23 | 23 | 465.8 |
| 49 | 234.5 | 355.6 | 207.3 | 1446.9 | 736.1 | 344.1 | 252.2 | 364.3 |
| 50 | 42 | 399.9 | 174.3 | 301.5 | 536.5 | 71.4 | 207.3 | 223.1 |
| 51 | 444.2 | 59.2 | 128.9 | 228.8 | 73.7 | 65.9 | 19.4 | 52.1 |
| 52 | 203.3 | 187.7 | 224 | 202.7 | 200.7 | 180.8 | 198.3 | 190.4 |
| 53 | 186.9 | 1.2 | 213.7 | 202.1 | 69.2 | 75.4 | 108.4 | 128.1 |
| 54 | 684.7 | 417.1 | 501.3 | 720.5 | 410.5 | 294 | 509.8 | 366.4 |
| 55 | 129.4 | 96.5 | 115.6 | 98.9 | 115.5 | 100.7 | 94.9 | 72.2 |
| 56 | 247.2 | 93.8 | 50 | 62 | 65.7 | 100.5 | 74 | 62.3 |
| 57 | 713.4 | 745.5 | 417.1 | 4396.2 | 674.3 | 127.1 | 1311.9 | 226 |
| 58 | 679.3 | 672.7 | 334.5 | 5289.1 | 905 | 113.7 | 1260.3 | 158.8 |
| 59 | 1114.3 | 488.5 | 1847.4 | 1440.1 | 1675.2 | 906.2 | 2348 | 1103.1 |
| 60 | 160.5 | 166.8 | 216.5 | 231.6 | 179.7 | 142.2 | 165.1 | 203.4 |
| 61 | 293.8 | 239.7 | 136.1 | 128 | 145.9 | 155.4 | 122.5 | 116.8 |
| 62 | 2975 | 2562.6 | 1652.4 | 1873.2 | 1527 | 526 | 60.4 | 3206.2 |
| 63 | 144.6 | 161.3 | 490.3 | 578.9 | 239.3 | 242 | 239.6 | 251.9 |
| 64 | 1905.1 | 179 | 138 | 290.9 | 184.1 | 124 | 138.2 | 156.2 |
| 65 | 54 | 106 | 187.6 | 155.7 | 243.6 | 75 | 166.3 | 146.9 |
| 66 | 676.3 | 527.1 | 840.5 | 1026.2 | 765.5 | 627.5 | 1284.4 | 290.3 |
| 67 | 33082 | 52249.5 | 37966 | 30570.8 | 32361.9 | 42996.5 | 29286 | 39242.9 |
| 68 | 4579.1 | 7275.8 | 2857.3 | 1137.2 | 4016.2 | 7822.9 | 2361.2 | 3436.9 |
| 69 | 7472.6 | 15249.7 | 5078.7 | 2335.4 | 5252.6 | 10446.3 | 7213.9 | 6679.9 |
| 70 | 9607.7 | 1458 | 3227.3 | 1337.8 | 1473.1 | 1664.6 | 444 | 1375.9 |
| 71 | 234 | 129.9 | 307.4 | 332.1 | 2083.3 | 87 | 105.8 | 493.5 |
| 72 | 117.7 | 616.5 | 195.6 | 1937.6 | 446.3 | 359.7 | 707 | 166.5 |
| 73 | 299.5 | 310.6 | 290.6 | 264.1 | 473 | 267.6 | 370.2 | 457.7 |
| 75 | 167.6 | 215.7 | 252.1 | 193 | 312.5 | 406 | 77.3 | 143.2 |
| 76 | 987.1 | 33.3 | 18.9 | 74.9 | 191.4 | 9.9 | 122.1 | 134.1 |
| 77 | 3486.8 | 9405.2 | 5866.2 | 3917.2 | 3054.2 | 10710.9 | 3845.4 | 7445 |
| 78 | 322.9 | 806.6 | 1125.4 | 721.3 | 791.7 | 1090.1 | 887.2 | 548.7 |
| 79 | 156.3 | 75.4 | 84.6 | 199.2 | 122.3 | 105 | 76.6 | 133.8 |
| 80 | 577.4 | 115.1 | 104.4 | 407.6 | 226.3 | 186.6 | 758.9 | 124.9 |
| 81 | 120.4 | 121.1 | 187.8 | 1222.5 | 315.8 | 185 | 177.3 | 358 |
| 82 | 480.2 | 436.7 | 398.1 | 285.9 | 256.1 | 480.6 | 350.3 | 306.7 |

(continued)

| 3h | | | | | | | | |
|---|---|---|---|---|---|---|---|---|
| SEQ ID NO: | 914TT | 915TT | 917TT | 918TT | 919TT | 920TT | 921TT | 922TT |
| 84 | 122.6 | 108.4 | 185.4 | 180.2 | 503.7 | 117.8 | 89.6 | 217.3 |
| 85 | 172.1 | 158.7 | 146.6 | 1050.6 | 166.4 | 134.4 | 197.3 | 142.6 |
| 86 | 99.3 | 87.7 | 80.9 | 78.1 | 93.6 | 56 | 77.2 | 98.4 |
| 87 | 346.4 | 214.6 | 478.3 | 693.6 | 282.7 | 172 | 329.4 | 231.5 |
| 88 | 2119.7 | 12099.4 | 6271.8 | 3285.9 | 4646.7 | 8968.2 | 1915.3 | 9385.7 |
| 89 | 308.6 | 290.4 | 303 | 232.4 | 299.7 | 233.4 | 163.8 | 190.9 |
| 90 | 98.7 | 279.4 | 108.4 | 89 | 113.4 | 81.7 | 100.6 | 149.5 |
| 91 | 582.6 | 482.3 | 275 | 6847.8 | 528.1 | 61.1 | 1397.9 | 71.5 |
| 92 | 2641.5 | 1419.6 | 2112.8 | 3432 | 3134.2 | 1442.4 | 4677.3 | 2547 |
| 93 | 88.7 | 211.3 | 338.9 | 402.7 | 236.9 | 286.5 | 297.3 | 310.6 |
| 94 | 89.7 | 1072.4 | 937.7 | 516.7 | 1228 | 974.1 | 1427 | 1237.6 |
| 95 | 510.2 | 756.1 | 652.5 | 461.5 | 647.6 | 779.5 | 500.1 | 747.8 |
| 96 | 142.3 | 129.6 | 170.2 | 169.9 | 161.5 | 179.1 | 155.6 | 168.2 |
| 97 | 169.9 | 186.3 | 239.2 | 240.4 | 173.7 | 176.8 | 317.9 | 243.4 |
| 98 | 37 | 25.2 | 39.9 | 87.1 | 52.8 | 25.5 | 28.4 | 39.9 |
| 99 | 287.4 | 268.3 | 235.3 | 210.6 | 248.3 | 226.3 | 210.8 | 292.3 |
| 100 | 289.9 | 8016.1 | 3817.9 | 5438.1 | 6332.1 | 6494.4 | 1146 | 13390 |
| 101 | 271.7 | 593.1 | 444.8 | 210.9 | 231.5 | 571.7 | 327.5 | 268.8 |
| 102 | 244.7 | 270.9 | 155.8 | 164.2 | 185.3 | 197.2 | 148.5 | 149 |
| 103 | 359.8 | 424.3 | 368.8 | 345.3 | 325.6 | 402.3 | 249.8 | 390.8 |
| 104 | 582.6 | 395.2 | 757 | 373.7 | 448.9 | 715.7 | 422.8 | 1046.9 |
| 105 | 605.4 | 439.1 | 711.2 | 498.5 | 563 | 713.5 | 478.7 | 844.5 |
| 106 | 573.3 | 421.3 | 707.9 | 394.3 | 496.4 | 727.1 | 411.8 | 1158.6 |
| 107 | 342.9 | 313.4 | 232.5 | 633.3 | 349.9 | 97.4 | 256.3 | 159.1 |
| 108 | 374.4 | 509.1 | 404.5 | 447.5 | 424 | 395.3 | 507 | 313.7 |
| 109 | 70.1 | 48.4 | 100.4 | 1071.3 | 216 | 52.3 | 605.8 | 112.2 |
| 110 | 1.3 | 1.1 | 56.4 | 2298.1 | 314.9 | 11.2 | 1496.5 | 175 |
| 111 | 618.4 | 1832.7 | 1179.5 | 1928 | 929.6 | 2094.9 | 301.9 | 2011.4 |
| 112 | 925.8 | 306.9 | 118.6 | 634.8 | 404.2 | 186.5 | 159.1 | 438 |
| 113 | 222.3 | 166.6 | 157.8 | 240.9 | 191.7 | 283.5 | 59.8 | 260.2 |
| 114 | 192.2 | 388.8 | 475 | 452.6 | 701.1 | 833.9 | 536.1 | 428.3 |
| 115 | 744.1 | 403.4 | 380.9 | 477.3 | 722 | 354.5 | 316 | 403.1 |
| 116 | 74 | 256.2 | 288.5 | 251.7 | 354.9 | 244.7 | 200.7 | 290.2 |
| 117 | 124.2 | 3117.3 | 3370.9 | 1392.1 | 2479.3 | 3139.4 | 3082.9 | 1506.6 |
| 118 | 45.9 | 127.9 | 220.2 | 543.8 | 654.1 | 106.6 | 76 | 302.5 |
| 119 | 73.6 | 109.6 | 79.5 | 187.2 | 253.7 | 129 | 89.7 | 180.1 |
| 120 | 63 | 151.9 | 118.9 | 83.3 | 329.6 | 139.5 | 96.5 | 196 |

(continued)

| SEQ ID NO: | 914TT | 915TT | 917TT | 918TT | 919TT | 920TT | 921TT | 922TT |
|---|---|---|---|---|---|---|---|---|
| 3h | | | | | | | | |
| 121 | 49.1 | 198.1 | 1209.5 | 1344.6 | 731.2 | 553.5 | 930.8 | 1026.3 |
| 122 | 325.5 | 74.7 | 168.8 | 279.7 | 220 | 128.3 | 73.4 | 226.7 |
| 123 | 191.5 | 158.6 | 161.6 | 149.7 | 200.5 | 180.6 | 123.1 | 147.8 |
| 124 | 208.1 | 325.2 | 147.4 | 136 | 144.3 | 215.6 | 157.8 | 152.9 |
| 125 | 1661.5 | 1623.3 | 1124.6 | 933.3 | 856.1 | 1323.7 | 974.7 | 1239.4 |
| 126 | 336.6 | 998.6 | 1518 | 1111.4 | 1195.1 | 749.3 | 1734.3 | 1307.5 |
| 127 | 114.1 | 160.9 | 127 | 108.1 | 99.9 | 108.8 | 94.3 | 121.3 |
| 128 | 377.5 | 502 | 304.4 | 273.4 | 240.5 | 360.7 | 256.7 | 248.3 |
| 129 | 152 | 156.1 | 228.4 | 614.3 | 248.8 | 187.1 | 182.1 | 174.4 |
| 130 | 201.8 | 125.2 | 123.1 | 91.1 | 86.5 | 146.4 | 126 | 102.5 |
| 131 | 171.6 | 96.4 | 92.7 | 100.4 | 74.1 | 81.2 | 64.7 | 62 |
| 132 | 194.7 | 290.5 | 224 | 204.2 | 202.2 | 246.9 | 198.3 | 213.9 |
| 133 | 185.3 | 28.5 | 90.5 | 95.6 | 99.8 | 53.6 | 115.1 | 67.8 |
| 134 | 108.7 | 105.8 | 146.2 | 129.8 | 136.1 | 152.6 | 101.4 | 104.9 |
| 135 | 329.3 | 300.4 | 422.9 | 350.2 | 459.4 | 315.2 | 405.3 | 583.7 |
| 138 | 141.3 | 138.1 | 148 | 138 | 261.5 | 129.3 | 112.3 | 140.1 |
| 139 | 64.7 | 72.7 | 91.7 | 113 | 89.4 | 72.3 | 78.5 | 70.3 |
| 140 | 12.8 | 3594.2 | 546.1 | 1731 | 1815.6 | 2735.6 | 7476.3 | 1203.1 |
| 141 | 765 | 2165 | 2264.2 | 1694.9 | 2113.3 | 2212.9 | 1964.9 | 2552.8 |
| 144 | 557.3 | 658.9 | 531.9 | 445.9 | 506.1 | 576 | 448.1 | 498.5 |
| 145 | 293.6 | 414.8 | 253.6 | 213.1 | 181.7 | 238.7 | 218 | 282.1 |
| 146 | 346.7 | 292.7 | 269.6 | 217.1 | 213.1 | 208.1 | 211.4 | 258.4 |
| 147 | 62.7 | 79.5 | 46.1 | 53.8 | 81.9 | 58.5 | 49.8 | 63.7 |
| 148 | 269.9 | 289.4 | 334.4 | 865.9 | 508.3 | 260.4 | 407.4 | 281.3 |
| 149 | 97.4 | 236.4 | 437.2 | 418.5 | 394.6 | 307.1 | 973.7 | 534.2 |
| 150 | 137.6 | 243.5 | 188.6 | 496 | 186.8 | 175.5 | 160 | 154.5 |
| 151 | 127.6 | 135.7 | 166.9 | 264 | 342.2 | 154.3 | 155.1 | 238.1 |
| 153 | 123.6 | 100.3 | 115.7 | 116.3 | 102.4 | 89.5 | 160.7 | 101.3 |
| 154 | 85 | 197.4 | 1113.2 | 453.2 | 438.4 | 272.8 | 68.6 | 476.9 |
| 155 | 278.9 | 193.9 | 179.6 | 197.2 | 142.9 | 121 | 155.5 | 125.7 |
| 156 | 223.2 | 67.3 | 125 | 73.3 | 134.8 | 87.9 | 62.7 | 94.5 |
| 157 | 191.4 | 141 | 80.7 | 108 | 126.5 | 132.4 | 139.9 | 153.4 |
| 158 | 120.4 | 27.9 | 18.7 | 22.2 | 13.5 | 8.3 | 48.3 | 1.1 |
| 159 | 2173.3 | 3351 | 4458.5 | 2838.9 | 3155.5 | 4574.9 | 3436 | 5276.1 |
| 161 | 321.7 | 4.9 | 48.5 | 38.3 | 51.1 | 73.2 | 32.3 | 115.5 |
| 162 | 57.7 | 109.5 | 90.4 | 127 | 451.2 | 53.5 | 97.4 | 109.9 |
| 164 | 18.3 | 107.3 | 133.5 | 945.5 | 294.7 | 100.8 | 471.3 | 145 |

(continued)

| 3h | | | | | | | | |
|---|---|---|---|---|---|---|---|---|
| SEQ ID NO: | 914TT | 915TT | 917TT | 918TT | 919TT | 920TT | 921TT | 922TT |
| 166 | 1789.6 | 8438 | 8113.2 | 9464 | 4481.5 | 10514.2 | 6218.7 | 8586.9 |
| 167 | 1221.4 | 1687.4 | 2694.8 | 870.2 | 2235.2 | 3402.4 | 2967 | 3999.6 |
| 168 | 1134.1 | 1571.9 | 233.8 | 389.6 | 470.2 | 1162.3 | 676.1 | 517.9 |
| 169 | 1299.1 | 3874.8 | 2937.3 | 20149.3 | 4579.7 | 3975.5 | 5507.1 | 3449.1 |
| 170 | 496.6 | 1040.8 | 993.1 | 1421.7 | 1194.6 | 1747 | 1725.6 | 975.7 |
| 171 | 1.7 | 726.5 | 381.8 | 1172.2 | 3538.7 | 1.7 | 311.2 | 923.1 |
| 172 | 787.9 | 831.5 | 671.7 | 556.8 | 576.2 | 725.4 | 702.2 | 758.7 |
| 173 | 325.2 | 174.5 | 231.2 | 453.8 | 404 | 104.2 | 388.1 | 164.7 |
| 176 | 1.1 | 61.7 | 1.1 | 901.4 | 45.4 | 116.1 | 25.7 | 13.1 |
| 177 | 1168.1 | 892.5 | 744.8 | 380 | 424.7 | 794 | 561 | 663.6 |
| 178 | 1040.7 | 147.4 | 125.3 | 225.6 | 118 | 65.1 | 137.1 | 105.3 |
| 179 | 175.2 | 142.5 | 80.7 | 229.8 | 86.5 | 162.9 | 95.4 | 82.1 |
| 180 | 219.9 | 159.1 | 69.7 | 168.4 | 130.9 | 108.4 | 102 | 102.9 |
| 181 | 7935.3 | 13498.8 | 13730.6 | 11985.5 | 10419.5 | 12804.4 | 11323.8 | 12979.3 |
| 182 | 150.3 | 447.5 | 499.1 | 465.6 | 614.8 | 394.1 | 697.3 | 535.6 |
| 183 | 1.2 | 302 | 315.6 | 55.7 | 3038.8 | 1.1 | 111.6 | 870.4 |
| 184 | 164.6 | 304.5 | 220.8 | 214.1 | 342.7 | 208.6 | 235.9 | 282.7 |
| 185 | 339.5 | 1227.3 | 1316 | 2812.4 | 2324.1 | 1291.1 | 801.3 | 951.8 |
| 186 | 1007.5 | 586.1 | 520.9 | 357.3 | 592.2 | 596 | 427.6 | 531 |
| 187 | 105.8 | 150.7 | 121.4 | 138.6 | 346.7 | 114.5 | 71.7 | 179.7 |
| 188 | 90.9 | 117.6 | 131.9 | 111.7 | 401.8 | 185.4 | 95.3 | 186.5 |
| 189 | 44.7 | 89.1 | 89.1 | 75.5 | 222.5 | 76.8 | 62.8 | 140 |
| 190 | 387.4 | 103.3 | 160.9 | 193.8 | 110.4 | 132.3 | 17.1 | 290.6 |
| 191 | 1052.5 | 301.2 | 1095.5 | 2271.6 | 1145.2 | 677.5 | 637 | 486.6 |
| 192 | 630.6 | 99.9 | 111.6 | 214 | 209.1 | 70.9 | 85.5 | 102.3 |
| 193 | 74.3 | 2980.5 | 1451.8 | 1464.1 | 967.9 | 3258.6 | 1246.7 | 1115.3 |
| 194 | 103.9 | 95.6 | 148.6 | 197.7 | 173.4 | 182.1 | 182.9 | 151.6 |
| 195 | 232 | 517.9 | 233.4 | 227.6 | 231.8 | 371.1 | 311.1 | 252.3 |
| 196 | 14368.7 | 9559.4 | 6515.4 | 4725.2 | 6534.5 | 6168.5 | 3594.5 | 10426.2 |
| 197 | 174.7 | 101.2 | 74.1 | 54.7 | 170.5 | 108.4 | 82.8 | 122.4 |
| 198 | 91 | 135.6 | 56.6 | 60.3 | 89.4 | 59.6 | 45.6 | 59.4 |
| 199 | 61 | 139.7 | 76.3 | 1103.8 | 315.1 | 93.4 | 1137.5 | 145 |
| 200 | 174.8 | 227.8 | 166 | 143.6 | 811.9 | 124 | 150.7 | 494.8 |
| 201 | 97.8 | 40.6 | 125.1 | 6018.8 | 363.4 | 16.6 | 745.3 | 54 |
| 203 | 248.8 | 51.5 | 202.6 | 274 | 233 | 114.9 | 678.6 | 137.4 |
| 204 | 355.2 | 445.7 | 563 | 594.4 | 551.8 | 780.5 | 259.1 | 741.3 |
| 205 | 3081.7 | 3669.5 | 4544.7 | 4530.2 | 5821 | 4180.6 | 2864.1 | 6443.9 |

(continued)

| 3h | | | | | | | | |
|---|---|---|---|---|---|---|---|---|
| SEQ ID NO: | 914TT | 915TT | 917TT | 918TT | 919TT | 920TT | 921TT | 922TT |
| 206 | 635.1 | 335.2 | 212.1 | 203.2 | 2002.9 | 327.3 | 212.8 | 296.9 |
| 207 | 108.8 | 465.2 | 1154 | 1017.8 | 856.5 | 253.4 | 2406.9 | 982.7 |
| 208 | 376.1 | 87.8 | 163.3 | 235.1 | 433.7 | 162 | 209.8 | 341.4 |
| 209 | 4098 | 6563.6 | 5001.8 | 2151.6 | 3441.1 | 5849.7 | 5350.8 | 5424.3 |
| 211 | 13293.2 | 56352.5 | 27543 | 16362.2 | 12402.5 | 30128.4 | 25478.3 | 26582.6 |
| 212 | 3366.9 | 504.5 | 937.3 | 451.1 | 493.9 | 512.1 | 182 | 356.5 |
| 213 | 557 | 72.1 | 102.2 | 72.7 | 87.2 | 125.6 | 68.6 | 84.7 |
| 215 | 69.6 | 611.1 | 1358.5 | 1255.1 | 1026.1 | 288 | 5253.8 | 1589.5 |
| 216 | 307.4 | 418 | 1035.4 | 885.1 | 913.2 | 784.3 | 771.5 | 663.5 |
| 217 | 122.9 | 221.4 | 276.8 | 282.9 | 382.1 | 276 | 250.6 | 310.7 |
| 218 | 405.2 | 348.1 | 325 | 389.5 | 308.3 | 374.2 | 366.4 | 279.9 |
| 220 | 133.1 | 120.1 | 104.4 | 90.8 | 74 | 87.2 | 60.6 | 104.9 |
| 221 | 202.1 | 462 | 707.8 | 738.8 | 744.5 | 462.6 | 507.6 | 536.4 |
| 223 | 401.3 | 290.1 | 265 | 373.6 | 219.2 | 205.5 | 250.1 | 279.2 |
| 227 | 96.8 | 1403.6 | 588.9 | 742.9 | 1379 | 767.3 | 484.7 | 435.2 |
| 228 | 744.1 | 1944.7 | 680.2 | 365.7 | 418 | 1097.5 | 375 | 675.9 |
| 229 | 192.8 | 268 | 148.6 | 179.1 | 300.2 | 245.5 | 147 | 234.8 |
| 230 | 587.1 | 143.8 | 108 | 519.8 | 327.3 | 137.2 | 90.6 | 168.9 |
| 231 | 159.4 | 112.3 | 101.9 | 123.3 | 147.3 | 95.5 | 310.1 | 105.2 |
| 232 | 86 | 1502.8 | 1501.4 | 235.4 | 15668.2 | 38.8 | 605.2 | 4886.3 |
| 233 | 813.8 | 1781.7 | 6349.7 | 5834.7 | 8518.5 | 4215.2 | 3924.8 | 4770.7 |
| 235 | 19.1 | 393.8 | 248.5 | 975.6 | 1843.2 | 72.6 | 302.8 | 584.4 |
| 236 | 135.6 | 172.6 | 172 | 178.6 | 303.1 | 218.4 | 107.7 | 240.4 |
| 237 | 1106.1 | 1789 | 2038.4 | 1890.3 | 2100.8 | 1798.9 | 1370.4 | 1717.2 |
| 238 | 366.2 | 375.6 | 1127.2 | 1261.7 | 1343.5 | 899.5 | 859 | 1414.4 |
| 239 | 61.2 | 182.2 | 230.2 | 474.9 | 731.7 | 98.3 | 176.9 | 251.2 |
| 240 | 91.7 | 193.4 | 362.9 | 596.3 | 923.8 | 202.3 | 144.4 | 289.6 |
| 241 | 26.6 | 324.2 | 559.9 | 1218.5 | 1761.7 | 170.8 | 321.8 | 584.8 |
| 242 | 4172.9 | 1891.1 | 1654.2 | 11791 | 2788.1 | 2452.4 | 1809.2 | 1758.5 |
| 243 | 248.1 | 152.6 | 323.5 | 711.2 | 510.7 | 217.7 | 135.2 | 244.7 |
| 244 | 68.8 | 98.9 | 183.4 | 204.9 | 179.5 | 139.4 | 209.7 | 132.7 |
| 246 | 385.3 | 80.7 | 187.9 | 227.3 | 218.7 | 122.1 | 230.3 | 250.4 |
| 247 | 115.2 | 187.8 | 109.8 | 134.8 | 162.4 | 139.5 | 154.3 | 176.4 |
| 248 | 175.8 | 124.7 | 211.6 | 157.6 | 122.1 | 213.7 | 119.3 | 151 |
| 249 | 78.8 | 1316.1 | 1303.2 | 1406.5 | 646.5 | 1094 | 89.7 | 316.7 |
| 251 | 41.9 | 62.5 | 102.3 | 171.4 | 129 | 87.1 | 73 | 44.9 |
| 253 | 297.4 | 222.6 | 147.6 | 339.3 | 377.1 | 276.6 | 218.4 | 296.8 |

(continued)

| 3h | | | | | | | | |
|---|---|---|---|---|---|---|---|---|
| SEQ ID NO: | 914TT | 915TT | 917TT | 918TT | 919TT | 920TT | 921TT | 922TT |
| 254 | 170 | 132 | 351.7 | 258.6 | 501.3 | 45.2 | 529.9 | 460.2 |
| 255 | 1140 | 47 | 85.6 | 207.2 | 108 | 89.8 | 121.1 | 51.6 |
| 256 | 509.5 | 559.2 | 396.4 | 6461.4 | 596.9 | 143.8 | 1128.1 | 72.8 |
| 257 | 111.3 | 113 | 208.1 | 145.8 | 103.3 | 155.1 | 204.9 | 122 |
| 258 | 373.9 | 20.9 | 71.2 | 831.4 | 206.8 | 7.3 | 19.2 | 134.7 |
| 259 | 1.2 | 346.7 | 153.9 | 329.1 | 1431.2 | 189.1 | 51.2 | 376.3 |
| 260 | 269.3 | 188.5 | 157.8 | 133.8 | 179.4 | 168.2 | 152.1 | 211.1 |
| 261 | 363.1 | 886.3 | 408.2 | 266.3 | 337.5 | 580.5 | 238.9 | 222.6 |
| 263 | 350.7 | 311.2 | 391.8 | 381.3 | 340.2 | 408 | 301.8 | 534.9 |
| 263 | 560.3 | 220 | 122.6 | 491.3 | 334.4 | 187.2 | 129.9 | 227 |
| 265 | 305.3 | 771.9 | 487.2 | 449.1 | 359.5 | 453.8 | 572.3 | 448.6 |
| 266 | 587.1 | 510.1 | 304.8 | 271.5 | 354.2 | 346.8 | 203.3 | 606.6 |
| 256 | 110.1 | 118.2 | 141.4 | 183.8 | 193.4 | 156.1 | 104.6 | 141.6 |
| 268 | 829.5 | 440.5 | 993.8 | 821.4 | 1312.7 | 938.4 | 407.2 | 1476.7 |
| 269 | 58.4 | 43.6 | 32.7 | 52.3 | 48.9 | 47.7 | 35.4 | 31.4 |
| 270 | 414.3 | 315 | 208 | 162.5 | 188.1 | 275.9 | 132.5 | 378.4 |
| 271 | 184.7 | 216.5 | 242.4 | 350 | 268.7 | 323.9 | 246.1 | 211.3 |
| 272 | 673.6 | 752.9 | 549.8 | 450 | 534.1 | 526.4 | 507.2 | 463.9 |
| 273 | 667.9 | 4430.8 | 2398.6 | 2278.9 | 2835.2 | 5763.4 | 3170.9 | 10524 |
| 274 | 309.4 | 563.1 | 382 | 416.7 | 1158.9 | 634.4 | 198.9 | 501.8 |
| 275 | 150.9 | 343.1 | 349.7 | 862.7 | 617.9 | 370.3 | 217.2 | 282.6 |
| 276 | 144.2 | 2577.7 | 914.9 | 1262.7 | 2706.1 | 873.2 | 822.4 | 731.1 |
| 277 | 221 | 264.6 | 204.6 | 186.4 | 182 | 199.3 | 212.4 | 205.9 |
| 278 | 5.1 | 445.5 | 286.4 | 951.3 | 2040.1 | 322.7 | 97.9 | 556.9 |
| 279 | 1964.2 | 1929.5 | 1874.1 | 1425.7 | 2873.1 | 2411.1 | 2753 | 4215.2 |
| 280 | 1725.8 | 1807.2 | 1790 | 2026.6 | 3105.8 | 3426.9 | 3401.6 | 4521.1 |
| 281 | 299.6 | 10.6 | 61.7 | 159.3 | 165.8 | 22.7 | 10.9 | 56.8 |
| 282 | 253.1 | 287.7 | 240.7 | 553.9 | 252 | 245.3 | 295.4 | 310.6 |
| 283 | 451.8 | 310.2 | 223.6 | 224.6 | 261.3 | 262.4 | 300.3 | 265.8 |
| 284 | 107.2 | 65 | 79.3 | 94.4 | 77.1 | 95.2 | 88.3 | 68.2 |
| 285 | 126.9 | 108.2 | 89.2 | 90.8 | 95.7 | 83.3 | 101.1 | 56.8 |
| 286 | 207.5 | 742.2 | 937.4 | 1260.1 | 840.3 | 441.7 | 1221.8 | 518 |
| 287 | 147.3 | 167.1 | 130.2 | 109.1 | 90 | 112.1 | 102.3 | 113 |
| 288 | 171.6 | 203.3 | 136.7 | 135.2 | 166.9 | 154.6 | 140.7 | 158 |
| 289 | 371.3 | 670.5 | 83.5 | 1902.3 | 2036.5 | 48 | 228 | 264.6 |
| 291 | 80.2 | 126.4 | 72.5 | 78.8 | 132.2 | 210.1 | 63.6 | 112.7 |
| 292 | 38.1 | 110.7 | 46.9 | 472.6 | 1250.2 | 27.4 | 126.2 | 66.3 |

(continued)

| 3h | | | | | | | | |
|---|---|---|---|---|---|---|---|---|
| SEQ ID NO: | 914TT | 915TT | 917TT | 918TT | 919TT | 920TT | 921TT | 922TT |
| 293 | 222.5 | 84 | 110.2 | 187.1 | 124 | 59.9 | 126.5 | 88.8 |
| 295 | 1213.6 | 281.8 | 785.8 | 379.3 | 174.4 | 535.7 | 147 | 292.6 |
| 296 | 898.3 | 582 | 827.5 | 972.1 | 1280.1 | 738.6 | 1194.8 | 908.9 |
| 297 | 58.4 | 68.5 | 79.3 | 128 | 150.2 | 55.4 | 43.3 | 58.5 |
| 298 | 389.2 | 195.7 | 352.2 | 318 | 292.9 | 275.4 | 295.4 | 345.4 |
| 299 | 1008.9 | 952.4 | 435.5 | 935.9 | 1207.2 | 1276.8 | 1004.8 | 874.4 |
| 300 | 616.3 | 416.1 | 93.2 | 235.8 | 253 | 285.2 | 321.8 | 218.3 |
| 301 | 787.4 | 7692.9 | 760.9 | 597.6 | 582 | 4161.5 | 866.6 | 1551.4 |
| 302 | 8.2 | 20.2 | 20.5 | 26.4 | 38.3 | 11.5 | 48.2 | 19.3 |
| 303 | 667.1 | 584.1 | 822.2 | 624 | 674.3 | 794.9 | 891.8 | 698 |
| 304 | 184.1 | 237.5 | 283.8 | 497 | 280.3 | 200.5 | 163.9 | 170.7 |
| 305 | 158.6 | 187.4 | 179.4 | 240.3 | 128.9 | 137.5 | 171.4 | 144 |
| 306 | 177.6 | 272.7 | 152.9 | 164.2 | 148.6 | 151.8 | 118.4 | 196.2 |
| 307 | 232.6 | 294 | 179.7 | 177.7 | 166.7 | 143.1 | 269.6 | 156.1 |
| 308 | 3916.9 | 1024.8 | 1901.6 | 978.1 | 2018.2 | 1367 | 1084.7 | 2030.4 |
| 310 | 840.8 | 1027 | 434.4 | 345.5 | 351.7 | 775.7 | 403.9 | 604 |
| 311 | 1.4 | 100.4 | 111.4 | 207.4 | 110.6 | 112.7 | 85.5 | 48.2 |
| 312 | 3441.6 | 2815.6 | 597.5 | 664 | 496.5 | 1479.8 | 176.1 | 1442.5 |
| 313 | 100.5 | 123.2 | 112.4 | 128.1 | 112.7 | 146.2 | 83.4 | 132.5 |
| 314 | 271.3 | 291.9 | 302.3 | 543.9 | 514 | 181.8 | 157.4 | 408.4 |
| 315 | 194.3 | 265 | 553.7 | 442.1 | 676.9 | 352.4 | 338.1 | 580.4 |
| 316 | 190.3 | 130.5 | 393.5 | 448.9 | 461.5 | 184.7 | 184 | 326.6 |
| 317 | 187.8 | 207.7 | 143.5 | 1020.4 | 167.2 | 168.1 | 180.4 | 189.7 |
| 318 | 41.3 | 18.4 | 63.6 | 115.2 | 161.2 | 34.4 | 15 | 117.9 |
| 319 | 860 | 151.7 | 173.4 | 206.1 | 185.7 | 184.2 | 193 | 172.4 |
| 320 | 509.6 | 854.4 | 499.3 | 516.6 | 643.1 | 697.6 | 512 | 842.6 |
| 321 | 856.4 | 633.2 | 596.3 | 403.9 | 477.8 | 545.6 | 282.5 | 377.9 |
| 322 | 433.2 | 202.4 | 278.6 | 249.3 | 234 | 227.3 | 234.3 | 199.3 |
| 323 | 841.5 | 2787.9 | 2937.2 | 1793.4 | 1324.1 | 4240.9 | 2486 | 2525.8 |
| 324 | 34.6 | 20.8 | 38.8 | 166.5 | 195.2 | 110.1 | 85.5 | 75.9 |
| 325 | 493.2 | 226.8 | 266.8 | 301.9 | 256.4 | 292.2 | 312.9 | 251.6 |
| 326 | 107.7 | 1500 | 4118.2 | 5094.1 | 4992.3 | 1863.8 | 3521.7 | 2850 |
| 327 | 8.4 | 1.2 | 6.1 | 58.1 | 53.1 | 10.1 | 1.1 | 19.4 |
| 328 | 448.4 | 1313.7 | 690.6 | 383.9 | 558.3 | 678.1 | 451.7 | 643.7 |
| 329 | 242.5 | 67.9 | 67.9 | 52 | 61.7 | 53.9 | 71.2 | 50.2 |
| 330 | 17.8 | 47.2 | 39.7 | 282.1 | 70.4 | 42.8 | 119.6 | 47.7 |
| 331 | 733.3 | 495.8 | 278.9 | 232 | 273.6 | 280.6 | 257 | 242.5 |

(continued)

| 3h | | | | | | | | |
|---|---|---|---|---|---|---|---|---|
| SEQ ID NO: | 914TT | 915TT | 917TT | 918TT | 919TT | 920TT | 921TT | 922TT |
| 333 | 302.3 | 1043.4 | 1067 | 536.5 | 739.2 | 789 | 589.6 | 857.9 |
| 334 | 135 | 171 | 185.4 | 142.8 | 175.4 | 125.4 | 150.7 | 191.2 |
| 335 | 114.3 | 112 | 135.6 | 198.2 | 140.2 | 65.3 | 82.4 | 146.1 |
| 336 | 273.5 | 427.6 | 549 | 929.9 | 527.7 | 486.6 | 685.7 | 484.4 |
| 337 | 127.6 | 450.5 | 304.7 | 308.8 | 228.9 | 259.2 | 192 | 337.1 |
| 338 | 122 | 131.1 | 153.1 | 314.9 | 206.1 | 109.7 | 203.8 | 162.9 |
| 339 | 589.7 | 576.7 | 629.8 | 367.4 | 474.8 | 496.3 | 533.8 | 528.2 |
| 340 | 345.7 | 362 | 289.7 | 277.4 | 243.4 | 291.8 | 246.1 | 272.2 |
| 341 | 601 | 1030.1 | 896.3 | 495.4 | 536.5 | 1091.4 | 1222.4 | 1064.8 |
| 343 | 4100.7 | 199.4 | 431 | 1240.1 | 83 | 30.4 | 75.2 | 123.1 |
| 344 | 1624.5 | 2249.2 | 1044.5 | 1143.8 | 1222.6 | 1571.5 | 1112.9 | 1923.9 |
| 345 | 238.1 | 412.6 | 253.9 | 217.5 | 231.2 | 189.6 | 247.2 | 211.3 |
| 347 | 2041.8 | 1350.1 | 3062.9 | 2896.2 | 3974.1 | 2601.7 | 1109.2 | 3899.9 |
| 348 | 2985.8 | 3296.8 | 7137.1 | 7441.2 | 9015.9 | 4507.6 | 2225.5 | 6141.5 |
| 349 | 242.2 | 171.3 | 270.3 | 274.6 | 214.3 | 206.7 | 232 | 284.3 |
| 350 | 145.3 | 165.3 | 253.4 | 465.2 | 473.9 | 247 | 260.1 | 349.3 |
| 351 | 221.5 | 71.2 | 134 | 484.9 | 440.9 | 92.2 | 237.3 | 182.5 |
| 352 | 302.8 | 377.2 | 355.1 | 306.4 | 343.9 | 332.8 | 471.2 | 707.4 |
| 353 | 485.1 | 155.6 | 246.1 | 298.4 | 282.3 | 160.5 | 283 | 245.4 |

| 3i | | | | | | | | |
|---|---|---|---|---|---|---|---|---|
| SEQ ID NO: | 923TT | 924TT | 925TT | 926TT | 927TT | 928TT | 932TT | 933TT |
| 354 | 4999.2 | 948.9 | 8.5 | 8107.3 | 357.6 | 741.1 | 2903.8 | 582.1 |
| 355 | 1542.9 | 255.1 | 105.5 | 4694.4 | 98.3 | 91.6 | 1287.2 | 466 |
| 356 | 955.2 | 717.8 | 1055 | 1279.5 | 479.1 | 524.8 | 624.3 | 654.8 |
| 357 | 742.1 | 866.6 | 793.6 | 981.1 | 533.1 | 757.7 | 765.3 | 734.4 |
| 358 | 619.2 | 1731.9 | 778.3 | 770.2 | 675 | 559.4 | 2309.3 | 1072.7 |
| 359 | 393.4 | 300.3 | 283.4 | 311.1 | 437.6 | 326 | 298.3 | 314.7 |
| 362 | 382.2 | 940.4 | 368.8 | 968.1 | 747.1 | 473.3 | 3226.3 | 666.7 |
| 360 | 305.2 | 656.7 | 364.5 | 362.7 | 233.2 | 217.2 | 638.3 | 448 |
| 361 | 455.4 | 543 | 660.9 | 437.4 | 376.4 | 1699.9 | 116.7 | 462.7 |
| 363 | 425.6 | 414.8 | 478.3 | 461 | 574.2 | 432.5 | 334.7 | 437.1 |
| 1 | 730.2 | 1032.9 | 682 | 844.1 | 393.9 | 674.5 | 1344.9 | 1001.2 |
| 2 | 3449 | 1961.4 | 3686.9 | 2642.7 | 1807.9 | 3449 | 6464 | 2719.4 |
| 7 | 354 | 463.2 | 545 | 364.5 | 928.7 | 902.8 | 291.2 | 1104.3 |
| 8 | 2391.2 | 881.8 | 994.1 | 2512.8 | 1239.1 | 1078.9 | 1105.4 | 1030.3 |
| 9 | 7066.7 | 3621.1 | 8955.9 | 10216 | 6146 | 1554.6 | 3073.1 | 2224.4 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 10 | 76.1 | 133.7 | 48.5 | 112.3 | 70.9 | 40.5 | 125.8 | 84.2 |
| 11 | 423.1 | 502.3 | 256.7 | 620.1 | 215.5 | 224.5 | 1442.7 | 450.7 |
| 13 | 208.3 | 631.4 | 132.2 | 270.3 | 127.5 | 202.1 | 244.8 | 524.1 |
| 14 | 1772 | 3284.9 | 595.1 | 1382.8 | 378.2 | 703.9 | 1281.9 | 1396.6 |
| 15 | 273.3 | 253.1 | 691.6 | 629.7 | 624.5 | 137.9 | 1375.3 | 988.3 |
| 16 | 1155.7 | 1826.6 | 1298.2 | 1503.2 | 766.7 | 976.8 | 2803.3 | 1587.5 |
| 17 | 981 | 1525 | 1084 | 1110.8 | 713.8 | 1879.1 | 2006.1 | 958.6 |
| 19 | 295.3 | 325.4 | 2332.9 | 154.5 | 766.2 | 143 | 5450.6 | 1516 |
| 20 | 5.6 | 231.8 | 1.1 | 406.2 | 93 | 7.7 | 717.2 | 285 |
| 21 | 216.9 | 1426.9 | 466.2 | 1058.3 | 233.1 | 89.3 | 2805.3 | 820.1 |
| 22 | 5541.1 | 5019.9 | 7633.3 | 7736.7 | 4422.4 | 4997.7 | 6683.9 | 5281.2 |
| 23 | 418.4 | 128.7 | 924.7 | 522.4 | 263.1 | 95.8 | 1540.6 | 1482.2 |
| 24 | 124.7 | 262.2 | 466.7 | 332.9 | 277.9 | 112.7 | 502.5 | 370 |
| 26 | 2003.6 | 326.4 | 9052 | 447.2 | 1915.3 | 199.6 | 1793.9 | 5637.4 |
| 27 | 903.7 | 1325.6 | 731.2 | 620.3 | 523.4 | 499.5 | 1436.8 | 1142.6 |
| 29 | 77.9 | 153.4 | 131.3 | 265.8 | 339.2 | 91 | 278.2 | 113.3 |
| 30 | 244.5 | 111.3 | 342.6 | 1082.1 | 219.4 | 136 | 880.6 | 493.2 |
| 31 | 717.2 | 5693.4 | 185.8 | 2809.1 | 627.1 | 214.1 | 13720.9 | 4115 |
| 33 | 942.9 | 1061.8 | 704.6 | 3949.8 | 244.3 | 466.4 | 5262.7 | 2453.2 |
| 34 | 3136.1 | 702.3 | 4502.9 | 3384.3 | 2498.9 | 1817.4 | 204.4 | 1637.3 |
| 35 | 823.2 | 709.6 | 179 | 1649.8 | 528.8 | 173.2 | 2586.2 | 722.3 |
| 36 | 196 | 1335.4 | 173.1 | 183.3 | 431.8 | 189.1 | 339.1 | 986.2 |
| 37 | 190.3 | 178.3 | 281.4 | 264.8 | 256.3 | 166 | 142.8 | 210.3 |
| 38 | 764.6 | 1248.7 | 543.5 | 2191.9 | 232.8 | 763.6 | 2898.9 | 1375.1 |
| 40 | 454.8 | 644.3 | 481.5 | 529.5 | 897.3 | 467 | 541 | 618.6 |
| 41 | 324.4 | 539.9 | 467.9 | 199.6 | 143.9 | 2803.9 | 331.2 | 668.8 |
| 42 | 245.1 | 482.5 | 178 | 170.4 | 246.6 | 572.5 | 528.4 | 345.2 |
| 43 | 312.4 | 237 | 363 | 359 | 522.3 | 337.5 | 229 | 305.9 |
| 44 | 485.5 | 1587.5 | 666.9 | 778.7 | 788.5 | 427.7 | 1308.4 | 1173.2 |
| 45 | 523.4 | 1739.2 | 564.2 | 954.7 | 723.9 | 426.5 | 1423.9 | 1045.9 |
| 46 | 219.6 | 289.5 | 291.3 | 270.7 | 363.6 | 297.2 | 236.2 | 308.8 |
| 47 | 358.3 | 1203.3 | 261.6 | 420.8 | 219.8 | 231.9 | 811 | 565.7 |
| 48 | 132.2 | 32.9 | 529.4 | 17.2 | 481.9 | 15 | 768.8 | 1447.3 |
| 49 | 502.3 | 3238.8 | 380.6 | 5448 | 761.8 | 211.7 | 13066 | 3743 |
| 50 | 203.5 | 179.7 | 458.7 | 481.9 | 247 | 130.4 | 1359.3 | 491.3 |
| 51 | 36.1 | 495.5 | 36.6 | 477.9 | 354.1 | 190.1 | 860.5 | 367.9 |
| 52 | 217.6 | 305.8 | 203.1 | 178.1 | 145.5 | 199.2 | 266.4 | 254.3 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 53 | 135.2 | 1943.2 | 130.6 | 584.4 | 98 | 144.2 | 2628.1 | 714.3 |
| 54 | 980.6 | 1795 | 433.4 | 795.1 | 591.6 | 1643.8 | 1486.9 | 1394.9 |
| 55 | 106.3 | 85.4 | 98.8 | 76.5 | 97.2 | 90.8 | 86.4 | 81.5 |
| 56 | 72.3 | 168.2 | 54.5 | 77.8 | 70.1 | 175.9 | 120.4 | 338.8 |
| 57 | 4530.2 | 5218.3 | 281.2 | 4508.1 | 1636.4 | 1547.5 | 4191.4 | 4170.2 |
| 58 | 8556.8 | 6205.1 | 264.9 | 5634.2 | 1765.7 | 2033.7 | 8482.4 | 4433.9 |
| 59 | 1700.3 | 4628.3 | 2056.3 | 1693.4 | 800.8 | 1336.9 | 5450.1 | 2540.6 |
| 60 | 185.2 | 235.6 | 229.2 | 272.6 | 145.4 | 296.3 | 224.9 | 319 |
| 61 | 115.2 | 98.8 | 149 | 130.4 | 193.6 | 134.3 | 90.8 | 139.6 |
| 62 | 3145.7 | 4454.6 | 2756.3 | 2855 | 5821 | 142.2 | 5129.2 | 2442.2 |
| 63 | 379.2 | 744.4 | 325.8 | 938.2 | 356.1 | 502 | 1395.4 | 539.9 |
| 64 | 238.6 | 3544.9 | 113.1 | 512.4 | 242.4 | 124.5 | 661.9 | 573.5 |
| 65 | 113.4 | 390.1 | 172.8 | 433.7 | 95.5 | 201.5 | 506.6 | 282.3 |
| 66 | 712.9 | 1199.2 | 529.5 | 816.9 | 392.7 | 1636.5 | 1229.2 | 1158.8 |
| 67 | 29201.1 | 24240.4 | 35951.3 | 30300.5 | 39570.8 | 34590.3 | 12924.3 | 33523.7 |
| 68 | 369.9 | 258.4 | 3692.3 | 955.1 | 3718.5 | 2125.3 | 13.8 | 2527.3 |
| 69 | 1786.8 | 1413.7 | 5838.9 | 2685.9 | 9047.2 | 4524.2 | 193.4 | 5503.7 |
| 70 | 1299.1 | 4941.4 | 1670.8 | 1399.3 | 4204.2 | 2761.2 | 1891.3 | 3217.7 |
| 71 | 319.8 | 3969.9 | 1361.7 | 304 | 214.9 | 196.2 | 7882.8 | 2439.7 |
| 72 | 858.1 | 2076.7 | 424 | 1838.1 | 291.2 | 256.6 | 4680.6 | 1518.6 |
| 73 | 330.7 | 633.1 | 342.2 | 295.9 | 422.6 | 202.3 | 561.1 | 512.8 |
| 75 | 160.9 | 1925.8 | 332.6 | 236.1 | 224.7 | 510.6 | 2680.7 | 1504.4 |
| 76 | 186.2 | 576.1 | 105.9 | 163.7 | 609.8 | 358.6 | 809.9 | 826.1 |
| 77 | 3237.7 | 1390.9 | 5841.2 | 3756.2 | 8578.9 | 3419.8 | 278.9 | 3072.7 |
| 78 | 390.6 | 630.4 | 1085.3 | 608.2 | 792.8 | 957.2 | 640.8 | 631.7 |
| 79 | 149 | 230.2 | 167.8 | 234.3 | 145.3 | 153.8 | 436.8 | 205.9 |
| 80 | 335 | 1124.8 | 139.3 | 306 | 338 | 751.9 | 1832.2 | 582.1 |
| 81 | 756.3 | 1099.9 | 314 | 2079.9 | 1185.6 | 169.3 | 5642.3 | 1783.2 |
| 82 | 204.4 | 219.8 | 346.6 | 218.1 | 417.4 | 382.9 | 97.4 | 242.2 |
| 84 | 161.4 | 305.1 | 329.2 | 121.2 | 177.5 | 155.6 | 567.2 | 276 |
| 85 | 268.5 | 148.9 | 131.5 | 9338.9 | 157.7 | 130.4 | 841 | 209.5 |
| 86 | 63.7 | 60.1 | 90.7 | 94.7 | 77.3 | 63.7 | 128.2 | 210.3 |
| 87 | 336.8 | 563.4 | 358.3 | 784 | 227.4 | 345.8 | 6260.3 | 351.1 |
| 88 | 2065.9 | 1415.1 | 5272.5 | 2763 | 4806.2 | 6097.4 | 444.1 | 3528.8 |
| 89 | 170 | 217.9 | 167.4 | 265.8 | 193.2 | 219.7 | 218.7 | 301.1 |
| 90 | 125.6 | 103.3 | 107.3 | 195.6 | 166.8 | 106.5 | 188.5 | 236.2 |
| 91 | 14152.6 | 4927.9 | 66.9 | 6763.6 | 1057.9 | 1430.5 | 7192.3 | 2761 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 92 | 4155.7 | 9975.4 | 3017.4 | 2430.3 | 1461.8 | 5499.5 | 10750.2 | 6764.7 |
| 93 | 253.7 | 200.3 | 376.1 | 458.2 | 173.2 | 390.5 | 123.5 | 213.3 |
| 94 | 2143.2 | 1723 | 1215.4 | 1528.1 | 389.5 | 566.5 | 1579.9 | 1152.9 |
| 95 | 493.2 | 509.2 | 582.1 | 343.1 | 199.5 | 575.4 | 147.5 | 827.4 |
| 96 | 154 | 286.2 | 193.1 | 193.6 | 157.8 | 242.7 | 283.8 | 221.5 |
| 97 | 289.4 | 1005.4 | 259.4 | 282.6 | 167.4 | 291 | 674.7 | 490.2 |
| 98 | 16.8 | 18.8 | 39.8 | 36.2 | 26.9 | 32.4 | 20.3 | 129.5 |
| 99 | 254.8 | 406.1 | 225.7 | 255.7 | 266 | 219.6 | 745 | 306.1 |
| 100 | 12961.3 | 633.2 | 23136.5 | 13157.8 | 4258.2 | 18457 | 92.1 | 9157.9 |
| 101 | 199.2 | 366.4 | 205.1 | 247.6 | 410.2 | 348.6 | 240.2 | 460.8 |
| 102 | 169.7 | 159.1 | 125.6 | 126.2 | 215.3 | 150.2 | 91.5 | 222 |
| 103 | 283.5 | 423.8 | 297.5 | 295.3 | 439.2 | 325.4 | 387.5 | 411.3 |
| 104 | 480.4 | 470.1 | 825.8 | 374.7 | 462.5 | 333.1 | 196.7 | 465.7 |
| 105 | 619.1 | 605.5 | 855.3 | 445.7 | 366.9 | 360.7 | 367.7 | 428.9 |
| 106 | 431 | 505.5 | 776.2 | 415.5 | 433 | 341.7 | 213.9 | 403.2 |
| 107 | 543.7 | 258.5 | 162.4 | 1100.5 | 444.6 | 341 | 846.1 | 327.4 |
| 108 | 369.2 | 171.8 | 356.8 | 273.4 | 382.3 | 416.2 | 184.3 | 269.6 |
| 109 | 2753.8 | 628.9 | 228.3 | 4738.3 | 100.4 | 1962.4 | 446.6 | 1981.7 |
| 110 | 4958.7 | 1350.1 | 414.3 | 7933.5 | 114.9 | 2744.5 | 1064.5 | 3917.5 |
| 111 | 1702 | 404.9 | 1150.9 | 1477.8 | 1041.7 | 814.8 | 162.5 | 988.1 |
| 112 | 1008.4 | 1893 | 534.7 | 114 | 706.3 | 402.2 | 1536.4 | 1239.5 |
| 113 | 151.5 | 782 | 135.7 | 174.6 | 265.6 | 218.3 | 1021.9 | 721.6 |
| 114 | 530.5 | 317.9 | 811.7 | 453.1 | 335.8 | 512.2 | 419.4 | 481.1 |
| 115 | 303.3 | 874.1 | 578.9 | 468.1 | 458.5 | 324.9 | 1401.9 | 823.6 |
| 116 | 268 | 457.4 | 242.4 | 317.8 | 206.9 | 239.4 | 1630.2 | 331.4 |
| 117 | 2483.3 | 467.5 | 3516.8 | 1446.4 | 1615.1 | 4189.5 | 228.4 | 1398 |
| 118 | 165.1 | 112 | 513.5 | 378.2 | 458.6 | 641.7 | 191.7 | 241.8 |
| 119 | 231.3 | 494.5 | 171.7 | 189.4 | 195.2 | 333.6 | 346.5 | 301.3 |
| 120 | 87.4 | 99.3 | 267.3 | 72.4 | 98.9 | 75.7 | 52 | 227.3 |
| 121 | 967.4 | 3533.9 | 789.3 | 1797 | 301.9 | 588.6 | 4329.5 | 1463.8 |
| 122 | 139.2 | 473.8 | 265.9 | 361.2 | 315 | 76.3 | 341.7 | 238.2 |
| 123 | 119.5 | 215.8 | 183.1 | 128.8 | 119.8 | 265.2 | 182.7 | 263.7 |
| 124 | 109.4 | 166.6 | 139.1 | 148.2 | 604.8 | 130.2 | 108.7 | 169.3 |
| 125 | 722.7 | 763.1 | 1037.4 | 1010.3 | 1402.8 | 848.6 | 637.5 | 908.8 |
| 126 | 1296.7 | 159.8 | 1627.8 | 874.6 | 884.3 | 818.1 | 110.9 | 767.8 |
| 127 | 104.8 | 194.8 | 105 | 95.2 | 146.2 | 98.1 | 305.7 | 145.8 |
| 128 | 224.9 | 308 | 285.9 | 277.2 | 363.9 | 241.5 | 136.6 | 243.7 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 129 | 545.4 | 2482.1 | 213.2 | 2357.2 | 237.7 | 1139.4 | 5618.8 | 1929.5 |
| 130 | 76.7 | 408.1 | 75.2 | 90.5 | 165.7 | 222.2 | 490.8 | 419.5 |
| 131 | 85.8 | 1368.9 | 53.3 | 125.2 | 103.8 | 83.8 | 726.3 | 483.3 |
| 132 | 172.5 | 245.6 | 197.9 | 213 | 230.3 | 179.6 | 212.1 | 210.3 |
| 133 | 146.7 | 108.3 | 66.2 | 57.7 | 106.6 | 66.4 | 177.9 | 79.6 |
| 134 | 143.3 | 290.6 | 125.8 | 87.5 | 146 | 152.7 | 489.7 | 203.5 |
| 135 | 483 | 571.2 | 802.1 | 590.4 | 416.8 | 293.2 | 388.6 | 465.7 |
| 138 | 139.2 | 119.6 | 184.4 | 178 | 169.6 | 75.2 | 370.9 | 179.5 |
| 139 | 121.1 | 452.7 | 115.4 | 122.7 | 113.3 | 162.6 | 239 | 194 |
| 140 | 4146.1 | 1876 | 1951.6 | 1338.3 | 211.9 | 1.2 | 179.7 | 833.9 |
| 141 | 1804.4 | 1208.1 | 1688 | 1567 | 2363.3 | 1017 | 699.3 | 1291.8 |
| 144 | 380.1 | 9550.3 | 459.6 | 472.6 | 561 | 7295.8 | 9513.3 | 10388.7 |
| 145 | 194.4 | 2091 | 162.4 | 172 | 259.9 | 188.3 | 229 | 274.5 |
| 146 | 205.4 | 202 | 199.5 | 239.1 | 259 | 184.3 | 149.4 | 241.4 |
| 147 | 52 | 107.5 | 75.7 | 55.9 | 66.3 | 46.2 | 53.6 | 102.4 |
| 148 | 718.7 | 437 | 450.1 | 1743.3 | 388 | 408.3 | 1716 | 259.8 |
| 149 | 513.6 | 418 | 556.5 | 723.8 | 170.1 | 284.3 | 345.2 | 370.6 |
| 150 | 340.8 | 469.1 | 189.2 | 647.8 | 337.7 | 133.8 | 357.6 | 199.7 |
| 151 | 378.3 | 501.3 | 350.8 | 284.8 | 186.5 | 295.1 | 1725 | 566.4 |
| 153 | 135.8 | 159.4 | 124.9 | 120.8 | 128.2 | 109.1 | 403.4 | 1078.5 |
| 154 | 315.7 | 3650.2 | 708.6 | 1687.8 | 203.3 | 594.7 | 7527.9 | 3225.5 |
| 155 | 173.6 | 320.6 | 127.8 | 180.3 | 169.7 | 101.4 | 200.9 | 264.6 |
| 156 | 45 | 254 | 61.8 | 80.7 | 117.5 | 103.3 | 127.8 | 570.5 |
| 157 | 136.9 | 129.1 | 120.6 | 168.2 | 166.4 | 137.2 | 114.9 | 166.3 |
| 158 | 1.2 | 666.7 | 6.7 | 71.9 | 42.6 | 71.2 | 1801.9 | 533.3 |
| 159 | 1953.4 | 2285.3 | 3885.5 | 2682.2 | 3034.8 | 3946.6 | 1345.5 | 2577.3 |
| 161 | 319.4 | 1302 | 72.9 | 3.4 | 29.5 | 683.5 | 715.7 | 2665.9 |
| 162 | 110.3 | 41 | 257 | 370 | 123.6 | 69.8 | 481.8 | 259.5 |
| 164 | 1671 | 9328.7 | 183.5 | 423.7 | 105.2 | 5095.9 | 2963.9 | 3993.3 |
| 166 | 4942.9 | 5074.8 | 7381.4 | 6040.8 | 6386.6 | 3311.8 | 2123.6 | 2331.2 |
| 167 | 749.2 | 988.4 | 3046.3 | 1523.6 | 2291.6 | 1961.5 | 174.8 | 2118.3 |
| 168 | 751.1 | 1098.9 | 495.4 | 268.2 | 581.5 | 558 | 495.7 | 1402.7 |
| 169 | 7503.7 | 2997.6 | 1624.4 | 7311.5 | 4532.9 | 8835.6 | 8236.4 | 8878.2 |
| 170 | 1092.7 | 3984.7 | 914.4 | 1353.1 | 1072.7 | 3583.1 | 6780.1 | 2356.3 |
| 171 | 1091.9 | 103.2 | 2345.5 | 1683.7 | 1336.1 | 67.4 | 1413.4 | 1527 |
| 172 | 647.2 | 545.2 | 686.6 | 676.8 | 882.2 | 643.6 | 381.4 | 537.6 |
| 173 | 310.1 | 807.9 | 316.1 | 454.7 | 125 | 570.2 | 2254.5 | 686.8 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 176 | 352.8 | 3659.5 | 161.7 | 682 | 66.9 | 511.8 | 1820.1 | 1938.3 |
| 177 | 266.4 | 422.7 | 627.2 | 464.7 | 698.3 | 660 | 182.2 | 629.9 |
| 178 | 78.1 | 2301.7 | 104.6 | 251.2 | 360.2 | 1258.1 | 2294.6 | 1651.5 |
| 179 | 128.9 | 1233.1 | 66.2 | 511.4 | 130.9 | 58.8 | 1647.2 | 1137.8 |
| 180 | 105.8 | 710.8 | 107 | 181.6 | 146.8 | 78.7 | 1093 | 420.8 |
| 181 | 9757 | 7620 | 13164.3 | 9078.6 | 11540.2 | 7914.9 | 4064.3 | 8345.1 |
| 182 | 496.8 | 473.5 | 600.9 | 574.6 | 265.4 | 353.5 | 605.3 | 428.7 |
| 183 | 392.6 | 39.9 | 1783.8 | 80.3 | 243.4 | 23.2 | 486.1 | 1115.1 |
| 184 | 259.1 | 22002.2 | 275.3 | 392.4 | 236.8 | 191.2 | 3061.9 | 2469.9 |
| 185 | 1311.7 | 3109.9 | 1695.2 | 2606.4 | 1042.9 | 801.2 | 1663.7 | 6383.1 |
| 186 | 309.2 | 248.2 | 731.9 | 456.2 | 752.1 | 448.3 | 238.7 | 330.6 |
| 187 | 360.2 | 80.5 | 282.7 | 112.1 | 108.2 | 52.7 | 216.2 | 310.5 |
| 188 | 146.5 | 209 | 366.3 | 96.4 | 142.6 | 103.3 | 898.6 | 358.8 |
| 189 | 84.1 | 133.6 | 168.1 | 73.6 | 133 | 48.1 | 304.3 | 209.3 |
| 190 | 66 | 370 | 79.8 | 99.8 | 90.5 | 387.4 | 665.7 | 640.7 |
| 191 | 1054.3 | 1687.1 | 1089.6 | 4008.9 | 457.1 | 1051.3 | 4042.4 | 1869.4 |
| 192 | 101.9 | 78.1 | 134.8 | 200.9 | 179.1 | 65.7 | 775.6 | 145.6 |
| 193 | 815.2 | 514.5 | 1367.9 | 1552.3 | 1059.7 | 2629.4 | 354.5 | 944.3 |
| 194 | 138.2 | 264.9 | 193.5 | 255.5 | 125.4 | 157.6 | 270.8 | 185.4 |
| 195 | 269.8 | 168.4 | 314.1 | 235.5 | 344.1 | 291 | 109.8 | 242.7 |
| 196 | 1835 | 1891 | 3022.5 | 2154.7 | 3025 | 5691.1 | 1224 | 6848.2 |
| 197 | 78 | 67 | 146.6 | 89.4 | 111.8 | 94.4 | 72.1 | 99.4 |
| 198 | 59.7 | 349.1 | 117.3 | 72.4 | 77.7 | 65.9 | 89.4 | 217.4 |
| 199 | 2202.9 | 301.5 | 120.6 | 6209.3 | 190.5 | 91 | 1705.6 | 642.7 |
| 200 | 369.9 | 467.8 | 572.4 | 164.5 | 285 | 358.3 | 533 | 694.8 |
| 201 | 4783.4 | 1093.3 | 42.1 | 7327.6 | 380.1 | 865.7 | 2830.3 | 670.3 |
| 203 | 229.2 | 565.9 | 244.9 | 314.3 | 126.5 | 350.8 | 4025.7 | 1159.8 |
| 204 | 1028.9 | 1353.2 | 742.4 | 630.8 | 467.1 | 794.8 | 1093.2 | 723.2 |
| 205 | 4521.1 | 3738.9 | 6165.2 | 5757.6 | 2714.1 | 3696 | 4479 | 5109.5 |
| 206 | 286.5 | 137.7 | 822.1 | 155.9 | 838.5 | 245.4 | 1616.9 | 203.7 |
| 207 | 1415.9 | 489.1 | 680.7 | 771.5 | 469.4 | 789.4 | 93.7 | 464.5 |
| 208 | 1235.8 | 212.4 | 285.9 | 104.7 | 204.8 | 139.6 | 283.2 | 359.2 |
| 209 | 833.2 | 1350.4 | 3254.1 | 1747.4 | 5771.7 | 1318.9 | 86.5 | 2452.8 |
| 211 | 11370.8 | 683.4 | 10746.5 | 11683.6 | 36602.3 | 14294.3 | 496.2 | 11150.1 |
| 212 | 417.3 | 1679.4 | 495.3 | 397.9 | 1235.8 | 814.5 | 486.7 | 989.5 |
| 213 | 80.3 | 68.5 | 84.3 | 144.4 | 80.3 | 59 | 74.4 | 100.7 |
| 215 | 1765.1 | 754.5 | 1147.4 | 1032.5 | 842.1 | 953 | 108 | 565.1 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 216 | 976.1 | 385.3 | 1162.1 | 844.6 | 488.1 | 945.3 | 574.9 | 513.2 |
| 217 | 246.7 | 1309.8 | 268.5 | 563.9 | 267.1 | 1014.6 | 687.1 | 752.5 |
| 218 | 309.8 | 166.4 | 348.5 | 328.9 | 276.7 | 314 | 111.9 | 166.4 |
| 220 | 75.3 | 89.1 | 81.1 | 68.8 | 102.4 | 64.6 | 58.5 | 66.7 |
| 221 | 711.2 | 968.6 | 587.7 | 780.3 | 463.3 | 617.2 | 1979.7 | 1046.5 |
| 223 | 360.9 | 409.7 | 327.9 | 542.2 | 323.6 | 203.4 | 535.9 | 305.2 |
| 227 | 720 | 681.3 | 761.1 | 2095.7 | 501.1 | 532.7 | 2050.6 | 2295.1 |
| 228 | 319.6 | 300.8 | 802.4 | 439.9 | 916.7 | 403.2 | 157.7 | 507 |
| 229 | 228.5 | 277.6 | 251.1 | 185.2 | 164.1 | 200.4 | 180.1 | 258.6 |
| 230 | 277.5 | 1169.1 | 166.3 | 609.9 | 138.4 | 112.4 | 1313.4 | 1176.2 |
| 231 | 146.9 | 281.7 | 111 | 166 | 120.6 | 124.9 | 1309.9 | 513.5 |
| 232 | 1325.8 | 198 | 6317 | 350.3 | 1427 | 140.7 | 1196.8 | 3680.1 |
| 233 | 6765.2 | 7965.7 | 6520.5 | 7610.7 | 2523.6 | 3946.5 | 15970.8 | 5207.9 |
| 235 | 798.9 | 137.6 | 1126.6 | 2196.9 | 740.6 | 73.9 | 877 | 866.4 |
| 236 | 109 | 495.7 | 368.7 | 241.9 | 407.9 | 187.5 | 496.9 | 426 |
| 237 | 1643.3 | 2222.9 | 1846 | 1647.1 | 1467.4 | 1755.2 | 1820.3 | 2097.6 |
| 238 | 1432.8 | 1140.6 | 1085.2 | 1024.6 | 592.9 | 1468.4 | 1314.4 | 1287.1 |
| 239 | 83.3 | 421.5 | 345.8 | 665.5 | 279.4 | 372.4 | 776.5 | 766.3 |
| 240 | 140.9 | 406.6 | 453 | 953.8 | 285.9 | 481.1 | 1780.9 | 951.5 |
| 241 | 234.7 | 932.4 | 931.7 | 1944 | 457.3 | 892.7 | 2797.1 | 2091.7 |
| 242 | 3314 | 21699.6 | 1079.3 | 21653.5 | 3263.3 | 3729.4 | 21913.7 | 24536 |
| 243 | 354 | 246.6 | 412.2 | 1107.5 | 269.7 | 217.5 | 691.3 | 311.1 |
| 244 | 222 | 388.3 | 205 | 223.8 | 105.6 | 325.5 | 474.4 | 399.6 |
| 246 | 472.3 | 1406 | 305.7 | 434.6 | 199.4 | 177.4 | 1088.7 | 901.8 |
| 247 | 92.8 | 98.8 | 151 | 126.2 | 202.2 | 107.4 | 83.4 | 102.1 |
| 248 | 212.2 | 3753.3 | 158.2 | 227.2 | 186.1 | 449.4 | 2480 | 3964.5 |
| 249 | 1409.4 | 1281.8 | 405.1 | 1043 | 649.5 | 1270.8 | 1958 | 1894.7 |
| 251 | 99.7 | 776.5 | 96.3 | 222.9 | 77.6 | 219.8 | 1995.8 | 859.5 |
| 253 | 275 | 293.5 | 302.1 | 128.1 | 252.8 | 311 | 316.4 | 291.9 |
| 254 | 695.9 | 1525.8 | 629.1 | 370.5 | 121.7 | 2570 | 1228.6 | 1090.4 |
| 255 | 60.9 | 2489.7 | 89.7 | 245.4 | 289 | 1251.3 | 2613.6 | 1698.6 |
| 256 | 12009.8 | 3823 | 76.1 | 6109 | 998.6 | 1154.1 | 6963.6 | 1470.4 |
| 257 | 181.8 | 218.1 | 208 | 382.8 | 124.9 | 107 | 369.9 | 216.5 |
| 258 | 864.1 | 663.9 | 212.2 | 1288.3 | 130 | 47.2 | 2891.5 | 350.9 |
| 259 | 235.3 | 658.4 | 1209.1 | 373.2 | 212.5 | 143.5 | 1662.8 | 1191.8 |
| 260 | 151.4 | 161.7 | 168.5 | 138.6 | 240.5 | 128.7 | 127.1 | 189.1 |
| 261 | 200.6 | 567.6 | 409.5 | 332.1 | 282.3 | 256.2 | 194 | 303.8 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 263 | 524.9 | 637.2 | 549.3 | 697.3 | 354.8 | 473.9 | 797.2 | 691.2 |
| 263 | 262.8 | 1110.6 | 213 | 541.8 | 164.6 | 154.5 | 1268.5 | 980.9 |
| 265 | 278.9 | 105.6 | 510.8 | 504.8 | 664 | 337.3 | 59.8 | 364 |
| 266 | 414.8 | 163.6 | 326.2 | 220.7 | 601.9 | 433.1 | 158.4 | 283.7 |
| 256 | 126.6 | 169.2 | 102.6 | 129.7 | 138.6 | 131.2 | 232.7 | 376.5 |
| 268 | 455 | 1366.4 | 1736.7 | 757.1 | 890.5 | 1003.2 | 289.5 | 1318 |
| 269 | 29.7 | 103.9 | 37 | 84.9 | 68.8 | 46 | 87.8 | 154.9 |
| 270 | 198.8 | 186.3 | 209.5 | 175.8 | 440 | 234.1 | 176.8 | 287.7 |
| 271 | 239.3 | 1388 | 167.3 | 2027.5 | 279.7 | 256.3 | 3061.5 | 829.7 |
| 272 | 368 | 514.5 | 501.9 | 443.2 | 535.3 | 513.2 | 464.6 | 370.6 |
| 273 | 3575.1 | 1142.1 | 4935.8 | 2965.1 | 4032.3 | 5489.5 | 628.9 | 5515.9 |
| 274 | 369 | 1242.3 | 929 | 638.7 | 910.6 | 393.1 | 3068.9 | 1428 |
| 275 | 331.8 | 1186.4 | 419.7 | 790.1 | 385.7 | 210.6 | 387.1 | 2099.4 |
| 276 | 1037.3 | 821.9 | 994.3 | 2971.4 | 802.9 | 655.6 | 3409.5 | 3911.4 |
| 277 | 154 | 235.3 | 180.6 | 192.9 | 222 | 146.1 | 209.9 | 210.9 |
| 278 | 393.7 | 2185.4 | 1095.9 | 1106.9 | 475.5 | 287.1 | 2762.2 | 1290.3 |
| 279 | 1931.9 | 1574.6 | 2162.6 | 1696.4 | 1482 | 1484.1 | 2472.1 | 2330 |
| 280 | 2954.9 | 2033.6 | 2673.9 | 2531.2 | 1688.4 | 1936.3 | 3691.5 | 2628.7 |
| 281 | 76.5 | 295.7 | 134.9 | 81.7 | 87.5 | 993.9 | 793 | 372.3 |
| 282 | 295.3 | 214.1 | 295 | 3209.3 | 379.2 | 245.1 | 981.6 | 261.1 |
| 283 | 249.5 | 269.5 | 265.3 | 232.1 | 431.8 | 208.7 | 205.3 | 271.7 |
| 284 | 73.5 | 112.2 | 61.9 | 75.7 | 76.4 | 97.7 | 57.1 | 92.7 |
| 285 | 111 | 99.4 | 75.2 | 101.1 | 94.7 | 68.6 | 93.9 | 98 |
| 286 | 629.8 | 52.2 | 799.8 | 1994.7 | 465.5 | 828.9 | 53.1 | 264.8 |
| 287 | 115.5 | 113 | 87.7 | 122.5 | 134.4 | 100.9 | 81.5 | 95.9 |
| 288 | 109.4 | 141.8 | 130.1 | 140.7 | 149.8 | 126.1 | 108.4 | 112.5 |
| 289 | 1666 | 1269.4 | 941.6 | 5416.8 | 893.2 | 209.9 | 5258.5 | 2320.1 |
| 291 | 79.6 | 122.1 | 96 | 292.2 | 114.6 | 97.9 | 167.1 | 160.9 |
| 292 | 226.5 | 5489.8 | 317.3 | 841.6 | 96.1 | 81.2 | 4786.4 | 2366.7 |
| 293 | 173.9 | 986.8 | 92 | 624.7 | 212.3 | 203.9 | 667.1 | 929.4 |
| 295 | 312.6 | 1375.5 | 229.5 | 1024.5 | 598 | 569.3 | 688.5 | 1755 |
| 296 | 1129.7 | 1734.4 | 1277.5 | 815.2 | 602.9 | 561.5 | 2271.7 | 1202.6 |
| 297 | 81.6 | 53.9 | 53.8 | 152.2 | 91.5 | 62.5 | 118.6 | 45.6 |
| 298 | 394.3 | 619.7 | 407.5 | 404.3 | 288.6 | 242.2 | 506.9 | 419.7 |
| 299 | 869.8 | 1276.7 | 1226.2 | 959.5 | 865.1 | 1714.9 | 2366.2 | 1985.2 |
| 300 | 273.3 | 1444.8 | 240.7 | 364.3 | 206.4 | 660 | 1568.7 | 998 |
| 301 | 354.5 | 293.6 | 797 | 439.2 | 5202.8 | 496.9 | 215.5 | 810.2 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 302 | 43 | 69.4 | 22.9 | 53.4 | 24.2 | 8.5 | 116.5 | 61.8 |
| 303 | 635.7 | 390 | 1008.6 | 773.9 | 568.9 | 855.4 | 413.7 | 576.5 |
| 304 | 565.4 | 436.2 | 225.3 | 950.6 | 633.7 | 252.3 | 868 | 425.2 |
| 305 | 213.5 | 1945 | 86.7 | 250.1 | 230.8 | 450.4 | 693.3 | 661.4 |
| 306 | 150.6 | 895.3 | 136.6 | 156.5 | 236.5 | 135.1 | 1745.4 | 158.5 |
| 307 | 127.6 | 203.9 | 134.7 | 192.6 | 264.9 | 138.8 | 211.5 | 228.6 |
| 308 | 1248.1 | 607.6 | 1715.7 | 815.5 | 1207.6 | 1667 | 526.5 | 1140.3 |
| 310 | 323.3 | 105.3 | 561.9 | 412.8 | 554.5 | 441.2 | 95.6 | 355.5 |
| 311 | 187.1 | 409 | 134.4 | 246.2 | 66.9 | 232.4 | 864.3 | 176.7 |
| 312 | 215.6 | 35.7 | 964.5 | 512.9 | 1442.3 | 743.1 | 1.2 | 712.1 |
| 313 | 102.1 | 614.8 | 77.9 | 187.7 | 226.5 | 400.1 | 210.6 | 454.4 |
| 314 | 681 | 1706.8 | 671.1 | 751.7 | 185.6 | 303.4 | 684.7 | 792.1 |
| 315 | 211.5 | 216.7 | 421.9 | 234 | 416.7 | 500.7 | 245.9 | 280 |
| 316 | 435.5 | 619.5 | 376.2 | 536.9 | 395.4 | 330.2 | 704.9 | 460.6 |
| 317 | 168.8 | 203.4 | 167.5 | 2721.9 | 212.3 | 142.8 | 142.3 | 295.9 |
| 318 | 129.1 | 300.4 | 132.8 | 150.6 | 54.1 | 39.7 | 1463.9 | 297.9 |
| 319 | 438.9 | 357.5 | 187.5 | 180.3 | 354.2 | 257.4 | 855.9 | 322.5 |
| 320 | 533.7 | 201.6 | 562 | 465.6 | 667.1 | 655.6 | 266.9 | 475.1 |
| 321 | 320.6 | 174.3 | 471.9 | 402.1 | 561.7 | 371.6 | 180.9 | 319.4 |
| 322 | 257.1 | 275.3 | 249 | 193.6 | 264.7 | 232 | 267.4 | 249.9 |
| 323 | 2838.3 | 3740 | 2267.7 | 2105.4 | 2020.1 | 7965.7 | 2455.6 | 6172.4 |
| 324 | 139.6 | 720.8 | 116.1 | 87.9 | 66.3 | 225.9 | 269 | 421.6 |
| 325 | 325.1 | 538.7 | 346.6 | 353 | 241.9 | 342.5 | 547.1 | 466.2 |
| 326 | 6936.9 | 3406 | 3724.5 | 6176 | 2949 | 3655.6 | 1280.4 | 2870.4 |
| 327 | 29.3 | 28.4 | 40.5 | 34.8 | 1.1 | 43.1 | 50.5 | 85.2 |
| 328 | 323.8 | 284.6 | 596.2 | 451.7 | 857.7 | 576.7 | 72.6 | 488.6 |
| 329 | 76 | 83.5 | 69.9 | 71.3 | 81 | 78.7 | 65.1 | 41.9 |
| 330 | 148.3 | 861.9 | 37.4 | 251.5 | 19.5 | 53.5 | 1666.2 | 255.1 |
| 331 | 232.2 | 284.6 | 291.4 | 279.4 | 449.1 | 243.7 | 257.3 | 263.5 |
| 333 | 420 | 491.9 | 777.5 | 589.6 | 728.3 | 591.1 | 250.1 | 419.1 |
| 334 | 179.2 | 509.5 | 135.4 | 165.9 | 136.1 | 152.8 | 170.2 | 551.5 |
| 335 | 109 | 243.2 | 144.4 | 104.1 | 452.1 | 72.9 | 710.8 | 914.6 |
| 336 | 765.5 | 230.7 | 670.7 | 465 | 499.8 | 294.9 | 129.3 | 318.8 |
| 337 | 312.9 | 1159 | 419.7 | 497.2 | 178 | 165.9 | 990.9 | 1049.7 |
| 338 | 577.4 | 617.7 | 185.9 | 401.6 | 177.5 | 251.9 | 956.2 | 491.5 |
| 339 | 392.3 | 332.8 | 470.3 | 340.9 | 615.4 | 377.2 | 170.7 | 322.8 |
| 340 | 246.7 | 275.9 | 213.8 | 279.9 | 271.3 | 234.8 | 170.6 | 251.1 |

(continued)

| 3i | | | | | | | | |
|---|---|---|---|---|---|---|---|---|
| SEQ ID NO: | 923TT | 924TT | 925TT | 926TT | 927TT | 928TT | 932TT | 933TT |
| 341 | 539.3 | 411.3 | 601 | 608.3 | 542.6 | 515.2 | 389.8 | 567.6 |
| 343 | 244.1 | 385 | 83 | 562.5 | 1435.6 | 259.1 | 2195.3 | 417.3 |
| 344 | 1212.4 | 452.6 | 1195.1 | 997.3 | 1945.3 | 1246.2 | 594.8 | 932 |
| 345 | 264.5 | 585.5 | 216.6 | 239.2 | 204.1 | 247.3 | 398.5 | 204.5 |
| 347 | 1738 | 3503.5 | 5107.2 | 2563.1 | 2564.3 | 2751.2 | 1484 | 4149.2 |
| 348 | 3976.7 | 7721.4 | 6875.5 | 4637.6 | 3708.3 | 6099.2 | 5834.6 | 7036.6 |
| 349 | 273.4 | 377.5 | 176.5 | 381.9 | 233.3 | 415.6 | 597.8 | 380.1 |
| 350 | 321.2 | 741.3 | 399.7 | 390.9 | 121.3 | 191.6 | 653.8 | 456.2 |
| 351 | 524.9 | 2215.5 | 274.7 | 977.6 | 159.5 | 507.2 | 2220.4 | 809.3 |
| 352 | 503.6 | 131.4 | 464 | 399.1 | 339.9 | 347.1 | 171.6 | 202.5 |
| 353 | 362.5 | 223.7 | 256.6 | 221.7 | 248.9 | 379.2 | 318.9 | 224.1 |
| | | | | | | | | |
| 3j | | | | | | | | |
| SEQ ID NO: | 938TT | 940TT | 941TT | 945TT | 946TT | 947TT | 948TT | 954TT |
| 354 | 124.7 | 131.5 | 68.5 | 1831.3 | 2991.1 | 1970.3 | 4151.3 | 697.9 |
| 355 | 60.5 | 58.5 | 18.7 | 651 | 2959.8 | 3221.3 | 3037.9 | 91.9 |
| 356 | 930.6 | 668.1 | 621.5 | 1481.2 | 524 | 619.9 | 852.9 | 343.7 |
| 357 | 668.5 | 1037.6 | 614.1 | 992.7 | 744.6 | 692.6 | 698.9 | 500.3 |
| 358 | 534.1 | 2157.9 | 778.4 | 766.2 | 2015.3 | 1757.9 | 1431.4 | 322.3 |
| 359 | 630.5 | 285.6 | 377.6 | 284.2 | 331.2 | 319.5 | 309.2 | 503.3 |
| 362 | 416.8 | 682.5 | 687 | 883.5 | 1080.8 | 836.2 | 746 | 476.8 |
| 360 | 275.7 | 208.2 | 221.9 | 220.7 | 571.6 | 596.9 | 804.3 | 159.4 |
| 361 | 583.8 | 838.6 | 283.7 | 134.5 | 371.1 | 271.3 | 137.7 | 156.1 |
| 363 | 425 | 358.8 | 438.2 | 340.4 | 460.3 | 536.2 | 369.7 | 849.5 |
| 1 | 912.1 | 505.2 | 651.7 | 1316.8 | 1552.6 | 1201.3 | 1465.6 | 256.1 |
| 2 | 2300.4 | 2928.6 | 1830.4 | 985.5 | 3226.8 | 1970 | 1555.6 | 876.4 |
| 7 | 656.3 | 686.7 | 1076.6 | 47.5 | 1068.9 | 536 | 365.4 | 1299.1 |
| 8 | 1044.1 | 2152.8 | 933.8 | 991 | 820.3 | 808.2 | 739.8 | 1547.8 |
| 9 | 4509.2 | 1083.6 | 1954.2 | 1300.1 | 2078.3 | 3908 | 1283.1 | 685.2 |
| 10 | 140.1 | 88.1 | 122.4 | 1873.2 | 92.1 | 150.4 | 164.5 | 113.4 |
| 11 | 70.2 | 163.3 | 140.5 | 707 | 443.9 | 558.3 | 863.6 | 82.9 |
| 13 | 151.7 | 615.2 | 230.4 | 207 | 349.6 | 491.1 | 268.6 | 107.8 |
| 14 | 292.7 | 1519.9 | 379.4 | 942.7 | 1077.9 | 1723.4 | 1237 | 119.8 |
| 15 | 287.3 | 218.1 | 204.7 | 3524.8 | 341.4 | 2287.5 | 744.4 | 147.9 |
| 16 | 1093.9 | 1353.6 | 1394.3 | 376.6 | 2242.4 | 2345.1 | 4508.1 | 532.5 |
| 17 | 1167.2 | 1155.9 | 933 | 517 | 1588.4 | 984.5 | 1068.3 | 312.3 |
| 19 | 241.1 | 159.7 | 215.2 | 431.1 | 311 | 2812 | 648.8 | 285.5 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 20 | 1.2 | 13.8 | 1.1 | 238.8 | 388.1 | 452.5 | 759.1 | 1.2 |
| 21 | 13.1 | 453.2 | 114.2 | 12979.2 | 1497.1 | 2096.8 | 4503 | 98.9 |
| 22 | 3034.3 | 2692.9 | 7458.4 | 7149.4 | 3919.7 | 5688.1 | 5625.7 | 1444.4 |
| 23 | 240.2 | 50.1 | 1.1 | 2306.1 | 246.9 | 2461.4 | 936.2 | 1.1 |
| 24 | 167.4 | 59.1 | 67 | 383.4 | 283.5 | 552.3 | 205.8 | 59.9 |
| 26 | 1363.1 | 333 | 155 | 3026.1 | 1238.7 | 9164 | 2694.5 | 66.1 |
| 27 | 664.2 | 691.5 | 441.3 | 957.5 | 1357 | 954.3 | 1589.7 | 352.8 |
| 29 | 89.7 | 109.7 | 122.7 | 3754.7 | 40.3 | 136.4 | 202.8 | 163.6 |
| 30 | 132.2 | 43.3 | 144.7 | 1236.6 | 203.1 | 1185.3 | 426.3 | 73.8 |
| 31 | 70.5 | 216.2 | 85.5 | 16661.7 | 8943.3 | 13168.1 | 16458.7 | 70.6 |
| 33 | 754.7 | 291.7 | 496 | 10017.8 | 2060.8 | 7990.6 | 3081.2 | 83.8 |
| 34 | 3799.4 | 1463.1 | 2114.5 | 557.9 | 529.3 | 1398.3 | 512.3 | 4496.7 |
| 35 | 49.6 | 245.8 | 449.4 | 283.1 | 1235.6 | 706.1 | 239.4 | 71 |
| 36 | 319.6 | 311.9 | 235.6 | 621.1 | 2677.9 | 398 | 273.3 | 356.8 |
| 37 | 165.2 | 175.1 | 219.1 | 198.1 | 231.1 | 292.9 | 220.8 | 375.8 |
| 38 | 588.7 | 850.6 | 583.8 | 5243.7 | 1146.5 | 3990 | 1615.2 | 79.3 |
| 40 | 474.3 | 418.3 | 456.1 | 5232.8 | 561 | 592.8 | 854.7 | 557.2 |
| 41 | 145.4 | 200.5 | 95.4 | 209.9 | 739.6 | 1408.3 | 703.3 | 263.3 |
| 42 | 259.7 | 422.1 | 296.8 | 134.4 | 388.8 | 299.2 | 320 | 346.4 |
| 43 | 312.7 | 469.7 | 413.4 | 844.8 | 254.3 | 342.2 | 278.6 | 1487.4 |
| 44 | 513.5 | 424.1 | 470.7 | 554 | 1223.3 | 1138.7 | 885.5 | 455.5 |
| 45 | 890.2 | 736.7 | 547.2 | 940.6 | 1426.2 | 1512.1 | 1930.9 | 1179.9 |
| 46 | 245 | 310.6 | 350.6 | 275.4 | 289.5 | 350 | 292.2 | 348.2 |
| 47 | 282.9 | 220.4 | 144.2 | 180.6 | 703.3 | 454.3 | 694.6 | 183.6 |
| 48 | 65.9 | 25.3 | 41.1 | 147.2 | 354.7 | 1468.6 | 413 | 15.7 |
| 49 | 257.1 | 1250.1 | 224.5 | 12537.4 | 5118.7 | 2265.3 | 4031 | 294.4 |
| 50 | 174.5 | 90.2 | 118.5 | 1385.9 | 212.4 | 962.9 | 427.9 | 32.6 |
| 51 | 95.1 | 552.7 | 56.4 | 170.8 | 478 | 419.9 | 827.7 | 730.2 |
| 52 | 197 | 196.4 | 147.1 | 401.4 | 251.1 | 232.6 | 352.8 | 240.3 |
| 53 | 48.5 | 381.9 | 96.1 | 1013.6 | 2192.5 | 931.3 | 1091.5 | 1.7 |
| 54 | 826.4 | 1710.8 | 514.8 | 1477.3 | 1185.6 | 797.4 | 1536.4 | 340.4 |
| 55 | 115.1 | 111.9 | 94.4 | 164.7 | 94 | 80.6 | 89.7 | 130.4 |
| 56 | 64 | 268.4 | 79.2 | 90.4 | 219.3 | 188.7 | 175.5 | 63.7 |
| 57 | 617.9 | 1038.1 | 375.9 | 868.2 | 12415.3 | 3983 | 1493.6 | 579.1 |
| 58 | 584.4 | 972.6 | 292.8 | 2931.7 | 11316.3 | 5093.9 | 2162.2 | 939.4 |
| 59 | 1552.8 | 1358.2 | 1824.4 | 3299.3 | 3044.8 | 3483.9 | 4854.3 | 122.2 |
| 60 | 123.4 | 154 | 130.4 | 239.2 | 412.9 | 378.4 | 248.4 | 139.6 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 61 | 193 | 106.4 | 98 | 402.3 | 130.9 | 79 | 142.9 | 155.7 |
| 62 | 1365.1 | 178 | 2305.2 | 2981.4 | 3752.1 | 2025.7 | 3563.2 | 4345.7 |
| 63 | 145.2 | 365.5 | 174.3 | 650.2 | 742.9 | 588.7 | 744.3 | 153.8 |
| 64 | 143.1 | 168.7 | 146.9 | 198.1 | 479.3 | 303.3 | 288.3 | 1482.7 |
| 65 | 149.3 | 80.8 | 54.7 | 446.7 | 254.3 | 265.4 | 155.3 | 56.9 |
| 66 | 834.1 | 822.9 | 752.9 | 332.7 | 1793.6 | 1625.8 | 2019.9 | 272.4 |
| 67 | 35954.4 | 28315.7 | 36691.2 | 2509.3 | 29331.4 | 28689.2 | 19265.7 | 24099.5 |
| 68 | 2348.5 | 1225 | 3289.6 | 23.4 | 760.4 | 1000 | 260.1 | 1110.3 |
| 69 | 3844.9 | 1911.7 | 4757.3 | 675.4 | 2667.4 | 3541.2 | 1114.3 | 7548.2 |
| 70 | 2817.2 | 5474 | 2510.6 | 6068.1 | 4627.5 | 5853 | 4221.9 | 8610 |
| 71 | 194.3 | 578.8 | 125.9 | 216.5 | 9508.5 | 3820.8 | 1470.2 | 84.3 |
| 72 | 588.1 | 1284.2 | 401.1 | 327.2 | 2278.5 | 1343.5 | 1418 | 131 |
| 73 | 258 | 239.7 | 218.8 | 232.7 | 766.7 | 694.8 | 726.4 | 198.6 |
| 75 | 252.1 | 212.9 | 239.3 | 123.9 | 1227.9 | 463.3 | 938.9 | 272.6 |
| 76 | 2962.6 | 845.5 | 915.9 | 198.8 | 1587.9 | 1178 | 574.9 | 3611 |
| 77 | 16342.3 | 2666.5 | 15026.8 | 503.1 | 1235 | 1954.2 | 2093.6 | 4483.2 |
| 78 | 729.3 | 762.4 | 748.4 | 157.2 | 758.2 | 290.5 | 576.4 | 1050.6 |
| 79 | 174.2 | 122.2 | 114.9 | 257.1 | 232.1 | 270.7 | 344.5 | 138 |
| 80 | 1248 | 787.5 | 1009.5 | 282.2 | 770.1 | 649.9 | 1098.8 | 266.3 |
| 81 | 115.6 | 272.3 | 127.3 | 229.2 | 5071.1 | 11031.3 | 7637.7 | 43.7 |
| 82 | 610.7 | 314.6 | 210.5 | 232 | 257.3 | 258.9 | 135.4 | 1823.3 |
| 84 | 94.2 | 94.6 | 92.6 | 83 | 304.7 | 340 | 208.7 | 87.3 |
| 85 | 140.2 | 169.4 | 138.1 | 2066.1 | 1108.1 | 1254 | 441.8 | 428.8 |
| 86 | 39.9 | 71.9 | 99.7 | 82.2 | 84.5 | 383.5 | 141 | 293.5 |
| 87 | 298.4 | 166.5 | 180.3 | 11204.7 | 1172.7 | 669.1 | 1630.9 | 199.4 |
| 88 | 5196.8 | 3340.8 | 5827.4 | 127.5 | 1059.3 | 1704.6 | 635.3 | 1431.5 |
| 89 | 235.6 | 196.8 | 215.8 | 267.6 | 325.5 | 267.5 | 617.5 | 237 |
| 90 | 115.4 | 85.7 | 96.9 | 368.5 | 152.8 | 292.9 | 344.8 | 75.4 |
| 91 | 522 | 538.6 | 254.8 | 3408.3 | 6047.2 | 2646.8 | 1460.5 | 1153.4 |
| 92 | 3787.5 | 7863.1 | 2264 | 316.1 | 7435.1 | 5854.5 | 5932.4 | 213.6 |
| 93 | 338.7 | 224 | 230.8 | 159.4 | 208.2 | 159.2 | 161.6 | 162.1 |
| 94 | 635.6 | 617.5 | 996.8 | 254.1 | 860.9 | 768 | 1265.5 | 29.8 |
| 95 | 699.7 | 1108.4 | 462.4 | 81.3 | 719 | 569.5 | 312.2 | 76.1 |
| 96 | 333 | 207.6 | 214.4 | 289.7 | 323.1 | 290.3 | 295.4 | 231.9 |
| 97 | 416.2 | 279.5 | 425.1 | 563.7 | 405.6 | 387 | 496.6 | 170.1 |
| 98 | 27.3 | 37.6 | 37.7 | 36.2 | 18.7 | 37.2 | 39.6 | 211.1 |
| 99 | 301.5 | 228.7 | 224.2 | 752.5 | 370.7 | 398.9 | 427.9 | 210 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 100 | 2314.7 | 3267.4 | 16920.7 | 300.1 | 254.3 | 4199.9 | 860.3 | 2812.9 |
| 101 | 330.3 | 321.7 | 727.2 | 74.4 | 500.6 | 272 | 225.2 | 171.9 |
| 102 | 169.4 | 127.4 | 144.6 | 162.3 | 150.2 | 178.5 | 170.9 | 171.9 |
| 103 | 381.1 | 314.1 | 488 | 277.5 | 491.6 | 397.1 | 366.3 | 377.4 |
| 104 | 692.9 | 415.8 | 921.4 | 315.6 | 185.7 | 234.9 | 58.6 | 310.2 |
| 105 | 372.6 | 381.6 | 637 | 159.3 | 149.3 | 300.4 | 79.5 | 455.3 |
| 106 | 646.1 | 411.7 | 1024.2 | 213.3 | 167.6 | 292.1 | 69.4 | 362 |
| 107 | 267 | 257.5 | 123.5 | 748.7 | 451.6 | 527.6 | 584.4 | 230.2 |
| 108 | 573.6 | 195.9 | 332.9 | 184.1 | 209.2 | 219.8 | 263 | 536.8 |
| 109 | 61.9 | 1019.9 | 56.4 | 186.2 | 3392.9 | 1439.4 | 1742.7 | 27.8 |
| 110 | 25.1 | 2062.4 | 22.9 | 215.1 | 7704.2 | 3177 | 4011.8 | 1.1 |
| 111 | 143.5 | 479.3 | 2111.4 | 41.4 | 269.7 | 337.1 | 266.3 | 190.6 |
| 112 | 551.8 | 742.9 | 559 | 155 | 1022.1 | 1281.4 | 1187.5 | 1294.4 |
| 113 | 99.7 | 563.8 | 103.4 | 189.8 | 444.1 | 262.8 | 316.1 | 223.5 |
| 114 | 503.8 | 603.2 | 350.4 | 104.9 | 714.7 | 677 | 361.5 | 212.4 |
| 115 | 401.8 | 436 | 616.1 | 1714.8 | 863.7 | 903.2 | 724.2 | 444.3 |
| 116 | 209.7 | 203.7 | 150.5 | 2167.8 | 763.4 | 502.8 | 591.3 | 201.2 |
| 117 | 3806.6 | 1057.1 | 3146.1 | 179.1 | 561.9 | 533.2 | 197.2 | 320 |
| 118 | 138.9 | 49.5 | 85.3 | 477.1 | 265.1 | 194.4 | 119.2 | 509.1 |
| 119 | 81.2 | 371.2 | 135.5 | 216 | 567.1 | 144.4 | 163.5 | 165.4 |
| 120 | 160.6 | 114.3 | 71.9 | 92.6 | 176.6 | 216.8 | 101.4 | 84.2 |
| 121 | 164.4 | 411.2 | 437.2 | 213.3 | 3724.5 | 1512.3 | 3717.5 | 67.3 |
| 122 | 98.8 | 80.1 | 243.2 | 1399.7 | 264.3 | 428.5 | 386 | 304.3 |
| 123 | 228.3 | 146.3 | 183.8 | 86 | 273.3 | 303.5 | 160.2 | 337.2 |
| 124 | 151.3 | 149.7 | 152.5 | 120.2 | 120.6 | 139.9 | 109.1 | 1219.8 |
| 125 | 909.1 | 689.8 | 819.3 | 575.9 | 33514.1 | 1041.2 | 939.8 | 1157.9 |
| 126 | 1425.9 | 1166.4 | 900.7 | 103.3 | 147.1 | 234.7 | 109.9 | 694.2 |
| 127 | 99.1 | 86.5 | 106.2 | 122.9 | 202.2 | 163.7 | 251.1 | 114.5 |
| 128 | 231.7 | 208 | 253.1 | 214 | 261.3 | 71697.2 | 219.5 | 291.6 |
| 129 | 202.2 | 353.5 | 133.8 | 1125.3 | 2440.8 | 1025.8 | 1063.6 | 165.3 |
| 130 | 345.4 | 395.1 | 121 | 69 | 388.5 | 391.7 | 446.8 | 121.4 |
| 131 | 168.1 | 194.1 | 81.9 | 126.4 | 965.8 | 403.9 | 495.8 | 134.7 |
| 132 | 181 | 188.5 | 164.5 | 532.9 | 286.9 | 263.7 | 252.1 | 213.2 |
| 133 | 101.3 | 107.4 | 143.6 | 234.5 | 193.6 | 164.6 | 236.4 | 134.6 |
| 134 | 208.4 | 308.8 | 149 | 144 | 300.6 | 320.7 | 552.5 | 107.9 |
| 135 | 238.9 | 233.2 | 326.6 | 225.1 | 687.6 | 468.7 | 352.5 | 301.7 |
| 138 | 96.3 | 108.4 | 108 | 222.7 | 408.8 | 249.3 | 192.4 | 170.3 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 139 | 165.6 | 99.9 | 76.6 | 136.8 | 269.3 | 296.3 | 159 | 95.6 |
| 140 | 2802.5 | 2059.9 | 1086.6 | 36.8 | 599.5 | 750.1 | 666.1 | 1.3 |
| 141 | 2290.9 | 2263.4 | 2358.9 | 108.9 | 934.1 | 627.7 | 325.8 | 732.5 |
| 144 | 7698.2 | 8602.5 | 444.5 | 323.9 | 12464.5 | 11467.6 | 7038 | 368 |
| 145 | 193.7 | 146.1 | 183.3 | 157.9 | 225.5 | 228.7 | 268.8 | 245.8 |
| 146 | 240.8 | 175.6 | 204.2 | 249.1 | 261 | 358.4 | 743.9 | 242.2 |
| 147 | 47.8 | 28.4 | 34.3 | 55 | 200.2 | 100.2 | 51 | 64.4 |
| 148 | 229.4 | 308.8 | 238.2 | 263.9 | 480.4 | 561.5 | 358.6 | 145.1 |
| 149 | 551.3 | 349.3 | 339.3 | 928.4 | 251.7 | 301.5 | 518.5 | 137 |
| 150 | 164.4 | 207.3 | 163.4 | 141.1 | 291.4 | 208.5 | 289.6 | 323.1 |
| 151 | 126.6 | 323.1 | 102.9 | 132 | 599.9 | 423.7 | 877.9 | 89.4 |
| 153 | 115.7 | 114.6 | 91 | 123.5 | 136.1 | 115.3 | 108.6 | 154.4 |
| 154 | 96.3 | 720.7 | 304.7 | 130.9 | 1984.8 | 5390.1 | 6547.4 | 79.7 |
| 155 | 97.9 | 124.5 | 101.4 | 112.9 | 272.1 | 587.2 | 922.9 | 167.6 |
| 156 | 87.7 | 90.5 | 95.8 | 49 | 682.8 | 491.1 | 331.1 | 59.2 |
| 157 | 111.6 | 117.5 | 128.1 | 151.8 | 208 | 161.9 | 186.4 | 149.3 |
| 158 | 65.6 | 1732.5 | 86.6 | 324.9 | 473.6 | 737.6 | 1773.8 | 15.3 |
| 159 | 3116.8 | 3452 | 5239.7 | 900.3 | 2646.8 | 1464.2 | 1902.5 | 980 |
| 161 | 14481.9 | 1741.5 | 2880.9 | 1.1 | 4831.7 | 8199.7 | 3949.6 | 50.7 |
| 162 | 89.7 | 55.3 | 28.4 | 589.7 | 490 | 589.7 | 221.5 | 35.9 |
| 164 | 177.5 | 8435.5 | 104.7 | 76.7 | 5265.1 | 293.6 | 4062.9 | 88.1 |
| 166 | 4359.3 | 2839.1 | 6163 | 1137.7 | 1081.2 | 2208.2 | 2366.9 | 3751.1 |
| 167 | 1749.1 | 1818.8 | 3470.5 | 1.2 | 1723.1 | 1291.3 | 832.1 | 565.3 |
| 168 | 710.2 | 1123.1 | 423.8 | 59.3 | 1688.4 | 894.7 | 1924 | 122.3 |
| 169 | 1524.8 | 2200.3 | 2148.9 | 1401.8 | 11518.5 | 12728.9 | 6382.1 | 1374.4 |
| 170 | 1062.4 | 2335.7 | 758 | 1597.8 | 2005.8 | 2041.3 | 3494.1 | 467.3 |
| 171 | 3638.6 | 58 | 1497.1 | 1638.6 | 545 | 2916.5 | 723.7 | 1.4 |
| 172 | 663.9 | 679.9 | 727.5 | 420.9 | 685.3 | 596.1 | 496.1 | 610.3 |
| 173 | 416.9 | 738.3 | 140.5 | 328.5 | 1336.6 | 772.4 | 1320.3 | 267.7 |
| 176 | 1.1 | 6520.4 | 150.5 | 99.2 | 3722.2 | 368.3 | 1845.4 | 29.3 |
| 177 | 545.8 | 562.4 | 1099.7 | 185.2 | 574 | 488 | 384.2 | 598 |
| 178 | 1775.4 | 1955 | 376.9 | 512.6 | 2127.5 | 1048.9 | 1852.4 | 1565.3 |
| 179 | 180.3 | 138.6 | 117.4 | 2012.3 | 1196.7 | 828.9 | 1163 | 106.1 |
| 180 | 74.4 | 892.7 | 105.8 | 237.5 | 803.4 | 419.6 | 473.4 | 114.3 |
| 181 | 9404.2 | 6484.3 | 10300.3 | 941.7 | 4892.7 | 6450.6 | 4354.9 | 9168.5 |
| 182 | 372 | 308.8 | 208.5 | 248.4 | 424.6 | 399.7 | 405 | 287.1 |
| 183 | 264 | 49.6 | 27.6 | 849.9 | 182.8 | 2050.1 | 619.5 | 1.1 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 184 | 340.2 | 3360.4 | 202.1 | 1220.7 | 1934.2 | 441.7 | 306.9 | 181.9 |
| 185 | 955.1 | 847.9 | 908.6 | 8586.1 | 5990 | 6583.4 | 4156.5 | 642.7 |
| 186 | 377.1 | 397.9 | 305.4 | 331.6 | 290.1 | 282.2 | 230.3 | 953.8 |
| 187 | 63 | 83.5 | 54.1 | 487.3 | 334.1 | 525.2 | 118.2 | 375.2 |
| 188 | 135.2 | 163.5 | 93.8 | 278.1 | 334.8 | 593.6 | 565 | 40.8 |
| 189 | 97.4 | 79.7 | 73.5 | 170.6 | 247.6 | 288.5 | 313.7 | 63.8 |
| 190 | 71.9 | 271.7 | 441.9 | 766.9 | 331.8 | 397.9 | 570.8 | 70.8 |
| 191 | 1184 | 1205.3 | 431.3 | 1127.3 | 2057.1 | 2357.6 | 1735.3 | 1441.4 |
| 192 | 637.6 | 81.1 | 90.9 | 98.5 | 97.8 | 124.5 | 152.4 | 136.2 |
| 193 | 1083.7 | 1465.1 | 2071.9 | 376.7 | 704.1 | 341.3 | 284.4 | 528.8 |
| 194 | 105.9 | 174.3 | 134.6 | 104.3 | 225.4 | 234.8 | 239.8 | 67.9 |
| 195 | 513.2 | 534.1 | 382 | 172.8 | 221 | 202.4 | 154.9 | 532.6 |
| 196 | 6150.6 | 6442.8 | 10030.2 | 655.3 | 4512.8 | 2383.7 | 1783.6 | 3777.8 |
| 197 | 119.9 | 70.8 | 66.6 | 405.3 | 140.1 | 126.5 | 270.4 | 248.4 |
| 198 | 86.4 | 67.1 | 56.7 | 167.2 | 622.1 | 1324.8 | 1830.6 | 65.7 |
| 199 | 63.7 | 49.5 | 36.5 | 946.4 | 3952.8 | 4557 | 3993.9 | 70 |
| 200 | 141.2 | 131.1 | 93.9 | 326.4 | 480.8 | 559.4 | 384.1 | 118.8 |
| 201 | 99.9 | 125.1 | 64.3 | 1769.6 | 3178.7 | 2044.9 | 3554.3 | 746.9 |
| 203 | 205.7 | 2333.5 | 101.7 | 55 | 1633.6 | 575.7 | 143.9 | 68.5 |
| 204 | 536.9 | 539.6 | 677.1 | 33.9 | 1308 | 734.6 | 891.1 | 781.5 |
| 205 | 2519.6 | 5145.4 | 5497.1 | 266.3 | 3930.6 | 3699 | 1755.2 | 3034.5 |
| 206 | 161.9 | 155.6 | 164.4 | 948.8 | 1321.7 | 387.6 | 243.4 | 466.4 |
| 207 | 632.9 | 3069.3 | 716.5 | 82.8 | 354.7 | 315 | 82.2 | 99.4 |
| 208 | 414.1 | 294.1 | 69.8 | 102.5 | 523.4 | 2129.8 | 361.4 | 69 |
| 209 | 2353.2 | 3015.8 | 3230.6 | 167.6 | 1434.9 | 1049.6 | 343.5 | 3631.8 |
| 211 | 25849.9 | 14301.8 | 31677 | 367.5 | 805.9 | 1538.7 | 536.8 | 17099.2 |
| 212 | 806.2 | 1682.7 | 669.3 | 1766.7 | 1465.3 | 1682.1 | 1111.3 | 2465.1 |
| 213 | 95.8 | 142 | 90.3 | 97.7 | 93.5 | 71.1 | 127 | 3700.6 |
| 215 | 908.3 | 4183.7 | 1058.1 | 54.1 | 546.4 | 345.5 | 127.3 | 143.3 |
| 216 | 474.8 | 805.2 | 456.5 | 290.5 | 173.7 | 292.6 | 242.1 | 234.8 |
| 217 | 310.4 | 448.5 | 198 | 253 | 2353.6 | 1241.6 | 1337.2 | 128.5 |
| 218 | 356.8 | 253.7 | 304.9 | 121 | 189.9 | 218 | 303.7 | 349.5 |
| 220 | 88.7 | 59 | 60.1 | 118.1 | 89.5 | 87.6 | 93.5 | 429 |
| 221 | 480.5 | 659.6 | 319.4 | 233.3 | 1004.2 | 1199.6 | 880.1 | 240.6 |
| 223 | 244.2 | 220.3 | 254.2 | 2169.6 | 392.2 | 560.5 | 572.7 | 233.1 |
| 227 | 757 | 501.6 | 622.8 | 6541.8 | 749.4 | 5527.9 | 2138.8 | 93.6 |
| 228 | 608.7 | 551 | 1481.7 | 131.1 | 431.4 | 377.7 | 265 | 587.1 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 229 | 493.1 | 133.9 | 211.8 | 452.4 | 129.8 | 269.4 | 179.7 | 599 |
| 230 | 161.6 | 103.2 | 123.8 | 1187.9 | 2084.8 | 2370.1 | 4149.4 | 75.2 |
| 231 | 118.2 | 789.1 | 176.3 | 80 | 752.2 | 257.9 | 129.9 | 148.9 |
| 232 | 941.1 | 198.3 | 129.7 | 1787.8 | 868.5 | 6851.2 | 1950.1 | 5.6 |
| 233 | 7265.5 | 4514.5 | 2133.7 | 1354.4 | 13569.2 | 13328.5 | 4088.9 | 897.7 |
| 235 | 824 | 100.3 | 178.6 | 1170.4 | 445.6 | 1811 | 569.9 | 75.9 |
| 236 | 205.3 | 143.9 | 239.7 | 348.8 | 434.4 | 422.2 | 446.1 | 266.4 |
| 237 | 1942.7 | 1890.5 | 1088.6 | 2229.9 | 2092.3 | 1996.6 | 2088.9 | 774.4 |
| 238 | 939.7 | 1507.6 | 905.5 | 237.4 | 920 | 814.4 | 934.8 | 213.8 |
| 239 | 155.3 | 1246.4 | 106.2 | 990 | 1304.2 | 963.5 | 415.2 | 125.6 |
| 240 | 105.3 | 1516.5 | 105.5 | 2154.1 | 1232.9 | 1172.8 | 394.5 | 111.2 |
| 241 | 298.2 | 3242 | 161.3 | 3194.2 | 2781.8 | 2574.4 | 962 | 156 |
| 242 | 6684 | 765 | 2229.5 | 13301.5 | 22076.9 | 31987.1 | 27579.2 | 969.6 |
| 243 | 154.1 | 116 | 133.6 | 2751 | 287.9 | 733.2 | 891.1 | 152.3 |
| 244 | 177 | 177.5 | 176.5 | 466.1 | 826.4 | 342.9 | 309.4 | 68.7 |
| 246 | 291.6 | 200.2 | 283.7 | 627.1 | 1322 | 1294.2 | 1779 | 143.8 |
| 247 | 98.8 | 89.9 | 132.5 | 88.7 | 163 | 133.7 | 73.4 | 283.9 |
| 248 | 126.4 | 1414.5 | 201.1 | 404 | 3465.6 | 1012.6 | 1020 | 178.9 |
| 249 | 782.4 | 3162 | 1403.3 | 76.1 | 1097.8 | 767.3 | 537.1 | 537.9 |
| 251 | 161.5 | 155.1 | 63.5 | 1785.1 | 627.7 | 1481.5 | 1000.6 | 82.3 |
| 253 | 264.5 | 218 | 442.9 | 168.6 | 424.5 | 446.9 | 362.6 | 279.9 |
| 254 | 105.6 | 2093.1 | 81.7 | 82.5 | 1493.7 | 652.8 | 622.8 | 398.9 |
| 255 | 1747.2 | 2263.9 | 395 | 659.6 | 2271.9 | 1148.5 | 1978.8 | 1845.7 |
| 256 | 435.9 | 539.6 | 279.6 | 2547.3 | 3827.2 | 1885.7 | 1217.9 | 1110.2 |
| 257 | 56 | 110.7 | 80.2 | 207.2 | 269.3 | 316 | 390.9 | 8.1 |
| 258 | 9.3 | 1.3 | 118.2 | 870 | 206.6 | 1331.3 | 3545.5 | 38.6 |
| 259 | 108.7 | 29.1 | 63 | 254.6 | 602.2 | 1965.4 | 1032.4 | 1.2 |
| 260 | 164.7 | 153.3 | 244.8 | 156.4 | 212.5 | 156.2 | 106.4 | 401.2 |
| 261 | 991.9 | 382.7 | 312.5 | 75.3 | 207.2 | 161.7 | 238.6 | 71.9 |
| 263 | 714.7 | 331.8 | 539.2 | 1150 | 469.9 | 540.1 | 765.1 | 394.2 |
| 263 | 186.9 | 138 | 97.6 | 1287.2 | 1762.6 | 2495.8 | 3807.6 | 183.3 |
| 265 | 788.6 | 425.8 | 473.1 | 76.4 | 87.4 | 170.1 | 56.1 | 398 |
| 266 | 353.2 | 233.3 | 346.6 | 277.1 | 270.4 | 331.4 | 210.9 | 954.6 |
| 256 | 72.3 | 174.2 | 105.6 | 365 | 383.6 | 281.3 | 202.2 | 129.4 |
| 268 | 281.1 | 1707.5 | 870.9 | 706.9 | 2068.2 | 688.8 | 318 | 283.3 |
| 269 | 39.1 | 44.6 | 40 | 54.8 | 173.2 | 430.9 | 328.8 | 58.9 |
| 270 | 215.2 | 272.7 | 184.8 | 275.7 | 242.6 | 247.3 | 201.7 | 957.5 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 271 | 235.7 | 337.1 | 250.4 | 160.2 | 871.9 | 511.6 | 907.5 | 198.1 |
| 272 | 413.3 | 461.6 | 314.7 | 697 | 411.8 | 753.7 | 501.7 | 539.4 |
| 273 | 1403.7 | 2489.3 | 5667.8 | 24.3 | 1615.9 | 2911.3 | 1683 | 814.1 |
| 274 | 614.7 | 410.3 | 702.2 | 2259.7 | 916.5 | 1540.8 | 1573.5 | 855 |
| 275 | 249.7 | 229.2 | 228.5 | 8716.5 | 2253.7 | 2452.5 | 1476.9 | 255.5 |
| 276 | 1102.2 | 729.4 | 823 | 8564.6 | 1180 | 8452.9 | 3389.5 | 78.2 |
| 277 | 166.3 | 154.9 | 175.4 | 443.3 | 274.9 | 287.7 | 236.4 | 171 |
| 278 | 409.1 | 57.7 | 133.6 | 461.1 | 1337.1 | 1922.6 | 1313.9 | 21.4 |
| 279 | 3251.5 | 3522.3 | 3542.3 | 82.4 | 1789.5 | 2134.1 | 1300.8 | 1253.5 |
| 280 | 3966.4 | 3899.2 | 3833.5 | 208.1 | 1888.5 | 3042.7 | 1982.8 | 1794.8 |
| 281 | 19.5 | 547.7 | 210.9 | 28.9 | 707.3 | 438.3 | 349.4 | 158 |
| 282 | 204.3 | 211.2 | 288.1 | 298.4 | 427.8 | 422.4 | 236.1 | 272.7 |
| 283 | 238.5 | 204.2 | 254 | 221.9 | 351.9 | 341.6 | 284.8 | 456.3 |
| 284 | 74.4 | 60.6 | 95.4 | 58.4 | 2258.3 | 84.1 | 82.5 | 93.7 |
| 285 | 116.8 | 93.3 | 101.5 | 95.5 | 93.9 | 98.8 | 108.7 | 120.7 |
| 286 | 151.8 | 621.9 | 136.9 | 73 | 31 | 112.7 | 64.6 | 214.1 |
| 287 | 106.5 | 73.4 | 112.1 | 121 | 113.4 | 88.9 | 92.4 | 149.7 |
| 288 | 165.7 | 147.9 | 139.4 | 188.3 | 157.7 | 159.2 | 153.4 | 210.6 |
| 289 | 361.8 | 386.8 | 175.9 | 1115.3 | 4422.6 | 4190.9 | 1131.9 | 143.3 |
| 291 | 48.4 | 122.1 | 79.7 | 80.2 | 146.2 | 305.5 | 248.8 | 57.2 |
| 292 | 105.8 | 222.9 | 15.8 | 2034.2 | 2474.9 | 1817.6 | 494.7 | 16.9 |
| 293 | 79.2 | 522.4 | 59 | 729.5 | 1441.8 | 926 | 922.7 | 76.1 |
| 295 | 337.6 | 458.1 | 827.4 | 131.1 | 2369 | 2332.1 | 1528.5 | 122.4 |
| 296 | 383.8 | 625.7 | 393.8 | 207.2 | 1441.2 | 1567.1 | 2234 | 1401.5 |
| 297 | 53 | 59 | 28.1 | 437.4 | 57.3 | 124.9 | 58.8 | 72.5 |
| 298 | 391.9 | 287.2 | 290.1 | 297.6 | 584.3 | 544.1 | 371.3 | 310.6 |
| 299 | 923.9 | 1489.6 | 612.9 | 234.9 | 1909.5 | 2964.3 | 2109.9 | 599.9 |
| 300 | 418.5 | 788.5 | 191.3 | 336 | 1170.1 | 795.1 | 1388 | 351.3 |
| 301 | 991 | 481.9 | 1125.5 | 359.4 | 219.4 | 308.6 | 234.6 | 849 |
| 302 | 23.5 | 10.4 | 20.2 | 207 | 135.3 | 153.1 | 376.5 | 9.2 |
| 303 | 1127.8 | 805.7 | 1035.8 | 76.4 | 260.7 | 316 | 336 | 830.1 |
| 304 | 168.9 | 328.4 | 141.9 | 2398 | 458.1 | 665 | 1628.3 | 347.3 |
| 305 | 615.6 | 310.9 | 1835.6 | 139.9 | 1686.4 | 577.1 | 314.2 | 187.1 |
| 306 | 157 | 151.3 | 142.3 | 1385.7 | 410.9 | 361.9 | 901.3 | 131.6 |
| 307 | 130.6 | 104.8 | 192.1 | 1073.9 | 343.2 | 232.4 | 160.4 | 141.2 |
| 308 | 2650.8 | 1762.2 | 3182.2 | 208.5 | 559.7 | 737.4 | 291.1 | 2599.7 |
| 310 | 283.4 | 464.8 | 253.6 | 85.6 | 100.7 | 156.8 | 136.2 | 1022.4 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 311 | 179.3 | 173.4 | 57.1 | 111.3 | 657.6 | 321.6 | 401.6 | 52.1 |
| 312 | 292.8 | 760.1 | 295.9 | 36.9 | 34.2 | 289.2 | 131.1 | 2976.1 |
| 313 | 430.3 | 1430.7 | 249 | 92.5 | 552.3 | 277.7 | 267 | 157.2 |
| 314 | 226.3 | 371.8 | 186.9 | 342.8 | 2406.4 | 1007.7 | 840.8 | 106.5 |
| 315 | 309.4 | 351.9 | 325.9 | 30.1 | 395.5 | 278.5 | 274.6 | 535.1 |
| 316 | 209.5 | 432.1 | 259.5 | 113.1 | 414.1 | 508.4 | 795.4 | 296.6 |
| 317 | 144.3 | 130.5 | 141.8 | 227.4 | 261.4 | 240 | 204 | 160.6 |
| 318 | 107.5 | 220.6 | 62.4 | 340.5 | 222.2 | 300.4 | 303.5 | 33.3 |
| 319 | 498.2 | 410.5 | 297.8 | 132.9 | 340.8 | 346.8 | 430.1 | 820.9 |
| 320 | 581.2 | 637.7 | 414.9 | 113.7 | 262.1 | 263.9 | 280.4 | 504.6 |
| 321 | 733.9 | 546.4 | 690.4 | 441.5 | 201.4 | 365.2 | 236.3 | 491.1 |
| 322 | 217.9 | 299 | 263.9 | 192.8 | 327.7 | 307.9 | 337.2 | 518.8 |
| 323 | 3187.2 | 4533.5 | 2223.1 | 758 | 4003.9 | 2801.5 | 1477.2 | 1113.1 |
| 324 | 69.4 | 506.8 | 68.6 | 123.4 | 493.6 | 420.7 | 324.7 | 47.1 |
| 325 | 450.9 | 358.4 | 218.3 | 215.6 | 509.5 | 446.4 | 622 | 185.7 |
| 326 | 2420.8 | 2944.2 | 761.2 | 377.6 | 2798.7 | 2211.2 | 3775.5 | 127.9 |
| 327 | 67.4 | 1.1 | 3.5 | 294.9 | 23.8 | 104.1 | 48.9 | 1.1 |
| 328 | 488.9 | 519.1 | 682.6 | 28.9 | 478.5 | 335.1 | 132.2 | 219.6 |
| 329 | 22.8 | 62 | 67.7 | 356.2 | 65 | 51.2 | 64.3 | 179.2 |
| 330 | 40.2 | 11.3 | 38.3 | 50 | 722.3 | 567.2 | 650.1 | 45.5 |
| 331 | 273.9 | 221.2 | 263.6 | 301.3 | 394.4 | 336.3 | 281.2 | 667.1 |
| 333 | 901.4 | 799.4 | 1778.7 | 71.1 | 251.5 | 317.3 | 197.7 | 472.4 |
| 334 | 142.6 | 391.3 | 161.2 | 132.6 | 683.2 | 297.3 | 816.3 | 123.4 |
| 335 | 92.1 | 83.6 | 139.7 | 122.3 | 635.2 | 109.4 | 93 | 650.1 |
| 336 | 415.5 | 361.2 | 472.6 | 55.1 | 215.3 | 220 | 144.3 | 242.6 |
| 337 | 248.1 | 1107.3 | 362.9 | 456.2 | 546.9 | 381.8 | 2046.3 | 137 |
| 338 | 116.6 | 210.1 | 98.7 | 249.2 | 759.2 | 1019.7 | 678.9 | 129.6 |
| 339 | 410.3 | 355.2 | 381.9 | 207.7 | 358.5 | 303.8 | 255.2 | 422.3 |
| 340 | 222.1 | 232.5 | 256.4 | 303.1 | 602.9 | 95894.7 | 123847.4 | 268.6 |
| 341 | 340.4 | 368.3 | 779 | 229.7 | 428.7 | 419.5 | 322 | 798.8 |
| 343 | 351.3 | 283.2 | 67.9 | 210.7 | 808.4 | 2720.6 | 7228.2 | 3446.2 |
| 344 | 1130.7 | 1272.9 | 909.5 | 155.6 | 531.7 | 706.2 | 595.7 | 1725.9 |
| 345 | 214.4 | 187.9 | 216.5 | 326.2 | 289.7 | 349.2 | 223.2 | 308.6 |
| 347 | 780 | 4322.6 | 1880.3 | 3519.1 | 4904.6 | 2435.8 | 1042.8 | 1057 |
| 348 | 2033.1 | 8452.1 | 3722.6 | 8444.3 | 8098.7 | 3669.4 | 2324.6 | 2993.4 |
| 349 | 414.1 | 298.8 | 450 | 384.7 | 420.6 | 314.2 | 331.6 | 323.1 |
| 350 | 193.6 | 155.1 | 166.7 | 164.9 | 603.4 | 717.3 | 1008.9 | 102.4 |

(continued)

| 3j | | | | | | | | |
|---|---|---|---|---|---|---|---|---|
| SEQ ID NO: | 938TT | 940TT | 941TT | 945TT | 946TT | 947TT | 948TT | 954TT |
| 351 | 149.8 | 87.7 | 51.7 | 177.8 | 1787.4 | 975.6 | 863.9 | 113 |
| 352 | 305.1 | 222.3 | 426.3 | 134.8 | 158.9 | 214.2 | 127.4 | 1132 |
| 353 | 228.2 | 245.9 | 178.8 | 150.1 | 197.2 | 301.1 | 428.1 | 413 |

| 3j | | | | | | | |
|---|---|---|---|---|---|---|---|
| SEQ ID NO: | 955TT | 956TT | 957TT | 959TT | 960TT | 961TT | 962TT |
| 354 | 87.9 | 200.9 | 8.7 | 55.2 | 27.7 | 427 | 68.1 |
| 355 | 94.6 | 90.3 | 49.7 | 131.5 | 43.1 | 452.4 | 87.9 |
| 356 | 581.7 | 511.3 | 566.1 | 708.3 | 716.3 | 1212.4 | 709.2 |
| 357 | 586.6 | 1247.1 | 595.1 | 664.9 | 783 | 891.1 | 787.2 |
| 358 | 714.8 | 1339.8 | 621 | 733.9 | 978.3 | 526.4 | 1369.9 |
| 359 | 451.3 | 315.7 | 386.8 | 424.9 | 420.5 | 309.2 | 381.1 |
| 362 | 290 | 512.7 | 459.8 | 835.1 | 394.4 | 252.8 | 419.6 |
| 360 | 221.5 | 346.6 | 132.9 | 201.9 | 228.2 | 220.9 | 348.6 |
| 361 | 182.4 | 412 | 1902 | 403.3 | 846.8 | 964.3 | 1117.4 |
| 363 | 416.2 | 327.3 | 406.2 | 502.2 | 548.9 | 506.1 | 522 |
| 1 | 675.4 | 869.8 | 676.6 | 267.5 | 452.3 | 717.4 | 496.3 |
| 2 | 1844.7 | 3591.1 | 3028.1 | 3641.6 | 3980.3 | 3803.6 | 3593.4 |
| 7 | 309.8 | 581.3 | 550.1 | 630.4 | 1040.2 | 1288.9 | 1720.8 |
| 8 | 1337.3 | 1229.5 | 1489.8 | 865.2 | 743.2 | 1080.8 | 1023.5 |
| 9 | 2938.3 | 3522 | 7292.5 | 1627.8 | 13667.8 | 4833.1 | 816.2 |
| 10 | 58.5 | 57.9 | 102.2 | 97.4 | 77.9 | 90.8 | 91 |
| 11 | 237.4 | 291.8 | 198.6 | 338.4 | 478.9 | 402.4 | 127 |
| 13 | 245.4 | 217.8 | 83.9 | 97 | 110.5 | 141.1 | 325.4 |
| 14 | 1390 | 1022.1 | 201.2 | 221.1 | 188.4 | 343.3 | 1201.7 |
| 15 | 92.3 | 394.2 | 192.7 | 775.3 | 322.5 | 337.5 | 375.9 |
| 16 | 1190 | 2000.9 | 686.2 | 1137.7 | 815.9 | 1366.2 | 1403.6 |
| 17 | 298.5 | 665.4 | 989 | 1727.8 | 1644 | 1382.7 | 773.2 |
| 19 | 176.4 | 249.5 | 161.8 | 245.1 | 209.8 | 154.9 | 170.7 |
| 20 | 1.1 | 58.3 | 1.4 | 133.8 | 1.1 | 2.2 | 1.1 |
| 21 | 354 | 1554.7 | 106.9 | 1.3 | 129.6 | 94.6 | 88.3 |
| 22 | 1772.4 | 3116.7 | 5851.7 | 6314.1 | 10192.9 | 7077.5 | 2802.8 |
| 23 | 44.4 | 809.8 | 34.3 | 489.7 | 33.2 | 163 | 1.1 |
| 24 | 86.3 | 453.2 | 147.6 | 353 | 90.7 | 121.5 | 117.5 |
| 26 | 645.4 | 2180.5 | 47.8 | 1478.1 | 169.6 | 380.5 | 74.7 |

(continued)

| 3j | | | | | | | |
|---|---|---|---|---|---|---|---|
| SEQ ID NO: | 955TT | 956TT | 957TT | 959TT | 960TT | 961TT | 962TT |
| 27 | 278.1 | 1212.1 | 159.1 | 236.2 | 335.3 | 339.4 | 261.4 |
| 29 | 78.4 | 81.9 | 23.5 | 82.1 | 53.5 | 68.7 | 59.6 |
| 30 | 71.2 | 234.9 | 129.8 | 239.5 | 51.6 | 114 | 15.5 |
| 31 | 294.9 | 587.2 | 48.2 | 138.9 | 86 | 174.5 | 43.6 |
| 33 | 162.4 | 998.1 | 428.5 | 1117.4 | 279.9 | 251.3 | 313.6 |
| 34 | 3053 | 2498.1 | 3095.7 | 2119.5 | 3047.9 | 2373.9 | 1094.7 |
| 35 | 181.9 | 491.2 | 74 | 441.4 | 22.7 | 965.8 | 17 |
| 36 | 127.6 | 209.5 | 637.3 | 2400.3 | 139.2 | 157.6 | 1485.1 |
| 37 | 166.9 | 139.5 | 190.8 | 131.7 | 237.7 | 198.7 | 233.9 |
| 38 | 130.1 | 678 | 413.5 | 1057.4 | 365.3 | 316.8 | 662.7 |
| 40 | 257 | 450.6 | 6557.8 | 482.9 | 628.5 | 522.6 | 591.2 |
| 41 | 134.2 | 455.4 | 123.4 | 163.6 | 368.6 | 605.8 | 254.3 |
| 42 | 257.3 | 576.9 | 200.4 | 398.3 | 154.3 | 212 | 244.1 |
| 43 | 446 | 239.2 | 748.7 | 367 | 323.5 | 371.3 | 664.8 |
| 44 | 546.1 | 682.4 | 841.1 | 846.6 | 457.9 | 413.3 | 620.1 |
| 45 | 1085.8 | 897.1 | 601.8 | 484.8 | 436.6 | 816.9 | 845.3 |
| 46 | 294.3 | 214.6 | 267 | 376.1 | 303.2 | 283 | 320 |
| 47 | 96.5 | 206 | 140.7 | 192.8 | 168.4 | 247 | 213.3 |
| 48 | 18 | 313.8 | 21.5 | 378.3 | 15 | 41.1 | 1.5 |
| 49 | 303.8 | 472.1 | 236.5 | 221.8 | 366.2 | 411.7 | 363.7 |
| 50 | 78.2 | 293.2 | 108 | 497.3 | 147.7 | 127.2 | 66.9 |
| 51 | 342.3 | 124.4 | 234.4 | 45.5 | 54.4 | 36.3 | 156.3 |
| 52 | 194.3 | 158.4 | 311.6 | 182.3 | 216.4 | 212.3 | 171.6 |
| 53 | 153.6 | 347.3 | 19 | 83.6 | 112.1 | 196.9 | 251.8 |
| 54 | 1255 | 1165.2 | 316.5 | 359.8 | 277.1 | 598.1 | 1248 |
| 55 | 116.8 | 117.3 | 120.3 | 89.7 | 118.2 | 109.3 | 88.7 |
| 56 | 219.8 | 182.9 | 88.1 | 65.6 | 49.5 | 53.3 | 172.8 |
| 57 | 257.9 | 611.8 | 345 | 831.2 | 168 | 1240.7 | 159.2 |
| 58 | 242.6 | 710 | 490.1 | 2548.4 | 205.5 | 1436.5 | 82.1 |
| 59 | 1047.2 | 2248.5 | 1125 | 813.5 | 918.1 | 1999 | 1120.9 |
| 60 | 97 | 300.2 | 154 | 85.5 | 160.5 | 117.3 | 185.6 |
| 61 | 90.4 | 103.2 | 161.9 | 145.1 | 203.2 | 134.9 | 108.2 |
| 62 | 300.7 | 1868.2 | 953.7 | 574.1 | 642.8 | 1633.8 | 139 |
| 63 | 297 | 536.7 | 193.6 | 228.4 | 311.9 | 261.2 | 255.9 |
| 64 | 109.8 | 100.1 | 170.4 | 227.4 | 176.6 | 190.7 | 115.1 |
| 65 | 87.5 | 158.6 | 150.9 | 81.8 | 71.6 | 67.2 | 102 |
| 66 | 1355.7 | 951.2 | 564.5 | 797.6 | 309.4 | 509.5 | 1530.7 |

(continued)

| 3j | | | | | | | |
|---|---|---|---|---|---|---|---|
| SEQ ID NO: | 955TT | 956TT | 957TT | 959TT | 960TT | 961TT | 962TT |
| 67 | 34223.4 | 31838 | 24742.4 | 36682.2 | 36457.8 | 32604.1 | 43684.2 |
| 68 | 3348.2 | 1692.5 | 1286.8 | 3946 | 5683.4 | 2624.1 | 1368.7 |
| 69 | 7947.5 | 4162 | 3970 | 11781.2 | 10279.8 | 7055 | 5076.1 |
| 70 | 4104.3 | 2568.6 | 4964 | 2477.4 | 881.6 | 933.2 | 2792.2 |
| 71 | 619.9 | 681.8 | 113.5 | 372.3 | 137.1 | 72 | 319.9 |
| 72 | 229.3 | 609.4 | 1269.4 | 639.7 | 229.4 | 694.7 | 159.4 |
| 73 | 260.4 | 365.9 | 207.3 | 238.1 | 275.9 | 264.9 | 416.7 |
| 75 | 149.7 | 302.2 | 171.8 | 538.4 | 453.7 | 249.2 | 115.4 |
| 76 | 2162.3 | 1321.7 | 189 | 2328.1 | 12.2 | 54.4 | 1010.4 |
| 77 | 5244.7 | 2705.9 | 4944.6 | 4760.4 | 4771.9 | 3213.4 | 3514.8 |
| 78 | 310.5 | 589.3 | 1329.2 | 1771 | 866.2 | 805.1 | 952.5 |
| 79 | 231.8 | 135.6 | 383 | 134.3 | 114.6 | 119.6 | 116.4 |
| 80 | 667.1 | 355.6 | 404.3 | 450.5 | 85 | 239.4 | 268.7 |
| 81 | 1253.3 | 1566.6 | 84.2 | 100.9 | 194.3 | 384.9 | 263.5 |
| 82 | 333.1 | 228.4 | 350.7 | 215.3 | 247.9 | 520.6 | 252.5 |
| 84 | 147 | 325.5 | 277 | 141.8 | 114.9 | 106 | 153.2 |
| 85 | 163.8 | 102.1 | 111.6 | 119.6 | 149.3 | 210.2 | 180.5 |
| 86 | 193.8 | 55.9 | 95.3 | 298.1 | 298.3 | 70.2 | 71.1 |
| 87 | 156.9 | 158.6 | 353.7 | 164.2 | 293.6 | 482.8 | 160.9 |
| 88 | 3325 | 4358.2 | 4541.7 | 6014.1 | 7677.1 | 10815.9 | 5006 |
| 89 | 140.5 | 120.8 | 185.6 | 259 | 178.4 | 199.4 | 228.6 |
| 90 | 68.4 | 170.5 | 91.9 | 205.9 | 87.5 | 144.7 | 114 |
| 91 | 249.8 | 383.1 | 110.8 | 1269.1 | 70.9 | 1429.6 | 73.4 |
| 92 | 4953.4 | 6959.3 | 1774.3 | 2917.7 | 2117.8 | 7949.3 | 7560.6 |
| 93 | 201 | 209.6 | 400.5 | 170.5 | 283.1 | 293.5 | 163.4 |
| 94 | 260.4 | 3158.7 | 52.2 | 867.5 | 660.8 | 606.6 | 836.7 |
| 95 | 593.4 | 622.7 | 577.7 | 104.7 | 388.1 | 646.7 | 1203.2 |
| 96 | 165.4 | 233.3 | 200.5 | 123.7 | 184.7 | 171 | 372 |
| 97 | 273.3 | 336.6 | 287.9 | 296.3 | 132.3 | 317 | 370.1 |
| 98 | 70.8 | 41.8 | 25.4 | 16.5 | 25.6 | 32.7 | 30 |
| 99 | 164.8 | 189.4 | 116 | 167.2 | 280.8 | 245.9 | 257.1 |
| 100 | 610.8 | 12721.1 | 16769.4 | 9378.2 | 4271.4 | 15550.1 | 1798.6 |
| 101 | 232.4 | 238.6 | 434.5 | 383.4 | 434.1 | 504.6 | 729.3 |
| 102 | 147.5 | 132.7 | 112.2 | 180.7 | 209.4 | 215.6 | 169.1 |
| 103 | 309.3 | 255.1 | 195.4 | 418.3 | 293.4 | 404.3 | 381.3 |
| 104 | 631.5 | 573.5 | 522.3 | 415.2 | 370.9 | 830.3 | 589 |
| 105 | 655.3 | 488.2 | 398.8 | 564 | 421.8 | 540.9 | 516.4 |

(continued)

| 3j | | | | | | | |
|---|---|---|---|---|---|---|---|
| SEQ ID NO: | 955TT | 956TT | 957TT | 959TT | 960TT | 961TT | 962TT |
| 106 | 562.5 | 491 | 440.4 | 361 | 470.2 | 736.1 | 469 |
| 107 | 170.3 | 157.5 | 207.8 | 405.5 | 147.6 | 558.5 | 118.6 |
| 108 | 260.5 | 430.5 | 385.5 | 555.3 | 419 | 273.4 | 323.9 |
| 109 | 6189.1 | 2697.1 | 42.6 | 65.5 | 78.8 | 577.2 | 6878.3 |
| 110 | 10703.6 | 3965.4 | 1.2 | 28.1 | 19.7 | 1384.5 | 13039 |
| 111 | 1743.4 | 1684.3 | 944.4 | 1390.4 | 2754.7 | 1147.9 | 3105.8 |
| 112 | 823.1 | 805.8 | 41.7 | 109.8 | 237.7 | 59.8 | 620.1 |
| 113 | 246 | 445 | 478 | 246 | 90.9 | 253.2 | 214.1 |
| 114 | 239.9 | 397.1 | 452.5 | 688.8 | 1076.1 | 566 | 830.3 |
| 115 | 441.3 | 600.8 | 435.6 | 627.2 | 408 | 348.9 | 523.2 |
| 116 | 223.5 | 218.9 | 200.8 | 251.8 | 236.2 | 182.6 | 216.1 |
| 117 | 413 | 582.6 | 4938.9 | 5309.2 | 2714.3 | 3839.8 | 902.6 |
| 118 | 203.1 | 268.2 | 141 | 148.8 | 118.4 | 34.2 | 105.3 |
| 119 | 45.1 | 322.8 | 110.9 | 119.5 | 75.9 | 246.3 | 233 |
| 120 | 63.4 | 106.6 | 118.6 | 184.1 | 122.3 | 82.1 | 114.7 |
| 121 | 461.9 | 1324 | 1289.4 | 211.1 | 422.1 | 737.7 | 749.7 |
| 122 | 243.1 | 213.9 | 52.5 | 140.8 | 84.5 | 274.4 | 144.3 |
| 123 | 189.9 | 195.8 | 480.9 | 135.7 | 146.6 | 163.4 | 216.1 |
| 124 | 130.1 | 117 | 258.6 | 193.4 | 182.9 | 170.1 | 178 |
| 125 | 984.3 | 573.5 | 539.9 | 1065.4 | 967.5 | 1025.7 | 1172 |
| 126 | 1322.2 | 965.1 | 1360 | 738.2 | 631.3 | 1011.4 | 682.8 |
| 127 | 131.7 | 96.4 | 132 | 117.3 | 113.4 | 116.7 | 127.7 |
| 128 | 194.1 | 182.2 | 236.4 | 276.3 | 285.9 | 310.1 | 367.6 |
| 129 | 130.1 | 229.7 | 252.6 | 224.8 | 171.9 | 522.6 | 200.2 |
| 130 | 185.4 | 150.7 | 162.7 | 99 | 93.7 | 89.6 | 207.8 |
| 131 | 95.8 | 152.7 | 148.3 | 47.3 | 74.7 | 96.5 | 94.2 |
| 132 | 163.2 | 168.1 | 192.6 | 193.3 | 258.1 | 217.7 | 272.4 |
| 133 | 204.5 | 249.2 | 84.8 | 160.3 | 70.8 | 65.2 | 75.8 |
| 134 | 287.4 | 159.6 | 98.4 | 197.6 | 118.4 | 129.9 | 184.5 |
| 135 | 373.8 | 551.6 | 416.6 | 392.1 | 459.3 | 328.2 | 406.9 |
| 138 | 137.1 | 115.6 | 126.5 | 195 | 133.1 | 149.9 | 117.4 |
| 139 | 116.4 | 142.3 | 79.7 | 71.6 | 80.2 | 87.7 | 77.6 |
| 140 | 74.5 | 3607.6 | 2148.2 | 1275.7 | 3106.6 | 2235.3 | 1653.3 |
| 141 | 1566.1 | 2429.4 | 761.5 | 1182 | 2077.2 | 1681.8 | 1605.6 |
| 144 | 12425.9 | 7539.3 | 395.2 | 417 | 450.5 | 512.8 | 7238.7 |
| 145 | 160.5 | 137.2 | 206.9 | 220 | 255.1 | 198.3 | 239.2 |
| 146 | 182.1 | 147.4 | 169.7 | 202.3 | 262.8 | 225.5 | 211.9 |

(continued)

| 3j | | | | | | | |
|---|---|---|---|---|---|---|---|
| SEQ ID NO: | 955TT | 956TT | 957TT | 959TT | 960TT | 961TT | 962TT |
| 147 | 28.6 | 43.9 | 91 | 66.4 | 26.5 | 59.8 | 20 |
| 148 | 205.3 | 553.1 | 180.2 | 198 | 270.3 | 668.6 | 306.4 |
| 149 | 325 | 326.5 | 236.6 | 362.1 | 407.1 | 765.1 | 342.1 |
| 150 | 197.1 | 234.6 | 172.3 | 171.9 | 270.8 | 246.9 | 159.2 |
| 151 | 123.1 | 264.2 | 95.7 | 127.8 | 158.8 | 148.1 | 257.2 |
| 153 | 103.8 | 118.2 | 112.8 | 119.6 | 110.6 | 115.4 | 113.9 |
| 154 | 304.4 | 1676.6 | 2431.9 | 408.8 | 152.5 | 2641.1 | 142.4 |
| 155 | 498.7 | 158.7 | 136.4 | 138 | 136.6 | 144.9 | 139.3 |
| 156 | 95.4 | 89.8 | 63.6 | 51.8 | 74.1 | 120.8 | 65.3 |
| 157 | 178 | 115.2 | 155.5 | 132.3 | 138.2 | 159.9 | 181.3 |
| 158 | 283.4 | 1174.2 | 19.8 | 16.2 | 1.2 | 13.6 | 578.5 |
| 159 | 1776.8 | 2641.6 | 1398.3 | 1601.9 | 3134.9 | 3315.7 | 3396 |
| 161 | 9817.8 | 2623.3 | 177.1 | 129.7 | 6.7 | 152.9 | 1887.4 |
| 162 | 45.7 | 101.6 | 27.6 | 150.8 | 40.3 | 76.2 | 46.9 |
| 164 | 78.1 | 707 | 463.5 | 110.3 | 193.8 | 333.7 | 3770.9 |
| 166 | 6384 | 2992.4 | 4919.5 | 5671.4 | 6028.1 | 6429.2 | 3769.1 |
| 167 | 1191.3 | 1999.3 | 1864 | 686.4 | 1834.7 | 2207.6 | 2362.6 |
| 168 | 1167.3 | 843.3 | 2714.3 | 1862.4 | 1004 | 922.5 | 1487.6 |
| 169 | 3437.7 | 4365.5 | 1880.1 | 3159.8 | 1921.4 | 5836 | 1307.9 |
| 170 | 1488.1 | 2441.6 | 1019.6 | 1307.1 | 1150.4 | 2496.9 | 1953.8 |
| 171 | 1.2 | 722.2 | 1.2 | 760.4 | 1.2 | 725.9 | 1.2 |
| 172 | 590.7 | 573.3 | 719.5 | 631.6 | 816.5 | 666.4 | 904.9 |
| 173 | 408.8 | 355.1 | 171 | 213.4 | 406.9 | 481.6 | 564.2 |
| 176 | 1175.5 | 1461.1 | 175.7 | 8.4 | 704.9 | 850.3 | 2537.2 |
| 177 | 536.9 | 672.1 | 597.5 | 376.7 | 544 | 817.5 | 635.8 |
| 178 | 385.8 | 730.5 | 91.3 | 209.5 | 184.7 | 233 | 1073.8 |
| 179 | 74.1 | 84.7 | 60.3 | 70.3 | 71.4 | 127.1 | 145.7 |
| 180 | 156.8 | 498.8 | 119.5 | 132.1 | 72.9 | 109.4 | 609.9 |
| 181 | 8682 | 7928.2 | 10784.9 | 10442 | 14140.4 | 12487.9 | 12014.6 |
| 182 | 323.1 | 400.6 | 375.2 | 349.8 | 535.4 | 405.4 | 334.3 |
| 183 | 128.7 | 461.7 | 1.2 | 388.5 | 15.5 | 53.1 | 1.3 |
| 184 | 279.4 | 565.1 | 227.8 | 327.3 | 211.1 | 272.9 | 2642.9 |
| 185 | 1142.8 | 848.2 | 1463.7 | 1098.5 | 1043 | 1044.1 | 911.4 |
| 186 | 444.5 | 436.7 | 360.3 | 324.6 | 419 | 403 | 314.3 |
| 187 | 214.8 | 123.4 | 366.1 | 170.7 | 79.5 | 90.4 | 76.6 |
| 188 | 94.7 | 141.3 | 105.2 | 357.2 | 157 | 110 | 170.5 |
| 189 | 49.2 | 74.3 | 80 | 146.6 | 60.9 | 66.5 | 88 |

(continued)

| 3j | | | | | | | |
|---|---|---|---|---|---|---|---|
| SEQ ID NO: | 955TT | 956TT | 957TT | 959TT | 960TT | 961TT | 962TT |
| 190 | 267.8 | 422 | 71.2 | 95.9 | 85.5 | 102.1 | 152.5 |
| 191 | 825.2 | 845.3 | 1874.8 | 739.8 | 734.8 | 1020.6 | 520.6 |
| 192 | 67.8 | 308.7 | 77.4 | 94.4 | 80.7 | 77.4 | 79.8 |
| 193 | 695 | 1573.5 | 2485.3 | 1849.3 | 2885.4 | 3460.8 | 1615.5 |
| 194 | 173.6 | 299.7 | 106.6 | 134.3 | 150.7 | 122.6 | 175.9 |
| 195 | 248.2 | 343.9 | 371.7 | 347.9 | 320.2 | 194.4 | 297.7 |
| 196 | 7207.4 | 7285.3 | 5142.4 | 2474.2 | 6626.6 | 8775.3 | 11352.4 |
| 197 | 50.6 | 93.6 | 113.8 | 142.3 | 96.4 | 86.5 | 58 |
| 198 | 56.8 | 44.6 | 36.9 | 51.1 | 66.8 | 47.9 | 128.9 |
| 199 | 135.2 | 104.6 | 57.8 | 204.7 | 35.2 | 599.8 | 103.1 |
| 200 | 110.8 | 310.7 | 104.4 | 327.5 | 121.9 | 121.6 | 147.5 |
| 201 | 125.9 | 200.1 | 46.9 | 69.2 | 25.3 | 505.3 | 54.7 |
| 203 | 163.6 | 319 | 130.2 | 85 | 252 | 312.1 | 621.1 |
| 204 | 826.2 | 879.7 | 567.6 | 726.8 | 704.1 | 434 | 980.1 |
| 205 | 4963.1 | 7695 | 2785.7 | 6249.9 | 6069.1 | 6172.2 | 6469.8 |
| 206 | 384 | 1284 | 196.8 | 1397.4 | 323.3 | 282.2 | 224.9 |
| 207 | 1180.7 | 1623.7 | 402.1 | 2190.2 | 453.5 | 1652.9 | 1647 |
| 208 | 495.8 | 653.7 | 150.2 | 227.7 | 257 | 340.5 | 253.6 |
| 209 | 1608.4 | 2981.5 | 2844.2 | 445.8 | 5286.9 | 4175.6 | 6653.1 |
| 211 | 7731.4 | 15392.6 | 3699.2 | 33617.1 | 23929.6 | 28201.9 | 37299.8 |
| 212 | 911.5 | 692 | 1485 | 440.7 | 207.7 | 396.6 | 971.4 |
| 213 | 58.3 | 77.2 | 150.2 | 91.5 | 119.6 | 83.2 | 84.4 |
| 215 | 1364.9 | 1835.2 | 477.2 | 1858.8 | 394.4 | 2522.1 | 2910.2 |
| 216 | 540.7 | 636.6 | 665 | 441.7 | 610.8 | 635.7 | 494.3 |
| 217 | 196.1 | 494.8 | 258 | 300.8 | 260 | 97.9 | 424.1 |
| 218 | 359.4 | 357 | 308.6 | 572.5 | 327.9 | 346.3 | 318.6 |
| 220 | 87.6 | 66.5 | 71.4 | 89.9 | 106 | 86.7 | 88.5 |
| 221 | 195.8 | 787.9 | 397 | 518.1 | 651.9 | 372.5 | 497.7 |
| 223 | 248.3 | 279.5 | 456 | 247.2 | 226.7 | 308.9 | 202.1 |
| 227 | 326.2 | 1074.9 | 579.8 | 1616.2 | 517.9 | 516.4 | 447.3 |
| 228 | 377.5 | 502.7 | 583.4 | 486.6 | 764.6 | 521.7 | 871.2 |
| 229 | 311.2 | 113.4 | 1212.9 | 377.4 | 152.7 | 93.4 | 149 |
| 230 | 417.2 | 297.5 | 120.9 | 131.8 | 135.8 | 242.5 | 285.4 |
| 231 | 108.5 | 189.8 | 88.2 | 67.4 | 140.1 | 160.7 | 309.4 |
| 232 | 465.5 | 1573.3 | 41.4 | 973.5 | 118.3 | 273.3 | 78.3 |
| 233 | 728.9 | 3634.1 | 7030.2 | 1396.1 | 3819.1 | 5175.1 | 2235.4 |
| 235 | 37.3 | 474.7 | 88.2 | 526 | 120.4 | 662.1 | 42.3 |

(continued)

| 3j | | | | | | | |
|---|---|---|---|---|---|---|---|
| SEQ ID NO: | 955TT | 956TT | 957TT | 959TT | 960TT | 961TT | 962TT |
| 236 | 199.7 | 143.3 | 108.3 | 196.9 | 220 | 171.3 | 142.5 |
| 237 | 1614.8 | 1725.3 | 2189.4 | 1342.6 | 2031.3 | 1672.9 | 1475 |
| 238 | 823.6 | 1424 | 1041.5 | 1173.3 | 691.2 | 1142.2 | 1169.5 |
| 239 | 56.1 | 641.7 | 183 | 140.5 | 95 | 88 | 683.1 |
| 240 | 98.3 | 853.8 | 155.4 | 330.5 | 155.1 | 106.4 | 574.8 |
| 241 | 84.4 | 1914.8 | 289.8 | 519.5 | 207.1 | 95 | 1367 |
| 242 | 2194.8 | 989.6 | 2107.8 | 1991.2 | 1042.5 | 1474.6 | 688.1 |
| 243 | 89.9 | 263.3 | 247.9 | 268.6 | 679.7 | 287.2 | 89 |
| 244 | 209.3 | 288.3 | 89.9 | 209.1 | 146.5 | 131 | 131 |
| 246 | 1348 | 810.8 | 270.7 | 434.5 | 85.9 | 160.3 | 487.6 |
| 247 | 113.4 | 92.8 | 68 | 145.3 | 125.8 | 139.4 | 150.2 |
| 248 | 503.9 | 571.2 | 120.3 | 199.5 | 153.6 | 226.1 | 529.7 |
| 249 | 650 | 1958.3 | 945.4 | 442.8 | 746.9 | 674.2 | 1861.5 |
| 251 | 149.3 | 159.2 | 98.7 | 67 | 54.5 | 98.7 | 133.7 |
| 253 | 458.5 | 369.7 | 392.2 | 512.7 | 189.7 | 171.8 | 255.1 |
| 254 | 759.1 | 1048.2 | 56.3 | 201.5 | 144.1 | 322.5 | 1876.1 |
| 255 | 395 | 847.1 | 92.8 | 241.2 | 190.6 | 204 | 937.8 |
| 256 | 497.3 | 504.3 | 202.9 | 1243.9 | 138.2 | 1112.8 | 153.2 |
| 257 | 96.8 | 170.3 | 61.6 | 110.7 | 240.3 | 224.1 | 83.2 |
| 258 | 80.1 | 318.2 | 1.1 | 4 | 1.2 | 194.6 | 1.3 |
| 259 | 31.3 | 422.4 | 35.1 | 291.4 | 170 | 194.9 | 6.1 |
| 260 | 144.2 | 118.9 | 152.1 | 196.1 | 181.1 | 162.3 | 192.9 |
| 261 | 72 | 118.5 | 599.4 | 788.2 | 759.5 | 233.2 | 707.9 |
| 263 | 414.9 | 536.2 | 500.4 | 605.6 | 316.7 | 492.7 | 460.5 |
| 263 | 550.2 | 391.2 | 170.3 | 206.7 | 218 | 261.4 | 299 |
| 265 | 334.6 | 245.7 | 405.7 | 534.5 | 619.5 | 1119 | 574.8 |
| 266 | 546.7 | 433.1 | 317.8 | 513.8 | 421.9 | 266.6 | 397.7 |
| 256 | 69.3 | 168.9 | 137.2 | 94.2 | 115.1 | 101 | 97.3 |
| 268 | 570.4 | 855.7 | 334.9 | 287.8 | 801.2 | 1200.2 | 1032.6 |
| 269 | 17.8 | 25.5 | 51.2 | 32.6 | 33.8 | 37.9 | 56.8 |
| 270 | 313.9 | 169.7 | 378.4 | 245.5 | 183.8 | 220.5 | 267 |
| 271 | 197.8 | 175.4 | 205.9 | 317.3 | 297.9 | 247.6 | 263.9 |
| 272 | 537.8 | 303.4 | 368 | 416.8 | 596.6 | 461.6 | 589.2 |
| 273 | 2896.5 | 2422.4 | 2113.9 | 4788.3 | 2936.5 | 2726.7 | 6835.9 |
| 274 | 736 | 340.7 | 226.1 | 863.7 | 465 | 215.6 | 180.7 |
| 275 | 312 | 220.3 | 428 | 292.8 | 254 | 301.5 | 300 |
| 276 | 290.2 | 1528.1 | 727.2 | 2093.9 | 539.5 | 692.9 | 622.8 |

(continued)

| 3j | | | | | | | |
|---|---|---|---|---|---|---|---|
| SEQ ID NO: | 955TT | 956TT | 957TT | 959TT | 960TT | 961TT | 962TT |
| 277 | 171.5 | 177.3 | 190.4 | 200.2 | 228.4 | 201.6 | 245.6 |
| 278 | 57 | 615.1 | 90.6 | 495.4 | 201.9 | 350.4 | 20.5 |
| 279 | 2553.9 | 3673.6 | 1781.1 | 3550.4 | 3051.6 | 3161.9 | 3164.5 |
| 280 | 4112.4 | 4496.7 | 2693.5 | 6671.4 | 4029.9 | 3715.1 | 3402.8 |
| 281 | 420.9 | 478.9 | 381.7 | 99.3 | 24.7 | 76.5 | 829.5 |
| 282 | 284.1 | 237.5 | 220.4 | 467.3 | 243.1 | 183.4 | 280.3 |
| 283 | 320.7 | 247.3 | 262.2 | 267.1 | 257.1 | 287.9 | 255.5 |
| 284 | 86.1 | 60.6 | 66.9 | 82.5 | 68 | 91 | 102.6 |
| 285 | 74.5 | 84.5 | 78.8 | 98.8 | 132.4 | 97.7 | 89.8 |
| 286 | 140.3 | 125.8 | 856.5 | 479.2 | 1253 | 1123.8 | 337.3 |
| 287 | 82.7 | 68.3 | 104.9 | 131 | 118.4 | 100.2 | 124.1 |
| 288 | 112.6 | 131.8 | 109.3 | 132.3 | 203.1 | 166.3 | 157.2 |
| 289 | 329.1 | 523 | 74.6 | 1912.1 | 5 | 1194.8 | 79.2 |
| 291 | 72.9 | 135.9 | 147.6 | 116.3 | 111.7 | 106 | 91.2 |
| 292 | 61.3 | 181.2 | 49.9 | 155.8 | 43.9 | 982.9 | 77.4 |
| 293 | 210.2 | 267.7 | 100.5 | 70.6 | 87.4 | 91.2 | 418.7 |
| 295 | 880.3 | 560.6 | 520.9 | 266.4 | 534.9 | 307.3 | 261.4 |
| 296 | 1067.2 | 1234.6 | 688.7 | 782.9 | 718.7 | 604.5 | 724.8 |
| 297 | 47.9 | 64.8 | 51.5 | 87.2 | 38.6 | 69.1 | 58.3 |
| 298 | 551.5 | 344.5 | 386.7 | 324.7 | 241.8 | 269 | 394.7 |
| 299 | 1468.8 | 1771.8 | 787.5 | 1048 | 935.5 | 572.9 | 948.9 |
| 300 | 817.3 | 680.9 | 461.3 | 248.2 | 137.3 | 294.2 | 748.4 |
| 301 | 608.4 | 1624.7 | 1490.7 | 3382.6 | 4444 | 1930.7 | 1769 |
| 302 | 10.4 | 26.6 | 30.4 | 33.2 | 26.3 | 35.7 | 24.2 |
| 303 | 710.1 | 705.1 | 1706.6 | 859.3 | 577.5 | 788.3 | 425.1 |
| 304 | 95.7 | 466.8 | 190.4 | 187.2 | 124.8 | 202.8 | 143.5 |
| 305 | 157.1 | 529.4 | 493.4 | 795.6 | 163.1 | 69.8 | 249 |
| 306 | 131.8 | 108.5 | 180.6 | 143.8 | 154.4 | 173 | 206 |
| 307 | 191.4 | 207.2 | 84.5 | 181.7 | 133.4 | 205.1 | 178.9 |
| 308 | 2703.4 | 1485.8 | 1302 | 2184.2 | 756.3 | 1330.1 | 1074.7 |
| 310 | 549.2 | 417.5 | 612 | 811.3 | 723.1 | 576 | 409.8 |
| 311 | 145 | 132 | 126 | 159.1 | 101.9 | 148.1 | 113.9 |
| 312 | 1216.9 | 574.9 | 992.1 | 892.8 | 612.7 | 695.2 | 1340.7 |
| 313 | 307.7 | 190.1 | 158.9 | 201.8 | 115.6 | 112.7 | 1437.5 |
| 314 | 416.9 | 725.7 | 134.8 | 157 | 168.8 | 428.1 | 448.6 |
| 315 | 192.5 | 412.2 | 496 | 467.2 | 366.2 | 506.8 | 341.4 |
| 316 | 444 | 470.1 | 95.3 | 286 | 281.8 | 131.4 | 291.2 |

(continued)

| 3j | | | | | | | |
|---|---|---|---|---|---|---|---|
| SEQ ID NO: | 955TT | 956TT | 957TT | 959TT | 960TT | 961TT | 962TT |
| 317 | 157.9 | 142 | 150.3 | 197.9 | 191.9 | 177.2 | 201.1 |
| 318 | 140.5 | 334.7 | 56.9 | 29.1 | 73.1 | 87 | 78.1 |
| 319 | 706.9 | 280.3 | 127.7 | 267.6 | 70.7 | 164.5 | 199.4 |
| 320 | 535.8 | 590.3 | 887.9 | 568 | 592.8 | 742.2 | 577.9 |
| 321 | 340.3 | 326.5 | 372 | 793.8 | 460.1 | 414.9 | 375.2 |
| 322 | 352.2 | 258.3 | 185.4 | 237.2 | 248.6 | 152.6 | 280.9 |
| 323 | 2455.7 | 4246.4 | 3485.2 | 2786.2 | 4469.1 | 2754.2 | 4942.2 |
| 324 | 348.9 | 411 | 169.2 | 70 | 73 | 87.9 | 460 |
| 325 | 449.5 | 519.2 | 168 | 216.7 | 282.5 | 299.4 | 469.4 |
| 326 | 1603.6 | 3167 | 3571.4 | 404.9 | 4260.9 | 3099.9 | 1692.6 |
| 327 | 26.8 | 44 | 10.3 | 12.2 | 25.7 | 20.6 | 8.2 |
| 328 | 277.9 | 499.5 | 114.4 | 194 | 624.6 | 445.4 | 617.5 |
| 329 | 63.1 | 51.9 | 64 | 54.1 | 56.3 | 67.2 | 81.9 |
| 330 | 26.8 | 398.3 | 51.1 | 38.3 | 53.4 | 44.1 | 28.4 |
| 331 | 353.6 | 211.8 | 246.9 | 326.3 | 313.7 | 256.2 | 346.2 |
| 333 | 628.2 | 817.1 | 534.6 | 764.7 | 963.2 | 1039.8 | 993.5 |
| 334 | 129 | 131.6 | 121.3 | 127.9 | 171.5 | 135.9 | 210.2 |
| 335 | 61.5 | 83.3 | 276.3 | 550.9 | 108.1 | 127.9 | 116.4 |
| 336 | 877.3 | 835 | 262.7 | 397.8 | 457.2 | 330 | 598.7 |
| 337 | 119 | 244.5 | 349.6 | 426.7 | 114.8 | 364.1 | 225.7 |
| 338 | 213.7 | 307.5 | 152.1 | 190 | 160 | 264.1 | 213.2 |
| 339 | 337.9 | 298.3 | 253.4 | 278.6 | 421.1 | 490.3 | 476.5 |
| 340 | 161.4 | 161 | 248.4 | 316.2 | 277.8 | 273.6 | 290.1 |
| 341 | 575.9 | 645.8 | 726.6 | 782.5 | 1057.6 | 767.6 | 465.1 |
| 343 | 1203 | 450.3 | 740.9 | 103.8 | 137.1 | 178 | 105 |
| 344 | 1275 | 1111.4 | 1819.2 | 1393.5 | 1260.3 | 1521.1 | 1289.7 |
| 345 | 164.6 | 211.9 | 210.1 | 208.7 | 288.4 | 221.3 | 290.7 |
| 347 | 2603 | 3049.5 | 1135.9 | 1212.8 | 2982.6 | 3471.6 | 2440.3 |
| 348 | 4636 | 4968.1 | 2088.3 | 2517.8 | 4998.7 | 6143.9 | 5642.9 |
| 349 | 231.2 | 321.5 | 329.2 | 238.9 | 199 | 275.7 | 273.9 |
| 350 | 306.8 | 357 | 165.3 | 139.9 | 214.3 | 201.7 | 315.7 |
| 351 | 661.6 | 248.9 | 70.2 | 75.7 | 64.2 | 140 | 76.9 |
| 352 | 228.8 | 337.7 | 313.8 | 812.7 | 340.3 | 238.1 | 322 |
| 353 | 268 | 118.5 | 158.9 | 211.1 | 171 | 284.3 | 124.5 |

| 3k | | | | | | |
|---|---|---|---|---|---|---|
| SEQ ID NO: | 967TT | 968TT | 969TT | 970TT | 982TT | 983TT |
| 354 | 23.2 | 55.9 | 169.3 | 22.6 | 1.2 | 136.2 |
| 355 | 28.5 | 50.2 | 1052.4 | 81.6 | 25.9 | 96.4 |
| 356 | 625.8 | 410.2 | 2722.4 | 190.1 | 528.5 | 449.5 |
| 357 | 301.6 | 385.5 | 990 | 299.7 | 781 | 321.6 |
| 358 | 855.6 | 468.3 | 570.2 | 419.9 | 530.5 | 768 |
| 359 | 336.5 | 327.4 | 464.6 | 420.5 | 326.9 | 556.2 |
| 362 | 247.8 | 386.1 | 248.5 | 248 | 355.4 | 570.5 |
| 360 | 219.2 | 183.9 | 501.1 | 198.9 | 170.3 | 376.9 |
| 361 | 248.2 | 544 | 730.9 | 108.3 | 746.6 | 231.5 |
| 363 | 716.9 | 541.2 | 438.2 | 3964.4 | 373.4 | 824.2 |
| 1 | 469.7 | 405.7 | 1234.4 | 133.8 | 970 | 527.9 |
| 2 | 135.1 | 2759.1 | 3994.4 | 239.3 | 3662.3 | 264.1 |
| 7 | 1.3 | 402.8 | 129 | 94.8 | 1127.5 | 460.2 |
| 8 | 1162.1 | 811.3 | 967.4 | 854.4 | 644.1 | 915.3 |
| 9 | 2702.9 | 1018.8 | 4839.3 | 217.3 | 1823.6 | 997.2 |
| 10 | 190.7 | 504.6 | 237.6 | 195.3 | 126.1 | 271.3 |
| 11 | 1104.8 | 1435.3 | 2566.4 | 297.7 | 275.4 | 422.7 |
| 13 | 198.3 | 136 | 54.3 | 73.1 | 68.5 | 266.9 |
| 14 | 545.3 | 572.6 | 232.1 | 73.2 | 69.4 | 676 |
| 15 | 82.9 | 465.9 | 409.7 | 155.7 | 537.1 | 134.5 |
| 16 | 981.5 | 987.6 | 1149.6 | 167.1 | 888.7 | 1443.1 |
| 17 | 178.5 | 639 | 1677.1 | 125.2 | 2064.5 | 437.9 |
| 19 | 289.1 | 542.1 | 212.7 | 359 | 106.4 | 291.4 |
| 20 | 1.1 | 118.7 | 73.8 | 1.2 | 19.5 | 6.1 |
| 21 | 928.1 | 1108.3 | 1144.3 | 77 | 10.6 | 499.2 |
| 22 | 1063.2 | 3119.6 | 4358 | 872.9 | 8269.8 | 577.8 |
| 23 | 1.2 | 379.9 | 35.2 | 1.2 | 1.1 | 61.4 |
| 24 | 73.7 | 59 | 226.7 | 57.2 | 297.5 | 65.5 |
| 26 | 117.4 | 453.2 | 143.8 | 58.4 | 89 | 440 |
| 27 | 1699.9 | 830.8 | 811.4 | 403.5 | 511.2 | 509.3 |
| 29 | 594.6 | 288 | 325.3 | 378 | 112.4 | 982.9 |
| 30 | 86.9 | 82.7 | 124.5 | 10.9 | 45 | 97 |
| 31 | 1573.7 | 38.8 | 497.9 | 471.5 | 34.3 | 72.6 |
| 33 | 441.8 | 872.9 | 1130.8 | 75.9 | 411.7 | 352 |
| 34 | 1018.8 | 2164.1 | 2701.3 | 2309.9 | 2634.9 | 4374.3 |
| 35 | 99.6 | 70.4 | 393.2 | 80.2 | 13.2 | 133.7 |
| 36 | 167.8 | 137.3 | 152.5 | 203.8 | 1201.5 | 111.3 |
| 37 | 329.9 | 181.7 | 190.5 | 777.4 | 172.2 | 464.8 |

(continued)

| 3k | | | | | | |
|---|---|---|---|---|---|---|
| SEQ ID NO: | 967TT | 968TT | 969TT | 970TT | 982TT | 983TT |
| 38 | 240.2 | 1359.2 | 1110.5 | 89.7 | 1069.5 | 51.1 |
| 40 | 666.4 | 451.5 | 462.6 | 593.8 | 349.6 | 703.8 |
| 41 | 180.9 | 1696.6 | 333.6 | 178.9 | 730 | 231.9 |
| 42 | 176.5 | 403.8 | 214 | 306.2 | 308.2 | 287.7 |
| 43 | 821.9 | 483.9 | 321 | 1489 | 232.8 | 1352.9 |
| 44 | 467.5 | 380 | 471.5 | 555.7 | 360.3 | 455 |
| 45 | 770.8 | 434.4 | 558.1 | 1817 | 1071 | 1373.8 |
| 46 | 411.4 | 330.8 | 255 | 409.7 | 273.6 | 401.2 |
| 47 | 166.6 | 296.7 | 247.1 | 277.4 | 321.6 | 130.9 |
| 48 | 1.2 | 116.9 | 1.2 | 69 | 28.9 | 111 |
| 49 | 627.1 | 965.5 | 130.2 | 225.8 | 251.8 | 772.3 |
| 50 | 142.5 | 216.3 | 192.3 | 15.1 | 77.1 | 161.5 |
| 51 | 57.4 | 149.4 | 38.2 | 2101.5 | 1490.9 | 1190.6 |
| 52 | 308.7 | 413.6 | 298.7 | 307.9 | 217.7 | 234.6 |
| 53 | 84.9 | 100.2 | 159.9 | 93.3 | 102 | 217.6 |
| 54 | 870.3 | 607.4 | 345.8 | 269.3 | 669.6 | 529 |
| 55 | 161.6 | 94.8 | 98.6 | 115.2 | 110.3 | 144.3 |
| 56 | 106.7 | 36.5 | 54 | 42.1 | 150.1 | 33.6 |
| 57 | 343.8 | 1228.1 | 103 | 770.3 | 171.3 | 966.7 |
| 58 | 392.8 | 1366.3 | 238.3 | 1256 | 263.5 | 1149.6 |
| 59 | 605.2 | 1677.6 | 2094 | 285.7 | 1088 | 1273.8 |
| 60 | 202.7 | 198 | 164.7 | 124.8 | 168.1 | 155.5 |
| 61 | 542.2 | 245.6 | 124.9 | 288 | 103.9 | 201.2 |
| 62 | 1650.5 | 7509.1 | 3612.1 | 1170.1 | 318.8 | 9459.8 |
| 63 | 259.6 | 202.4 | 365.9 | 187.3 | 388.3 | 316.8 |
| 64 | 540.3 | 166.5 | 122.8 | 1276.6 | 123.7 | 1611 |
| 65 | 88.4 | 78.4 | 66.1 | 90.1 | 66.4 | 98.4 |
| 66 | 351.3 | 424.5 | 843.2 | 182.5 | 1223.8 | 103.2 |
| 67 | 8409.7 | 26223.6 | 34005.1 | 9041.4 | 32601.5 | 3094.7 |
| 68 | 2810.9 | 3260.1 | 2929.3 | 4961.6 | 4818.8 | 513.1 |
| 69 | 11767.3 | 6789.1 | 10957 | 11079.3 | 10388.2 | 3855.9 |
| 70 | 2845.6 | 1603 | 1889.8 | 8120.7 | 1927.8 | 1628.7 |
| 71 | 268.6 | 92.8 | 207.5 | 74.8 | 92.7 | 322.2 |
| 72 | 139.6 | 120.7 | 919.8 | 113.4 | 282.2 | 275.2 |
| 73 | 237.7 | 278.3 | 786.2 | 192.5 | 388.7 | 263.9 |
| 75 | 91.9 | 141.7 | 446.1 | 57.6 | 157.7 | 455.9 |
| 76 | 554.2 | 24.1 | 14.5 | 498.3 | 903.9 | 2000.7 |

(continued)

| 3k | | | | | | |
|---|---|---|---|---|---|---|
| SEQ ID NO: | 967TT | 968TT | 969TT | 970TT | 982TT | 983TT |
| 77 | 2410.2 | 1463.1 | 4948 | 4569.6 | 2755.1 | 5975.3 |
| 78 | 315.7 | 431.5 | 516.1 | 324.3 | 1188.7 | 81 |
| 79 | 162.4 | 146 | 171.7 | 204.3 | 106.5 | 265.2 |
| 80 | 100.7 | 790.8 | 491.3 | 95.1 | 372.8 | 933.3 |
| 81 | 2776.2 | 78.6 | 226.5 | 49.7 | 116.4 | 232.7 |
| 82 | 1230.5 | 231.4 | 120 | 1479.5 | 468.6 | 3720.2 |
| 84 | 308.9 | 961.7 | 101 | 78.7 | 204.4 | 653.1 |
| 85 | 350.3 | 125.3 | 159.1 | 154.7 | 129 | 484.4 |
| 86 | 91 | 85.7 | 73.9 | 126.3 | 52.9 | 189.6 |
| 87 | 296.2 | 320.3 | 1151.2 | 607.3 | 338 | 598.1 |
| 88 | 1909 | 1350 | 5707.2 | 1415.8 | 10923.2 | 860.8 |
| 89 | 206.8 | 203.5 | 145.5 | 160.9 | 154.6 | 303.9 |
| 90 | 95.9 | 93.9 | 95.8 | 99.7 | 108.7 | 108.2 |
| 91 | 362 | 346.8 | 219.6 | 491.8 | 78.8 | 550.5 |
| 92 | 3647.7 | 833.5 | 7305.9 | 83.4 | 3870.6 | 3178.4 |
| 93 | 158.2 | 341.1 | 321.3 | 94.2 | 217.3 | 459 |
| 94 | 199.5 | 488 | 854.3 | 71.2 | 251.2 | 114.4 |
| 95 | 90.1 | 99 | 154.9 | 131 | 686.8 | 242.9 |
| 96 | 152.2 | 124.4 | 172.1 | 130.6 | 192.9 | 111.2 |
| 97 | 251.1 | 342.4 | 429.8 | 95.2 | 143.5 | 354.9 |
| 98 | 240.9 | 37.1 | 30.6 | 67.9 | 14.8 | 257.9 |
| 99 | 321.4 | 281.7 | 244.4 | 316 | 192.3 | 311.7 |
| 100 | 288.5 | 356.2 | 13918.1 | 777.1 | 7145.1 | 1884.5 |
| 101 | 158.2 | 192.6 | 116.4 | 133.6 | 117.1 | 224.1 |
| 102 | 353.9 | 153.6 | 142.8 | 363.4 | 159 | 275.5 |
| 103 | 351.3 | 300.2 | 333.9 | 367.9 | 291.8 | 152.5 |
| 104 | 274.1 | 45.1 | 405.3 | 200.1 | 1282.8 | 873.3 |
| 105 | 449.3 | 51.2 | 538 | 259.7 | 701.6 | 1037.6 |
| 106 | 297.6 | 32.8 | 390.4 | 273.1 | 945.8 | 933.9 |
| 107 | 169.5 | 176.1 | 188.6 | 213.6 | 121.6 | 426.3 |
| 108 | 160.8 | 676.4 | 409.7 | 356.4 | 505.9 | 328.9 |
| 109 | 3423.5 | 49.2 | 4125.3 | 130.2 | 29.4 | 301.6 |
| 110 | 5966.7 | 10.7 | 4569.2 | 200.4 | 25.8 | 956.3 |
| 111 | 253.1 | 609.4 | 71.4 | 158.5 | 134.5 | 223.5 |
| 112 | 1062.8 | 94.2 | 773.3 | 1195.8 | 507.3 | 1.2 |
| 113 | 221.5 | 123.7 | 67.4 | 210.1 | 274.1 | 7.7 |
| 114 | 123.6 | 108.3 | 382.3 | 250.2 | 272.6 | 105.3 |

(continued)

| 3k | | | | | | |
|---|---|---|---|---|---|---|
| SEQ ID NO: | 967TT | 968TT | 969TT | 970TT | 982TT | 983TT |
| 115 | 397.9 | 320.1 | 369.8 | 242.8 | 282.1 | 292 |
| 116 | 279.4 | 395 | 237.5 | 165.5 | 184.5 | 311.1 |
| 117 | 200.4 | 1406.9 | 3545.8 | 607.8 | 2324.7 | 349.8 |
| 118 | 336.5 | 116.3 | 86.4 | 5808.9 | 64.9 | 2556.4 |
| 119 | 365.6 | 76.7 | 50.2 | 122.9 | 83.3 | 80.8 |
| 120 | 48.1 | 344 | 49.8 | 99.6 | 3044.7 | 110.5 |
| 121 | 259.6 | 336.7 | 403.1 | 87.6 | 1213.5 | 143.5 |
| 122 | 292.8 | 185.3 | 455.1 | 158.5 | 34.9 | 507.8 |
| 123 | 139.7 | 152.8 | 81.1 | 352.1 | 126 | 366.5 |
| 124 | 172.2 | 143.6 | 148.2 | 5160.7 | 124 | 172 |
| 125 | 1555.3 | 978.9 | 878.3 | 1620.9 | 867.1 | 1453.5 |
| 126 | 87.5 | 59.3 | 1558.6 | 257.7 | 1842.3 | 258.4 |
| 127 | 140.3 | 83.5 | 117.6 | 156 | 105 | 192 |
| 128 | 320 | 249.4 | 167.1 | 365.5 | 265.9 | 418.6 |
| 129 | 189.6 | 189.4 | 161.7 | 86 | 198.2 | 224.2 |
| 130 | 122.9 | 84.1 | 71.2 | 115.6 | 87.4 | 72 |
| 131 | 140.1 | 39.7 | 182.4 | 236.8 | 71.2 | 535 |
| 132 | 243.2 | 184 | 208.8 | 237.5 | 218.6 | 256.7 |
| 133 | 174.8 | 338.7 | 113.2 | 82.8 | 67.1 | 321.5 |
| 134 | 182.1 | 160.9 | 497.9 | 87.7 | 278.3 | 109.1 |
| 135 | 286.8 | 205.4 | 723.3 | 307 | 241.7 | 336.9 |
| 138 | 146.4 | 136.3 | 144.8 | 124 | 114.4 | 176.2 |
| 139 | 202.8 | 189.9 | 152 | 72.1 | 169.5 | 132.4 |
| 140 | 18.2 | 1010.6 | 2927.9 | 23.6 | 1469.1 | 1.5 |
| 141 | 178.6 | 283.5 | 2257.9 | 3.4 | 1606.2 | 310 |
| 144 | 6356.1 | 261.5 | 311.4 | 419.2 | 349.3 | 527.5 |
| 145 | 298.7 | 186.1 | 180.4 | 255.9 | 190 | 279.2 |
| 146 | 272 | 229.4 | 195.1 | 295.7 | 196 | 343.1 |
| 147 | 42.5 | 44.6 | 35.8 | 58.6 | 44.5 | 29.3 |
| 148 | 357.4 | 192.1 | 831.9 | 156.5 | 464.4 | 1068.9 |
| 149 | 272.6 | 184 | 1807.9 | 33 | 284.7 | 171 |
| 150 | 182.2 | 502.5 | 356 | 148.7 | 526.3 | 948.6 |
| 151 | 265.1 | 174.8 | 181.4 | 138.7 | 143.7 | 162.4 |
| 153 | 132.4 | 185.7 | 157.8 | 103.5 | 113.3 | 218.6 |
| 154 | 145.6 | 46.6 | 390.9 | 121.8 | 68.4 | 160.8 |
| 155 | 226.6 | 139.4 | 130.3 | 224.6 | 96.3 | 238.7 |
| 156 | 81.5 | 50.5 | 20.5 | 169.1 | 107.5 | 88.7 |

(continued)

| 3k | | | | | | |
|---|---|---|---|---|---|---|
| SEQ ID NO: | 967TT | 968TT | 969TT | 970TT | 982TT | 983TT |
| 157 | 226.7 | 170.6 | 136.4 | 131.5 | 112.2 | 189.1 |
| 158 | 236.4 | 237.1 | 1.1 | 202.4 | 39.1 | 650.6 |
| 159 | 1520.9 | 4126.2 | 3171.7 | 755.4 | 4600.4 | 3258.9 |
| 161 | 31.6 | 790.1 | 3.5 | 12.1 | 2.5 | 1.3 |
| 162 | 62.1 | 71.5 | 45.6 | 40.8 | 21.6 | 57.1 |
| 164 | 391.7 | 235.7 | 1236.8 | 91.4 | 215.8 | 40.9 |
| 166 | 4422.1 | 4880.3 | 7064.9 | 2557.6 | 3776.6 | 8467.5 |
| 167 | 544.2 | 1623 | 684.2 | 1035.6 | 3875 | 2178.2 |
| 168 | 2410.7 | 82.3 | 985.8 | 19.1 | 523.6 | 43 |
| 169 | 485.1 | 1840.5 | 7446.2 | 1266.9 | 1301.4 | 2686.1 |
| 170 | 971.7 | 643 | 1478.2 | 166.9 | 2916.1 | 760.7 |
| 171 | 38.2 | 233.9 | 733 | 1.2 | 1.2 | 503.5 |
| 172 | 492.3 | 472 | 656 | 483.1 | 510.4 | 757.2 |
| 173 | 542.8 | 375.8 | 794.6 | 114.4 | 595 | 647.8 |
| 176 | 10.6 | 32.1 | 1.2 | 69.1 | 991.6 | 1.2 |
| 177 | 224.2 | 1237.4 | 236.6 | 1044.6 | 789.8 | 993.8 |
| 178 | 1934.6 | 409 | 470.7 | 650.5 | 1387 | 941.3 |
| 179 | 145.3 | 412.9 | 60.7 | 161.2 | 85.7 | 245.7 |
| 180 | 161 | 92.4 | 67.4 | 128.2 | 94.3 | 131.7 |
| 181 | 9117.6 | 8152.5 | 11035.8 | 13798.5 | 15093.4 | 8202.9 |
| 182 | 189.4 | 445.2 | 574.2 | 128.1 | 524.1 | 227.5 |
| 183 | 1.1 | 64.7 | 11.7 | 1.2 | 1.1 | 72.1 |
| 184 | 215 | 541.8 | 290 | 167.9 | 286.9 | 190 |
| 185 | 973.9 | 711.7 | 1014 | 679.8 | 914.9 | 1030.3 |
| 186 | 377 | 560.6 | 395.4 | 1755.4 | 302.8 | 732.2 |
| 187 | 53.8 | 67.3 | 41.8 | 81.4 | 33.1 | 104.6 |
| 188 | 43.6 | 33.7 | 103.2 | 61.3 | 1218.5 | 94.9 |
| 189 | 86.4 | 37.9 | 69.2 | 84.5 | 695.6 | 108.4 |
| 190 | 407.6 | 372.7 | 283.5 | 135.6 | 90.2 | 162.9 |
| 191 | 359.8 | 2605 | 2070.6 | 919.3 | 2961.2 | 4325.2 |
| 192 | 856.8 | 383.6 | 183.3 | 146.2 | 69.3 | 827.1 |
| 193 | 75.8 | 1538.4 | 2686.5 | 638.6 | 2367.3 | 33.3 |
| 194 | 121.6 | 135.5 | 230 | 28.4 | 238.5 | 127.6 |
| 195 | 159.5 | 372.6 | 180 | 614.6 | 333.1 | 355.6 |
| 196 | 7404.9 | 4791.5 | 952.5 | 7933.6 | 7823.2 | 8113.2 |
| 197 | 133.6 | 44.6 | 130.5 | 218.5 | 71.7 | 113.9 |
| 198 | 129.4 | 323.2 | 103.3 | 138.9 | 56.8 | 123.9 |

(continued)

| 3k | | | | | | |
|---|---|---|---|---|---|---|
| SEQ ID NO: | 967TT | 968TT | 969TT | 970TT | 982TT | 983TT |
| 199 | 105.7 | 36 | 1584.1 | 2.7 | 42.7 | 162 |
| 200 | 251.1 | 102.9 | 160.6 | 270.3 | 374.1 | 170.5 |
| 201 | 70.3 | 49.7 | 172.9 | 58.1 | 1.6 | 125.6 |
| 203 | 155.2 | 84.1 | 172.2 | 202.8 | 393.4 | 343.4 |
| 204 | 258.9 | 711.5 | 1093.8 | 268.3 | 710.3 | 515.6 |
| 205 | 1215.1 | 1501.8 | 7081.6 | 570.2 | 5009.6 | 1308.5 |
| 206 | 328 | 173 | 298.2 | 310.4 | 185.1 | 597.2 |
| 207 | 172.9 | 68.6 | 1428.1 | 46.5 | 2541.6 | 144.3 |
| 208 | 1492.3 | 7 | 24.8 | 10.1 | 783.8 | 45.3 |
| 209 | 667.1 | 1776.2 | 311.6 | 1203.9 | 2745 | 1499.4 |
| 211 | 1193.2 | 2651.6 | 12245 | 343.1 | 25458 | 17479.9 |
| 212 | 837.5 | 433 | 245 | 2353.6 | 512.9 | 728.8 |
| 213 | 203.3 | 87 | 71.4 | 3629.9 | 93.5 | 468.6 |
| 215 | 176 | 71.1 | 554.7 | 57.5 | 3381.9 | 202 |
| 216 | 611.8 | 308.3 | 915.4 | 490.4 | 896.8 | 690.5 |
| 217 | 118.5 | 146.7 | 191.2 | 121 | 293.9 | 120.7 |
| 218 | 252.2 | 466.5 | 274.1 | 124.9 | 335.3 | 428.6 |
| 220 | 125.2 | 75.4 | 108.2 | 10561 | 77.9 | 131.8 |
| 221 | 139.2 | 264.1 | 452 | 146.5 | 699.3 | 143.9 |
| 223 | 415.6 | 845.6 | 362.8 | 477.7 | 165.6 | 689.8 |
| 227 | 314.8 | 2480.3 | 1008.1 | 182.6 | 1091.2 | 223.2 |
| 228 | 432.9 | 294.9 | 213.2 | 461.6 | 568.4 | 517.8 |
| 229 | 143.7 | 774 | 171.7 | 1810.6 | 305 | 446.6 |
| 230 | 179 | 63.3 | 79.4 | 202.4 | 63.6 | 37.6 |
| 231 | 136.2 | 113.7 | 127 | 180.7 | 207.4 | 205 |
| 232 | 45.3 | 272.3 | 86.5 | 8.9 | 63.8 | 337.4 |
| 233 | 1483 | 4545.5 | 6134.3 | 1579.1 | 7762.7 | 3649.3 |
| 235 | 85.7 | 443.4 | 423.8 | 66.6 | 211.8 | 323.6 |
| 236 | 110.2 | 202.4 | 106.8 | 196.1 | 123.9 | 257.5 |
| 237 | 1495.9 | 564.8 | 1641 | 1057.2 | 1533.6 | 704.4 |
| 238 | 744.2 | 349.2 | 899.4 | 244.1 | 1354 | 349.4 |
| 239 | 186.3 | 100.4 | 196.3 | 93 | 124 | 153.1 |
| 240 | 195.9 | 71.5 | 727.2 | 65.8 | 131.3 | 148 |
| 241 | 237.2 | 205.2 | 1034.3 | 94.1 | 300.5 | 145.6 |
| 242 | 5003.3 | 13867.1 | 2129.7 | 15742.7 | 4086.9 | 11985.6 |
| 243 | 803.1 | 288.9 | 174.5 | 134.2 | 226.2 | 143.1 |
| 244 | 97.8 | 194.6 | 282.9 | 8.3 | 256.1 | 109.7 |

(continued)

| 3k | | | | | | |
|---|---|---|---|---|---|---|
| SEQ ID NO: | 967TT | 968TT | 969TT | 970TT | 982TT | 983TT |
| 246 | 359 | 276.8 | 388.3 | 169.2 | 115.4 | 448.4 |
| 247 | 170.8 | 140.7 | 109.7 | 303.9 | 118.1 | 139.1 |
| 248 | 245.6 | 109.7 | 217.3 | 113.2 | 196.9 | 135.7 |
| 249 | 96.3 | 216.6 | 223.8 | 135.9 | 48.3 | 585.6 |
| 251 | 187.9 | 61.1 | 115.5 | 59.8 | 112.5 | 93.9 |
| 253 | 260.8 | 275.7 | 327.1 | 165.6 | 468.1 | 240.6 |
| 254 | 63.2 | 64.3 | 465.3 | 121 | 2267.5 | 15.9 |
| 255 | 1923 | 352.4 | 648.8 | 811.1 | 1499.8 | 1126.3 |
| 256 | 563.4 | 448.2 | 228.9 | 490.4 | 82.7 | 573.9 |
| 257 | 378.8 | 648.4 | 475.3 | 161.3 | 185.7 | 145.8 |
| 258 | 582.1 | 257.2 | 5241.4 | 168 | 40.2 | 1024 |
| 259 | 30 | 73.9 | 532.4 | 28.9 | 44.5 | 13.3 |
| 260 | 209.5 | 228.1 | 128.5 | 237.3 | 114 | 618.7 |
| 261 | 135.7 | 834.3 | 320.6 | 115.8 | 259.6 | 134.1 |
| 263 | 320.6 | 673.5 | 537 | 470.6 | 473.9 | 2280.3 |
| 263 | 316.2 | 103.4 | 161.5 | 369.4 | 123.6 | 137.2 |
| 265 | 106.3 | 287.4 | 601.3 | 317.4 | 934.8 | 119.6 |
| 266 | 216.8 | 145.8 | 179.8 | 955 | 181.8 | 470.8 |
| 256 | 147.4 | 89.7 | 97.7 | 81.8 | 124.2 | 119.5 |
| 268 | 304.8 | 19.7 | 60.1 | 175.6 | 2929.8 | 220.9 |
| 269 | 61.3 | 84.9 | 35 | 57.5 | 38 | 158.7 |
| 270 | 276.1 | 144.9 | 183.1 | 1394.4 | 176.9 | 726.2 |
| 271 | 139.7 | 191.4 | 249.6 | 243.4 | 292.7 | 190 |
| 272 | 1068.5 | 485.1 | 605.5 | 722 | 403 | 558.4 |
| 273 | 145.8 | 421.9 | 4104.1 | 1.3 | 1705.7 | 34.7 |
| 274 | 178.2 | 517.1 | 426.4 | 468.2 | 206.6 | 681.6 |
| 275 | 271.4 | 219.3 | 164 | 299.8 | 209.9 | 428.2 |
| 276 | 396.3 | 3030.8 | 977.3 | 188 | 1246.2 | 307.4 |
| 277 | 228.6 | 153.3 | 181.7 | 212.1 | 199 | 244.3 |
| 278 | 50.3 | 1164.1 | 1019 | 52 | 88.6 | 105.7 |
| 279 | 985.7 | 1407 | 2129.1 | 232.5 | 2794.6 | 1002 |
| 280 | 1510.7 | 2192.5 | 6373 | 286.6 | 3920.2 | 1434.5 |
| 281 | 24.5 | 1.2 | 32.8 | 20.6 | 66.1 | 40.3 |
| 282 | 4703.6 | 248.3 | 312.9 | 291 | 241.6 | 404.4 |
| 283 | 413.8 | 290.4 | 220 | 660.8 | 232 | 552.8 |
| 284 | 106.4 | 84.7 | 78.8 | 140.9 | 65.1 | 99.7 |
| 285 | 148.4 | 92.7 | 162.5 | 143.5 | 104.4 | 219 |

(continued)

| 3k | | | | | | |
|---|---|---|---|---|---|---|
| SEQ ID NO: | 967TT | 968TT | 969TT | 970TT | 982TT | 983TT |
| 286 | 129 | 10.1 | 1134.4 | 36.1 | 1.3 | 510.6 |
| 287 | 127.8 | 111.9 | 67.9 | 468.2 | 86.5 | 147.5 |
| 288 | 267.1 | 129.5 | 153.3 | 256.4 | 171.7 | 231.2 |
| 289 | 91.1 | 390.9 | 271 | 465.5 | 214.2 | 1306.3 |
| 291 | 133.2 | 72.5 | 78.4 | 5.9 | 133.9 | 35.1 |
| 292 | 75.7 | 25.7 | 293.3 | 71.5 | 18.4 | 104.7 |
| 293 | 438 | 86.4 | 72.7 | 37.3 | 77.1 | 371 |
| 295 | 244.8 | 581.6 | 30.1 | 979.1 | 569.4 | 507.9 |
| 296 | 1349.4 | 504 | 1210.8 | 671.3 | 716.7 | 813.5 |
| 297 | 64.9 | 45.4 | 49.4 | 853.9 | 62 | 98.3 |
| 298 | 186.2 | 307.3 | 416.8 | 311.1 | 232.4 | 211.4 |
| 299 | 306.5 | 444.4 | 962 | 703.8 | 375.8 | 401.4 |
| 300 | 420.6 | 101.7 | 176.4 | 125.4 | 475.3 | 235.4 |
| 301 | 507.2 | 2222 | 808.6 | 528.9 | 1281.3 | 340.3 |
| 302 | 29.2 | 22.3 | 23.2 | 31.4 | 9.5 | 10.1 |
| 303 | 457.4 | 993.6 | 1360.4 | 930 | 836.6 | 1031.2 |
| 304 | 262.8 | 452.7 | 227.3 | 209.2 | 208.1 | 397.9 |
| 305 | 531.2 | 6335.1 | 145.3 | 300 | 1625.9 | 5308.7 |
| 306 | 262.8 | 287.6 | 155.9 | 258.3 | 126 | 317.5 |
| 307 | 150.7 | 121.6 | 150 | 167.9 | 118.6 | 139.3 |
| 308 | 1991.2 | 756 | 1163.4 | 4741.6 | 1375.7 | 4290.1 |
| 310 | 950.2 | 194.6 | 1093.2 | 1092.8 | 219.2 | 640.7 |
| 311 | 64.6 | 25.1 | 144.2 | 55.8 | 44.7 | 73.8 |
| 312 | 797.1 | 35.8 | 1289.1 | 5881.4 | 362.4 | 1192 |
| 313 | 542.5 | 719.4 | 122 | 119.1 | 139.3 | 161.9 |
| 314 | 938.2 | 209.6 | 551.3 | 68.6 | 147.2 | 345 |
| 315 | 123.7 | 274.1 | 155.2 | 218.6 | 299.1 | 52.8 |
| 316 | 289.8 | 413.1 | 415.9 | 205.6 | 84.3 | 704.2 |
| 317 | 202.4 | 156.1 | 133.7 | 210.8 | 138.1 | 206.3 |
| 318 | 126.8 | 259.1 | 109.3 | 26.5 | 16.2 | 157.6 |
| 319 | 362 | 168.9 | 161.6 | 713.5 | 195.9 | 1566.2 |
| 320 | 503.3 | 277.3 | 356.8 | 1176.5 | 487.2 | 297.3 |
| 321 | 218.1 | 470.4 | 426.1 | 337.2 | 517.9 | 711.3 |
| 322 | 375.3 | 251.1 | 292.4 | 504 | 186.1 | 609.6 |
| 323 | 553 | 949.4 | 2829.3 | 768.9 | 4431.8 | 1365.8 |
| 324 | 179.2 | 52.7 | 227.9 | 81.1 | 81.7 | 41.1 |
| 325 | 335.3 | 242.7 | 242.8 | 170.7 | 198.5 | 313.4 |

(continued)

| 3k | | | | | | |
|---|---|---|---|---|---|---|
| SEQ ID NO: | 967TT | 968TT | 969TT | 970TT | 982TT | 983TT |
| 326 | 1260.9 | 2604.5 | 4986.5 | 584.6 | 5373.5 | 643.7 |
| 327 | 29.1 | 14.2 | 26.9 | 21.4 | 61.9 | 2.8 |
| 328 | 58.8 | 218.5 | 113.1 | 63.9 | 223.4 | 73.3 |
| 329 | 191.7 | 61.4 | 40.3 | 147.8 | 62.3 | 144.2 |
| 330 | 134.1 | 100.9 | 58.4 | 36 | 18.7 | 37.3 |
| 331 | 445.5 | 369.7 | 271.4 | 981.9 | 227.2 | 893.2 |
| 333 | 76.9 | 473 | 115.9 | 230.1 | 287.1 | 484.1 |
| 334 | 172.8 | 136 | 152.4 | 100.5 | 143.3 | 120.5 |
| 335 | 122.9 | 126.9 | 113.2 | 212.1 | 846.9 | 658.3 |
| 336 | 308.8 | 716 | 987.8 | 236.4 | 665.2 | 600.4 |
| 337 | 43.9 | 98.3 | 1139.9 | 53.3 | 129.7 | 270.9 |
| 338 | 290.4 | 144.6 | 420.3 | 105.8 | 134.4 | 220.2 |
| 339 | 338.2 | 255.3 | 217.6 | 483.7 | 351.7 | 459.4 |
| 340 | 241.7 | 256.2 | 235.6 | 263.3 | 247.8 | 313.8 |
| 341 | 693.6 | 1174.2 | 706.5 | 545.1 | 590.9 | 506.5 |
| 343 | 2675.2 | 997.1 | 235.2 | 5179.8 | 686.5 | 3885.2 |
| 344 | 1600.8 | 650.5 | 866.5 | 3374.5 | 937.5 | 785.9 |
| 345 | 371 | 324 | 225.2 | 332.3 | 208.4 | 349.4 |
| 347 | 930.2 | 70.2 | 922.3 | 734.3 | 7238.6 | 705.8 |
| 348 | 2298.3 | 37.7 | 1946.1 | 988.1 | 10651.2 | 2867.2 |
| 349 | 208.2 | 402.1 | 215.3 | 174.1 | 296.4 | 343.8 |
| 350 | 2.9 | 34.4 | 518.4 | 27.6 | 143.5 | 191.8 |
| 351 | 188.2 | 69.4 | 94.8 | 40.4 | 46.1 | 744.7 |
| 352 | 269.7 | 230.4 | 500.5 | 390.8 | 298.9 | 319.5 |
| 353 | 757.9 | 154.1 | 248.3 | 481.8 | 1304.4 | 432.8 |

Table 4. 4-Gene signature performance in 98 training samples

| | Tumor | Benign |
|---|---|---|
| Positive | 48 | 18 |
| Negative | 4 | 28 |
| Sensitivity | 92% (0.82, 0.97) | |
| Specificity | 61 % (0.46, 0.74) | |

B. Signature Identification using Linear Discrimination Analysis

We used a forward selection process that adds one gene at a time until the posterior error as evaluated by a linear discriminator is less than or equal to 0.1. A four-gene signature was discovered using this approach with the 322 genes. The identities of these 4 genes are listed in Tables 5 and 16 and their chip data are shown in Tables 6 and 7.

Table 5. 4-Gene Signature

| SEQ ID NO: | Gene Name |
|---|---|
| 354 | Chemokine (C-C motif) ligand 18 (pulmonary and activation-regulated) |
| 199 | Pulmonary surfactant-associated protein B (SP-B) |
| 207 | K + channel beta subunit |
| 255 | Putative prostate cancer tumor suppressor |

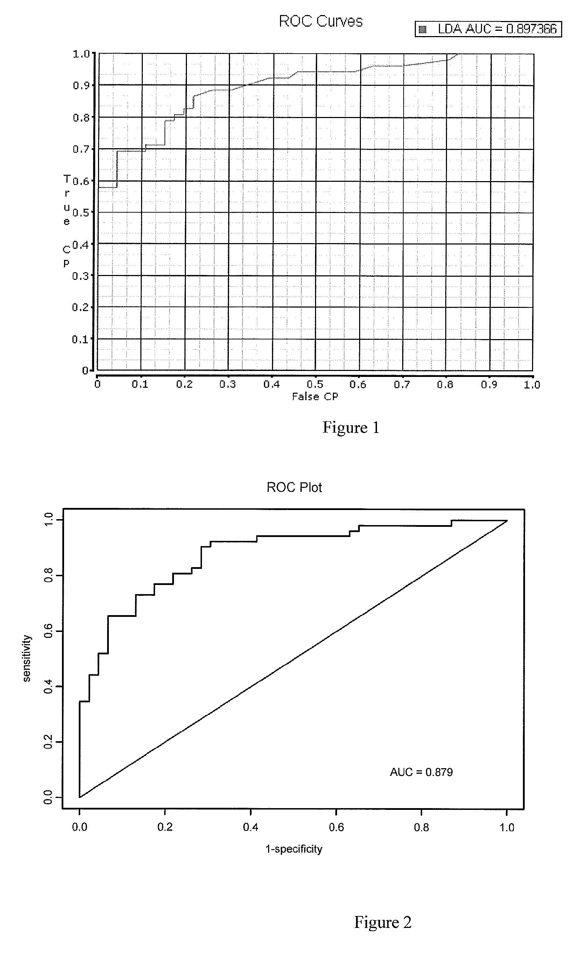

Leave One Out Cross Validation (LOOCV) resulted in 92% sensitivity and 61% specificity, shown in Table 4. The ROC curve gave an AUC of 0.897, as shown in Figure 1.

Table 6

| SEQ ID | Signal | | | | | | |
|---|---|---|---|---|---|---|---|
| | PC_984TT | PC_986TT | FA_987TT | PC_988TT | PC_989TT | FA_992TT | FA_993TT |
| 12 | 3399.2 | 7041.3 | 5438 | 6376.7 | 2734.6 | 3569.5 | 4305.9 |
| 18 | 6550.8 | 5870.6 | 5815.9 | 4265.1 | 8856.4 | 3454.4 | 20405.3 |
| 29 | 4578.6 | 6455.8 | 2329.4 | 425.9 | 2666.8 | 3694 | 7531.2 |
| | FA_994TT | FA_995TT | FA_996TT | FA_998TT | FA_999TT | FA_1001TT | FA_1002TT |
| 12 | 2545.7 | 2451.8 | 4418 | 3938.3 | 3648.5 | 6834.5 | 4882 |
| 18 | 12566.4 | 9889.5 | 4341.5 | 5210.1 | 6639.5 | 4794.8 | 3921.9 |
| 29 | 2239.8 | 1834.5 | 4607.1 | 6424.1 | 3069.6 | 4301.4 | 3164 |
| | FA_1004TT | FA_1005TT | FA_1006TT | FA_1010TT | FA_1013TT | FA_1014TT | FA_1017TT |
| 12 | 2274.9 | 2644.2 | 3978.8 | 2999.7 | 4012.5 | 1724 | 5005.8 |
| 18 | 10402 | 7376.3 | 9985.1 | 1330.3 | 4618.8 | 6132 | 4562.6 |
| 29 | 1688.9 | 4771.2 | 3159.1 | 3082.6 | 6861.7 | 1465 | 3384.7 |
| | FA_1018TT | FA_1020TT | FA_1023TT | FA_1024TT | FA_1026TT | FA_1027TT | FA_1028TT |
| 12 | 2847.6 | 7102 | 7513.5 | 2086.3 | 2431.3 | 1318.2 | 1231 |
| 18 | 6388.5 | 2640.8 | 1967.7 | 6853.3 | 5068.5 | 2006.4 | 1804.9 |
| 29 | 3829.5 | 4235 | 4272.5 | 924 | 1702.3 | 1691.5 | 1731.3 |
| | FA_1029TT | FA_1030TT | FA_1031TT | FA_1032TT | FA_1034TT | FA_1035TT | FC_1037TT |
| 12 | 4607.6 | 2416.1 | 2203.7 | 6489.9 | 2095.3 | 1547.9 | 1847.4 |
| 18 | 3507.3 | 2270.8 | 8058 | 11247.5 | 8258.1 | 7485.8 | 8309 |
| 29 | 1530.5 | 5641.4 | 3460.1 | 4802.1 | 1650.7 | 854.6 | 2418.4 |
| | PC_1039TT | PC_1040TT | PC_1041TT | PC_1042TT | PC_1043TT | PC_1044TT | PC_1045TT |
| 12 | 8204.9 | 2638.5 | 4795 | 2607.8 | 6725.4 | 5178.7 | 2111.1 |
| 18 | 6454.7 | 10129.3 | 7964 | 4952.6 | 3663.3 | 1887.7 | 19840.6 |
| 29 | 8858.5 | 4136.8 | 8434 | 2230.7 | 3434 | 2770 | 2243.1 |

|  | PC_1046TT | PC_1047TT | PC_1048TT | PC_1049TT | PC_1050TT | PC_1051TT | PC-FV_1052TT |
|---|---|---|---|---|---|---|---|
| 12 | 4403.6 | 9394.7 | 2262.5 | 4831.2 | 2826.9 | 1288.6 | 1953.2 |
| 18 | 2417.7 | 5678.4 | 6655.2 | 4325.2 | 1706 | 6904.8 | 4596.1 |
| 29 | 2319.8 | 20509.6 | 1176.1 | 5375.3 | 2053.1 | 4027.5 | 3332.3 |
|  | PC_1053TT | PC_1054TT | PC_1055TT | PC_1059TT | PC_1060TT | PC_1061TT | PC_1062TT |
| 12 | 4150.4 | 6180.4 | 1835.7 | 2447.2 | 4201.3 | 4984.3 | 3399.3 |
| 18 | 5965.7 | 13847.6 | 3740.6 | 4167.7 | 14516.2 | 4896.7 | 8891.6 |
| 29 | 3718.1 | 4818.3 | 1728.8 | 2972.2 | 4851 | 3469 | 3718.6 |
|  | PC-FV_1064TT | PC-FV_1065TT | PC-FV_1066TT | PC-FV_1067TT | PC-FV_1068TT | PC-FV_1069TT | PC-FV_1071TT |
| 12 | 4221 | 4884.5 | 7256.6 | 640 | 4055 | 3208.2 | 6301.3 |
| 18 | 4095 | 6627.6 | 1427.1 | 5405.5 | 10722 | 11509.9 | 10314.3 |
| 29 | 5896.7 | 5461.2 | 9437.3 | 1634.4 | 1344.2 | 2186.4 | 1794.8 |
|  | PC-FV_1072TT | FA_1073TT | FA_1074TT | FA_1075TT | FA_1076TT | FC_1077TT | FC_1078TT |
| 12 | 1390.3 | 3325.5 | 1430.8 | 1784.4 | 1563.9 | 1999.4 | 1830.7 |
| 18 | 5629 | 10010.5 | 3459.2 | 11454 | 10807.8 | 1384.8 | 11906.2 |
| 29 | 2974.5 | 7133.1 | 3009 | 4184.9 | 1673.9 | 1044.5 | 4264.1 |
|  | FC_1079TT | PC-FV_1080TT | PC-FV_1081TT | FC_1082TT | pb1 | pb2 | pb3 |
| 12 | 2856.2 | 2437.9 | 3541.2 | 4439.8 | 11.4 | 9.1 | 9.9 |
| 18 | 22738 | 4344.1 | 4108.3 | 3312.3 | 94.2 | 50.7 | 67.6 |
| 29 | 1092.8 | 3052.8 | 3302.6 | 2383 | 8.7 | 21.6 | 30.5 |
|  | pb4 | pb5' | pb6' | pb7' | pb8' | pd10 | pd11 |
| 12 | 33.8 | 8.3 | 6.6 | 18.8 | 61.4 | 38.2 | 10.7 |
| 18 | 74.9 | 29.7 | 53.6 | 37.8 | 31.8 | 68.8 | 117.5 |
| 29 | 66.1 | 64.6 | 36.4 | 51.5 | 25.1 | 17.2 | 17.2 |
|  | pd12 | pd9 |  |  |  |  |  |
| 12 | 4.1 | 11.6 |  |  |  |  |  |

(continued)

| | pd12 | pd9 |
|---|---|---|
| 18 | 70.8 | 23.4 |
| 29 | 19.7 | 11 |

Table 7

Signal

| SEQ ID | FA_987TT | FA_992TT | FA_993TT | FA_994TT | FA_995TT | FA_996TT | FA_998TT |
|---|---|---|---|---|---|---|---|
| 354 | 50.9 | 100.4 | 63.7 | 14 | 699.7 | 181.7 | 11.9 |
| 199 | 81.7 | 18 | 43.3 | 130.3 | 461.9 | 469.4 | 135.6 |
| 207 | 3844.9 | 1325.1 | 77.9 | 397.2 | 518.3 | 807.5 | 1084.3 |
| 255 | 1374.4 | 2332.1 | 1631 | 611.1 | 1616.7 | 419.2 | 230.7 |

| SEQ ID | FA_999TT | FA_1001TT | FA_1002TT | FA_1004TT | FA_1005TT | FA_1006TT | FA_1010TT |
|---|---|---|---|---|---|---|---|
| 354 | 194.7 | 2397.3 | 50.6 | 205.2 | 9.6 | 29.1 | 341.5 |
| 199 | 124.1 | 574.9 | 206 | 158.5 | 423.6 | 121 | 2489.4 |
| 207 | 1861.5 | 1325.1 | 846.6 | 308.8 | 1765.9 | 598.9 | 1067.7 |
| 255 | 266.5 | 186.7 | 324.2 | 94 | 1071.3 | 1098.5 | 1294 |

| SEQ ID | FA_1013TT | FA_1014TT | FA_1017TT | FA_1018TT | FA_1020TT | FA_1023TT | FA_1024TT |
|---|---|---|---|---|---|---|---|
| 354 | 207.5 | 69 | 21.7 | 20.1 | 64.7 | 87.4 | 372.5 |
| 199 | 440.8 | 99.8 | 221.6 | 226.9 | 108.2 | 155.2 | 112.6 |
| 207 | 535.7 | 1910.9 | 1185.2 | 571.8 | 1552.7 | 2739.5 | 692.6 |
| 255 | 437.2 | 655.6 | 165.1 | 178.4 | 86.4 | 436.5 | 40.7 |

| SEQ ID | FA_1026TT | FA_1027TT | FA_1028TT | FA_1029TT | FA_1030TT | FA_1031TT | FA_1032TT |
|---|---|---|---|---|---|---|---|
| 354 | 21.5 | 18.1 | 3.8 | 13.3 | 66 | 15.1 | 38.8 |
| 199 | 70.9 | 46.1 | 561.3 | 67.7 | 35.8 | 48 | 107.3 |
| 207 | 1230.4 | 80.5 | 732 | 3216.4 | 2253.5 | 1989.9 | 2115.2 |
| 255 | 479.9 | 226.5 | 35.9 | 1403.4 | 1144.3 | 247.9 | 1989.4 |

| SEQ ID | FA_1034TT | FA_1035TT | FA_1073TT | FA_1074TT | FA_1075TT | FA_1076TT | PC_984TT |
|---|---|---|---|---|---|---|---|
| 354 | 61.3 | 25.3 | 1355.7 | 188.5 | 14.7 | 8.9 | 97.5 |
| 199 | 216.5 | 56.1 | 1980.6 | 454.1 | 86.2 | 173.8 | 154.2 |
| 207 | 421.8 | 1493.5 | 771.6 | 1291.4 | 116.5 | 1169.9 | 759.2 |
| 255 | 489.2 | 229.5 | 205.3 | 655 | 152.7 | 156.3 | 152 |

(continued)

| | PC_986TT | PC_988TT | PC_989TT | PC_1039TT | PC_1040TT | PC_1041TT | PC_1042TT |
|---|---|---|---|---|---|---|---|
| 354 | 966.6 | 619 | 12.9 | 2577 | 540.4 | 1229.7 | 320 |
| 199 | 103.9 | 142.5 | 288.5 | 1006.6 | 3990.7 | 17402.7 | 1023.2 |
| 207 | 400.5 | 2357.5 | 1213.8 | 627.5 | 62.6 | 133.6 | 981.5 |
| 255 | 299.7 | 2137.5 | 131.9 | 500.6 | 2412.4 | 2763.1 | 303.4 |

| | PC_1043TT | PC_1044TT | PC_1045TT | PC_1046TT | PC_1047TT | PC_1048TT | PC_1049TT |
|---|---|---|---|---|---|---|---|
| 354 | 444.3 | 170.8 | 817.6 | 1095.1 | 966.8 | 1263.5 | 800.7 |
| 199 | 604.3 | 222.7 | 22961.4 | 9488.3 | 10311.6 | 1702.7 | 1366 |
| 207 | 1680.3 | 594.2 | 136.5 | 673.2 | 755 | 20.8 | 254.8 |
| 255 | 407.6 | 4162.4 | 872.5 | 2472.3 | 1497.3 | 2944.8 | 2399.9 |

| | PC_1050TT | PC_1051TT | PC_1053TT | PC_1054TT | PC_1055TT | PC_1059TT | PC_1060TT |
|---|---|---|---|---|---|---|---|
| 354 | 292.9 | 614.4 | 1035.4 | 1545.3 | 139.5 | 500.2 | 37.1 |
| 199 | 117 | 29233.8 | 1435 | 8550.5 | 242.8 | 13914.6 | 2377.3 |
| 207 | 87.8 | 113.4 | 73.3 | 187.7 | 491.6 | 158.4 | 207.3 |
| 255 | 2331.8 | 917.2 | 1505.9 | 2186.4 | 3033.2 | 2191.1 | 215.4 |

| | PC_1061TT | PC_1062TT | FC_1037TT | FC_1077TT | FC_1078TT | FC_1079TT | FC_1082TT |
|---|---|---|---|---|---|---|---|
| 354 | 71 | 23.8 | 18.4 | 28.9 | 87.2 | 13.5 | 27.7 |
| 199 | 6933.5 | 616.2 | 21.2 | 226.7 | 200.8 | 40.8 | 144.4 |
| 207 | 1088.5 | 1133.5 | 838.8 | 2012.3 | 24.9 | 196.8 | 1356.4 |
| 255 | 3084.8 | 1017.8 | 1355.3 | 199 | 1352.4 | 1578 | 1294.4 |

| | PC-FV_1052TT | PC-FV_1064TT | PC-FV_1065TT | PC-FV_1066TT | PC-FV_1067TT | PC-FV_1068TT | PC-FV_1069TT |
|---|---|---|---|---|---|---|---|
| 354 | 394.8 | 131.4 | 143.7 | 281.9 | 302.1 | 973.6 | 44.9 |
| 199 | 4620.7 | 1931 | 1613.2 | 170.8 | 579.2 | 20066.3 | 96.9 |
| 207 | 88.2 | 1033.1 | 705 | 244.1 | 2559 | 8.8 | 12.3 |
| 255 | 2479.7 | 242.2 | 1322 | 641.9 | 1222.8 | 1914.8 | 93.2 |

(continued)

| | PC-FV_1071TT | PC-FV_1072TT | PC-FV_1080TT | PC-FV_1081TT |
|---|---|---|---|---|
| 354 | 109.5 | 127.2 | 205.7 | 12.4 |
| 199 | 257.5 | 2302.7 | 320 | 206 |
| 207 | 42.9 | 72.2 | 2795.9 | 145 |
| 255 | 1128.1 | 2320.8 | 1705.2 | 196.8 |

C. Manual Selection of Markers

Individual genes were selected with an aim to formulate a RT-PCR based assay. Comparison of gene expression profiles between thyroid cancers and non-cancer tissues has identified a five-gene signature from these 322 genes. The identities of these five genes are shown in Tables 8 and 16 and the chip data are shown in Table 9. The performance of this signature was assessed using LDA in the 98 samples, and the signature gives 92% sensitivity and 70% specificity, shown in Table 10. The ROC curve gave an AUC of 0.88, as shown in Figure 2.

Table 8. 5-Gene signature

| SEQ ID NO: | Gene Name |
| --- | --- |
| 53 | Cadherin 3, type 1 (P cadherin) |
| 242 | Fibronectin |
| 73 | Secretory granule, neuroendocrine protein 1 |
| 36 | Testican-1 |
| 211 | Thyroid Peroxidase (TPO) |

Table 9

| SEQ ID | BN_800TT | BN_801TT | BN_802TT | BN_804TT | BN_805TT | BN_806TT | BN_807TT |
|---|---|---|---|---|---|---|---|
| 36 | 908.3 | 247 | 118.9 | 349.7 | 425.1 | 229.8 | 227.7 |
| 53 | 48.6 | 25 | 24.1 | 34 | 17.8 | 32.2 | 16.7 |
| 76 | 252.3 | 272.9 | 75.2 | 260.6 | 169 | 587.1 | 189.8 |
| 242 | 22247.9 | 1204.2 | 676.7 | 2441.5 | 3900.1 | 2073.4 | 2060.1 |
| 211 | 24.2 | 16983.5 | 32579.5 | 20189.3 | 24480.5 | 14186.3 | 6488.1 |
|  | BN_871TT | BN_913TT | BN_914TT | BN_915TT | FA_917TT | FA_918TT | FA_919TT |
| 36 | 897.2 | 435.1 | 442.3 | 508.7 | 169.6 | 184.3 | 213.3 |
| 53 | 192.4 | 28 | 76.1 | 44.2 | 67.7 | 135.7 | 25.7 |
| 76 | 2134.6 | 252.5 | 956.3 | 174.3 | 62 | 144 | 233.8 |
| 242 | 13722.5 | 10481.9 | 4172.9 | 1891.1 | 1654.2 | 11791 | 2788.1 |
| 211 | 6243.3 | 16339.1 | 14355 | 36350.1 | 29876.5 | 19395.7 | 15935.8 |
|  | FA_818TT | FA_819TT | FA_820TT | FA_821TT | FA_822TT | FA_842TT | FA_862TT |
| 36 | 120.4 | 155.8 | 210.6 | 31.7 | 283.3 | 122.2 | 24.1 |
| 53 | 27.2 | 26.9 | 34 | 33.2 | 34.4 | 27.8 | 66.1 |
| 76 | 13 | 69.8 | 36.2 | 171.2 | 116.2 | 190.8 | 15.9 |
| 242 | 1433.3 | 2211.8 | 2714.7 | 325 | 2278 | 3959.5 | 1573.1 |
| 211 | 15976.6 | 19882.4 | 10029.6 | 29411.3 | 25179.3 | 12492.5 | 32721.4 |
|  | FA_863TT | FA_864TT | FA_865TT | FA_866TT | FA_867TT | FA_869TT | FA_907TT |
| 36 | 955.1 | 173.1 | 400.5 | 230.8 | 233.5 | 2581.5 | 207.8 |
| 53 | 333.9 | 27.9 | 147.1 | 155 | 25 | 1224.9 | 52.8 |
| 76 | 2614.7 | 624.1 | 614.9 | 336.7 | 163.5 | 1921.3 | 38.5 |
| 242 | 930.4 | 1419.2 | 4708.4 | 2169.8 | 1081.9 | 3241.5 | 4555.3 |
| 211 | 19631.3 | 27382.8 | 29846 | 25011.6 | 30599.1 | 23680.6 | 35313.3 |

(continued)

| | FA_908TT | FA_920TT | FA_938TT | FA_940TT | FA_941TT | Fa_EA4037 4_921TT | Fa_EA40376_923TT |
|---|---|---|---|---|---|---|---|
| 36 | 329.6 | 165.4 | 331.5 | 298.8 | 267.4 | 211.3 | 188.6 |
| 53 | 35.8 | 28.5 | 21.7 | 291.4 | 45.3 | 23.8 | 62.8 |
| 76 | 253.8 | 64.7 | 3312.7 | 870.1 | 1038.3 | 153.3 | 236.1 |
| 242 | 1630.4 | 2452.4 | 6684 | 765 | 2229.5 | 1809.2 | 3314 |
| 211 | 21639.5 | 31897.6 | 26420.3 | 18786.4 | 31922.5 | 36025.3 | 15065.2 |

| | BN_EA40377_9 24TT | Fa_EA40378_92 5TT | Fa_EA40379_926TT | BN_EA4038 0_927TT | Fa_EA4038 7_955TT | Fa_EA4038 8_956TT | Fa_EA40389_957TT |
|---|---|---|---|---|---|---|---|
| 36 | 1409.1 | 215.1 | 228.5 | 833.3 | 94.7 | 259.7 | 786.2 |
| 53 | 2294.2 | 40.5 | 496.1 | 64.9 | 197.4 | 230.4 | 17.3 |
| 76 | 624.1 | 149.1 | 211.3 | 501.1 | 2225.4 | 1443.7 | 223.7 |
| 242 | 21699.6 | 1079.3 | 21653.5 | 3263.3 | 2194.8 | 989.6 | 2107.8 |
| 211 | 664.1 | 12583.3 | 13036.6 | 43191.4 | 9409.2 | 19630.9 | 4160.9 |

| | Fa_EA40390_9 59TT | Fa_EA40391_96 0TT | Fa_EA40392_961TT | Fa_EA40393_962TT | PC_829TT | PC_830TT | PC_831TT |
|---|---|---|---|---|---|---|---|
| 36 | 2496.1 | 175.3 | 58.2 | 1470.7 | 330.3 | 210.3 | 432.7 |
| 53 | 24.4 | 40.3 | 52.6 | 183 | 73 | 49.6 | 1916.2 |
| 76 | 2397 | 47.6 | 77.2 | 1048.1 | 758.4 | 576.2 | 394.3 |
| 242 | 1991.2 | 1042.5 | 1474.6 | 688.1 | 6760 | 6785 | 25733 |
| 211 | 38927.5 | 31764 | 34287.3 | 44314.7 | 25565 | 31930.4 | 138.4 |

| | PC_832TT | PC_834TT | PC_835TT | PC_836TT | PC_837TT | PC_838TT | PC_839TT |
|---|---|---|---|---|---|---|---|
| 36 | 1376.8 | 4076.8 | 1208.3 | 585 | 1015.5 | 1620.2 | 71 |
| 53 | 1909.1 | 2701 | 1178.5 | 710.7 | 1469.6 | 3009.9 | 41.2 |
| 76 | 433.6 | 665.5 | 654.3 | 2432.3 | 412.1 | 1946.7 | 327.2 |
| 242 | 9423.8 | 18037.6 | 23053.8 | 1044.8 | 22442.7 | 27313.9 | 3641 |
| 211 | 7174.2 | 1961.7 | 282.1 | 24646.9 | 15840.2 | 264.2 | 17049.3 |

(continued)

| | 36 | 53 | 76 | 242 | 211 |
|---|---|---|---|---|---|
| PC_879TT | 457.4 | 2036.8 | 1168 | 46283.3 | 86.8 |
| PC_881TT | 1779.4 | 3121.5 | 1829.9 | 32477.3 | 613.5 |
| PC_882TT | 561.2 | 2072.4 | 1210.6 | 33142.3 | 48.3 |
| PC_883TT | 722.1 | 2753 | 2663.6 | 38026.7 | 126.2 |
| PC_884TT | 1129.2 | 2221.1 | 1076 | 42087.5 | 1565.2 |
| PC_885TT | 552.5 | 1703.8 | 1232.2 | 49249 | 120 |
| PC_886TT | 1244.9 | 1325.9 | 1866.8 | 39172.5 | 1553 |
| PC_890TT | 813.3 | 4685.6 | 3926.2 | 36114.4 | 1569.2 |
| PC_892TT | 2211.4 | 3536.8 | 2787 | 31599.6 | 1703.6 |
| PC_893TT | 1046.2 | 1363.5 | 2359.3 | 34700.3 | 234.5 |
| PC_894TT | 585.3 | 1244.4 | 1020.3 | 41193.8 | 1594 |
| PC_903TT | 3395 | 3057.5 | 1564 | 18485.8 | 4063.1 |
| PC_904TT | 767.9 | 199.9 | 203.6 | 19181.5 | 5354.1 |
| PC_928TT | 246.6 | 41.1 | 421.5 | 3729.4 | 18199.2 |
| PC_932TT | 352.4 | 3447.4 | 850.2 | 21913.7 | 490.5 |
| PC_933TT | 920.3 | 788.3 | 865.6 | 24536 | 13171.6 |
| Pc_EA40375_922TT | 316.8 | 41.8 | 211.2 | 1758.5 | 30959.7 |
| Pc_EA40381_945TT | 818.6 | 803.9 | 237.9 | 13301.5 | 331 |
| Pc_EA40382_946TT | 3078.8 | 2330.1 | 1830 | 22076.9 | 857.8 |
| Pc_EA40383_947TT | 532.1 | 1337.6 | 1279.7 | 31987.1 | 2007.7 |
| Pc_EA40384_948TT | 311.1 | 1556.2 | 598 | 27579.2 | 556.5 |
| FC_823TT | 282.5 | 23.2 | 460.1 | 12129 | 30520.5 |
| FC_824TT | 2413 | 24.1 | 2833.9 | 22000.8 | 496.2 |
| FC_825TT | 1506.3 | 25.5 | 1128.9 | 874.8 | 7923.9 |
| FC_827TT | 821.8 | 348.1 | 1421.6 | 2544.9 | 11508.2 |
| FC_828TT | 564.4 | 27.8 | 705.3 | 845.1 | 33401.7 |
| FC_840TT | 331.6 | 32.3 | 341.8 | 3459.7 | 10882.8 |
| FC_896TT | 857.1 | 245.4 | 358.9 | 1998.1 | 1625.3 |

| | FC_898TT | FC_899TT | FC_900TT | FC_901TT | FC_902TT | FC_909TT | FC_910TT |
|---|---|---|---|---|---|---|---|
| 36 | 827.5 | 1152.4 | 778.8 | 11199.3 | 578.7 | 713 | 132.4 |
| 53 | 38.1 | 1949.7 | 2221 | 29.8 | 185.9 | 21.5 | 57.2 |
| 76 | 2517.5 | 1009.8 | 3523.8 | 28603.2 | 2201.1 | 195.6 | 4744.8 |
| 242 | 39786.8 | 38117 | 29127.6 | 4032.5 | 43673.6 | 3478.5 | 1273.2 |
| 211 | 1160.7 | 132.9 | 79.4 | 26.8 | 251.4 | 488 | 8979.7 |
| | Fc_EA40386_9 54TT | Fc_EA40394_96 7TT | Fc_EA40395_ 968TT | Fc_EA40396 _969TT | Fc_EA4039 7_970TT | Fc_EA4040 5_982TT | Fc_EA40406 _983TT |
| 36 | 619.3 | 412.7 | 134 | 218.6 | 463 | 1233.6 | 234.6 |
| 53 | 15.8 | 31 | 23.8 | 55.9 | 65.5 | 31.6 | 120.8 |
| 76 | 4136.1 | 682.5 | 41.2 | 18.4 | 594.7 | 931 | 2232 |
| 242 | 969.6 | 5003.3 | 13867.1 | 2129.7 | 15742.7 | 4086.9 | 11985.6 |
| 211 | 20581.7 | 1509.8 | 2603.1 | 15427.4 | 369.3 | 30161.2 | 19869.7 |

Table 10. 5-Gene signature performance in 98 training samples

|  | Tumor | Benign |
|---|---|---|
| Positive | 48 | 14 |
| Negative | 4 | 32 |
| Sensitivity | 92% (0.82, 0.97) | |
| Specificity | 70% (0.55, 0.81) | |

D. Cross Validation with the 74 Independent Thyroid Samples

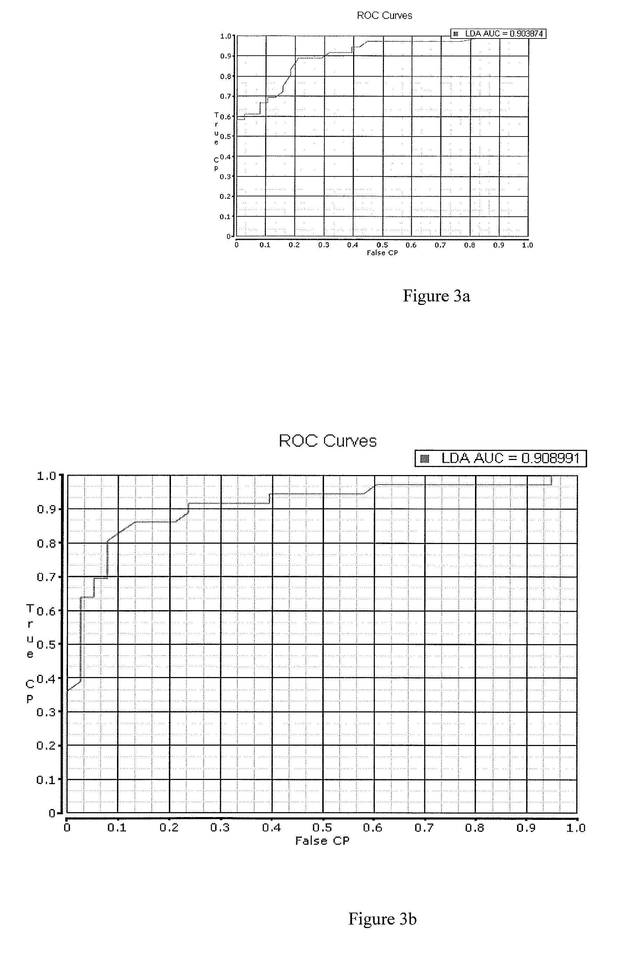

74 independent thyroid samples were processed and profiled with the U133a chip, and the chip data for these two signatures are shown in the Table 11. The performances of the 4-gene and the 5-gene signatures were assessed with LDA. Both signatures gave equivalent performance in these samples compared to the 98 training samples. The sensitivity and specificity for both signatures are shown in Table 12, and the ROC curves are demonstrated in Figure 3a and 3b.

Table 11

| SEQ ID | Signal | | | | | | |
|---|---|---|---|---|---|---|---|
| | FA_987TT | FA_992TT | FA_993TT | FA_994TT | FA_995TT | FA_996TT | FA_998TT |
| 36 | 92.8 | 437.8 | 640.5 | 4152.5 | 714.7 | 254.8 | 303.4 |
| 53 | 15.8 | 422.8 | 25.2 | 29.5 | 258.9 | 45.6 | 26.8 |
| 76 | 485.1 | 2536.5 | 1708.6 | 288.3 | 643.3 | 605.6 | 341 |
| 242 | 686.5 | 971.4 | 24532.8 | 895.7 | 6424.8 | 842.1 | 905.1 |
| 211 | 28269.8 | 14689.4 | 357.5 | 33711.6 | 34943.2 | 16131 | 22376.1 |
| | FA_999TT | FA_1001TT | FA_1002TT | FA_1004TT | FA_1005TT | FA_1006TT | FA_1010TT |
| 36 | 63.7 | 22.3 | 55.6 | 408.5 | 128.3 | 353 | 442 |
| 53 | 30.9 | 31.8 | 39.6 | 70.8 | 37.9 | 38.9 | 119.6 |
| 76 | 110.7 | 90.2 | 253.5 | 159.1 | 1559.6 | 413.7 | 1222.5 |
| 242 | 1550.6 | 1689.3 | 256.5 | 1668 | 3921.5 | 1013.2 | 1837.3 |
| 211 | 26779.6 | 15870.7 | 24525.5 | 43040.2 | 25392.9 | 32641.9 | 20140.7 |
| | FA_1013TT | FA_1014TT | FA_1017TT | FA_1018TT | FA_1020TT | FA_1023TT | FA_1024TT |
| 36 | 453.5 | 333.7 | 179.6 | 146.6 | 417.3 | 75.1 | 129.2 |
| 53 | 39.8 | 21.4 | 99.9 | 23.7 | 26.8 | 62.3 | 22.2 |
| 76 | 333.7 | 1231.2 | 241.1 | 131 | 1435.3 | 1491.6 | 260.8 |
| 242 | 1459.4 | 415 | 1696.3 | 287.1 | 617.5 | 1932.6 | 1689.2 |
| 211 | 7911.3 | 19359.4 | 18089 | 18222.8 | 30038.8 | 9053 | 15611.2 |
| | FA_1026TT | FA_1027TT | FA_1028TT | FA_1029TT | FA_1030TT | FA_1031 TT | FA_1032TT |
| 36 | 281.1 | 130.5 | 150.4 | 629.1 | 760.4 | 191.6 | 1671.5 |
| 53 | 125.5 | 62.3 | 118.2 | 230.7 | 420.5 | 27.7 | 519 |
| 76 | 77.4 | 111.9 | 184 | 2100.9 | 1741.3 | 338.1 | 3643.5 |
| 242 | 1091.6 | 754.3 | 705.9 | 684.7 | 372.4 | 1848.8 | 3094.7 |
| 211 | 21979.1 | 7890.5 | 26226 | 15344.1 | 10368 | 30439.9 | 23943.3 |

| | FA_1034TT | FA_1035TT | FA_1073TT | FA_1074TT | FA_1075TT | FA_1076TT | PC_984TT |
|---|---|---|---|---|---|---|---|
| 36 | 24.8 | 119.9 | 111.7 | 1010.9 | 26 | 224.5 | 303 |
| 53 | 29.6 | 30.4 | 366.7 | 96.9 | 94.3 | 31 | 37.2 |
| 76 | 289.3 | 239.7 | 167.4 | 326.8 | 818.9 | 248.5 | 198.3 |
| 242 | 1482.1 | 590.9 | 4842.5 | 2815.5 | 2726.8 | 754.8 | 785.1 |
| 211 | 38135.4 | 30510.6 | 29406.4 | 31075.4 | 20833.9 | 34960.4 | 24938.2 |
| | PC_986TT | PC_988TT | PC_989TT | PC_1039TT | PC_1040TT | PC_1041TT | PC_1042TT |
| 36 | 676.3 | 391.7 | 264.9 | 735.7 | 524.7 | 769.9 | 1125 |
| 53 | 32.5 | 395.6 | 305.5 | 655 | 1153.9 | 2194.1 | 111.5 |
| 76 | 287.9 | 1384.8 | 888.3 | 1048.4 | 1381.1 | 412.6 | 1384.9 |
| 242 | 2450.7 | 2243.2 | 698.6 | 20366.4 | 32090.9 | 27480.9 | 1915.7 |
| 211 | 2689.5 | 19903.2 | 32075.9 | 9197.7 | 152.2 | 1862.2 | 10375.9 |
| | PC_1043TT | PC_1044TT | PC_1045TT | PC_1046TT | PC_1047TT | PC_1048TT | PC_1049TT |
| 36 | 1013.6 | 1023.8 | 809.8 | 1811.1 | 1546.2 | 481.8 | 2491.9 |
| 53 | 730.7 | 2965.3 | 2533.7 | 2052.8 | 756.1 | 988.3 | 2000.6 |
| 76 | 1977.4 | 3380.4 | 422 | 4343.9 | 3570.2 | 1786.8 | 1619.4 |
| 242 | 3907.5 | 20074.8 | 41761.2 | 33253.5 | 28124.7 | 30516 | 21324.5 |
| 211 | 7015.5 | 11.5 | 136.1 | 59.1 | 3841.7 | 31.1 | 1476 |
| | PC_1050TT | PC_1051TT | PC_1053TT | PC_1054TT | PC_1055TT | PC_1059TT | PC_1060TT |
| 36 | 443.9 | 669 | 477.1 | 952.8 | 645.3 | 660.2 | 648.5 |
| 53 | 4037.8 | 1290.7 | 933.9 | 1336.9 | 2123.2 | 1353.8 | 66.9 |
| 76 | 856.7 | 1039 | 463.2 | 1399.8 | 4385 | 365.8 | 160.7 |
| 242 | 1261.6 | 58258.5 | 26004.9 | 21219.8 | 12760.8 | 26084.4 | 3277.6 |
| 211 | 1150.2 | 375.9 | 73.2 | 2575.8 | 400.5 | 24.2 | 1637.7 |
| | PC_1061TT | PC_1062TT | FC_1037TT | FC_1077TT | FC_1078TT | FC_1079TT | FC_1082TT |
| 36 | 3523.2 | 1912.5 | 234.2 | 128.3 | 825.1 | 297.5 | 1447.6 |

|      | PC_1061TT | PC_1062TT | FC_1037TT | FC_1077TT | FC_1078TT | FC_1079TT | FC_1082TT |
|------|-----------|-----------|-----------|-----------|-----------|-----------|-----------|
| 53   | 2348.3    | 975.7     | 97.6      | 46.2      | 15.8      | 38.5      | 254.6     |
| 76   | 4994.9    | 3891.7    | 5559.8    | 252       | 1103.5    | 700       | 1754.7    |
| 242  | 15397.4   | 2006.1    | 1826      | 2407.5    | 22661.8   | 1595.5    | 713.7     |
| 211  | 3379.4    | 2201.9    | 41570     | 15804.5   | 3572.1    | 4377.9    | 16863.7   |
|      | PC-FV_1052TT | PC-FV_1064TT | PC-FV_1065TT | PC-FV_1066TT | PC-FV_1067TT | PC-FV_1068TT | PC-FV_1069TT |
| 36   | 1993.3    | 230.6     | 1128.8    | 708.4     | 1348      | 331.4     | 33.2      |
| 53   | 2450.3    | 72.7      | 610.3     | 72.5      | 146.4     | 1073.7    | 21.5      |
| 76   | 2075      | 451.3     | 1350.2    | 922.5     | 1743.7    | 950.5     | 332.8     |
| 242  | 21494     | 7110.9    | 10228.8   | 2206.3    | 3319.2    | 28034.2   | 430.1     |
| 211  | 1581.5    | 29936.9   | 1594.7    | 5693.1    | 11288.8   | 573.6     | 12485.5   |
|      | PC-FV_1071TT | PC-FV_1072TT | PC-FV_1080TT | PC-FV_1081TT |           |           |           |
| 36   | 148.9     | 332.2     | 1288.7    | 672       |           |           |           |
| 53   | 1836.9    | 1450.3    | 2221      | 22.9      |           |           |           |
| 76   | 4053      | 1628.6    | 2275.9    | 62.6      |           |           |           |
| 242  | 1810.6    | 26396.9   | 683.4     | 984       |           |           |           |
| 211  | 8.9       | 24        | 3416.7    | 2630.8    |           |           |           |

EP 2 518 166 B1

135

Table 12. 4-Gene and 5-gene signatures performance in 74 validation samples

| 4-Gene Signature | | |
|---|---|---|
| | Tumor | Benign |
| Positive | 33 | 12 |
| Negative | 3 | 26 |
| Sensitivity | 92% (0.78, 0.97) | |
| Specificity | 68% (0.53, 0.81) | |
| 5-Gene Signature | | |
| | Tumor | Benign |
| Positive | 33 | 9 |
| Negative | 3 | 29 |
| Sensitivity | 92% (0.78, 0.97) | |
| Specificity | 76% (0.61, 0.87) | |

E. Control Gene Marker Identification

With the 98 thyroid samples and 12 PBL samples we selected two groups of genes as sampling control. One group consists of genes that are expressed in thyroid but not in PBL, the second group includes genes that are expressed in PBL but not in thyroid. The full gene list and corresponding chip data are shown in Tables 13a and 13b. From these genes we selected six genes that are abundant and the differentiation between thyroid and PBL is relatively large. Their expression profile was validated in the 74 independent thyroid samples. The identities of these six genes are listed in Table 14 and their chip data are shown in Tables 15a and 15b.

Table 13a
13a1

ThyMixBen_......._Signal

| SEQ ID | 02014_40062_H133A_800TT | 02014_40063_H133A_801TT | 02014_40064_H133A_802TT | 02014_40065_H133A_804TT | 02014_40066_H133A_805TT | 02014_40067_H133A_806TT |
|---|---|---|---|---|---|---|
| 2 | 2789.6 | 2676.2 | 3009.2 | 2957.5 | 3644.5 | |
| 3 | 6346.1 | 794.7 | 623.9 | 772 | 2977.3 | 622.7 |
| 5 | 845.5 | 3039.1 | 4452.6 | 2982.5 | 8283.2 | 1627.6 |
| 6 | 2730.4 | 1108.5 | 1155.6 | 2201.2 | 1954.3 | 1750 |
| 9 | 2248.9 | 12094.5 | 7529.2 | 2827.3 | 7701.7 | 1548.3 |
| 12 | 10625.3 | 3137.2 | 2382.7 | 5727.8 | 6238 | 4685.1 |
| 18 | 3171 | 5947.9 | 13241.7 | 10294.9 | 7849.3 | 4037.4 |
| 22 | 2715.2 | 9522.5 | 6429.7 | 2590.9 | 5294.3 | 6490.4 |
| 25 | 19159.3 | 1467.3 | 550.1 | 2838 | 11294.7 | 1374.2 |
| 28 | 722.7 | 835.1 | 727.1 | 568.1 | 1336.7 | 740.7 |
| 32 | 4160.6 | 8115.2 | 4061.8 | 6625.3 | 7348.9 | 3964.1 |
| 39 | 314.3 | 1243.1 | 1486.8 | 742.5 | 1027.7 | 1185.9 |
| 74 | 323.9 | 1227.6 | 1965.1 | 1281.5 | 760.1 | 505.4 |
| 78 | 250.8 | 677 | 1029.5 | 403.7 | 795.2 | 355 |
| 143 | 1337.3 | 1339.1 | 2607.9 | 1758.8 | 1933.5 | 1809 |
| 174 | 1263.1 | 4430.7 | 6041.6 | 3543.2 | 7346 | 3920.5 |
| 175 | 1996.3 | 1829.5 | 3632.7 | 1646.5 | 2847.5 | 3054.2 |
| 191 | 7108.4 | 848.3 | 2063 | 1133.9 | 1482.4 | 1149 |
| 212 | 258.2 | 1405.8 | 1196.9 | 882.4 | 1296.8 | 1790 |
| 222 | 20586.8 | 1371.2 | 2411.9 | 2573.9 | 1228.6 | 1342 |
| 233 | 1410.6 | 14126.8 | 3020.4 | 2186.1 | 8439.4 | 6024.4 |
| 234 | 5048.1 | 2183.3 | 3215.5 | 4103.9 | 4096.9 | 2220.2 |
| 237 | 905 | 3397.9 | 2525.1 | 2135.4 | 2822 | 2492.3 |
| 238 | 198 | 1937.7 | 1289.5 | 804.5 | 1538.6 | 1454.7 |
| 245 | 143.7 | 1598.5 | 1964.2 | 897.9 | 2276.6 | 1348.2 |
| 250 | 1300.9 | 777 | 1405.9 | 1466.4 | 1301.3 | 1865.3 |
| 252 | 945.6 | 1547.3 | 1210.2 | 1357 | 1437.1 | 1141.1 |

| | | | | | | |
|---|---|---|---|---|---|---|
| 264 | 455.1 | 988.1 | 932.8 | 631.7 | 1253.9 | 845.4 |
| 290 | 345.6 | 327.2 | 533.1 | 470.2 | 700.5 | 231.9 |
| 294 | 1043 | 3655.8 | 4435.4 | 3563.9 | 4377.4 | 2734.8 |
| 296 | 845.5 | 1242.5 | 1068.6 | 1506.5 | 2423.4 | 1527.3 |
| 342 | 771.4 | 535.9 | 998.5 | 922.5 | 1108.1 | 605.4 |

13a2

| | | | Signal | | | |
|---|---|---|---|---|---|---|
| SEQ ID | ThyMixBen_020140 2014_40198 _40068_H133A_8 07TT | _H133A_871T T | 02014_40321_ H133A_913TT | 02014_40322_ H133A_914TT | 02014_40323_ H133A_915TT | 02014_40324_H 133A_917TT |
| 2 | 1749.6 | 1571.1 | 2634.4 | 2419.2 | 2697.4 | 2901.1 |
| 3 | 377.2 | 508 | 2322.7 | 259.8 | 1123.4 | 875.2 |
| 5 | 2762.9 | 1795.8 | 6670.2 | 2094.7 | 11425.7 | 4957.2 |
| 6 | 1280.1 | 2033.3 | 1910.3 | 2543.7 | 2173.2 | 1358.7 |
| 9 | 3346.8 | 548.3 | 11398.5 | 953.4 | 2085.2 | 10684.4 |
| 12 | 6927.1 | 4060.5 | 6664.4 | 2730.5 | 4322.8 | 2616.2 |
| 18 | 7418.9 | 14860.4 | 3134.7 | 26576.3 | 9585.4 | 8588.9 |
| 22 | 1797.5 | 744.5 | 7334.3 | 317.9 | 5120.1 | 8611.7 |
| 25 | 1245.8 | 815.8 | 7201.1 | 253.7 | 2882.9 | 1053.4 |
| 28 | 301.1 | 640.8 | 1325.9 | 409.2 | 762.7 | 838.8 |
| 32 | 2992.4 | 3260.2 | 3588.1 | 792.5 | 5413.2 | 2581 |
| 39 | 435.8 | 958.1 | 1152.1 | 499.6 | 556.9 | 898.6 |
| 74 | 689.2 | 1453.7 | 491.7 | 736.6 | 986.9 | 1217 |
| 78 | 160.1 | 383 | 151.2 | 172.9 | 597.4 | 745.6 |
| 143 | 1338.8 | 1466.6 | 1069.9 | 2070.9 | 2377.2 | 1976.3 |
| 174 | 2464.3 | 1616.3 | 829.8 | 1365.2 | 4938 | 4804.3 |
| 175 | 1701 | 1511.5 | 1101.1 | 2325.4 | 2530.2 | 2237.6 |
| 191 | 763.6 | 2383.3 | 2013.2 | 1274.2 | 601.2 | 1100.2 |
| 212 | 481 | 1131.9 | 912.5 | 663.2 | 666.4 | 1081 |
| 222 | 280.8 | 1574 | 4944.1 | 342.2 | 1225.9 | 449 |
| 233 | 1289.5 | 1272.4 | 6285.6 | 866.8 | 1839.3 | 5797.4 |
| 234 | 1645.4 | 3271.1 | 2263.3 | 1789.3 | 6497.4 | 3416 |
| 237 | 1732.6 | 879.6 | 3780 | 1345.3 | 2154.2 | 2802.8 |
| 238 | 493.5 | 893.1 | 1098.7 | 405.9 | 592.8 | 1326.4 |
| 245 | 661.9 | 1378.3 | 1244.1 | 645.4 | 1124.2 | 1171.1 |
| 250 | 736.7 | 1305.6 | 633 | 1409.4 | 1368.9 | 1146.7 |
| 252 | 1145.8 | 1986 | 1203.2 | 1323.5 | 1544.6 | 757 |
| 264 | 462.7 | 891.3 | 629.4 | 649.7 | 872.1 | 1319.5 |
| 290 | 295.8 | 697.1 | 987.8 | 819.9 | 440.6 | 268.7 |
| 294 | 1909.1 | 1128.4 | 924.1 | 1519.2 | 3225.3 | 2954.9 |
| 296 | 898.4 | 1232.2 | 925.1 | 1290.3 | 738.1 | 1069.6 |
| 342 | 482.4 | 712.6 | 476.1 | 694.8 | 801.2 | 605.7 |

13a3

| | | | Signal | | | |
|---|---|---|---|---|---|---|
| SEQ ID | 02014_40325_ H133A_918TT | 02014_40326_ H133A_919TT | ThyFolBen_0201 4_40079_H133A _818TT | ThyFolBen_02014 _40080_H133A_8 19TT | ThyFolBen_02 014_40081_H 133A_820TT | ThyFolBen_020 14_40082_H13 3A_821TT |
| 2 | 3457.7 | 2769.1 | 4234.5 | 5589.2 | 3035.3 | 2681.6 |
| 3 | 920.5 | 1289.3 | 627 | 690.9 | 585.4 | 610 |
| 5 | 1805.2 | 5184.1 | 1487.2 | 1942 | 2663.8 | 1231.8 |
| 6 | 1560.8 | 1519.3 | 996.2 | 1140.3 | 2093.4 | 841.6 |
| 9 | 11354.8 | 9921.9 | 3894 | 2165.4 | 7467 | 7685.6 |

| | | | | | | |
|---|---|---|---|---|---|---|
| 12 | 7531.7 | 6887.2 | 1379.1 | 1292.4 | 5085.1 | 3585.9 |
| 18 | 8220.1 | 5452.2 | 7762.8 | 7277.4 | 5374.9 | 2379.1 |
| 22 | 4750.8 | 5739.1 | 5655.3 | 7618.6 | 6227.9 | 1551.9 |
| 25 | 1557.9 | 3017.5 | 1200.4 | 881.1 | 1279.8 | 406.4 |
| 28 | 886.6 | 1189.2 | 731.4 | 681.6 | 1225.7 | 473.9 |
| 32 | 4134.1 | 10964.4 | 927.6 | 3075.2 | 2775 | 608.8 |
| 39 | 1259.2 | 1022.1 | 1423.7 | 1632.9 | 899.5 | 688.9 |
| 74 | 741.8 | 948.9 | 770 | 674.6 | 1139.7 | 213 |
| 78 | 447.9 | 488.2 | 790.6 | 602.8 | 776.3 | 332.1 |
| 143 | 3165.1 | 1865.5 | 2330.7 | 1160.7 | 1676 | 1672.4 |
| 174 | 607.5 | 2151.8 | 4026.2 | 4702.6 | 10677.1 | 3826.8 |
| 175 | 5358.5 | 2402.7 | 3765.6 | 1231.9 | 2531.9 | 2307.7 |
| 191 | 2685.9 | 1183.6 | 1661.4 | 1657.1 | 1377.8 | 368.2 |
| 212 | 1306.3 | 1072.1 | 2181.8 | 2035.6 | 954.2 | 1663.4 |
| 222 | 2332.8 | 431.2 | 1015.6 | 1651.7 | 3755.4 | 1669.9 |
| 233 | 5181.8 | 12377 | 3400.9 | 4679.3 | 2476 | 1506.6 |
| 234 | 3760.2 | 1988.2 | 3527.4 | 3971.4 | 4526.5 | 997.6 |
| 237 | 2712 | 3073.8 | 1930.6 | 3521.8 | 2951.3 | 2673.5 |
| 238 | 1552.2 | 1636.1 | 1333.5 | 837.6 | 1069.7 | 1860.5 |
| 245 | 1722.2 | 1981.5 | 1978.8 | 2170 | 763.6 | 1392.9 |
| 250 | 1502.7 | 1094 | 1083.3 | 710.8 | 864.9 | 1823.2 |
| 252 | 1609.9 | 1679.3 | 1599.4 | 1463.9 | 1199.9 | 1019.2 |
| 264 | 860.1 | 835.6 | 1451.1 | 1315.6 | 1221.6 | 936.8 |
| 290 | 773.4 | 490.4 | 218.3 | 507.2 | 648.3 | 294.6 |
| 294 | 555.3 | 2490.7 | 1854.9 | 2997.4 | 7108.2 | 2160.7 |
| 296 | 1240.1 | 1863 | 1139.6 | 1245.4 | 1172.5 | 1318.3 |
| 342 | 738.7 | 509.3 | 1018.4 | 728.8 | 977.2 | 710.6 |

13a4

| | | | Signal | | | |
|---|---|---|---|---|---|---|
| SEQ ID | ThyFolBen_02014_40083_H133A_822TT | fa__EA40173_VDX842TT | fa__EA40191_faVDX862TT | fa__EA40192_VDX863TT | fa__EA40193_VDX864TT | fa__EA40194_VDX865TT |
| 2 | 3403.4 | 3423.5 | 2246.6 | 2723.3 | 4130.4 | 2828.3 |
| 3 | 644.4 | 950.1 | 1313.2 | 344.4 | 1731.1 | 2576.9 |
| 5 | 3439.5 | 754.3 | 2119 | 3324.7 | 1663.9 | 4423 |
| 6 | 1373.7 | 987.7 | 834.7 | 1399.9 | 1115.4 | 1872.1 |
| 9 | 7457.5 | 2730.9 | 9307.8 | 7048.5 | 3775.7 | 6931.3 |
| 12 | 3978.9 | 9462.8 | 777.5 | 4280.8 | 4821 | 2268.2 |
| 18 | 6386.9 | 1591.2 | 9546.6 | 4979.5 | 5005.2 | 4409.5 |
| 22 | 2652.5 | 7124.7 | 11054.7 | 4669.9 | 4776.9 | 5782.5 |
| 25 | 1887.7 | 470.8 | 703.9 | 714.5 | 445.9 | 698.2 |
| 28 | 548.5 | 562.4 | 596.8 | 915.9 | 1194.1 | 1041 |
| 32 | 4425.5 | 2120.3 | 1084.1 | 2638.9 | 2593.1 | 1919.9 |
| 39 | 1103.1 | 1110.2 | 898.4 | 1375.1 | 1520.4 | 1744.1 |
| 74 | 285.9 | 895.8 | 532.1 | 841.2 | 1938.6 | 1109.5 |
| 78 | 643.1 | 346.6 | 533.3 | 680.5 | 621.5 | 385.8 |
| 143 | 1979.9 | 3302.9 | 1686.6 | 2406.6 | 2255.4 | 3419.1 |
| 174 | 5408.1 | 608.2 | 5072.8 | 5907.1 | 2956.1 | 2670.2 |
| 175 | 2650.3 | 6596.8 | 2600.8 | 4704.8 | 3123 | 5286.8 |
| 191 | 667.7 | 2665.7 | 1382.1 | 740.2 | 1133.3 | 3678.2 |
| 212 | 1357.8 | 2545.2 | 1590.2 | 1326.3 | 1135.1 | 1079 |
| 222 | 2239.9 | 471 | 548.8 | 584.1 | 899.4 | 1002.2 |
| 233 | 7318.7 | 11617.9 | 10868.4 | 6925.9 | 5416.9 | 14212.9 |
| 234 | 2395.9 | 2832.7 | 6005.3 | 2493.8 | 2128.8 | 6818.7 |

| 237 | 2275.3 | 2639.3 | 2406.6 | 2595.1 | 1857.2 | 2546.1 |
| 238 | 1181.8 | 2124.4 | 1586.7 | 2125.3 | 2039.5 | 1859.1 |
| 245 | 2082.5 | 2302.1 | 2846.4 | 2280.8 | 3973.8 | 1520.6 |
| 250 | 1684 | 1718 | 862.9 | 2782.6 | 1973.4 | 1594.3 |
| 252 | 1166.7 | 1579.6 | 590.4 | 1623.2 | 1735.2 | 1584.3 |
| 264 | 654.2 | 1604.2 | 3062.9 | 1460.8 | 1510.1 | 2018.9 |
| 290 | 342 | 431.8 | 458.8 | 602.3 | 745 | 977.6 |
| 294 | 2978.3 | 651.5 | 3533.7 | 4065.9 | 2720.4 | 2292.8 |
| 296 | 2132.4 | 1762.9 | 815.3 | 1040.3 | 1787.6 | 932.6 |
| 342 | 1058.5 | 488.3 | 475.4 | 759.3 | 658.3 | 851.2 |

13a5

| SEQ ID | | | Signal | | | |
|---|---|---|---|---|---|---|
| | fa__EA40195_VDX866TT | fa__EA40196_VDX867TT | fa__EA40197_VDX869TT | fa__EA40219_VDX907TT | fa__EA40220_VDX908TT | 02014_40327_H133A_920TT |
| 2 | 3243.6 | 2969.9 | 3761.4 | 3352.1 | 2486.7 | 3109.7 |
| 3 | 728.9 | 548.2 | 531.1 | 796 | 1204.6 | 981.6 |
| 5 | 2392.7 | 6268.7 | 3870.9 | 10216 | 3635.2 | 11319.3 |
| 6 | 1534 | 965.4 | 2782.8 | 2638.6 | 1155.7 | 2100.9 |
| 9 | 8238.3 | 8829.9 | 4175.6 | 1650.4 | 7363.2 | 3172 |
| 12 | 3334.6 | 2752 | 3382.5 | 3589.1 | 6717.7 | 3191.1 |
| 18 | 9146.3 | 8150.5 | 7796.3 | 8021.5 | 8185 | 8907.1 |
| 22 | 4665.5 | 8163.5 | 3812 | 4001.7 | 5539.5 | 6346.7 |
| 25 | 555.6 | 524.6 | 1592.8 | 2057.7 | 4103.3 | 886.1 |
| 28 | 1115.3 | 604 | 918.9 | 1190.9 | 999.1 | 998.2 |
| 32 | 1089.3 | 1808.7 | 1245.6 | 4202 | 7454.6 | 1100.2 |
| 39 | 1606.8 | 1418.2 | 1392.4 | 1443.3 | 949.8 | 1388.7 |
| 74 | 638.2 | 2085.2 | 694.7 | 1778.1 | 557.7 | 1486.9 |
| 78 | 480.6 | 603.6 | 959.8 | 1000.3 | 476 | 721.1 |
| 143 | 3003 | 2033.2 | 3441.7 | 3224.1 | 2461.3 | 2701.2 |
| 174 | 3322 | 4573.5 | 4168.2 | 5982 | 3022.2 | 5710.7 |
| 175 | 4747.7 | 2573.2 | 5834.5 | 4666.3 | 3540.8 | 3751 |
| 191 | 2724.1 | 726 | 831.2 | 1991.3 | 1556.8 | 643.4 |
| 212 | 2362.7 | 1705.9 | 2344.8 | 1259.8 | 1288.5 | 1033.6 |
| 222 | 271.7 | 2039.4 | 683.4 | 1370.8 | 728.8 | 1675.2 |
| 233 | 10892.9 | 6981.9 | 10048.5 | 6773.9 | 15803 | 3520.9 |
| 234 | 4015.4 | 3229.5 | 4734.4 | 7233.3 | 2712.8 | 8148.3 |
| 237 | 2159.9 | 2415.2 | 2194.8 | 2990.7 | 2565.8 | 2364 |
| 238 | 1451 | 1111.6 | 1037.4 | 914.9 | 1634.9 | 1078.9 |
| 245 | 2059.1 | 2548.6 | 2180.9 | 2093.2 | 1730 | 1329.4 |
| 250 | 3534.3 | 993.3 | 2513.3 | 878.9 | 686.3 | 980.4 |
| 252 | 1589.8 | 1334.1 | 2762.4 | 1267.3 | 1857.6 | 1608.2 |
| 264 | 1335.1 | 1560.1 | 1656.9 | 942.7 | 950.4 | 1076.1 |
| 290 | 496.3 | 626.2 | 584.9 | 549.5 | 309.7 | 613.4 |
| 294 | 2869.4 | 4842.4 | 2762.9 | 4119.2 | 2299.8 | 3497.8 |
| 296 | 1414.4 | 882.4 | 1284 | 1106.4 | 1493.4 | 888.5 |
| 342 | 595.6 | 660.6 | 1442.5 | 1050.3 | 607.5 | 1278.6 |

13a6

| SEQ ID | | | Signal | | | |
|---|---|---|---|---|---|---|
| | 02014_40332_H133A_938TT | 02014_40334_H133A_941TT | 02014_40335_H133A_940TT | EA02014_40374_H133A_921TT | EA02014_40376_H133A_923TT | EA02014_40377_H133A_924TT |
| 2 | 1963.7 | 2662.2 | 2416.5 | 3047.7 | 2043.6 | 3322 |
| 3 | 942.5 | 342 | 749.1 | 1300.6 | 549.3 | 1182 |

| | | | | | | |
|---|---|---|---|---|---|---|
| 5 | 2218 | 3903.1 | 3011.1 | 1669.8 | 1508.3 | 1453.3 |
| 6 | 939.5 | 1239.9 | 1853.8 | 837.7 | 1477.8 | 1129.4 |
| 9 | 5245.3 | 1988.9 | 1165.8 | 4137 | 8735.7 | 4453.3 |
| 12 | 2500.4 | 2100 | 3597.7 | 5716.2 | 6694.6 | 3667.8 |
| 18 | 5929.7 | 7819.7 | 5335.4 | 4017.5 | 2899.7 | 5013.3 |
| 22 | 2265.8 | 6298.2 | 2112.7 | 3386.6 | 4519.5 | 4186.6 |
| 25 | 1160.2 | 916.5 | 614.7 | 1055.5 | 1184.4 | 828.4 |
| 28 | 617.6 | 408.5 | 740.7 | 477.3 | 701.3 | 550.8 |
| 32 | 2918.8 | 3242.3 | 3083.9 | 1411.4 | 4079.5 | 2063.5 |
| 39 | 1356.6 | 876.8 | 1855 | 1231.3 | 982.1 | 1248 |
| 74 | 1102.1 | 826.7 | 1384.4 | 296.9 | 632.9 | 787.7 |
| 78 | 361.5 | 383.5 | 482.7 | 578.5 | 351.8 | 389.9 |
| 143 | 1394.9 | 2889.2 | 1838.1 | 1776 | 1563 | 3881.9 |
| 174 | 2198.3 | 1887.5 | 3921.8 | 3076.4 | 1858.1 | 1967.3 |
| 175 | 1391.4 | 3677.8 | 2169.3 | 2169 | 2397.2 | 5896.8 |
| 191 | 1235.1 | 364.7 | 1283.5 | 616.4 | 1037.7 | 1957 |
| 212 | 1625.4 | 871.1 | 1083.8 | 1086.2 | 1450.7 | 981.1 |
| 222 | 503.4 | 638.5 | 320.1 | 1707.8 | 1089.4 | 640.8 |
| 233 | 8499.4 | 1999.9 | 4012.1 | 3345.8 | 10991.6 | 11095.5 |
| 234 | 2067.7 | 2973.7 | 2630.4 | 2027.1 | 1597 | 3240 |
| 237 | 2763.1 | 1501.1 | 2724 | 1997.9 | 2249.3 | 2941.1 |
| 238 | 1055.6 | 1055.7 | 1596.5 | 1035.6 | 1810.9 | 1257.5 |
| 245 | 751.4 | 1076.1 | 915.2 | 2714.4 | 2120.1 | 1553.6 |
| 250 | 1050.6 | 1474.3 | 2517.1 | 1428.1 | 1169.4 | 2857.1 |
| 252 | 987.8 | 1382.5 | 2070.7 | 881.9 | 1589.3 | 2217.2 |
| 264 | 1278.3 | 1261.1 | 826.2 | 865.3 | 1055.9 | 1034.7 |
| 290 | 786.4 | 936.8 | 952.1 | 312.4 | 385.7 | 1231.1 |
| 294 | 2197.5 | 2064.7 | 2836.2 | 2573.5 | 1835.4 | 2657.6 |
| 296 | 635.2 | 572.8 | 700.9 | 1768.2 | 1426.3 | 2462.5 |
| 342 | 718.6 | 866.8 | 735.7 | 770.7 | 565.3 | 1695.1 |

13a7

| SEQ ID | 40378_H133A_925TT | 40379_H133A_926TT | 40380_H133A_927TT | EA02014_40387_H133A_955TT | Signal 40388_H133A_956TT | 40389_H133A_957TT |
|---|---|---|---|---|---|---|
| 2 | 3274 | 3025.4 | 2424.9 | 2302.6 | 2724.6 | 2641.3 |
| 3 | 1545.1 | 1151.1 | 687.6 | 424.7 | 420.6 | 609.2 |
| 5 | 3661.8 | 1323.2 | 7949.9 | 4860 | 4059.3 | 4895.3 |
| 6 | 1371.5 | 1220.8 | 1831 | 2330.9 | 1642.8 | 1963.8 |
| 9 | 10387.7 | 11670.1 | 6402.7 | 3105.3 | 4256.6 | 8744.8 |
| 12 | 5829.9 | 6903.8 | 4783.9 | 2874.5 | 3494.1 | 2451 |
| 18 | 6648 | 4053.1 | 7410.2 | 4600.2 | 3912.9 | 16235.3 |
| 22 | 6859.7 | 6621.2 | 3008.1 | 1369.9 | 2480.5 | 4685 |
| 25 | 1035.6 | 3448.2 | 1347.1 | 365.3 | 1587.2 | 816.8 |
| 28 | 1087 | 917.1 | 747.8 | 1057 | 1169.8 | 974.1 |
| 32 | 5212.9 | 6841.6 | 3282.5 | 2814.2 | 4083.1 | 8121.2 |
| 39 | 1228.7 | 1245.7 | 762.4 | 904.9 | 1136.2 | 1862.7 |
| 74 | 823.9 | 721.6 | 1460.4 | 2781.4 | 970.2 | 839 |
| 78 | 713.8 | 377.6 | 621.3 | 212.8 | 421.3 | 891.4 |
| 143 | 1953.5 | 2925.7 | 2861.8 | 1243.3 | 2335.1 | 2564.3 |
| 174 | 3029 | 1818.4 | 5264.1 | 3925.2 | 4789.1 | 4553.5 |
| 175 | 3229.1 | 4827.2 | 3821.1 | 1561.7 | 4603.8 | 4098.3 |
| 191 | 1112.4 | 5308.2 | 841 | 861.6 | 865.2 | 1964.9 |
| 212 | 890.9 | 915.6 | 729.1 | 1264.1 | 1494.2 | 1177.2 |

| | | | | | | |
|---|---|---|---|---|---|---|
| 222 | 480.1 | 2895.3 | 605.6 | 145.2 | 425.3 | 1712.4 |
| 233 | 6680.7 | 9190.5 | 2236.9 | 441.5 | 3626.6 | 11326.8 |
| 234 | 1871.2 | 4194.1 | 4428.8 | 1260.8 | 1599 | 4627.7 |
| 237 | 2758.4 | 2108.7 | 2070.1 | 1655.9 | 2140.3 | 2820.6 |
| 238 | 1210.7 | 1289.5 | 774.3 | 1017.9 | 1645.6 | 1150.6 |
| 245 | 2369.7 | 1585.8 | 785.1 | 1808.4 | 2014.9 | 1185.6 |
| 250 | 971.8 | 1387.3 | 1193.5 | 2014.8 | 2033 | 608.6 |
| 252 | 1417.1 | 990.9 | 1186.9 | 1561.4 | 2006.1 | 1838 |
| 264 | 1089.9 | 538.3 | 758.6 | 1340.4 | 1171.9 | 933.1 |
| 290 | 565.1 | 601.8 | 485.3 | 597.5 | 511.7 | 852.7 |
| 294 | 2574.6 | 1541.3 | 3862.3 | 2639.9 | 3968.8 | 3955.7 |
| 296 | 1821.8 | 1115.3 | 1015.1 | 1379.4 | 1444.1 | 867.3 |
| 342 | 621 | 757.4 | 791.6 | 504.5 | 671.6 | 1320.3 |

13a8

| | Signal | | | | | |
|---|---|---|---|---|---|---|
| SEQ ID | EA02014_40390_H133A_959TT | EA02014_40391_H133A_960TT | EA02014_40392_H133A_961TT | EA02014_40393_H133A_962TT | ThyPapCan_02014_40090_H133A_829TT | ThyPapCan_02014_40091_H133A_830TT@IC |
| 2 | 1817.9 | 1937.6 | 2632.3 | 2613.7 | 2167.7 | 1372.7 |
| 3 | 605.6 | 546.8 | 697.5 | 480.4 | 1793.5 | 1236.1 |
| 5 | 8452.7 | 11532.9 | 5852.5 | 5194.8 | 3735.8 | 2822.7 |
| 6 | 2508.7 | 1035.1 | 1581.2 | 1561.5 | 1568.4 | 1723.3 |
| 9 | 1557.3 | 17159.3 | 4950.2 | 1272.3 | 3597.7 | 3243.8 |
| 12 | 8423.6 | 3364.4 | 3626.3 | 1198.7 | 5938.4 | 3878.6 |
| 18 | 9560.7 | 5478.7 | 8997.6 | 4601.2 | 6673.3 | 6322.9 |
| 22 | 5745.2 | 8584.6 | 5849.4 | 2275.2 | 4413.3 | 2539.9 |
| 25 | 2003.7 | 619.6 | 797.7 | 887.6 | 2860.8 | 3257.2 |
| 28 | 1280.7 | 1023.3 | 810.5 | 604.5 | 703.2 | 592.2 |
| 32 | 5819.5 | 1430 | 6760.1 | 736.3 | 5057.7 | 6877.1 |
| 39 | 844.6 | 1193.7 | 2035 | 1643.1 | 906.4 | 977.6 |
| 74 | 1794.4 | 871.9 | 1342.7 | 1207.3 | 873.7 | 438.1 |
| 78 | 1290.5 | 705.2 | 582.1 | 644.6 | 514.2 | 463 |
| 143 | 3606.5 | 1206.5 | 2610 | 3369.8 | 2462.6 | 1861.9 |
| 174 | 5367.9 | 3197 | 2890.4 | 2641.3 | 4481.7 | 4198.6 |
| 175 | 6112 | 1232.1 | 3258.2 | 5085.3 | 3246.9 | 2442.8 |
| 191 | 628.6 | 681.7 | 1024.6 | 538.5 | 913.3 | 723.7 |
| 212 | 762 | 850.9 | 1201 | 690.9 | 797.9 | 784 |
| 222 | 3380.6 | 2257.2 | 1397.9 | 127.7 | 892.4 | 646.7 |
| 233 | 1273.2 | 3459.2 | 5807.2 | 1846.1 | 4170.6 | 4565.3 |
| 234 | 4522.8 | 4556.7 | 4306.9 | 2687.8 | 3024.6 | 3006.1 |
| 237 | 1813.8 | 2740.9 | 2387.6 | 1999.7 | 1879.2 | 1708 |
| 238 | 1451 | 897.7 | 1411.1 | 1416.3 | 966.6 | 803.6 |
| 245 | 841.7 | 1542 | 1434.2 | 1628.4 | 1415.8 | 1521.5 |
| 250 | 834.9 | 839.6 | 1111.7 | 2545.8 | 1268.4 | 993.6 |
| 252 | 1612 | 1068.9 | 1122.2 | 1770.9 | 1379.1 | 1201.1 |
| 264 | 1720.7 | 1090.3 | 766.9 | 838.8 | 728.4 | 1170.1 |
| 290 | 548.7 | 268.4 | 292.1 | 366.4 | 284.9 | 221.6 |
| 294 | 4894.2 | 2878.6 | 1768.4 | 2181.6 | 2628.2 | 1888.8 |
| 296 | 1074.9 | 850.9 | 729.8 | 975.7 | 1061.8 | 946.4 |
| 342 | 1199.4 | 802.1 | 874.5 | 1428.7 | 842.7 | 661 |

13a9

ThyPapCan_02014_....Signal

| SEQ ID | 40092_H133A_831TT | 40093_H133A_832TT | 40095_H133A_834TT | 40096_H133A_835TT | 40097_H133A_836TT | 40098_H133A_837TT@I2 |
|---|---|---|---|---|---|---|
| 2 | 2960.8 | 3724.8 | 3502.5 | 2185.5 | 2399.1 | 2900.3 |
| 3 | 1034.7 | 1002.2 | 539.6 | 891.3 | 452.9 | 1885.7 |
| 5 | 1648.6 | 2929.1 | 3651.5 | 1611.8 | 3731.1 | 3572.5 |
| 6 | 1182.8 | 1644.7 | 1603.7 | 966.3 | 2282.6 | 1818.1 |
| 9 | 1637.6 | 5720.9 | 3045.3 | 1451 | 5197.6 | 7768.2 |
| 12 | 4300.1 | 3654.1 | 3555.9 | 3946.6 | 2299 | 2877.5 |
| 18 | 6335.8 | 3922.6 | 3579.6 | 3282.2 | 3591.8 | 9567 |
| 22 | 3973 | 4533 | 5478.5 | 2546.7 | 2145.4 | 7779.3 |
| 25 | 7110.3 | 2680 | 2117.2 | 5212.6 | 869.3 | 8325.2 |
| 28 | 550.3 | 1205.7 | 660.4 | 730.8 | 642.1 | 734.4 |
| 32 | 3476.1 | 8075.3 | 2580 | 2524.9 | 2454.3 | 7864.8 |
| 39 | 973.9 | 1322.9 | 1283.6 | 847.1 | 1328.7 | 1793.3 |
| 74 | 611.8 | 1044.9 | 1510.9 | 831.5 | 519.6 | 513.4 |
| 78 | 179.8 | 404.5 | 344.8 | 133.7 | 582.7 | 482.5 |
| 143 | 2333.5 | 2803.8 | 3730.7 | 2073.1 | 2033.9 | 3437.3 |
| 174 | 2628.9 | 5254.6 | 2333.9 | 922.6 | 5487.5 | 2544 |
| 175 | 3237.9 | 5586.3 | 5010.7 | 2460.9 | 2800.9 | 4480.6 |
| 191 | 3519.9 | 1826.9 | 1761.9 | 1360.1 | 531.4 | 2426.7 |
| 212 | 957 | 1085.3 | 1218.4 | 764.9 | 1307.9 | 1161.5 |
| 222 | 3373.3 | 1016 | 723.7 | 3010.1 | 829.7 | 1642.5 |
| 233 | 11130.3 | 16096.3 | 16100.3 | 10534.6 | 2873.8 | 13024.1 |
| 234 | 2382.4 | 2253.4 | 2236.6 | 842.4 | 1767 | 3577 |
| 237 | 2141.4 | 2712.7 | 2749.7 | 2120.8 | 2269.8 | 2385.2 |
| 238 | 719.8 | 2489.2 | 1179.1 | 806.1 | 1516.4 | 1336.1 |
| 245 | 873.3 | 1325.6 | 1693 | 870.8 | 1916.1 | 1869.5 |
| 250 | 2074.6 | 1295 | 2234.8 | 1654.7 | 2588.9 | 1104.7 |
| 252 | 1496.7 | 2444.6 | 2031.7 | 1605.8 | 1808.1 | 1897.2 |
| 264 | 931.2 | 1035.7 | 1188 | 746.7 | 1074.1 | 1170.2 |
| 290 | 499.8 | 667.3 | 663.2 | 626.9 | 634.4 | 625.8 |
| 294 | 2010 | 4135.1 | 2098.4 | 1207.1 | 3722.1 | 2055.4 |
| 296 | 2128.8 | 2051.6 | 2507.5 | 2156.5 | 1001 | 2034.3 |
| 342 | 835.3 | 1453.2 | 1071.6 | 615.5 | 1084 | 1209 |

13a10

| SEQ ID | ThyPapCan_02014_40099_H133A_838TT@I2 | 02014_40317_H133A_839TT | Signal pc__EA40200_VDX879TT | pc__EA40201_VDX881TT | pc__EA40202_VDX882TT | pc__EA40203_VDX883TT |
|---|---|---|---|---|---|---|
| 2 | 3540.3 | 1657 | 3331 | 3253.1 | 3349.3 | 3642 |
| 3 | 1796.3 | 750.6 | 1334.9 | 1248.1 | 477.8 | 697.9 |
| 5 | 4954 | 2602 | 3011.2 | 2106.6 | 1504.9 | 2070.9 |
| 6 | 2558.4 | 1246.9 | 1008.5 | 1708.8 | 874.6 | 704.3 |
| 9 | 4538 | 3328.9 | 1097.2 | 1789.6 | 857.2 | 1327.3 |
| 12 | 2544 | 6420.6 | 5055.9 | 4641.7 | 3423.2 | 2283 |
| 18 | 9287.1 | 5085 | 6074.5 | 5157.6 | 7305.1 | 5996.2 |
| 22 | 5415.4 | 3850 | 3537 | 3100.8 | 2588.4 | 2125.9 |
| 25 | 12197.9 | 1296 | 3368.6 | 4941.8 | 1669.9 | 1166.3 |
| 28 | 915.5 | 631 | 842.7 | 869.9 | 606.5 | 459.6 |
| 32 | 4804.9 | 3883.4 | 3039.3 | 5722.3 | 3401.7 | 1964.8 |
| 39 | 1409.2 | 756 | 1855 | 1356 | 1421 | 1878.4 |
| 74 | 992.8 | 349.3 | 597.9 | 708.5 | 741.6 | 421.6 |
| 78 | 352 | 259.9 | 342.4 | 434.8 | 172.4 | 207.2 |
| 143 | 4199.6 | 1314.8 | 2573.1 | 5070.9 | 4302.9 | 3455.3 |

| | | | | | | |
|---|---|---|---|---|---|---|
| 174 | 3924.5 | 1399.1 | 5622.6 | 4217.6 | 1231.4 | 1538.6 |
| 175 | 6697.7 | 1590 | 4211.2 | 7931.3 | 7786.3 | 5294.6 |
| 191 | 3745.9 | 816.2 | 4875.5 | 3853.9 | 2370.5 | 1807.8 |
| 212 | 1219.1 | 1064.8 | 1480.3 | 1571.7 | 1350.2 | 874.7 |
| 222 | 8593.9 | 434.6 | 1994.5 | 2235.1 | 980.8 | 748.4 |
| 233 | 20899.6 | 4657 | 37907.7 | 22959.2 | 12092.7 | 30504.2 |
| 234 | 3763.9 | 1496.4 | 3667.8 | 2431.2 | 1077.6 | 1447.8 |
| 237 | 2711.1 | 2046.9 | 1892.2 | 2919 | 2374.3 | 2011.2 |
| 238 | 994.1 | 1047.3 | 1092.4 | 929.6 | 877.7 | 776.8 |
| 245 | 1339.1 | 1019.4 | 2826.7 | 1538.1 | 1379.8 | 1254.9 |
| 250 | 1736 | 710.8 | 2570.5 | 1597.7 | 1899 | 1923.8 |
| 252 | 2933.1 | 1316.3 | 1849.2 | 2395.5 | 2621 | 1992.7 |
| 264 | 1426.6 | 987 | 2826.4 | 1272.7 | 2511.5 | 1495.4 |
| 290 | 666.3 | 368.1 | 487 | 665.4 | 692.1 | 706 |
| 294 | 3175.8 | 1237.3 | 4622.7 | 3449.7 | 976.8 | 1074.7 |
| 296 | 1837.4 | 899.1 | 2639 | 2217.5 | 2398.4 | 2049.3 |
| 342 | 1355.2 | 406.1 | 1662.9 | 803.3 | 1501.3 | 863 |

13a11

| SEQ ID | | Signal | | | | |
|---|---|---|---|---|---|---|
| | pc__EA40204_VDX884TT | pc__EA40205_VDX885TT | pc__EA40206_VDX886TT | pc__EA40207_VDX890TT | pc__EA40208_VDX892TT | pc__EA40209_VDX893TT |
| 2 | 2895.1 | 2994.7 | 3397.6 | 4328.1 | 4435.3 | 5980.3 |
| 3 | 1144 | 932.3 | 1277.6 | 3294.1 | 1361.2 | 475.7 |
| 5 | 1096.3 | 1151.4 | 4943.4 | 1741.8 | 3416.4 | 653.5 |
| 6 | 916.1 | 945 | 1696.2 | 1593.6 | 1278.6 | 740.7 |
| 9 | 2396.9 | 3699.9 | 1671.3 | 1488.6 | 2591.9 | 1644.7 |
| 12 | 3762.2 | 3308.4 | 3095.3 | 2865.4 | 5996.8 | 1644.2 |
| 18 | 9468.3 | 6669.2 | 10475.1 | 18044.8 | 9220.2 | 11224.4 |
| 22 | 3823.4 | 2813.4 | 4326.9 | 7811.1 | 7837 | 1650.1 |
| 25 | 1322.8 | 496 | 4227.5 | 4437.5 | 1271.9 | 878.1 |
| 28 | 563.6 | 333.7 | 602.3 | 782.9 | 724.2 | 636.5 |
| 32 | 4985.4 | 854.7 | 1788.8 | 2423.6 | 2099.4 | 629.4 |
| 39 | 963 | 1562 | 1937.7 | 1973.7 | 1774.1 | 1387.6 |
| 74 | 417 | 443.9 | 383.6 | 600.6 | 814.2 | 578.6 |
| 78 | 169.9 | 266.2 | 346.4 | 207.7 | 507.4 | 117.7 |
| 143 | 2357.9 | 2936.4 | 2961 | 6472.6 | 4513.4 | 3395.7 |
| 174 | 1096.5 | 2227.9 | 1825 | 1268.5 | 5715 | 380.6 |
| 175 | 3559.1 | 4964.6 | 5395 | 12711.3 | 7961.8 | 5835.7 |
| 191 | 5320.2 | 8526.6 | 4668.1 | 4129.5 | 8005 | 4597.4 |
| 212 | 1168.2 | 1634.1 | 1396.4 | 1197.9 | 1161.5 | 1523.4 |
| 222 | 805.9 | 739.2 | 2330.2 | 3890.4 | 873.8 | 1137.4 |
| 233 | 25920.6 | 37919.3 | 33438 | 7762.1 | 23325.9 | 20940.7 |
| 234 | 1882.8 | 1513.3 | 2438.5 | 4528.9 | 2232.7 | 1253.9 |
| 237 | 2950.6 | 1089.9 | 1886.5 | 2930.8 | 2484.4 | 2082.4 |
| 238 | 1037.6 | 1240 | 818.8 | 709.9 | 1096.2 | 877.7 |
| 245 | 1151.5 | 1872.7 | 2007.7 | 1316.8 | 2042.3 | 1717.4 |
| 250 | 3324 | 2153.4 | 2363.7 | 1885.4 | 2787.9 | 3192.2 |
| 252 | 1641 | 1236.4 | 1558.6 | 2605.7 | 1944.3 | 2613.4 |
| 264 | 1120.1 | 2994.2 | 1776.5 | 1401.3 | 1517.1 | 2362.8 |
| 290 | 624.5 | 516.4 | 692.1 | 520.9 | 778.6 | 1209.7 |
| 294 | 1273.1 | 3050.2 | 1557 | 1442.8 | 3564.9 | 424.1 |
| 296 | 2601.3 | 2023.6 | 2244.2 | 2054.1 | 2777.3 | 4347 |
| 342 | 723.2 | 973.5 | 587 | 2028.3 | 679.8 | 746.2 |

13a12

| SEQ ID | pc__EA40210_VDX894TT | EA02014_40319_H133A_903TT | Signal 02014_40320_H133A_904TT | 02014_40328_H133A_928TT | 02014_40330_H133A_932TT | 02014_40331_H133A_933TT |
|---|---|---|---|---|---|---|
| 2 | 4563.8 | 3729.2 | 2859.9 | 2475.3 | 3977.2 | 3386.3 |
| 3 | 475.3 | 827.4 | 4626.7 | 246.3 | 2009.2 | 1481.9 |
| 5 | 1717.5 | 3130.1 | 1505.9 | 1737.4 | 1875.4 | 4763.8 |
| 6 | 698.9 | 1153.2 | 2668.3 | 1104 | 1295.6 | 1385.4 |
| 9 | 3017.4 | 3184.7 | 6316.2 | 1911.3 | 3801.9 | 2397.6 |
| 12 | 1662.3 | 4850 | 7880.2 | 3035 | 5874.2 | 3637.2 |
| 18 | 7186 | 6997.2 | 14099 | 11077.7 | 9724.6 | 10100.8 |
| 22 | 1195.2 | 3583.4 | 6058.2 | 4793.7 | 6322.5 | 4421.3 |
| 25 | 587.7 | 868.9 | 28372.6 | 667.2 | 3526 | 4180.2 |
| 28 | 728.8 | 621.5 | 1685.2 | 927.3 | 646.4 | 882.8 |
| 32 | 1503.2 | 2517.7 | 19668.7 | 3607.7 | 1986.9 | 6399.2 |
| 39 | 1609.6 | 1035.8 | 1140.8 | 1205 | 1028.3 | 1359.6 |
| 74 | 240.3 | 718.9 | 623.5 | 583.2 | 953 | 1021.1 |
| 78 | 196.7 | 180.1 | 370.1 | 543 | 465.1 | 373.7 |
| 143 | 1838.3 | 3062.8 | 1513.2 | 2903.2 | 3977.3 | 3056.1 |
| 174 | 1548.4 | 1810.3 | 1087.6 | 2910.3 | 3511.6 | 3939.4 |
| 175 | 2462.7 | 5545.8 | 1615.4 | 4518.2 | 7011.2 | 5184.6 |
| 191 | 3292.3 | 2497.1 | 3479.2 | 1085 | 5805.4 | 2286.3 |
| 212 | 1289.5 | 1230.1 | 645.1 | 1795.6 | 639 | 1130.8 |
| 222 | 502.2 | 315.3 | 8484.1 | 1685.8 | 4996.9 | 611.8 |
| 233 | 40786.7 | 16562 | 17697.8 | 3636.9 | 19787.1 | 4648.9 |
| 234 | 1248.4 | 1443.8 | 3706.7 | 2565.5 | 2733.9 | 2463.9 |
| 237 | 1286.1 | 2852.7 | 2832.7 | 2610.7 | 2178.6 | 2681.6 |
| 238 | 1382.7 | 1243.2 | 990.6 | 1636.4 | 1423.2 | 1469.2 |
| 245 | 2174 | 1000.7 | 703.1 | 2029.7 | 1000.1 | 1302.2 |
| 250 | 2738.6 | 2792.3 | 1076.8 | 1439.3 | 3692.8 | 2113.9 |
| 252 | 1078.4 | 1615.7 | 2091.1 | 2366.6 | 1895.9 | 2343.2 |
| 264 | 2550.8 | 948.7 | 659.3 | 1351 | 811.7 | 1104.5 |
| 290 | 518.9 | 745.9 | 1057.1 | 320.1 | 924.2 | 1016.3 |
| 294 | 1936.2 | 1348.3 | 757.5 | 2287.3 | 2252.8 | 2351.2 |
| 296 | 2194.3 | 1614.6 | 1446.4 | 729.6 | 2955.6 | 1690.2 |
| 342 | 612.9 | 748.4 | 366.7 | 1061 | 1424.5 | 894.4 |

13a13

| SEQ ID | EA02014_40375_H133A_922TT | EA02014_40381_H133A_945TT | EA02014_40382_H133A_946TT | Signal EA02014_40383_H133A_947TT | EA02014_40384_H133A_948T T | ThyFolCan_02014_40084_H133A_823TT |
|---|---|---|---|---|---|---|
| 2 | 1365.7 | 2250.1 | 3658.1 | 2299 | 3252.8 | 3923.8 |
| 3 | 935.6 | 1124.4 | 1023.9 | 2053.6 | 1187.9 | 1004.7 |
| 5 | 7421.5 | 656.7 | 3000.3 | 4151.2 | 1730.9 | 3450.3 |
| 6 | 1026.6 | 982.6 | 1628.3 | 1939.7 | 1023.9 | 1961.6 |
| 9 | 10992.7 | 1452.2 | 2478.2 | 4581.5 | 1567.6 | 3548.1 |
| 12 | 3007 | 2413.7 | 5402.5 | 7248.9 | 4382.6 | 6659.3 |
| 18 | 4106.5 | 1506.8 | 9107.3 | 6629.5 | 9741.9 | 7072.7 |
| 22 | 4766.3 | 5493.6 | 3034.7 | 4839.3 | 4984.8 | 4552 |
| 25 | 900.4 | 10351.1 | 1252.1 | 10112.4 | 3841.3 | 7385.3 |
| 28 | 685.8 | 1022.4 | 714.8 | 1191.6 | 656.5 | 625.4 |
| 32 | 3099 | 1984 | 3215.8 | 12202.3 | 3643.5 | 4762.1 |

| SEQ ID | | | | | | |
|---|---|---|---|---|---|---|
| 39 | 1012.3 | 480.5 | 1173 | 1094.8 | 781.3 | 1099.1 |
| 74 | 987.5 | 61.1 | 894.2 | 877.1 | 629 | 887.2 |
| 78 | 418.8 | 111.9 | 529.1 | 188.7 | 371.9 | 650.2 |
| 143 | 2030.1 | 1411.5 | 3847.8 | 2492.6 | 2091.2 | 2930.6 |
| 174 | 2288.4 | 269.9 | 4741.5 | 2231.5 | 2488.1 | 7050.3 |
| 175 | 2412.3 | 2138.9 | 6427.7 | 3285.3 | 3691.4 | 4648.5 |
| 191 | 617.2 | 983.6 | 2182.4 | 2820.3 | 2081 | 1043.3 |
| 212 | 1018.3 | 208.9 | 1054.6 | 964.8 | 771.1 | 591.5 |
| 222 | 725.6 | 8103.4 | 443.9 | 4302.9 | 1433 | 10512.7 |
| 233 | 4450.9 | 1177.3 | 20110.2 | 19683.9 | 4061.3 | 2305.1 |
| 234 | 1926 | 2079.3 | 2095.5 | 2384.8 | 2482.7 | 6283.2 |
| 237 | 2547.1 | 2912.4 | 3040.2 | 2736.1 | 3005.4 | 2681.3 |
| 238 | 1822.6 | 264.6 | 1023.9 | 929.2 | 1088.1 | 1176.9 |
| 245 | 2699.8 | 113.4 | 1230.8 | 1359.8 | 887 | 1067.6 |
| 250 | 1198.1 | 249 | 1802.6 | 1764.8 | 2220.8 | 1112.4 |
| 252 | 1311.3 | 613.6 | 2434.6 | 2260.9 | 1332.7 | 1526.7 |
| 264 | 1364.1 | 382.4 | 1206.6 | 1095.1 | 1210.5 | 1451.3 |
| 290 | 463.8 | 1223.5 | 805.1 | 865.9 | 867.2 | 932.7 |
| 294 | 2024.9 | 340.9 | 4085.3 | 1859.8 | 2226.1 | 3796.8 |
| 296 | 1214 | 407.7 | 1761.4 | 1917.2 | 3476.1 | 1298.6 |
| 342 | 619 | 828.4 | 1154.4 | 639.7 | 934.8 | 1110 |

13a14

| SEQ ID | Signal | | | | | |
|---|---|---|---|---|---|---|
| | ThyFolCan_02014_40085_H133A_824TT | ThyFolCan_02014_40086_H133A_825TT | ThyFolCan_02014_40088_H133A_827TT | ThyFolCan_02014_40089_H133A_828TT | 02014_4031 8_H133A_840TT | fc__EA402 12_VDX896TT |
| 2 | 4103.9 | 1719.4 | 2824.5 | 3008.4 | 2542.7 | 2391.5 |
| 3 | 2480.6 | 144.5 | 880.5 | 497.6 | 1222.4 | 1069.4 |
| 5 | 2190.2 | 1199.7 | 4743.6 | 2936.8 | 3696.7 | 776.8 |
| 6 | 678.7 | 2589.2 | 2824.2 | 1605.5 | 1411.7 | 1403.4 |
| 9 | 6166.7 | 326.1 | 5428.5 | 2131.9 | 9187.9 | 10000.2 |
| 12 | 2872.9 | 1257.4 | 4087.8 | 3981.7 | 7045.4 | 3506.5 |
| 18 | 2329.3 | 4765.3 | 11522.2 | 7266.2 | 3045.4 | 4683.8 |
| 22 | 4848.8 | 1224.5 | 3611.1 | 6001.8 | 8784.3 | 1719.5 |
| 25 | 11319.1 | 234.5 | 3308.3 | 557.1 | 3685.8 | 383.8 |
| 28 | 772.7 | 206.8 | 830.6 | 1057.1 | 1256.5 | 1118.8 |
| 32 | 2272 | 1393.6 | 5410.1 | 3330.7 | 13825 | 869 |
| 39 | 892.8 | 541.9 | 838.5 | 941.3 | 1041 | 3461.7 |
| 74 | 126.8 | 2071.9 | 988.3 | 1357.4 | 696.8 | 1290.4 |
| 78 | 253.6 | 307.6 | 635.6 | 920.6 | 422.6 | 705.5 |
| 143 | 2718.2 | 1570.7 | 2322.8 | 2621.6 | 1038.8 | 4569.2 |
| 174 | 323 | 1399.8 | 2817 | 9345.5 | 876 | 8849.8 |
| 175 | 4437.4 | 1584 | 4411.6 | 4784.9 | 1387.4 | 7804.9 |
| 191 | 2214.1 | 575.3 | 641 | 1208.4 | 1370.9 | 4609.8 |
| 212 | 1667.2 | 662.2 | 577.7 | 1864 | 1229 | 590.6 |
| 222 | 13107.5 | 470.6 | 1030.1 | 3020.4 | 431.9 | 641.9 |
| 233 | 3196 | 1087.3 | 2932.3 | 7081 | 12626.7 | 4567.8 |
| 234 | 1659.8 | 875 | 2243.3 | 1839.4 | 1328 | 7454.4 |
| 237 | 4693.9 | 830.4 | 3700.2 | 2563.5 | 3040.5 | 1008.9 |
| 238 | 206.3 | 244.8 | 1695.9 | 2546.2 | 1614.2 | 1880.4 |
| 245 | 248.7 | 425 | 1273.9 | 1215.4 | 1333.6 | 2807.1 |
| 250 | 935.9 | 379.8 | 2284.4 | 894.4 | 867.2 | 844.3 |
| 252 | 1005.5 | 1023.3 | 1166.6 | 1884.3 | 1524.6 | 678.4 |

| | | | | | | |
|---|---|---|---|---|---|---|
| 264 | 231.5 | 589 | 1480.9 | 1740.6 | 670.9 | 2459.5 |
| 290 | 638.4 | 233 | 961.8 | 668.6 | 430.5 | 996 |
| 294 | 253.4 | 1349.8 | 3087.1 | 7034.2 | 1137.3 | 7044.6 |
| 296 | 1530.8 | 1570.6 | 1591.5 | 1110.6 | 1647.7 | 1498.9 |
| 342 | 1457.8 | 712.8 | 1052.1 | 946.9 | 436.8 | 1893.8 |

13a15

| | Signal | | | | | |
|---|---|---|---|---|---|---|
| SEQ ID | fc__EA40214_VDX898TT | fc__EA40215_VDX899TT | fc__EA40216_VDX900TT | fc__EA40217_VDX901TT | fc__EA40218_VDX902TT | fc__EA40221_VDX909TT |
| 2 | 2470.7 | 3328.2 | 4429.9 | 993.8 | 2649.9 | 4427.4 |
| 3 | 226.4 | 523.3 | 1216.2 | 230.6 | 417 | 2364.4 |
| 5 | 1037.2 | 3614.5 | 1440.6 | 3319 | 1487.4 | 9590 |
| 6 | 997 | 685.7 | 1641.1 | 967.8 | 2453.4 | 2537.2 |
| 9 | 384.9 | 1897.9 | 973.8 | 468.7 | 915.8 | 3339.3 |
| 12 | 9940.9 | 1131.5 | 4352.5 | 3202.2 | 4269.6 | 15203.8 |
| 18 | 4150.7 | 7715.2 | 5736.9 | 555.2 | 9139.4 | 7575.1 |
| 22 | 499.6 | 4466 | 2120.9 | 208.2 | 1563.8 | 1471.9 |
| 25 | 1721.2 | 1912 | 10485.2 | 826.1 | 1002 | 6640.8 |
| 28 | 232.1 | 706.8 | 501.4 | 122.6 | 779.4 | 645.5 |
| 32 | 2728.8 | 3753 | 2644.6 | 1820.8 | 3404.5 | 10045.3 |
| 39 | 1006.6 | 1103.3 | 1192.6 | 880.8 | 2310.8 | 346.3 |
| 74 | 2642.5 | 794.9 | 374.8 | 1621.8 | 1057.9 | 294 |
| 78 | 294.6 | 280.8 | 131.2 | 136.4 | 344.4 | 391.3 |
| 143 | 2141.4 | 2616.6 | 5902.7 | 3882.4 | 3860.3 | 2140.3 |
| 174 | 407.1 | 2263.7 | 4270 | 2006.5 | 2076.1 | 4365.1 |
| 175 | 2416.2 | 3697.7 | 9897.3 | 6489.5 | 5706.6 | 2832.4 |
| 191 | 1436.8 | 2900.8 | 5153.2 | 165.9 | 963.5 | 512.1 |
| 212 | 839.5 | 1241.8 | 173.4 | 186.6 | 451.1 | 539.9 |
| 222 | 293.5 | 1318.1 | 6645.4 | 426.3 | 623.3 | 2727.5 |
| 233 | 17314.7 | 18164.1 | 2971.6 | 2885.8 | 2068.7 | 15099.5 |
| 234 | 2834.4 | 1575.6 | 3547 | 904.6 | 3783.5 | 6440.1 |
| 237 | 945.9 | 3105.6 | 1268.9 | 2192.9 | 893.1 | 2333.7 |
| 238 | 702.9 | 1189 | 744.7 | 554.4 | 383.3 | 908.6 |
| 245 | 914.6 | 1729.7 | 1038.5 | 839.4 | 784.2 | 459.1 |
| 250 | 845.9 | 2673.5 | 2175.8 | 702.9 | 1305.7 | 723.2 |
| 252 | 1300.4 | 2454.3 | 1377.7 | 1061.4 | 1235.8 | 2869.5 |
| 264 | 467.6 | 1277.4 | 638.7 | 2058 | 1076.7 | 569.3 |
| 290 | 573.7 | 617.2 | 993.4 | 767.4 | 1281.3 | 866.1 |
| 294 | 507.1 | 1922.1 | 2827.1 | 1753.3 | 1585.8 | 2926.6 |
| 296 | 2156.2 | 2563 | 1832.8 | 533.7 | 2130.3 | 2647.8 |
| 342 | 535.6 | 937.4 | 2261.2 | 1949.2 | 975.7 | 618.5 |

13a16

| | Signal | | | | | |
|---|---|---|---|---|---|---|
| SEQ ID | fc_EA40222_VDX910TT | EA02014_40386_H133A_954TT | EA02014_40394_H133A_967TT | EA02014_40395_H133A_968TT | EA02014_40396_H133A_969TT | EA02014_40397_H133A_970TT |
| 2 | 2362.1 | 1417 | 1834.4 | 2135.5 | 3006.2 | 1378.8 |
| 3 | 254.5 | 263.8 | 293.1 | 346.3 | 346.5 | 230.2 |
| 5 | 2789.1 | 8005.6 | 1358.8 | 637.3 | 1214.1 | 1214.7 |
| 6 | 1096.6 | 1050.7 | 1291.7 | 987.4 | 1223.8 | 2564.6 |
| 9 | 834.8 | 1052.4 | 2701.2 | 1171.4 | 6080 | 440.4 |
| 12 | 2031.6 | 4283.7 | 2805.1 | 4294.1 | 4966.4 | 1686.4 |
| 18 | 9498.5 | 18893.2 | 15454.8 | 2385.4 | 3575.2 | 14192.8 |

| SEQ ID | | | | | |
|---|---|---|---|---|---|
| 22 | 1449.5 | 1020 | 888.3 | 2585.4 | 3721.5 | 727.2 |
| 25 | 541.3 | 253.5 | 572.5 | 3378.9 | 709.2 | 372 |
| 28 | 502.3 | 443 | 868.6 | 378.9 | 964.1 | 341.9 |
| 32 | 1205.9 | 7122.3 | 811.1 | 2553.9 | 797 | 1753.4 |
| 39 | 1215.4 | 1031.1 | 511 | 722 | 2166.1 | 527.2 |
| 74 | 1385.4 | 1477.5 | 509.1 | 1159.3 | 1031.4 | 495.4 |
| 78 | 367.5 | 834.2 | 200.7 | 235.3 | 433.7 | 262.9 |
| 143 | 1956.7 | 1083.1 | 1486.7 | 2746 | 2538.4 | 1943.7 |
| 174 | 4466.4 | 2823.7 | 1156.3 | 3547.9 | 1541.5 | 2214.3 |
| 175 | 1799.1 | 1264 | 1758.7 | 4539.5 | 3663.3 | 2264.4 |
| 191 | 560.2 | 1494 | 607.9 | 2943.2 | 2727.4 | 1474.7 |
| 212 | 1046.1 | 307.8 | 548.8 | 340.4 | 982.9 | 647 |
| 222 | 248.3 | 533.1 | 1356.4 | 1194.2 | 1169.8 | 228.2 |
| 233 | 1183.6 | 612.7 | 1063.5 | 5528.1 | 6265.3 | 1076 |
| 234 | 1886.7 | 3460.3 | 1207.2 | 2384.1 | 3860.6 | 3322.7 |
| 237 | 934.6 | 933.5 | 2133.3 | 791 | 1890.7 | 1257.5 |
| 238 | 834.9 | 291.1 | 1034.1 | 380.8 | 946.4 | 415 |
| 245 | 1509.7 | 786.6 | 657.1 | 384.9 | 4773.4 | 447.6 |
| 250 | 2406.8 | 1245.7 | 1247.1 | 659 | 1647.1 | 595 |
| 252 | 1536.8 | 1182.4 | 1035.6 | 526.7 | 893 | 1276.7 |
| 264 | 1315.8 | 582.8 | 1373.3 | 958 | 1622.3 | 508.6 |
| 290 | 732.4 | 835.3 | 683.8 | 1744.3 | 523 | 850.2 |
| 294 | 2817 | 2372.1 | 929.5 | 2611.8 | 1279.4 | 2097.3 |
| 296 | 1088.4 | 1541 | 2014.6 | 621.4 | 1368.3 | 981.8 |
| 342 | 763.6 | 866.9 | 722.2 | 559 | 594.3 | 445.9 |

13a17

| SEQ ID | EA02014_40405_H133A_982TT | EA02014_40406_H133A_983TT | Signal pb1 | pb2 | pb3 | pb4 | pb5' |
|---|---|---|---|---|---|---|---|
| 2 | 3615.6 | 2106 | 204.1 | 324.2 | 178.6 | 206.6 | 249.1 |
| 3 | 765 | 372.2 | 171.5 | 303.8 | 159.1 | 61.1 | 90.2 |
| 5 | 2446.1 | 394 | 107.9 | 19.9 | 68.8 | 74.6 | 69 |
| 6 | 1051.6 | 1176 | 211.6 | 214.4 | 318.6 | 218.8 | 355.4 |
| 9 | 2059.2 | 1081.4 | 18 | 18.8 | 29.2 | 9.7 | 134 |
| 12 | 3520 | 391.5 | 11.4 | 9.1 | 9.9 | 33.8 | 8.3 |
| 18 | 4633.4 | 13339 | 94.2 | 50.7 | 67.6 | 74.9 | 29.7 |
| 22 | 6678.8 | 403.5 | 80.1 | 8.8 | 58.9 | 6.8 | 5 |
| 25 | 1229.9 | 601.5 | 197.1 | 106.7 | 65.2 | 18.7 | 87.1 |
| 28 | 778.2 | 197.6 | 11.4 | 9 | 6.6 | 11.3 | 1.7 |
| 32 | 1302.9 | 4182.6 | 8.7 | 21.6 | 30.5 | 66.1 | 64.6 |
| 39 | 2104 | 699 | 246.6 | 121.1 | 42.8 | 122.8 | 171.7 |
| 74 | 1189.7 | 1142.3 | 59.9 | 94.4 | 70.3 | 29 | 5.2 |
| 78 | 833.6 | 80.9 | 48 | 45.5 | 66.9 | 49.5 | 119.7 |
| 143 | 2842.2 | 1778.2 | 290 | 291.2 | 190.4 | 65.6 | 203.5 |
| 174 | 3307.3 | 329.8 | 29.8 | 42.8 | 25.8 | 11.5 | 13.9 |
| 175 | 3878.9 | 1966.8 | 165.7 | 282.2 | 158.3 | 67.2 | 249.2 |
| 191 | 3952.2 | 5278 | 46.8 | 181.4 | 148.2 | 97.3 | 152.1 |
| 212 | 2201.7 | 1134.3 | 70.4 | 55.7 | 3.5 | 87 | 99.8 |
| 222 | 469 | 451.7 | 120.4 | 148.4 | 57.7 | 49.6 | 58 |
| 233 | 11069.2 | 5607 | 13 | 9.8 | 76.9 | 7.6 | 5.6 |
| 234 | 3590 | 3449.5 | 186 | 201.3 | 165.3 | 54.8 | 141.1 |
| 237 | 2057.6 | 740.2 | 135.3 | 150.7 | 16.6 | 14.5 | 19 |
| 238 | 1679.4 | 432.3 | 22.5 | 43.8 | 11.4 | 6.2 | 5.1 |

| | | | | | | | |
|---|---|---|---|---|---|---|---|
| 245 | 2301.6 | 1040.5 | 7.1 | 12.7 | 22.5 | 5.9 | 4.6 |
| 250 | 434.6 | 1079.4 | 97.8 | 138.9 | 33.7 | 71.3 | 46.8 |
| 252 | 844.6 | 1446.1 | 35.6 | 80.3 | 29.6 | 63.3 | 88.5 |
| 264 | 1125.7 | 1084.5 | 102.8 | 31.6 | 147.6 | 101.5 | 163.3 |
| 290 | 347.9 | 466.9 | 35.8 | 7.3 | 14.4 | 87.2 | 71.7 |
| 294 | 1601.7 | 575.2 | 105.1 | 94.5 | 41.5 | 31.9 | 35.3 |
| 296 | 872.8 | 1349.1 | 217.8 | 162.6 | 186.2 | 171.2 | 212.5 |
| 342 | 948.7 | 482.8 | 296.5 | 220.2 | 308.1 | 126.3 | 298.6 |

13a18

| SEQ ID | pb6' | pb7' | pb8' | Signal pd10 | pd11 | pd12 | pd9 |
|---|---|---|---|---|---|---|---|
| 2 | 167.6 | 233.9 | 232.6 | 116.9 | 204.5 | 293.2 | 79.6 |
| 3 | 110.3 | 88 | 221 | 90.7 | 74.9 | 90.9 | 129.5 |
| 5 | 29.7 | 70.2 | 182.5 | 32.6 | 34.5 | 130.5 | 12.9 |
| 6 | 226.5 | 440.1 | 207 | 219.7 | 275.2 | 134.1 | 193.1 |
| 9 | 28.9 | 26.8 | 30.9 | 32.4 | 5.3 | 25.7 | 20.3 |
| 12 | 6.6 | 18.8 | 61.4 | 38.2 | 10.7 | 4.1 | 11.6 |
| 18 | 53.6 | 37.8 | 31.8 | 68.8 | 117.5 | 70.8 | 23.4 |
| 22 | 16.9 | 10.6 | 32.1 | 6.4 | 29.2 | 21.2 | 3.1 |
| 25 | 127 | 123.7 | 118.7 | 9.7 | 10.6 | 64.9 | 6.6 |
| 28 | 13.2 | 4.9 | 43.3 | 8 | 8.1 | 12 | 26.6 |
| 32 | 36.4 | 51.5 | 25.1 | 17.2 | 17.2 | 19.7 | 11 |
| 39 | 326.5 | 182.6 | 27.3 | 126.2 | 151.9 | 26 | 182.7 |
| 74 | 48.2 | 29 | 39.8 | 55.3 | 35.3 | 76.9 | 6.2 |
| 78 | 92.1 | 20 | 93.1 | 26.9 | 11.9 | 56.6 | 104.8 |
| 143 | 176.4 | 224.5 | 248.5 | 126.2 | 184.6 | 162.8 | 249.6 |
| 174 | 40.8 | 149.8 | 24.2 | 10.6 | 15.6 | 81.4 | 16.8 |
| 175 | 262.5 | 355.4 | 207.8 | 21.6 | 213.9 | 175.8 | 193.7 |
| 191 | 151.2 | 310.7 | 93.3 | 65.7 | 108.4 | 95.9 | 79.3 |
| 212 | 93.6 | 121.7 | 141.7 | 30.8 | 42.6 | 108.4 | 93.2 |
| 222 | 83.5 | 82.8 | 60.7 | 16.4 | 50 | 71.1 | 66.9 |
| 233 | 144.7 | 64.1 | 16.5 | 7 | 6.4 | 70.8 | 8.1 |
| 234 | 40.4 | 248.1 | 43.8 | 45.5 | 143.3 | 30.9 | 77.2 |
| 237 | 223.8 | 141.1 | 129.5 | 57.6 | 21.9 | 11.5 | 18.7 |
| 238 | 32.1 | 27.6 | 66.5 | 10.5 | 33.4 | 7.7 | 61.6 |
| 245 | 19.8 | 7.2 | 4.8 | 2.4 | 11.2 | 4.7 | 6.7 |
| 250 | 75.3 | 109.2 | 65.3 | 85.7 | 119.9 | 118.7 | 66.4 |
| 252 | 103.8 | 91.2 | 94.8 | 17 | 69.3 | 76.1 | 22.5 |
| 264 | 151 | 224.6 | 129.9 | 122.8 | 99.8 | 61.5 | 24.8 |
| 290 | 21.8 | 27.6 | 50.7 | 49.3 | 6.2 | 2.7 | 3 |
| 294 | 91.1 | 58.4 | 40 | 24.8 | 52.6 | 67.8 | 31.7 |
| 296 | 282.4 | 299.2 | 143.7 | 149.7 | 197.9 | 205.2 | 147.7 |
| 342 | 304.5 | 437.8 | 327.4 | 90.9 | 247.6 | 224.4 | 268.6 |

Table 13b
13b1

| SEQ ID | ThyMixBen_02014_......_Signal | | | | | | |
| | 40062_H133A_800TT | 40063_H133A_801TT | 40064_H133A_802TT | 40065_H133A_804TT | 40066_H133A_805TT | 40067_H133A_806TT | 40068_H133A_807TT |
| --- | --- | --- | --- | --- | --- | --- | --- |
| 83 | 156.7 | 114.3 | 73.8 | 240.3 | 173 | 193.6 | 291.2 |
| 142 | 28.3 | 7.9 | 15 | 28.4 | 26.9 | 25.8 | 14.2 |
| 136 | 100.4 | 23.4 | 15.3 | 106.9 | 28.3 | 14.9 | 56 |

| SEQ ID | | | | | | | |
|---|---|---|---|---|---|---|---|
| 137 | 527.2 | 47 | 32.3 | 177.2 | 19.5 | 53.1 | 404.6 |
| 152 | 237.5 | 230.4 | 188.7 | 465.2 | 183 | 260.9 | 190.9 |
| 160 | 291.6 | 141.9 | 80.9 | 323.1 | 98.1 | 120.3 | 219.7 |
| 163 | 15.6 | 53.1 | 25.1 | 70.8 | 20.1 | 56.1 | 47.8 |
| 165 | 190.2 | 230.7 | 142.4 | 483.5 | 179.3 | 241.7 | 410.9 |
| 210 | 436.9 | 8.6 | 18.4 | 343.7 | 31.8 | 8 | 148.1 |
| 214 | 269.2 | 134.4 | 167.7 | 141.8 | 145.7 | 140.5 | 349.1 |
| 219 | 217 | 19.6 | 7.9 | 85.6 | 21.8 | 26.7 | 115.1 |
| 224 | 588.2 | 121.4 | 119.2 | 275.2 | 136.6 | 240.5 | 392.5 |
| 225 | 508.1 | 30.1 | 42.3 | 107.3 | 59.4 | 29.5 | 147.3 |
| 226 | 57.3 | 48.2 | 80.9 | 103.9 | 43.5 | 12.3 | 88.9 |
| 309 | 257.7 | 20.6 | 20.7 | 75.5 | 19.2 | 24 | 833.9 |
| 332 | 287.1 | 17 | 26.1 | 103.8 | 115.9 | 68.8 | 37.9 |
| 346 | 622.2 | 18.3 | 24 | 17.6 | 20.7 | 13.2 | 442.1 |

13b2

| | 02014_...._Signal | | | | | | |
|---|---|---|---|---|---|---|---|
| SEQ ID | 40198_H133A_871TT | 40321_H133A_913TT | 40322_H133A_914TT | 40323_H133A_915TT | 40324_H133A_917TT | 40325_H133A_918TT | 40326_H133A_919TT |
| 83 | 39.5 | 90.2 | 76.4 | 255.8 | 150.8 | 109.7 | 142.6 |
| 142 | 25.3 | 22.5 | 12.9 | 34.4 | 8.5 | 20.6 | 67.7 |
| 136 | 9.8 | 31.8 | 16.8 | 132 | 115.4 | 130 | 7.2 |
| 137 | 227.8 | 53.6 | 68.7 | 60.1 | 29.6 | 133.2 | 29 |
| 152 | 184.4 | 214.3 | 253.2 | 294.7 | 313.8 | 251.3 | 294.2 |
| 160 | 267.5 | 250.7 | 206.5 | 328.8 | 191.1 | 220.8 | 114.4 |
| 163 | 44.3 | 56.4 | 23.9 | 98.5 | 35.2 | 68 | 41.8 |
| 165 | 174.3 | 221.8 | 185.5 | 266.3 | 104.6 | 217.1 | 159.9 |
| 210 | 25.8 | 153.6 | 42 | 55.5 | 8.2 | 152 | 49.1 |
| 214 | 43.2 | 83.6 | 110.6 | 213.7 | 132.6 | 89.3 | 138.7 |
| 219 | 56.6 | 53.5 | 45.2 | 122.1 | 24.4 | 124 | 41.3 |
| 224 | 269.7 | 357.4 | 260 | 191.5 | 168.8 | 330.8 | 169.8 |
| 225 | 204.8 | 322.6 | 114.2 | 55.6 | 121.8 | 317.6 | 95.8 |
| 226 | 11.9 | 57.4 | 69.9 | 22 | 67.5 | 33.6 | 19.3 |
| 309 | 37.9 | 37 | 28.2 | 53 | 10.2 | 32.6 | 24.2 |
| 332 | 9.9 | 59.2 | 66.7 | 57.8 | 13 | 40.5 | 9 |
| 346 | 28.6 | 263.8 | 11.1 | 27.2 | 32.5 | 153.4 | 61.1 |

13b3

| | ThyFolBen_02014_...._Signal | | | | |
|---|---|---|---|---|---|
| SEQ ID | 40079_H133A_818TT | 40080_H133A_819TT | 40081_H133A_820TT | 40082_H133A_821TT | 40083_H133A_822TT |
| 83 | 63.4 | 151.8 | 104.1 | 27.5 | 138.9 |
| 142 | 15 | 20.2 | 18.8 | 18.4 | 23 |
| 136 | 17.6 | 135.4 | 77.1 | 31.7 | 17.7 |
| 137 | 24.7 | 36.1 | 18 | 28.4 | 42.6 |
| 152 | 62.1 | 176.7 | 146.4 | 273.3 | 328.2 |
| 160 | 153 | 138.1 | 131 | 192.4 | 124.1 |
| 163 | 6.9 | 21.4 | 35 | 63.5 | 13.4 |
| 165 | 28.7 | 155.3 | 112.6 | 135.2 | 222.6 |
| 210 | 27.5 | 10.4 | 8.1 | 9.9 | 32.7 |
| 214 | 211.7 | 210.8 | 83.9 | 166.4 | 160.2 |
| 219 | 18.7 | 111.8 | 45.7 | 10.9 | 29.6 |
| 224 | 114.7 | 241.8 | 100 | 160.9 | 242.4 |

| | | | | | |
|---|---|---|---|---|---|
| 225 | 69.4 | 63.8 | 145.4 | 110.9 | 126.1 |
| 226 | 92.1 | 95.5 | 36.3 | 74.5 | 67.2 |
| 309 | 16.8 | 17.1 | 12.1 | 18.8 | 27.3 |
| 332 | 40 | 20.5 | 21 | 11 | 6.6 |
| 346 | 7.2 | 27.1 | 18.6 | 29.3 | 26.7 |

13b4

fa__EA40...._Signal

| SEQ ID | 191_VDX 862TT | 192_VDX 863TT | 193_VDX 864TT | 194_VDX 865TT | 195_VDX 866TT | 196_VDX 867TT | 197_VDX 869TT | 219_VDX 907TT | 220_VDX 908TT |
|---|---|---|---|---|---|---|---|---|---|
| 83 | 26.9 | 24.4 | 56.7 | 155.1 | 22.3 | 94.8 | 63 | 41.9 | 256 |
| 142 | 16.1 | 22.4 | 14.5 | 54.8 | 17.1 | 15.8 | 15 | 21.9 | 23.9 |
| 136 | 23 | 59 | 20.4 | 121.4 | 12.5 | 88.4 | 77.1 | 47.1 | 36.8 |
| 137 | 26.7 | 32.4 | 19.4 | 45.2 | 32.8 | 30.8 | 55.2 | 97 | 63.4 |
| 152 | 75.7 | 189.4 | 211.4 | 320.4 | 194 | 75.5 | 314 | 114.8 | 260 |
| 160 | 239.5 | 101.4 | 91.3 | 292.1 | 55.8 | 86.8 | 53.7 | 47.4 | 145.4 |
| 163 | 48.5 | 47.6 | 23.1 | 168.3 | 35.4 | 13.4 | 51.5 | 13.5 | 60.4 |
| 165 | 26.5 | 94.3 | 81.1 | 197.9 | 112.9 | 164.6 | 155.9 | 29.7 | 250.4 |
| 210 | 32.2 | 16.2 | 23.8 | 81.4 | 25.3 | 22.7 | 140.5 | 15.5 | 200.8 |
| 214 | 119.9 | 130.9 | 141.7 | 235.4 | 263.7 | 30.4 | 124.9 | 57 | 241 |
| 219 | 80.6 | 7.6 | 15.9 | 20.9 | 25 | 10 | 16.5 | 23.4 | 20.1 |
| 224 | 50.9 | 25.8 | 50 | 80 | 32.6 | 99.7 | 136.4 | 137.5 | 167 |
| 225 | 66.7 | 41.4 | 64.4 | 69.5 | 18.1 | 94.7 | 51.1 | 51.6 | 149.8 |
| 226 | 76 | 2.8 | 3.5 | 99.1 | 88.6 | 18.3 | 60.6 | 6.4 | 11.6 |
| 309 | 6.7 | 15.2 | 9.4 | 62.6 | 20.2 | 18.4 | 15.9 | 10.2 | 33.3 |
| 332 | 9.6 | 36.9 | 10.8 | 91.1 | 103 | 30.1 | 12.3 | 19.7 | 36.5 |
| 346 | 25.1 | 16.5 | 20.8 | 89.5 | 34.2 | 20.9 | 18.3 | 11.3 | 114.6 |

13b5

02014_...._Signal

| SEQ ID | 40327_H133A_920TT | 40332_H133A_938TT | 40334_H133A_941TT | 40335_H133A_940TT |
|---|---|---|---|---|
| 83 | 216.8 | 145.1 | 58.1 | 102.1 |
| 142 | 22.2 | 7.9 | 6.6 | 14.5 |
| 136 | 71.7 | 30.1 | 47.9 | 57.1 |
| 137 | 47 | 48.1 | 69.1 | 57.4 |
| 152 | 252.7 | 189 | 184.9 | 138.7 |
| 160 | 153.6 | 179.9 | 121.9 | 66.6 |
| 163 | 53.3 | 13 | 69.1 | 38.6 |
| 165 | 146.9 | 146.7 | 85.1 | 120 |
| 210 | 24 | 59.1 | 21.5 | 23.8 |
| 214 | 90.5 | 224.7 | 178.7 | 139.3 |
| 219 | 36.4 | 17.5 | 14.9 | 63.3 |
| 224 | 180.2 | 126.6 | 154.2 | 161 |
| 225 | 59.7 | 197.5 | 83.8 | 115.7 |
| 226 | 31.9 | 58.1 | 33.7 | 34.5 |
| 309 | 27.1 | 7.7 | 6.2 | 15.6 |
| 332 | 83.8 | 7.3 | 40.7 | 47.6 |
| 346 | 17 | 18.8 | 27.6 | 24.4 |

13b6

EA02014_...._Signal

SEQ   40374_H133A_9 40376_H133A_ 40377_H133A_ 40378_H133A 40379_H133A_9 40380_H133A_

| ID | 21TT | 923TT | 924TT | _925TT | 26TT | 927TT | |
|----|------|-------|-------|--------|------|-------|---|
| 83 | 174.4 | 32.7 | 69.1 | 86.6 | 67.7 | 204.9 | |
| 142 | 13.7 | 19.8 | 16.5 | 57.7 | 22.9 | 18.9 | |
| 136 | 24.2 | 9.3 | 12.1 | 25.3 | 190.4 | 16.3 | |
| 137 | 38.3 | 143.4 | 28.2 | 77.5 | 156.1 | 255.2 | |
| 152 | 178.2 | 230.8 | 155 | 237.4 | 235.7 | 70 | |
| 160 | 125.2 | 133.5 | 74.9 | 197 | 170.1 | 234 | |
| 163 | 54.4 | 4.9 | 35.5 | 46.1 | 65.2 | 59.9 | |
| 165 | 218.9 | 155.1 | 206 | 85.6 | 158.1 | 532.5 | |
| 210 | 36.8 | 25 | 20.6 | 25.8 | 19.8 | 551.3 | |
| 214 | 126.7 | 186.1 | 167.2 | 114.1 | 191.9 | 161.4 | |
| 219 | 32.3 | 25.5 | 15.3 | 10.2 | 95.6 | 18.5 | |
| 224 | 220.8 | 301.9 | 129.7 | 178.8 | 308.2 | 270.9 | |
| 225 | 158 | 221.8 | 127.9 | 65.6 | 269.4 | 60.6 | |
| 226 | 37.5 | 33.4 | 9.6 | 57.2 | 80.8 | 18.1 | |
| 309 | 26.1 | 16 | 17.8 | 31.8 | 32.6 | 49.2 | |
| 332 | 26.3 | 38.5 | 6.7 | 11.6 | 73.5 | 21.6 | |
| 346 | 20.7 | 151.4 | 21.6 | 19.2 | 179.4 | 53.6 | |

13b7

EA02014_...._Signal

| SEQ ID | 40387_H133A_955TT | 40388_H133A_956TT | 40389_H133A_957TT | 40390_H133A_959TT | 40391_H133A_960TT | 40392_H133A_961TT | 40393_H133A_962TT |
|--------|------|------|------|------|------|------|------|
| 83 | 29.1 | 121.5 | 70.7 | 28.8 | 121.6 | 120.5 | 34 |
| 142 | 16.7 | 14.2 | 10.8 | 21.4 | 18.6 | 16.5 | 21.9 |
| 136 | 15.7 | 27.8 | 67.7 | 45.9 | 15.4 | 8.2 | 43 |
| 137 | 28.5 | 44.8 | 34.5 | 50 | 31.3 | 162.2 | 18.5 |
| 152 | 85.4 | 163 | 183.4 | 127.1 | 298.3 | 193 | 221.2 |
| 160 | 121.9 | 51.9 | 175 | 137.4 | 208.5 | 125.9 | 162.9 |
| 163 | 47 | 27.7 | 43.6 | 80.1 | 29.7 | 33.8 | 44.4 |
| 165 | 42.2 | 122.9 | 103.8 | 150.6 | 253.3 | 106.9 | 221.6 |
| 210 | 27.1 | 5.2 | 20.4 | 73.7 | 29.4 | 38.7 | 85 |
| 214 | 36.4 | 68.3 | 165.9 | 25.3 | 129.8 | 165.9 | 100.9 |
| 219 | 23.3 | 51 | 29.1 | 25.7 | 67 | 20.8 | 21 |
| 224 | 143.8 | 95.1 | 128.8 | 78.7 | 108.8 | 152 | 109 |
| 225 | 140.5 | 83.1 | 34.3 | 54 | 62.8 | 114.7 | 94.3 |
| 226 | 4.3 | 24.7 | 84.1 | 47.7 | 33.3 | 66.1 | 13.3 |
| 309 | 18.7 | 11.4 | 26.7 | 44.2 | 20.6 | 34.1 | 35.2 |
| 332 | 11.1 | 4 | 37.9 | 14.1 | 17.8 | 13.1 | 11.5 |
| 346 | 21.8 | 22.1 | 12.8 | 35.7 | 38.7 | 29.6 | 10.1 |

13b8

ThyPapCan_02014_...._Signal

| SEQ ID | 40090_H133A_829TT | 40091_H133A_830T T@IC | 40092_H133A_831T T | 40093_H133A_832T T | 40095_H133A_834T T | 40096_H133A_835T T | 40097_H133A_836T T | 40098_H133A_837T T@I2 | 40099_H133A_838T T@I2 |
|--------|------|------|------|------|------|------|------|------|------|
| 83 | 304.3 | 199.4 | 258.5 | 171.4 | 112.3 | 186.5 | 28.5 | 137.1 | 85.9 |
| 142 | 26 | 34.7 | 22.4 | 19.1 | 16.4 | 25.8 | 12.1 | 22.5 | 15.4 |
| 136 | 122.2 | 116.6 | 21.7 | 27.3 | 30.7 | 110.2 | 143.8 | 14.1 | 140.7 |
| 137 | 40.7 | 72 | 23.8 | 36.8 | 19.5 | 31.5 | 29.6 | 29.4 | 32.5 |
| 152 | 611.5 | 682.6 | 236.2 | 170.5 | 200.6 | 104 | 136.4 | 118 | 155.9 |
| 160 | 218.1 | 244.7 | 225.4 | 68.9 | 76.4 | 137.3 | 83.2 | 171.7 | 66.7 |
| 163 | 17.8 | 119.7 | 38.9 | 51.8 | 52.9 | 42.7 | 25.5 | 57.2 | 23.6 |

| 165 | 370.6 | 263.3 | 353.8 | 154.1 | 165.1 | 233.2 | 100.4 | 227.9 | 213.3 |
| 210 | 223.2 | 249.8 | 303 | 9.7 | 30.2 | 94.5 | 21.9 | 42.1 | 23.1 |
| 214 | 178.4 | 246.7 | 155.4 | 188.3 | 138.6 | 133.7 | 69 | 298.6 | 90.8 |
| 219 | 36.8 | 136.4 | 64.9 | 24.3 | 19.1 | 25.9 | 11.7 | 13.6 | 23.8 |
| 224 | 364.7 | 427.6 | 180 | 191.1 | 163.6 | 211.5 | 36.2 | 303.4 | 164.5 |
| 225 | 333.9 | 213.5 | 221 | 124.6 | 137.2 | 195.1 | 46.9 | 145.6 | 34.3 |
| 226 | 179.2 | 142 | 57 | 15.4 | 13.7 | 91.8 | 33.5 | 132.6 | 38 |
| 309 | 88.8 | 136.6 | 48.8 | 22.1 | 23.6 | 40.5 | 16.5 | 33.9 | 24.7 |
| 332 | 92 | 56.9 | 42.4 | 61.1 | 34.4 | 70.6 | 14.5 | 108.1 | 60.8 |
| 346 | 58.4 | 61 | 197.1 | 38 | 29.1 | 281.5 | 37.5 | 30.8 | 33 |

13b9

| | | | | Signal | | |
| SEQ ID | 02014_40317_H133A_839TT | pc__EA40200_VDX879TT | pc__EA40201_VDX881TT | pc__EA40202_VDX882TT | pc__EA40203_VDX883TT | pc__EA40204_VDX884TT |
| --- | --- | --- | --- | --- | --- | --- |
| 83 | 288.1 | 25.7 | 32.9 | 29.2 | 58.6 | 184.4 |
| 142 | 16.1 | 21.2 | 23.5 | 7.6 | 21.5 | 30.6 |
| 136 | 62.8 | 160.5 | 141.2 | 8.7 | 13.2 | 19.4 |
| 137 | 45.4 | 155.5 | 35.5 | 63.9 | 41.5 | 31.1 |
| 152 | 383.4 | 197 | 90.6 | 123.7 | 188 | 240 |
| 160 | 142.9 | 79.7 | 181.6 | 205.4 | 157.5 | 137.8 |
| 163 | 60.7 | 102.6 | 138.6 | 54.2 | 51.3 | 9.1 |
| 165 | 238.4 | 123.6 | 227.1 | 113.8 | 224.6 | 241.9 |
| 210 | 134.1 | 184.4 | 28.4 | 42.7 | 12.1 | 10 |
| 214 | 189.2 | 173 | 50 | 164.7 | 109.2 | 250.7 |
| 219 | 26.8 | 30.4 | 13.3 | 78.6 | 34.3 | 34.8 |
| 224 | 215.8 | 139.5 | 255.3 | 96.9 | 181.3 | 163.2 |
| 225 | 167.5 | 35.2 | 98.1 | 32.4 | 221.4 | 54.3 |
| 226 | 49.4 | 43.4 | 84.3 | 16.4 | 141.3 | 27.2 |
| 309 | 340.8 | 33.7 | 32 | 22.8 | 29.8 | 45.9 |
| 332 | 15.9 | 39.9 | 67.1 | 14.3 | 88.5 | 25.2 |
| 346 | 14.3 | 37.9 | 36.5 | 33.5 | 28.7 | 37.4 |

13b10

| | | | pc__EA40....Signal | | | |
| SEQ ID | 205_VDX885TT | 206_VDX886TT | 207_VDX890TT | 208_VDX892TT | 209_VDX893TT | 210_VDX894TT |
| --- | --- | --- | --- | --- | --- | --- |
| 83 | 22.9 | 81.5 | 34.1 | 159.2 | 88.7 | 43.6 |
| 142 | 26.4 | 22.6 | 8.7 | 10.8 | 18.5 | 23.6 |
| 136 | 28.1 | 18.2 | 9.6 | 14.6 | 17.3 | 22.5 |
| 137 | 207.7 | 94.4 | 68.8 | 83.7 | 17.8 | 140.4 |
| 152 | 77.3 | 225.5 | 227.5 | 95.5 | 301.1 | 59.9 |
| 160 | 294.5 | 164.6 | 110.3 | 84.5 | 105.6 | 163 |
| 163 | 140.9 | 34 | 25.9 | 9.3 | 41.9 | 83.5 |
| 165 | 241.8 | 101.4 | 193.6 | 161.9 | 112.8 | 190.6 |
| 210 | 416.8 | 24.1 | 45.5 | 15.1 | 26.9 | 15.6 |
| 214 | 323.3 | 139.5 | 148.2 | 174.1 | 104.5 | 142.1 |
| 219 | 131.8 | 12 | 25.9 | 48.9 | 14.4 | 96.2 |
| 224 | 215.3 | 136.2 | 126 | 193.8 | 195.4 | 67.1 |
| 225 | 49.5 | 105 | 54.2 | 97.4 | 33 | 38.8 |
| 226 | 181.1 | 55.2 | 34.1 | 4 | 15 | 125.1 |
| 309 | 89.7 | 47.4 | 58.6 | 17.8 | 23.5 | 72.3 |
| 332 | 73 | 66.8 | 48.2 | 27.5 | 41.1 | 25.3 |

| 346 | 13.6 | 107.7 | 145.3 | 12.7 | 18.3 | 20 |

## 13b11

02014_40...._Signal

| SEQ ID | 319_H133A_903TT | 320_H133A_904TT | 328_H133A_928TT | 330_H133A_932TT | 331_H133A_933TT |
|---|---|---|---|---|---|
| 83 | 58.3 | 226.6 | 92.7 | 79.2 | 106.8 |
| 142 | 14.5 | 118.3 | 7.4 | 8 | 13 |
| 136 | 18.9 | 87 | 29.4 | 86.9 | 9.3 |
| 137 | 24.4 | 174.5 | 25.7 | 135.9 | 31.8 |
| 152 | 178.1 | 137.4 | 176.6 | 151.1 | 166.2 |
| 160 | 83.2 | 205 | 93.5 | 103.6 | 73.3 |
| 163 | 31.5 | 100.2 | 19.2 | 40.5 | 33.4 |
| 165 | 223.6 | 235.9 | 161.5 | 132.8 | 132 |
| 210 | 35.5 | 99.9 | 16.4 | 124.9 | 17 |
| 214 | 118.8 | 178 | 25.6 | 115.6 | 117 |
| 219 | 15.1 | 80.2 | 27.7 | 54.2 | 38.2 |
| 224 | 174.5 | 323.7 | 223.3 | 203 | 185.3 |
| 225 | 170.3 | 200.3 | 180.3 | 157.3 | 157.2 |
| 226 | 65.2 | 26.9 | 25.2 | 4.6 | 35.7 |
| 309 | 11.1 | 270.7 | 15.9 | 91.5 | 18.6 |
| 332 | 12 | 77.4 | 15.1 | 7.2 | 7.3 |
| 346 | 30.4 | 387.2 | 25.4 | 204.4 | 14.7 |

## 13b12

EA02014_403...._Signal

| SEQ ID | 75_H133A_922TT | 381_H133A_945TT | 382_H133A_946TT | 383_H133A_947TT | 384_H133A_948TT |
|---|---|---|---|---|---|
| 83 | 88.1 | 22.1 | 193.5 | 166.9 | 73.7 |
| 142 | 12 | 19.6 | 13.2 | 21.6 | 16.9 |
| 136 | 11.8 | 12.3 | 52 | 46.7 | 107.9 |
| 137 | 51.3 | 182.2 | 43.4 | 23.9 | 14.5 |
| 152 | 341 | 139 | 248.1 | 299.5 | 84.2 |
| 160 | 95.6 | 102 | 99.5 | 184.6 | 90.4 |
| 163 | 77.6 | 41.6 | 25.9 | 51.7 | 4.9 |
| 165 | 254.8 | 63.5 | 172.6 | 209.2 | 81.1 |
| 210 | 8.8 | 78.8 | 28.8 | 44 | 92.5 |
| 214 | 79 | 94.5 | 148.7 | 101.7 | 82.7 |
| 219 | 22.7 | 31.8 | 63.9 | 23.8 | 18.1 |
| 224 | 190.5 | 194.7 | 212 | 278.9 | 201.1 |
| 225 | 48.8 | 135.5 | 46.8 | 274.4 | 97.5 |
| 226 | 20 | 24 | 90.8 | 45.3 | 8.8 |
| 309 | 30.5 | 195 | 29.7 | 53.4 | 37.9 |
| 332 | 10.4 | 4.2 | 9.2 | 75 | 44 |
| 346 | 19.1 | 298.1 | 35.4 | 251.2 | 37.2 |

## 13b13

ThyFolCan_02014_400...._Signal

| SEQ ID | 84_H133A_823TT | 85_H133A_824TT | 86_H133A_825TT | 88_H133A_827TT | 89_H133A_828TT |
|---|---|---|---|---|---|
| 83 | 242 | 70.1 | 71.2 | 24.8 | 145.4 |
| 142 | 19.8 | 12.5 | 35.7 | 17.7 | 19.6 |
| 136 | 35.7 | 21 | 59.4 | 20.3 | 9 |
| 137 | 86.1 | 23.7 | 116.3 | 18.2 | 48.6 |

| 152 | 271.9 | 199.6 | 213.1 | 140.9 | 202.5 |
|---|---|---|---|---|---|
| 160 | 134.5 | 134.1 | 415.1 | 162.4 | 154 |
| 163 | 64.5 | 46.1 | 82.1 | 15.5 | 57.8 |
| 165 | 189.3 | 222.6 | 239 | 223.8 | 145.3 |
| 210 | 8.8 | 236.3 | 50 | 21.8 | 33.8 |
| 214 | 33.1 | 71.3 | 121.6 | 157.3 | 100.8 |
| 219 | 24.8 | 64.8 | 82.1 | 14.1 | 60.2 |
| 224 | 213 | 96.4 | 371.4 | 159.1 | 92.8 |
| 225 | 164.9 | 103.3 | 224.4 | 145.2 | 98.3 |
| 226 | 10.3 | 5 | 24 | 30.5 | 21.6 |
| 309 | 15 | 33.3 | 38.4 | 14.9 | 19.5 |
| 332 | 77.6 | 55.7 | 124.1 | 10.6 | 84.5 |
| 346 | 39 | 124.6 | 31.5 | 29.5 | 21.3 |

13b14

| SEQ ID | 02014_40318_H133A_840TT | Signal fc__EA40212_VDX896TT | fc__EA40214_VDX898TT | fc__EA40215_VDX899TT | fc__EA40216_VDX900TT | fc__EA40217_VDX901TT | fc__EA40218_VDX902TT |
|---|---|---|---|---|---|---|---|
| 83 | 189.9 | 110.7 | 51.4 | 35.8 | 104.6 | 22.8 | 44.7 |
| 142 | 18.9 | 20.5 | 246.2 | 18.5 | 20.7 | 25.8 | 28.6 |
| 136 | 22.6 | 105.2 | 21.3 | 20.3 | 33.6 | 24.5 | 24.4 |
| 137 | 32.3 | 40.6 | 46.3 | 33.8 | 23.9 | 90.6 | 48.5 |
| 152 | 306 | 216.1 | 317.8 | 169.4 | 154.6 | 169.3 | 374.5 |
| 160 | 99.5 | 197.5 | 240.4 | 68.7 | 146.7 | 210.5 | 196.7 |
| 163 | 52.1 | 29.7 | 70.7 | 75.3 | 13.6 | 25.1 | 59.3 |
| 165 | 236.7 | 142.2 | 147 | 168 | 174.2 | 28.5 | 89.4 |
| 210 | 26.5 | 108.8 | 39.9 | 25.5 | 42.2 | 11 | 24.4 |
| 214 | 115.1 | 63.9 | 59 | 129.9 | 150.1 | 194.1 | 218.2 |
| 219 | 16.2 | 46.2 | 62.7 | 39.4 | 42.1 | 92.3 | 39.4 |
| 224 | 203.9 | 194.6 | 321.6 | 225.2 | 238 | 212.5 | 187.3 |
| 225 | 198 | 53.6 | 82.8 | 52.5 | 250.7 | 72 | 53.5 |
| 226 | 31 | 57.3 | 111.5 | 66.7 | 24.8 | 41 | 79.9 |
| 309 | 17.2 | 40.2 | 44.3 | 43 | 20.2 | 21.3 | 63.8 |
| 332 | 51.4 | 26.5 | 129.1 | 7.8 | 5.6 | 37.8 | 31.6 |
| 346 | 11 | 27.8 | 21.6 | 19.4 | 320.2 | 24.3 | 210.6 |

13b15

| SEQ ID | fc__EA40221_VDX909TT | fc__EA40222_VDX910TT | Signal EA02014_40386_H133A_954TT | EA02014_40394_H133A_967TT | EA02014_40395_H133A_968TT | EA02014_40396_H133A_969TT | EA02014_40397_H133A_970TT |
|---|---|---|---|---|---|---|---|
| 83 | 312.2 | 30.6 | 105.2 | 42.2 | 47.9 | 24.4 | 46.5 |
| 142 | 17.8 | 9.9 | 41.7 | 30.5 | 11.8 | 16.7 | 25.7 |
| 136 | 63.1 | 87.5 | 38 | 29.7 | 206.6 | 10.1 | 99.2 |
| 137 | 170.4 | 111.7 | 78.6 | 32.1 | 20.5 | 29.4 | 18.2 |
| 152 | 624.7 | 121 | 215.4 | 212.7 | 160.9 | 216.7 | 214.3 |
| 160 | 257.9 | 30.5 | 247.6 | 161.6 | 192.1 | 87.9 | 538.8 |
| 163 | 21.6 | 27 | 43.5 | 50.7 | 56.9 | 72.4 | 48.7 |
| 165 | 251.9 | 105 | 92.7 | 136.3 | 166.5 | 123.8 | 180.7 |
| 210 | 34 | 37.1 | 49 | 43.9 | 21.1 | 7.4 | 167.3 |
| 214 | 257.9 | 176.9 | 168.4 | 69.9 | 113.6 | 140.1 | 14 |
| 219 | 117.7 | 25.9 | 43.2 | 42.1 | 24 | 12.6 | 57.9 |
| 224 | 55.1 | 150 | 176.9 | 280.1 | 184.5 | 194.4 | 374.4 |

| 225 | 205.8 | 42.3 | 66.7 | 320.3 | 27 | 66.2 | 54.6 |
| 226 | 84.9 | 14.8 | 148.6 | 26 | 47.6 | 36.9 | 21.7 |
| 309 | 745.6 | 9.7 | 53.6 | 42 | 23.5 | 14.6 | 71 |
| 332 | 15.9 | 7.6 | 69.9 | 10.3 | 63.6 | 13.3 | 26.8 |
| 346 | 48 | 25 | 54.7 | 142.6 | 73.6 | 16.6 | 79.7 |

13b16

Signal

| SEQ ID | EA02014_40405_H133A_982TT | EA02014_40406_H133A_983TT | pb1 | pb2 | pb3 | pb4 | pb5' |
|---|---|---|---|---|---|---|---|
| 83 | 58.1 | | 37.8 | 524.7 | 768.1 | 855.6 | 1163.9 | 876.3 |
| 142 | 19.4 | | 37.4 | 1289.3 | 1088.9 | 659.7 | 732.6 | 1415 |
| 136 | 94.2 | | 11 | 1770.1 | 1622.7 | 523 | 979.3 | 617.3 |
| 137 | 17.6 | | 111.2 | 1871 | 2554 | 1313.7 | 781.1 | 2087.1 |
| 152 | 255.8 | | 37.6 | 9696.4 | 9404.8 | 1099.8 | 9900.7 | 7060 |
| 160 | 54.7 | | 347.1 | 1273.2 | 1171.6 | 907 | 937.4 | 926.2 |
| 163 | 32.9 | | 59.7 | 833 | 682.5 | 559.6 | 358.6 | 467.8 |
| 165 | 71.8 | | 232.2 | 1217.8 | 986.3 | 1278.9 | 2537.1 | 1464.3 |
| 210 | 5.2 | | 33.2 | 3401 | 2546.6 | 756.3 | 4893 | 1770.8 |
| 214 | 212.6 | | 165 | 1384.3 | 1540 | 636.7 | 656.5 | 1213.9 |
| 219 | 14.1 | | 43.6 | 1692.6 | 1911.7 | 912 | 2112.2 | 1417.9 |
| 224 | 137 | | 228.2 | 2481.3 | 3104.2 | 1905.4 | 2292.4 | 2242.7 |
| 225 | 109.6 | | 153.4 | 2367.8 | 2860.8 | 1887.6 | 2052.6 | 2116.6 |
| 226 | 41.6 | | 11 | 797.2 | 969.9 | 617.1 | 626.6 | 920 |
| 309 | 20.7 | | 30.6 | 5357.5 | 5904 | 3572.6 | 6641.9 | 3580.9 |
| 332 | 6.8 | | 60.2 | 258.8 | 304.2 | 110.1 | 255.3 | 363.3 |
| 346 | 27 | | 57.3 | 1584.2 | 1705.7 | 1507.5 | 1626.3 | 1587.1 |

13b17

Signal

| SEQ ID | pb6' | pb7' | pb8' | pd10 | pd11 | pd12 | pd9 |
|---|---|---|---|---|---|---|---|
| 83 | 818.3 | 333.4 | 692.1 | 709.8 | 699.7 | 926.2 | 534.7 |
| 142 | 1287.9 | 565.2 | 2004.2 | 821.9 | 904.8 | 1619.6 | 282.5 |
| 136 | 1039 | 962.2 | 1013.5 | 1147.9 | 840.9 | 599.8 | 284 |
| 137 | 1881.4 | 667.8 | 2058.1 | 2182.1 | 1678.9 | 1632.9 | 912.1 |
| 152 | 3657.7 | 2025 | 2993.5 | 11129.5 | 11824.2 | 11963.5 | 1607 |
| 160 | 1154.5 | 301.4 | 473.2 | 1849.5 | 1424.7 | 660 | 242 |
| 163 | 628.5 | 6534.3 | 1863.4 | 148.7 | 247.4 | 230.2 | 4365.5 |
| 165 | 1485.2 | 889 | 1904.7 | 1404.2 | 1560.4 | 1123.6 | 1919.6 |
| 210 | 2729.4 | 595.1 | 5286.8 | 7026.5 | 3861.8 | 1028.8 | 1724.8 |
| 214 | 871.1 | 442.3 | 644.5 | 936.1 | 939.1 | 852 | 347.7 |
| 219 | 1216.7 | 411.6 | 959.7 | 1085.9 | 1974.4 | 1580.2 | 245.9 |
| 224 | 2039.1 | 1488.3 | 1844.2 | 2280.1 | 2823.4 | 1252.3 | 1246.6 |
| 225 | 2214.8 | 1494.6 | 2186 | 1961.1 | 2628.3 | 1184.1 | 1230.7 |
| 226 | 822.2 | 437.3 | 684.5 | 540.5 | 839.5 | 1062.8 | 273.7 |
| 309 | 3787.2 | 1401.5 | 3537 | 3522.1 | 4518.1 | 5118.1 | 931.7 |
| 332 | 218.7 | 630.6 | 462.4 | 168.4 | 220.4 | 242.2 | 322.8 |
| 346 | 1682.4 | 909 | 2317.6 | 1193.5 | 1748.9 | 2159 | 622.7 |

Table 14 Control Genes

| Control Genes for Blood | |
|---|---|
| SEQ ID NO: | Gene Name |
| 142 | Bone marrow stromal cell antigen 1 (BST1) |
| 219 | Leucocyte immunoglobulin-like receptor-6b (LIR-6) |
| 309 | Bridging integrator 2 (BIN2) |
| Control Genes for Thyroid | |
| 9 | Cysteine-rich, angiogenic inducer, 61 (CYR61) |
| 12 | Selenoprotein P, plasma, 1 (SEPP1) |
| 18 | Insulin-like growth factor-binding protein 4 (IGFBP4) |

Table 15a

| SEQ ID | BN_800TT | BN_801TT | BN_802TT | BN_804TT | BN_805TT | BN_806TT | BN_807TT |
|---|---|---|---|---|---|---|---|
| 142 | 28.3 | 7.9 | 15 | 28.4 | 26.9 | 25.8 | 14.2 |
| 219 | 217 | 19.6 | 7.9 | 85.6 | 21.8 | 26.7 | 115.1 |
| 309 | 257.7 | 20.6 | 20.7 | 75.5 | 19.2 | 24 | 833.9 |

| | BN_871TT | BN_913TT | BN_914TT | BN_915TT | FA_917TT | FA_918TT | FA_919TT |
|---|---|---|---|---|---|---|---|
| 142 | 25.3 | 22.5 | 12.9 | 34.4 | 8.5 | 20.6 | 67.7 |
| 219 | 56.6 | 53.5 | 45.2 | 122.1 | 24.4 | 124 | 41.3 |
| 309 | 37.9 | 37 | 28.2 | 53 | 10.2 | 32.6 | 24.2 |

| | FA_818TT | FA_819TT | FA_820TT | FA_821TT | FA_822TT | FA_842TT | FA_862TT |
|---|---|---|---|---|---|---|---|
| 142 | 15 | 20.2 | 18.8 | 18.4 | 23 | 10 | 16.1 |
| 219 | 18.7 | 111.8 | 45.7 | 10.9 | 29.6 | 28.9 | 80.6 |
| 309 | 16.8 | 17.1 | 12.1 | 18.8 | 27.3 | 15.2 | 6.7 |

| | FA_863TT | FA_864TT | FA_865TT | FA_866TT | FA_867TT | FA_869TT | FA_907TT |
|---|---|---|---|---|---|---|---|
| 142 | 22.4 | 14.5 | 54.8 | 17.1 | 15.8 | 15 | 21.9 |
| 219 | 7.6 | 15.9 | 20.9 | 25 | 10 | 16.5 | 23.4 |
| 309 | 15.2 | 9.4 | 62.6 | 20.2 | 18.4 | 15.9 | 10.2 |

| | FA_908TT | FA_920TT | FA_938TT | FA_940TT | FA_941TT | Fa_EA403741_921TT | Fa_EA40376_923TT |
|---|---|---|---|---|---|---|---|
| 142 | 23.9 | 22.2 | 7.9 | 6.6 | 14.5 | 13.7 | 19.8 |
| 219 | 20.1 | 36.4 | 17.5 | 14.9 | 63.3 | 32.3 | 25.5 |
| 309 | 33.3 | 27.1 | 7.7 | 6.2 | 15.6 | 26.1 | 16 |

| | BN_EA40377_924TT | Fa_EA40378_925TT | Fa_EA40379_926TT | BN_EA40380_927TT | Fa_EA40387_955TT | Fa_EA40388_956TT | Fa_EA40389_957TT |
|---|---|---|---|---|---|---|---|
| 142 | 16.5 | 57.7 | 22.9 | 18.9 | 16.7 | 14.2 | 10.8 |
| 219 | 15.3 | 10.2 | 95.6 | 18.5 | 23.3 | 51 | 29.1 |
| 309 | 17.8 | 31.8 | 32.6 | 49.2 | 18.7 | 11.4 | 26.7 |

| | Fa_EA40390_959TT | Fa_EA40391_960TT | Fa_EA40392_961TT | Fa_EA40393_962TT | PC_829TT | PC_830TT | PC_831TT |
|---|---|---|---|---|---|---|---|
| 142 | 21.4 | 18.6 | 16.5 | 21.9 | 26 | 34.7 | 22.4 |
| 219 | 25.7 | 67 | 20.8 | 21 | 36.8 | 136.4 | 64.9 |
| 309 | 44.2 | 20.6 | 34.1 | 35.2 | 88.8 | 136.6 | 48.8 |

| | PC_832TT | PC_834TT | PC_835TT | PC_836TT | PC_837TT | PC_838TT | PC_839TT |
|---|---|---|---|---|---|---|---|
| 142 | 19.1 | 16.4 | 25.8 | 12.1 | 22.5 | 15.4 | 16.1 |
| 219 | 24.3 | 19.1 | 25.9 | 11.7 | 13.6 | 23.8 | 26.8 |
| 309 | 22.1 | 23.6 | 40.5 | 16.5 | 33.9 | 24.7 | 340.8 |

| | PC_879TT | PC_881TT | PC_882TT | PC_883TT | PC_884TT | PC_885TT | PC_886TT |
|---|---|---|---|---|---|---|---|
| 142 | 21.2 | 23.5 | 7.6 | 21.5 | 30.6 | 26.4 | 22.6 |
| 219 | 30.4 | 13.3 | 78.6 | 34.3 | 34.8 | 131.8 | 12 |
| 309 | 33.7 | 32 | 22.8 | 29.8 | 45.9 | 89.7 | 47.4 |
| | PC_890TT | PC_892TT | PC_893TT | PC_894TT | PC_903TT | PC_904TT | PC_928TT |
| 142 | 8.7 | 10.8 | 18.5 | 23.6 | 14.5 | 118.3 | 7.4 |
| 219 | 25.9 | 48.9 | 14.4 | 96.2 | 15.1 | 80.2 | 27.7 |
| 309 | 58.6 | 17.8 | 23.5 | 72.3 | 11.1 | 270.7 | 15.9 |
| | PC_932TT | PC_933TT | Pc_EA40375_922TT | Pc_EA40381_945TT | Pc_EA40382_946TT | Pc_EA40383_947TT | Pc_EA40384_948TT |
| 142 | 8 | 13 | 12 | 19.6 | 13.2 | 21.6 | 16.9 |
| 219 | 54.2 | 38.2 | 22.7 | 31.8 | 63.9 | 23.8 | 18.1 |
| 309 | 91.5 | 18.6 | 30.5 | 195 | 29.7 | 53.4 | 37.9 |
| | FC_823TT | FC_824TT | FC_825TT | FC_827TT | FC_828TT | FC_840TT | FC_896TT |
| 142 | 19.8 | 12.5 | 35.7 | 17.7 | 19.6 | 18.9 | 20.5 |
| 219 | 24.8 | 64.8 | 82.1 | 14.1 | 60.2 | 16.2 | 46.2 |
| 309 | 15 | 33.3 | 38.4 | 14.9 | 19.5 | 17.2 | 40.2 |
| | FC_898TT | FC_899TT | FC_900TT | FC_901TT | FC_902TT | FC_909TT | FC_910TT |
| 142 | 246.2 | 18.5 | 20.7 | 25.8 | 28.6 | 17.8 | 9.9 |
| 219 | 62.7 | 39.4 | 42.1 | 92.3 | 39.4 | 117.7 | 25.9 |
| 309 | 44.3 | 43 | 20.2 | 21.3 | 63.8 | 745.6 | 9.7 |
| | Fc_EA40386_954TT | Fc_EA40394_967TT | Fc_EA40395_968TT | Fc_EA40396_969TT | Fc_EA40397_970TT | Fc_EA40405_982TT | Fc_EA40406_983TT |
| 142 | 41.7 | 30.5 | 11.8 | 16.7 | 25.7 | 19.4 | 37.4 |
| 219 | 43.2 | 42.1 | 24 | 12.6 | 57.9 | 14.1 | 43.6 |
| 309 | 53.6 | 42 | 23.5 | 14.6 | 71 | 20.7 | 30.6 |
| | pbl_Signal | pb2_Signal | pb3_Signal | pb4_Signal | pb5'_Signal | pb6'_Signal | pb7'_Signal |
| 142 | 1289.3 | 1088.9 | 659.7 | 732.6 | 1415 | 1287.9 | 565.2 |
| 219 | 1692.6 | 1911.7 | 912 | 2112.2 | 1417.9 | 1216.7 | 411.6 |
| 309 | 5357.5 | 5904 | 3572.6 | 6641.9 | 3580.9 | 3787.2 | 1401.5 |
| | pb8_Signal | pd10_Signal | pd11_Signal | pd12_Signal | pd9_Signal | | |
| 142 | 2004.2 | 821.9 | 904.8 | 1619.6 | 282.5 | | |
| 219 | 959.7 | 1085.9 | 1974.4 | 1580.2 | 245.9 | | |
| 309 | 3537 | 3522.1 | 4518.1 | 5118.1 | 931.7 | | |

EP 2 518 166 B1

160

## Table 15b
### Signal

| SEQ ID | PC_984TT | PC_986TT | FA_987TT | PC_988TT | PC_989TT | FA_992TT | FA_993TT |
|---|---|---|---|---|---|---|---|
| 142 | 25.1 | 125.8 | 18.5 | 11.4 | 13.7 | 11.8 | 113.6 |
| 219 | 17.5 | 25.7 | 22.6 | 11.3 | 32.4 | 61.9 | 25.2 |
| 309 | 27.6 | 661.8 | 15.7 | 47.4 | 29.8 | 33.2 | 28.4 |
| | FA_994TT | FA_995TT | FA_996TT | FA_998TT | FA_999TT | FA_1001TT | FA_1002TT |
| 142 | 20.6 | 25.2 | 34.1 | 21.8 | 31.4 | 12.7 | 74.6 |
| 219 | 25.9 | 36.3 | 41.5 | 24.9 | 16.4 | 26.5 | 25.5 |
| 309 | 16.8 | 26.3 | 19.4 | 22.5 | 14.6 | 32.3 | 47.7 |
| | FA_1004TT | FA_1005TT | FA_1006TT | FA_1010TT | FA_1013TT | FA_1014TT | FA_1017TT |
| 142 | 24.7 | 20.1 | 67.7 | 14.9 | 48.9 | 29.8 | 26.9 |
| 219 | 27.5 | 11.7 | 53.3 | 112.9 | 35.1 | 39.5 | 27.2 |
| 309 | 39.5 | 10.3 | 34.6 | 83.2 | 46.8 | 20 | 25.7 |
| | FA_1018TT | FA_1020TT | FA_1023TT | FA_1024TT | FA_1026TT | FA_1027TT | FA_1028TT |
| 142 | 25.1 | 25.5 | 35.6 | 26.9 | 27.9 | 15.2 | 24.5 |
| 219 | 66.3 | 26 | 6.1 | 26 | 59.3 | 18.8 | 31.7 |
| 309 | 31 | 37.4 | 20.6 | 46.9 | 51.4 | 8.8 | 33.3 |
| | FA_1029TT | FA_1030TT | FA_1031TT | FA_1032TT | FA_1034TT | FA_1035TT | FC_1037TT |
| 142 | 10.6 | 14 | 20 | 21.6 | 31.9 | 17.1 | 17.8 |
| 219 | 12.5 | 33.5 | 15.1 | 15 | 68.5 | 25.6 | 63.5 |
| 309 | 20.7 | 13 | 21.5 | 32.2 | 52.6 | 24.6 | 26.2 |
| | PC_1039TT | PC_1040TT | PC_1041TT | PC_1042TT | PC_1043TT | PC_1044TT | PC_1045TT |
| 142 | 16.1 | 22.7 | 99.5 | 129.8 | 20.7 | 79.2 | 16.4 |
| 219 | 27.9 | 29.5 | 23.6 | 29 | 21.5 | 19.8 | 38.4 |
| 309 | 585.3 | 37.6 | 48.7 | 41 | 32.8 | 59.2 | 112.3 |
| | PC_1046TT | PC_1047TT | PC_1048TT | PC_1049TT | PC_1050TT | PC_1051TT | PC-FV_1052TT |
| 142 | 22.4 | 81.2 | 24.7 | 37.5 | 25.9 | 26.6 | 26.4 |
| 219 | 11.1 | 97.5 | 61.8 | 14.9 | 14.7 | 109.1 | 30.6 |
| 309 | 22.5 | 207.8 | 35.4 | 64 | 39.5 | 56.7 | 28.2 |
| | PC_1053TT | PC_1054TT | PC_1055TT | PC_1059TT | PC_1060TT | PC_1061TT | PC_1062TT |
| 142 | 12.8 | 18.5 | 24.4 | 22.7 | 24.6 | 15.8 | 25.6 |
| 219 | 12.6 | 19.6 | 10.2 | 24.5 | 82.6 | 53.7 | 20.7 |
| 309 | 20.4 | 22.1 | 24 | 18.9 | 32.3 | 54.5 | 29.1 |

EP 2 518 166 B1

(continued)

| | PC-FV_1064TT | PC-FV_1065TT | PC-FV_1066TT | PC-FV_1067TT | PC-FV_1068TT | PC-FV_1069TT | PC-FV_1071TT |
|---|---|---|---|---|---|---|---|
| 142 | 16.7 | 11.3 | 25.5 | 17.4 | 17.5 | 14 | 16.7 |
| 219 | 174.4 | 20.3 | 132.4 | 24.8 | 12.3 | 14.8 | 18.8 |
| 309 | 30.4 | 169.6 | 49.4 | 16.9 | 135.5 | 18.1 | 7.6 |
| | PC-FV_1072TT | FA_1073TT | FA_1074TT | FA_1075TT | FA_1076TT | FC_1077TT | FC_1078TT |
| 142 | 14 | 25.5 | 22.7 | 27 | 16.4 | 15.8 | 38.9 |
| 219 | 8.7 | 73.2 | 57 | 54.3 | 69.9 | 20 | 17.1 |
| 309 | 29.9 | 145.4 | 21 | 53 | 24.3 | 9.1 | 32.9 |
| | FC_1079TT | PC-FV_1080TT | PC-FV_1081TT | FC_1082TT | pb1 | pb2 | pb3 |
| 142 | 31.9 | 10.2 | 22 | 19.5 | 1289.3 | 1088.9 | 659.7 |
| 219 | 40.1 | 17.4 | 38.3 | 88.1 | 1692.6 | 1911.7 | 912 |
| 309 | 27.1 | 23.4 | 40.8 | 12.9 | 5357.5 | 5904 | 3572.6 |
| | pb4 | pb5' | pb6' | pb7' | pb8' | pd10 | pd11 |
| 142 | 732.6 | 1415 | 1287.9 | 565.2 | 2004.2 | 821.9 | 904.8 |
| 219 | 2112.2 | 1417.9 | 1216.7 | 411.6 | 959.7 | 1085.9 | 1974.4 |
| 309 | 6641.9 | 3580.9 | 3787.2 | 1401.5 | 3537 | 3522.1 | 4518.1 |
| | pd12 | pd9 | | | | | |
| 142 | 1619.6 | 282.5 | | | | | |
| 219 | 1580.2 | 245.9 | | | | | |
| 309 | 5118.1 | 931.7 | | | | | |

F. Signature Normalized to Control Genes

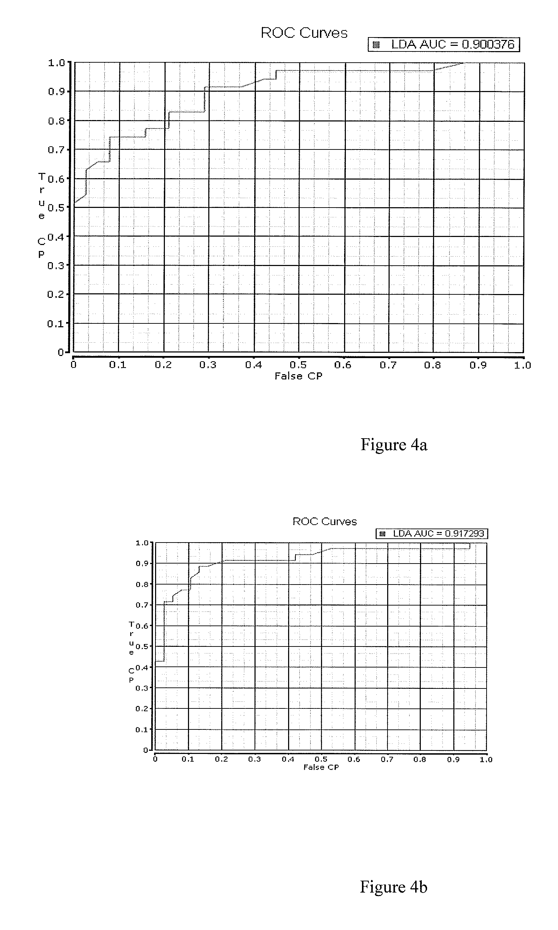

We further examined our 5-gene and 4-gene signatures by normalizing these genes to the three selected thyroid control genes as an algorithm for gene chip data normalization. The average fluorescent intensities of the three thyroid control genes were used for signature gene signal normalization. The performance of both signatures improved slightly when these two signatures were normalized. The sensitivity and specificity of the two signatures are listed in Table 16, and the ROC curves are shown in Figure 4a and 4b.

Table 16. The performances of the 4-gene and 5-gene signatures normalized to control genes

| 4-Gene Signature | | |
| --- | --- | --- |
| | Tumor | Benign |
| Positive | 33 | 11 |
| Negative | 3 | 27 |
| Sensitivity | 92% (0.78, 0.97) | |
| Specificity | 71% (0.55, 0.83) | |
| 5-Gene Signature | | |
| | Tumor | Benign |
| Positive | 33 | 8 |
| Negative | 3 | 30 |
| Sensitivity | 92% (0.78, 0.97) | |
| Specificity | 79% (0.64, 0.89) | |

G. Signature Validation with Two-Round Amplified Probes

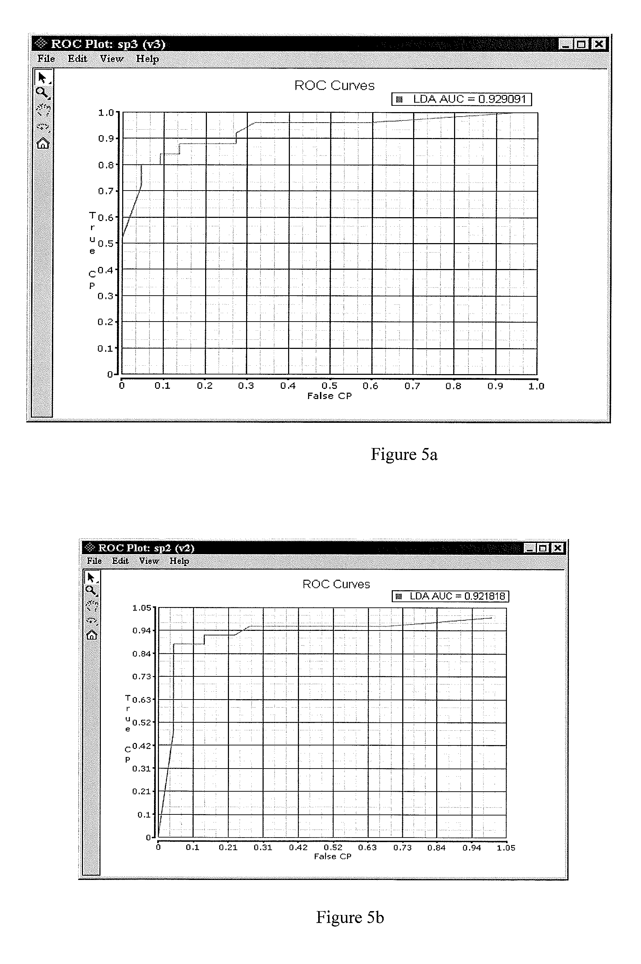

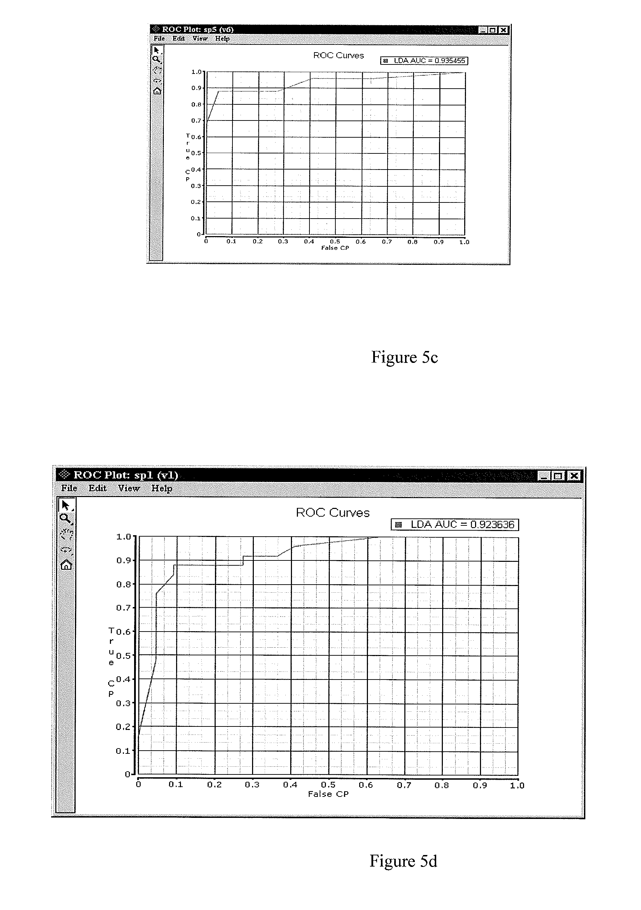

To determine if the FNA samples lack sufficient thyroid cells to provide enough probe material for hybridizing to the Affymetrix U133a gene chips after one round of amplification, two-round amplification of the target RNAs we performed two-round amplification with 47 samples that are among the 74 independent validation sample set. The data obtained show that the performances of the 5-gene and 4-gene signatures are identical with either one-round or two-round amplifications. The ROC curves of the two gene signatures with two different target preparations are shown in Figures 5a, 5b, 5c, and 5d.

**Example 4**

**Cross validation with independent samples**

A. Cross Validation with the 83 Independent Fresh Frozen Thyroid Samples

83 independent thyroid samples were processed and profiled with the U133a chip. The number of samples in each category is list in Table 17.

Table 17. Fresh Frozen sample collection for signature validation

| Sample Type | Number of Samples |
| --- | --- |
| Follicular Adenoma | 18 |
| Follicular Thyroid Carcinoma | 1 |
| Papillary Carcinoma, Follicular Variant | 18 |
| Papillary Thyroid Carcinoma | 11 |
| Adenomatoid Nodules | 18 |
| Adenomatoid Nodules w/Hashimoto | 1 |
| Multinodular Hyperplasia | 16 |

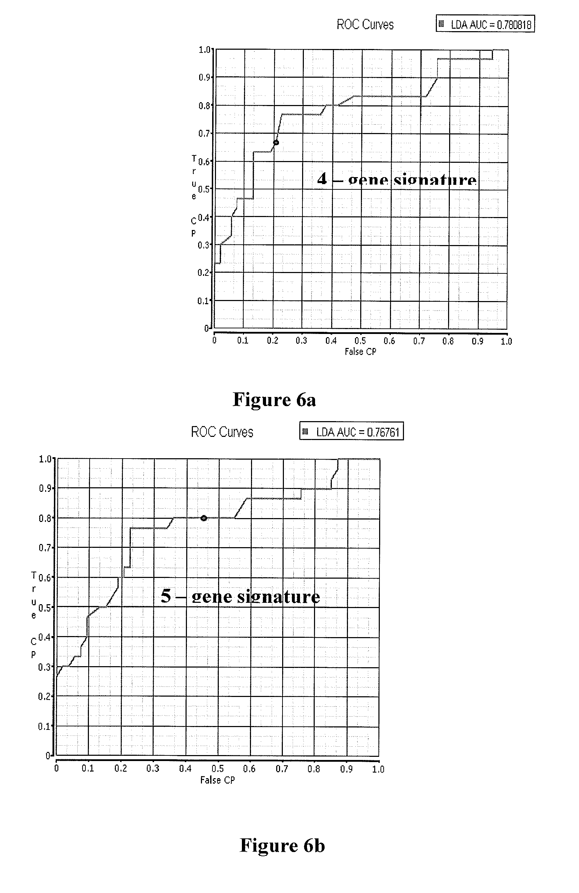

The performance of the 4-gene signature and the 5-gene signature was assessed with LDA using the same cut-off value as in the training set. Both signatures gave equivalent performance in these samples, and they are comparable with the performance in the 98 training set. The sensitivity and specificity for both signatures are shown in Table 18, and the ROC curves are demonstrated in Figures 6a and 6b.

Table 18. 4-Gene and 5-gene signatures performance in 83 validation samples

**4-Gene Signature**

|  | Tumor | Benign |
|---|---|---|
| Positive | 20 | 11 |
| Negative | 10 | 42 |
| Sensitivity | 67% (0.49, 0.81) | |
| Specificity | 79% (0.67, 0.88) | |

**5-Gene Signature**

|  | Tumor | Benign |
|---|---|---|
| Positive | 24 | 24 |
| Negative | 6 | 29 |
| Sensitivity | 80% (0.63, 0.90) | |
| Specificity | 55% (0.41, 0.67) | |

B. Signature Validation with the 47 Fine Needle Aspirate (FNA) Thyroid Samples

47 thyroid FNA samples were processed and profiled with the U133a chip. The number of samples in each category is list in Table 19.

Table 19. FNA sample collection for signature validation

| Sample Type | Number of Samples |
|---|---|
| Follicular Adenoma | 10 |
| Follicular Thyroid Carcinoma | 2 |
| Papillary Carcinoma, Follicular Variant | 3 |
| Papillary Thyroid Carcinoma | 13 |
| Adenomatoid Nodules | 10 |
| Adenomatoid Nodules w/Hashimoto | 1 |
| Multinodular Hyperplasia | 8 |

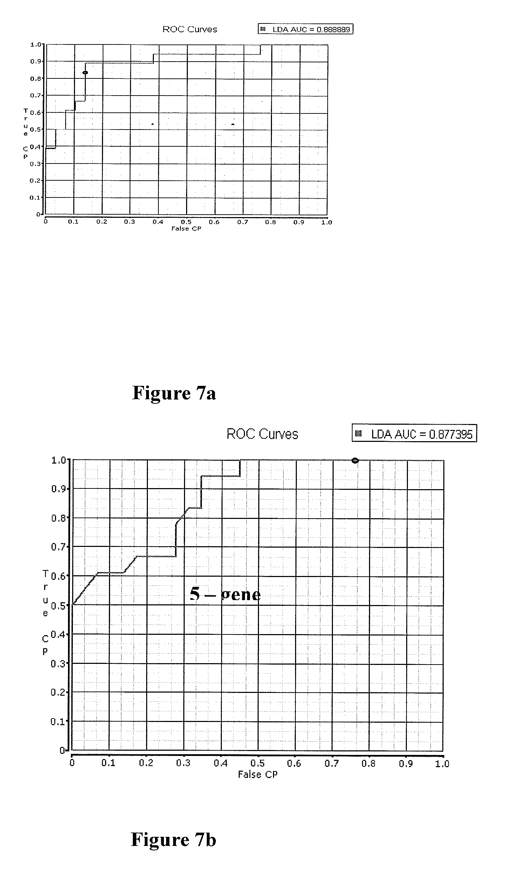

The performance of the 4-gene signature and the 5-gene signature was assessed with LDA model. Both signatures gave equivalent performance in the FNA samples, and they are comparable with the performance in the 98 training set. The sensitivity and specificity for both signatures are shown in Table 20, and the ROC curves are demonstrated in Figures 7a and 7b.

Table 20. 4-Gene and 5-gene signatures performance in 47 FNA samples

**4-Gene Signature**

|  | Tumor | Benign |
|---|---|---|
| Positive | 15 | 4 |
| Negative | 3 | 25 |
| Sensitivity | 94% (0.74, 0.99) | |
| Specificity | 62% (0.44, 0.77) | |

**5-Gene Signature**

|  | Tumor | Benign |
|---|---|---|
| Positive | 17 | 10 |
| Negative | 1 | 19 |
| Sensitivity | 94% (0.74, 0.99 | |
| Specificity | 66% (0.47, 0.80) | |

C. Signature Performance in 28 Paired Fresh Frozen and FNA Thyroid Samples

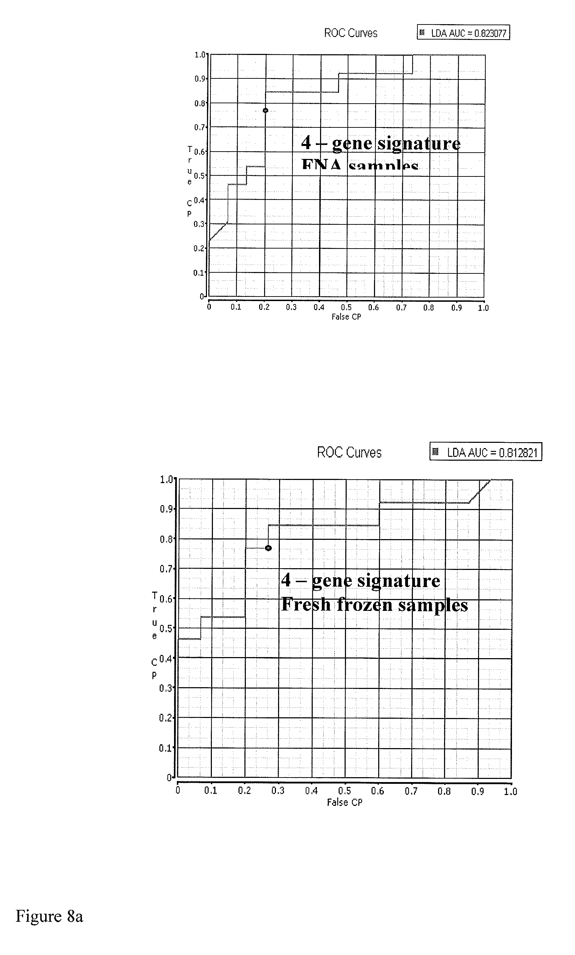

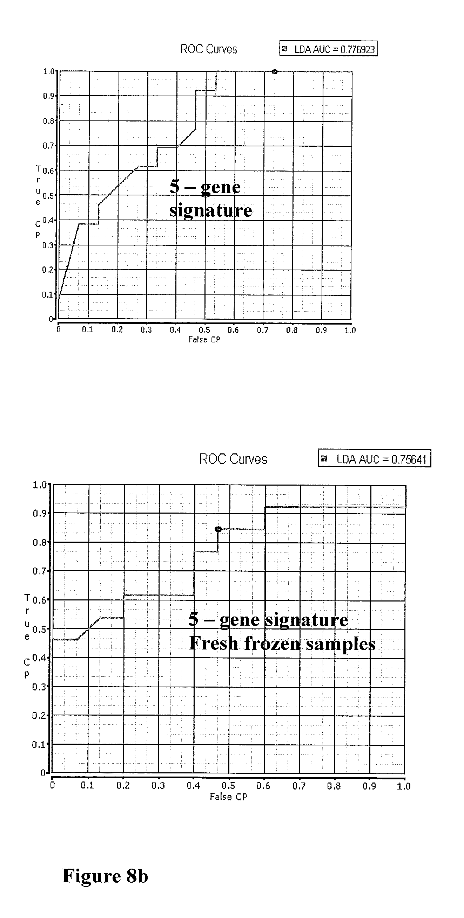

Within the 83 fresh frozen and the 47 FNA sample collections there are 28 samples that were from the same patient. The direct comparison of our signatures in these paired samples demonstrates how well the signature will translate into the final molecular assay. The performance of the 4-gene signature and the 5-gene signature was assessed with the LDA model. Both signatures gave equivalent performance in the fresh frozen and FNA samples. These results demonstrated that our 4-gene and 5-gene signatures can perform equally well in both sample types, and proved the approach using fresh frozen samples for gene/signature identification is valid. The sensitivity and specificity for both signatures are shown in Table 21, and the ROC curves are demonstrated in Figures 8a and 8b.

Table 21. 4-Gene and 5-gene signatures performance in 28 matched fresh frozen and FNA samples

| 4-Gene Signature | | | | | |
|---|---|---|---|---|---|
| | Fresh Frozen | | FNA | | |
| | Tumor | Benign | Tumor | Benign | |
| Positive | 10 | 4 | 12 | 7 | |
| Negative | 3 | 11 | 1 | 8 | |
| Sensitivity | 77% (0.50 - 0.92) | | 92% (0.67 - 0.99) | | |
| Specificity | 73% (0.48 - 0.89) | | 53% (0.30 - 0.75) | | |
| 5-Gene Signature | | | | | |
| | Fresh Frozen | | FNA | | |
| | Tumor | Benign | Tumor | Benign | |
| Positive | 11 | 7 | 12 | 7 | |
| Negative | 2 | 8 | 1 | 8 | |
| Sensitivity | 85% (0.58 - 0.96) | | 92% (0.67 - 0.99) | | |
| Specificity | 53% (0.30 - 0.75) | | 53% (0.30 - 0.75) | | |

**Example 5**

**Real-time quantitative RT-PCR**

**Sample Acquisition:**

In order to determine whether a subset of the gene profiles and/or controls would give adequate specificity and sensitivity with RT-PCR, the following experiment was performed. The following has the advantage of requiring only one round of RNA amplification.

A total of 107 thyroid biopsies were analyzed in our study: 26 follicular adenoma, 23 follicular carcinoma, 38 papillary carcinoma, 5 normal, 3 papillary carcinoma follicular variant, 3 Hashimoto thyroiditis, 2 microfollicular adenoma, 1 diffuse goiter, 1 goiter with papillary hyperplasia, 1 Hurthle cell adenoma, 1 hyperplasia with papillary structure, 1 multinodular goiter, 1 oncocytic hyperplasia, 1 thyroiditis. Total RNA isolation was extracted by using the Trizol reagent according to the manufacturer's instructions. RNA concentrations were determined by absorbance readings at 260 nm with the Gene-Spec (Hitachi) spectrophotometer. The isolated RNA was stored in RNase-free water at -80°C until use.

**Primer and Probe Design:**

The primers and hydrolysis probes were designed using Oligo 6.0 and the Genebank sequences for thyroid cancer status markers (Table 22). These primers and probe sets were designed such that the annealing temperature of the primers was 62°C and the probes 5-10°C higher and amplicon size ranges from 100-150bp. Genomic DNA amplification was excluded by designing our primers around exon-intron splicing sites. Hydrolysis probes were labeled at the 5' nucleotide with FAM as the reporter dye and at 3' nucleotide with TAMRA as the quenching dye.

*Table 22*

| Target | Accession # | Forward Primer (SEQ ID NO:) | Reverse Primer (SEQ ID NO:) | Probe Sequence (SEQ ID NO:) | Product Length |
|---|---|---|---|---|---|
| SGENE | NM_003020 | gaaagcggaggagtgtcaatcca (364) | ggttttcgtctagcatcttctcttta (365) | atctacaaggacagaqactggataatgttg (366) | 130 |
| TESTICAN1 | AF231124 | gtgagctgtgaagaggagcaggaa (367) | ctttggtcccagctcccgttca (368) | cctcaggggattttggcagtggtgggtccg (369) | 102 |
| GABRE | NM_004961 | taaactccgccatcctcgtatcaa (370) | cagtggtgacaatctggcacacaa (371) | ccgtcccatgcccagtacccgtgca (372) | 113 |
| CDH3 | NM_001793 | ctgaagcaggatacatatgacgtgca (373) | aggggtccagggcaggtttcgaca (374) | catggcagtcgcacacagtggccctga (375) | 124 |
| FN1 | NM_002026 | tggtgccatgacaatggtgtgaacta (376) | catcatcgtaacacgttgcctcatga (377) | aagattggagagaagtgggaccatcaggga (378) | 148 |
| TPO-1 | NM_000547 | catctgtgacaacactggcctca (379) | gccacacttgtcgtcttgaggaa (380) | caaattccccgaagactttgagtcttgtgacagc (381) | 148 |
| TPO-2 | NM_000547 | aacctgcgtagactccgggaggc (382) | gccagtgcgtgccacctgca (383) | ctcgggtgacttggatctccatgtcgctgg (384) | 124 |
| KCNAB1-1 | NM_003471 | tgaaagttccagggcttcactgaa (385) | agctgaggtagtgtgcatcccaga (386) | ctaccagtggttgaaagaaagaattgtaagtqaag (387) | 138 |
| KCNAB1-2 | NM_003471 | tagctgttgcgtggtgcctgagaa (388) | tgatgtcatctttgggagaacctgaa (389) | aaggtgtgagttctgtgctcctgggatcat (390) | 119 |
| FABP4-1 | NM_001442 | gaaagaagtaggagtgggctttgcca (391) | ggcccagtatgaagaaaatctcagta (392) | aaaggtactttcagatttaatggtgatcacatccc (393) | 143 |
| FABP4-2 | NM_001442 | aggaaagtcaagagcaccataacctta (394) | gacgcattccaccaccagtttatca (395) | ttgattttccatcccatttctgcacatgta (396) | 123 |
| DOI1-1 | NM_000792 | gggtaaatctgqcccttggaactaca (397) | cccgttggtcacctagaattgaggta (398) | cccagaggaagttcgtgctgttctggaaaa (399) | 106 |
| DOI1-2 | NM_000792 | tgcatcagatggctgggctttta (400) | gctctggttctgcatggtgtcca (401) | catcagaaatcaccagaaccttcaggatcg (402) | 142 |
| B-ACTI N | NM_001101 | tcacccacactgtgcccatctacga (403) | cagcggaaccgctcattgccaatgg (404) | atgccctcccccatgccatcctgcgt (405) | 295 |
| GOLGIN67 | NM_015003 | gcatggtgatctttgtgaggcga (406) | ctcctggtggtcctgcatctca (407) | ctcaccaacagcgtggagcctgcacaagga (408) | 139 |
| PAX8 | BC001060 | actccagcttgctgagttcccca (409) | actccagcttgctgagttcccca (410) | attattacagttccacatcaaggccgagtgca (411) | 125 |
| HERC | NM_003922 | gggtgcttttcatgaggtttgtgtca (412) | tgaggtaggcagactgtcgtaaggcc (413) | ccaacactgctgacatttctcagagatttcaaat (414) | 219 |
| DDIT3 | NM_004083 | cctggaaatgaagagqaagaatcaa (415) | agctctgactggaatctggagagtga (416) | aatcttcaccactcttgaccctctcttctctgg (417) | 142 |
| ITM1 | NM_152713 | tggctggtcaggatatacaaggtaaa (418) | tcaacgtcctaaatgtgatgtgctca (419) | cctggataatcgaggcttgtcaaggacataaa (420) | 117 |
| C1OF24 | NM_052966 | tctgaaagtgataaaggaagctgcta (421) | ctttagatctgttaagctggacacac (422) | cttgaagaaacacaacttatttgaagataacatg (423) | 103 |
| MOT8 | NM_018836 | acggcctataacaaaaccctgca (424) | gaagaggagggtcggtttccattaa (425) | ttctcacgagtgcgtcagggcatctgtgc (426) | 133 |
| ARG2 | NM_00172 | atttgaccctacactggctccagc (427) | gatccagtgctgatagcaaccctgta (428) | actcctgttgtcgggggactaacctatcgaga(429) | 119 |

**Real-Time Quantitative RT-PCR:**

Gene specific real-time quantitative RT-PCR amplification of 21 thyroid cancer status genes and a housekeeping gene was performed using the TaqMan One-Step RT-PCR Master Mix (2x) (Applied Biosystems) and the ABI Prism 7900HT sequence detection system (Applied Biosystems). In a 25μl one-step reaction total RNA (10ng) was added to a mix that contained: 1x RT-PCR Master Mix, 0.25U/μl Multiscribe Enzyme, 0.6uM primers and 0.25μM probe. Cycling parameters were 48°C for 30min and 95°C 10min, followed by 40 cycles of 95°C 15 sec and 62°C 1min. Real-time PCR monitoring was achieved by measuring fluorescent signal at the end of the annealing phase for each cycle. The number of cycles to reach the fluorescence threshold was defined as the cycle threshold (Ct value). To minimize the errors arising from the integrity of the RNA in the samples β-actin mRNA was amplified as an internal reference. External standards were prepared by 10-fold serial dilutions of known thyroid cancer positive RNA and used to ensure linearity throughout our assays. Results were expressed in mean Ct value and samples were excluded that had a standard deviation greater then one. The results are provided in Tables 23a, 23b, 23c, 24a and 24b.

The data show that the two gene signature shown in Table 23b is not as sensitive and specific as the four-gene signature from which it was derived. Table 24 shows that use of the PAX8 gene in an RT-PCR reaction as a control for thyroid-specific tissue is effective in an RT-PCR reaction.

Table 23a

|  |  |  | Bactin | CDH3 | FN1 | GAB | TEST | SGE NE | KCN12 57 | KCN1 120 | TPO2 166 | TPO2 592 | FABP 4;110 | FABP 4-2 | DIO1; 480 | DIO1; 687 | DDIT 3 | C1OF 24 | Itm1 |
|---|---|---|---|---|---|---|---|---|---|---|---|---|---|---|---|---|---|---|---|
| % total |  |  |  | 44.86 | 44.86 | 48.60 | 58.88 | 57.01 | 62.62 | 48.60 | 41.12 | 30.84 | 38.32 | 42.06 | 39.25 | 56.07 | 44.86 | 45.79 | 42.99 |
| % false positives |  |  |  |  |  |  |  |  |  |  |  |  |  |  |  |  |  |  |  |

(Thyroid markers span: KCN12 57, KCN1 120, TPO2 166, TPO2 592)

Positive
Outliers
Markers with best performance

|  |  |  | Bactin | CDH3 | FN1 | GAB | TEST | SGE NE | KCN12 57 | KCN1 120 | TPO2 166 | TPO25 92 | FABP 4;110 | FABP 4-2 | DIO1; 480 | DIO1; 687 | DDIT 3 | C1OF 24 | Itm1 |
|---|---|---|---|---|---|---|---|---|---|---|---|---|---|---|---|---|---|---|---|
| 70 | 915 | ben DG | 26.69 | 34.23 | 29.35 | 31.94 | 32.00 | 33.87 | 30.70 | 32.46 | 20.69 | 21.83 | 29.87 | 28.76 | 24.78 | 24.09 | 29.54 | 31.01 | 26.46 |
| 7 | 818 | ben FA | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 |
| 24 | 842 | ben FA | 25.92 | 31.74 | 29.97 | 30.96 | 32.98 | 32.54 | 27.45 | 29.62 | 23.88 | 24.44 | 32.08 | 34.09 | 27.82 | 27.42 | 29.76 | 33.81 | 27.09 |
| 28 | 862 | ben FA | 26.86 | 33.35 | 30.38 | 30.92 | 41.29 | 50.00 | 33.60 | 32.95 | 22.50 | 22.54 | 32.02 | 32.16 | 25.63 | 25.24 | 28.44 | 38.86 | 27.25 |
| 29 | 863 | ben FA | 24.56 | 30.03 | 29.91 | 29.85 | 28.49 | 27.17 | 25.73 | 26.11 | 22.03 | 22.89 | 29.05 | 28.82 | 24.74 | 24.42 | 27.71 | 30.25 | 24.57 |
| 30 | 864 | ben FA | 26.15 | 32.18 | 30.74 | 31.97 | 34.52 | 30.22 | 26.65 | 28.72 | 21.76 | 22.65 | 30.06 | 30.15 | 25.67 | 25.30 | 28.23 | 31.93 | 25.59 |
| 31 | 865 | ben FA | 26.21 | 32.94 | 27.23 | 31.29 | 32.07 | 30.73 | 29.70 | 31.31 | 21.87 | 22.37 | 31.25 | 31.31 | 24.25 | 23.83 | 30.19 | 47.61 | 26.83 |
| 32 | 866 | ben FA | 27.00 | 33.10 | 29.02 | 32.26 | 34.44 | 32.77 | 29.59 | 31.19 | 23.32 | 23.43 | 33.18 | 33.81 | 25.94 | 25.57 | 29.26 | 31.60 | 26.44 |
| 33 | 867 | ben FA | 24.89 | 31.72 | 30.52 | 31.88 | 32.29 | 33.85 | 29.50 | 30.65 | 21.88 | 21.95 | 27.71 | 27.69 | 24.90 | 24.73 | 29.33 | 31.53 | 26.71 |
| 34 | 869 | ben FA | 24.99 | 29.22 | 28.94 | 29.99 | 27.17 | 27.98 | 27.32 | 28.52 | 21.24 | 22.14 | 30.69 | 30.08 | 25.88 | 25.39 | 27.39 | 29.46 | 25.00 |
| 64 | 907 | ben FA | 25.09 | 31.42 | 26.64 | 29.49 | 33.83 | 35.86 | 29.98 | 31.05 | 20.92 | 21.81 | 31.21 | 50.00 | 22.49 | 22.55 | 29.63 | 30.46 | 25.79 |
| 73 | 920 | ben FA | 25.37 | 34.29 | 28.40 | 30.93 | 33.48 | 50.00 | 31.87 | 32.83 | 21.43 | 21.83 | 30.14 | 28.91 | 22.40 | 23.30 | 29.53 | 28.90 | 25.04 |
| 83 | 937 | ben FA | 23.32 | 33.55 | 28.06 | 32.56 | 34.45 | 36.65 | 25.48 | 26.62 | 19.17 | 20.06 | 27.50 | 25.18 | 24.69 | 23.50 | 27.15 | 32.90 | 24.30 |
| 84 | 938 | ben FA | 23.39 | 31.45 | 25.13 | 31.58 | 29.82 | 26.36 | 27.98 | 28.60 | 20.84 | 21.34 | 29.53 | 27.24 | 22.26 | 22.03 | 27.77 | 29.88 | 25.01 |
| 85 | 939 | ben FA | 25.58 | 34.57 | 29.28 | 31.87 | 32.01 | 34.13 | 32.02 | 32.95 | 23.17 | 23.85 | 31.29 | 29.78 | 27.69 | 27.32 | 30.66 | 31.70 | 28.01 |
| 86 | 940 | ben FA | 25.33 | 34.29 | 27.70 | 31.38 | 31.19 | 29.22 | 28.87 | 29.60 | 21.47 | 21.28 | 33.44 | 31.36 | 24.78 | 23.85 | 28.28 | 31.78 | 24.92 |
| 87 | 941 | ben FA | 26.84 | 31.44 | 31.05 | 33.27 | 31.66 | 31.12 | 27.82 | 28.50 | 23.57 | 24.30 | 32.70 | 30.48 | 26.36 | 25.46 | 30.37 | 40.24 | 26.75 |
| 88 | 957 | ben FA | 24.37 | 31.53 | 27.42 | 28.56 | 28.99 | 31.34 | 30.23 | 30.98 | 24.86 | 25.62 | 29.60 | 28.10 | 22.90 | 21.85 | 28.77 | 31.38 | 25.18 |
| 90 | 923 | ben FA | 23.12 | 30.71 | 26.38 | 28.44 | 30.31 | 30.94 | 26.76 | 27.47 | 21.47 | 22.43 | 29.22 | 28.27 | 24.34 | 23.24 | 26.80 | 30.17 | 24.06 |
| 91 | 926 | ben FA | 22.89 | 28.49 | 21.65 | 28.23 | 30.01 | 30.63 | 27.07 | 28.13 | 21.86 | 22.86 | 30.71 | 29.07 | 23.50 | 22.23 | 28.06 | 27.40 | 24.56 |
| 92 | 955 | ben FA | 26.97 | 31.90 | 28.78 | 31.72 | 33.70 | 29.15 | 28.93 | 29.88 | 24.36 | 25.76 | 35.04 | 36.25 | 28.43 | 27.07 | 28.34 | 31.37 | 25.77 |
| 93 | 956 | ben FA | 27.07 | 31.06 | 29.52 | 30.12 | 32.02 | 30.01 | 29.24 | 29.69 | 23.30 | 24.09 | 31.88 | 30.98 | 27.17 | 25.99 | 28.79 | 32.24 | 25.83 |
| 94 | 959 | ben FA | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 | 46.83 |
| 95 | 960 | ben FA | 25.12 | 32.35 | 27.30 | 28.93 | 27.04 | 27.77 | 26.04 | 27.77 | 19.93 | 20.86 | 28.25 | 26.54 | 23.59 | 23.03 | 28.19 | 30.38 | 25.70 |
| 96 | 921 | ben FA | 22.10 | 29.70 | 25.75 | 28.05 | 31.95 | 31.03 | 24.57 | 25.75 | 18.33 | 19.18 | 26.27 | 24.77 | 24.04 | 22.51 | 26.50 | 34.76 | 22.35 |
| 97 | 961 | ben FA | 28.34 | 32.16 | 30.98 | 31.66 | 30.29 | 35.61 | 29.39 | 30.95 | 23.34 | 24.61 | 31.93 | 30.46 | 26.99 | 26.64 | 30.93 | 31.46 | 27.40 |
| 98 | 962 | ben FA | 23.81 | 28.50 | 28.66 | 28.64 | 26.46 | 27.68 | 25.81 | 26.91 | 19.33 | 20.25 | 29.59 | 29.19 | 23.12 | 22.37 | 25.86 | 29.61 | 23.12 |
| 106 | 924 | ben GPH | 21.48 | 25.85 | 21.39 | 27.18 | 25.94 | 28.07 | 27.04 | 27.93 | 24.88 | 26.58 | 31.30 | 29.97 | 26.64 | 25.82 | 41.06 | 50.00 | 34.50 |
| 35 | 871 | ben HCA | 23.42 | 31.13 | 25.74 | 31.80 | 29.20 | 26.91 | 30.89 | 32.50 | 23.62 | 24.19 | 30.68 | 31.30 | 24.86 | 24.70 | 24.75 | 25.78 | 24.93 |

| | | | | | | | | | | | | | | | | | | | |
|---|---|---|---|---|---|---|---|---|---|---|---|---|---|---|---|---|---|---|---|
| 2 | 805 | ben HPS | 26.84 | 33.76 | 28.86 | 31.08 | 31.63 | 33.82 | 27.13 | 29.29 | 22.12 | 22.53 | 28.81 | 28.65 | 25.93 | 25.17 | 27.57 | 29.63 | 26.20 |
| 1 | 804 | ben HT | 24.43 | 32.77 | 29.24 | 30.49 | 32.51 | 31.61 | 29.05 | 31.35 | 22.27 | 23.13 | 27.21 | 27.10 | 27.07 | 26.58 | 26.62 | 26.91 | 25.55 |
| 107 | 927 | ben HT | 23.24 | 30.81 | 25.97 | 30.09 | 28.33 | 28.01 | 28.49 | 28.83 | 18.52 | 19.83 | 28.34 | 27.17 | 23.11 | 22.07 | 25.75 | 28.52 | 24.46 |
| 71 | 917 | ben MA | 28.22 | 33.78 | 32.31 | 32.93 | 46.96 | 37.89 | 30.89 | 33.73 | 23.92 | 24.95 | 31.34 | 30.82 | 27.29 | 26.77 | 31.83 | 33.95 | 28.04 |
| 72 | 918 | ben MA | 26.91 | 32.97 | 28.17 | 33.26 | 41.60 | 38.14 | 31.18 | 33.17 | 24.49 | 25.37 | 33.96 | 33.85 | 27.51 | 26.76 | 31.16 | 33.08 | 28.81 |
| 3 | 806 | ben MG | 25.34 | 31.47 | 29.22 | 30.09 | 32.70 | 29.85 | 27.05 | 28.35 | 23.30 | 23.58 | 28.29 | 27.73 | 25.79 | 25.61 | 27.14 | 29.36 | 25.78 |
| 69 | 914 | ben OH | 24.00 | 30.88 | 26.46 | 31.98 | 31.46 | 28.02 | 33.41 | 36.09 | 23.70 | 23.41 | 29.62 | 29.06 | 29.92 | 30.02 | 24.91 | 26.56 | 26.38 |
| 68 | 913 | ben T | 25.53 | 32.08 | 26.07 | 31.03 | 31.68 | 33.27 | 30.28 | 32.61 | 23.95 | 24.88 | 31.69 | 31.43 | 26.95 | 26.52 | 28.53 | 31.91 | 27.72 |
| 4 | 807 | Benign HT | 24.58 | 30.66 | 29.49 | 30.94 | 32.66 | 31.29 | 29.94 | 31.69 | 24.08 | 24.85 | 29.48 | 30.38 | 27.11 | 26.54 | 28.68 | 27.69 | 25.83 |
| 8 | 823 | mal FC | 23.53 | 28.87 | 24.89 | 28.10 | 30.51 | 29.23 | 29.60 | 32.36 | 20.43 | 20.63 | 27.38 | 27.02 | 24.02 | 23.51 | 27.45 | 28.44 | 24.52 |
| 9 | 825 | mal FC | 25.84 | 31.66 | 31.99 | 32.23 | 29.58 | 28.78 | 30.39 | 33.25 | 24.22 | 24.59 | 29.69 | 30.60 | 25.40 | 24.94 | 26.58 | 28.29 | 25.85 |
| 10 | 826 | mal FC | 24.61 | 31.98 | 29.17 | 31.38 | 33.08 | 31.99 | 29.75 | 31.99 | 24.64 | 25.74 | 28.87 | 28.78 | 26.18 | 25.35 | 30.48 | 28.45 | 26.32 |
| 11 | 827 | mal FC | 24.99 | 30.42 | 28.44 | 29.30 | 29.64 | 28.48 | 29.90 | 31.21 | 22.95 | 23.06 | 29.02 | 29.01 | 27.49 | 27.27 | 27.98 | 26.03 | 24.14 |
| 12 | 828 | mal FC | 24.15 | 29.15 | 30.34 | 29.43 | 29.43 | 28.88 | 25.14 | 26.79 | 19.74 | 20.43 | 25.99 | 26.55 | 22.44 | 22.20 | 27.15 | 29.42 | 25.68 |
| 13 | 830 | mal FC | 23.53 | 27.36 | 30.58 | 28.91 | 25.23 | 27.30 | 26.34 | 28.11 | 24.97 | 25.54 | 28.12 | 27.60 | 23.23 | 23.27 | 26.81 | 29.30 | 24.68 |
| 22 | 840 | mal FC | 26.21 | 33.44 | 29.31 | 30.23 | 32.41 | 31.76 | 29.77 | 30.79 | 23.63 | 24.82 | 28.00 | 27.53 | 27.43 | 27.04 | 29.36 | 29.31 | 26.05 |
| 52 | 895 | mal FC | 24.03 | 31.04 | 26.30 | 31.86 | 34.24 | 29.65 | 29.80 | 32.00 | 21.45 | 22.96 | 31.13 | 30.80 | 22.47 | 21.66 | 27.59 | 32.15 | 25.87 |
| 53 | 896 | mal FC | 24.42 | 30.91 | 26.39 | 28.65 | 29.19 | 30.21 | 31.46 | 33.38 | 25.11 | 26.15 | 31.99 | 31.29 | 25.37 | 24.28 | 30.13 | 30.06 | 26.60 |
| 54 | 897 | mal FC | 24.87 | 29.74 | 26.02 | 29.08 | 26.85 | 27.22 | 26.89 | 28.65 | 20.75 | 22.10 | 27.59 | 27.46 | 25.72 | 24.59 | 27.14 | 26.16 | 24.69 |
| 55 | 898 | mal FC | 23.17 | 29.62 | 20.08 | 29.43 | 28.66 | 25.80 | 33.27 | 36.19 | 24.82 | 26.33 | 31.06 | 31.76 | 24.16 | 24.79 | 25.87 | 26.05 | 23.82 |
| 56 | 899 | mal FC | 26.16 | 28.89 | 21.24 | 29.06 | 28.98 | 29.92 | 33.33 | 36.57 | 29.63 | 30.55 | 31.44 | 33.12 | 29.39 | 30.45 | 28.85 | 29.38 | 27.21 |
| 57 | 900 | mal FC | 22.66 | 27.23 | 19.42 | 30.42 | 28.50 | 26.44 | 33.08 | 35.24 | 32.84 | 32.09 | 32.51 | 32.58 | 30.58 | 31.09 | 27.76 | 27.53 | 25.29 |
| 58 | 901 | mal FC | 24.63 | 31.68 | 26.95 | 28.12 | 24.03 | 21.53 | 34.01 | 37.22 | 50.00 | 45.31 | 30.02 | 30.53 | 26.03 | 26.82 | 29.16 | 30.77 | 24.73 |
| 59 | 902 | mal FC | 22.48 | 28.15 | 18.99 | 29.15 | 28.98 | 26.07 | 33.19 | 35.81 | 27.04 | 28.18 | 29.44 | 30.53 | 26.82 | 25.63 | 25.24 | 25.71 | 25.13 |
| 65 | 910 | mal FC | 24.98 | 29.80 | 28.65 | 30.68 | 32.34 | 26.61 | 28.00 | 29.55 | 23.29 | 24.76 | 31.28 | 30.76 | 25.86 | 25.58 | 26.04 | 28.49 | 25.00 |
| 82 | 936 | mal FC | 22.15 | 29.12 | 19.17 | 29.63 | 28.53 | 26.87 | 31.43 | 32.16 | 22.84 | 28.37 | 32.59 | 31.01 | 25.96 | 24.27 | 24.33 | 25.07 | 24.09 |
| 99 | 954 | mal FC | 21.76 | 31.46 | 27.47 | 29.92 | 27.89 | 24.71 | 30.58 | 30.93 | 19.16 | 20.78 | 27.09 | 25.74 | 23.98 | 23.30 | 23.99 | 25.51 | 22.72 |
| 100 | 968 | mal FC | 28.23 | 34.38 | 25.51 | 34.81 | 35.43 | 49.41 | 34.83 | 35.75 | 25.93 | 27.17 | 34.49 | 33.11 | 25.13 | 23.80 | 28.62 | 33.17 | 26.20 |
| 101 | 969 | mal FC | 25.80 | 31.58 | 27.15 | 30.36 | 36.99 | 41.12 | 28.36 | 28.72 | 22.94 | 23.59 | 32.18 | 31.16 | 26.90 | 25.60 | 27.72 | 45.43 | 25.30 |
| 102 | 970 | mal FC | 27.36 | 32.81 | 25.71 | 34.70 | 31.77 | 30.95 | 34.85 | 36.34 | 28.96 | 30.88 | 33.85 | 31.88 | 25.34 | 25.47 | 26.39 | 29.19 | 27.33 |
| 103 | 982 | mal FC | 26.16 | 32.15 | 26.80 | 31.90 | 27.73 | 29.11 | 26.34 | 27.26 | 20.54 | 22.21 | 30.50 | 29.66 | 24.51 | 23.58 | 28.32 | 35.41 | 25.41 |
| 104 | 983 | mal FC | 22.95 | 28.24 | 22.55 | 31.18 | 33.32 | 25.69 | 29.51 | 30.51 | 20.34 | 20.87 | 32.37 | 33.25 | 22.14 | 20.88 | 22.81 | 24.79 | 23.78 |
| 105 | 967 | mal FC | 23.84 | 30.25 | 25.35 | 29.45 | 31.34 | 28.53 | 29.90 | 31.58 | 24.77 | 25.88 | 31.53 | 31.48 | 25.77 | 25.25 | 25.39 | 25.78 | 23.18 |
| 14 | 831 | mal PC | 22.92 | 27.95 | 21.34 | 29.29 | 30.14 | 28.78 | 33.96 | 34.32 | 29.49 | 29.59 | 32.39 | 32.51 | 30.97 | 31.19 | 27.66 | 26.16 | 25.08 |
| 15 | 832 | mal PC | 25.88 | 29.76 | 27.56 | 29.72 | 29.51 | 30.92 | 30.43 | 31.16 | 24.72 | 25.60 | 30.07 | 29.22 | 29.54 | 29.10 | 29.21 | 29.25 | 26.29 |
| 16 | 834 | mal PC | 24.30 | 27.10 | 22.28 | 27.01 | 25.87 | 28.52 | 29.84 | 31.40 | 25.02 | 25.33 | 32.68 | 31.88 | 29.54 | 28.66 | 27.67 | 26.69 | 24.92 |
| 17 | 835 | mal PC | 23.68 | 28.39 | 21.08 | 29.07 | 28.75 | 29.02 | 32.71 | 35.57 | 28.42 | 29.43 | 32.36 | 32.27 | 31.11 | 30.86 | 28.05 | 26.23 | 25.36 |

| | | | | | | | | | | | | | | | | | | | |
|---|---|---|---|---|---|---|---|---|---|---|---|---|---|---|---|---|---|---|---|
| 18 | 836 | mal PC | 26.41 | 30.99 | 27.82 | 25.62 | 26.19 | 29.63 | 29.85 | 23.30 | 22.99 | 28.76 | 27.47 | 28.09 | 30.26 | 30.39 | 30.76 | 30.43 | 26.05 |
| 19 | 837 | mal PC | 24.06 | 27.16 | 27.43 | 25.43 | 25.99 | 29.14 | 28.94 | 22.10 | 21.85 | 28.96 | 27.94 | 28.55 | 27.05 | 26.96 | 22.57 | 28.10 | 23.03 |
| 20 | 838 | mal PC | 27.12 | 29.34 | 29.76 | 33.13 | 34.41 | 30.29 | 30.61 | 31.04 | 30.58 | 35.07 | 36.39 | 29.35 | 29.61 | 28.85 | 23.74 | 29.60 | 27.10 |
| 23 | 841 | mal PC | 24.87 | 24.71 | 25.08 | 28.83 | 29.02 | 28.91 | 29.05 | 32.06 | 35.72 | 35.53 | 50.00 | 25.74 | 25.18 | 29.59 | 25.50 | 29.87 | 24.61 |
| 36 | 874 | mal PC | 25.28 | 24.49 | 25.93 | 32.29 | 31.97 | 35.64 | 34.19 | 33.93 | 50.00 | 31.62 | 50.00 | 27.08 | 29.68 | 30.73 | 21.18 | 27.35 | 23.75 |
| 37 | 875 | mal PC | 24.90 | 26.82 | 27.26 | 27.87 | 29.08 | 30.30 | 31.28 | 25.32 | 24.54 | 32.27 | 29.84 | 27.88 | 27.86 | 28.75 | 20.76 | 28.79 | 22.97 |
| 38 | 876 | mal PC | 25.25 | 25.46 | 26.74 | 29.26 | 29.70 | 33.43 | 32.28 | 30.22 | 28.51 | 34.43 | 32.69 | 27.93 | 29.60 | 28.57 | 19.17 | 26.83 | 24.02 |
| 39 | 879 | mal PC | 26.14 | 28.76 | 27.90 | 31.46 | 32.62 | 36.11 | 33.28 | 33.05 | 30.62 | 37.76 | 35.70 | 29.61 | 31.07 | 27.68 | 20.03 | 28.19 | 24.09 |
| 40 | 880 | mal PC | 25.22 | 28.46 | 28.76 | 29.98 | 26.16 | 30.42 | 30.21 | 29.22 | 28.60 | 30.70 | 28.45 | 26.84 | 28.09 | 28.59 | 21.95 | 27.05 | 23.73 |
| 41 | 881 | mal PC | 26.21 | 28.45 | 28.45 | 29.51 | 29.93 | 30.82 | 30.53 | 28.12 | 27.44 | 33.33 | 30.89 | 29.02 | 28.39 | 29.43 | 21.56 | 27.96 | 25.76 |
| 42 | 882 | mal PC | 23.94 | 28.21 | 26.52 | 28.54 | 29.19 | 33.33 | 32.26 | 30.32 | 30.58 | 34.90 | 33.48 | 26.89 | 28.47 | 27.51 | 19.22 | 25.88 | 23.60 |
| 43 | 883 | mal PC | 26.98 | 31.77 | 29.74 | 32.65 | 33.41 | 33.40 | 33.16 | 34.10 | 39.38 | 34.87 | 32.77 | 27.92 | 30.28 | 30.46 | 21.95 | 28.69 | 27.24 |
| 44 | 884 | mal PC | 25.54 | 28.97 | 28.64 | 27.64 | 28.51 | 32.03 | 30.08 | 25.94 | 26.63 | 34.96 | 32.34 | 28.66 | 28.25 | 29.17 | 20.52 | 27.33 | 24.78 |
| 45 | 885 | mal PC | 25.53 | 28.41 | 27.90 | 28.90 | 29.74 | 34.75 | 32.27 | 30.49 | 30.78 | 35.97 | 33.18 | 27.46 | 31.08 | 29.04 | 18.88 | 27.18 | 24.40 |
| 46 | 886 | mal PC | 26.66 | 29.99 | 30.42 | 31.08 | 31.81 | 32.31 | 32.02 | 27.57 | 26.11 | 31.40 | 29.64 | 29.10 | 29.15 | 30.22 | 21.23 | 29.02 | 26.66 |
| 47 | 887 | mal PC | 26.52 | 29.03 | 28.77 | 30.19 | 30.28 | 36.74 | 33.81 | 30.00 | 28.38 | 37.77 | 35.66 | 30.80 | 30.46 | 30.61 | 20.15 | 28.99 | 26.37 |
| 48 | 890 | mal PC | 24.25 | 27.50 | 27.82 | 27.54 | 28.77 | 32.64 | 31.50 | 25.53 | 24.36 | 29.01 | 27.45 | 25.44 | 28.07 | 27.68 | 19.55 | 24.56 | 23.01 |
| 49 | 892 | mal PC | 25.49 | 28.93 | 28.89 | 30.66 | 30.31 | 33.10 | 32.91 | 26.38 | 25.29 | 31.68 | 29.38 | 27.88 | 27.38 | 28.60 | 21.08 | 27.10 | 25.50 |
| 50 | 893 | mal PC | 24.53 | 25.24 | 26.08 | 30.32 | 30.51 | 34.42 | 29.47 | 28.93 | 28.40 | 37.83 | 35.53 | 27.03 | 27.51 | 28.92 | 18.16 | 26.90 | 22.27 |
| 51 | 894 | mal PC | 26.58 | 27.59 | 28.57 | 31.97 | 32.53 | 36.95 | 35.91 | 27.92 | 26.40 | 37.83 | 34.74 | 29.00 | 30.81 | 29.72 | 21.00 | 28.95 | 25.06 |
| 60 | 903 | mal PC | 25.84 | 29.55 | 29.10 | 27.01 | 27.66 | 30.92 | 31.52 | 25.56 | 24.33 | 30.67 | 28.93 | 27.98 | 26.53 | 29.80 | 22.79 | 27.79 | 24.67 |
| 61 | 904 | mal PC | 27.30 | 29.39 | 31.19 | 28.28 | 28.43 | 29.40 | 29.77 | 25.87 | 25.37 | 32.92 | 30.64 | 31.91 | 29.39 | 29.32 | 24.59 | 30.50 | 25.60 |
| 62 | 905 | mal PC | 26.43 | 27.66 | 29.11 | 31.44 | 32.32 | 33.96 | 32.89 | 32.35 | 50.00 | 37.87 | 37.22 | 30.18 | 29.27 | 29.38 | 20.25 | 27.83 | 23.52 |
| 63 | 906 | mal PC | 25.78 | 25.31 | 26.38 | 33.85 | 33.75 | 36.36 | 36.00 | 39.67 | 50.00 | 38.43 | 36.14 | 27.28 | 30.53 | 30.82 | 20.50 | 28.80 | 24.75 |
| 67 | 912 | mal PC | 28.95 | 28.35 | 32.22 | 29.76 | 31.15 | 29.40 | 29.96 | 28.77 | 27.54 | 33.19 | 31.43 | 34.61 | 31.91 | 30.31 | 28.67 | 32.23 | 26.06 |
| 74 | 928 | mal PC | 25.24 | 29.72 | 26.20 | 22.28 | 22.88 | 27.50 | 29.21 | 22.43 | 20.69 | 29.35 | 28.56 | 30.42 | 31.21 | 29.75 | 26.35 | 31.29 | 24.74 |
| 75 | 929 | mal PC | 26.20 | 28.31 | 31.53 | 35.60 | 33.94 | 28.05 | 29.15 | 30.77 | 30.24 | 33.95 | 33.19 | 29.63 | 29.23 | 28.84 | 21.44 | 28.64 | 25.88 |
| 76 | 930 | mal PC | 25.23 | 28.36 | 29.63 | 40.98 | 32.01 | 28.48 | 29.55 | 28.42 | 27.51 | 28.85 | 27.63 | 26.54 | 27.74 | 28.29 | 22.06 | 27.11 | 23.24 |
| 77 | 931 | mal PC | 25.73 | 31.99 | 28.83 | 23.53 | 24.27 | 37.03 | 36.74 | 30.19 | 29.61 | 25.72 | 25.10 | 27.15 | 25.85 | 30.21 | 28.87 | 26.79 | 24.56 |
| 78 | 932 | mal PC | 25.37 | 28.07 | 29.96 | 50.00 | 32.08 | 35.13 | 35.90 | 27.96 | 26.75 | 35.50 | 33.42 | 29.15 | 29.50 | 26.22 | 21.01 | 26.40 | 23.10 |
| 79 | 933 | mal PC | 25.92 | 29.28 | 30.83 | 27.30 | 27.61 | 30.49 | 31.53 | 24.64 | 23.90 | 31.16 | 30.71 | 30.03 | 29.00 | 31.17 | 24.22 | 30.24 | 26.28 |
| 80 | 934 | mal PC | 25.52 | 28.02 | 30.27 | 32.87 | 31.25 | 31.58 | 32.43 | 28.02 | 27.25 | 35.30 | 33.71 | 29.57 | 30.07 | 28.17 | 25.81 | 30.08 | 24.62 |
| 81 | 935 | mal PC | 24.40 | 24.60 | 26.39 | 50.00 | 34.23 | 33.71 | 35.17 | 29.46 | 27.75 | 35.02 | 33.18 | 28.45 | 28.93 | 30.39 | 18.78 | 26.41 | 22.89 |
| 21 | 839 | mal PC FV | 26.05 | 28.72 | 29.67 | 26.14 | 26.31 | 29.10 | 29.37 | 23.13 | 22.79 | 30.78 | 29.58 | 31.95 | 32.12 | 30.32 | 28.20 | 31.46 | 24.92 |
| 66 | 911 | mal PC FV | 29.47 | 31.23 | 33.24 | 31.21 | 31.83 | 29.38 | 29.33 | 30.24 | 29.75 | 34.06 | 32.08 | 30.23 | 30.06 | 31.99 | 27.26 | 31.06 | 30.42 |
| 89 | 981 | mal PC FV | 24.99 | 30.03 | 26.46 | 26.57 | 26.74 | 29.47 | 30.24 | 22.90 | 21.80 | 27.23 | 26.79 | 26.85 | 29.04 | 29.14 | 27.55 | 28.29 | 25.69 |
| 5 | 809 | normal | 26.19 | 28.56 | 29.19 | 26.36 | 27.08 | 29.03 | 29.27 | 23.74 | 23.11 | 31.61 | 30.19 | 32.53 | 32.57 | 30.44 | 30.82 | 32.31 | 26.05 |
| 6 | 817 | normal | 26.14 | 30.18 | 29.39 | 26.15 | 26.61 | 28.29 | 28.69 | 23.44 | 23.24 | 30.34 | 29.04 | 33.50 | 32.11 | 31.12 | 31.69 | 32.62 | 26.92 |

| 25 | 844 | normal | 26.61 | 33.39 | 32.33 | 32.40 | 32.36 | 34.60 | 31.34 | 33.27 | 24.60 | 24.75 | 30.80 | 29.99 | 28.03 | 27.35 | 30.12 | 31.79 | 28.13 |
|---|---|---|---|---|---|---|---|---|---|---|---|---|---|---|---|---|---|---|---|
| 26 | 845 | normal | 25.08 | 31.37 | 31.11 | 31.87 | 33.50 | 30.13 | 31.27 | 32.54 | 21.65 | 22.02 | 29.87 | 29.42 | 28.26 | 27.38 | 25.86 | 27.57 | 25.65 |
| 27 | 851 | normal | 25.92 | 31.32 | 30.18 | 29.49 | 30.03 | 31.08 | 29.30 | 30.46 | 24.11 | 24.94 | 28.78 | 28.12 | 27.48 | 27.16 | 29.29 | 29.50 | 26.05 |
|  |  |  | 28.00 | 30.00 | 26.00 | 30.00 | 31.00 | 30.00 | 31.00 | 31.50 | 23.50 | 23.25 | 30.00 | 30.00 | 26.00 | 27.00 | 28.00 | 29.00 | 25.50 |

Table 23b

| | | Thyroid UR Markers | | | | | | | | | | |
|---|---|---|---|---|---|---|---|---|---|---|---|---|
| | | CDH3 | FN1 | GAB | SGENE | DDIT3 | C10f24 | ITM1 | TEST | KCN1257 | TPO2166 | TPO2592 |
| % Sensitivity | | 76.56 | 71.88 | 39.06 | 23.44 | 23.44 | 78.13 | 43.75 | 57.81 | 48.44 | 71.88 | 51.56 |
| % Specificity | | 83.33 | 80.56 | 80.56 | 94.44 | 86.11 | 77.78 | 63.89 | 77.78 | 86.11 | 88.89 | 97.22 |
| | | | | | | | | | | | | |
| | | CDH3/KCN | CDH3/TPO-1 | CDH3/TPO-2 | SGENE/GAB | | | | FN1/KCN | FN1/TPO-1 | FN1/TPO-2 | TEST/KCN |
| | Sensitivity% | 85.94 | 89.06 | 84.38 | 96.88 | | | | 76.56 | 84.38 | 78.13 | 81.25 |
| | Specificity% | 69.44 | 75.00 | 83.33 | 5.56 | | | | 66.67 | 72.22 | 80.56 | 63.89 |
| | | CDH3/FN1/TEST/TPO2166 | | | | | FN1/SGENE/TPO2166 | | | | | |
| | Sensitivity% | 95.31 | | | | | 89.06 | | | | | |
| | Specificity% | 61.11 | | | | | 72.22 | | | | | |

| Thyroid DR Markers | | | |
|---|---|---|---|
| | | DIO1;480 | DIO1;687 |
| | | 54.69 | 53.13 |
| % Sensitivity | | 86.11 | 97.22 |
| % Specificity | | | |

Positive
Outliers
Markers with Best Performance

| | | BACTIN | CDH3 | FN1 | GAB | SGENE | DDIT3 | C10f24 | ITM1 | TEST | KCN1257 | TPO2166 | TPO2592 | FABP4;110 | FABP4-2 | DIO1;480 | DIO1;687 |
|---|---|---|---|---|---|---|---|---|---|---|---|---|---|---|---|---|---|
| 915TT | benign DG | 26.69 | 34.23 | 30.09 | 31.94 | 33.60 | 29.54 | 31.01 | 26.46 | 32.00 | 30.70 | 20.69 | 21.83 | 29.87 | 28.76 | 24.78 | 24.09 |
| 924TT | benign GPH | 21.48 | 25.85 | 22.00 | 27.18 | 27.96 | 41.06 | 50.00 | 34.50 | 25.94 | 27.04 | 24.88 | 26.58 | 31.30 | 29.97 | 26.64 | 25.82 |
| 871TT | benign HCA | 23.42 | 31.13 | 25.17 | 31.80 | 26.99 | 24.75 | 25.78 | 24.93 | 29.20 | 30.89 | 23.62 | 24.19 | 30.68 | 31.30 | 24.86 | 24.70 |
| 805TT | benign HPS | 26.84 | 33.76 | 28.61 | 31.08 | 33.72 | 27.57 | 29.63 | 26.20 | 31.63 | 27.13 | 22.12 | 22.53 | 28.81 | 28.65 | 25.93 | 25.17 |
| 804TT | benign HT | 24.43 | 32.77 | 28.98 | 30.49 | 31.62 | 26.62 | 26.91 | 25.55 | 32.51 | 29.05 | 22.27 | 23.13 | 27.21 | 27.10 | 27.07 | 26.58 |
| 927TT | benign HT | 23.24 | 30.81 | 25.87 | 30.09 | 28.23 | 25.75 | 28.52 | 24.46 | 28.33 | 28.49 | 18.52 | 19.83 | 28.34 | 27.17 | 23.11 | 22.07 |

| | | | | | | | | | | | | | | | | |
|---|---|---|---|---|---|---|---|---|---|---|---|---|---|---|---|---|
| 917TT | benign MA | 28.22 | 33.78 | 33.13 | 32.93 | 37.79 | 31.83 | 33.95 | 28.04 | 46.96 | 30.89 | 23.92 | 24.95 | 31.34 | 30.82 | 27.29 | 26.77 |
| 918TT | benign MA | 26.91 | 32.97 | 28.74 | 33.26 | 38.32 | 31.16 | 33.08 | 28.81 | 41.60 | 31.18 | 24.49 | 25.37 | 33.96 | 33.85 | 27.51 | 26.76 |
| 806TT | benign MG | 25.34 | 31.47 | 28.77 | 30.09 | 29.84 | 27.14 | 29.36 | 25.78 | 32.70 | 27.05 | 23.30 | 23.58 | 28.29 | 27.73 | 25.79 | 25.61 |
| 914TT | benign OH | 24.00 | 30.88 | 27.01 | 31.98 | 28.33 | 24.91 | 26.56 | 26.38 | 31.46 | 33.41 | 23.70 | 23.41 | 29.62 | 29.06 | 29.92 | 30.02 |
| 913TT | benign T | 25.53 | 32.08 | 26.17 | 31.03 | 34.07 | 28.53 | 31.91 | 27.72 | 31.68 | 30.28 | 23.95 | 24.88 | 31.69 | 31.43 | 26.95 | 26.52 |
| 807TT | benignHT | 24.58 | 30.66 | 28.98 | 30.94 | 31.35 | 28.68 | 27.69 | 25.83 | 32.66 | 29.94 | 24.08 | 24.85 | 29.48 | 30.38 | 27.11 | 26.54 |
| 842TT | benign FA | 25.92 | 31.74 | 29.53 | 30.96 | 32.71 | 29.76 | 33.81 | 27.09 | 32.98 | 27.45 | 23.88 | 24.44 | 32.08 | 34.09 | 27.82 | 27.42 |
| 862TT | benign FA | 26.86 | 33.35 | 29.92 | 30.92 | 50.00 | 28.44 | 38.86 | 27.25 | 41.29 | 33.60 | 22.50 | 22.54 | 32.02 | 32.16 | 25.63 | 25.24 |
| 863TT | benign FA | 24.56 | 30.03 | 29.48 | 29.85 | 27.28 | 27.71 | 30.25 | 24.57 | 28.49 | 25.73 | 22.03 | 22.89 | 29.05 | 28.82 | 24.74 | 24.42 |
| 864TT | benign FA | 26.15 | 32.18 | 30.08 | 31.97 | 30.48 | 28.23 | 31.93 | 25.59 | 34.52 | 26.65 | 21.76 | 22.65 | 30.06 | 30.15 | 25.67 | 25.30 |
| 865TT | benign FA | 26.21 | 32.94 | 26.92 | 31.29 | 30.93 | 30.19 | 47.61 | 26.83 | 32.07 | 29.70 | 21.87 | 22.37 | 31.25 | 31.31 | 24.25 | 23.83 |
| 866TT | benign FA | 27.00 | 33.10 | 28.77 | 32.26 | 32.61 | 29.26 | 31.60 | 26.44 | 34.44 | 29.59 | 23.32 | 23.43 | 33.18 | 33.81 | 25.94 | 25.57 |
| 867TT | benign FA | 24.89 | 31.72 | 30.37 | 31.88 | 33.81 | 29.33 | 31.53 | 26.71 | 32.29 | 29.50 | 21.88 | 21.95 | 27.71 | 27.69 | 24.90 | 24.73 |
| 869TT | benign FA | 24.99 | 29.22 | 28.58 | 29.99 | 28.11 | 27.39 | 29.46 | 25.00 | 27.17 | 27.32 | 21.24 | 22.14 | 30.69 | 30.08 | 25.88 | 25.39 |
| 907TT | benign FA | 25.09 | 31.42 | 27.00 | 29.49 | 35.86 | 29.63 | 30.46 | 25.79 | 33.83 | 29.98 | 20.92 | 21.81 | 31.21 | 50.00 | 22.49 | 22.55 |
| 920TT | benign FA | 25.37 | 34.29 | 28.37 | 30.93 | 48.63 | 29.53 | 28.90 | 25.04 | 33.48 | 31.87 | 21.43 | 21.83 | 30.14 | 28.91 | 22.40 | 23.30 |
| 937TT | benign FA | 23.32 | 33.55 | 28.00 | 32.56 | 36.65 | 27.15 | 32.90 | 24.30 | 34.45 | 25.48 | 19.17 | 20.06 | 27.50 | 25.18 | 24.69 | 23.50 |
| 938TT | benign FA | 23.39 | 31.45 | 26.20 | 31.58 | 26.34 | 27.77 | 29.88 | 25.01 | 29.82 | 27.98 | 20.84 | 21.34 | 29.53 | 27.24 | 22.26 | 22.03 |
| 939TT | benign FA | 25.58 | 34.57 | 29.13 | 31.87 | 34.24 | 30.66 | 31.70 | 28.01 | 32.01 | 32.02 | 23.17 | 23.85 | 31.29 | 29.78 | 27.69 | 27.32 |
| 940TT | benign FA | 25.33 | 34.29 | 27.51 | 31.38 | 29.38 | 28.28 | 31.78 | 24.92 | 31.19 | 28.87 | 21.47 | 21.28 | 33.44 | 31.36 | 24.78 | 23.85 |
| 941TT | benign FA | 26.84 | 31.44 | 30.89 | 33.27 | 31.13 | 30.37 | 40.24 | 26.75 | 31.66 | 27.82 | 23.57 | 24.30 | 32.70 | 30.48 | 26.36 | 25.46 |
| 957TT | benign FA | 24.37 | 31.53 | 27.49 | 28.56 | 31.29 | 28.77 | 31.38 | 25.18 | 28.99 | 30.23 | 24.86 | 25.62 | 29.60 | 28.10 | 22.90 | 21.85 |
| 923TT | benign FA | 23.12 | 30.71 | 26.83 | 28.44 | 31.48 | 26.80 | 30.17 | 24.06 | 30.31 | 26.76 | 21.47 | 22.43 | 29.22 | 28.27 | 24.34 | 23.24 |
| 926TT | benign FA | 22.89 | 28.49 | 22.10 | 28.23 | 30.84 | 28.06 | 27.40 | 24.56 | 30.01 | 27.07 | 21.86 | 22.86 | 30.71 | 29.07 | 23.50 | 22.23 |
| 955TT | benign FA | 26.97 | 31.90 | 29.65 | 31.72 | 29.22 | 28.34 | 31.37 | 25.77 | 33.70 | 28.93 | 24.36 | 25.76 | 35.04 | 36.25 | 28.43 | 27.07 |
| 956TT | benign FA | 27.07 | 31.06 | 29.58 | 30.12 | 30.07 | 28.79 | 32.24 | 25.83 | 32.02 | 29.24 | 23.30 | 24.09 | 31.88 | 30.98 | 27.17 | 25.99 |
| 960TT | benign FA | 25.12 | 32.35 | 27.33 | 28.93 | 27.89 | 28.19 | 30.38 | 25.70 | 27.04 | 26.04 | 19.93 | 20.86 | 28.25 | 26.54 | 23.59 | 23.03 |
| 921TT | benign FA | 22.10 | 29.70 | 25.87 | 28.05 | 30.89 | 26.50 | 34.76 | 22.35 | 31.95 | 24.57 | 18.33 | 19.18 | 26.27 | 24.77 | 24.04 | 22.51 |
| 961TT | benign FA | 28.34 | 32.16 | 31.04 | 31.66 | 35.11 | 30.93 | 31.46 | 27.40 | 30.29 | 29.39 | 23.34 | 24.61 | 31.93 | 30.46 | 26.99 | 26.64 |
| 962TT | benign FA | 23.81 | 28.50 | 28.78 | 28.64 | 27.51 | 25.86 | 29.61 | 23.12 | 26.46 | 25.81 | 19.33 | 20.25 | 29.59 | 29.19 | 23.12 | 22.37 |
| 823TT | malignant FC | 23.53 | 28.87 | 24.33 | 28.10 | 29.23 | 27.45 | 28.44 | 24.52 | 30.51 | 29.60 | 20.43 | 20.63 | 27.38 | 27.02 | 24.02 | 23.51 |
| 825TT | malignant FC | 25.84 | 31.66 | 31.36 | 32.23 | 28.74 | 26.58 | 28.29 | 25.85 | 29.58 | 30.39 | 24.22 | 24.59 | 29.69 | 30.60 | 25.40 | 24.94 |
| 826TT | malignant FC | 24.61 | 31.98 | 28.66 | 31.38 | 32.18 | 30.48 | 28.45 | 26.32 | 33.08 | 29.75 | 24.64 | 25.74 | 28.87 | 28.78 | 26.18 | 25.35 |
| 827TT | malignant FC | 24.99 | 30.42 | 28.10 | 29.30 | 28.53 | 27.98 | 26.03 | 24.14 | 29.64 | 29.90 | 22.95 | 23.06 | 29.02 | 29.01 | 27.49 | 27.27 |
| 828TT | malignant FC | 24.15 | 29.15 | 29.96 | 29.43 | 28.86 | 27.15 | 29.42 | 25.68 | 29.43 | 25.14 | 19.74 | 20.43 | 25.99 | 26.55 | 22.44 | 22.20 |
| 830TT | malignant FC | 23.53 | 27.36 | 30.09 | 28.91 | 27.51 | 26.81 | 29.30 | 24.68 | 25.23 | 26.34 | 24.97 | 25.54 | 28.12 | 27.60 | 23.23 | 23.27 |
| 840TT | malignant FC | 26.21 | 33.44 | 28.95 | 30.23 | 31.79 | 29.36 | 29.31 | 26.05 | 32.41 | 29.77 | 23.63 | 24.82 | 28.00 | 27.53 | 27.43 | 27.04 |

| Sample | Type | | | | | | | | | | | | | | | | |
|---|---|---|---|---|---|---|---|---|---|---|---|---|---|---|---|---|---|
| 895TT | malignant FC | 21.66 | 22.47 | 30.80 | 31.13 | 22.96 | 21.45 | 29.80 | 34.24 | 25.87 | 32.15 | 27.59 | 29.67 | 31.86 | 26.39 | 31.04 | 24.03 |
| 896TT | malignant FC | 24.28 | 25.37 | 31.29 | 31.99 | 26.15 | 25.11 | 31.46 | 29.19 | 26.60 | 30.06 | 30.13 | 30.77 | 28.65 | 26.55 | 30.91 | 24.42 |
| 897TT | malignant FC | 24.59 | 25.72 | 27.46 | 27.59 | 22.10 | 20.86 | | 26.85 | 24.69 | 26.16 | 27.14 | 27.13 | 29.08 | 26.27 | | 24.87 |
| 898TT | malignant FC | 24.79 | 24.16 | 31.76 | 31.06 | 26.33 | 24.82 | 33.27 | 28.66 | 23.82 | 26.05 | 25.87 | 25.74 | 29.43 | 20.35 | 29.62 | 23.17 |
| 899TT | malignant FC | 30.45 | 29.39 | 33.12 | 31.44 | 30.55 | 29.63 | 33.33 | 28.98 | 27.21 | 29.38 | 28.85 | 29.92 | 29.06 | 21.34 | 28.89 | 26.16 |
| 900TT | malignant FC | 31.09 | 30.58 | 32.58 | 32.51 | 32.09 | 32.84 | 33.08 | 28.50 | 25.29 | 27.53 | 27.76 | 26.40 | 30.42 | 19.58 | 27.23 | 22.66 |
| 901TT | malignant FC | 26.82 | 26.03 | 30.53 | 30.02 | 45.31 | 50.00 | 34.01 | 24.03 | 24.73 | 30.77 | 29.16 | 21.76 | 28.12 | 27.38 | 31.68 | 24.63 |
| 902TT | malignant FC | 25.63 | 26.82 | 30.53 | 29.44 | 28.18 | 27.04 | 33.19 | 28.98 | 25.13 | 25.71 | 25.24 | 26.11 | 29.15 | 19.07 | 28.15 | 22.48 |
| 910TT | malignant FC | 25.58 | 25.86 | 30.76 | 31.28 | 24.76 | 23.29 | 28.00 | 32.34 | 25.00 | 28.49 | 26.04 | 26.34 | 30.68 | 28.71 | 29.80 | 24.98 |
| 936TT | malignant FC | 24.27 | 25.96 | 31.01 | 32.59 | 28.37 | 22.84 | 31.43 | 28.53 | 24.09 | 25.07 | 24.33 | 26.83 | 29.63 | 19.40 | 29.12 | 22.15 |
| 954TT | malignant FC | 23.30 | 23.98 | 25.74 | 27.09 | 20.78 | 19.16 | 30.58 | 27.89 | 22.72 | 25.51 | 23.99 | 24.64 | 29.92 | 28.01 | 31.46 | 21.76 |
| 968TT | malignant FC | 23.80 | 25.13 | 33.11 | 34.49 | 27.17 | 25.93 | 34.83 | 35.43 | 26.20 | 33.17 | 28.62 | 46.83 | 34.81 | 26.32 | 34.38 | 28.23 |
| 969TT | malignant FC | 25.60 | 26.90 | 31.16 | 32.18 | 23.59 | 22.94 | 28.36 | 36.99 | 25.30 | 45.43 | 27.72 | 42.77 | 30.36 | 27.84 | 31.58 | 25.80 |
| 970TT | malignant FC | 25.47 | 25.34 | 31.88 | 33.85 | 30.88 | 28.96 | 34.85 | 31.77 | 27.33 | 29.19 | 26.39 | 30.90 | 34.70 | 26.30 | 32.81 | 27.36 |
| 982TT | malignant FC | 23.58 | 24.51 | 29.66 | 30.50 | 22.21 | 20.54 | 26.34 | 27.73 | 25.41 | 35.41 | 28.32 | 29.32 | 31.90 | 27.30 | 32.15 | 26.16 |
| 983TT | malignant FC | 20.88 | 22.14 | 33.25 | 32.37 | 20.87 | 20.34 | 29.51 | 33.32 | 23.78 | 24.79 | 22.81 | 25.74 | 31.18 | 23.09 | 28.24 | 22.95 |
| 967TT | malignant FC | 25.25 | 25.77 | 31.48 | 31.53 | 25.88 | 24.77 | 29.90 | 31.34 | 23.18 | 25.78 | 25.39 | 28.46 | 29.45 | 25.99 | 30.25 | 23.84 |
| 831TT | malignant PC | 31.19 | 30.97 | 32.51 | 32.39 | 29.59 | 29.49 | 33.96 | 30.14 | 25.08 | 26.16 | 27.66 | 28.98 | 29.29 | 20.74 | 27.95 | 22.92 |
| 832TT | malignant PC | 29.10 | 29.54 | 29.22 | 30.07 | 25.60 | 24.72 | 30.43 | 29.51 | 26.29 | 29.25 | 29.21 | 30.99 | 29.72 | 26.78 | 29.76 | 25.88 |
| 834TT | malignant PC | 28.66 | 29.54 | 31.88 | 32.68 | 25.33 | 25.02 | 29.84 | 25.87 | 24.92 | 26.69 | 27.67 | 28.67 | 27.01 | 21.77 | 27.10 | 24.30 |
| 835TT | malignant PC | 30.86 | 31.11 | 32.27 | 32.36 | 29.43 | 28.42 | 32.71 | 28.75 | 25.36 | 26.23 | 28.05 | 29.06 | 29.07 | 20.52 | 28.39 | 23.68 |
| 836TT | malignant PC | 25.62 | 26.19 | 29.63 | 29.85 | 23.30 | 22.99 | 27.47 | 30.26 | 26.41 | 30.99 | 27.82 | 28.40 | 30.39 | 30.35 | 30.43 | 26.05 |
| 837TT | malignant PC | 25.43 | 25.99 | 29.14 | 28.94 | 22.10 | 21.85 | 27.94 | 27.05 | 24.06 | 27.16 | 27.43 | 28.84 | 26.96 | 21.98 | 28.10 | 23.03 |
| 838TT | malignant PC | 33.13 | 34.41 | 30.29 | 30.61 | 31.04 | 30.58 | 36.39 | 29.61 | 27.12 | 29.34 | 29.76 | 29.67 | 28.85 | 23.25 | 29.60 | 27.10 |
| 841TT | malignant PC | 28.83 | 29.02 | 28.91 | 29.05 | 32.06 | 35.72 | 50.00 | 25.18 | 24.87 | 24.71 | 25.08 | 25.80 | 29.59 | 25.17 | 29.87 | 24.61 |
| 874TT | malignant PC | 32.29 | 31.97 | 35.64 | 34.19 | 33.93 | 50.00 | 50.00 | 29.68 | 25.28 | 24.49 | 25.93 | 27.01 | 30.73 | 20.38 | 27.35 | 23.75 |
| 875TT | malignant PC | 27.87 | 29.08 | 30.30 | 31.28 | 25.32 | 24.54 | 29.84 | 27.86 | 24.90 | 26.82 | 27.26 | 27.93 | 28.75 | 21.28 | 28.79 | 22.97 |
| 876TT | malignant PC | 29.26 | 29.70 | 33.43 | 32.28 | 30.22 | 28.51 | 32.69 | 29.60 | 25.25 | 25.46 | 26.74 | 27.87 | 28.57 | 19.37 | 26.83 | 24.02 |
| 879TT | malignant PC | 31.46 | 32.62 | 36.11 | 33.28 | 33.05 | 30.62 | 35.70 | 31.07 | 26.14 | 28.76 | 27.90 | 29.49 | 27.68 | 20.13 | 28.19 | 24.09 |
| 880TT | malignant PC | 29.98 | 26.16 | 30.42 | 30.21 | 29.22 | 28.60 | 28.45 | 28.09 | 25.22 | 28.46 | 28.76 | 26.84 | 28.59 | 21.98 | 27.05 | 23.73 |
| 881TT | malignant PC | 29.51 | 29.93 | 30.82 | 30.53 | 28.12 | 27.44 | 30.89 | 28.39 | 26.21 | 28.45 | 28.45 | 29.01 | 29.43 | 21.57 | 27.96 | 25.76 |
| 882TT | malignant PC | 28.54 | 29.19 | 33.33 | 32.26 | 30.32 | 30.58 | 33.48 | 28.47 | 23.94 | 28.21 | 26.52 | 26.85 | 27.51 | 19.25 | 25.88 | 23.60 |
| 883TT | malignant PC | 32.65 | 33.41 | 33.40 | 33.16 | 34.10 | 39.38 | 32.77 | 30.28 | 26.98 | 31.77 | 29.74 | 28.08 | 30.46 | 21.80 | 28.69 | 27.24 |
| 884TT | malignant PC | 27.64 | 28.51 | 32.03 | 30.08 | 25.94 | 26.63 | 32.34 | 28.25 | 25.54 | 28.97 | 28.64 | 28.48 | 29.17 | 20.55 | 27.33 | 24.78 |
| 885TT | malignant PC | 28.90 | 29.74 | 34.75 | 32.27 | 30.49 | 30.78 | 33.18 | 31.08 | 25.53 | 28.41 | 27.90 | 27.45 | 29.04 | 18.86 | 27.18 | 24.40 |
| 886TT | malignant PC | 31.08 | 31.81 | 32.31 | 32.02 | 27.57 | 26.11 | 29.64 | 29.15 | 26.66 | 29.99 | 30.42 | 28.93 | 30.22 | 21.62 | 29.02 | 26.66 |
| 887TT | malignant PC | 30.19 | 30.28 | 36.74 | 33.81 | 30.00 | 28.38 | 35.66 | 30.46 | 26.52 | 29.03 | 28.77 | 30.98 | 30.61 | 20.39 | 28.99 | 26.37 |

| ID | Type | | | | | | | | | | | | | | | | |
|---|---|---|---|---|---|---|---|---|---|---|---|---|---|---|---|---|---|
| 890TTT | malignant PC | 23.01 | 24.56 | 19.82 | 27.68 | 25.47 | 27.82 | 27.50 | 24.25 | 28.07 | 27.45 | 24.36 | 25.53 | 31.50 | 32.64 | 28.77 | 27.54 |
| 892TTT | malignant PC | 25.50 | 27.10 | 21.43 | 28.60 | 27.93 | 28.89 | 28.93 | 25.49 | 27.38 | 29.38 | 25.29 | 26.38 | 32.91 | 33.10 | 30.31 | 30.66 |
| 893TTT | malignant PC | 22.27 | 26.90 | 18.05 | 28.92 | 26.79 | 26.08 | 25.24 | 24.53 | 27.51 | 35.53 | 28.40 | 28.93 | 29.47 | 34.42 | 30.51 | 30.32 |
| 894TTT | malignant PC | 25.06 | 28.95 | 21.59 | 29.72 | 29.12 | 28.57 | 27.59 | 26.58 | 30.81 | 34.74 | 26.40 | 27.92 | 35.91 | 36.95 | 32.53 | 31.97 |
| 903TTT | malignant PC | 24.67 | 27.79 | 23.24 | 29.80 | 28.03 | 29.10 | 29.55 | 25.84 | 26.53 | 28.93 | 24.33 | 25.56 | 31.52 | 30.92 | 27.66 | 27.01 |
| 904TTT | malignant PC | 25.60 | 30.50 | 24.86 | 29.32 | 32.31 | 31.19 | 29.39 | 27.30 | 29.39 | 30.64 | 25.37 | 25.87 | 29.77 | 29.40 | 28.43 | 28.28 |
| 905TTT | malignant PC | 23.52 | 27.83 | 20.64 | 29.38 | 30.32 | 29.11 | 27.66 | 26.43 | 29.27 | 37.22 | 50.00 | 32.35 | 32.89 | 33.96 | 32.32 | 31.44 |
| 906TTT | malignant PC | 24.75 | 28.80 | 20.46 | 30.82 | 27.25 | 26.38 | 25.31 | 25.78 | 30.53 | 36.14 | 50.00 | 39.67 | 36.00 | 36.36 | 33.75 | 33.85 |
| 912TTT | malignant PC | 26.06 | 32.23 | 28.74 | 30.31 | 35.11 | 32.22 | 28.35 | 28.95 | 31.91 | 31.43 | 27.54 | 28.77 | 29.96 | 29.40 | 31.15 | 29.76 |
| 928TTT | malignant PC | 24.74 | 31.29 | 26.57 | 29.75 | 30.63 | 26.20 | 29.72 | 25.24 | 31.21 | 28.56 | 20.69 | 22.43 | 29.21 | 27.50 | 22.88 | 22.28 |
| 929TTT | malignant PC | 25.88 | 28.64 | 21.77 | 28.84 | 29.79 | 31.53 | 28.31 | 26.20 | 29.23 | 33.19 | 30.24 | 30.77 | 29.15 | 28.05 | 33.94 | 35.60 |
| 930TTT | malignant PC | 23.24 | 27.11 | 22.84 | 28.29 | 26.72 | 29.63 | 28.36 | 25.23 | 27.74 | 27.63 | 27.51 | 28.42 | 29.55 | 28.48 | 32.01 | 40.98 |
| 931TTT | malignant PC | 24.56 | 26.79 | 29.48 | 30.21 | 27.22 | 28.83 | 31.99 | 25.73 | 25.85 | 25.10 | 29.61 | 30.19 | 36.74 | 37.03 | 24.27 | 23.53 |
| 932TTT | malignant PC | 23.10 | 26.40 | 21.26 | 26.22 | 29.13 | 29.96 | 28.07 | 25.37 | 29.50 | 33.42 | 26.75 | 27.96 | 35.90 | 35.13 | 32.08 | 50.00 |
| 933TTT | malignant PC | 26.28 | 30.24 | 24.46 | 31.17 | 30.25 | 30.83 | 29.28 | 25.92 | 29.00 | 30.71 | 23.90 | 24.64 | 31.53 | 30.49 | 27.61 | 27.30 |
| 934TTT | malignant PC | 24.62 | 30.08 | 26.38 | 28.17 | 29.63 | 30.27 | 28.02 | 25.52 | 30.07 | 33.71 | 27.25 | 28.02 | 32.43 | 31.58 | 31.25 | 32.87 |
| 935TTT | malignant PC | 22.89 | 26.41 | 19.38 | 30.39 | 28.59 | 26.39 | 24.60 | 24.40 | 28.93 | 33.18 | 27.75 | 29.46 | 35.17 | 33.71 | 34.23 | 50.00 |
| 839TTT | mal PCFV | 24.92 | 31.46 | 27.85 | 30.32 | 31.81 | 29.67 | 28.72 | 26.05 | 32.12 | 29.58 | 22.79 | 23.13 | 29.37 | 29.10 | 26.31 | 26.14 |
| 911TTT | mal PCFV | 30.42 | 31.06 | 27.58 | 31.99 | 30.59 | 33.24 | 31.23 | 29.47 | 30.06 | 32.08 | 29.75 | 30.24 | 29.33 | 29.38 | 31.83 | 31.21 |
| 981TTT | mal PCFV | 25.69 | 28.29 | 27.55 | 29.14 | 26.95 | 26.46 | 30.03 | 24.99 | 29.04 | 26.79 | 21.80 | 22.90 | 30.24 | 29.47 | 26.74 | 26.57 |
| | | 28.00 | 30.50 | 26.58 | 29.15 | 27.00 | 26.50 | 29.42 | 25.30 | 29.60 | 31.00 | 24.20 | 26.45 | 32.00 | 30.50 | 27.50 | 27.50 |

Table 23c

| | FN1/SGENE/C1of24/TPO2592 |
|---|---|
| Sensitivity | 95.31% |
| Specificity | 66.67% |

| | % Within +/- .5 of Cutoff | | | |
|---|---|---|---|---|
| | FN1 | SGENE | C1of24 | TPO2592 |
| Benign | 16.67% | 8.33% | 13.90% | 5.56% |
| Cancer | 12.50% | 15.60% | 20.30% | 10.93% |

Positive
Outliers
Markers with Best Performance

| BACTIN | FN1 | SGENE | C10f24 | TPO2592 |
|---|---|---|---|---|

| | | | | | |
|---|---|---|---|---|---|
| 915TT | benign DG | 26.69 | 30.09 | 33.60 | 31.01 | 21.83 |
| 924TT | benign GPH | 21.48 | 22.00 | 27.96 | 50.00 | 26.58 |
| 871TT | benign HCA | 23.42 | 25.17 | 26.99 | 25.78 | 24.19 |
| 805TT | benign HPS | 26.84 | 28.61 | 33.72 | 29.63 | 22.53 |
| 804TT | benign HT | 24.43 | 28.98 | 31.62 | 26.91 | 23.13 |
| 927TT | benign HT | 23.24 | 25.87 | 28.23 | 28.52 | 19.83 |
| 917TT | benign MA | 28.22 | 33.13 | 37.79 | 33.95 | 24.95 |
| 918TT | benign MA | 26.91 | 28.74 | 38.32 | 33.08 | 25.37 |
| 806TT | benign MG | 25.34 | 28.77 | 29.84 | 29.36 | 23.58 |
| 914TT | benign OH | 24.00 | 27.01 | 28.33 | 26.56 | 23.41 |
| 913TT | benign T | 25.53 | 26.17 | 34.07 | 31.91 | 24.88 |
| 807TT | benignHT | 24.58 | 28.98 | 31.35 | 27.69 | 24.85 |
| 842TT | benign FA | 25.92 | 29.53 | 32.71 | 33.81 | 24.44 |
| 862TT | benign FA | 26.86 | 29.92 | 50.00 | 38.86 | 22.54 |
| 863TT | benign FA | 24.56 | 29.48 | 27.28 | 30.25 | 22.89 |
| 864TT | benign FA | 26.15 | 30.08 | 30.48 | 31.93 | 22.65 |
| 865TT | benign FA | 26.21 | 26.92 | 30.93 | 47.61 | 22.37 |
| 866TT | benign FA | 27.00 | 28.77 | 32.61 | 31.60 | 23.43 |
| 867TT | benign FA | 24.89 | 30.37 | 33.81 | 31.53 | 21.95 |
| 869TT | benign FA | 24.99 | 28.58 | 28.11 | 29.46 | 22.14 |
| 907TT | benign FA | 25.09 | 27.00 | 35.86 | 30.46 | 21.81 |
| 920TT | benign FA | 25.37 | 28.37 | 48.63 | 28.90 | 21.83 |
| 937TT | benign FA | 23.32 | 28.00 | 36.65 | 32.90 | 20.06 |
| 938TT | benign FA | 23.39 | 26.20 | 26.34 | 29.88 | 21.34 |
| 939TT | benign FA | 25.58 | 29.13 | 34.24 | 31.70 | 23.85 |
| 940TT | benign FA | 25.33 | 27.51 | 29.38 | 31.78 | 21.28 |
| 941TT | benign FA | 26.84 | 30.89 | 31.13 | 40.24 | 24.30 |
| 957TT | benign FA | 24.37 | 27.49 | 31.29 | 31.38 | 25.62 |
| 923TT | benign FA | 23.12 | 26.83 | 31.48 | 30.17 | 22.43 |
| 926TT | benign FA | 22.89 | 22.10 | 30.84 | 27.40 | 22.86 |
| 955TT | benign FA | 26.97 | 29.65 | 29.22 | 31.37 | 25.76 |
| 956TT | benign FA | 27.07 | 29.58 | 30.07 | 32.24 | 24.09 |
| 960TT | benign FA | 25.12 | 27.33 | 27.89 | 30.38 | 20.86 |
| 921TT | benign FA | 22.10 | 25.87 | 30.89 | 34.76 | 19.18 |
| 961TT | benign FA | 28.34 | 31.04 | 35.11 | 31.46 | 24.61 |
| 962TT | benign FA | 23.81 | 28.78 | 27.51 | 29.61 | 20.25 |
| 823TT | malignant FC | 23.53 | 24.33 | 29.23 | 28.44 | 20.63 |

EP 2 518 166 B1

| | | | | | |
|---|---|---|---|---|---|
| 825TT | malignant FC | 25.84 | 31.36 | 28.74 | 28.29 | 24.59 |
| 826TT | malignant FC | 24.61 | 28.66 | 32.18 | 28.45 | 25.74 |
| 827TT | malignant FC | 24.99 | 28.10 | 28.53 | 26.03 | 23.06 |
| 828TT | malignant FC | 24.15 | 29.96 | 28.86 | 29.42 | 20.43 |
| 830TT | malignant FC | 23.53 | 30.09 | 27.51 | 29.30 | 25.54 |
| 840TT | malignant FC | 26.21 | 28.95 | 31.79 | 29.31 | 24.82 |
| 895TT | malignant FC | 24.03 | 26.39 | 29.67 | 32.15 | 22.96 |
| 896TT | malignant FC | 24.42 | 26.55 | 30.77 | 30.06 | 26.15 |
| 897TT | malignant FC | 24.87 | 26.27 | 27.13 | 26.16 | 22.10 |
| 898TT | malignant FC | 23.17 | 20.35 | 25.74 | 26.05 | 26.33 |
| 899TT | malignant FC | 26.16 | 21.34 | 29.92 | 29.38 | 30.55 |
| 900TT | malignant FC | 22.66 | 19.58 | 26.40 | 27.53 | 32.09 |
| 901TT | malignant FC | 24.63 | 27.38 | 21.76 | 30.77 | 45.31 |
| 902TT | malignant FC | 22.48 | 19.07 | 26.11 | 25.71 | 28.18 |
| 910TT | malignant FC | 24.98 | 28.71 | 26.34 | 28.49 | 24.76 |
| 936TT | malignant FC | 22.15 | 19.40 | 26.83 | 25.07 | 28.37 |
| 954TT | malignant FC | 21.76 | 28.01 | 24.64 | 25.51 | 20.78 |
| 968TT | malignant FC | 28.23 | 26.32 | 46.83 | 33.17 | 27.17 |
| 969TT | malignant FC | 25.80 | 27.84 | 42.77 | 45.43 | 23.59 |
| 970TT | malignant FC | 27.36 | 26.30 | 30.90 | 29.19 | 30.88 |
| 982TT | malignant FC | 26.16 | 27.30 | 29.32 | 35.41 | 22.21 |
| 983TT | malignant FC | 22.95 | 23.09 | 25.74 | 24.79 | 20.87 |
| 967TT | malignant FC | 23.84 | 25.99 | 28.46 | 25.78 | 25.88 |
| 831TT | malignant PC | 22.92 | 20.74 | 28.98 | 26.16 | 29.59 |
| 832TT | malignant PC | 25.88 | 26.78 | 30.99 | 29.25 | 25.60 |
| 834TT | malignant PC | 24.30 | 21.77 | 28.67 | 26.69 | 25.33 |
| 835TT | malignant PC | 23.68 | 20.52 | 29.06 | 26.23 | 29.43 |
| 836TT | malignant PC | 26.05 | 30.35 | 28.40 | 30.99 | 23.30 |
| 837TT | malignant PC | 23.03 | 21.98 | 28.84 | 27.16 | 22.10 |
| 838TT | malignant PC | 27.10 | 23.25 | 29.67 | 29.34 | 31.04 |
| 841TT | malignant PC | 24.61 | 25.17 | 25.80 | 24.71 | 32.06 |
| 874TT | malignant PC | 23.75 | 20.38 | 27.01 | 24.49 | 33.93 |
| 875TT | malignant PC | 22.97 | 21.28 | 27.93 | 26.82 | 25.32 |
| 876TT | malignant PC | 24.02 | 19.37 | 27.87 | 25.46 | 30.22 |
| 879TT | malignant PC | 24.09 | 20.13 | 29.49 | 28.76 | 33.05 |
| 880TT | malignant PC | 23.73 | 21.98 | 26.84 | 28.46 | 29.22 |

| | | | | | | |
|---|---|---|---|---|---|---|
| 881TT | malignant PC | 25.76 | 21.57 | 29.01 | 28.45 | 28.12 |
| 882TT | malignant PC | 23.60 | 19.25 | 26.85 | 28.21 | 30.32 |
| 883TT | malignant PC | 27.24 | 21.80 | 28.08 | 31.77 | 34.10 |
| 884TT | malignant PC | 24.78 | 20.55 | 28.48 | 28.97 | 25.94 |
| 885TT | malignant PC | 24.40 | 18.86 | 27.45 | 28.41 | 30.49 |
| 886TT | malignant PC | 26.66 | 21.62 | 28.93 | 29.99 | 27.57 |
| 887TT | malignant PC | 26.37 | 20.39 | 30.98 | 29.03 | 30.00 |
| 890TT | malignant PC | 23.01 | 19.82 | 25.47 | 27.50 | 25.53 |
| 892TT | malignant PC | 25.50 | 21.43 | 27.93 | 28.93 | 26.38 |
| 893TT | malignant PC | 22.27 | 18.05 | 26.79 | 25.24 | 28.93 |
| 894TT | malignant PC | 25.06 | 21.59 | 29.12 | 27.59 | 27.92 |
| 903TT | malignant PC | 24.67 | 23.24 | 28.03 | 29.55 | 25.56 |
| 904TT | malignant PC | 25.60 | 24.86 | 32.31 | 29.39 | 25.87 |
| 905TT | malignant PC | 23.52 | 20.64 | 30.32 | 27.66 | 32.35 |
| 906TT | malignant PC | 24.75 | 20.46 | 27.25 | 25.31 | 39.67 |
| 912TT | malignant PC | 26.06 | 28.74 | 35.11 | 28.35 | 28.77 |
| 928TT | malignant PC | 24.74 | 26.57 | 30.63 | 29.72 | 22.43 |
| 929TT | malignant PC | 25.88 | 21.77 | 29.79 | 28.31 | 30.77 |
| 930TT | malignant PC | 23.24 | 22.84 | 26.72 | 28.36 | 28.42 |
| 931TT | malignant PC | 24.56 | 29.48 | 27.22 | 31.99 | 30.19 |
| 932TT | malignant PC | 23.10 | 21.26 | 29.13 | 28.07 | 27.96 |
| 933TT | malignant PC | 26.28 | 24.46 | 30.25 | 29.28 | 24.64 |
| 934TT | malignant PC | 24.62 | 26.38 | 29.63 | 28.02 | 28.02 |
| 935TT | malignant PC | 22.89 | 19.38 | 28.59 | 24.60 | 29.46 |
| 839TT | malignant PCFV | 24.92 | 27.85 | 31.81 | 28.72 | 23.13 |
| 911TT | malignant PCFV | 30.42 | 27.58 | 30.59 | 31.23 | 30.24 |
| 981TT | malignant PCFV | 25.69 | 27.55 | 26.95 | 30.03 | 22.90 |
| | | 28.00 | 26.58 | 27.16 | 29.42 | 26.14 |

EP 2 518 166 B1

Table 24a

| | Thyroid Markers | | |
|---|---|---|---|
| | GLOGIN67 | HERC | PAX8 |
| % Total | 46.73% | 79.44% | 93.46% |

| Positive Outliers | | | |
|---|---|---|---|
| BACTIN | GLOGIN67 | HERC | PAX8 |

| 70 | 915TT | benign DG | 26.69 | 26.62 | 28.56 | 24.37 |
| 7 | 818TT | benign FA | 50.00 | 46.96 | 50.00 | 50.00 |
| 24 | 842TT | benign FA | 25.92 | 24.15 | 26.50 | 23.98 |
| 28 | 862TT | benign FA | 26.86 | 23.89 | 27.23 | 23.35 |
| 29 | 863TT | benign FA | 24.56 | 23.66 | 26.08 | 22.59 |
| 30 | 864TT | benign FA | 26.15 | 23.76 | 26.54 | 23.32 |
| 31 | 865TT | benign FA | 26.21 | 25.67 | 27.24 | 23.05 |
| 32 | 866TT | benign FA | 27.00 | 24.33 | 27.24 | 23.73 |
| 33 | 867TT | benign FA | 24.89 | 25.28 | 26.99 | 23.16 |
| 34 | 869TT | benign FA | 24.99 | 22.80 | 26.19 | 22.20 |
| 64 | 907TT | benign FA | 25.09 | 25.28 | 27.11 | 22.07 |
| 73 | 920TT | benign FA | 25.37 | 25.55 | 27.85 | 20.73 |
| 83 | 937TT | benign FA | 23.32 | 23.51 | 26.11 | 21.40 |
| 84 | 938TT | benign FA | 23.39 | 24.95 | 26.31 | 21.50 |
| 85 | 939TT | benign FA | 25.58 | 28.18 | 29.45 | 25.14 |
| 86 | 940TT | benign FA | 25.33 | 26.11 | 27.22 | 22.96 |
| 87 | 941TT | benign FA | 26.84 | 26.10 | 28.84 | 25.31 |
| 88 | 957TT | benign FA | 24.37 | 26.24 | 27.00 | 21.79 |
| 90 | 923TT | benign FA | 23.12 | 22.28 | 25.30 | 21.11 |
| 91 | 926TT | benign FA | 22.89 | 21.73 | 25.36 | 21.34 |
| 92 | 955TT | benign FA | 26.97 | 23.11 | 28.14 | 24.78 |
| 93 | 956TT | benign FA | 27.07 | 24.84 | 27.67 | 24.15 |
| 94 | 959TT | benign FA | 50.00 | 50.00 | 50.00 | 50.00 |
| 95 | 960TT | benign FA | 25.12 | 25.29 | 27.11 | 22.36 |
| 96 | 921TT | benign FA | 22.10 | 21.24 | 24.81 | 20.24 |
| 97 | 961TT | benign FA | 28.34 | 26.42 | 29.37 | 24.98 |
| 98 | 962TT | benign FA | 23.81 | 23.06 | 25.83 | 21.14 |
| 106 | 924TT | benign GPH | 21.48 | 21.31 | 24.55 | 20.45 |
| 35 | 871TT | benign HCA | 23.42 | 24.65 | 26.21 | 23.21 |
| 2 | 805TT | benign HPS | 26.84 | 24.49 | 26.94 | 23.37 |
| 1 | 804TT | benign HT | 24.43 | 24.27 | 26.79 | 23.92 |
| 107 | 927TT | benign HT | 23.24 | 23.24 | 25.48 | 21.50 |
| 71 | 917TT | benign MA | 28.22 | 34.84 | 30.68 | 25.79 |
| 72 | 918TT | benign MA | 26.91 | 29.71 | 29.63 | 25.71 |
| 3 | 806TT | benign MG | 25.34 | 24.33 | 26.04 | 22.91 |
| 69 | 914TT | benign OH | 24.00 | 24.85 | 28.46 | 24.56 |
| 68 | 913TT | benign T | 25.53 | 26.79 | 28.47 | 24.46 |

| | | | | | | |
|---|---|---|---|---|---|---|
| 4 | 807TT | benignHT | 24.58 | 25.72 | 26.37 | 24.73 |
| 8 | 823TT | malignant FC | 23.53 | 24.32 | 25.72 | 21.25 |
| 9 | 825TT | malignant FC | 25.84 | 26.16 | 27.73 | 23.30 |
| 10 | 826TT | malignant FC | 24.61 | 25.14 | 26.32 | 23.91 |
| 11 | 827TT | malignant FC | 24.99 | 23.16 | 26.52 | 23.29 |
| 12 | 828TT | malignant FC | 24.15 | 23.94 | 25.85 | 21.23 |
| 13 | 830TT | malignant FC | 23.53 | 22.72 | 25.01 | 22.05 |
| 22 | 840TT | malignant FC | 26.21 | 25.45 | 27.05 | 23.34 |
| 52 | 895TT | malignant FC | 24.03 | 23.04 | 26.40 | 20.15 |
| 53 | 896TT | malignant FC | 24.42 | 24.49 | 26.14 | 22.46 |
| 54 | 897TT | malignant FC | 24.87 | 22.14 | 26.67 | 20.56 |
| 55 | 898TT | malignant FC | 23.17 | 23.11 | 25.38 | 22.13 |
| 56 | 899TT | malignant FC | 26.16 | 23.13 | 28.13 | 24.19 |
| 57 | 900TT | malignant FC | 22.66 | 24.75 | 26.81 | 25.09 |
| 58 | 901TT | malignant FC | 24.63 | 34.54 | 25.44 | 50.00 |
| 59 | 902TT | malignant FC | 22.48 | 23.60 | 25.29 | 23.60 |
| 65 | 910TT | malignant FC | 24.98 | 24.23 | 26.69 | 22.61 |
| 82 | 936TT | malignant FC | 22.15 | 24.20 | 25.30 | 23.02 |
| 99 | 954TT | malignant FC | 21.76 | 21.98 | 25.33 | 21.25 |
| 100 | 968TT | malignant FC | 28.23 | 27.18 | 28.85 | 24.69 |
| 101 | 969TT | malignant FC | 25.80 | 22.92 | 26.95 | 22.97 |
| 102 | 970TT | malignant FC | 27.36 | 26.46 | 28.56 | 25.99 |
| 103 | 982TT | malignant FC | 26.16 | 24.58 | 26.80 | 22.11 |
| 104 | 983TT | malignant FC | 22.95 | 21.86 | 25.20 | 19.72 |
| 105 | 967TT | malignant FC | 23.84 | 21.66 | 25.76 | 22.21 |
| 14 | 831TT | malignant PC | 22.92 | 23.30 | 26.22 | 23.15 |
| 15 | 832TT | malignant PC | 25.88 | 24.82 | 26.66 | 23.64 |
| 16 | 834TT | malignant PC | 24.30 | 23.66 | 25.39 | 22.54 |
| 17 | 835TT | malignant PC | 23.68 | 23.83 | 26.43 | 23.57 |
| 18 | 836TT | malignant PC | 26.05 | 23.63 | 27.23 | 23.30 |
| 19 | 837TT | malignant PC | 23.03 | 21.96 | 24.90 | 21.36 |
| 20 | 838TT | malignant PC | 27.10 | 23.89 | 28.01 | 25.02 |
| 23 | 841TT | malignant PC | 24.61 | 21.73 | 26.09 | 24.13 |
| 36 | 874TT | malignant PC | 23.75 | 24.09 | 26.07 | 23.55 |
| 37 | 875TT | malignant PC | 22.97 | 22.16 | 26.39 | 22.01 |
| 38 | 876TT | malignant PC | 24.02 | 22.27 | 25.44 | 22.64 |
| 39 | 879TT | malignant PC | 24.09 | 22.84 | 26.63 | 23.56 |

| | | | | | | |
|---|---|---|---|---|---|---|
| 40 | 880TT | malignant PC | 23.73 | 23.63 | 26.14 | 23.77 |
| 41 | 881TT | malignant PC | 25.76 | 23.74 | 27.61 | 24.12 |
| 42 | 882TT | malignant PC | 23.60 | 22.39 | 25.12 | 22.19 |
| 43 | 883TT | malignant PC | 27.24 | 22.74 | 28.54 | 24.87 |
| 44 | 884TT | malignant PC | 24.78 | 23.40 | 27.50 | 22.92 |
| 45 | 885TT | malignant PC | 24.40 | 21.98 | 26.89 | 22.62 |
| 46 | 886TT | malignant PC | 26.66 | 24.24 | 28.20 | 24.30 |
| 47 | 887TT | malignant PC | 26.37 | 24.60 | 27.77 | 24.56 |
| 48 | 890TT | malignant PC | 23.01 | 22.33 | 26.20 | 23.04 |
| 49 | 892TT | malignant PC | 25.50 | 25.21 | 26.30 | 23.42 |
| 50 | 893TT | malignant PC | 22.27 | 22.67 | 25.76 | 22.25 |
| 51 | 894TT | malignant PC | 25.06 | 24.39 | 27.38 | 24.22 |
| 60 | 903TT | malignant PC | 24.67 | 24.29 | 26.64 | 23.20 |
| 61 | 904TT | malignant PC | 25.60 | 26.45 | 28.57 | 25.53 |
| 62 | 905TT | malignant PC | 23.52 | 23.33 | 27.22 | 24.11 |
| 63 | 906TT | malignant PC | 24.75 | 23.57 | 27.28 | 27.56 |
| 67 | 912TT | malignant PC | 26.06 | 25.15 | 28.99 | 26.56 |
| 74 | 928TT | malignant PC | 24.74 | 23.13 | 27.06 | 22.00 |
| 75 | 929TT | malignant PC | 25.88 | 24.40 | 27.97 | 25.41 |
| 76 | 930TT | malignant PC | 23.24 | 23.43 | 26.78 | 23.78 |
| 77 | 931TT | malignant PC | 24.56 | 23.61 | 27.11 | 22.39 |
| 78 | 932TT | malignant PC | 23.10 | 23.17 | 25.82 | 22.39 |
| 79 | 933TT | malignant PC | 26.28 | 24.09 | 27.35 | 23.29 |
| 80 | 934TT | malignant PC | 24.62 | 30.20 | 28.82 | 27.39 |
| 81 | 935TT | malignant PC | 22.89 | 23.75 | 26.11 | 23.15 |
| 21 | 839TT | malignant PCFV | 24.92 | 24.22 | 26.85 | 23.39 |
| 66 | 911TT | malignant PCFV | 30.42 | 25.66 | 30.30 | 27.22 |
| 89 | 981TT | malignant PCFV | 25.69 | 22.54 | 26.67 | 23.12 |
| 5 | 809TT | normal | 26.05 | 24.93 | 27.35 | 23.75 |
| 6 | 817TT | normal | 26.92 | 25.30 | 27.61 | 23.68 |
| 25 | 844TT | normal | 26.61 | 26.30 | 28.34 | 24.68 |
| 26 | 845TT | normal | 25.08 | 25.10 | 26.89 | 23.85 |
| 27 | 851TT | normal | 25.92 | 24.42 | 26.81 | 23.16 |
| | | | 28.00 | 24.00 | 28.00 | 26.00 |

183

|  | | GOLGIN67 | HERC | PAX8 |
|---|---|---|---|---|
| Sensitivity | | 66.00% | 22.00% | 99.00% |
| Specificity | | 50.00% | 44.74% | 100.00% |

Positive
Outliers
Markers with Best Performance

|  | | BACTIN | GOLGIN67 | HERC | PAX8 |
|---|---|---|---|---|---|
| 915TT | benign DG | 26.69 | 26.62 | 28.56 | 24.37 |
| 924TT | benign GPH | 21.48 | 21.31 | 24.55 | 20.45 |
| 871TT | benign HCA | 23.42 | 24.65 | 26.21 | 23.21 |
| 805TT | benign HPS | 26.84 | 24.49 | 26.94 | 23.37 |
| 804TT | benign HT | 24.43 | 24.27 | 26.79 | 23.92 |
| 927TT | benign HT | 23.24 | 23.24 | 25.48 | 21.50 |
| 917TT | benign MA | 28.22 | 34.84 | 30.68 | 25.79 |
| 918TT | benign MA | 26.91 | 29.71 | 29.63 | 25.71 |
| 806TT | benign MG | 25.34 | 24.33 | 26.04 | 22.91 |
| 914TT | benign OH | 24.00 | 24.85 | 28.46 | 24.56 |
| 913TT | benign T | 25.53 | 26.79 | 28.47 | 24.46 |
| 807TT | benignHT | 24.58 | 25.72 | 26.37 | 24.73 |
| 842TT | benign FA | 25.92 | 24.15 | 26.50 | 23.98 |
| 862TT | benign FA | 26.86 | 23.89 | 27.23 | 23.35 |
| 863TT | benign FA | 24.56 | 23.66 | 26.08 | 22.59 |
| 864TT | benign FA | 26.15 | 23.76 | 26.54 | 23.32 |
| 865TT | benign FA | 26.21 | 25.67 | 27.24 | 23.05 |
| 866TT | benign FA | 27.00 | 24.33 | 27.24 | 23.73 |
| 867TT | benign FA | 24.89 | 25.28 | 26.99 | 23.16 |
| 869TT | benign FA | 24.99 | 22.80 | 26.19 | 22.20 |
| 907TT | benign FA | 25.09 | 25.28 | 27.11 | 22.07 |
| 920TT | benign FA | 25.37 | 25.55 | 27.85 | 20.73 |
| 937TT | benign FA | 23.32 | 23.51 | 26.11 | 21.40 |
| 938TT | benign FA | 23.39 | 24.95 | 26.31 | 21.50 |
| 939TT | benign FA | 25.58 | 28.18 | 29.45 | 25.14 |
| 940TT | benign FA | 25.33 | 26.11 | 27.22 | 22.96 |
| 941TT | benign FA | 26.84 | 26.10 | 28.84 | 25.31 |
| 957TT | benign FA | 24.37 | 26.24 | 27.00 | 21.79 |

Table 24b

EP 2 518 166 B1

| ID | Classification | | | | |
|---|---|---|---|---|---|
| 923TT | benign FA | 23.12 | 22.28 | 25.30 | 21.11 |
| 926TT | benign FA | 22.89 | 21.73 | 25.36 | 21.34 |
| 955TT | benign FA | 26.97 | 23.11 | 28.14 | 24.78 |
| 956TT | benign FA | 27.07 | 24.84 | 27.67 | 24.15 |
| 960TT | benign FA | 25.12 | 25.29 | 27.11 | 22.36 |
| 921TT | benign FA | 22.10 | 21.24 | 24.81 | 20.24 |
| 961TT | benign FA | 28.34 | 26.42 | 29.37 | 24.98 |
| 962TT | benign FA | 23.81 | 23.06 | 25.83 | 21.14 |
| 823TT | malignant FC | 23.53 | 24.32 | 25.72 | 21.25 |
| 825TT | malignant FC | 25.84 | 26.16 | 27.73 | 23.30 |
| 826TT | malignant FC | 24.61 | 25.14 | 26.32 | 23.91 |
| 827TT | malignant FC | 24.99 | 23.16 | 26.52 | 23.29 |
| 828TT | malignant FC | 24.15 | 23.94 | 25.85 | 21.23 |
| 830TT | malignant FC | 23.53 | 22.72 | 25.01 | 22.05 |
| 840TT | malignant FC | 26.21 | 25.45 | 27.05 | 23.34 |
| 895TT | malignant FC | 24.03 | 23.04 | 26.40 | 20.15 |
| 896TT | malignant FC | 24.42 | 24.49 | 26.14 | 22.46 |
| 897TT | malignant FC | 24.87 | 22.14 | 26.67 | 20.56 |
| 898TT | malignant FC | 23.17 | 23.11 | 25.38 | 22.13 |
| 899TT | malignant FC | 26.16 | 23.13 | 28.13 | 24.19 |
| 900TT | malignant FC | 22.66 | 24.75 | 26.81 | 25.09 |
| 901TT | malignant FC | 24.63 | 34.54 | 25.44 | 50.00 |
| 902TT | malignant FC | 22.48 | 23.60 | 25.29 | 23.60 |
| 910TT | malignant FC | 24.98 | 24.23 | 26.69 | 22.61 |
| 936TT | malignant FC | 22.15 | 24.20 | 25.30 | 23.02 |
| 954TT | malignant FC | 21.76 | 21.98 | 25.33 | 21.25 |
| 968TT | malignant FC | 28.23 | 27.18 | 28.85 | 24.69 |
| 969TT | malignant FC | 25.80 | 22.92 | 26.95 | 22.97 |
| 970TT | malignant FC | 27.36 | 26.46 | 28.56 | 25.99 |
| 982TT | malignant FC | 26.16 | 24.58 | 26.80 | 22.11 |
| 983TT | malignant FC | 22.95 | 21.86 | 25.20 | 19.72 |
| 967TT | malignant FC | 23.84 | 21.66 | 25.76 | 22.21 |
| 831TT | malignant PC | 22.92 | 23.30 | 26.22 | 23.15 |
| 832TT | malignant PC | 25.88 | 24.82 | 26.66 | 23.64 |
| 834TT | malignant PC | 24.30 | 23.66 | 25.39 | 22.54 |
| 835TT | malignant PC | 23.68 | 23.83 | 26.43 | 23.57 |

| | | | | | |
|---|---|---|---|---|---|
| 836TT | malignant PC | 26.05 | 23.63 | 27.23 | 23.30 |
| 837TT | malignant PC | 23.03 | 21.96 | 24.90 | 21.36 |
| 838TT | malignant PC | 27.10 | 23.89 | 28.01 | 25.02 |
| 841TT | malignant PC | 24.61 | 21.73 | 26.09 | 24.13 |
| 874TT | malignant PC | 23.75 | 24.09 | 26.07 | 23.55 |
| 875TT | malignant PC | 22.97 | 22.16 | 26.39 | 22.01 |
| 876TT | malignant PC | 24.02 | 22.27 | 25.44 | 22.64 |
| 879TT | malignant PC | 24.09 | 22.84 | 26.63 | 23.56 |
| 880TT | malignant PC | 23.73 | 23.63 | 26.14 | 23.77 |
| 881TT | malignant PC | 25.76 | 23.74 | 27.61 | 24.12 |
| 882TT | malignant PC | 23.60 | 22.39 | 25.12 | 22.19 |
| 883TT | malignant PC | 27.24 | 22.74 | 28.54 | 24.87 |
| 884TT | malignant PC | 24.78 | 23.40 | 27.50 | 22.92 |
| 885TT | malignant PC | 24.40 | 21.98 | 26.89 | 22.62 |
| 886TT | malignant PC | 26.66 | 24.24 | 28.20 | 24.30 |
| 887TT | malignant PC | 26.37 | 24.60 | 27.77 | 24.56 |
| 890TT | malignant PC | 23.01 | 22.33 | 26.20 | 23.04 |
| 892TT | malignant PC | 25.50 | 25.21 | 26.30 | 23.42 |
| 893TT | malignant PC | 22.27 | 22.67 | 25.76 | 22.25 |
| 894TT | malignant PC | 25.06 | 24.39 | 27.38 | 24.22 |
| 903TT | malignant PC | 24.67 | 24.29 | 26.64 | 23.20 |
| 904TT | malignant PC | 25.60 | 26.45 | 28.57 | 25.53 |
| 905TT | malignant PC | 23.52 | 23.33 | 27.22 | 24.11 |
| 906TT | malignant PC | 24.75 | 23.57 | 27.28 | 27.56 |
| 912TT | malignant PC | 26.06 | 25.15 | 28.99 | 26.56 |
| 928TT | malignant PC | 24.74 | 23.13 | 27.06 | 22.00 |
| 929TT | malignant PC | 25.88 | 24.40 | 27.97 | 25.41 |
| 930TT | malignant PC | 23.24 | 23.43 | 26.78 | 23.78 |
| 931TT | malignant PC | 24.56 | 23.61 | 27.11 | 22.39 |
| 932TT | malignant PC | 23.10 | 23.17 | 25.82 | 22.39 |
| 933TT | malignant PC | 26.28 | 24.09 | 27.35 | 23.29 |
| 934TT | malignant PC | 24.62 | 30.20 | 28.82 | 27.39 |
| 935TT | malignant PC | 22.89 | 23.75 | 26.11 | 23.15 |
| 839TT | malignant PCFV | 24.92 | 24.22 | 26.85 | 23.39 |
| 911TT | malignant PCFV | 30.42 | 25.66 | 30.30 | 27.22 |
| 981TT | malignant PCFV | 25.69 | 22.54 | 26.67 | 23.12 |
| S1 | SkinN | 20.34 | 23.78 | 24.91 | 49.98 |

| | | | | | |
|---|---|---|---|---|---|
| S2 | SkinN | 22.37 | 24.46 | 26.28 | 50.00 |
| S3 | SkinN | 20.02 | 22.53 | 25.16 | 50.00 |
| S4 | SkinN | 23.90 | 26.08 | 28.22 | 33.80 |
| S5 | SkinN | 21.64 | 23.53 | 26.22 | 33.76 |
| S6 | SkinN | 21.57 | 24.11 | 26.45 | 50.00 |
| S7 | SkinN | 20.88 | 22.50 | 25.75 | 38.50 |
| S8 | SkinN | 21.56 | 23.95 | 25.81 | 50.00 |
| S9 | SkinN | 21.27 | 22.88 | 24.29 | 50.00 |
| S10 | SkinN | 21.15 | 23.39 | 25.77 | 50.00 |
| Mu1 | MuscleN | 23.31 | 27.76 | 27.24 | 50.00 |
| Mu2 | MuscleN | 25.07 | 43.49 | 28.72 | 50.00 |
| Mu3 | MuscleN | 24.56 | 23.94 | 24.62 | 50.00 |
| Mu4 | MuscleN | 25.07 | 29.17 | 26.64 | 50.00 |
| Mu5 | MuscleN | 24.57 | 32.11 | 25.22 | 50.00 |
| Mu6 | MuscleN | 23.11 | 25.30 | 23.66 | 50.00 |
| M1 | Blood | 21.38 | 25.62 | 26.20 | 50.00 |
| M2 | Blood | 21.02 | 24.07 | 25.61 | 37.18 |
| M3 | Blood | 21.44 | 25.60 | 26.33 | 50.00 |
| M4 | Blood | 21.04 | 24.35 | 26.15 | 50.00 |
| M5 | Blood | 20.41 | 23.17 | 25.33 | 50.00 |
| M6 | Blood | 20.43 | 25.72 | 25.54 | 38.36 |
| M7 | Blood | 20.76 | 23.66 | 25.79 | 50.00 |
| M8 | Blood | 20.91 | 25.23 | 25.66 | 50.00 |
| M9 | Blood | 21.31 | 24.69 | 25.46 | 50.00 |
| M10 | Blood | 21.84 | 28.56 | 26.55 | 50.00 |
| M12 | Blood | 20.84 | 23.65 | 25.79 | 50.00 |
| M14 | Blood | 21.45 | 25.02 | 25.91 | 50.00 |
| M15 | Blood | 21.10 | 24.80 | 26.01 | 50.00 |
| M16 | Blood | 22.13 | 25.11 | 26.17 | 50.00 |
| M17 | Blood | 20.85 | 24.31 | 25.79 | 50.00 |
| M18 | Blood | 20.97 | 25.33 | 26.14 | 50.00 |
| M19 | Blood | 21.97 | 25.22 | 26.45 | 50.00 |
| M20 | Blood | 20.52 | 23.67 | 25.20 | 38.40 |
| M21 | Blood | 22.29 | 24.91 | 26.41 | 50.00 |
| M22 | Blood | 22.10 | 25.56 | 26.86 | 50.00 |
| M23 | Blood | 20.92 | 24.49 | 25.52 | 50.00 |
| M24 | Blood | 21.30 | 24.03 | 25.93 | 50.00 |

28.00

26.00

24.50

28.00

Table 25

| Sequence identifications | | | | |
|---|---|---|---|---|
| SEQ NO: | psid | Name | Accession No. | Description |
| 1 | 200635_s_at | | AU145351 | Hs.75216 prot tyrosine phosphatase, rec. type, F |
| 2 | 200771_at | | NM_002293 | laminin, gamma 1 |
| 3 | 201069_at | | NM_004530 | matrix metalloproteinase 2 |
| 4 | 201117_s_at | CPE | NM_001873 | carboxypeptidase E |
| 5 | 201150_s_at | | NM_000362 | tissue inhibitor of metalloproteinase 3 |
| 6 | 201185_at | | NM_002775 | protease, serine, 11 |
| 7 | 201203_s_at | | AI921320 | ribosome binding protein 1 |
| 8 | 201212_at | | NM_005606 | cysteine protease |
| 9 | 201289_at | CYR61 | NM_001554 | cysteine-rich, angiogenic inducer, 61 |
| 10 | 201292_at | | NM_001067 | topoisomerase (DNA) II alpha |
| 11 | 201418_s_at | SOX4 | NM_003107 | SRY (sex determining region Y)-box 4 |
| 12 | 201427_s_at | SEPP1 | NM_005410 | Selenoprotein P, plasma, 1 |

(continued)

| Sequence identifications | | | | |
|---|---|---|---|---|
| SEQ NO: | psid | Name | Accession No. | Description |
| 13 | 201430_s_at | DPYSL3 | NM_001387 | dihydropyrimidinase-like 3 |
| 14 | 201431_s_at | DPYSL3 | NM_001387 | dihydropyrimidinase-like 3 |
| 15 | 201438_at | COL6A3 | NM_004369 | collagen, type VI, alpha 3 |
| 16 | 201474_s_at | ITGA3 | NM_002204 | integrin, $\alpha$ 3 transcript variant a |
| 17 | 201505_at | LAMB1 | NM_002291 | laminin, beta 1 |
| 18 | 201508_at | IGFBP4 | NM_001552 | Insulin-like growth factor-binding protein 4 |
| 19 | 201525_at | APOD | NM_001647 | apolipoprotein D |
| 20 | 201645_at | HXB | NM_002160 | hexabrachion (tenascin C, cytotactin) |
| 21 | 201650_at | KRT19 | NM_002276 | keratin 19 |
| 22 | 201667_at | GJA1 | NM_000165 | gap junction protein, $\alpha$ 1, 43kD (connexin 43) |
| 23 | 201744_s_at | LUM | NM_002345 | lumican |
| 24 | 201792_at | AEBP1 | NM_001129 | AE-binding protein 1 |
| 25 | 201852_x_at | | AI813758 | Collaqen, type III, alpha 1 |
| 26 | 201893_x_at | | AF138300 | decorin variant A |
| 27 | 201983_s_at | | AW157070 | epidermal growth factor receptor |
| 28 | 202133_at | | AA081084 | Transcriptional co-activator w PDZ-binding motif |
| 29 | 202219_at | SLC6A8 | NM_005629 | solute carrier family 6, member 8 |
| 30 | 202237_at | NNMT | NM_006169 | nicotinamide N-methyltransferase |
| 31 | 202286_s_at | | NM_002353 | tumor-associated calcium signal transducer 2 |
| 32 | 202291_s_at | | NM_000900 | matrix Gla protein |
| 33 | 202310_s_at | | NM_000088 | proalpha 1 (I) chain of type I procollagen |
| 34 | 202350_s_at | MATN2 | NM_002380 | matrilin 2 precursor, transcript variant 1 |
| 35 | 202357_s_at | BF | NM_001710 | B-factor, properdin |
| 36 | 202363_at | | AF231124 | testican-1 |
| 37 | 202376_at | SERPINA3 | NM_001085 | Ser (or Cys) proteinase inhib clade A mem 3 |
| 38 | 202404_s_at | COL1A2 | NM_000089 | collagen, type I, alpha 2 |
| 39 | 202440_s_at | | NM_005418 | suppression of tumorigenicity 5 |
| 40 | 202504_at | ATDC | NM_012101 | ataxia-telangiectasia group D-assoc. protein |
| 41 | 202575_at | CRABP2 | NM_001878 | cellular retinoic acid-binding protein 2 |
| 42 | 202588_at | AK1 | NM_000476 | adenylate kinase 1 |
| 43 | 202712_s_at | CKMT1 | NM_020990 | creatine kinase, mitochondrial 1 |
| 44 | 202796_at | KIAA1029 | NM_007286 | synaptopodin |
| 45 | 202826_at | SPINT1 | NM_003710 | serine protease inhibitor, Kunitz type 1 |
| 46 | 202834_at | SERPINA8 | NM_000029 | Ser (or Cys) proteinase inhib, clade A mem 8 |
| 47 | 202898_at | KIAA0468 | NM_014654 | KIAA0468 gene product |
| 48 | 202992_at | C7 | NM_000587 | complement component 7 |

(continued)

| | | | | Sequence identifications |
|---|---|---|---|---|
| SEQ NO: | psid | Name | Accession No. | Description |
| 49 | 203021_at | SLPI | NM_003064 | secretory leukocyte protease inhibitor |
| 50 | 203083_at | THBS2 | NM_003247 | thrombospondin 2 |
| 51 | 203180_at | ALDH1A3 | NM_000693 | aldehyde dehydrogenase 1 family, mem. A3 |
| 52 | 203228_at | PAFAH1B3 | NM_002573 | platelet-activating factor acetylhydrolase, isoform Ib, γ sub |
| 53 | 203256_at | CDH3 | NM_001793 | cadherin 3, type 1, P-cadherin (placental) |
| 54 | 203349_s_at | ETV5 | NM_004454 | ets variant gene 5 (ets-related molecule) |
| 55 | 203352_at | ORC4L | NM_002552 | origin recognition complex, subunit 4-like |
| 56 | 203354_s_at | | NM_015310 | |
| 57 | 203381_s_at | | NM_000041 | |
| 58 | 203382_s_ at | APOE | NM_000041 | apolipoprotein E |
| 59 | 203407_at | PPL | NM_002705 | periplakin |
| 60 | 203417_at | MFAP2 | NM_017459 | microfibrillar-associated protein 2, tran var 1 |
| 61 | 203438_at | | NM_003714 | stanniocalcin 2 |
| 62 | 203453_at | SCNN1A | NM_001038 | sodium channel, nonvoltage-gated 1 α |
| 63 | 203499_at | EPHA2 | NM_004431 | EphA2 |
| 64 | 203548_s_at | | NM_000237 | lipoprotein lipase |
| 65 | 203570_at | LOXL1 | NM_005576 | lysyl oxidase-like 1 |
| 66 | 203632_s_at | GPRC5B | NM_016235 | G protein-coupled rec, fam C, group 5, mem B |
| 67 | 203673_at | TG | NM_003235 | thyroglobulin |
| 68 | 203699_s_at | | NM_013989 | type II iodothyronine deiodinase |
| 69 | 203700_s_at | DIO2 | NM_013989 | deiodinase, iodothyronine, type II, tran var 1 |
| 70 | 203786_s_at | TPD52L1 | NM_003287 | tumor protein D52-like 1 |
| 71 | 203851_at | IGFBP6 | NM_002178 | insulin-like growth factor binding protein 6 |
| 72 | 203854_at | IF | NM_000204 | I factor (complement) |
| 73 | 203859_s_at | PALM | NM_002579 | paralemmin |
| 74 | 203875_at | | NM_003069 | SWISNF related, matrix assoc, actin dep regulator of chromatin subfam a mem 1 |
| 75 | 203881_s_at | | NM_004010 | dystrophin, includes DXS142, DXS164, DXS206, DXS230, DXS239, DXS268, DXS269, DXS270, DXS272 (DMD), transcript variant Dp427p2 |
| 76 | 203889_at | SGNE1 | NM_003020 | secretory granule, neuroendocrine protein 1 |
| 77 | 203911_at | RAP1GA1 | NM_002885 | RAP1, GTPase activating protein 1 |
| 78 | 203934_at | KDR | NM_002253 | kinase insert domain receptor |
| 79 | 203986_at | GENX-3414 | NM_003943 | genethonin 1 |
| 80 | 204105_s_at | NRCAM | NM_005010 | neuronal cell adhesion molecule |
| 81 | 204124_at | NaPi-IIb | AF146796 | Na dependent phosphate transporter isoform |

(continued)

| SEQ NO: | psid | Name | Accession No. | Description |
|---|---|---|---|---|
| | | Sequence identifications | | |
| 82 | 204149_s_at | GSTM4 | NM_000850 | glutathione S-transferase M4 |
| 83 | 204152_s_at | | AI738965 | manic fringe (Drosophila) homolog |
| 84 | 204154_at | CDO1 | NM_001801 | cysteine dioxygenase, type I |
| 85 | 204259_at | MMP7 | NM_002423 | matrix metalloproteinase 7 (matrilysin, uterine) |
| 86 | 204260_at | CHGB | NM_001819 | chromogranin B (secretogranin 1) |
| 87 | 204268_at | S100A2 | NM_005978 | S100 calcium-binding protein A2 |
| 88 | 204288_s_at | ARGBP2 | NM_021069 | ArgAbl-interacting prot ArgBP2, trans var 2 |
| 89 | 204298_s_at | LOX | NM_002317 | lysyl oxidase |
| 90 | 204337_at | | NM_005613 | regulator of G-protein signalling 4 |
| 91 | 204416_x_at | APOC1 | NM_001645 | apolipoprotein C-I |
| 92 | 204424_s_at | | NM_018640 | neuronal specific transcription factor DAT1 |
| 93 | 204433_s_at | | NM_006038 | spermatogenesis associated PD1 |
| 94 | 204442_x_at | LTBP4 | NM_003573 | latent transforming GFβ binding protein 4 |
| 95 | 204452_s_at | | NM_003505 | frizzled 1 |
| 96 | 204476_s_at | PC | NM_022172 | pyruvate carboxylase |
| 97 | 204503_at | EVPL | NM_001988 | envoplakin |
| 98 | 204591_at | CHL1 | NM_006614 | cell adhesion molecule w/ homology to homolog L1 |
| 99 | 204600_at | EPHB3 | NM_004443 | EphB3 |
| 100 | 204623_at | TFF3 | NM_003226 | trefoil factor 3 (intestinal) |
| 101 | 204625_s_at | | NM_000212 | integrin, β3 (platelet glycoprotein IIIa, CD61) |
| 102 | 204697_s_at | CHGA | NM_001275 | chromogranin A |
| 103 | 204741_at | BICD1 | NM_001714 | Bicaudal D (Drosophila) homolog 1 |
| 104 | 204753_s_at | HLF | NM_002126 | hepatic leukemia factor |
| 105 | 204754_at | HLF | NM_002126 | hepatic leukemia factor |
| 106 | 204755_x_at | HLF | NM_002126 | hepatic leukemia factor |
| 107 | 204787_at | Z391G | NM_007268 | Ig superfamily protein |
| 108 | 204797_s_at | EMAPL | NM_004434 | echinoderm microtubule-associated pro-like |
| 109 | 204869_at | | AL031664 | DNA seq RP4-531 H16 chrom 20p11.22-12 |
| 110 | 204870_s_at | PCSK2 | NM_002594 | proprotein convertase subtilisinkexin type 2 |
| 111 | 204933_s_at | TNFRSF11 B | NM_002546 | TNFR superfamily, member 11b |
| 112 | 204934_s_at | HPN | NM_002151 | hepsin (transmembrane protease, serine 1) |
| 113 | 204944_at | PTPRG | NM_002841 | protein tyrosine phosphatase receptor type G |
| 114 | 204964_s_at | SSPN | NM_005086 | sarcospan (Kras oncogene-associated gene) |
| 115 | 204975_at | EMP2 | NM_001424 | epithelial membrane protein 2 |
| 116 | 204990_s_at | ITGB4 | NM_000213 | integrin, beta 4 |

(continued)

| SEQ NO: | psid | Name | Accession No. | Description |
|---|---|---|---|---|
| | | | **Sequence identifications** | |
| 117 | 205051_s_at | KIT | NM_000222 | v-kit Hardy-Zuckerman 4 feline sarcoma viral oncogene homolog |
| 118 | 205110_s_at | FGF13 | NM_004114 | fibroblast qrowth factor 13 |
| 119 | 205153_s_at | TNFRSF5 | NM_001250 | TNFR superfamily, mem 5 |
| 120 | 205168_at | DDR2 | NM_006182 | discoidin domain receptor family, member 2 |
| 121 | 205258_at | INHBB | NM_002193 | inhibin, β B (activin AB β polypeptide) |
| 122 | 205286_at | | NM_003222 | transcription factor AP-2 gamma |
| 123 | 205325_at | KIAA0273 | NM_014759 | KIAA0273 gene product |
| 124 | 205336_at | PVALB | NM_002854 | parvalbumin |
| 125 | 205402_x_at | PRSS2 | NM_002770 | protease, serine, 2 (trypsin 2) |
| 126 | 205413_at | C11ORF8 | NM_001584 | chromosome 11 open reading frame 8 |
| 127 | 205455_at | MST1R | NM_002447 | macrophage stimulating 1 receptor |
| 128 | 205470_s_ at | KLK11 | NM_006853 | kallikrein 11 |
| 129 | 205479_s_at | PLAU | NM_002658 | plasminogen activator, urokinase |
| 130 | 205481_at | ADORA1 | NM_000674 | adenosine A1 receptor |
| 131 | 205485_at | RYR1 | NM_000540 | ryanodine receptor 1 (skeletal) |
| 132 | 205490_x_at | connexin 31 | NM_024009 | gap junction protein, beta 3, 31 kD |
| 133 | 205531_s_at | GA | NM_013267 | breast cell glutaminase |
| 134 | 205593_s_at | PDE9A | NM_002606 | phosphodiesterase 9A |
| 135 | 205614_x_at | MST1 | NM_020998 | macrophage stimulating 1 |
| 136 | 205627_at | | NM_001785 | cytidine deaminase |
| 137 | 205639_at | | NM_001637 | acyloxyacyl hydrolase |
| 138 | 205683_x_at | TPSB1 | NM_003294 | tryptase beta 1 |
| 139 | 205689_at | KIAA0435 | NM_014801 | KIAA0435 gene product |
| 140 | 205700_at | RODH | NM_003725 | oxidative 3 α hydroxysteroid dehydrogenase; retinol dehydrogenase; 3-hydroxysteroid epimerase |
| 141 | 205710_at | LRP2 | NM_004525 | low density lipoprotein-related protein 2 |
| 142 | 205715_at | BST1 | NM_004334 | bone marrow stromal cell antigen 1 |
| 143 | 205717_x_at | | NM_002588 | protocadherin gamma subfamily C, 3 |
| 144 | 205728_at | | AL022718 | DNA seq from clone 1052M9 on chrom Xq25 |
| 145 | 205747_at | CBLN1 | NM_004352 | cerebellin 1 precursor |
| 146 | 205778_at | KLK7 | NM_005046 | kallikrein 7 (chymotryptic, stratum corneum) |
| 147 | 205858_at | NGFR | NM_002507 | nerve growth factor receptor |
| 148 | 205927_s_at | CTSE | NM_001910 | cathepsin E |
| 149 | 205980_s_at | ARHGAP8 | NM_015366 | Rho GTPase activating protein 8 |
| 150 | 206002_at | GPR64 | NM_005756 | G protein-coupled receptor 64 |

(continued)

| | Sequence identifications | | | |
|---|---|---|---|---|
| SEQ NO: | psid | Name | Accession No. | Description |
| 151 | 206114_at | EPHA4 | NM_004438 | EphA4 |
| 152 | 206390_x_at | | NM_002619 | platelet factor 4 |
| 153 | 206594_at | KIAA0135 | NM_015148 | KIAA0135 protein |
| 154 | 206595_at | CST6 | NM_001323 | cystatin EM |
| 155 | 206714_at | ALOX15B | NM_001141 | arachidonate 15-lipoxygenase, second type |
| 156 | 206757_at | PDE5A | NM_001083 | phosphodiesterase 5A, cGMP-specific |
| 157 | 206866_at | CDH4 | NM_001794 | cadherin 4, type 1, R-cadherin (retinal) |
| 158 | 206884_s_at | SCEL | NM_003843 | sciellin |
| 159 | 206912_at | FOXE1 | NM_004473 | forkhead box E1(thyroid transcription factor2) |
| 160 | 207111_at | | NM_001974 | egf-like module cont., mucin-like, hormone rec-like seq 1 |
| 161 | 207144_s_at | CITED1 | NM_004143 | Cbpp300-interacting transactivator, with GluAsp-rich carboxy-terminal domain, 1 |
| 162 | 207173_x_at | | NM_001797 | OB-cadherin-1 |
| 163 | 207674_at | FCAR | NM_002000 | Fc fragment of IgA |
| 164 | 207695_s_at | IGSF1 | NM_001555 | immunoglobulin superfamily, member 1 |
| 165 | 207795_s_at | CD94 | AB009597 | |
| 166 | 207826_s_at | ID3 | NM_002167 | inhibitor of DNA binding 3, dominant negative protein |
| 167 | 207923_x_at | PAX8 | NM_013953 | paired box gene 8 |
| 168 | 208396_s_at | PDE1A | NM_005019 | phosphodiesterase 1A, calmodulin-dependent |
| 169 | 208451_s_at | C4B | NM_000592 | complement component 4B |
| 170 | 208712_at | PRAD1 | M73554 | cyclinD1 |
| 171 | 208747_s_at | | NM_001734 | subcomponent C1s, α- and β-chains |
| 172 | 209021_x_at | | BC001331 | Similar to KIAA0652 gene product |
| 173 | 209035_at | | NM_002391 | midkine |
| 174 | 209071_s_at | | AF159570 | regulator of G-protein signalling 5 |
| 175 | 209079_x_at | | AF152318 | protocadherin gamma A1 |
| 176 | 209173_at | | NM_006408 | putative secreted protein XAG |
| 177 | 209208_at | | NM_004870 | clone 015e11 My008 protein |
| 178 | 209228_x_at | | NM_006765 | Putative prostate cancer tumor suppressor |
| 179 | 209270_at | LAMB3 | NM_000228 | laminin S B3 chain |
| 180 | 209280_at | | NM_006039 | chromosome 17 unknown product mRNA |
| 181 | 209291_at | helix-loop-helix | NM_001546 | inhibitor of DNA binding 4, dominant negative helix-loop-helix protein |
| 182 | 209297_at | ITSN | AF114488 | intersectin short isoform |
| 183 | 209335_at | | AI281593 | |

(continued)

| SEQ NO: | psid | Name | Accession No. | Description |
|---|---|---|---|---|
| | | | | Sequence identifications |
| 184 | 209365_s_at | ECM1 | NM_004425 | extracellular matrix protein 1 |
| 185 | 209386_at | | AI346835 | transmembrane 4 superfamily member 1 |
| 186 | 209485_s_at | ORP1 | AF274714 | oxysterol-binding protein-related protein |
| 187 | 209496_at | | BC000069 | retinoic acid receptor responder 2 |
| 188 | 209505_at | | NM_005654 | nuclear receptor subfam 2, group F, mem 1 |
| 189 | 209506_s_at | | BC004154 | nuclear receptor subfam 2, group F, mem 1 |
| 190 | 209529_at | | AF047760 | phosphatidic acid phosphohydrolase type-2c |
| 191 | 209596_at | | AF245505 | adlican |
| 192 | 209598_at | KIAA0883 | AB020690 | KIAA0883 protein |
| 193 | 209652_s_at | | BC001422 | Similar to placental growth factor, vascular endothelial growth factor-related protein |
| 194 | 209691_s_at | | BC003541 | hypothetical protein FLJ10488 |
| 195 | 209739_s_at | | AI814551 | GS2 gene |
| 196 | 209772_s_at | | X69397 | cell surface antigen |
| 197 | 209781_s_at | T-Star | AF069681 | T-Star |
| 198 | 209792_s_at | | BC002710 | kallikrein 10 |
| 199 | 209810_at | SP-B | J02761 | pulmonary surfactant-associated protein B |
| 200 | 209897_s_at | | AF055585 | neurogenic extracellular slit protein Slit2 |
| 201 | 209924_at | | AB000221 | small inducible cytokine subfamily A (CysCys), mem18, pulmonary/activation-reg |
| 202 | 209946_at | | U58111 | FLT4 ligand |
| 203 | 209990_s_at | | NM_005458 | GABA-B receptor |
| 204 | 210051_at | CAMP-GEFI | U78168 | cAMP-regulated guanine nucleotide exchange factor I |
| 205 | 210055_at | | NM_000369 | thyroid stimulating hormone receptor |
| 206 | 210072_at | | NM_006274 | beta chemokine Exodus-3 |
| 207 | 210078_s_at | | L39833 | K+ channel beta subunit |
| 208 | 210096_at | | NM_000779 | lung cytochrome P450 (IV subfamily) BI |
| 209 | 210298_x_at | FHL1 | AF098518 | four and 1/2 LIM domains1 protein isoform B |
| 210 | 210321_at | | M36118 | cytotoxin serine protease-C |
| 211 | 210342_s_at | TPO | NM_000547 | thyroid peroxidase |
| 212 | 210372_s_at | TPD52L2 | AF208012 | tumor protein D52-like 2 |
| 213 | 210397_at | | U73945 | beta-defensin-1 |
| 214 | 210401_at | | U45448 | P2x1 receptor |
| 215 | 210471_s_at | | U33428 | K+ channel β 1a subunit mRNA, alt spliced |
| 216 | 210473_s_at | p58GTA | M37712 | galactosyltransferase assoc protein kinase |
| 217 | 210605_s_at | | BC003610 | Similar to milk fat globule-EGF factor 8 |

(continued)

| Sequence identifications | | | | |
|---|---|---|---|---|
| SEQ NO: | psid | Name | Accession No. | Description |
| 218 | 210640_s_at | GPCR-Br | U63917 | G protein coupled receptor |
| 219 | 210660_at | LI R-6 | AF025529 | leucocyte immunoglobulin-like receptor-6b |
| 220 | 210727_at | | NM_001741 | Calcitonin, calcitonincalcitonin-rel polypeptide, α |
| 221 | 210762_s_at | HP | NM_006094 | HP protein |
| 222 | 210809_s_at | | D13665 | osf-2 mRNA for osteoblast specific factor 2 |
| 223 | 210827_s_at | ESE-1 | U73844 | epithelial-specific transcription factor ESE-1a |
| 224 | 211100_x_at | | U82278 | immunoglobulin-like transcript 1c |
| 225 | 211101_x_at | | U82276 | immunoglobulin-like transcript 1a |
| 226 | 211102_s_at | | U82277 | immunoglobulin-like transcript 1 b |
| 227 | 211161_s_at | | AF130082 | collagen, type III, alpha 1 |
| 228 | 211217_s_at | | AF051426 | slow delaved rectifier channel subunit |
| 229 | 211538_s_at | | U56725 | heat shock 70kD protein 2 |
| 230 | 211564_s_at | | BC003096 | Similar to LIM domain protein |
| 231 | 211679_x_at | GABBR2 | AF095784 | GABA-B receptor R2 |
| 232 | 211813_x_at | | AF138303 | decorin D |
| 233 | 211959_at | IGFBP5 | AW007532 | insulin-like growth factor binding protein 5 |
| 234 | 211964_at | | AK025912 | type IV collagen alpha (2) chain |
| 235 | 212067_s_at | | AL573058 | complement component 1, r subcomponent |
| 236 | 212253_x_at | KIAA0728 | BG253119 | KIAA0728 protein |
| 237 | 212294_at | | BG111761 | DKFZp586B0918 |
| 238 | 212328_at | KIAA1102 | AB029025 | KIAA1102 protein |
| 239 | 212344_at | KIAA1077 | AW043713 | KIAA1077 protein |
| 240 | 212353_at | KIAA1077 | AI479175 | KIAA1077 protein |
| 241 | 212354_at | KIAA1077 | BE500977 | KIAA1077 protein |
| 242 | 212464_s_at | FN | X02761 | fibronectin precursor |
| 243 | 212724_at | | NM_005168 | ras homolog gene family, member E |
| 244 | 212738_at | | AV717623 | |
| 245 | 212775_at | | AI978623 | KIAA0657 protein |
| 246 | 212803_at | | NM 005967 | NGFI-A binding protein 2 |
| 247 | 212806_at | KIAA0367 | AL138349 | KIAA0367 protein |
| 248 | 212850_s_at | | AA584297 | low density lipoprotein receptor-rel protein 4 |
| 249 | 212865_s_at | | BF449063 | collagen, type XIV, alpha 1 (undulin) |
| 250 | 212912_at | | AI992251 | Ribosomal pro S6 kinase, 90 kDa, polypep 2 |
| 251 | 212992_at | | AI935123 | |
| 252 | 213029_at | | AL110126 | DKFZp564H1916 |
| 253 | 213306_at | | AA917899 | multiple PDZ domain protein |

(continued)

| SEQ NO: | psid | Name | Accession No. | Description |
|---|---|---|---|---|
| | Sequence identifications | | | |
| 254 | 213381_at | | N91149 | DKFZp586M2022 |
| 255 | 213423_x_ at | | AI884858 | Putative prostate cancer tumor suppressor |
| 256 | 213553_x_at | | W79394 | apolipoprotein C-I |
| 257 | 213668_s_at | | AI989477 | SRY (sex determining region Y)-box 4 |
| 258 | 213693_s_at | | AI610869 | mucin 1, transmembrane |
| 259 | 213800_at | | X04697 | complement factor H 38-kDa N-term frag |
| 260 | 213904_at | | AL390170 | DKFZp547E184 |
| 261 | 213924_at | FLJ11585 | BF476502 | hypothetical protein FLJ11585 |
| 262 | 214023_x_at | | AL533838 | tubulin, beta polypeptide |
| 263 | 214175_x_at | | AI254547 | LIM domain protein |
| 264 | 214239_x_at | Mel-18 | AI560455 | Zinc finger protein 144 |
| 265 | 214307_at | | AI478172 | homogentisate 1,2-dioxygenase |
| 266 | 214434_at | KIAA0417 | AB007877 | KIAA0417 gene product |
| 267 | 214632_at | | NM_003872 | neuropilin 2 |
| 268 | 214680_at | | BF674712 | neurotrophic tyrosine kinase receptor 2 |
| 269 | 214702_at | MSF-FN70 | AJ276395 | migration stimulation factor FN70 |
| 270 | 214763_at | FLJ13875 | AK023937 | FLJ13875 |
| 271 | 214803_at | | BF344237 | DKFZp564N1116 |
| 272 | 214955_at | | AI912086 | DNA seq clone 1170K4 chrom 22q12.2-13.1 |
| 273 | 214977_at | FLJ13790 | AK023852 | FLJ13790 |
| 274 | 215016_x_at | KIAA0728 | BC004912 | KLAA0728 protein |
| 275 | 215034_s_at | FLJ13302 | AI189753 | FLJ13302 |
| 276 | 215076_s_at | FLJ11428 | AU144167 | FLJ11428 |
| 277 | 215243_s_at | GJB3 | AF099730 | connexin 31 |
| 278 | 215388_s_at | | X56210 | complement Factor H-related protein 1 |
| 279 | 215442_s_at | | BE740743 | thyroid stimulating hormone receptor |
| 280 | 215443_at | | BE740743 | thyroid stimulating hormone receptor |
| 281 | 215506_s_at | | AK021882 | highly sim to putative tumor sup NOEY2 |
| 282 | 215536_at | | X87344 | DMA, DMB, HLA-Z1, IPP2, LMP2, TAP1, LMP7, TAP2, DOB, DQB2 and RINGB, 9, 13, 14 genes |
| 283 | 216356_x_at | KIAA0734 | AB018277 | KIAA0734 protein |
| 284 | 216470_x_at | | AF009664 | T cell receptor β locus, 3 trypsinogen repeats |
| 285 | 216569_at | FABP3-ps | U72237 | fatty acid-binding protein pseudogene |
| 286 | 217546_at | | R06655 | |
| 287 | 217561_at | | BF447272 | |
| 288 | 217592_at | | AV684859 | |

(continued)

| SEQ NO: | psid | Name | Accession No. | Description |
|---|---|---|---|---|
| | | | | Sequence identifications |
| 289 | 217767_at | | NM_000064 | |
| 290 | 217820_s_at | FLJ10773 | NM_018212 | hypothetical protein FLJ10773 |
| 291 | 217875_s_at | TMEPAI | NM_020182 | transmem, prostate androgen induced RNA |
| 292 | 218002_s_at | SCYB14 | NM_004887 | small inducible cytokine subfam B (Cys-X-mem 14 (BRAK) |
| 293 | 218182_s_at | CLDN1 | NM_021101 | claudin 1 |
| 294 | 218353_at | | NM_025226 | MSTP032 protein |
| 295 | 218368_s_at | FN14 | NM_016639 | type I transmembrane protein Fn14 |
| 296 | 218418_s_at | | NM_015493 | DKFZP434N161 |
| 297 | 218469_at | CKTSF1B1 | NM_013372 | Cvs knot superfamily 1, BMP antagonist 1 |
| 298 | 218537_at | FLJ20568 | NM_017885 | hypothetical protein FLJ20568 |
| 299 | 218546_at | FLJ14146 | NM_024709 | hypothetical protein FLJ14146 |
| 300 | 218613_at | | NM_018422 | hypothetical protein DKFZp761 K1423 |
| 301 | 218653_at | SLC25A15 | NM_014252 | solute carrier family 25 (mitochondrial carrier; ornithine transporter) member 15 |
| 302 | 218691_s_at | RIL | NM_003687 | reversion-induced LIM protein |
| 303 | 218844_at | FLJ20920 | NM_025149 | hypothetical protein FLJ20920 |
| 304 | 218856_at | LOC51323 | NM_016629 | hypothetical protein LOC51323 |
| 305 | 218952_at | SAAS | NM_013271 | granin-like neuroendocrine peptide precursor |
| 306 | 218960_at | TMPRSS4 | NM_016425 | transmembrane protease, serine 4 |
| 307 | 219010_at | FLJ10901 | NM_018265 | hypothetical protein FLJ10901 |
| 308 | 219127_at | MGC11242 | NM_024320 | hypothetical protein MGC11242 |
| 309 | 219191_s_at | BIN2 | NM_016293 | bridging integrator 2 |
| 310 | 219195_at | PPARGC1 | NM_013261 | peroxisome proliferative activated receptor, y, coactivator 1 |
| 311 | 219211_at | USP18 | NM_017414 | ubiquitin specific protease 18 |
| 312 | 219277_s_at | FLJ10851 | NM_018245 | hypothetical protein FLJ10851 |
| 313 | 219331_s at | FLJ10748 | NM_018203 | hypothetical protein FLJ10748 |
| 314 | 219416_at | CSR1 | NM_016240 | CSR1 protein |
| 315 | 219436_s_at | LOC51705 | NM_016242 | endomucin-2 |
| 316 | 219440_at | RAI2 | NM_021785 | retinoic acid induced 2 |
| 317 | 219463_at | HS1119D91 | NM_012261 | sim to S68401 (cattle) glucose induced gene |
| 318 | 219476_at | MGC4309 | NM_024115 | hypothetical protein MGC4309 |
| 319 | 219525_at | FLJ10847 | NM_018242 | hypothetical protein FLJ10847 |
| 320 | 219527_at | FLJ20605 | NM_017898 | hypothetical protein FLJ20605 |
| 321 | 219561_at | LOC51226 | NM_016429 | COPZ2 for nonclathrin coat protein zeta-COP |
| 322 | 219596_at | LOC56906 | NM_020147 | hyp protein from EUROIMAGE 511235 |

(continued)

| Sequence identifications | | | | |
|---|---|---|---|---|
| SEQ NO: | psid | Name | Accession No. | Description |
| 323 | 219597_s_at | DUOX1 | NM_017434 | dual oxidase 1 |
| 324 | 219743_at | HEY2 | NM_012259 | hairyenhancer-of-split rel with YRPW motif 2 |
| 325 | 219749_at | FLJ20967 | NM_022071 | hypothetical protein FLJ20967 |
| 326 | 219836_at | MGC10796 | NM_024508 | hypothetical protein MGC10796 |
| 327 | 219855_at | FLJ10628 | NM_018159 | hypothetical protein FLJ10628 |
| 328 | 219856_at | MGC2742 | NM_023938 | hypothetical protein MGC2742 |
| 329 | 219926_at | POP3 | NM_022361 | popeye protein 3 |
| 330 | 219932_at | VLCS-H1 | NM_014031 | v long-chain acyl-CoA synthetase homolog 1 |
| 331 | 219958_at | FLJ11190 | NM_018354 | hypothetical protein FLJ11190 |
| 332 | 220034_at | | NM_007199 | interleukin-1 receptor-associated kinase M |
| 333 | 220108_at | GNA14 | NM_004297 | guanine nucl binding protein (G protein), $\alpha$ 14 |
| 334 | 220332_at | CLDN16 | NM_006580 | claudin 16 |
| 335 | 220595_at | DKFZp434B0 417 | NM_013377 | hypothetical protein DKFZp434B0417 |
| 336 | 220751_s_at | C5ORF4 | NM_016348 | chromosome 5 open reading frame 4 |
| 337 | 221009_s_at | PGAR | NM_016109 | PPAR(gamma) angiopoietin related protein |
| 338 | 221073_s_at | NOD1 | NM_006092 | caspase recruitment domain 4 |
| 339 | 221147_x_at | wwox | NM_018560 | WW domain-containing oxidoreductase |
| 340 | 221266_s_at | LOC81501 | NM_030788 | DC-specific transmembrane protein |
| 341 | 221270_s_at | TGT | NM_031209 | tRNA-guanine transglycosylase |
| 342 | 221489_s_at | | AF227517 | sprouty (Drosophila) homolog 4 |
| 343 | 221577_x_at | | AF003934 | prostate differentiation factor |
| 344 | 221636_s_at | | AL136931 | DKFZp586G2122 |
| 345 | 221701_s_at | | AF352728 | STRA6 isoform 1 mRNA, alternatively spliced |
| 346 | 221724_s_at | | AF200738 | C-type lectin DDB27 short form |
| 347 | 221795_at | | AI346341 | Similar to hypothetical protein FLJ20093 |
| 348 | 221796_at | | AA707199 | Similar to hypothetical protein FLJ20093 |
| 349 | 221799_at | KIAA1402 | AB037823 | KIAA1402 protein |
| 350 | 221870_at | | AI417917 | FLJ22356 fis |
| 351 | 221900_at | | AI806793 | collagen, type VIII, alpha 2 |
| 352 | 221928_at | | AI057637 | Weakly sim to 2109260A B cell growth factor |
| 353 | 221959_at | | BE672313 | highly sim to HSU79298 Human clone 23803 |
| 354 | 32128_at | | Y13710 | alternative activated macrophage specific CC chemokine 1 |
| 355 | 37004_at | SP-B | J02761 | pulmonary surfactant-associated protein B |
| 356 | 37117_at | | Z83838 | GTPase-activating protein sim to rhoGAP protein. ribosomal protein L6 pseudogene |
| 357 | 37152_at | | L07592 | peroxisome proliferator activated receptor |

(continued)

| SEQ NO: | psid | Name | Accession No. | Description |
|---|---|---|---|---|
| Sequence identifications | | | | |
| 358 | 37408_at | KIAA0709 | AB014609 | KIAA0709 protein |
| 359 | 38691_s_at | SP5 | J03553 | pulmonary surfactant protein |
| 360 | 45297_at | | AI417917 | |
| 361 | 63305_at | | D81792 | |
| 362 | 823_at | | HSU84487 | CX3C chemokine precursor, alt spliced |
| 363 | 91920_at | | AI205180 | |
| 364 | | | | SGENE forward primer |
| 365 | | | | SGENE reverse primer |
| 366 | | | | SGENE probe |
| 367 | | | | TESTICAN1 forward primer |
| 368 | | | | TESTICAN1 reverse primer |
| 369 | | | | TESTICAN1 probe |
| 370 | | | | GABRE forward primer |
| 371 | | | | GABRE reverse primer |
| 372 | | | | GABRE probe |
| 373 | | | | CDH3 forward primer |
| 374 | | | | CDH3 reverse primer |
| 375 | | | | CDH3 probe |
| 376 | | | | FN1 forward primer |
| 377 | | | | FN1 reverse primer |
| 378 | | | | FN1 probe |
| 379 | | | | TPO-1 forward primer |
| 380 | | | | TPO-1 reverse primer |
| 381 | | | | TPO-1 probe |
| 382 | | | | TPO-2 forward primer |
| 383 | | | | TPO-2 reverse primer |
| 384 | | | | TPO-2 probe |
| 385 | | | | KCNAB1-1 forward primer |
| 386 | | | | KCNAB1-1 reverse primer |
| 387 | | | | KCNAB1-1 probe |
| 388 | | | | KCNAB1-2 forward primer |
| 389 | | | | KCNAB1-2 reverse primer |
| 390 | | | | KCNAB1-2 probe |
| 391 | | | | FABP4-1 forward primer |
| 392 | | | | FABP4-1 reverse primer |
| 393 | | | | FABP4-1 probe |

(continued)

| Sequence identifications | | | | |
|---|---|---|---|---|
| SEQ NO: | psid | Name | Accession No. | Description |
| 394 | | | | FABP4-2 forward primer |
| 395 | | | | FABP4-2 reverse primer |
| 396 | | | | FABP4-2 probe |
| 397 | | | | DOI1-1 forward primer |
| 398 | | | | DOI1-1 reverse primer |
| 399 | | | | DOI1-1 probe |
| 400 | | | | DOI1-2 forward primer |
| 401 | | | | DOI1-2 reverse primer |
| 402 | | | | DOI1-2 probe |
| 403 | | | | B-ACTIN forward primer |
| 404 | | | | B-ACTIN reverse primer |
| 405 | | | | B-ACTIN probe |
| 406 | | | | GOLGIN67 forward primer |
| 407 | | | | GOLGIN67 reverse primer |
| 408 | | | | GOLGIN67 probe |
| 409 | | | | PAX8 forward primer |
| 410 | | | | PAX8 reverse primer |
| 411 | | | | PAX8 probe |
| 412 | | | | HERC forward primer |
| 413 | | | | HERC reverse primer |
| 414 | | | | HERC probe |
| 415 | | | | DDIT3 forward primer |
| 416 | | | | DDIT3 reverse primer |
| 417 | | | | DDIT3 probe |
| 418 | | | | ITM1 forward primer |
| 419 | | | | ITM1 reverse primer |
| 420 | | | | ITM1 probe |
| 421 | | | | C1OF24 forward primer |
| 422 | | | | C1OF2 reverse primer |
| 423 | | | | C1OF2 probe |
| 424 | | | | MOT8 forward primer |
| 425 | | | | MOT8 reverse primer |
| 426 | | | | MOT8 probe |
| 427 | | | | ARG2 forward primer |
| 428 | | | | ARG2 reverse primer |
| 429 | | | | ARG2 probe |

**Claims**

1. A method of diagnosing thyroid cancer, testing indeterminate thyroid fine needle aspirate (FNA) of thyroid nodule samples, or of differentiating between thyroid carcinoma and benign thyroid diseases in a biological sample obtained from a patient; comprising the step of measuring the expression levels in the sample of genes encoding mRNA corresponding to SEQ ID NOs: 199, 207, 255 and 354;
wherein the gene expression levels above or below pre-determined cut-off levels are indicative of thyroid cancer or of thyroid carcinoma; and wherein gene expression is (a) measured on a microarray or gene chip, or

> (b) determined by nucleic acid amplification conducted by polymerase chain reaction (PCR) of RNA extracted from the sample.

2. The method of claim 1 wherein the sample was prepared by a method selected from the group consisting of fine needle aspiration, bulk tissue preparation and laser capture microdissection.

3. The method of claim 2, wherein the bulk tissue preparation is from a biopsy or a surgical specimen.

4. The method of claim 1 further comprising measuring

> a) the expression level of at least one gene encoding mRNA corresponding to SEQ ID NOs: 142, 219, 309, 9, 12 and/or 18; or
> b) the expression level of at least one gene constitutively expressed in the sample.

5. The method of claim 1 wherein the comparison of expression patterns is conducted with pattern recognition methods, which can optionally include the use of a Cox proportional hazards analysis.

6. The method of claim 1 wherein the microarray is a cDNA array or an oligonucleotide array which may optionally comprise one or more internal control reagents.

7. The method of claim 1(b) wherein said PCR is reverse transcription polymerase chain reaction (RT-PCR).

8. The method of claim 7, wherein the RT-PCR further comprises one or more internal controls, for example a method of detecting PAX8 gene expression which may optionally be measured using SEQ ID NOs: 409-411.

9. The use of a composition comprising at least one probe set consisting of SEQ ID NOs: 199, 207, 255 and 354 in the method of claim 1.

10. The use of a kit in the method of claim 1, wherein the kit comprises: materials for detecting isolated nucleic acid sequences, their complements, or portions thereof of a combination of genes encoding mRNA corresponding to SEQ ID NOs: 199, 207, 255 and 354, wherein the kit optionally further comprises reagents for conducting a microarray analysis or a medium through which said nucleic acid sequences, their complements, or portions thereof are assayed.

11. The use of a microarray or gene chip for performing the method of claim 1.

12. The use of claim 11 wherein:

> a) the measurement or characterization is at least about 1.5-fold over- or under-expression; or
> b) the microarray or gene chip comprises a cDNA array or an oligonucleotide array; or
> c) the microarray or gene chip comprises one or more internal control reagents.

**Patentansprüche**

1. Verfahren zur Diagnose von Schilddrüsenkrebs, wobei unbestimmtes Schilddrüsen-Feinnadelaspirat (FNA) von Schilddrüsenknotenproben getestet wird, oder zur Differenzierung zwischen Schilddrüsenkarzinom und gutartigen Schilddrüsen-Krankheiten in einer von einem Patienten erhaltenen biologischen Probe; wobei man in einem Verfahrensschritt die Expressionsniveaus von SEQ ID NO: 199, 207, 255 und 354 entsprechende mRNA codierenden Genen in der Probe misst;

wobei die Genexpressionsniveaus ober- oder unterhalb vorbestimmter Ausschlussniveaus Schilddrüsenkrebs oder Schilddrüsenkarzinom anzeigen; und wobei die Genexpression (a) auf einem Mikroarray oder Gen-Chip gemessen oder

(b) über mittels Polymerasekettenreaktion (PCR) ausgeführter Nukleinsäureamplifikation von aus der Probe extrahierter RNA bestimmt wird.

2. Verfahren nach Anspruch 1, wobei die Probe mit einem aus der aus Feinnadelbiopsie, Gesamtgewebepräparation und LCM (Laser Capture Microdissection) bestehenden Gruppe ausgewählten Verfahren hergestellt wurde.

3. Verfahren nach Anspruch 2, wobei die Gesamtgewebepräparation von einer Biopsie oder einem chirurgischen Präparat erfolgt.

4. Verfahren nach Anspruch 1, bei dem man ferner

a) das Expressionsniveau wenigstens eines SEQ ID NO: 142, 219, 309, 9, 12 und/oder 18 entsprechende mRNA codierenden Gens; oder
b) das Expressionsniveau wenigstens eines konstitutiv in der Probe exprimierten Gens

misst.

5. Verfahren nach Anspruch 1, wobei der Vergleich von Expressionsmustern mit Mustererkennungsmethoden, die gegebenenfalls die Verwendung einer Cox-Regressionsanalyse (Cox Proportional Hazards Analysis) beinhalten können, ausgeführt wird.

6. Verfahren nach Anspruch 1, wobei es sich bei dem Mikroarray um ein cDNA-Array oder ein Oligonukleotid-Array, das gegebenenfalls ein oder mehrere interne Kontrollreagentien enthalten kann, handelt.

7. Verfahren nach Anspruch 1(b), wobei es sich bei der PCR um Reverse-Transkription-Polymerasekettenreaktion (RT-PCR) handelt.

8. Verfahren nach Anspruch 7, wobei die RT-PCR ferner eine oder mehrere interne Kontrollen umfasst, zum Beispiel ein Verfahren zum Nachweis von PAX8-Gen-expression, die gegebenenfalls unter Verwendung von SEQ ID NO: 409-411 gemessen werden kann.

9. Verwendung einer wenigstens einen aus SEQ ID NO: 199, 207, 255 und 354 bestehenden Sondensatz umfassenden Zusammensetzung beim Verfahren nach Anspruch 1.

10. Verwendung eines Kits beim Verfahren nach Anspruch 1, wobei das Kit Materialien zum Nachweisen isolierter Nukleinsäuresequenzen, ihrer Komplemente oder von Teilen davon einer Kombination von SEQ ID NO: 199, 207, 255 und 354 entsprechende mRNA codierenden Genen umfasst, wobei das Kit gegebenenfalls ferner Reagentien zum Ausführen einer Mikroarray-Analyse oder ein Medium, über das die Nukleinsäuresequenzen, ihre Komplemente oder Teile davon getestet werden, umfasst.

11. Verwendung eines Mikroarrays oder Gen-Chips zur Durchführung des Verfahrens nach Anspruch 1.

12. Verwendung nach Anspruch 11, wobei:

a) es sich bei der Messung oder Charakterisierung um wenigstens etwa 1,5-fache Über- oder Unterexpression handelt; oder
b) das Mikroarray bzw. der Gen-Chip ein cDNA-Array oder ein Oligonukleotid-Array umfasst; oder
c) das Mikroarray bzw. der Gen-Chip ein oder mehrere interne Kontrollreagentien umfasst.

**Revendications**

1. Procédé de diagnostic du cancer de la thyroïde, de test d'aspiration à l'aiguille fine (FNA) de thyroïde indéterminé d'échantillons de nodules thyroïdiens, ou de différenciation entre un carcinome thyroïdien et des maladies thyroïdiennes bénignes dans un échantillon biologique obtenu à partir d'un patient ; comprenant l'étape de mesure des taux d'expression dans l'échantillon de gènes codant pour des ARNm correspondant aux SEQ ID NO: 199, 207, 255 et 354 ; dans lequel les taux d'expression génique au-dessus ou au-dessous de seuils de coupure prédéterminés sont indicatifs d'un cancer de la thyroïde d'un carcinome de la thyroïde ; et dans lequel l'expression génique est (a) mesurée sur une micromatrice ou une puce à ADN, ou

(b) déterminée par amplification d'acide nucléique conduite par réaction de polymérase en chaîne (PCR) d'ARN extrait de l'échantillon.

2. Procédé de la revendication 1 dans lequel l'échantillon est préparé par un procédé choisi dans le groupe constitué de l'aspiration à l'aiguille fine, la préparation de tissu en bloc et la microdissection par capture laser.

3. Procédé de la revendication 2, dans lequel la préparation de tissu en bloc provient d'une biopsie ou d'un spécimen chirurgical.

4. Procédé de la revendication 1 comprenant en outre la mesure des

a) taux d'expression d'au moins un gène codant pour un ARNm correspondant à SEQ ID NO: 142, 219, 309, 9, 12 et/ou 18 ; ou
b) taux d'expression d'au moins un gène constitutivement exprimé dans l'échantillon.

5. Procédé de la revendication 1 dans lequel la comparaison de profils d'expression est conduite par des procédés de reconnaissance de profil, qui peut facultativement comprendre l'utilisation d'une analyse des risques proportionnels de Cox.

6. Procédé de la revendication 1 dans lequel la micromatrice est une matrice d'ADNc ou une matrice d'oligonucléotides qui peut facultativement comprendre un ou plusieurs réactifs témoins internes.

7. Procédé de la revendication 1(b) dans lequel ladite PCR est la réaction de polymérase en chaîne par transcription inverse (RT-PCR).

8. Procédé de la revendication 7, dans lequel la RT-PCR comprend en outre un ou plusieurs témoins internes, par exemple un procédé de détection de l'expression génique de PAX8 qui peut facultativement être mesurée en utilisant SEQ ID NO: 409-411.

9. Utilisation d'une composition comprenant au moins un ensemble de sondes constitué de SEQ ID NO: 199, 207, 255 et 354 dans le procédé de la revendication 1.

10. Utilisation d'un kit dans le procédé de la revendication 1, le kit comprenant : des matériaux pour détecter des séquences d'acide nucléique isolées, leurs compléments, ou des parties de celles-ci d'une combinaison de gènes codant pour un ARNm correspondant à SEQ ID NO: 199, 207, 255 et 354, le kit comprenant facultativement en outre des réactifs pour conduire une analyse sur micromatrice ou un support par l'intermédiaire duquel lesdites séquences d'acide nucléique, leurs compléments, ou des parties de celles-ci sont analysés.

11. Utilisation d'une micromatrice ou puce à ADN pour conduire le procédé de la revendication 1.

12. Utilisation de la revendication 11 dans laquelle :

a) la mesure ou caractérisation est une surexpression ou sous-expression d'au moins environ 1,5 fois ; ou
b) la micromatrice ou puce à ADN comprend une matrice d'ADNc ou une matrice d'oligonucléotides ; ou
c) la micromatrice ou puce à ADN comprend un ou plusieurs réactifs témoins internes.

ROC Curves

Figure 1

ROC Plot

Figure 2

204

Figure 3a

Figure 3b

Figure 4a

Figure 4b

Figure 5a

Figure 5b

Figure 5c

Figure 5d

**Figure 6a**

**Figure 6b**

**Figure 7a**

**Figure 7b**

Figure 8a

ROC Curves — LDA AUC = 0.776923

5 – gene signature

ROC Curves — LDA AUC = 0.75641

5 – gene signature
Fresh frozen samples

**Figure 8b**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6136182 A
- US 20030194406 A
- US 20050037439 A
- US 20040137539 A
- US 6436642 B
- US 20030104419 A
- US 20030232350 A
- US 20040002067 A
- US 20030108963 A
- US 20050037463 A
- US 6066449 A
- US 20030118553 A
- US 20030054571 A
- WO 9102061 A
- WO 9856953 A
- WO 2005005601 A
- US 20020114806 A
- US 20030219760 A
- US 20030096782 A
- US 20020168638 A

- WO 2005020784 A
- WO 2004040014 A
- US 20030060614 A
- EP 1393776 A
- WO 02057414 A
- WO 0116158 A
- US 6831063 B
- WO 2004030615 A
- WO 9733995 A
- US 20040241653 A
- WO 2005015236 A
- WO 9203469 A
- WO 9203152 A
- EP 0546053 A
- US 6271002 B
- US 6218122 B
- US 6218114 B
- US 6004755 B
- US 20030194734 A

**Non-patent literature cited in the description**

- **HUANG et al.** *PNAS,* 18 December 2001, vol. 98 (26), 15044-9

- **WEBER et al.** *J Clin Endocrinol Metab.,* May 2005, vol. 90 (5), 2512-21